(19) Europäisches Patentamt
European Patent Office
Office européen des brevets

(11) **EP 3 332 028 B1**

(12) **EUROPEAN PATENT SPECIFICATION**

(45) Date of publication and mention
of the grant of the patent:
**06.01.2021   Bulletin 2021/01**

(21) Application number: **16751551.9**

(22) Date of filing: **05.08.2016**

(51) Int Cl.:
*C12Q 1/689* (2018.01)     *G16B 15/00* (2019.01)
*G16B 20/00* (2019.01)     *G16B 30/00* (2019.01)
*G16B 40/00* (2019.01)

(86) International application number:
**PCT/EP2016/068731**

(87) International publication number:
**WO 2017/021529 (09.02.2017 Gazette 2017/06)**

(54) **GENETIC RESISTANCE PREDICTION AGAINST ANTIMICROBIAL DRUGS IN MICROORGANISM USING STRUCTURAL CHANGES IN THE GENOME**

VORHERSAGE VON GENETISCHER RESISTENZ GEGENÜBER ANTIMIKROBIELLEN ARZNEIMITTELN BEI MIKROORGANISMEN ANHAND VON STRUKTURELLEN VERÄNDERUNGEN IM GENOM

PRÉDICTION DE LA RÉSISTANCE GÉNÉTIQUE AUX MÉDICAMENTS ANTIMICROBIENS CHEZ UN MICRO-ORGANISME BASÉE SUR DES MODIFICATIONS STRUCTURALES DANS LE GÉNOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **06.08.2015   EP 15180042**

(43) Date of publication of application:
**13.06.2018   Bulletin 2018/24**

(73) Proprietor: **Ares Genetics GmbH**
**1030 Vienna (AT)**

(72) Inventors:
• **KELLER, Andreas**
  **66346 Püttlingen (DE)**
• **SCHMOLKE, Susanne**
  **91052 Erlangen (DE)**
• **STÄHLER, Cord Friedrich**
  **69493 Hirschberg an der Bergstraße (DE)**
• **BACKES, Christina**
  **66125 Saarbrücken (DE)**
• **GALATA, Valentina**
  **66123 Saarbrücken (DE)**

(74) Representative: **Schnappauf, Georg**
**ZSP Patentanwälte PartG mbB**
**Hansastraße 32**
**80686 München (DE)**

(56) References cited:
WO-A1-2013/142378     WO-A2-2006/097232
WO-A2-2012/027302     WO-A2-2012/106432

• **D. A. RASKO ET AL: "The Pangenome Structure of Escherichia coli: Comparative Genomic Analysis of E. coli Commensal and Pathogenic Isolates", JOURNAL OF BACTERIOLOGY, vol. 190, no. 20, 1 August 2008 (2008-08-01), pages 6881-6893, XP55303440, US ISSN: 0021-9193, DOI: 10.1128/JB.00619-08 -& RASKO DAVID A ET AL: "Supplemental Material: The pangenome structure of Escherichia coli: comparative genomic analysis of E. coli commensal and pathogenic isolates.", October 2008 (2008-10), JOURNAL OF BACTERIOLOGY OCT 2008, VOL. 190, NR. 20, PAGE(S) 6881 - 6893, XP002764170, ISSN: 1098-5530 pages 1-8, the whole document**
• **MICHAL WOZNIAK ET AL: "An approach to identifying drug resistance associated mutations in bacterial strains", BMC GENOMICS, BIOMED CENTRAL LTD, LONDON, UK, vol. 13, no. Suppl 7, 7 December 2012 (2012-12-07), page S23, XP021127984, ISSN: 1471-2164, DOI: 10.1186/1471-2164-13-S7-S23**

**(Cont. next page)**

- BENJAMIN P. HOWDEN ET AL: "Evolution of Multidrug Resistance during Staphylococcus aureus Infection Involves Mutation of the Essential Two Component Regulator WalKR", PLOS PATHOGENS, vol. 7, no. 11, 10 November 2011 (2011-11-10), pages 1-15, XP55169475, DOI: 10.1371/journal.ppat.1002359
- YU GONGXIN: "GenHtr: a tool for comparative assessment of genetic heterogeneity in microbial genomes generated by massive short-read sequencing", BMC BIOINFORMATICS, BIOMED CENTRAL, LONDON, GB, vol. 11, no. 1, 12 October 2010 (2010-10-12), page 508, XP021071835, ISSN: 1471-2105, DOI: 10.1186/1471-2105-11-508
- B. P. HOWDEN ET AL: "Genomic Analysis Reveals a Point Mutation in the Two-Component Sensor Gene graS That Leads to Intermediate Vancomycin Resistance in Clinical Staphylococcus aureus", ANTIMICROBIAL AGENTS AND CHEMOTHERAPY, vol. 52, no. 10, 21 July 2008 (2008-07-21) , pages 3755-3762, XP55228904, US ISSN: 0066-4804, DOI: 10.1128/AAC.01613-07
- A. D. KENNEDY ET AL: "Epidemic community-associated methicillin-resistant Staphylococcus aureus: Recent clonal expansion and diversification", PROCEEDINGS OF THE NATIONAL ACADEMY OF SCIENCES, vol. 105, no. 4, 29 January 2008 (2008-01-29), pages 1327-1332, XP55228900, US ISSN: 0027-8424, DOI: 10.1073/pnas.0710217105
- CLAIRE CHEWAPREECHA ET AL: "Comprehensive Identification of Single Nucleotide Polymorphisms Associated with Beta-lactam Resistance within Pneumococcal Mosaic Genes", PLOS GENETICS, vol. 10, no. 8, 7 August 2014 (2014-08-07) , page e1004547, XP55204428, DOI: 10.1371/journal.pgen.1004547 cited in the application
- N. STOESSER ET AL: "Predicting antimicrobial susceptibilities for Escherichia coli and Klebsiella pneumoniae isolates using whole genomic sequence data", JOURNAL OF ANTIMICROBIAL CHEMOTHERAPY, vol. 4, 30 May 2013 (2013-05-30), XP55157002, ISSN: 0305-7453, DOI: 10.1093/jac/dkt180 cited in the application
- BANKEVICH ANTON ET AL: "SPAdes: a new genome assembly algorithm and its applications to single-cell sequencing.", JOURNAL OF COMPUTATIONAL BIOLOGY : A JOURNAL OF COMPUTATIONAL MOLECULAR CELL BIOLOGY MAY 2012, vol. 19, no. 5, May 2012 (2012-05), pages 455-477, XP002764171, ISSN: 1557-8666
- PAGE ANDREW J ET AL: "Roary: rapid large-scale prokaryote pan genome analysis.", BIOINFORMATICS (OXFORD, ENGLAND) 15 NOV 2015, vol. 31, no. 22, 20 July 2015 (2015-07-20) , pages 3691-3693, XP002764172, ISSN: 1367-4811

Remarks:
    The complete document including Reference Tables and the Sequence Listing can be downloaded from the EPO website

**Description**

The present invention relates to a method of determining an infection of a patient with at least one microorganism, particularly a bacterial microorganism, potentially resistant to antimicrobial drug treatment, a method of selecting a treatment of a patient suffering from an infection with at least one microorganism, particularly bacterial microorganism, and a method of determining structural changes of the genome of the microorganism, particularly bacterial microorganism, comprising at least one gene, as well as computer program products used in these methods.

Antibiotic resistance is a form of drug resistance whereby a sub-population of a microorganism, e.g. a strain of a bacterial species, can survive and multiply despite exposure to an antibiotic drug. It is a serious health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. According to the World Health Organization's antimicrobial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:

1) Efflux pumps are high-affinity reverse transport systems located in the membrane that transports the antibiotic out of the cell, e.g. resistance to tetracycline.

2) Specific enzymes modify the antibiotic in a way that it loses its activity. In the case of streptomycin, the antibiotic is chemically modified so that it will no longer bind to the ribosome to block protein synthesis.

3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation.

Generally, testing for susceptibility/resistance to antimicrobial agents is performed by culturing organisms in different concentrations of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days during which the patient is treated empirically. Automated systems exist from several companies, e.g. Biomeriux (Vitek), Beckman Coulter (Microscan). A significant reduction of time-to-result is needed especially in patients with life-threatening disease and to overcome the widespread misuse of antibiotics.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

The use of molecular techniques for direct detection of MRSA has become more commonplace especially for screening purposes. Resistance to methicillin is mediated via the mec operon which is part of the staphylococcal cassette chromosome mec (SCCmec). Recently PCR tests were introduced that are based on the detection of the right extremity sequence of the SCCmec in combination with S. aureus specific marker. Initial reports exist that describe culture based susceptibility reports despite detection of the presence of a resistance conferring gene.

For some drugs such it is known that at least two targets are addressed, e.g. in case of Ciprofloxacin (drug bank ID 00537; http://www.drugbank.ca/drugs/DB00537) targets include DNA Topoisomerase IV, DNA Topoisomerase II and DNA Gyrase. It can be expected that this is also the case for other drugs although the respective secondary targets have not been identified yet. In case of a common regulation, both relevant genetic sites would naturally show a co-correlation or redundancy.

It is known that drug resistance can be associated with genetic polymorphisms. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al. (Chewapreecha et al (2014) Comprehensive Identification of single nucleotid polymorphisms associated with beta-lactam resistance within pneumococcal mosaic genes. PLoS Genet 10(8): e1004547) used a comparable approach to identify mutations in gram-positive Streptococcus Pneumonia.

Rasko et al., 2008, J. Bacteriology 190:6881-6893 disclose a comparative genomic analysis of E. coli commensal and pathogenic isolates.

Howden et al., 2011, PLoS 7:e1002359 disclose that the evolution of multidrug resistance during Staphylococcus aureus infection involves mutation of the essential two component regulator WalKR.

Yu, 2010, BMC Bioinformatics 11:508 discloses a tool for comparative assessment of genetic heterogeneity in microbial genomes generated by massive short-read sequencing.

Howden et al., 2008, Antimicrobial Agents and Chemotherapy 52:3755-3762 disclose that a genomic analysis reveals that a point mutation in the two-component sensor gene graS leads to intermediate vancomycin resistance in clinical Staphylococcus aureus.

Kennedy et al., 2008, Proc. Natl. Acad. Sci. USA 105:1327-1332 disclose recent clonal expansion and diversification of epidemic community-associated methicillin-resistant Staphylococcus aureus.

International Patent Publication No. WO 2012/027302 A2 discloses systems and methods to facilitate the study of epidemiology and microbial ecology of antibiotic resistance and to be an invaluable tool to rapidly and simultaneously identify organisms and their antimicrobial resistance elements in environmental, food, and clinical samples.

International Patent Publication No. WO 2013/142378 A1 discloses methods for diagnosing and treating patients with acne.

International Patent Publication No. WO 2012/106432 A2 discloses genotypic identification of bacteria that are resistant and subsequent determination of an appropriate therapy.

International Patent Publication No. WO 2006/097232 A2 discloses a micro-array for the detection of antibiotic resistance determinants and mutations in an organism, offering a rapid, sensitive and specific identification of antibiotic resistance profiles.

Bankevich et al., 2012, J Comp. Biol. 19:455-477 disclose a new genome assembly algorithm and its application to single-cell sequencing.

Page et al., 2015, Bioinformatics 31:3691-3693 disclose a rapid large scale prokaryote pan genome analysis using Roary, a tool that rapidly builds large-scale pan-genomes.

In recent studies, genetic tests are taken into account that consider variations in the genome of a microorganism, e.g. a bacterial microorganism. In previous works it could be shown that a faster decision for a treatment could be made using changes in single bases. However, this does not necessarily apply to all antimicrobial drugs, e.g. antibiotics, tested.

The fast and accurate detection of infections with microorganisms, particularly microbial species, e.g. Staphylococcus aureus, and the prediction of response to anti-microbial therapy represent a high unmet clinical need.

This need is addressed by the present invention.

Summary of the Invention

The present invention is directed to the subject-matter set forth in the appended claims.

The inventors found out that structural variations in the genome that relate to more than one base, particularly at least one gene or more genes in an open reading frame can be related to resistance / susceptibility of microorganisms, particularly bacterial microorganisms, to antimicrobial, e.g. antibiotic, drugs. For example, an efflux pump can be present on a plasmid additionally in a genome. Such efflux pump then can transport a medicine/drug like an antibiotic out of the organism, so that it cannot be effective. Thus, a bacterium having such efflux pump on a plasmid is resistant.

According to a first aspect, the present invention relates to a computer implemented method of determining structural variations in a genome of a microorganism, particularly a bacterial microorganism, comprising:

obtaining or providing a first data set of gene sequences of a plurality of clinical isolates of the microorganism, wherein optionally at least a part of the gene sequences of the first data set are assembled;

analyzing the gene sequences of the first data set for structural variations of the genome to obtain a third data set of structural variants, wherein a structural variation of the genome is where at least two adjacent bases in a gene sequence are changed, and wherein the structural variations are detected alignment-free or annotated to a pan-genome;

providing a second data set of antimicrobial drug, e.g. antibiotic, resistance and/or susceptibility of the plurality of clinical isolates of the microorganism;

correlating the third data set with the second data set and statistically analyzing the correlation; and

determining the structural variations in the genome of the microorganism associated with antimicrobial drug, e.g. antibiotic, resistance.

A second aspect of the present invention relates to a diagnostic method of determining an infection of a patient with a microorganism, particularly a bacterial microorganism potentially resistant to antimicrobial drug treatment, comprising the steps of:

a) determining, in sample obtained or provided from the patient, the presence of at least one structural variation in at least one genetic sequence of the microorganism, particularly bacterial microorganism contained in the sample, wherein the structural variation is where at least two adjacent bases in a gene sequence are changed, and wherein the structural variation is determined alignment-free or annotated to a pan-genome, and

b) determining whether the at least one structural variation is associated with antimicrobial drug, e.g., antibiotic, resistance by correlating the at least one structural variation with a data set of antimicrobial drug, e.g., antibiotic, resistance and/or susceptibility of a plurality of clinical isolates of the microorganism and statistically analyzing the correlation, wherein the presence of said at least one structural variation associated with antimicrobial drug, e.g., antibiotic, resistance is indicative of an infection with an antimicrobial drug resistant microorganism in said patient.

A third aspect of the present invention relates to a method of selecting a treatment of a patient suffering from an infection with a potentially resistant microorganism, particularly bacterial microorganism, comprising the steps of:

a) determining, in a sample obtained or provided from the patient, the presence of at least one structural variation in at least one genetic sequence of the microorganism, particularly bacterial microorganism contained in the sample, wherein the structural variation is where at least two adjacent bases in a gene sequence are changed, and wherein the structural variation is determined alignment-free or annotated to a pan-genome,

b) determining whether the at least one structural variation is associated with antimicrobial drug, e.g., antibiotic, resistance by correlating the at least one structural variation with a data set of antimicrobial drug, e.g., antibiotic, resistance and/or susceptibility of a plurality of clinical isolates of the microorganism and statistically analyzing the correlation, wherein the presence of said at least one structural variation associated with antimicrobial drug, e.g., antibiotic, resistance is indicative of a resistance to one or more antimicrobial drugs;

c) identifying said at least one or more antimicrobial drugs; and

d) selecting one or more antimicrobial drugs different from the ones identified in step c) and being suitable for the treatment of the infection with the microorganism, particularly the bacterial microorganism.

A fourth aspect of the present invention relates to a method of determining structural variations of a genome of a microorganism for a clinical isolate of the microorganism, particularly a bacterial microorganism, comprising:

obtaining or providing at least one gene sequence of the clinical isolate of the microorganism, particularly the bacterial microorganism; and

determining the presence of structural variations in the at least one gene sequence of the clinical isolate of the microorganism, particularly bacterial microorganism, wherein a structural variation of the genome is where at least two adjacent bases in a gene sequence are changed, and wherein the structural variation is determined alignment-free or annotated to a pan-genome, providing a data set of antimicrobial drug, e.g. antibiotic, resistance and/or susceptibility of the plurality of clinical isolates of the microorganism;

correlating the data set with the structural variations and statistically analyzing the correlation; and

determining the structural variations in the genome of the microorganism associated with antimicrobial drug, e.g. antibiotic, resistance.

In a fifth aspect the present invention relates to one or more computer program products comprising computer executable instructions which, when executed, perform a method according to the first aspect of the present invention

Further aspects and embodiments of the invention are disclosed in the dependent claims and can be taken from the following description, figures and examples, Figures
The enclosed drawings should illustrate embodiments of the present disclosure and convey a further understanding thereof.

In connection with the description they serve as explanation of concepts and principles of the disclosure. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

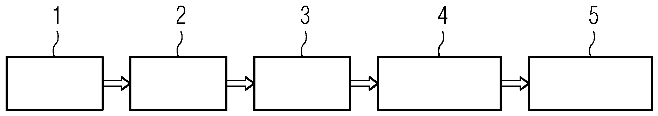

Fig. 1 shows schematically a read-out concept for a diagnostic test according to a method of the present disclosure.

Detailed description of the present disclosure.

Definitions

Unless defined otherwise, technical and scientific terms used herein have the same meaning as commonly understood by one of ordinary skill in the art to which this invention belongs.

An "antimicrobial drug" in the present disclosure refers to a group of drugs that includes antibiotics, antifungals, anti-protozoals, and antivirals. According to certain embodiments, the antimicrobial drug is an antibiotic.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to information which can be derived from the sequence of a nucleic acid molecule, such as the sequence itself or a variation in the sequence as compared to a reference sequence.

The term "mutation" relates to a variation in the sequence as compared to a reference sequence. Such a reference sequence can be a sequence determined in a predominant wild type organism or a reference organism, e.g. a defined and known bacterial strain or substrain. A mutation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, e.g. also a single nucleotide polymorphism (SNP).

In the context of the present disclosure a "sample" is a sample which comprises at least one nucleic acid molecule from a bacterial microorganism. Examples for samples are: cells, tissue, body fluids, biopsy specimens, blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to high-throughput sequencing technologies that parallelize the sequencing process, producing thousands or millions of sequences at once. Examples include Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain aspects, it refers to one or more bacterial species, being either Gram-negative or Gram-positive, particularly one or more of Acinetobacter, Escherichia, e.g. E.coli, Enterobacter, Klebsiella, Proteus, Pseudomonas, Salmonella, Serratia, Shigella and/or Staphylococcus species.

A reference to a microorganism or microorganisms in the present description comprises a reference to one microorganism as well a plurality of microorganisms, e.g. two, three, four, five, six or more microorganisms.

A vertebrate within the present disclosure refers to animals having a vertebrae, which includes mammals - including humans, birds, reptiles, amphibians and fishes. The present disclosure thus is not only suitable for human medicine, but also for veterinary medicine.

According to certain embodiments, the patient in the present methods is a vertebrate, more preferably a mammal and most preferred a human patient.

Before the disclosure is described in exemplary detail, it is to be understood that this disclosure is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005, pp. 781 - 919, might be used as a guideline. Regarding the formulation of a ready-to-use medicament, reference is made to "Remington, The Science and Practice of Pharmacy", 22nd edition, 2013, pp. 777 - 1070.

Assembling of a gene sequence can be carried out by any known method and is not particularly limited.

According to certain embodiments, mutations that were found using alignments can also be compared or matched with alignment-free methods, e.g. for detecting single base exchanges, for example based on contigs that were found by assemblies. For example, reads obtained from sequencing can be assembled to contigs and the contigs can be compared to each other.

A structural variation comprising a change in the genome comprising more than one base refers to a structural variation wherein at least two bases in a gene sequence of a genome of a microorganism that are adjacent are changed, and can refer to e.g. a deletion of multiple (2 or more) nucleotides, an insertion of multiple (2 or more) nucleotides, a substitution of multiple (2 or more) nucleotides, a duplication of a sequence of multiple (2 or more) nucleotides, or a translocation of a sequence of multiple (2 or more) nucleotide. According to certain embodiments, a structural variation can comprise bigger parts sections of the genome, e.g. at least one whole gene in the genome of the microorganism, or even more genes in an open reading frame.

In the present disclosure, not specific changes of single nucleotides are determined, but a determination is based on bigger parts of the genome, as above.

In the present disclosure, a reference sequence is not particularly limited, as long as it is useful as a reference for one or more unknown gene sequences in one or more samples. It can for example be one or more reference genomes, a pan genome or one or more centroids. According to certain embodiments, the reference sequences comprise one or more centroids, wherein a centroid is a representative of a gene group/family/cluster of a genome, e.g. of a microorganism. Centroids can be for example extracted from the database MetaRef (http://metaref.org/), which was used in the present examples, with the extraction from the data base being carried out particularly on November 24, 2014. After the extraction the data from the MetaRef database can be updated continually for further experiments. A list of centroids can be extracted for each organism separately or as a whole. In the present examples a list of centroids was extracted for each organism. The centroid information, e.g. for annotation, can be extracted from databases like IMG (http://img.jgi.doe.gov/), as in the present case, or NCBI.

According to a first aspect, the present disclosure relates to a method of determining structural variations in a genome of a microorganism, particularly a bacterial microorganism, comprising at least a change in the genome comprising more than one base, comprising:

obtaining or providing a first data set of gene sequences of a plurality of clinical isolates of the microorganism, wherein optionally at least a part of the gene sequences of the first data set are assembled; and/or obtaining or providing a first data set of gene sequences of a plurality of clinical isolates of the microorganism and aligning the gene sequences of the first data set to at least one, preferably one, reference sequence;

analyzing the gene sequences of the first data set for structural variations of the genome comprising at least a change in the genome comprising more than one base to obtain a third data set of structural variants;

providing a second data set of antimicrobial drug, e.g. antibiotic, resistance and/or susceptibility of the plurality of clinical isolates of the microorganism;

correlating the third data set with the second data set and statistically analyzing the correlation; and

determining the structural variations in the genome of the microorganism associated with antimicrobial drug, e.g. antibiotic, resistance.

In this method, as well as the other methods of the disclosure, the first data set of gene sequences of a plurality of clinical isolates can be provided or obtained in any way, preferably non-invasive, and can be e.g. provided from in vitro samples.

According to certain embodiments, the obtaining or providing of gene sequences of a plurality of clinical isolates in this method - as well as the other methods of the disclosure - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly a bacterial microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be used to identify the nucleic acids of the microorganism to be identified, by known methods, e.g. fingerprinting methods, comparing genomes and/or aligning to at least one, or more, reference sequences of one or more species of the microorganism of interest, e.g. a reference genome and/or centroids, etc., forming a third data set of, optionally aligned, genes for a microorganism- discarding additional data from other sources, e.g. the vertebrate. For the present method, also the raw data can be used and/or assemblies, at least in part, can be used for forming the third data set. Thus, according to certain embodiments, at least a part of the gene sequences of the first data set can be assembled, wherein assembly can be carried out by any known method and is not particularly limited. In addition, also data from reference sequences, e.g. centroids and/or genomes of known species, e.g. from bacterial species that are already known, e.g. from databases like MetaRef and/or at the NCBI, can be used in the first data set and/or for evaluation of the first data set.

For some organisms, it might be useful in genome-wide association studies to reference the points of interest, e.g. structural variations, to one constant reference for enhanced standardization. In case of the human with a high consistency of the genome and 99% identical sequences among individuals this is easy and represents the standard, as corresponding reference genomes are available in databases.

In case of organisms that trigger infectious diseases (e.g. bacteria and viruses) this is much more difficult, though, and particularly also genetic variations that are not on genes, particularly known genes, can be missed when aligning sequence data to a reference genome. One possibility to overcome this is to fall back on a virtual pan-genome which contains all sequences of a certain genus or to perform reference free variation calling. A further possibility is the analysis of a huge amout of reference sequences, e.g. from MetaRef, and even all available references, which is much more complex. Therein all *n* references from a database (e.g. RefSeq) are extracted and compared with the newly sequenced bacterial genomes *k*. After this, matrices (% of mapped reads, % of covered genome) can be applied and the data can be compared to several reference sequences. In such a case, *n* x *k* complete alignments are carried out. Having a big number of references, stable results can be obtained.

In the present method, gene sequence of the first data set can also be assembled, at least in part, according to certain embodiments with known methods, e.g. by de-novo assembly or mapping assembly. The sequence assembly is not particularly limited, and any known genome assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. a bacterial microorganism, can be removed after the nucleic acids of interest are identified, e.g. by filtering the data out. Such data can e.g. include nucleic acids of a patient, e.g. the vertebrate, e.g. human, and/or other microorganisms, etc. This can be done by e.g. computational subtraction, as developed by Meyerson et al. 2002. For this, also aligning to the genome of the vertebrate, etc., is possible. For aligning, several alignment-tools are available. This way the original data amount from the sample can be drastically reduced.

After such removal of "excess" data, obtaining the third data set can be carried out for the microorganism, e.g. a bacterial microorganism, as described above.

Using these techniques, structural variations in the genome, e.g. in the gene sequences, of the microorganism of interest, e.g. a bacterial microorganism, can be obtained for various species.

When testing these same species for antimicrobial drug, e.g. antibiotic, susceptibility of a number of antimicrobial drugs, e.g. antibiotics, e.g. using standard culturing methods on dishes with antimicrobial drug, e.g. antibiotic, intake, as e.g. described below, the results of these antimicrobial drug, e.g. antibiotic, susceptibility tests can then be cross-referenced/correlated with the structural variations in the genome of the respective microorganism. Using several, e.g. 50 or more than 50, 100 or more than 100, 200 or more than 200, 400 or more than 400, 800 or more than 800, or 900 or more than 900 different isolates of the same or different species of a microorganism, statistical analysis can be carried out on the obtained cross-referenced data between genetic variations and antimicrobial drug, e.g. antibiotic, susceptibility for these microorganisms, using known methods.

Regarding culturing methods, samples of microorganisms can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Also, resistance testing can be carried out by determining e.g. known resistance genes in the different isolates, like in case of methicillin resistant Staphylococcus aureus (MRSA) and methicillin susceptible Staphylococcus aureus (MSSA). For determining resistances, respectively susceptibility, the data from culturing methods and/or from determining known resistance genes, as well as data obtained in different ways, e.g. based on mass spectrometry (possibly also in connection with culturing) can be used.

Correlation of the genetic variations with antimicrobial drug, e.g. antibiotic, resistance can be carried out in a usual way and is not particularly limited. For example, resistances can be correlated to structural variances in the whole genome of the respective microorganism or only parts thereof, for example only coding parts of the genome. In some cases even only genetic variations in genes, e.g. certain genes, or certain mutations in genes can be determined. After correlation, statistical analysis can be carried out.

According to certain embodiments, the data of the first data set can be filtered prior to a possible annotation to a pan-genome and/or reference genome(s) and the correlation with the resistance/susceptibility data.

For example, to reduce the number of similar annotations they can be filtered and aggregated by one or more of the following:

- Only annotations for which the considered structural variation lies on a protein can be kept and the further data discarded
- Only annotations which do not contain "hypothetical proteins" can be kept
- Annotations can be sorted by identification number (ID) and gene product
- For a unique pair of IDs and gene products only the first annotation can be kept, e.g in case of multiple genes in a genome

Also, according to certain embodiments, the following structural variations can be excluded:

1. Constant features and phenotypes (same value or only NA) can be removed (e.g. centroids present in all samples or phenotypes with the result "resistant" for all samples)
2. Almost constant features and phenotypes can also removed, e.g. features whose most frequent value was in >=95% of all samples, ignoring NA values, can be removed (e.g. a centroid is present in >=95% of all samples)
∘ Also phenotypes whose most frequent value was in >=90% of all samples, ignoring NA values, can be removed (e.g. >=90% of all samples are resistant)
3. In addition, as is the case in the present examples for the Gram-positive bacteria S. aureus (Gram neg. bacteria in the Examples did not have partially missing data for phenotypes), only drugs with non-missing data for at least 10% of the samples can be kept.

For statistical analysis, as in the examples, e.g. Fisher's exact two-sided test can be applied with subsequent p-value adjustment over all phenotypes together using FDR and p-value threshold of 0.01 (1e-2). Additionally, 10 permutation tests can be performed by permuting each phenotype separately and applying Fisher's exact test to the centroid presence matrix and permuted phenotypes. The results then can be further filtered by centroid annotation, i.e.

1. Centroids without a gene product name can be removed
2. Centroids whose gene product name contains "putative", "predicted" or "hypothetical" can be removed
3. If there are centroids with same gene product name and gene symbol than only the first one can be kept
4. Centroids without GeneBank accession can be removed

According to certain embodiments, the structural variations can be annotated to a pan-genome of the microorganism and/or annotated to one or more reference sequences, e.g. centroids, of the microorganism. The construction of a pan-genome is not particularly limited and can be done using known methods.

However, other suitable reference genomes (e.g. used in the Examples, but also for other microorganisms) can be found at publicly available data bases like at the NCBI or from MetaRef.

Statistical analysis of the correlation of the gene mutations with antimicrobial drug, e.g. antibiotic, resistance is not particularly limited and can be carried out, depending on e.g. the amount of data, in different ways, for example using analysis of variance (ANOVA), Student's t-test or Fisher's exact test, for example with a sample size n of 50 or more, 100 or more, 200 or more, 300 or more, 400 or more, 500 or more or 600 or more, and a level of significance (a-error-level) of e.g. 0.05 or smaller, e.g. 0.05, preferably 0.01 or smaller. A statistical value can be obtained for each structural variation and/or each genetic sequence in the genome as well as for all antibiotics tested, a group of antibiotics or a single antibiotic. The obtained p-values can also be adapted for statistical errors, if needed.

For statistically sound results a multitude of individuals should be sampled, with $n$ = 50, 100, 200, 300, 400, 500 or 600, and a level of significance (a-error-level) of e.g. 0.05 or smaller, e.g. 0.05, preferably 0.01 or smaller. According to certain embodiments, particularly significant results can be obtained for n = 200, 300, 400, 500 or 600.

For statistically sound results a multitude of individuals should be sampled, with $n$ = 50 or more, 100 or more, 200 or more, 300 or more, 400 or more, 500 or more or 600 or more, and a level of significance (a-error-level) of e.g. 0.05 or smaller, e.g. 0.05, preferably 0.01 or smaller. According to certain embodiments, particularly significant results can be obtained for n = 200 or more, 300 or more, 400 or more, 500 or more or 600 or more.

After the above procedure has been carried out for the individual strains of bacterial species in the Examples, the data disclosed in Tables 1 to 24 were obtained for the statistically best correlations between structural variations and antimicrobial drug, e.g. antibiotic, resistances. In the tables, tables with an odd number, e.g. 1, 3, 5, represent the top 50 results for the statistical analysis as described above, i.e. the structural variations with the best p-value (denoted best_pv in the tables) for at least one antibiotic, whereas the tables with an even number show the top 50 results regarding p-value with at least one drug class ratio of 1.0, so that the structural variation applies to a multitude of drugs and at least a specific drug class, wherein the following applies:

$$\text{Drug class ratio} = \text{number of significant drugs of that class} / \text{number of tested drugs of that class}$$

For example, if two fluoroquinolones were tested but only one has significant p-value, then the drug class ratio for fluoroquinolones is 0.5.

Genetic variations in the gene sequences given in Tables 1 to 24, with regard to the several reference sequences as above, respectively with the sequences with the SEQ ID NOs given in Tables 26a to 26l - attached in the sequence listing, were proven as valid markers for antimicrobial drug, e.g. antibiotic, resistance. According to certain embodiments, structural variations in at least the gene sequences and/or relating to at least the whole sequences between the start coordinate (denoted "Start.Coord" in the tables) and the end coordinate (denoted "End.Coord" in the tables) of the genes, particularly relating to the whole sequence between the start coordinate and the end coordinate, given for consecutive numbers ("No.") 1 to 50 with the highest p-values, as above, in Tables 1 - 24 are obtained by the present method.

When referring to the second data set, wherein the second data set e.g. comprises, respectively is, a set of antimicrobial drug, e.g. antibiotic, resistances of a plurality of clinical isolates, this can, within the scope of the disclosure, also refer to a self-learning data base that, whenever a new sample is analyzed, can take this sample into the second data set and thus expand its data base. The second data set thus does not have to be static and can be expanded, either by external input or by incorporating new data due to self-learning. This is, however, not restricted to the first aspect of the disclosure, but applies to other aspects of the disclosure that refer to a second data set, which does not necessarily have to refer to antimicrobial drug resistance. The same applies, where applicable, to the first data set, e.g. in the first aspect.

According to certain embodiments of the first aspect, the structural variations are detected alignment-free. According to certain embodiments, the structural variations are annotated to a pan-genome of the microorganism and/or annotated to one or more reference sequences.

According to certain embodiments, statistical analysis in the present methods is carried can be carried using Fisher's test with $p < 10^{-3}$, preferably $p < 10^{-6}$, further preferably $p < 10^{-9}$.

The method of the first aspect of the present disclosure, as well as related methods, e.g. according to the 2nd, 3rd and 4th aspect, can, according to certain embodiments, comprise correlating different genetic sites to each other, e.g. structural variations in at least two, three, four, five, six, seven, eight, nine or ten genes. This way even higher statistical significance can be achieved.

According to certain embodiments of the method of the first aspect and related methods - as above, the second data set can be provided by culturing the clinical isolates of the microorganism on agar plates provided with antimicrobial drugs, e.g. antibiotics, at different concentrations, and the second data can be obtained by taking the minimal concentration of the plates that inhibits growth of the respective microorganism.

According to certain embodiments of the method of the first aspect and related methods, the microorganism is a Gram positive bacterial microorganism, particularly a Staphylococcus species, particularly Staphylococcus aureus, and the antimicrobial drug, e.g. antibiotic drug, is selected from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, preferably from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Methicillin, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin, further preferably from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

According to certain embodiments of the method of the first aspect and related methods, the microorganism is a Gram negative bacterial microorganism, particularly one or more of Acinetobacter, Escherichia, e.g. E.coli, Enterobacter, Klebsiella, Proteus, Pseudomonas, Salmonella, Serratia, and/or Shigella species, particularly as denoted in the Examples, and the antimicrobial drug, e.g. antibiotic, is at least one selected from the group of β-lactams, β-lactam inhibitors, quinolines and derivatives thereof, aminoglycosides, polyketides, respectively tetracyclines, and folate synthesis inhibitors, preferably from the group consisting of Amoxicillin/K Clavulanate (AUG), Ampicillin (AM), Aztreonam (AZT), Cefazolin (CFZ), Cefepime (CPM), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).

According to certain embodiments, structural variations in at least the gene sequences and/or relating to at least the whole sequences between the start coordinate (denoted "Start.Coord" in the tables) and the end coordinate (denoted "End.Coord" in the tables) of the genes, particularly relating to the whole sequence between the start coordinate and the end coordinate, given for consecutive numbers ("No.") 1 to 50 with the highest p-values, as above, in Tables 1 - 24, respectively with the sequences with the SEQ ID NOs given in Tables 26a to 26l - attached in the sequence listing, are obtained by the present method, and the antibiotic is at least one chosen from the class of antibiotics in the column designated "sign_phenos_class", particularly the one in the column designated "best_pheno_class", preferably wherein the antibiotic is at least one chosen from the ones in the column designated "sign_phenos", particularly the one in the colum designated "best_pheno".

A second aspect of the present disclosure relates to a diagnostic method of determining an infection of a patient with a microorganism, particularly a bacterial microorganism potentially resistant to antimicrobial drug treatment, comprising the steps of:

a) obtaining or providing a sample containing or suspected of containing a microorganism, particularly a bacterial microorganism, from the patient;
b) determining the presence of at least one structural variation in at least one genetic sequence of the microorganism, particularly bacterial microorganism, as determined by the method of the first aspect, wherein the presence of said at least one structural variation is indicative of an infection with an antimicrobial drug resistant microorganism in said patient.

With this method, any mutations in the genome of a microorganism, e.g. the bacterial species given above and below in the Examples, e.g. a clinical isolate with an unknown strain of the microorganism, particularly bacterial microorganism, correlated with antimicrobial drug, e.g. antibiotic, resistance can be determined and a thorough antimicrobial drug, e.g. antibiotic, resistance profile can be established which can add information to a profile which considers only changes in single bases, e.g. single nucleotide polymorphisms (SNPs).

Again, the different steps can herein be carried out as described with regard to the first aspect of the present disclosure.

According to this aspect, an infection with a microorganism, particularly a bacterial microorganism, in a patient can be determined using sequencing methods, as well as a resistance to antimicrobial drugs, e.g. antibiotics, of the microorganism can be determined in a short amount of time compared to conventional methods.

According to certain aspects, structural variations in at least two, three, four, five, six, seven, eight, nine or ten genetic sequences are determined. This way even higher statistical significance can be achieved.

A third aspect of the present disclosure relates to a method of selecting a treatment of a patient suffering from an infection with a potentially resistant microorganism, particularly bacterial microorganism, comprising the steps of:

a) obtaining or providing a sample containing or suspected of containing a microorganism, particularly a bacterial microorganism, from the patient;
b) determining the presence of at least one structural variation in at least one genetic sequence of the microorganism, particularly bacterial microorganism, as determined by the method of the first aspect, wherein the presence of said at least one structural variation is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism, particularly the bacterial microorganism.

This method can be carried out similarly to the second aspect of the disclosure and enables a fast was to select a suitable treatment with antibiotics for any infection with an unknown microorganism, particularly bacterial microorganism, e.g. Staphylococcus aureus.

In this method, as well as similar ones, no aligning is necessary, as the unknown sample can be directly correlated, after the genome or genome sequences are produced, with the second data set, and thus genetic variations and antimicrobial drug, e.g. antibiotic, resistances can be determined. The first data set can be assembled, for example, using known techniques.

According to certain embodiments, statistical analysis in the present method is carried out using Fisher's test with $p < 10^{-3}$, preferably $p < 10^{-6}$, preferably $p < 10^{-9}$. Also, according to certain embodiments, the method further comprises correlating different genetic sites to each other.

According to certain aspects, structural variations in at least two, three, four, five, six, seven, eight, nine or ten genetic sequences are determined. This way even higher statistical significance can be achieved.

A fourth aspect of the present disclosure relates to a method of determining structural variations of a genome of a microorganism for a clinical isolate of the microorganism, particularly a bacterial microorganism, comprising:

   obtaining or providing at least one gene sequence of the clinical isolate of the microorganism, particularly the bacterial microorganism; and
   determining the presence of structural variations in the at least one gene sequence of the clinical isolate of the microorganism, particularly bacterial microorganism, as determined by the method of the first aspect.

With this method, antimicrobial drug, e.g. antibiotic, resistances in an unknown isolate of a microorganism, e.g. Staphylococcus aureus, can be determined.

A simple read out concept for a diagnostic test as described in this aspect is shown schematically in Fig. 1.

According to Fig. 1, a sample 1, e.g. blood from a patient, is used for molecular testing 2, e.g. using next generation sequencing (NGS), and then a molecular fingerprint 3 is taken, e.g. in case of NGS a sequence of selected genomic/plasmid regions or the whole genome is assembled. This is then compared to a reference library 4 containing several reference sequences and/or a pan-genome, i.e. selected sequences or the whole sequence are/is compared to one or more reference sequences and/or a pan-genome, and structural variations (, sequence / gene additions/deletions, etc.) are correlated with susceptibility/resistance profiles of reference sequences the reference library. The reference library 4 herein contains many genomes and/or a pan-genome and is different from a reference genome. Then the result 5 is reported which can comprise ID (pathogen identification), i.e. a list of all (pathogenic) species identified in the sample, and AST (antimicrobial susceptibility testing), i.e. a list including a susceptibility /resistance profile for all species listed, based on structural variations.

According to certain embodiments, statistical analysis in the present method is carried out using Fisher's test with $p < 10^{-3}$, preferably $p < 10^{-6}$, preferably $p < 10^{-9}$. Also, according to certain embodiments, the method further comprises correlating different genetic sites to each other.

According to certain aspects, structural variations in at least two, three, four, five, six, seven, eight, nine or ten genetic sequences are determined. This way even higher statistical significance can be achieved.

Again, in the third and fourth aspect, the different steps can herein be carried out as described with regard to the first aspect of the present disclosure.

In a fifth aspect the present disclosure relates to one or more computer program products comprising computer executable instructions which, when executed, perform a method according to any one of the first to the fourth aspect of the present disclosure.

In certain embodiments the computer program product is one on which program commands or program codes of a computer program for executing said method are stored. According to certain embodiments the computer program product is a storage medium. As noted above, the computer program products of the present disclosure can be self-learning, e.g. with respect to the first and second data sets.

In order to obtain the best possible information from the highly complex genetic data and develop an optimum model for diagnostic and therapeutical uses as well as the methods of the present disclosure - which can be applied stably in clinical routine - a thorough in silico analysis can be necessary. The proposed principle is based on a combination of different approaches, e.g. assembly of the gene sequences and/or genome of the microorganisms, at least in part and optionally annotating the sequences to one or more reference sequences and/or a pan-genome, and/or alignment of the sequence data of the clinical isolate to be determined with one or more reference sequences and/or a pan-genome, and correlation of structural variations found in every sample, e.g. from each patient, respectively an unknown clinical isolate, with all references and drugs, e.g. antibiotics, or only one or some of them, and search for structural variations which occur in one or several drug and one or several strains.

Using the above steps a list of structural variations s with regard to one or more reference sequences and/or a pan-genome is generated. These can be stored in databases and statistical models can be derived from the databases. The statistical models can be based on at least one or more structural variations in at least one or more sequences.

Statistical models that can be trained can be combined from structural variations and sequences. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, gene sequences or parts thereof can be sequenced from a patient to be diagnosed. Afterwards, core characteristics can be derived from the sequence data which can be used to predict resistance. These are the points in the database used for the final model, i.e. at least one structural variation, but also combinations of structural variations, etc.

The corresponding characteristics can be used as input for the statistical model and thus enable a prognosis for new patients. Not only the information regarding all resistances of all microorganisms, against all or only some or one drugs, e.g. antibiotics, can be integrated in a computer decision support tool, but also corresponding directives (e.g. EUCAST) so that only treatment proposals are made that are in line with the directives.

An eighth aspect of the present disclosure relates to the use of the computer program product according to the fifth aspect, e.g. for determining structural variations of a genome of a microorganism for a clinical isolate of the microorganism in the fourth aspect of the disclosure and/or for use in the diagnostic method of the second method of the disclosure and/or for selecting a treatment in the third aspect of the present disclosure and/or in the method of the first aspect of the present disclosure.

A sixth aspect of the present disclosure discloses a diagnostic method of determining an infection of a patient with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), potentially resistant to antimicrobial drug, e.g. antibiotic, treatment, comprising the steps of:

> a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;
> b) determining the presence of at least one structural variation in at least two sequences from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, wherein the presence of said at least two structural variations is indicative of an infection with an antimicrobial drug, e.g. antibiotic, resistant microorganism, preferably bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), in said patient.

As noted above, genetic variations, particularly structural variations, in the gene sequences given in Tables 1 to 24, with regard to the several reference sequences as above, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, were proven as valid markers for antimicrobial drug, e.g. antibiotic, resistance. According to certain embodiments, structural variations in at least the gene sequences and/or relating to at least the whole sequences between the start coordinate (denoted "Start.Coord" in the tables) and the end coordinate (denoted "End.Coord" in the tables) of the genes, particularly relating to the whole sequence between the start coordinate and the end coordinate, given for consecutive numbers ("No.") 1 to 50 with the highest p-values, as above, in Tables 1 - 24, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, are obtained by the present method.

According to certain embodiments, structural variations in at least the gene sequences and/or relating to at least the whole sequences between the start coordinate (denoted "Start.Coord" in the tables) and the end coordinate (denoted "End.Coord" in the tables) of the genes, particularly relating to the whole sequence between the start coordinate and the end coordinate, given for consecutive numbers ("No.") 1 to 50 with the highest p-values, as above, in Tables 1 - 24, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, are obtained by the present method, and the antibiotic is at least one chosen from the class of antibiotics in the column designated "sign_phenos_class", particularly the one in the column designated "best_pheno_class",preferably wherein the antibiotic is at least one chosen from the ones in the column designated "sign_phenos", particularly the one in the colum designated "best_pheno".

An infection of a patient with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), potentially resistant to antimicrobial drug treatment herein means an infection of a patient with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), wherein it is unclear if the microorganism, preferably bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), is susceptible to treatment with a specific antimicrobial drug or if it is resistant to the antimicrobial drug.

In step b) above, as well as corresponding steps, at least one structural variation in at least two positions is determined, so that in total at least two structural variations are determined, wherein the two structural variations are in different positions, respectively are different sequences.

In this method, as well as the other methods of the disclosure, the sample can be provided or obtained in any way, preferably non-invasive, and can be e.g. provided as an *in vitro* sample or prepared as *in vitro* sample.

According to certain aspects, structural variations in at least two, three, four, five, six, seven, eight, nine or ten positions, respectively sequences, are determined in any of the methods of the present disclosure, e.g. in at least two positions, respectively sequences, or in at least three positions, respectively sequences. Instead of testing only single positions, respectively sequences, a combination of several variant positions, respectively sequences, can improve the prediction accuracy and further reduce false positive findings that are influenced by other factors. Therefore, it is in particular preferred to determine the presence of structural variations in 2, 3, 4, 5, 6, 7, 8 or 9 (or more) sequences selected from the respective Table 1 - 24, respectively the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l.

According to certain embodiments, the obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient in this method - as well as the other methods of the disclosure - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of the microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the first to fourth aspect of the present disclosure.

In a seventh aspect, the present disclosure relates to a method of selecting a treatment of a patient suffering from an infection with a potentially resistant microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), comprising the steps of:

a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;

b) determining the presence of at least one structural variation in at least two sequences from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, wherein the presence of said at least two structural variations is indicative of a resistance to one or more antimicrobial, e.g. antibiotic, drugs;

c) identifying said at least one or more antimicrobial, e.g. antibiotic, drugs; and

d) selecting one or more antimicrobial, e.g. antibiotic, drugs different from the ones identified in step c) and being suitable for the treatment of the infection with the microorganism, preferably bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species) .

In this method, the steps a) of obtaining or providing a sample and b) of determining the presence of at least one structural variation are as in the method of the sixth aspect.

The identification of the at least one or more antimicrobial, e.g. antibiotic, drug in step c) is then based on the results obtained in step b) and corresponds to the antimicrobial, e.g. antibiotic, drug(s) that correlate(s) with the structural variations. Once these antimicrobial drugs, e.g. antibiotics, are ruled out, the remaining antimicrobial drugs, e.g. antibiotic drugs/antibiotics, can be selected in step d) as being suitable for treatment.

In the description, references to the sixth and seventh aspect also apply to the 9th, 10th, 11th and 12th aspect, referring to the same positions, respectively sequences, unless clear from the context that they don't apply.

According to certain embodiments, the microorganism is a Gram positive bacterial microorganism, particularly a Staphylococcus species, particularly Staphylococcus aureus, and the anti-microbial drug, e.g. antibiotic drug, is selected from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, preferably from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Methicillin, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin, further preferably from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

According to certain embodiments, the microorganism is a Gram negative bacterial microorganism, particularly one or more of Acinetobacter, Escherichia, e.g. E.coli, Enterobacter, Klebsiella, Proteus, Pseudomonas, Salmonella, Serratia, and/or Shigella species, particularly as denoted in the Examples, and the antimicrobial drug, e.g. antibiotic, is at least one selected from the group of β-lactams, β-lactam inhibitors, quinolines and derivatives thereof, aminoglycosides, polyketides, respectively tetracyclines, and folate synthesis inhibitors, preferably from the group consisting of Amoxicillin/K Clavulanate (AUG), Ampicillin (AM), Aztreonam (AZT), Cefazolin (CFZ), Cefepime (CPM), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).

In the methods of the disclosure the resistance of the microorganism, preferably bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), to one or more antimicrobial, e.g. antibiotic, drugs can be determined according to certain embodiments.

According to certain embodiments of the sixth and/or seventh aspect of the disclosure, the resistance of the microorganism, preferably bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), against 1, 2, 3, 4, 5, 6, 7, 8, 9, 10, 11, 12, 13, 14, 15 or 16, 17, 18, 19, 20 or more antibiotic drugs is determined.

According to certain embodiments of the sixth and/or seventh aspect of the disclosure, determining the nucleic acid sequence information with the sequences having a structural variation or the presence of a structural variation comprises using a next generation sequencing or high throughput sequencing method. According to preferred embodiments of the sixth and/or seventh aspect of the disclosure, a partial or entire genome sequence of a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), is determined by using a next generation sequencing or high throughput sequencing method.

A ninth aspect of the present disclosure is directed to a method of treating a patient suffering from an antimicrobial drug, e.g. antibiotic, resistant infection with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), comprising the steps of:

    a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;

b) determining the presence of at least one structural variation in at least two sequences from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, wherein the presence of said at least two structural variations is indicative of a resistance to one or more antimicrobial, e.g. antibiotic, drugs;

c) identifying said at least one or more antimicrobial, e.g. antibiotic, drugs;

d) selecting one or more antimicrobial, e.g. antibiotic, drugs different from the ones identified in step c) and being suitable for the treatment of the infection with the microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species); and

e) treating the patient with said one or more antimicrobial, e.g. antibiotic, drugs.

Herein, steps a) to d) can be carried out as described with respect to the seventh aspect. Step e) can be sufficiently carried out without being restricted and can be done e.g. non-invasively.

A tenth aspect of the present disclosure discloses a diagnostic method of determining an infection of a patient with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), potentially resistant to antimicrobial drug, e.g. antibiotic, treatment, comprising the steps of:

a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;

b) determining the presence of at least one structural variation in at least one sequence from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, wherein the presence of said at least one structural variation is indicative of an infection with an antimicrobial drug, e.g. antibiotic, resistant microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), in said patient.

In an eleventh aspect, the present disclosure relates to a method of selecting a treatment of a patient suffering from an infection with a potentially resistant microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), comprising the steps of:

a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;

b) determining the presence of at least one structural variation in at least one sequence from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, wherein the presence of said at least one structural variation is indicative of a resistance to one or more antimicrobial, e.g. antibiotic, drugs;

c) identifying said at least one or more antimicrobial, e.g. antibiotic, drugs; and

d) selecting one or more antimicrobial, e.g. antibiotic, drugs different from the ones identified in step c) and being suitable for the treatment of the infection with the microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species).

Again, in the tenth and the eleventh aspect the steps correspond to those in the sixth or seventh aspect, although only a mutation in at least one gene is determined.

A twelfth aspect of the present disclosure is directed to a method of treating a patient suffering from an antimicrobial drug, e.g. antibiotic, resistant infection with a microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), comprising the steps of:

a) obtaining or providing a sample containing or suspected of containing at least one microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species), from the patient;

b) determining the presence of at least one structural variation in at least one sequence from the group of sequences annotated with Nos. 1 - 50 with regard to the reference sequences, particularly centroids (denoted "Scaffold.External.Accession"), with the sequences names, particularly centroid names (denoted "Scaffold.Name"), given in Tables 1 to 24, particularly with the reference genomes and genome names given in Tables 1a to 24 a, respectively with the sequences with the SEQ ID NOs given in Tables 26a to 26l - attached in the sequence listing, particularly with the sequences with the SEQ ID NOs given in Tables 26a, 26b, 26c, 26d, 26e, 26f, 26g, 26h, 26i, 26j, 26k, or 26l, wherein the presence of said at least one structural variation is indicative of a resistance to one or more antimicrobial, e.g. antibiotic, drugs;

c) identifying said at least one or more antimicrobial, e.g. antibiotic, drugs;

d) selecting one or more antimicrobial, e.g. antibiotic, drugs different from the ones identified in step c) and being suitable for the treatment of the infection with the microorganism, preferably a bacterial microorganism, particularly one as noted above (i.e. one or more of Acinetobacter, Escherichia, e.g. E.coli, Enterobacter, Klebsiella, Proteus, Pseudomonas, Salmonella, Serratia, Shigella and/or Staphylococcus species); and

e) treating the patient with said one or more antimicrobial, e.g. antibiotic, drugs.

Also in the twelfth aspect of the disclosure, steps a) to d) are analogous to the steps in the method of the ninth aspect of the present disclosure. Step e) can again be sufficiently carried out without being restricted and can be done e.g. non-invasively.

In the sixth, seventh, 9th, 10th, 11th and 12th aspect particularly at least 1, 2, 3, 4, 5, 6, 7, 8, 9, 10 or more structural variations are determined for the sequences with the SEQ ID NOs given in Tables 26a, 26b, 26c, 26d, 26e, 26f, 26g, 26h, 26i, 26j, 26k, or 26l, particularly with the SEQ ID NOs given in Tables 26a for Acinetobacter species, particularly A. baumannii, 26b for Enterobacter species, particularly E. aerogenes, 26c for Enterobacter species, particularly E. cloacae, 26d for Escherichia species, particularly E. coli, 26e for Klebsiella species, particularly K. oxytoca, 26f for Klebsiella species, particularly K. pneumoniae, 26g for Proteus species, 26h for Pseudomonas species, particularly P. aeruginosa, 26i for Salmonella species, 26j Serratia species, particularly S. marcescens, 26k for Shigella species, or 26l for Staphylococcus species, particularly S. aureus.

Examples

The present disclosure will now be described in detail with reference to several examples thereof. However, these examples are illustrative and do not limit the scope of the disclosure.

Example 1: Acinetobacter, particularly Acinetobacter baumanii

Whole genome sequencing was carried out in addition to classical antimicrobial susceptibility testing of the same isolates for a cohort of 448 specimens. This allowed performing genome wide correlation studies to find genetic variants (e.g. point mutations, small insertions and deletion, larger structural variants, plasmid copy number gains, gene dosage effects) in the genome and plasmids that are significantly correlated to the resistance against one or several drugs. The approach also allows for comparing the relevant sites in the genome to each other.

In the approach the different sources of genetic resistance regarding structural variances as well as the different ways of how bacteria can become resistant were covered. By measuring clinical isolates collected in a broad geographical area and across a broad time span of three decades a complete picture going far beyond the rather artificial step of laboratory generated resistance mechanisms was tried to be generated.

To this end, a set of 21 clinically relevant antimicrobial agents with 5 different modes of action was put together, and the minimally inhibitory concentration (MIC) of the 21 drugs for the Acinetobacter isolates was measured.

The detailed procedure is given in the following:

Bacterial Strains

The inventors selected 448 Acinetobacter strains from the microbiology strain collection at Siemens Healthcare Diagnostics (West Sacramento, CA) for susceptibility testing and whole genome sequencing.

Antimicrobial Susceptibility Testing (AST) Panels

Frozen reference AST panels were prepared following Clinical Laboratory Standards Institute (CLSI) recommendations. The following antimicrobial agents (with $\mu$g/ml concentrations shown in parentheses) were included in the panels: Amoxicillin/K Clavulanate (0.5/0.25-64/32), Ampicillin (0.25-128), Ampicillin/Sulbactam (0.5/0.25-64/32), Aztreonam (0.25-64), Cefazolin (0.5-32), Cefepime (0.25-64), Cefotaxime (0.25-128), Ceftazidime (0.25-64), Ceftriaxone (0.25-128), Cefuroxime (1-64), Cephalothin (1-64), Ciprofloxacin (0.015-8), Ertepenem (0.12-32), Gentamicin (0.12-32), Imipenem (0.25-32), Levofloxacin (0.25-16), Meropenem (0.12-32), Piperacillin/Tazobactam (0.25/4-256/4), Tetracycline (0.5-64), Tobramycin (0.12-32), and Trimethoprim/Sulfamethoxazole (0.25/4.7-32/608). Prior to use with clinical isolates, AST panels were tested with QC strains. AST panels were considered acceptable for testing with clinical isolates when the QC results met QC ranges described by CLSI16.

Inoculum Preparation

Isolates were cultured on trypticase soy agar with 5% sheep blood (BBL, Cockeysville, Md.) and incubated in ambient air at 35±1°C for 18-24 h. Isolated colonies (4-5 large colonies or 5-10 small colonies) were transferred to a 3 ml Sterile Inoculum Water (Siemens) and emulsified to a final turbidity of a 0.5 McFarland standard. 2 ml of this suspension was added to 25 ml Inoculum Water with Pluronic-F (Siemens). Using the Inoculator (Siemens) specific for frozen AST panels, 5 $\mu$l of the cell suspension was transferred to each well of the AST panel. The inoculated AST panels were incubated in ambient air at 35±1°C for 16-20 h. Panel results were read visually, and minimal inhibitory concentrations (MIC) were determined.

DNA extraction

Four streaks of each Gram-negative bacterial isolate cultured on trypticase soy agar containing 5% sheep blood and cell suspensions were made in sterile 1.5 ml collection tubes containing 50 $\mu$l Nuclease-Free Water (AM9930, Life Technologies). Bacterial isolate samples were stored at -20 °C until nucleic acid extraction. The Tissue Preparation System (TPS) (096D0382-02_01_B, Siemens) and the VERSANT® Tissue Preparation Reagents (TPR) kit (10632404B, Siemens) were used to extract DNA from these bacterial isolates. Prior to extraction, the bacterial isolates were thawed at room temperature and were pelleted at 2000 G for 5 seconds. The DNA extraction protocol DNAext was used for complete total nucleic acid extraction of 48 isolate samples and eluates, 50 $\mu$l each, in 4 hours. The total nucleic acid eluates were then transferred into 96-Well qPCR Detection Plates (401341, Agilent Technologies) for RNase A digestion, DNA quantitation, and plate DNA concentration standardization processes. RNase A (AM2271, Life Technologies) which was diluted in nuclease-free water following manufacturer's instructions was added to 50 $\mu$l of the total nucleic acid eluate for a final working concentration of 20 $\mu$g/ml. Digestion enzyme and eluate mixture were incubated at 37°C for 30 minutes using Siemens VERSANT® Amplification and Detection instrument. DNA from the RNase digested eluate was quantitated using the Quant-iT™ PicoGreen dsDNA Assay (P11496, Life Technologies) following the assay kit instruction, and fluorescence was determined on the Siemens VERSANT® Amplification and Detection instrument. Data analysis was performed using Microsoft® Excel 2007. 25 $\mu$l of the quantitated DNA eluates were transferred into a new 96-Well PCR plate for plate DNA concentration standardization prior to library preparation. Elution buffer from the TPR kit was used to adjust DNA concentration. The standardized DNA eluate plate was then stored at -80°C until library preparation.

Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequncing chemistry (Illumina). Basic sequencing quality parameters were determined using the FastQC quality control tool for high throughput sequence data (Babraham Bioinformatics Institute).

Data analysis

For each organism in the examples, a list of centroids was extracted from MetaRef (http://metaref.org/). Herein, centroids were representatives of genes, etc. in a group/family/cluster. Data for information/annotation regarding the centroids was extracted from the database IMG (http://img.jgi.doe.gov/).

For Acinetobacter, 1380 centroids of A. baumannii were used as reference sequences, extracted from MetaRef.

The centroid presence in samples was evaluated as follows: Sequences of centroids were aligned against the de novo assemblies using BLASTn (http://blast.ncbi.nlm.nih.gov/Blast.cgi).

The following parameters were used:

- BLAST db type: "fasta" and "nucl"
- word size: 11 (default)
- gapopen: 3 (default 5)
- gapextend: 2 (default)
- penalty: -2 (default -3)
- reward: 1 (default)

A centroids was considered as present if the alignment result contained at least one hit with

○ min. 80% identity of assembly sequence and centroid sequence
○ min. 80% of centroid sequence was aligned, computed as

$$centroidcoverage = \frac{cenrtoidalignmentend - centroidalignmentstart + 1}{centroidlength}$$

In the results, a centroid was coded as 0/1 (absent/present)

Association testing and result filtering:
The input data was further filtered as follows to reduce the amount of data:

1. Constant features and phenotypes (same value or only NA) were removed (e.g. centroids present in all samples or phenotypes with "resistant" for all samples)
2. Almost constant features and phenotypes were removed:

○ Features whose most frequent value was in >=95% of all samples, ignoring NA values (e.g. a centroid is present in >=95% of all samples), were removed

○ Penotypes whose most frequent value was in >=90% of all samples, ignoring NA values (e.g. >=95% of all samples are resistant), were removed

3. Particularly for *S. aureus* in Example 12, only drugs with non-missing data for at least 10% of the samples were kept

For statistical analysis, Fisher's exact two-sided test was applied with subsequent p-value adjustment over all phenotypes together using FDR and p-value threshold of 0.01 (1e-2). Additionally, 10 permutation tests were performed by permuting each phenotype separately and applying Fisher's exact test to the centroid presence matrix and permuted phenotypes. The results were further filtered by centroid annotation, i.e.

1. Centroids without a gene product name were removed
2. Centroids whose gene product name contains "putative", "predicted" or "hypothetical" were removed

3. If there are centroids with same gene product name and gene symbol than only the first one was kept

4. Centroids without GeneBank accession were removed

After the above procedure has been carried out, the data disclosed in Tables 1 and 2 were obtained for the statistically best correlations between structural variations and antimicrobial drug, e.g. antibiotic, resistances. Table 1 (as well as the odd Tables 3, 5, 7, 9, 11, 13, 15, 17, 19, 21 and 23 in Examples 2 to 12) represents the top 50 results for structural variations with the best p-value (denoted best_pv in the tables) for at least one antibiotic, whereas Table 2 (as well as the even Tables 4, 6, 8, 10, 12, 14, 16, 18, 20, 22 and 24 in Examples 2 to 12) shows the top 50 results regarding p-value with at least one drug class ratio of 1.0, so that the structural variation applies to a multitude of drugs and at least a specific drug class, wherein the following applies:

$$\texttt{Drug class ratio = number of significant drugs of that}$$

$$\texttt{class / number of tested drugs of that class}$$

For example, if two fluoroquinolones were tested but only one has significant p-value, then the drug class ratio for fluoroquinolones is 0.5.

In Tables 1 and 2, as well as also the following Tables 3 to 24 in Examples 2 to 12, the columns are designated as follows:

◦ No.: Consecutive number from 1 - 50, corresponding to the best p-values

◦ best_pheno: Phenotype (drug) with smallest adj. p-value

◦ best_pheno_class: drug class of best drug

◦ best_pv: adj. p-value of best phenotype calculated using Fisher's exact test and adjusted by FDR (Benjamini Hochberg) method (Benjamini Hochberg, 1995)

◦ sign_phenos: names of all phenotypes with significant adj. p-value separated by ";"

◦ sign_phenos_class: drug classes of all significant drugs

◦ num_<drug class>: number of significant drugs of that drug class

◦ <phenotype>_pv_adj: adjusted p-value of the phenotype calculated using Fisher's exact test and adjusted by FDR (Benjamini Hochberg) method (Benjamini Hochberg, 1995)

◦ Centroid (gene) annotation:

▪ Locus.Tag: Locus of the gene in the genome

▪ Gene.Symbol: Gene symbol

▪ GeneBank.Accession: GeneBank ID for the gene

▪ Chromosom: If gene lies on a plasmids, this column contains plasmid name

▪ Start.Coord/End.Coord: Start and end coordinates of the gene

▪ Strand: "+" for forward and "-" for reverse strand

▪ Scaffold.ID: (IMG) ID of the scaffold containing the gene

▪ Scaffold.External.Accession: Non-IMG ID, e.g. as annotated at the NCBI, RefSeq ID

▪ Scaffold.Name: Genome name and scaffold external accession ID

Table 1a: Data for Acinetobacter, particularly Acinetobacter baumanii

| No. | Scaffold.External Accession | Scaffold.Name | best_pv |
|---|---|---|---|
| 1 | CP001921 | Acinetobacter baumannii 1656-2: CP001921 | 7.16783907123597e-62 |
| 2 | NZ_ACYR01000063 | Acinetobacter sp. 6013113 NZ_ ACYR01000063: NZ_ACYR01000063 | 1.39202289199418e-61 |
| 3 | NC_009085 | Acinetobacter baumannii ATCC 17978: NC_ 009085 | 1.43720401820086e-56 |
| 4 | NC_011595 | Acinetobacter baumannii AB307-0294: NC_ 011595 | 5.41948460225177e-51 |
| 5 | NC_011595 | Acinetobacter baumannii AB307-0294: NC_ 011595 | 7.00413054521082e-47 |
| 6 | NC_011586 | Acinetobacter baumannii AB0057: NC_011586 | 2.06435524702153e-46 |
| 7 | NC_011586 | Acinetobacter baumannii AB0057: NC_011586 | 8.98962816077673e-46 |
| 8 | NC_011595 | Acinetobacter baumannii AB307-0294: NC_ 011595 | 2.76883973579195e-44 |
| 9 | NC_011586 | Acinetobacter baumannii AB0057: NC_011586 | 6.86281420221907e-43 |
| 10 | NZ_ABXK01000013 | Acinetobacter baumannii AB900: NZ_ ABXK01000013 | 5.20862955909241e-42 |
| 11 | NZ_GG704495 | Acinetobacter sp. RUH2624 genomic scaffold supercont1.1: NZ_GG704495 | 9.06181183555984e-42 |
| 12 | NZ_GG704515 | Acinetobacter sp. RUH2624 genomic scaffold supercont1.21: NZ_GG704515 | 9.06181183555984e-42 |
| 13 | NZ_GG704515 | Acinetobacter sp. RUH2624 genomic scaffold supercont1.21: NZ_GG704515 | 9.06181183555984e-42 |
| 14 | NC_014259 | Acinetobacter sp. DR1 chromosome: NC_ 014259 | 9.06181183555984e-42 |
| 15 | NZ_GG704949 | Acinetobacter calcoaceticus RUH2202 genomic scaffold supercont1.1: NZ_GG704949 | 9.06181183555984e-42 |
| 16 | NZ_GG704515 | Acinetobacter sp. RUH2624 genomic scaffold supercont1.21: NZ_GG704515 | 9.06181183555984e-42 |
| 17 | CP002177 | Acinetobacter calcoaceticus PHEA-2: CP002177 | 9.06181183555984e-42 |
| 18 | NZ_GG704515 | Acinetobacter sp. RUH2624 genomic scaffold supercont1.21: NZ_GG704515 | 3.84309843417235e-41 |
| 19 | NZ_GG704495 | Acinetobacter sp. RUH2624 genomic scaffold supercont1.1: NZ_GG704495 | 3.89818602393582e-41 |
| 20 | NC_005966 | Acinetobacter sp. ADP1: NC_005966 | 1.28694655921313e-40 |
| 21 | NZ_ GG704515 | Acinetobacter sp. RUH2624 genomic scaffold supercont1.21: NZ_GG704515 | 1.49093940399756e-40 |
| 22 | NC_011595 | Acinetobacter baumannii AB307-0294: NC_ 011595 | 7.70079013246134e-40 |
| 23 | CP002522 | Acinetobacter baumannii TCDC-AB0715: CP002522 | 9.96749396638303e-40 |
| 24 | NC_011595 | Acinetobacter baumannii AB307-0294: NC_ 011595 | 1.66049576626689e-39 |

(continued)

| No. | Scaffold.External Accession | Scaffold.Name | best_pv |
|-----|------------------------------|---------------|---------|
| 25 | NC_011586 | Acinetobacter baumannii AB0057: NC_011586 | 2.08472354496451e-39 |
| 26 | NC_014259 | Acinetobacter sp. DR1 chromosome: NC_014259 | 6.40954171412503e-39 |
| 27 | NZ_GG705093 | Acinetobacter lwoffii SH145 genomic scaffold supercont1.39: NZ_GG705093 | 1.2155660857651e-38 |
| 28 | NZ_ACYQ01000024 | Acinetobacter sp. 6013150 NZ_ACYQ01000024: NZ_ACYQ01000024 | 2.19910980623625e-38 |
| 29 | NZ_GG704532 | Acinetobacter sp. RUH2624 genomic scaffold supercont1.38: NZ_GG704532 | 5.31361687748575e-38 |
| 30 | NZ_ACYS01000159 | Acinetobacter sp. 6014059 NZ_ACYS01000159: NZ_ACYS01000159 | 6.25755791192393e-38 |
| 31 | NC_010611 | Acinetobacter baumannii ACICU: NC_010611 | 1.6998208344511e-37 |
| 32 | NZ_GG753601 | Acinetobacter sp. SH024 genomic scaffold supercontl.2: NZ_ GG753601 | 2.25174704599278e-37 |
| 33 | CP002522 | Acinetobacter baumannii TCDC-AB0715: CP002522 | 2.43979456355917e-37 |
| 34 | NZ_GG704979 | Acinetobacter johnsonii SH046 genomic scaffold supercont1.16: NZ_ GG704979 | 3.21441196797496e-37 |
| 35 | NC_011586 | Acinetobacter baumannii AB0057: NC_011586 | 4.65662453496211e-37 |
| 36 | NC_011586 | Acinetobacter baumannii AB0057: NC_011586 | 4.97462543315347e-37 |
| 37 | NC_010611 | Acinetobacter baumannii ACICU: NC_010611 | 5.86970772802613e-37 |
| 38 | CP002522 | Acinetobacter baumannii TCDC-AB0715: CP002522 | 5.86970772802613e-37 |
| 39 | NZ_ABXK01000021 | Acinetobacter baumannii AB900: NZ_ABXK01000021 | 7.97065298524188e-37 |
| 40 | CP001921 | Acinetobacter baumannii 1656-2: CP001921 | 8.02210311363916e-37 |
| 41 | NZ_ACYQ01000067 | Acinetobacter sp. 6013150 NZ_ACYQ01000067: NZ_ACYQ01000067 | 1.04988843673196e-36 |
| 42 | NZ_GG704579 | Acinetobacter baumannii ATCC 19606 genomic scaffold supercont1.8: NZ_GG704579 | 2.27416603584469e-36 |
| 43 | NC_010611 | Acinetobacter baumannii ACICU: NC_010611 | 2.41369225680075e-36 |
| 44 | NZ_ADMT01000124 | Acinetobacter haemolyticus ATCC 19194 contig00185: NZ_ADMT01000124 | 8.1885528903387e-36 |
| 45 | NC_011586 | Acinetobacter baumannii AB0057: NC_011586 | 8.6668830227109e-36 |
| 46 | CP002177 | Acinetobacter calcoaceticus PHEA-2: CP002177 | 1.06688951972352e-35 |
| 47 | NZ_GG704579 | Acinetobacter baumannii ATCC 19606 genomic scaffold supercont1.8: NZ_GG704579 | 1.73119071134862e-35 |
| 48 | NC_011595 | Acinetobacter baumannii AB307-0294: NC_011595 | 2.17923094612776e-35 |
| 49 | NZ_GG704499 | Acinetobacter sp. RUH2624 genomic scaffold supercont1.5: NZ_GG704499 | 2.56827101997287e-35 |

(continued)

| No. | Scaffold.External Accession | Scaffold.Name | best_pv |
|---|---|---|---|
| 50 | CP002522 | Acinetobacter baumannii TCDC-AB0715: CP002522 | 2.89444275071491e-35 |

Table 1b: Data for Acinetobacter, particularly Acinetobacter baumanii (continued)

| No. | sign_phenos | sign_phenos_class | best_pheno | best_pheno_class |
|---|---|---|---|---|
| 1 | CP;GM;IMP;LVX;MER;TO;T/S | aminoglycoside;fluoroquinolone;lactam;other | CP | fluoroquinolone |
| 2 | CP;GM;IMP;LVX;MER;TO;T/S | aminoglycoside;fluoroquinolone;lactam;other | CP | fluoroquinolone |
| 3 | CP;GM;IMP;LVX;MER;TO;T/S | aminoglycoside;fluoroquinolone;lactam;other | CP | fluoroquinolone |
| 4 | CP;GM;IMP;LVX;MER;TO;T/S | aminoglycoside;fluoroquinolone;lactam;other | CP | fluoroquinolone |
| 5 | CP;GM;IMP;LVX;MER;TO;T/S | aminoglycoside;fluoroquinolone;lactam;other | CP | fluoroquinolone |
| 6 | CP;GM;IMP;LVX;MER;TO;T/S | aminoglycoside;fluoroquinolone;lactam;other | CP | fluoroquinolone |
| 7 | CP;GM;IMP;LVX;MER;TO;T/S | aminoglycoside;fluoroquinolone;lactam;other | CP | fluoroquinolone |
| 8 | CP;GM;IMP;LVX;MER;TO;T/S | aminoglycoside;fluoroquinolone;lactam;other | CP | fluoroquinolone |
| 9 | CP;GM;IMP;LVX;MER;TO;T/S | aminoglycoside;fluoroquinolone;lactam;other | CP | fluoroquinolone |
| 10 | CP;GM;IMP;LVX;MER;TO;T/S | aminoglycoside;fluoroquinolone;lactam;other | CP | fluoroquinolone |
| 11 | CP;GM;IMP;LVX;MER;TO;T/S | aminoglycoside;fluoroquinolone;lactam;other | CP | fluoroquinolone |
| 12 | CP;GM;IMP;LVX;MER;TO;T/S | aminoglycoside;fluoroquinolone;lactam;other | CP | fluoroquinolone |
| 13 | CP;GM;IMP;LVX;MER;TO;T/S | aminoglycoside;fluoroquinolone;lactam;other | CP | fluoroquinolone |
| 14 | CP;GM;IMP;LVX;MER;TO;T/S | aminoglycoside;fluoroquinolone;lactam;other | CP | fluoroquinolone |
| 15 | CP;GM;IMP;LVX;MER;TO;T/S | aminoglycoside;fluoroquinolone;lactam;other | CP | fluoroquinolone |
| 16 | CP;GM;IMP;LVX;MER;TO;T/S | aminoglycoside;fluoroquinolone;lactam;other | CP | fluoroquinolone |
| 17 | CP;GM;IMP;LVX;MER;TO;T/S | aminoglycoside;fluoroquinolone;lactam;other | CP | fluoroquinolone |
| 18 | CP;GM;IMP;LVX;MER;TO;T/S | aminoglycoside;fluoroquinolone;lactam;other | CP | fluoroquinolone |
| 19 | CP;GM;IMP;LVX;MER;TO;T/S | aminoglycoside;fluoroquinolone;lactam;other | CP | fluoroquinolone |

(continued)

| No. | sign_phenos | sign_phenos_class | best_pheno | best_pheno_class |
|---|---|---|---|---|
| 20 | CP;GM;IMP;LVX;MER;TO;T/S | aminoglycoside;fluoroquinolone;lactam;other | CP | fluoroquinolone |
| 21 | CP;GM;IMP;LVX;MER;TO;T/S | aminoglycoside;fluoroquinolone;lactam;other | CP | fluoroquinolone |
| 22 | CP;GM;IMP;LVX;MER;TO;T/S | aminoglycoside;fluoroquinolone;lactam;other | CP | fluoroquinolone |
| 23 | CP;GM;IMP;LVX;MER;TO;T/S | aminoglycoside;fluoroquinolone;lactam;other | CP | fluoroquinolone |
| 24 | CP;GM;IMP;LVX;MER;TO;T/S | aminoglycoside;fluoroquinolone;lactam;other | CP | fluoroquinolone |
| 25 | CP;GM;IMP;LVX;MER;TO;T/S | aminoglycoside;fluoroquinolone;lactam;other | LVX | fluoroquinolone |
| 26 | CP;GM;IMP;LVX;MER;TO;T/S | aminoglycoside;fluoroquinolone;lactam;other | CP | fluoroquinolone |
| 27 | CP;GM;IMP;LVX;MER;TO;T/S | aminoglycoside;fluoroquinolone;lactam;other | LVX | fluoroquinolone |
| 28 | CP;GM;IMP;LVX;MER;TO;T/S | aminoglycoside;fluoroquinolone;lactam;other | CP | fluoroquinolone |
| 29 | CP;GM;IMP;LVX;MER;TO;T/S | aminoglycoside;fluoroquinolone;lactam;other | LVX | fluoroquinolone |
| 30 | CP;GM;IMP;LVX;MER;TO;T/S | aminoglycoside;fluoroquinolone;lactam;other | CP | fluoroquinolone |
| 31 | CP;GM;IMP;LVX;MER;TO;T/S | aminoglycoside;fluoroquinolone;lactam;other | CP | fluoroquinolone |
| 32 | CP;GM;IMP;LVX;MER;TO;T/S | aminoglycoside;fluoroquinolone;lactam;other | CP | fluoroquinolone |
| 33 | CP;GM;IMP;LVX;MER;TO;T/S | aminoglycoside;fluoroquinolone;lactam;other | CP | fluoroquinolone |
| 34 | CP;GM;IMP;LVX;MER;TO;T/S | aminoglycoside;fluoroquinolone;lactam;other | CP | fluoroquinolone |
| 35 | CP;GM;IMP;LVX;MER;TO;T/S | aminoglycoside;fluoroquinolone;lactam;other | CP | fluoroquinolone |
| 36 | CP;GM;IMP;LVX;MER;TO;T/S | aminoglycoside;fluoroquinolone;lactam;other | CP | fluoroquinolone |
| 37 | CP;GM;IMP;LVX;MER;TO;T/S | aminoglycoside;fluoroquinolone;lactam;other | CP | fluoroquinolone |
| 38 | CP;GM;IMP;LVX;MER;TO;T/S | aminoglycoside;fluoroquinolone;lactam;other | CP | fluoroquinolone |
| 39 | CP;GM;IMP;LVX;MER;TO;T/S | aminoglycoside;fluoroquinolone;lactam;other | CP | fluoroquinolone |
| 40 | CP;GM;IMP;LVX;MER;TO;T/S | aminoglycoside;fluoroquinolone;lactam;other | LVX | fluoroquinolone |
| 41 | CP;GM;IMP;LVX;MER;TO;T/S | aminoglycoside;fluoroquinolone;lactam;other | CP | fluoroquinolone |

(continued)

| No. | sign_phenos | sign_phenos_class | best_ pheno | best_pheno_class |
|---|---|---|---|---|
| 42 | CP;GM;IMP;LVX;MER; TO;T/S | aminoglycoside;fluoroquinolone;lactam; other | T/S | other |
| 43 | CP;GM;IMP;LVX;MER; TO;T/S | aminoglycoside;fluoroquinolone;lactam; other | LVX | fluoroquinolone |
| 44 | CP;GM;IMP;LVX;MER; TO;T/S | aminoglycoside;fluoroquinolone;lactam; other | LVX | fluoroquinolone |
| 45 | CP;GM;IMP;LVX;MER; TO;T/S | aminoglycoside;fluoroquinolone;lactam; other | CP | fluoroquinolone |
| 46 | CP;GM;IMP;LVX;MER; TO;T/S | aminoglycoside;fluoroquinolone;lactam; other | CP | fluoroquinolone |
| 47 | CP;GM;IMP;LVX;MER; TO;T/S | aminoglycoside;fluoroquinolone;lactam; other | T/S | other |
| 48 | CP;GM;IMP;LVX;MER; TO;T/S | aminoglycoside;fluoroquinolone;lactam; other | CP | fluoroquinolone |
| 49 | CP;GM;IMP;LVX;MER; TO;T/S | aminoglycoside;fluoroquinolone;lactam; other | CP | fluoroquinolone |
| 50 | CP;GM;IMP;LVX;MER; TO;T/S | aminoglycoside;fluoroquinolone;lactam; other | CP | fluoroquinolone |

Table 1c: Data for Acinetobacter, particularly Acinetobacter baumanii (continued)

| No. | num_aminoglycoside | num_fluoroquinolone | num_lactam | num_other |
|---|---|---|---|---|
| 1 | 2 | 2 | 2 | 1 |
| 2 | 2 | 2 | 2 | 1 |
| 3 | 2 | 2 | 2 | 1 |
| 4 | 2 | 2 | 2 | 1 |
| 5 | 2 | 2 | 2 | 1 |
| 6 | 2 | 2 | 2 | 1 |
| 7 | 2 | 2 | 2 | 1 |
| 8 | 2 | 2 | 2 | 1 |
| 9 | 2 | 2 | 2 | 1 |
| 10 | 2 | 2 | 2 | 1 |
| 11 | 2 | 2 | 2 | 1 |
| 12 | 2 | 2 | 2 | 1 |
| 13 | 2 | 2 | 2 | 1 |
| 14 | 2 | 2 | 2 | 1 |
| 15 | 2 | 2 | 2 | 1 |
| 16 | 2 | 2 | 2 | 1 |
| 17 | 2 | 2 | 2 | 1 |
| 18 | 2 | 2 | 2 | 1 |

(continued)

| No. | num_aminoglycoside | num_fluoroquinolone | num_lactam | num_other |
|---|---|---|---|---|
| 19 | 2 | 2 | 2 | 1 |
| 20 | 2 | 2 | 2 | 1 |
| 21 | 2 | 2 | 2 | 1 |
| 22 | 2 | 2 | 2 | 1 |
| 23 | 2 | 2 | 2 | 1 |
| 24 | 2 | 2 | 2 | 1 |
| 25 | 2 | 2 | 2 | 1 |
| 26 | 2 | 2 | 2 | 1 |
| 27 | 2 | 2 | 2 | 1 |
| 28 | 2 | 2 | 2 | 1 |
| 29 | 2 | 2 | 2 | 1 |
| 30 | 2 | 2 | 2 | 1 |
| 31 | 2 | 2 | 2 | 1 |
| 32 | 2 | 2 | 2 | 1 |
| 33 | 2 | 2 | 2 | 1 |
| 34 | 2 | 2 | 2 | 1 |
| 35 | 2 | 2 | 2 | 1 |
| 36 | 2 | 2 | 2 | 1 |
| 37 | 2 | 2 | 2 | 1 |
| 38 | 2 | 2 | 2 | 1 |
| 39 | 2 | 2 | 2 | 1 |
| 40 | 2 | 2 | 2 | 1 |
| 41 | 2 | 2 | 2 | 1 |
| 42 | 2 | 2 | 2 | 1 |
| 43 | 2 | 2 | 2 | 1 |
| 44 | 2 | 2 | 2 | 1 |
| 45 | 2 | 2 | 2 | 1 |
| 46 | 2 | 2 | 2 | 1 |
| 47 | 2 | 2 | 2 | 1 |
| 48 | 2 | 2 | 2 | 1 |
| 49 | 2 | 2 | 2 | 1 |
| 50 | 2 | 2 | 2 | 1 |

Table 1d: Data for Acinetobacter, particularly Acinetobacter baumanii (continued)

| No. | CP_pv_adj | GM_pv_adj | IMP_pv_adj | LVX_pv_adj | MER_pv_adj | TO_pv_adj | T/S_pv_adj |
|---|---|---|---|---|---|---|---|
| 1 | 7.17E-34 | 7.62E-37 | 2.99E-16 | 2.51E-58 | 1.71E-29 | 3.85E-19 | 7.12E-37 |
| 2 | 1.39E-61 | 2.93E-36 | 2.52E-16 | 1.14E-57 | 1.26E-28 | 1.01E-18 | 2.48E-36 |

(continued)

| No. | CP_pv_adj | GM_pv_adj | IMP_pv_adj | LVX_pv_adj | MER_pv_adj | TO_pv_adj | T/S_pv_adj |
|---|---|---|---|---|---|---|---|
| 3 | 1.44E-56 | 1.41E-30 | 7.92E-24 | 2.33E-56 | 4.63E-35 | 1.23E-15 | 1.16E-31 |
| 4 | 5.42E-51 | 1.20E-26 | 3.83E-16 | 1.97E-49 | 3.77E-19 | 6.39E-14 | 5.70E-30 |
| 5 | 7.00E-47 | 7.36E-25 | 7.07E-15 | 3.32E-45 | 1.76E-24 | 2.16E-14 | 1.06E-32 |
| 6 | 2.06E-46 | 1.72E-23 | 1.92E-08 | 3.46E-46 | 2.40E-18 | 1.64E-13 | 3.34E-31 |
| 7 | 8.99E-46 | 1.85E-24 | 2.19E-15 | 2.93E-44 | 1.16E-24 | 3.33E-14 | 3.29E-31 |
| 8 | 2.77E-44 | 1.51E-23 | 2.19E-15 | 5.38E-43 | 1.16E-24 | 3.33E-14 | 3.45E-30 |
| 9 | 6.86E-43 | 1.15E-22 | 4.78E-15 | 1.09E-41 | 3.50E-24 | 5.85E-13 | 2.90E-29 |
| 10 | 5.21E-42 | 7.13E-19 | 3.88E-19 | 3.01E-41 | 3.46E-19 | 1.35E-06 | 1.20E-22 |
| 11 | 9.06E-42 | 1.74E-21 | 5.28E-11 | 6.20E-39 | 2.05E-18 | 9.48E-14 | 2.09E-27 |
| 12 | 9.06E-42 | 1.40E-20 | 1.57E-11 | 1.29E-40 | 2.50E-19 | 2.12E-13 | 1.47E-25 |
| 13 | 9.06E-42 | 1.40E-20 | 1.57E-11 | 1.29E-40 | 2.50E-19 | 2.12E-13 | 1.47E-25 |
| 14 | 9.06E-42 | 1.26E-22 | 4.71E-10 | 1.24E-38 | 1.59E-17 | 1.36E-14 | 1.16E-28 |
| 15 | 9.06E-42 | 1.40E-20 | 1.57E-11 | 1.29E-40 | 2.50E-19 | 2.12E-13 | 1.47E-25 |
| 16 | 9.06E-42 | 1.40E-20 | 1.57E-11 | 1.29E-40 | 2.50E-19 | 2.12E-13 | 1.47E-25 |
| 17 | 9.06E-42 | 1.40E-20 | 1.57E-11 | 1.29E-40 | 2.50E-19 | 2.12E-13 | 1.47E-25 |
| 18 | 3.84E-41 | 4.26E-21 | 5.48E-11 | 2.25E-38 | 2.28E-18 | 1.85E-13 | 1.34E-28 |
| 19 | 3.90E-41 | 3.00E-22 | 8.17E-10 | 4.60E-38 | 3.07E-17 | 2.60E-14 | 3.05E-28 |
| 20 | 1.29E-40 | 2.54E-23 | 8.32E-09 | 2.28E-37 | 2.98E-16 | 7.17E-15 | 4.45E-29 |
| 21 | 1.49E-40 | 1.06E-20 | 9.78E-11 | 8.02E-38 | 4.26E-18 | 3.58E-13 | 1.41E-26 |
| 22 | 7.70E-40 | 2.19E-21 | 2.55E-13 | 8.73E-40 | 2.71E-22 | 1.30E-12 | 9.73E-29 |
| 23 | 9.97E-40 | 1.94E-20 | 5.69E-10 | 6.32E-37 | 5.09E-15 | 3.42E-13 | 4.53E-25 |
| 24 | 1.66E-39 | 2.25E-21 | 1.11E-11 | 4.74E-37 | 9.11E-19 | 4.30E-12 | 6.58E-28 |
| 25 | 6.97E-38 | 2.51E-23 | 4.39E-17 | 2.08E-39 | 2.11E-31 | 2.81E-20 | 2.62E-29 |
| 26 | 6.41E-39 | 8.82E-20 | 8.32E-09 | 2.28E-37 | 8.45E-15 | 5.48E-12 | 1.80E-23 |
| 27 | 1.21E-36 | 6.79E-22 | 2.00E-18 | 1.22E-38 | 1.37E-30 | 1.91E-20 | 1.86E-29 |
| 28 | 2.20E-38 | 6.34E-16 | 1.51E-04 | 3.00E-37 | 3.02E-15 | 2.23E-05 | 5.58E-26 |
| 29 | 2.16E-37 | 5.62E-21 | 1.99E-11 | 5.31E-38 | 4.07E-20 | 1.62E-14 | 1.79E-33 |
| 30 | 6.26E-38 | 1.31E-11 | 3.04E-05 | 7.55E-37 | 2.46E-19 | 5.99E-05 | 3.36E-19 |
| 31 | 1.70E-37 | 9.89E-20 | 4.00E-15 | 6.17E-33 | 1.59E-19 | 1.06E-07 | 5.60E-19 |
| 32 | 2.25E-37 | 3.62E-20 | 5.26E-16 | 3.31E-34 | 9.82E-23 | 4.38E-09 | 5.82E-22 |
| 33 | 2.44E-37 | 6.97E-16 | 4.33E-14 | 7.77E-37 | 9.30E-19 | 4.18E-07 | 1.39E-20 |
| 34 | 3.21E-37 | 1.12E-20 | 1.05E-11 | 3.70E-36 | 1.02E-20 | 3.75E-13 | 1.16E-24 |
| 35 | 4.66E-37 | 5.92E-20 | 3.00E-11 | 4.04E-32 | 8.17E-17 | 5.55E-10 | 2.22E-21 |
| 36 | 4.97E-37 | 9.12E-20 | 3.90E-11 | 7.27E-35 | 8.20E-18 | 2.95E-11 | 2.78E-26 |
| 37 | 5.87E-37 | 1.44E-15 | 3.36E-14 | 2.00E-36 | 3.55E-19 | 3.60E-07 | 3.09E-20 |
| 38 | 5.87E-37 | 1.44E-15 | 2.29E-13 | 2.00E-36 | 2.38E-18 | 1.01E-06 | 4.30E-21 |
| 39 | 7.97E-37 | 1.41E-20 | 4.11E-10 | 1.69E-34 | 6.22E-17 | 1.57E-12 | 9.61E-26 |
| 40 | 8.02E-36 | 1.02E-16 | 8.36E-13 | 8.02E-37 | 6.77E-18 | 2.61E-10 | 1.91E-19 |

(continued)

| No. | CP_pv_adj | GM_pv_adj | IMP_pv_adj | LVX_pv_adj | MER_pv_adj | TO_pv_adj | T/S_pv_adj |
|---|---|---|---|---|---|---|---|
| 41 | 1.05E-36 | 5.82E-22 | 6.09E-07 | 1.09E-33 | 3.93E-12 | 9.82E-13 | 1.13E-26 |
| 42 | 2.17E-18 | 1.05E-13 | 2.19E-05 | 7.67E-17 | 1.44E-05 | 1.58E-19 | 2.27E-36 |
| 43 | 2.04E-35 | 2.18E-16 | 4.05E-13 | 2.41E-36 | 4.89E-18 | 7.30E-10 | 2.59E-18 |
| 44 | 2.30E-34 | 4.36E-15 | 1.44E-11 | 8.19E-36 | 1.06E-16 | 9.26E-09 | 1.67E-18 |
| 45 | 8.67E-36 | 6.11E-17 | 3.23E-06 | 1.48E-32 | 1.25E-11 | 1.06E-09 | 8.79E-25 |
| 46 | 1.07E-35 | 6.07E-21 | 9.46E-09 | 3.15E-33 | 4.04E-15 | 6.09E-14 | 1.52E-24 |
| 47 | 2.15E-17 | 3.02E-13 | 2.37E-05 | 3.26E-16 | 2.56E-05 | 2.12E-19 | 1.73E-35 |
| 48 | 2.18E-35 | 2.69E-18 | 1.65E-04 | 2.17E-32 | 2.13E-10 | 9.38E-10 | 2.81E-21 |
| 49 | 2.57E-35 | 5.01E-23 | 1.62E-06 | 3.27E-32 | 1.19E-11 | 4.78E-13 | 2.98E-26 |
| 50 | 2.89E-35 | 4.58E-19 | 5.87E-06 | 4.77E-32 | 5.92E-13 | 2.05E-11 | 8.30E-23 |

Table 1e: Data for Acinetobacter, particularly Acinetobacter baumanii (continued)

| No. | Locus.Tag | Gene. Symbol | GenBank.Accession | Chromosome |
|---|---|---|---|---|
| 1 | ABK1_1135 | | ADX02769 | |
| 2 | A60131_010100011170 | | ZP_06783174 | |
| 3 | A1S_0690 | | YP_001083736 | |
| 4 | ABBFA_001710 | | YP_002325613 | |
| 5 | ABBFA_001640 | | YP_002325543 | |
| 6 | AB57_1876 | | YP_002319238 | |
| 7 | AB57_2151 | | YP_002319507 | |
| 8 | ABBFA_001638 | | YP_002325541 | |
| 9 | AB57_2147 | | YP_002319503 | |
| 10 | AbauAB_010100009672 | | ZP_04661877 | |
| 11 | HMPREF0014_00181 | | ZP_05823167 | |
| 12 | HMPREF0014_02450 | | ZP_05825436 | |
| 13 | HMPREF0014_02452 | | ZP_05825438 | |
| 14 | AOLE_17735 | | YP_003733805 | |
| 15 | HMPREF0012_01352 | | ZP_06057184 | |
| 16 | HMPREF0014_02453 | | ZP_05825439 | |
| 17 | BDGL_001498 | ocd2 | ADY82084 | |
| 18 | HMPREF0014_02489 | | ZP_05825475 | |
| 19 | HMPREF0014_00004 | | ZP_05822990 | |
| 20 | ACIAD0370 | rbfA | YP_045140 | |
| 21 | HMPREF0014_02492 | | ZP_05825478 | |
| 22 | ABBFA_001637 | | YP_002325540 | |
| 23 | ABTW07_1894 | | ADX92323 | |
| 24 | ABBFA_ 000343 | | YP_002324276 | |

(continued)

| No. | Locus.Tag | Gene. Symbol | GenBank.Accession | Chromosome |
|-----|-----------|--------------|-------------------|------------|
| 25 | AB57_2771 | | YP_002320110 | |
| 26 | AOLE_17690 | | YP_003733796 | |
| 27 | HMPREF0017_03097 | | ZP_06071176 | |
| 28 | A6013_010100003618 | | ZP_06795427 | |
| 29 | HMPREF0014_03338 | | ZP_05826324 | |
| 30 | A6014_010100015962 | | ZP_06788050 | |
| 31 | ACICU_00639 | filE | YP_001845298 | |
| 32 | HMPREF0013_02137 | | ZP_06692288 | |
| 33 | ABTW07_2865 | | ADX93289 | |
| 34 | HMPREF0016_03291 | | ZP_06064821 | |
| 35 | AB57_2131 | | YP_002319487 | |
| 36 | AB57_3624 | pilV | YP_002320921 | |
| 37 | ACICU_02614 | | YP_001847273 | |
| 38 | ABTW07_2863 | | ADX93287 | |
| 39 | AbauAB_010100013685 | | ZP_04662667 | |
| 40 | ABK1_2278 | | ADX03912 | |
| 41 | A6013_010100009420 | | ZP_06796568 | |
| 42 | HMPREF0010_03320 | | ZP_05829937 | |
| 43 | ACICU_01819 | | YP_001846478 | |
| 44 | HMP0015_1294 | | ZP_06727085 | |
| 45 | AB57_1430 | | YP_002318806 | |
| 46 | BDGL_000595 | | ADY81181 | |
| 47 | HMPREF0010_03319 | | ZP_0582 9936 | |
| 48 | ABBFA_001847 | | YP_002325750 | |
| 49 | HMPREF0014_00860 | | ZP_05823846 | |
| 50 | ABTW07_1891 | | ADX92320 | |

Table 1f: Data for Acinetobacter, particularly Acinetobacter baumanii (continued)

| No. | Start.Coord | End.Coord | Strand | Scaffold.ID |
|-----|-------------|-----------|--------|-------------|
| 1 | 1235814 | 1236425 | + | 650377914 |
| 2 | 64 | 768 | + | 647008664 |
| 3 | 821275 | 822057 | + | 640069344 |
| 4 | 1834753 | 1835667 | - | 643348583 |
| 5 | 1759838 | 1761280 | + | 643348583 |
| 6 | 1966831 | 1969149 | + | 643348581 |
| 7 | 2232790 | 2233716 | - | 643348581 |
| 8 | 1757930 | 1758571 | - | 643348583 |

(continued)

| No. | Start.Coord | End.Coord | Strand | Scaffold.ID |
|---|---|---|---|---|
| 9 | 2226774 | 2228099 | + | 643348581 |
| 10 | 63870 | 65015 | - | 645061599 |
| 11 | 195165 | 195419 | + | 647535325 |
| 12 | 27528 | 28301 | - | 647535345 |
| 13 | 31818 | 33023 | - | 647535345 |
| 14 | 3783514 | 3783789 | + | 648028017 |
| 15 | 1494626 | 1495630 | + | 646207323 |
| 16 | 33027 | 34796 | - | 647535345 |
| 17 | 1603798 | 1604811 | + | 650377930 |
| 18 | 74534 | 74893 | + | 647535345 |
| 19 | 1819 | 2325 | - | 647535325 |
| 20 | 363575 | 363976 | + | 637000277 |
| 21 | 76454 | 76681 | + | 647535345 |
| 22 | 1757594 | 1757890 | - | 643348583 |
| 23 | 2004366 | 2004983 | + | 650378014 |
| 24 | 370977 | 371978 | + | 643348583 |
| 25 | 2838051 | 2838620 | - | 643348581 |
| 26 | 3773081 | 3773467 | + | 648028017 |
| 27 | 716 | 1162 | + | 646207467 |
| 28 | 6958 | 7416 | - | 647008417 |
| 29 | 12541 | 13101 | - | 647535362 |
| 30 | 7588 | 7713 | + | 647009002 |
| 31 | 705957 | 707216 | + | 642555129 |
| 32 | 856694 | 857281 | - | 647536755 |
| 33 | 3040319 | 3041707 | - | 650378014 |
| 34 | 4553 | 4768 | - | 646207353 |
| 35 | 2213458 | 2214801 | - | 643348581 |
| 36 | 3730668 | 3731222 | - | 643348581 |
| 37 | 2775538 | 2776941 | + | 642555129 |
| 38 | 3038397 | 3039893 | - | 650378014 |
| 39 | 70983 | 71513 | - | 645061607 |
| 40 | 2403829 | 2405136 | + | 650377914 |
| 41 | 2603 | 3454 | + | 647008460 |
| 42 | 159696 | 159998 | + | 647535409 |
| 43 | 1941428 | 1942675 | + | 642555129 |
| 44 | 1088 | 2629 | - | 647018944 |
| 45 | 1505450 | 1506382 | + | 643348581 |
| 46 | 622112 | 622732 | + | 650377930 |

(continued)

| No. | Start.Coord | End.Coord | Strand | Scaffold.ID |
|-----|-------------|-----------|--------|-------------|
| 47 | 158794 | 159609 | + | 647535409 |
| 48 | 1980078 | 1981988 | - | 643348583 |
| 49 | 126664 | 127845 | + | 647535329 |
| 50 | 1999835 | 2001676 | + | 650378014 |

Table 2a: Data for Acinetobacter, particularly Acinetobacter baumanii

| No. | Scaffold.External.Accession | Scaffold.Name | best_pv |
|-----|------------------------------|---------------|---------|
| 1 | CP001921 | Acinetobacter baumannii 1656-2: CP001921 | 7.16783907123597e-62 |
| 2 | NZ_ACYR01000063 | Acinetobacter sp. 6013113 NZ_ACYR01000063: NZ_ACYR01000063 | 1.39202289199418e-61 |
| 3 | NC_009085 | Acinetobacter baumannii ATCC 17978: NC_009085 | 1.43720401820086e-56 |
| 4 | NC_011595 | Acinetobacter baumannii AB307-0294: NC_011595 | 5.41948460225177e-51 |
| 5 | NC_011595 | Acinetobacter baumannii AB307-0294: NC_011595 | 7.00413054521082e-47 |
| 6 | NC_011586 | Acinetobacter baumannii AB0057: NC_011586 | 2.06435524702153e-46 |
| 7 | NC_011586 | Acinetobacter baumannii AB0057: NC_011586 | 8.98962816077673e-46 |
| 8 | NC_011595 | Acinetobacter baumannii AB307-0294: NC_011595 | 2.76883973579195e-44 |
| 9 | NC_011586 | Acinetobacter baumannii AB0057: NC_011586 | 6.86281420221907e-43 |
| 10 | NZ_ABXK01000013 | Acinetobacter baumannii AB900: NZ_ABXK01000013 | 5.20862955909241e-42 |
| 11 | NZ_GG704495 | Acinetobacter sp. RUH2624 genomic scaffold supercont1.1: NZ_GG704495 | 9.06181183555984e-42 |
| 12 | NZ_GG704515 | Acinetobacter sp. RUH2624 genomic scaffold supercont1.21: NZ_GG704515 | 9.06181183555984e-42 |
| 13 | NZ_GG704515 | Acinetobacter sp. RUH2624 genomic scaffold supercont1.21: NZ_GG704515 | 9.06181183555984e-42 |
| 14 | NC_014259 | Acinetobacter sp. DR1 chromosome: NC_014259 | 9.06181183555984e-42 |
| 15 | NZ_GG704949 | Acinetobacter calcoaceticus RUH2202 genomic scaffold supercont1.1: NZ_GG704949 | 9.06181183555984e-42 |
| 16 | NZ_GG704515 | Acinetobacter sp. RUH2624 genomic scaffold supercont1.21: NZ_GG704515 | 9.06181183555984e-42 |
| 17 | CP002177 | Acinetobacter calcoaceticus PHEA-2: CP002177 | 9.06181183555984e-42 |
| 18 | NZ_GG704515 | Acinetobacter sp. RUH2624 genomic scaffold supercont1.21: NZ_GG704515 | 3.84309843417235e-41 |
| 19 | NZ_GG704495 | Acinetobacter sp. RUH2624 genomic scaffold supercont1.1: NZ_GG704495 | 3.89818602393582e-41 |
| 20 | NC_005966 | Acinetobacter sp. ADP1: NC_005966 | 1.28694655921313e-40 |

(continued)

| No. | Scaffold.External.Accession | Scaffold.Name | best_pv |
|-----|------------------------------|----------------|---------|
| 21 | NZ_GG704515 | Acinetobacter sp. RUH2624 genomic scaffold supercont1.21: NZ_GG704515 | 1.49093940399756e-40 |
| 22 | NC_011595 | Acinetobacter baumannii AB307-0294: NC_011595 | 7.70079013246134e-40 |
| 23 | CP002522 | Acinetobacter baumannii TCDC-AB0715: CP002522 | 9.96749396638303e-40 |
| 24 | NC_011595 | Acinetobacter baumannii AB307-0294: NC_011595 | 1.66049576626689e-39 |
| 25 | NC_011586 | Acinetobacter baumannii AB0057: NC_011586 | 2.08472354496451e-39 |
| 26 | NC_014259 | Acinetobacter sp. DR1 chromosome: NC_014259 | 6.40954171412503e-39 |
| 27 | NZ_GG705093 | Acinetobacter lwoffii SH145 genomic scaffold supercont1.39: NZ_GG705093 | 1.2155660857651e-38 |
| 28 | NZ_ACYQ01000024 | Acinetobacter sp. 6013150 NZ_ACYQ01000024: NZ_ACYQ01000024 | 2.19910980623625e-38 |
| 29 | NZ_GG704532 | Acinetobacter sp. RUH2624 genomic scaffold supercont1.38: NZ_GG704532 | 5.31361687748575e-38 |
| 30 | NZ_ACYS01000159 | Acinetobacter sp. 6014059 NZ_ACYS01000159: NZ_ACYS01000159 | 6.25755791192393e-38 |
| 31 | NC_010611 | Acinetobacter baumannii ACICU: NC_010611 | 1.6998208344511e-37 |
| 32 | NZ_GG753601 | Acinetobacter sp. SH024 genomic scaffold supercontl.2: NZ_GG753601 | 2.25174704599278e-37 |
| 33 | CP002522 | Acinetobacter baumannii TCDC-AB0715: CP002522 | 2.43979456355917e-37 |
| 34 | NZ_GG704979 | Acinetobacter johnsonii SH046 genomic scaffold supercont1.16: NZ_GG704979 | 3.21441196797496e-37 |
| 35 | NC_011586 | Acinetobacter baumannii AB0057: NC_011586 | 4.65662453496211e-37 |
| 36 | NC_011586 | Acinetobacter baumannii AB0057: NC_011586 | 4.97462543315347e-37 |
| 37 | NC_010611 | Acinetobacter baumannii ACICU: NC_010611 | 5.86970772802613e-37 |
| 38 | CP002522 | Acinetobacter baumannii TCDC-AB0715: CP002522 | 5.86970772802613e-37 |
| 39 | NZ_ABXK01000021 | Acinetobacter baumannii AB900: NZ_ABXK01000021 | 7.97065298524188e-37 |
| 40 | CP001921 | Acinetobacter baumannii 1656-2: CP001921 | 8.02210311363916e-37 |
| 41 | NZ_ACYQ01000067 | Acinetobacter sp. 6013150 NZ_ACYQ01000067: NZ_ACYQ01000067 | 1.04988843673196e-36 |
| 42 | NZ_GG704579 | Acinetobacter baumannii ATCC 19606 genomic scaffold supercont1.8: NZ_GG704579 | 2.27416603584469e-36 |
| 43 | NC_010611 | Acinetobacter baumannii ACICU: NC_010611 | 2.41369225680075e-36 |
| 44 | NZ_ADMT01000124 | Acinetobacter haemolyticus ATCC 19194 contig00185: NZ_ADMT01000124 | 8.1885528903387e-36 |
| 45 | NC_011586 | Acinetobacter baumannii AB0057: NC_011586 | 8.6668830227109e-36 |

(continued)

| No. | Scaffold.External.Accession | Scaffold.Name | best_pv |
|---|---|---|---|
| 46 | CP002177 | Acinetobacter calcoaceticus PHEA-2: CP002177 | 1.06688951972352e-35 |
| 47 | NZ_GG704579 | Acinetobacter baumannii ATCC 19606 genomic scaffold supercont1.8: NZ_GG704579 | 1.73119071134862e-35 |
| 48 | NC_011595 | Acinetobacter baumannii AB307-0294: NC_011595 | 2.17923094612776e-35 |
| 49 | NZ_GG704499 | Acinetobacter sp. RUH2624 genomic scaffold supercont1.5: NZ_ GG704499 | 2.56827101997287e-35 |
| 50 | CP002522 | Acinetobacter baumannii TCDC-AB0715: CP002522 | 2.89444275071491e-35 |

Table 2b: Data for Acinetobacter, particularly Acinetobacter baumanii (continued)

| No. | sign_phenos | sign_phenos_class | best_pheno | best_pheno_class |
|---|---|---|---|---|
| 1 | CP;GM;IMP;LVX;MER;TO;T/S | aminoglycoside;fluoroquinolone;lactam;other | CP | fluoroquinolone |
| 2 | CP;GM;IMP;LVX;MER;TO;T/S | aminoglycoside;fluoroquinolone;lactam;other | CP | fluoroquinolone |
| 3 | CP;GM;IMP;LVX;MER;TO;T/S | aminoglycoside;fluoroquinolone;lactam;other | CP | fluoroquinolone |
| 4 | CP;GM;IMP;LVX;MER;TO;T/S | aminoglycoside;fluoroquinolone;lactam;other | CP | fluoroquinolone |
| 5 | CP;GM;IMP;LVX;MER;TO;T/S | aminoglycoside;fluoroquinolone;lactam;other | CP | fluoroquinolone |
| 6 | CP;GM;IMP;LVX;MER;TO;T/S | aminoglycoside;fluoroquinolone;lactam;other | CP | fluoroquinolone |
| 7 | CP;GM;IMP;LVX;MER;TO;T/S | aminoglycoside;fluoroquinolone;lactam;other | CP | fluoroquinolone |
| 8 | CP;GM;IMP;LVX;MER;TO;T/S | aminoglycoside;fluoroquinolone;lactam;other | CP | fluoroquinolone |
| 9 | CP;GM;IMP;LVX;MER;TO;T/S | aminoglycoside;fluoroquinolone;lactam;other | CP | fluoroquinolone |
| 10 | CP;GM;IMP;LVX;MER;TO;T/S | aminoglycoside;fluoroquinolone;lactam;other | CP | fluoroquinolone |
| 11 | CP;GM;IMP;LVX;MER;TO;T/S | aminoglycoside;fluoroquinolone;lactam;other | CP | fluoroquinolone |
| 12 | CP;GM;IMP;LVX;MER;TO;T/S | aminoglycoside;fluoroquinolone;lactam;other | CP | fluoroquinolone |
| 13 | CP;GM;IMP;LVX;MER;TO;T/S | aminoglycoside;fluoroquinolone;lactam;other | CP | fluoroquinolone |
| 14 | CP;GM;IMP;LVX;MER;TO;T/S | aminoglycoside;fluoroquinolone;lactam;other | CP | fluoroquinolone |
| 15 | CP;GM;IMP;LVX;MER;TO;T/S | aminoglycoside;fluoroquinolone;lactam;other | CP | fluoroquinolone |

(continued)

| No. | sign_phenos | sign_phenos_class | best_pheno | best_pheno_class |
|-----|-------------|-------------------|------------|------------------|
| 16 | CP;GM;IMP;LVX;MER; TO;T/S | aminoglycoside;fluoroquinolone;lactam; other | CP | fluoroquinolone |
| 17 | CP;GM;IMP;LVX;MER; TO;T/S | aminoglycoside;fluoroquinolone;lactam; other | CP | fluoroquinolone |
| 18 | CP;GM;IMP;LVX;MER; TO;T/S | aminoglycoside;fluoroquinolone;lactam; other | CP | fluoroquinolone |
| 19 | CP;GM;IMP;LVX;MER; TO;T/S | aminoglycoside;fluoroquinolone;lactam; other | CP | fluoroquinolone |
| 20 | CP;GM;IMP;LVX;MER; TO;T/S | aminoglycoside;fluoroquinolone;lactam; other | CP | fluoroquinolone |
| 21 | CP;GM;IMP;LVX;MER; TO;T/S | aminoglycoside;fluoroquinolone;lactam; other | CP | fluoroquinolone |
| 22 | CP;GM;IMP;LVX;MER; TO;T/S | aminoglycoside;fluoroquinolone;lactam; other | CP | fluoroquinolone |
| 23 | CP;GM;IMP;LVX;MER; TO;T/S | aminoglycoside;fluoroquinolone;lactam; other | CP | fluoroquinolone |
| 24 | CP;GM;IMP;LVX;MER; TO;T/S | aminoglycoside;fluoroquinolone;lactam; other | CP | fluoroquinolone |
| 25 | CP;GM;IMP;LVX;MER; TO;T/S | aminoglycoside;fluoroquinolone;lactam; other | LVX | fluoroquinolone |
| 26 | CP;GM;IMP;LVX;MER; TO;T/S | aminoglycoside;fluoroquinolone;lactam; other | CP | fluoroquinolone |
| 27 | CP;GM;IMP;LVX;MER; TO;T/S | aminoglycoside;fluoroquinolone;lactam; other | LVX | fluoroquinolone |
| 28 | CP;GM;IMP;LVX;MER; TO;T/S | aminoglycoside;fluoroquinolone;lactam; other | CP | fluoroquinolone |
| 29 | CP;GM;IMP;LVX;MER; TO;T/S | aminoglycoside;fluoroquinolone;lactam; other | LVX | fluoroquinolone |
| 30 | CP;GM;IMP;LVX;MER; TO;T/S | aminoglycoside;fluoroquinolone;lactam; other | CP | fluoroquinolone |
| 31 | CP;GM;IMP;LVX;MER; TO;T/S | aminoglycoside;fluoroquinolone;lactam; other | CP | fluoroquinolone |
| 32 | CP;GM;IMP;LVX;MER; TO;T/S | aminoglycoside;fluoroquinolone;lactam; other | CP | fluoroquinolone |
| 33 | CP;GM;IMP;LVX;MER; TO;T/S | aminoglycoside;fluoroquinolone;lactam; other | CP | fluoroquinolone |
| 34 | CP;GM;IMP;LVX;MER; TO;T/S | aminoglycoside;fluoroquinolone;lactam; other | CP | fluoroquinolone |
| 35 | CP;GM;IMP;LVX;MER; TO;T/S | aminoglycoside;fluoroquinolone;lactam; other | CP | fluoroquinolone |
| 36 | CP;GM;IMP;LVX;MER; TO;T/S | aminoglycoside;fluoroquinolone;lactam; other | CP | fluoroquinolone |
| 37 | CP;GM;IMP;LVX;MER; TO;T/S | aminoglycoside;fluoroquinolone;lactam; other | CP | fluoroquinolone |

(continued)

| No. | sign_phenos | sign_phenos_class | best_pheno | best_pheno_class |
|---|---|---|---|---|
| 38 | CP;GM;IMP;LVX;MER;TO;T/S | aminoglycoside;fluoroquinolone;lactam;other | CP | fluoroquinolone |
| 39 | CP;GM;IMP;LVX;MER;TO;T/S | aminoglycoside;fluoroquinolone;lactam;other | CP | fluoroquinolone |
| 40 | CP;GM;IMP;LVX;MER;TO;T/S | aminoglycoside;fluoroquinolone;lactam;other | LVX | fluoroquinolone |
| 41 | CP;GM;IMP;LVX;MER;TO;T/S | aminoglycoside;fluoroquinolone;lactam;other | CP | fluoroquinolone |
| 42 | CP;GM;IMP;LVX;MER;TO;T/S | aminoglycoside;fluoroquinolone;lactam;other | T/S | other |
| 43 | CP;GM;IMP;LVX;MER;TO;T/S | aminoglycoside;fluoroquinolone;lactam;other | LVX | fluoroquinolone |
| 44 | CP;GM;IMP;LVX;MER;TO;T/S | aminoglycoside;fluoroquinolone;lactam;other | LVX | fluoroquinolone |
| 45 | CP;GM;IMP;LVX;MER;TO;T/S | aminoglycoside;fluoroquinolone;lactam;other | CP | fluoroquinolone |
| 46 | CP;GM;IMP;LVX;MER;TO;T/S | aminoglycoside;fluoroquinolone;lactam;other | CP | fluoroquinolone |
| 47 | CP;GM;IMP;LVX;MER;TO;T/S | aminoglycoside;fluoroquinolone;lactam;other | T/S | other |
| 48 | CP;GM;IMP;LVX;MER;TO;T/S | aminoglycoside;fluoroquinolone;lactam;other | CP | fluoroquinolone |
| 49 | CP;GM;IMP;LVX;MER;TO;T/S | aminoglycoside;fluoroquinolone;lactam;other | CP | fluoroquinolone |
| 50 | CP;GM;IMP;LVX;MER;TO;T/S | aminoglycoside;fluoroquinolone;lactam;other | CP | fluoroquinolone |

Table 2c: Data for Acinetobacter, particularly Acinetobacter baumanii (continued)

| No. | num_aminoglycoside | num_fluoroquinolone | num_lactam | num_other |
|---|---|---|---|---|
| 1 | 2 | 2 | 2 | 1 |
| 2 | 2 | 2 | 2 | 1 |
| 3 | 2 | 2 | 2 | 1 |
| 4 | 2 | 2 | 2 | 1 |
| 5 | 2 | 2 | 2 | 1 |
| 6 | 2 | 2 | 2 | 1 |
| 7 | 2 | 2 | 2 | 1 |
| 8 | 2 | 2 | 2 | 1 |
| 9 | 2 | 2 | 2 | 1 |
| 10 | 2 | 2 | 2 | 1 |
| 11 | 2 | 2 | 2 | 1 |
| 12 | 2 | 2 | 2 | 1 |

(continued)

| No. | num_aminoglycoside | num_fluoroquinolone | num_lactam | num_other |
|-----|--------------------|---------------------|-----------|-----------|
| 13 | 2 | 2 | 2 | 1 |
| 14 | 2 | 2 | 2 | 1 |
| 15 | 2 | 2 | 2 | 1 |
| 16 | 2 | 2 | 2 | 1 |
| 17 | 2 | 2 | 2 | 1 |
| 18 | 2 | 2 | 2 | 1 |
| 19 | 2 | 2 | 2 | 1 |
| 20 | 2 | 2 | 2 | 1 |
| 21 | 2 | 2 | 2 | 1 |
| 22 | 2 | 2 | 2 | 1 |
| 23 | 2 | 2 | 2 | 1 |
| 24 | 2 | 2 | 2 | 1 |
| 25 | 2 | 2 | 2 | 1 |
| 26 | 2 | 2 | 2 | 1 |
| 27 | 2 | 2 | 2 | 1 |
| 28 | 2 | 2 | 2 | 1 |
| 29 | 2 | 2 | 2 | 1 |
| 30 | 2 | 2 | 2 | 1 |
| 31 | 2 | 2 | 2 | 1 |
| 32 | 2 | 2 | 2 | 1 |
| 33 | 2 | 2 | 2 | 1 |
| 34 | 2 | 2 | 2 | 1 |
| 35 | 2 | 2 | 2 | 1 |
| 36 | 2 | 2 | 2 | 1 |
| 37 | 2 | 2 | 2 | 1 |
| 38 | 2 | 2 | 2 | 1 |
| 39 | 2 | 2 | 2 | 1 |
| 40 | 2 | 2 | 2 | 1 |
| 41 | 2 | 2 | 2 | 1 |
| 42 | 2 | 2 | 2 | 1 |
| 43 | 2 | 2 | 2 | 1 |
| 44 | 2 | 2 | 2 | 1 |
| 45 | 2 | 2 | 2 | 1 |
| 46 | 2 | 2 | 2 | 1 |
| 47 | 2 | 2 | 2 | 1 |
| 48 | 2 | 2 | 2 | 1 |
| 49 | 2 | 2 | 2 | 1 |
| 50 | 2 | 2 | 2 | 1 |

Table 2d: Data for Acinetobacter, particularly Acinetobacter baumanii (continued)

| No. | CP_pv_adj | GM_pv_adj | IMP_pv_adj | LVX_pv_adj | MER_pv_adj | TO_pv_adj | T/S_pv_adj |
|-----|-----------|-----------|------------|------------|------------|-----------|------------|
| 1 | 7.17E-62 | 7.62E-37 | 2.99E-16 | 2.51E-58 | 1.71E-29 | 3.85E-19 | 7.12E-37 |
| 2 | 1.39E-61 | 2.93E-36 | 2.52E-16 | 1.14E-57 | 1.26E-28 | 1.01E-18 | 2.48E-36 |
| 3 | 1.44E-56 | 1.41E-30 | 7.92E-24 | 2.33E-56 | 4.63E-35 | 1.23E-15 | 1.16E-31 |
| 4 | 5.42E-51 | 1.20E-26 | 3.83E-16 | 1.97E-49 | 3.77E-19 | 6.39E-14 | 5.70E-30 |
| 5 | 7.00E-47 | 7.36E-25 | 7.07E-15 | 3.32E-45 | 1.76E-24 | 2.16E-14 | 1.06E-32 |
| 6 | 2.06E-46 | 1.72E-23 | 1.92E-08 | 3.46E-46 | 2.40E-18 | 1.64E-13 | 3.34E-31 |
| 7 | 8.99E-46 | 1.85E-24 | 2.19E-15 | 2.93E-44 | 1.16E-24 | 3.33E-14 | 3.29E-31 |
| 8 | 2.77E-44 | 1.51E-23 | 2.19E-15 | 5.38E-43 | 1.16E-24 | 3.33E-14 | 3.45E-30 |
| 9 | 6.86E-43 | 1.15E-22 | 4.78E-15 | 1.09E-41 | 3.50E-24 | 5.85E-13 | 2.90E-29 |
| 10 | 5.21E-42 | 7.13E-19 | 3.88E-19 | 3.01E-41 | 3.46E-19 | 1.35E-06 | 1.20E-22 |
| 11 | 9.06E-42 | 1.74E-21 | 5.28E-11 | 6.20E-39 | 2.05E-18 | 9.48E-14 | 2.09E-27 |
| 12 | 9.06E-42 | 1.40E-20 | 1.57E-11 | 1.29E-40 | 2.50E-19 | 2.12E-13 | 1.47E-25 |
| 13 | 9.06E-42 | 1.40E-20 | 1.57E-11 | 1.29E-40 | 2.50E-19 | 2.12E-13 | 1.47E-25 |
| 14 | 9.06E-42 | 1.26E-22 | 4.71E-10 | 1.24E-38 | 1.59E-17 | 1.36E-14 | 1.16E-28 |
| 15 | 9.06E-42 | 1.40E-20 | 1.57E-11 | 1.29E-40 | 2.50E-19 | 2.12E-13 | 1.47E-25 |
| 16 | 9.06E-42 | 1.40E-20 | 1.57E-11 | 1.29E-40 | 2.50E-19 | 2.12E-13 | 1.47E-25 |
| 17 | 9.06E-42 | 1.40E-20 | 1.57E-11 | 1.29E-40 | 2.50E-19 | 2.12E-13 | 1.47E-25 |
| 18 | 3.84E-41 | 4.26E-21 | 5.48E-11 | 2.25E-38 | 2.28E-18 | 1.85E-13 | 1.34E-28 |
| 19 | 3.90E-41 | 3.00E-22 | 8.17E-10 | 4.60E-38 | 3.07E-17 | 2.60E-14 | 3.05E-28 |
| 20 | 1.29E-40 | 2.54E-23 | 8.32E-09 | 2.28E-37 | 2.98E-16 | 7.17E-15 | 4.45E-29 |
| 21 | 1.49E-40 | 1.06E-20 | 9.78E-11 | 8.02E-38 | 4.26E-18 | 3.58E-13 | 1.41E-26 |
| 22 | 7.70E-40 | 2.19E-21 | 2.55E-13 | 8.73E-40 | 2.71E-22 | 1.30E-12 | 9.73E-29 |
| 23 | 9.97E-40 | 1.94E-20 | 5.69E-10 | 6.32E-37 | 5.09E-15 | 3.42E-13 | 4.53E-25 |
| 24 | 1.66E-39 | 2.25E-21 | 1.11E-11 | 4.74E-37 | 9.11E-19 | 4.30E-12 | 6.58E-28 |
| 25 | 6.97E-38 | 2.51E-23 | 4.39E-17 | 2.08E-39 | 2.11E-31 | 2.81E-20 | 2.62E-29 |
| 26 | 6.41E-39 | 8.82E-20 | 8.32E-09 | 2.28E-37 | 8.45E-15 | 5.48E-12 | 1.80E-23 |
| 27 | 1.21E-36 | 6.79E-22 | 2.00E-18 | 1.22E-38 | 1.37E-30 | 1.91E-20 | 1.86E-29 |
| 28 | 2.20E-38 | 6.34E-16 | 0.00015078 | 3.00E-37 | 3.02E-15 | 2.23E-05 | 5.58E-26 |
| 29 | 2.16E-37 | 5.62E-21 | 1.99E-11 | 5.31E-38 | 4.07E-20 | 1.62E-14 | 1.79E-33 |
| 30 | 6.26E-38 | 1.31E-11 | 3.04E-05 | 7.55E-37 | 2.46E-19 | 5.99E-05 | 3.36E-19 |
| 31 | 1.70E-37 | 9.89E-20 | 4.00E-15 | 6.17E-33 | 1.59E-19 | 1.06E-07 | 5.60E-19 |
| 32 | 2.25E-37 | 3.62E-20 | 5.26E-16 | 3.31E-34 | 9.82E-23 | 4.38E-09 | 5.82E-22 |
| 33 | 2.44E-37 | 6.97E-16 | 4.33E-14 | 7.77E-37 | 9.30E-19 | 4.18E-07 | 1.39E-20 |
| 34 | 3.21E-37 | 1.12E-20 | 1.05E-11 | 3.70E-36 | 1.02E-20 | 3.75E-13 | 1.16E-24 |
| 35 | 4.66E-37 | 5.92E-20 | 3.00E-11 | 4.04E-32 | 8.17E-17 | 5.55E-10 | 2.22E-21 |
| 36 | 4.97E-37 | 9.12E-20 | 3.90E-11 | 7.27E-35 | 8.20E-18 | 2.95E-11 | 2.78E-26 |
| 37 | 5.87E-37 | 1.44E-15 | 3.36E-14 | 2.00E-36 | 3.55E-19 | 3.60E-07 | 3.09E-20 |

(continued)

| No. | CP_pv_adj | GM_pv_adj | IMP_pv_adj | LVX_pv_adj | MER_pv_adj | TO_pv_adj | T/S_pv_adj |
|---|---|---|---|---|---|---|---|
| 38 | 5.87E-37 | 1.44E-15 | 2.29E-13 | 2.00E-36 | 2.38E-18 | 1.01E-06 | 4.30E-21 |
| 39 | 7.97E-37 | 1.41E-20 | 4.11E-10 | 1.69E-34 | 6.22E-17 | 1.57E-12 | 9.61E-26 |
| 40 | 8.02E-36 | 1.02E-16 | 8.36E-13 | 8.02E-37 | 6.77E-18 | 2.61E-10 | 1.91E-19 |
| 41 | 1.05E-36 | 5.82E-22 | 6.09E-07 | 1.09E-33 | 3.93E-12 | 9.82E-13 | 1.13E-26 |
| 42 | 2.17E-18 | 1.05E-13 | 2.19E-05 | 7.67E-17 | 1.44E-05 | 1.58E-19 | 2.27E-36 |
| 43 | 2.04E-35 | 2.18E-16 | 4.05E-13 | 2.41E-36 | 4.89E-18 | 7.30E-10 | 2.59E-18 |
| 44 | 2.30E-34 | 4.36E-15 | 1.44E-11 | 8.19E-36 | 1.06E-16 | 9.26E-09 | 1.67E-18 |
| 45 | 8.67E-36 | 6.11E-17 | 3.23E-06 | 1.48E-32 | 1.25E-11 | 1.06E-09 | 8.79E-25 |
| 46 | 1.07E-35 | 6.07E-21 | 9.46E-09 | 3.15E-33 | 4.04E-15 | 6.09E-14 | 1.52E-24 |
| 47 | 2.15E-17 | 3.02E-13 | 2.37E-05 | 3.26E-16 | 2.56E-05 | 2.12E-19 | 1.73E-35 |
| 48 | 2.18E-35 | 2.69E-18 | 0.00016455 | 2.17E-32 | 2.13E-10 | 9.38E-10 | 2.81E-21 |
| 49 | 2.57E-35 | 5.01E-23 | 1.62E-06 | 3.27E-32 | 1.19E-11 | 4.78E-13 | 2.98E-26 |
| 50 | 2.89E-35 | 4.58E-19 | 5.87E-06 | 4.77E-32 | 5.92E-13 | 2.05E-11 | 8.30E-23 |

Table 2e: Data for Acinetobacter, particularly Acinetobacter baumanii (continued)

| No. | Locus.Tag | Gene. Symbol | GenBank.Accession | Chromosome |
|---|---|---|---|---|
| 1 | ABK1_1135 | | ADX02769 | |
| 2 | A60131_010100011170 | | ZP_06783174 | |
| 3 | A1S_0690 | | YP_001083736 | |
| 4 | ABBFA_001710 | | YP_002325613 | |
| 5 | ABBFA_001640 | | YP_002325543 | |
| 6 | AB57_1876 | | YP_002319238 | |
| 7 | AB57_2151 | | YP_002319507 | |
| 8 | ABBFA_001638 | | YP_002325541 | |
| 9 | AB57_2147 | | YP_002319503 | |
| 10 | AbauAB_010100009672 | | ZP_04661877 | |
| 11 | HMPREF0014_00181 | | ZP_05823167 | |
| 12 | HMPREF0014_02450 | | ZP_05825436 | |
| 13 | HMPREF0014_02452 | | ZP_05825438 | |
| 14 | AOLE_17735 | | YP_003733805 | |
| 15 | HMPREF0012_01352 | | ZP_06057184 | |
| 16 | HMPREF0014_02453 | | ZP_05825439 | |
| 17 | BDGL_001498 | ocd2 | ADY82084 | |
| 18 | HMPREF0014_02489 | | ZP_05825475 | |
| 19 | HMPREF0014_00004 | | ZP_05822990 | |
| 20 | ACIAD0370 | rbfA | YP_045140 | |
| 21 | HMPREF0014_02492 | | ZP_05825478 | |

(continued)

| 22 | ABBFA_001637 | | YP_002325540 | |
|----|--------------|---|--------------|---|
| 23 | ABTW07_1894 | | ADX92323 | |
| 24 | ABBFA_ 000343 | | YP_002324276 | |
| 25 | AB57_2771 | | YP_002320110 | |
| 26 | AOLE_17690 | | YP_003733796 | |
| 27 | HMPREF0017_03097 | | ZP_06071176 | |
| 28 | A6013_010100003618 | | ZP_06795427 | |
| 29 | HMPREF0014_03338 | | ZP_05826324 | |
| 30 | A6014_010100015962 | | ZP_06788050 | |
| 31 | ACICU_00639 | filE | YP_001845298 | |
| 32 | HMPREF0013_02137 | | ZP_06692288 | |
| 33 | ABTW07_2865 | | ADX93289 | |
| 34 | HMPREF0016_03291 | | ZP_06064821 | |
| 35 | AB57_2131 | | YP_002319487 | |
| 36 | AB57_3624 | pilV | YP_002320921 | |
| 37 | ACICU_02614 | | YP_001847273 | |
| 38 | ABTW07_2863 | | ADX93287 | |
| 39 | AbauAB_010100013685 | | ZP_04662667 | |
| 40 | ABK1_2278 | | ADX03912 | |
| 41 | A6013_010100009420 | | ZP_06796568 | |
| 42 | HMPREF0010_03320 | | ZP_05829937 | |
| 43 | ACICU_01819 | | YP_001846478 | |
| 44 | HMP0015_1294 | | ZP_06727085 | |
| 45 | AB57_1430 | | YP_002318806 | |
| 46 | BDGL_000595 | | ADY81181 | |
| 47 | HMPREF0010_03319 | | ZP_05829936 | |
| 48 | ABBFA_001847 | | YP_002325750 | |
| 49 | HMPREF0014_00860 | | ZP_05823846 | |
| 50 | ABTW07_1891 | | ADX92320 | |

Table 2f: Data for Acinetobacter, particularly Acinetobacter baumanii (continued)

| No. | Start.Coord | End.Coord | Strand | Scaffold.ID |
|-----|-------------|-----------|--------|-------------|
| 1 | 1235814 | 1236425 | + | 650377914 |
| 2 | 64 | 768 | + | 647008664 |
| 3 | 821275 | 822057 | + | 640069344 |
| 4 | 1834753 | 1835667 | - | 643348583 |
| 5 | 1759838 | 1761280 | + | 643348583 |
| 6 | 1966831 | 1969149 | + | 643348581 |

(continued)

| No. | Start.Coord | End.Coord | Strand | Scaffold.ID |
|-----|-------------|-----------|--------|-------------|
| 7 | 2232790 | 2233716 | - | 643348581 |
| 8 | 1757930 | 1758571 | - | 643348583 |
| 9 | 2226774 | 2228099 | + | 643348581 |
| 10 | 63870 | 65015 | - | 645061599 |
| 11 | 195165 | 195419 | + | 647535325 |
| 12 | 27528 | 28301 | - | 647535345 |
| 13 | 31818 | 33023 | - | 647535345 |
| 14 | 3783514 | 3783789 | + | 648028017 |
| 15 | 1494626 | 1495630 | + | 646207323 |
| 16 | 33027 | 34796 | - | 647535345 |
| 17 | 1603798 | 1604811 | + | 650377930 |
| 18 | 74534 | 74893 | + | 647535345 |
| 19 | 1819 | 2325 | - | 647535325 |
| 20 | 363575 | 363976 | + | 637000277 |
| 21 | 76454 | 76681 | + | 647535345 |
| 22 | 1757594 | 1757890 | - | 643348583 |
| 23 | 2004366 | 2004983 | + | 650378014 |
| 24 | 370977 | 371978 | + | 643348583 |
| 25 | 2838051 | 2838620 | - | 643348581 |
| 26 | 3773081 | 3773467 | + | 648028017 |
| 27 | 716 | 1162 | + | 646207467 |
| 28 | 6958 | 7416 | - | 647008417 |
| 29 | 12541 | 13101 | - | 647535362 |
| 30 | 7588 | 7713 | + | 647009002 |
| 31 | 705957 | 707216 | + | 642555129 |
| 32 | 856694 | 857281 | - | 647536755 |
| 33 | 3040319 | 3041707 | - | 650378014 |
| 34 | 4553 | 4768 | - | 646207353 |
| 35 | 2213458 | 2214801 | - | 643348581 |
| 36 | 3730668 | 3731222 | - | 643348581 |
| 37 | 2775538 | 2776941 | + | 642555129 |
| 38 | 3038397 | 3039893 | - | 650378014 |
| 39 | 70983 | 71513 | - | 645061607 |
| 40 | 2403829 | 2405136 | + | 650377914 |
| 41 | 2603 | 3454 | + | 647008460 |
| 42 | 159696 | 159998 | + | 647535409 |
| 43 | 1941428 | 1942675 | + | 642555129 |
| 44 | 1088 | 2629 | - | 647018944 |

(continued)

| No. | Start.Coord | End.Coord | Strand | Scaffold.ID |
|---|---|---|---|---|
| 45 | 1505450 | 1506382 | + | 643348581 |
| 46 | 622112 | 622732 | + | 650377930 |
| 47 | 158794 | 159609 | + | 647535409 |
| 48 | 1980078 | 1981988 | - | 643348583 |
| 49 | 126664 | 127845 | + | 647535329 |
| 50 | 1999835 | 2001676 | + | 650378014 |

Examples 2 and 3: Enterobacter, particularly Enterobacter aerogenes and Enterobacter cloacae

The procedure was carried out as in Example 1, except that the following microorganisms were used:+

Bacterial Strains

The inventors selected 695 Enterobacter strains, particularly 298 for Enterobacter aerogenes and 397 for Enterobacter cloacae, from the microbiology strain collection at Siemens Healthcare Diagnostics (West Sacramento, CA) for susceptibility testing and whole genome sequencing.
From MetaRef, 5220 centroids of E. aerogenes were used as reference sequences for E. aerogenes, and 4734 centroids of E. cloacae were used as reference sequences for E. cloacae.
The results for Enterobacter aerogenes are shown in Tables 3 (corresponding to Table 1) and 4 (corresponding to Table 2), and the results for Enterobacter cloacae are shown in Tables 5 (corresponding to Table 1) and 6 (corresponding to Table 2).

Table 3a: Data for Enterobacter, particularly Enterobacter aerogenes

| No. | Scaffold.External. Accession | Scaffold.Name | best_pv |
|---|---|---|---|
| 1 | NC_015663 | Enterobacter aerogenes KCTC 2190 chromosome: NC_015663 | 1.01047730430126e-10 |
| 2 | NC_015663 | Enterobacter aerogenes KCTC 2190 chromosome: NC_015663 | 1.01047730430126e-10 |
| 3 | NC_015663 | Enterobacter aerogenes KCTC 2190 chromosome: NC_015663 | 2.21893843673589e-10 |
| 4 | NC_015663 | Enterobacter aerogenes KCTC 2190 chromosome: NC_015663 | 7.36056945497466e-08 |
| 5 | NC_015663 | Enterobacter aerogenes KCTC 2190 chromosome: NC_015663 | 9.81164607784395e-08 |
| 6 | NC_015663 | Enterobacter aerogenes KCTC 2190 chromosome: NC_015663 | 9.81164607784395e-08 |
| 7 | NC_015663 | Enterobacter aerogenes KCTC 2190 chromosome: NC_015663 | 3.92206261774063e-06 |
| 8 | NC_015663 | Enterobacter aerogenes KCTC 2190 chromosome: NC_015663 | 9.95623777570528e-06 |
| 9 | NC_015663 | Enterobacter aerogenes KCTC 2190 chromosome: NC_015663 | 0.000635187711382771 |
| 10 | NC_009778 | Enterobacter sakazakii ATCC BAA-894: NC_009778 | 0.000701852165838025 |
| 11 | NC_015663 | Enterobacter aerogenes KCTC 2190 chromosome: NC_015663 | 0.000717841336438945 |

(continued)

| No. | Scaffold.External. Accession | Scaffold.Name | best_pv |
|---|---|---|---|
| 12 | NZ_GG745509 | Klebsiella sp. 1_1_55 genomic scaffold supercontl.2: NZ_GG745509 | 0.000881981942215321 |
| 13 | NC_013850 | Klebsiella variicola At-22 chromosome: NC_013850 | 0.000881981942215321 |
| 14 | NC_015663 | Enterobacter aerogenes KCTC 2190 chromosome: NC_015663 | 0.000960532001115215 |
| 15 | NC_015663 | Enterobacter aerogenes KCTC 2190 chromosome: NC_015663 | 0.00108645793188005 |
| 16 | NZ_ADTJ01000031 | Escherichia coli MS 198-1 E_coli198-1-1.0_Cont45.1: NZ_ADTJ01000031 | 0.00108724371491948 |
| 17 | NC_015663 | Enterobacter aerogenes KCTC 2190 chromosome: NC_015663 | 0.0016524197402272 |
| 18 | NC_015663 | Enterobacter aerogenes KCTC 2190 chromosome: NC_015663 | 0.00184130315909336 |
| 19 | NC_015663 | Enterobacter aerogenes KCTC 2190 chromosome: NC_015663 | 0.00187260357604506 |
| 20 | NC_015663 | Enterobacter aerogenes KCTC 2190 chromosome: NC_015663 | 0.00191687062572367 |
| 21 | NZ_ACZD01000042 | Klebsiella pneumoniae subsp. rhinoscleromatis ATCC 13884 contig00042: NZ_ACZD01000042 | 0.00208337132265191 |
| 22 | NC_015663 | Enterobacter aerogenes KCTC 2190 chromosome: NC_015663 | 0.00208337132265191 |
| 23 | NZ_ADTJ01000031 | Escherichia coli MS 198-1 E_coli198-1-1.0_Cont45.1: NZ_ADTJ01000031 | 0.00241352185546859 |
| 24 | NZ_ADTJ01000031 | Escherichia coli MS 198-1 E_coli198-1-1.0_Cont45.1: NZ_ADTJ01000031 | 0.00245969973695216 |
| 25 | AM946981 | Escherichia coli BL21 (DE3): AM946981 | 0.00327316953478549 |
| 26 | NC_015663 | Enterobacter aerogenes KCTC 2190 chromosome: NC_015663 | 0.00327864620009511 |
| 27 | NC_015663 | Enterobacter aerogenes KCTC 2190 chromosome: NC_015663 | 0.00334082507673483 |
| 28 | NC_015663 | Enterobacter aerogenes KCTC 2190 chromosome: NC_015663 | 0.00342044194519721 |
| 29 | NC_015663 | Enterobacter aerogenes KCTC 2190 chromosome: NC_015663 | 0.00349516820070151 |
| 30 | NC_015663 | Enterobacter aerogenes KCTC 2190 chromosome: NC_015663 | 0.00391582859992096 |
| 31 | NC_015663 | Enterobacter aerogenes KCTC 2190 chromosome: NC_015663 | 0.0039468230846432 |
| 32 | NC_015663 | Enterobacter aerogenes KCTC 2190 chromosome: NC_015663 | 0.0042274756954506 |
| 33 | NC_015663 | Enterobacter aerogenes KCTC 2190 chromosome: NC_015663 | 0.00458480687490629 |

(continued)

| No. | Scaffold.External. Accession | Scaffold.Name | best_pv |
|---|---|---|---|
| 34 | NC_015663 | Enterobacter aerogenes KCTC 2190 chromosome: NC_ 015663 | 0.00458480687490629 |
| 35 | NC_015663 | Enterobacter aerogenes KCTC 2190 chromosome: NC_ 015663 | 0.00458480687490629 |
| 36 | NC_012731 | Klebsiella pneumoniae NTUH-K2044: NC_012731 | 0.00458480687490629 |
| 37 | NC_015663 | Enterobacter aerogenes KCTC 2190 chromosome: NC_ 015663 | 0.00482300882771186 |
| 38 | NC_015663 | Enterobacter aerogenes KCTC 2190 chromosome: NC_ 015663 | 0.00495283743056749 |
| 39 | NC_015663 | Enterobacter aerogenes KCTC 2190 chromosome: NC_ 015663 | 0.00495283743056749 |
| 40 | NC_015663 | Enterobacter aerogenes KCTC 2190 chromosome: NC_ 015663 | 0.005030809826688659 |
| 41 | NC_015663 | Enterobacter aerogenes KCTC 2190 chromosome: NC_ 015663 | 0.00529748534762393 |
| 42 | NC_015663 | Enterobacter aerogenes KCTC 2190 chromosome: NC_ 015663 | 0.00585931541937669 |
| 43 | NC_015663 | Enterobacter aerogenes KCTC 2190 chromosome: NC_ 015663 | 0.00585931541937669 |
| 44 | NC_015663 | Enterobacter aerogenes KCTC 2190 chromosome: NC_ 015663 | 0.00585931541937669 |
| 45 | NC_015663 | Enterobacter aerogenes KCTC 2190 chromosome: NC_ 015663 | 0.00585931541937669 |
| 46 | NC_015663 | Enterobacter aerogenes KCTC 2190 chromosome: NC_ 015663 | 0.00648651344338336 |
| 47 | NC_015663 | Enterobacter aerogenes KCTC 2190 chromosome: NC_ 015663 | 0.00648651344338336 |
| 48 | NC_015663 | Enterobacter aerogenes KCTC 2190 chromosome: NC_ 015663 | 0.00648651344338336 |
| 49 | NZ_GG745510 | Klebsiella sp. 1_1_55 genomic scaffold supercont1.3: NZ_ GG745510 | 0.00659680763861341 |
| 50 | NC_015663 | Enterobacter aerogenes KCTC 2190 chromosome: NC_ 015663 | 0.00667294667231188 |

Table 3b: Data for Enterobacter, particularly Enterobacter aerogenes (continued)

| No. | sign_phenos | sign_phenos_class | best_ pheno | best_pheno_class |
|---|---|---|---|---|
| 1 | AZT;CAX;CAZ;CFT;CP; CRM;LVX;P/T;TO;T/S | aminoglycoside;fluoroquinolone; lactam; other | CP | fluoroquinolone |
| 2 | AZT;CAX;CAZ;CFT;CP; CRM;LVX;P/T;TO;T/S | aminoglycoside;fluoroquinolone; lactam; other | CP | fluoroquinolone |
| 3 | AZT;CAX;CAZ;CFT;CP; CRM;LVX;P/T;TO;T/S | aminoglycoside;fluoroquinolone; lactam; other | CP | fluoroquinolone |

(continued)

| No. | sign_phenos | sign_phenos_class | best_pheno | best_pheno_class |
|---|---|---|---|---|
| 4 | CP;LVX;TO;T/S | aminoglycoside;fluoroquinolone; other | CP | fluoroquinolone |
| 5 | AZT;CAX;CAZ;CFT;CP; CRM;LVX;P/T;T/S | fluoroquinolone;lactam;other | CP | fluoroquinolone |
| 6 | AZT;CP;LVX;P/T;T/S | fluoroquinolone;lactam;other | CP | fluoroquinolone |
| 7 | CP;LVX;T/S | fluoroquinolone;other | CP | fluoroquinolone |
| 8 | CP;LVX;T/S | fluoroquinolone;other | CP | fluoroquinolone |
| 9 | AZT;CAX;CAZ;CFT;P/T | lactam | CFT | lactam |
| 10 | AZT;CAX;CAZ;CFT;CRM; P/T | lactam | AZT | lactam |
| 11 | CAX;CFT;CRM | lactam | CRM | lactam |
| 12 | AZT;CAX;CAZ;CFT;P/T | lactam | CAX | lactam |
| 13 | AZT;CAX;CAZ;CFT;P/T | lactam | CAX | lactam |
| 14 | TO | aminoglycoside | TO | aminoglycoside |
| 15 | AZT;CAX;CAZ;CFT;CRM; P/T | lactam | CAX | lactam |
| 16 | CP;LVX | fluoroquinolone | CP | fluoroquinolone |
| 17 | AZT;CAX;CAZ;CFT;CP;P/T | fluoroquinolone;lactam | P/T | lactam |
| 18 | AZT;CAX;CAZ;CFT;CP;P/T | fluoroquinolone;lactam | CAX | lactam |
| 19 | AZT;CAX;CAZ;CFT;CRM | lactam | CAX | lactam |
| 20 | CAX;CAZ;CFT | lactam | CAX | lactam |
| 21 | CAX;CAZ;CFT;CRM;P/T | lactam | CAX | lactam |
| 22 | CAX;CAZ;CFT;CRM;P/T | lactam | CAX | lactam |
| 23 | TO | aminoglycoside | TO | aminoglycoside |
| 24 | TO | aminoglycoside | TO | aminoglycoside |
| 25 | AZT;CAX;CAZ;CFT | lactam | CAX | lactam |
| 26 | CP | fluoroquinolone | CP | fluoroquinolone |
| 27 | AZT;CAX;CAZ;CFT;CRM; P/T | lactam | CAX | lactam |
| 28 | CAX;CAZ;CFT;CRM;P/T | lactam | CAX | lactam |
| 29 | AZT;CAX;CAZ;CFT | lactam | CAX | lactam |
| 30 | AZT;CAX;CAZ;CFT;P/T | lactam | AZT | lactam |
| 31 | AZT;CAX;CFT | lactam | CAX | lactam |
| 32 | AZT;CAX | lactam | CAX | lactam |
| 33 | CAX;CFT | lactam | CAX | lactam |
| 34 | CAX;CFT | lactam | CAX | lactam |
| 35 | CAX;CP | fluoroquinolone;lactam | CP | fluoroquinolone |
| 36 | CAX;CFT | lactam | CAX | lactam |

(continued)

| No. | sign_phenos | sign_phenos_class | best_pheno | best_pheno_class |
|-----|-------------|-------------------|------------|------------------|
| 37 | CAX;CFT;CRM;P/T | lactam | CRM | lactam |
| 38 | AZT;CAX;CFT | lactam | CFT | lactam |
| 39 | AZT;CAX;CFT | lactam | CFT | lactam |
| 40 | CAX | lactam | CAX | lactam |
| 41 | CAX | lactam | CAX | lactam |
| 42 | AZT;CAX | lactam | AZT | lactam |
| 43 | CAX | lactam | CAX | lactam |
| 44 | AZT;CAX | lactam | AZT | lactam |
| 45 | AZT;CAX;CFT | lactam | CAX | lactam |
| 46 | AZT;CAX | lactam | CAX | lactam |
| 47 | AZT;CAX | lactam | CAX | lactam |
| 48 | AZT;CAX | lactam | CAX | lactam |
| 49 | CAX | lactam | CAX | lactam |
| 50 | CAX | lactam | CAX | lactam |

Table 3c: Data for Enterobacter, particularly Enterobacter aerogenes (continued)

| No. | num_aminoglycoside | num_fluoroquinolone | num_lactam | num_other |
|-----|--------------------|--------------------|-----------|-----------|
| 1 | 1 | 2 | 6 | 1 |
| 2 | 1 | 2 | 6 | 1 |
| 3 | 1 | 2 | 6 | 1 |
| 4 | 1 | 2 | 0 | 1 |
| 5 | 0 | 2 | 6 | 1 |
| 6 | 0 | 2 | 2 | 1 |
| 7 | 0 | 2 | 0 | 1 |
| 8 | 0 | 2 | 0 | 1 |
| 9 | 0 | 0 | 5 | 0 |
| 10 | 0 | 0 | 6 | 0 |
| 11 | 0 | 0 | 3 | 0 |
| 12 | 0 | 0 | 5 | 0 |
| 13 | 0 | 0 | 5 | 0 |
| 14 | 1 | 0 | 0 | 0 |
| 15 | 0 | 0 | 6 | 0 |
| 16 | 0 | 2 | 0 | 0 |
| 17 | 0 | 1 | 5 | 0 |
| 18 | 0 | 1 | 5 | 0 |
| 19 | 0 | 0 | 5 | 0 |
| 20 | 0 | 0 | 3 | 0 |

(continued)

| No. | num_aminoglycoside | num_fluoroquinolone | num_lactam | num_other |
|---|---|---|---|---|
| 21 | 0 | 0 | 5 | 0 |
| 22 | 0 | 0 | 5 | 0 |
| 23 | 1 | 0 | 0 | 0 |
| 24 | 1 | 0 | 0 | 0 |
| 25 | 0 | 0 | 4 | 0 |
| 26 | 0 | 1 | 0 | 0 |
| 27 | 0 | 0 | 6 | 0 |
| 28 | 0 | 0 | 5 | 0 |
| 29 | 0 | 0 | 4 | 0 |
| 30 | 0 | 0 | 5 | 0 |
| 31 | 0 | 0 | 3 | 0 |
| 32 | 0 | 0 | 2 | 0 |
| 33 | 0 | 0 | 2 | 0 |
| 34 | 0 | 0 | 2 | 0 |
| 35 | 0 | 1 | 1 | 0 |
| 36 | 0 | 0 | 2 | 0 |
| 37 | 0 | 0 | 4 | 0 |
| 38 | 0 | 0 | 3 | 0 |
| 39 | 0 | 0 | 3 | 0 |
| 40 | 0 | 0 | 1 | 0 |
| 41 | 0 | 0 | 1 | 0 |
| 42 | 0 | 0 | 2 | 0 |
| 43 | 0 | 0 | 1 | 0 |
| 44 | 0 | 0 | 2 | 0 |
| 45 | 0 | 0 | 3 | 0 |
| 46 | 0 | 0 | 2 | 0 |
| 47 | 0 | 0 | 2 | 0 |
| 48 | 0 | 0 | 2 | 0 |
| 49 | 0 | 0 | 1 | 0 |
| 50 | 0 | 0 | 1 | 0 |

Table 3d: Data for Enterobacter, particularly Enterobacter aerogenes (continued)

| No. | AZT_pv_adj | CAX_pv_adj | CAZ_pv_adj | CFT_pv_adj | CP_pv_adj |
|---|---|---|---|---|---|
| 1 | 8.22E-04 | 2.45E-04 | 3.83E-04 | 4.31E-04 | 1.01E-10 |
| 2 | 1.32E-03 | 4.48E-04 | 7.06E-04 | 7.76E-04 | 1.01E-10 |
| 3 | 3.27E-03 | 1.99E-03 | 4.43E-03 | 3.01E-03 | 2.22E-10 |
| 4 | 2.06E-02 | 1.86E-02 | 2.29E-02 | 2.41E-02 | 7.36E-08 |

(continued)

| No. | AZT_pv_adj | CAX_pv_adj | CAZ_pv_adj | CFT_pv_adj | CP_pv_adj |
|---|---|---|---|---|---|
| 5 | 4.29E-03 | 2.32E-04 | 6.91E-04 | 7.06E-04 | 9.81E-08 |
| 6 | 8.86E-03 | 1.82E-02 | 1.77E-02 | 1.84E-02 | 9.81E-08 |
| 7 | 5.47E-01 | 1.61E-02 | 4.75E-02 | 4.90E-02 | 3.92E-06 |
| 8 | 6.96E-01 | 3.29E-02 | 9.63E-02 | 9.80E-02 | 9.96E-06 |
| 9 | 4.82E-03 | 9.01E-04 | 1.84E-03 | 6.35E-04 | 3.94E-02 |
| 10 | 7.02E-04 | 3.95E-03 | 6.47E-03 | 6.18E-03 | 2.19E-02 |
| 11 | 1.74E-02 | 4.82E-03 | 1.41E-02 | 4.93E-03 | 4.78E-01 |
| 12 | 5.48E-03 | 8.82E-04 | 6.43E-03 | 2.81E-03 | 2.06E-01 |
| 13 | 5.48E-03 | 8.82E-04 | 6.43E-03 | 2.81E-03 | 2.06E-01 |
| 14 | 6.13E-01 | 3.30E-01 | 3.24E-01 | 4.36E-01 | 1.68E-02 |
| 15 | 3.27E-03 | 1.09E-03 | 2.08E-03 | 3.16E-03 | 1.86E-02 |
| 16 | 1.54E-01 | 7.15E-02 | 8.90E-02 | 1.12E-01 | 1.09E-03 |
| 17 | 3.27E-03 | 5.44E-03 | 6.66E-03 | 5.44E-03 | 6.21E-03 |
| 18 | 5.86E-03 | 1.84E-03 | 2.44E-03 | 3.43E-03 | 5.30E-03 |
| 19 | 2.04E-03 | 1.87E-03 | 6.34E-03 | 2.86E-03 | 4.83E-01 |
| 20 | 2.06E-02 | 1.92E-03 | 6.18E-03 | 3.56E-03 | 1.74E-01 |
| 21 | 2.06E-02 | 2.08E-03 | 6.49E-03 | 5.34E-03 | 4.77E-01 |
| 22 | 2.06E-02 | 2.08E-03 | 6.49E-03 | 5.34E-03 | 4.77E-01 |
| 23 | 3.45E-01 | 2.11E-01 | 2.09E-01 | 2.56E-01 | 4.61E-02 |
| 24 | 2.34E-01 | 1.31E-01 | 1.03E-01 | 1.64E-01 | 1.86E-02 |
| 25 | 3.92E-03 | 3.27E-03 | 9.44E-03 | 4.58E-03 | 6.15E-01 |
| 26 | 1.66E-02 | 3.39E-02 | 3.18E-02 | 4.22E-02 | 3.28E-03 |
| 27 | 3.46E-03 | 3.34E-03 | 5.74E-03 | 5.48E-03 | 2.58E-01 |
| 28 | 1.26E-02 | 3.42E-03 | 6.74E-03 | 5.59E-03 | 6.42E-02 |
| 29 | 5.74E-03 | 3.50E-03 | 9.56E-03 | 4.82E-03 | 4.25E-01 |
| 30 | 3.92E-03 | 5.86E-03 | 9.75E-03 | 7.85E-03 | 4.70E-01 |
| 31 | 5.99E-03 | 3.95E-03 | 1.08E-02 | 5.34E-03 | 5.16E-01 |
| 32 | 8.78E-03 | 4.23E-03 | 2.75E-02 | 1.06E-02 | 3.44E-01 |
| 33 | 2.32E-02 | 4.58E-03 | 2.06E-02 | 9.82E-03 | 6.54E-01 |
| 34 | 2.32E-02 | 4.58E-03 | 2.06E-02 | 9.82E-03 | 6.54E-01 |
| 35 | 2.01E-02 | 5.71E-03 | 1.85E-02 | 1.46E-02 | 4.58E-03 |
| 36 | 2.32E-02 | 4.58E-03 | 2.06E-02 | 9.82E-03 | 6.54E-01 |
| 37 | 3.09E-02 | 6.66E-03 | 2.19E-02 | 9.73E-03 | 4.29E-01 |
| 38 | 9.88E-03 | 8.78E-03 | 1.16E-02 | 4.95E-03 | 1.65E-01 |
| 39 | 9.88E-03 | 8.78E-03 | 1.16E-02 | 4.95E-03 | 1.65E-01 |
| 40 | 3.62E-02 | 5.03E-03 | 2.31E-02 | 1.38E-02 | 2.47E-01 |
| 41 | 1.54E-02 | 5.30E-03 | 1.43E-02 | 1.12E-02 | 6.43E-02 |
| 42 | 5.86E-03 | 9.44E-03 | 1.23E-02 | 2.06E-02 | 7.63E-02 |

(continued)

| No. | AZT_pv_adj | CAX_pv_adj | CAZ_pv_adj | CFT_pv_adj | CP_pv_adj |
|---|---|---|---|---|---|
| 43 | 1.15E-02 | 5.86E-03 | 1.39E-02 | 1.38E-02 | 2.62E-01 |
| 44 | 5.86E-03 | 9.44E-03 | 1.23E-02 | 2.06E-02 | 7.63E-02 |
| 45 | 9.73E-03 | 5.86E-03 | 1.64E-02 | 6.17E-03 | 4.25E-01 |
| 46 | 7.89E-03 | 6.49E-03 | 3.09E-02 | 1.64E-02 | 2.98E-01 |
| 47 | 7.89E-03 | 6.49E-03 | 3.09E-02 | 1.64E-02 | 2.98E-01 |
| 48 | 7.89E-03 | 6.49E-03 | 3.09E-02 | 1.64E-02 | 2.98E-01 |
| 49 | 1.20E-02 | 6.60E-03 | 1.12E-02 | 1.82E-02 | 2.16E-01 |
| 50 | 1.38E-02 | 6.67E-03 | 2.06E-02 | 1.64E-02 | 3.23E-01 |

Table 3e: Data for Enterobacter, particularly Enterobacter aerogenes (continued)

| No. | CRM_pv_adj | LVX_pv_adj | P/T_pv_adj | TO_pv_adj | T/S_pv_adj |
|---|---|---|---|---|---|
| 1 | 4.55E-04 | 5.22E-07 | 2.23E-04 | 2.03E-04 | 1.88E-06 |
| 2 | 8.93E-04 | 7.25E-07 | 2.87E-04 | 2.45E-04 | 2.53E-06 |
| 3 | 1.47E-03 | 8.82E-07 | 4.54E-03 | 2.55E-04 | 3.69E-06 |
| 4 | 1.86E-02 | 5.08E-05 | 2.73E-02 | 3.92E-03 | 3.62E-05 |
| 5 | 9.61E-04 | 7.96E-07 | 9.64E-04 | 1.26E-02 | 1.69E-03 |
| 6 | 1.46E-02 | 1.31E-04 | 8.95E-03 | 2.06E-02 | 8.55E-03 |
| 7 | 4.85E-02 | 6.35E-04 | 2.98E-01 | 9.28E-02 | 9.01E-04 |
| 8 | 1.03E-01 | 9.82E-04 | 4.28E-01 | 1.13E-01 | 1.44E-03 |
| 9 | 1.08E-02 | 2.67E-01 | 3.56E-03 | 5.09E-01 | 2.87E-01 |
| 10 | 1.66E-03 | 1.39E-01 | 5.16E-03 | 8.93E-02 | 1.08E-01 |
| 11 | 7.18E-04 | 5.91E-01 | 1.76E-02 | 7.39E-01 | 9.14E-01 |
| 12 | 7.03E-02 | 6.77E-01 | 6.85E-03 | 7.65E-01 | 9.68E-01 |
| 13 | 7.03E-02 | 6.77E-01 | 6.85E-03 | 7.65E-01 | 9.68E-01 |
| 14 | 8.23E-01 | 8.17E-02 | 2.13E-01 | 9.61E-04 | 2.24E-02 |
| 15 | 4.37E-03 | 2.22E-01 | 9.03E-03 | 2.02E-01 | 2.91E-01 |
| 16 | 4.57E-02 | 7.49E-03 | 2.26E-01 | 4.29E-01 | 4.38E-02 |
| 17 | 3.26E-02 | 1.15E-01 | 1.65E-03 | 1.93E-02 | 4.43E-02 |
| 18 | 1.64E-02 | 1.66E-01 | 9.73E-03 | 4.25E-01 | 6.31E-01 |
| 19 | 3.50E-03 | 6.96E-01 | 3.47E-02 | 1 | 1 |
| 20 | 1.20E-02 | 4.90E-01 | 1.60E-02 | 1 | 7.91E-01 |
| 21 | 2.40E-03 | 9.70E-01 | 9.40E-03 | 4.93E-01 | 6.15E-01 |
| 22 | 2.40E-03 | 9.70E-01 | 9.40E-03 | 4.93E-01 | 6.15E-01 |
| 23 | 5.50E-01 | 2.07E-01 | 1.23E-01 | 2.41E-03 | 4.88E-02 |
| 24 | 5.46E-01 | 9.58E-02 | 1.16E-01 | 2.46E-03 | 7.04E-02 |
| 25 | 1.40E-02 | 8.28E-01 | 4.99E-02 | 9.45E-01 | 8.28E-01 |
| 26 | 4.65E-02 | 5.56E-02 | 1.64E-02 | 4.80E-02 | 1.26E-02 |

(continued)

| No. | CRM_pv_adj | LVX_pv_adj | P/T_pv_adj | TO_pv_adj | T/S_pv_adj |
|---|---|---|---|---|---|
| 27 | 9.73E-03 | 5.14E-01 | 9.59E-03 | 7.93E-01 | 8.06E-01 |
| 28 | 5.86E-03 | 3.56E-01 | 4.54E-03 | 3.41E-01 | 5.47E-01 |
| 29 | 2.85E-02 | 7.23E-01 | 6.42E-02 | 8.28E-01 | 6.13E-01 |
| 30 | 1.82E-02 | 6.97E-01 | 5.99E-03 | 6.87E-01 | 9.71E-01 |
| 31 | 1.59E-02 | 8.28E-01 | 7.52E-02 | 8.28E-01 | 7.23E-01 |
| 32 | 1.61E-02 | 6.13E-01 | 9.20E-02 | 9.51E-01 | 8.47E-01 |
| 33 | 8.16E-02 | 9.71E-01 | 3.93E-02 | 3.34E-01 | 7.11E-01 |
| 34 | 8.16E-02 | 9.71E-01 | 3.93E-02 | 3.34E-01 | 7.11E-01 |
| 35 | 5.70E-02 | 3.62E-02 | 1.61E-02 | 1.47E-01 | 4.48E-02 |
| 36 | 8.16E-02 | 9.71E-01 | 3.93E-02 | 3.34E-01 | 7.11E-01 |
| 37 | 4.82E-03 | 3.59E-01 | 5.54E-03 | 7.80E-01 | 2.77E-01 |
| 38 | 1.33E-02 | 4.16E-01 | 4.96E-02 | 7.39E-01 | 5.42E-01 |
| 39 | 1.33E-02 | 4.16E-01 | 4.96E-02 | 7.39E-01 | 5.42E-01 |
| 40 | 1.95E-02 | 5.02E-01 | 5.98E-02 | 2.35E-01 | 2.07E-01 |
| 41 | 4.20E-02 | 2.18E-01 | 1.19E-02 | 2.02E-01 | 4.53E-01 |
| 42 | 7.24E-02 | 4.76E-01 | 1.76E-02 | 9.62E-01 | 7.80E-01 |
| 43 | 6.56E-02 | 5.59E-01 | 9.19E-02 | 8.66E-01 | 1 |
| 44 | 7.24E-02 | 4.76E-01 | 1.76E-02 | 9.62E-01 | 7.80E-01 |
| 45 | 1.76E-02 | 7.23E-01 | 1.10E-01 | 8.28E-01 | 7.24E-01 |
| 46 | 1.84E-02 | 6.37E-01 | 9.12E-02 | 7.39E-01 | 1 |
| 47 | 1.84E-02 | 6.37E-01 | 9.12E-02 | 7.39E-01 | 1 |
| 48 | 1.84E-02 | 6.37E-01 | 9.12E-02 | 7.39E-01 | 1 |
| 49 | 7.35E-02 | 7.11E-01 | 1.15E-02 | 5.12E-01 | 3.68E-01 |
| 50 | 1.28E-01 | 6.61E-01 | 1.11E-01 | 9.60E-01 | 1 |

Table 3f: Data for Enterobacter, particularly Enterobacter aerogenes (continued)

| No. | Locus.Tag | Gene. Symbol | GenBank.Accession | Chromosome |
|---|---|---|---|---|
| 1 | EAE_18905 | | YP_004593967 | |
| 2 | EAE_18910 | | YP_004593968 | |
| 3 | EAE_20750 | | YP_004594334 | |
| 4 | EAE_20680 | | YP_004594322 | |
| 5 | EAE_10325 | | YP_004592260 | |
| 6 | EAE_15945 | | YP_004593379 | |
| 7 | EAE_24270 | | YP_004595026 | |
| 8 | EAE_19175 | | YP_004594021 | |
| 9 | EAE_17360 | | YP_004593662 | |
| 10 | ESA_03685 | | YP_001439725 | |

(continued)

| No. | Locus.Tag | Gene. Symbol | GenBank.Accession | Chromosome |
|-----|-----------|--------------|-------------------|------------|
| 11 | EAE_20530 | tynA | YP_004594292 | |
| 12 | HMPREF0485_ 02019 | | ZP_06549619 | |
| 13 | Kvar_3843 | | YP_003440755 | |
| 14 | EAE_19000 | | YP_004593986 | |
| 15 | EAE_21240 | | YP_004594432 | |
| 16 | HMPREF9552_00257 | | ZP_07114469 | |
| 17 | EAE_11550 | | YP_004592503 | |
| 18 | EAE_21235 | | YP_004594431 | |
| 19 | EAE_24605 | flk | YP_004595093 | |
| 20 | EAE_17355 | | YP_004593661 | |
| 21 | HMPREF0484_ 0684 | | ZP_06013668 | |
| 22 | EAE_06855 | | YP_004591574 | |
| 23 | HMPREF9552_00255 | | ZP_07114467 | |
| 24 | HMPREF9552_00247 | | ZP_07114459 | |
| 25 | B21_02377 | ybl112 | CAQ32894 | |
| 26 | EAE_00560 | | YP_004590329 | |
| 27 | EAE_02625 | aphA | YP_004590739 | |
| 28 | EAE_10300 | | YP_004592255 | |
| 29 | EAE_23410 | | YP_004594860 | |
| 30 | EAE_02640 | | YP_004590742 | |
| 31 | EAE_23150 | | YP_004594808 | |
| 32 | EAE_23330 | | YP_004594844 | |
| 33 | EAE_06345 | | YP_004591472 | |
| 34 | EAE_06340 | | YP_004591471 | |
| 35 | EAE_23435 | | YP_004594865 | |
| 36 | KP1_5319 | wabN | YP_002921814 | |
| 37 | EAE_22395 | | YP_004594657 | |
| 38 | EAE_06770 | | YP_004591557 | |
| 39 | EAE_06775 | | YP_004591558 | |
| 40 | EAE_06430 | ligB | YP_004591489 | |
| 41 | EAE_15075 | | YP_004593207 | |
| 42 | EAE_03420 | | YP_004590898 | |
| 43 | EAE_23445 | | YP_004594867 | |
| 44 | EAE_03430 | | YP_004590900 | |
| 45 | EAE_23415 | | YP_004594861 | |
| 46 | EAE_06330 | | YP_004591469 | |
| 47 | EAE_06320 | | YP_004591467 | |
| 48 | EAE_06325 | | YP_004591468 | |

(continued)

| No. | Locus.Tag | Gene. Symbol | GenBank.Accession | Chromosome |
|-----|-----------|--------------|-------------------|------------|
| 49 | HMPREF0485_ 03181 | | ZP_06550780 | |
| 50 | EAE_23450 | | YP_004594868 | |

Table 3g: Data for Enterobacter, particularly Enterobacter aerogenes (continued)

| No. | Start.Coord | End.Coord | Strand | Scaffold.ID |
|-----|-------------|-----------|--------|-------------|
| 1 | 4057377 | 4057994 | - | 650716122 |
| 2 | 4057994 | 4059313 | - | 650716122 |
| 3 | 4444576 | 4447206 | - | 650716122 |
| 4 | 4432502 | 4433842 | - | 650716122 |
| 5 | 2202535 | 2203893 | - | 650716122 |
| 6 | 3439556 | 3442066 | + | 650716122 |
| 7 | 5174177 | 5175436 | + | 650716122 |
| 8 | 4113374 | 4114306 | - | 650716122 |
| 9 | 3748049 | 3748279 | + | 650716122 |
| 10 | 3623778 | 3624146 | + | 640753067 |
| 11 | 4396852 | 4399119 | + | 650716122 |
| 12 | 347124 | 347486 | + | 647536390 |
| 13 | 4046094 | 4046396 | - | 646312028 |
| 14 | 4078519 | 4079427 | + | 650716122 |
| 15 | 4545796 | 4546983 | - | 650716122 |
| 16 | 14233 | 15036 | + | 648286894 |
| 17 | 2477524 | 2479728 | + | 650716122 |
| 18 | 4544187 | 4545296 | + | 650716122 |
| 19 | 5241493 | 5242410 | + | 650716122 |
| 20 | 3747138 | 3747791 | + | 650716122 |
| 21 | 5606 | 5998 | + | 647009789 |
| 22 | 1421376 | 1421699 | + | 650716122 |
| 23 | 12357 | 13376 | + | 648286894 |
| 24 | 7786 | 8685 | - | 648286894 |
| 25 | 2517630 | 2518469 | + | 646862300 |
| 26 | 114571 | 115482 | + | 650716122 |
| 27 | 557964 | 558680 | - | 650716122 |
| 28 | 2197515 | 2198873 | + | 650716122 |
| 29 | 4974131 | 4974946 | - | 650716122 |
| 30 | 560166 | 561344 | - | 650716122 |
| 31 | 4919559 | 4920611 | + | 650716122 |
| 32 | 4955309 | 4956457 | + | 650716122 |

(continued)

| No. | Start.Coord | End.Coord | Strand | Scaffold.ID |
|-----|-------------|-----------|--------|-------------|
| 33 | 1317545 | 1318672 | + | 650716122 |
| 34 | 1316472 | 1317548 | + | 650716122 |
| 35 | 4979540 | 4980544 | + | 650716122 |
| 36 | 5064271 | 5065293 | - | 646564530 |
| 37 | 4777378 | 4777638 | - | 650716122 |
| 38 | 1404935 | 1406026 | - | 650716122 |
| 39 | 1406073 | 1406483 | + | 650716122 |
| 40 | 1331758 | 1333431 | - | 650716122 |
| 41 | 3235642 | 3239709 | + | 650716122 |
| 42 | 718097 | 718942 | - | 650716122 |
| 43 | 4982582 | 4983136 | - | 650716122 |
| 44 | 720009 | 720686 | + | 650716122 |
| 45 | 4975309 | 4976445 | - | 650716122 |
| 46 | 1314302 | 1315078 | - | 650716122 |
| 47 | 1312221 | 1313129 | + | 650716122 |
| 48 | 1313194 | 1314276 | + | 650716122 |
| 49 | 538177 | 539649 | + | 647536391 |
| 50 | 4983148 | 4984038 | - | 650716122 |

Table 4a: Data for Enterobacter, particularly Enterobacter aerogenes

| No. | Scaffold.External .Accession | Scaffold.Name | best_pv |
|-----|------------------------------|---------------|---------|
| 1 | NC_015663 | Enterobacter aerogenes KCTC 2190 chromosome: NC_015663 | 1.01047730430126e-10 |
| 2 | NC_015663 | Enterobacter aerogenes KCTC 2190 chromosome: NC_015663 | 1.01047730430126e-10 |
| 3 | NC_015663 | Enterobacter aerogenes KCTC 2190 chromosome: NC_015663 | 2.21893843673589e-10 |
| 4 | NC_015663 | Enterobacter aerogenes KCTC 2190 chromosome: NC_015663 | 7.36056945497466e-08 |
| 5 | NC_015663 | Enterobacter aerogenes KCTC 2190 chromosome: NC_015663 | 9.81164607784395e-08 |
| 6 | NC_015663 | Enterobacter aerogenes KCTC 2190 chromosome: NC_015663 | 9.81164607784395e-08 |
| 7 | NC_015663 | Enterobacter aerogenes KCTC 2190 chromosome: NC_015663 | 3.92206261774063e-06 |
| 8 | NC_015663 | Enterobacter aerogenes KCTC 2190 chromosome: NC_015663 | 9.95623777570528e-06 |
| 9 | NC_009778 | Enterobacter sakazakii ATCC BAA-894: NC_009778 | 0.000701852165838025 |

(continued)

| No. | Scaffold.External .Accession | Scaffold.Name | best_pv |
|---|---|---|---|
| 10 | NC_015663 | Enterobacter aerogenes KCTC 2190 chromosome: NC_015663 | 0.000960532001115215 |
| 11 | NC_015663 | Enterobacter aerogenes KCTC 2190 chromosome: NC_015663 | 0.00108645793188005 |
| 12 | NZ_ADTJ01000031 | Escherichia coli MS 198-1 E_coli198-1-1.0_Cont45.1: NZ_ADTJ01000031 | 0.00108724371491948 |
| 13 | NZ_ADTJ01000031 | Escherichia coli MS 198-1 E_coli198-1-1.0_Cont45.1: NZ_ADTJ01000031 | 0.00241352185546859 |
| 14 | NZ_ADTJ01000031 | Escherichia coli MS 198-1 E_coli198-1-1.0_Cont45.1: NZ_ADTJ01000031 | 0.00245969973695216 |
| 15 | NC_015663 | Enterobacter aerogenes KCTC 2190 chromosome: NC_015663 | 0.00334082507673483 |
| 16 | NC_015663 | Enterobacter aerogenes KCTC 2190 chromosome: NC_015663 | 0.00795925223735345 |

Table 4b: Data for Enterobacter, particularly Enterobacter aerogenes (continued)

| No. | sign_phenos | sign_phenos_class | best_pheno | best_pheno_class |
|---|---|---|---|---|
| 1 | AZT;CAX;CAZ;CFT;CP;CRM;LVX;P/T;TO;T/S | aminoglycoside;fluoroquinolone;lactam; other | CP | fluoroquinolone |
| 2 | AZT;CAX;CAZ;CFT;CP;CRM;LVX;P/T;TO;T/S | aminoglycoside;fluoroquinolone;lactam; other | CP | fluoroquinolone |
| 3 | AZT;CAX;CAZ;CFT;CP;CRM;LVX;P/T;TO;T/S | aminoglycoside;fluoroquinolone;lactam; other | CP | fluoroquinolone |
| 4 | CP;LVX;TO;T/S | aminoglycoside;fluoroquinolone;other | CP | fluoroquinolone |
| 5 | AZT;CAX;CAZ;CFT;CP;CRM;LVX;P/T;T/S | fluoroquinolone;lactam;other | CP | fluoroquinolone |
| 6 | AZT;CP;LVX;P/T;T/S | fluoroquinolone;lactam;other | CP | fluoroquinolone |
| 7 | CP;LVX;T/S | fluoroquinolone;other | CP | fluoroquinolone |
| 8 | CP;LVX;T/S | fluoroquinolone;other | CP | fluoroquinolone |
| 9 | AZT;CAX;CAZ;CFT;CRM;P/T | lactam | AZT | lactam |
| 10 | TO | aminoglycoside | TO | aminoglycoside |
| 11 | AZT;CAX;CAZ;CFT;CRM;P/T | lactam | CAX | lactam |
| 12 | CP;LVX | fluoroquinolone | CP | fluoroquinolone |
| 13 | TO | aminoglycoside | TO | aminoglycoside |
| 14 | TO | aminoglycoside | TO | aminoglycoside |
| 15 | AZT;CAX;CAZ;CFT;CRM;P/T | lactam | CAX | lactam |
| 16 | TO | aminoglycoside | TO | aminoglycoside |

Table 4c: Data for Enterobacter, particularly Enterobacter aerogenes (continued)

| No. | num_aminoglycoside | num_fluoroquinolone | num_lactam | num_other |
|---|---|---|---|---|
| 1 | 1 | 2 | 6 | 1 |
| 2 | 1 | 2 | 6 | 1 |
| 3 | 1 | 2 | 6 | 1 |
| 4 | 1 | 2 | 0 | 1 |
| 5 | 0 | 2 | 6 | 1 |
| 6 | 0 | 2 | 2 | 1 |
| 7 | 0 | 2 | 0 | 1 |
| 8 | 0 | 2 | 0 | 1 |
| 9 | 0 | 0 | 6 | 0 |
| 10 | 1 | 0 | 0 | 0 |
| 11 | 0 | 0 | 6 | 0 |
| 12 | 0 | 2 | 0 | 0 |
| 13 | 1 | 0 | 0 | 0 |
| 14 | 1 | 0 | 0 | 0 |
| 15 | 0 | 0 | 6 | 0 |
| 16 | 1 | 0 | 0 | 0 |

Table 4d: Data for Enterobacter, particularly Enterobacter aerogenes (continued)

| No. | AZT_pv_adj | CAX_pv_adj | CAZ_pv_adj | CFT_pv_adj | CP_pv_adj |
|---|---|---|---|---|---|
| 1 | 8.22E-04 | 2.45E-04 | 3.83E-04 | 4.31E-04 | 1.01E-10 |
| 2 | 1.32E-03 | 4.48E-04 | 7.06E-04 | 7.76E-04 | 1.01E-10 |
| 3 | 3.27E-03 | 1.99E-03 | 4.43E-03 | 3.01E-03 | 2.22E-10 |
| 4 | 2.06E-02 | 1.86E-02 | 2.29E-02 | 2.41E-02 | 7.36E-08 |
| 5 | 4.29E-03 | 2.32E-04 | 6.91E-04 | 7.06E-04 | 9.81E-08 |
| 6 | 8.86E-03 | 1.82E-02 | 1.77E-02 | 1.84E-02 | 9.81E-08 |
| 7 | 5.47E-01 | 1.61E-02 | 4.75E-02 | 4.90E-02 | 3.92E-06 |
| 8 | 6.96E-01 | 3.29E-02 | 9.63E-02 | 9.80E-02 | 9.96E-06 |
| 9 | 7.02E-04 | 3.95E-03 | 6.47E-03 | 6.18E-03 | 2.19E-02 |
| 10 | 6.13E-01 | 3.30E-01 | 3.24E-01 | 4.36E-01 | 1.68E-02 |
| 11 | 3.27E-03 | 1.09E-03 | 2.08E-03 | 3.16E-03 | 1.86E-02 |
| 12 | 1.54E-01 | 7.15E-02 | 8.90E-02 | 1.12E-01 | 1.09E-03 |
| 13 | 3.45E-01 | 2.11E-01 | 2.09E-01 | 2.56E-01 | 4.61E-02 |
| 14 | 2.34E-01 | 1.31E-01 | 1.03E-01 | 1.64E-01 | 1.86E-02 |
| 15 | 3.46E-03 | 3.34E-03 | 5.74E-03 | 5.48E-03 | 2.58E-01 |
| 16 | 3.30E-01 | 2.07E-01 | 1.34E-01 | 1.68E-01 | 8.94E-01 |

Table 4e: Data for Enterobacter, particularly Enterobacter aerogenes (continued)

| No. | CRM_pv_adj | LVX_pv_adj | P/T_pv_adj | TO_pv_adj | T/S_pv_adj |
|-----|-----------|-----------|-----------|-----------|-----------|
| 1 | 4.55E-04 | 5.22E-07 | 2.23E-04 | 2.03E-04 | 1.88E-06 |
| 2 | 8.93E-04 | 7.25E-07 | 2.87E-04 | 2.45E-04 | 2.53E-06 |
| 3 | 1.47E-03 | 8.82E-07 | 4.54E-03 | 2.55E-04 | 3.69E-06 |
| 4 | 1.86E-02 | 5.08E-05 | 2.73E-02 | 3.92E-03 | 3.62E-05 |
| 5 | 9.61E-04 | 7.96E-07 | 9.64E-04 | 1.26E-02 | 1.69E-03 |
| 6 | 1.46E-02 | 1.31E-04 | 8.95E-03 | 2.06E-02 | 8.55E-03 |
| 7 | 4.85E-02 | 6.35E-04 | 2.98E-01 | 9.28E-02 | 9.01E-04 |
| 8 | 1.03E-01 | 9.82E-04 | 4.28E-01 | 1.13E-01 | 1.44E-03 |
| 9 | 1.66E-03 | 1.39E-01 | 5.16E-03 | 8.93E-02 | 1.08E-01 |
| 10 | 8.23E-01 | 8.17E-02 | 2.13E-01 | 9.61E-04 | 2.24E-02 |
| 11 | 4.37E-03 | 2.22E-01 | 9.03E-03 | 2.02E-01 | 2.91E-01 |
| 12 | 4.57E-02 | 7.49E-03 | 2.26E-01 | 4.29E-01 | 4.38E-02 |
| 13 | 5.50E-01 | 2.07E-01 | 1.23E-01 | 2.41E-03 | 4.88E-02 |
| 14 | 5.46E-01 | 9.58E-02 | 1.16E-01 | 2.46E-03 | 7.04E-02 |
| 15 | 9.73E-03 | 5.14E-01 | 9.59E-03 | 7.93E-01 | 8.06E-01 |
| 16 | 8.88E-01 | 8.87E-01 | 4.35E-02 | 7.96E-03 | 8.87E-01 |

Table 4f: Data for Enterobacter, particularly Enterobacter aerogenes (continued)

| No. | Locus.Tag | Gene. Symbol | GenBank.Accession | Chromosome |
|-----|-----------|--------------|-------------------|------------|
| 1 | EAE_18905 | | YP_004593967 | |
| 2 | EAE_18910 | | YP_004593968 | |
| 3 | EAE_20750 | | YP_004594334 | |
| 4 | EAE_20680 | | YP_004594322 | |
| 5 | EAE_10325 | | YP_004592260 | |
| 6 | EAE_15945 | | YP_004593379 | |
| 7 | EAE_24270 | | YP_004595026 | |
| 8 | EAE_19175 | | YP_004594021 | |
| 9 | ESA_03685 | | YP_001439725 | |
| 10 | EAE_19000 | | YP_004593986 | |
| 11 | EAE_21240 | | YP_004594432 | |
| 12 | HMPREF9552_00257 | | ZP_07114469 | |
| 13 | HMPREF9552_00255 | | ZP_07114467 | |
| 14 | HMPREF9552_00247 | | ZP_07114459 | |
| 15 | EAE_02625 | aphA | YP_004590739 | |
| 16 | EAE_18950 | | YP_004593976 | |

Table 4g: Data for Enterobacter, particularly Enterobacter aerogenes (continued)

| No. | Start.Coord | End.Coord | Strand | Scaffold.ID |
|-----|-------------|-----------|--------|-------------|
| 1 | 4057377 | 4057994 | - | 650716122 |
| 2 | 4057994 | 4059313 | - | 650716122 |
| 3 | 4444576 | 4447206 | - | 650716122 |
| 4 | 4432502 | 4433842 | - | 650716122 |
| 5 | 2202535 | 2203893 | - | 650716122 |
| 6 | 3439556 | 3442066 | + | 650716122 |
| 7 | 5174177 | 5175436 | + | 650716122 |
| 8 | 4113374 | 4114306 | - | 650716122 |
| 9 | 3623778 | 3624146 | + | 640753067 |
| 10 | 4078519 | 4079427 | + | 650716122 |
| 11 | 4545796 | 4546983 | - | 650716122 |
| 12 | 14233 | 15036 | + | 648286894 |
| 13 | 12357 | 13376 | + | 648286894 |
| 14 | 7786 | 8685 | - | 648286894 |
| 15 | 557964 | 558680 | - | 650716122 |
| 16 | 4069127 | 4070251 | - | 650716122 |

Table 5a: Data for Enterobacter, particularly Enterobacter cloacae

| No. | Scaffold.External. Accession | Scaffold.Name | best_pv |
|-----|------------------------------|---------------|---------|
| 1 | NC_013509 | Edwardsiella tarda EIB202 plasmid pEIB202: NC_013509 | 5.49490970435242e-27 |
| 2 | NC_014107 | Enterobacter cloacae subsp. cloacae ATCC 13047 plasmid pECL_A: NC_014107 | 5.83313344212793e-25 |
| 3 | NC_009838 | Escherichia coli APEC O1 plasmid pAPEC-O1-R: NC_009838 | 5.83313344212793e-25 |
| 4 | NC_009838 | Escherichia coli APEC O1 plasmid pAPEC-O1-R: NC_009838 | 5.83313344212793e-25 |
| 5 | NC_014107 | Enterobacter cloacae subsp. cloacae ATCC 13047 plasmid pECL_A: NC_014107 | 1.25162242820201e-24 |
| 6 | NC_014107 | Enterobacter cloacae subsp. cloacae ATCC 13047 plasmid pECL_A: NC_014107 | 2.31689992231266e-24 |
| 7 | NC_014107 | Enterobacter cloacae subsp. cloacae ATCC 13047 plasmid pECL_A: NC_014107 | 2.46438038993034e-24 |
| 8 | NC_014107 | Enterobacter cloacae subsp. cloacae ATCC 13047 plasmid pECL_A: NC_014107 | 2.46438038993034e-24 |
| 9 | NZ_GG657370 | Citrobacter sp. 30_2 genomic scaffold supercont1.5: NZ_GG657370 | 2.46438038993034e-24 |
| 10 | NZ_GG657370 | Citrobacter sp. 30_2 genomic scaffold supercont1.5: NZ_GG657370 | 1.59357817134779e-23 |
| 11 | NC_009838 | Escherichia coli APEC O1 plasmid pAPEC-O1-R: NC_009838 | 1.59357817134779e-23 |

(continued)

| No. | Scaffold.External. Accession | Scaffold.Name | best_pv |
|---|---|---|---|
| 12 | NC_009778 | Enterobacter sakazakii ATCC BAA-894: NC_009778 | 2.83245228849161e-23 |
| 13 | NC_014107 | Enterobacter cloacae subsp. cloacae ATCC 13047 plasmid pECL_A: NC_014107 | 2.83245228849161e-23 |
| 14 | NZ_ ADUD01000537 | Escherichia coli MS 196-1 E_coli196-1-1.0_Cont1482.1: NZ_ADUD01000537 | 9.1281268279348e-23 |
| 15 | NC_014121 | Enterobacter cloacae subsp. cloacae ATCC 13047 chromosome: NC_014121 | 2.16502306009163e-22 |
| 16 | NZ_ ABFH01000001 | Salmonella enterica subsp. enterica serovar Virchow str. SL491, unfinished sequence: NZ_ABFH01000001 | 4.49434330590708e-22 |
| 17 | NC_004741 | Shigella flexneri 2a str. 2457T: NC_004741 | 1.02679325501045e-21 |
| 18 | NC_014121 | Enterobacter cloacae subsp. cloacae ATCC 13047 chromosome: NC_014121 | 1.4942758061424e-21 |
| 19 | NZ_ AFBO01000168 | Klebsiella sp. MS 92-3 K_spMS92-3-1.0_Cont398.10: NZ_AFBO01000168 | 2.33876952186319e-21 |
| 20 | NC_014121 | Enterobacter cloacae subsp. cloacae ATCC 13047 chromosome: NC_014121 | 7.16913512003583e-16 |
| 21 | CP001383 | Shigella flexneri 2002017: CP001383 | 1.0532279235518e-15 |
| 22 | CP001637 | Escherichia coli DH1: CP001637 | 4.34714801916322e-15 |
| 23 | NZ_ ADWT01000006 | Escherichia coli MS 124-1 E_coliMS124-1-1.0.1_Cont5.1: NZ_ADWT01000006 | 2.30933700130366e-14 |
| 24 | NZ_ ADTR01000359 | Escherichia coli MS 21-1 E_coli21-1-1.0_Cont669.1: NZ_ADTR01000359 | 3.84987338572512e-13 |
| 25 | NC_014121 | Enterobacter cloacae subsp. cloacae ATCC 13047 chromosome: NC_014121 | 5.47488441522503e-11 |
| 26 | NZ_ AAJV01000019 | Escherichia coli E22, unfinished sequence: NZ_AAJV01000019 | 9.06569667406974e-10 |
| 27 | FP929040 | Enterobacter cloacae subsp. cloacae NCTC 9394 draft genome.: FP929040 | 1.83557061686134e-09 |
| 28 | FP929040 | Enterobacter cloacae subsp. cloacae NCTC 9394 draft genome.: FP929040 | 3.37768924887661e-09 |
| 29 | FP929040 | Enterobacter cloacae subsp. cloacae NCTC 9394 draft genome.: FP929040 | 5.84222468117604e-09 |
| 30 | NZ_ AFHR01000043 | Enterobacter hormaechei ATCC 49162 contig00043: NZ_AFHR01000043 | 8.80996353751187e-09 |
| 31 | FP929040 | Enterobacter cloacae subsp. cloacae NCTC 9394 draft genome.: FP929040 | 1.29388450812614e-08 |
| 32 | FP929040 | Enterobacter cloacae subsp. cloacae NCTC 9394 draft genome.: FP929040 | 1.29388450812614e-08 |
| 33 | FP929040 | Enterobacter cloacae subsp. cloacae NCTC 9394 draft genome.: FP929040 | 1.29388450812614e-08 |
| 34 | NZ_ AFHR01000073 | Enterobacter hormaechei ATCC 49162 contig00073: NZ_AFHR01000073 | 1.29388450812614e-08 |

(continued)

| No. | Scaffold.External. Accession | Scaffold.Name | best_pv |
|-----|------------------------------|---------------|---------|
| 35 | NZ_ AFHR01000073 | Enterobacter hormaechei ATCC 49162 contig00073: NZ_ AFHR01000073 | 1.29388450812614e-08 |
| 36 | NZ_ AFHR01000034 | Enterobacter hormaechei ATCC 49162 contig00034: NZ_ AFHR01000034 | 1.29388450812614e-08 |
| 37 | NZ_ AFHR01000073 | Enterobacter hormaechei ATCC 49162 contig00073: NZ_ AFHR01000073 | 2.23051874499664e-08 |
| 38 | FP929040 | Enterobacter cloacae subsp. cloacae NCTC 9394 draft genome.: FP929040 | 2.39066831640169e-08 |
| 39 | NZ_ AFHR01000062 | Enterobacter hormaechei ATCC 49162 contig00062: NZ_ AFHR01000062 | 2.39066831640169e-08 |
| 40 | NC_014618 | Enterobacter cloacae SCF1 chromosome: NC_014618 | 2.76892470483105e-08 |
| 41 | NC_014618 | Enterobacter cloacae SCF1 chromosome: NC_014618 | 3.01843352491008e-08 |
| 42 | NZ_ ABWM01000064 | Enterobacter cancerogenus ATCC 35316, unfinished sequence: NZ_ABWM01000064 | 4.40355882800235e-08 |
| 43 | NC_014121 | Enterobacter cloacae subsp. cloacae ATCC 13047 chromosome: NC_014121 | 4.44394973624704e-08 |
| 44 | NZ_GG657370 | Citrobacter sp. 30_2 genomic scaffold supercont1.5: NZ_ GG657370 | 5.11952538533182e-08 |
| 45 | NC_014618 | Enterobacter cloacae SCF1 chromosome: NC_014618 | 5.14762193286233e-08 |
| 46 | NZ_ AFHR01000015 | Enterobacter hormaechei ATCC 49162 contig00015: NZ_ AFHR01000015 | 5.14762193286233e-08 |
| 47 | FP929040 | Enterobacter cloacae subsp. cloacae NCTC 9394 draft genome.: FP929040 | 5.31046635969439e-08 |
| 48 | FP929040 | Enterobacter cloacae subsp. cloacae NCTC 9394 draft genome.: FP929040 | 5.31046635969439e-08 |
| 49 | FP929040 | Enterobacter cloacae subsp. cloacae NCTC 9394 draft genome.: FP929040 | 5.31046635969439e-08 |
| 50 | FP929040 | Enterobacter cloacae subsp. cloacae NCTC 9394 draft genome.: FP929040 | 5.31046635969439e-08 |

Table 5b: Data for Enterobacter, particularly Enterobacter cloacae (continued)

| No | sign_phenos | sign_phenos_class | best_ pheno | best_pheno_ class |
|----|-------------|-------------------|-------------|-------------------|
| 1 | AZT;CAX;CAZ;CFT;CP;CPE; CRM;ETP;GM;LVX;P/T;TO ;T/S | aminoglycoside;fluoroquinolone; lactam; other | T/S | other |
| 2 | AZT;CAX;CAZ;CFT;CP;CPE; CRM;GM;LVX;P/T;TO;T/S | aminoglycoside;fluoroquinolone; lactam; other | T/S | other |
| 3 | AZT;CAX;CAZ;CFT;CP;CPE; CRM;GM;LVX;P/T;TO;T/S | aminoglycoside;fluoroquinolone; lactam; other | T/S | other |
| 4 | AZT;CAX;CAZ;CFT;CP;CPE; CRM;GM;LVX;P/T;TO;T/S | aminoglycoside;fluoroquinolone; lactam; other | T/S | other |
| 5 | AZT;CAX;CAZ;CFT;CP;CPE; CRM;GM;LVX;P/T;TO;T/S | aminoglycoside;fluoroquinolone; lactam; other | T/S | other |

(continued)

| No | sign_phenos | sign_phenos_class | best_pheno | best_pheno_ class |
|---|---|---|---|---|
| 6 | AZT;CAX;CAZ;CFT;CP;CPE; ETP;GM;LVX;P/T;TO;T/S | aminoglycoside;fluoroquinolone; lactam; other | T/S | other |
| 7 | AZT;CAX;CAZ;CFT;CP;CPE; GM;LVX;TO;T/S | aminoglycoside;fluoroquinolone; lactam; other | T/S | other |
| 8 | AZT;CAX;CAZ;CFT;CP;CPE; GM;LVX;TO;T/S | aminoglycoside;fluoroquinolone; lactam; other | T/S | other |
| 9 | AZT;CAX;CAZ;CFT;CP;CPE; CRM;GM;LVX;TO;T/S | aminoglycoside;fluoroquinolone; lactam; other | T/S | other |
| 10 | AZT;CAX;CAZ;CFT;CP;CPE; CRM;GM;LVX;P/T;TO;T/S | aminoglycoside;fluoroquinolone; lactam; other | T/S | other |
| 11 | AZT;CAX;CAZ;CFT;CP;CPE; GM;LVX;TO;T/S | aminoglycoside;fluoroquinolone; lactam; other | T/S | other |
| 12 | AZT;CAX;CAZ;CFT;CP;CPE; CRM;GM;LVX;TO;T/S | aminoglycoside;fluoroquinolone; lactam; other | T/S | other |
| 13 | AZT;CAX;CAZ;CFT;CP;CPE; GM;LVX;TO;T/S | aminoglycoside;fluoroquinolone; lactam; other | T/S | other |
| 14 | AZT;CAX;CAZ;CFT;CP;CPE; CRM;ETP;GM;LVX;P/T;TO ;T/S | aminoglycoside;fluoroquinolone; lactam; other | GM | aminoglycoside |
| 15 | AZT;CAX;CAZ;CFT;CP;CPE; CRM;ETP;GM;LVX;P/T;TO ;T/S | aminoglycoside;fluoroquinolone; lactam; other | T/S | other |
| 16 | AZT;CAX;CAZ;CFT;CP;CPE; CRM;ETP;GM;LVX;P/T;TO ;T/S | aminoglycoside;fluoroquinolone; lactam; other | GM | aminoglycoside |
| 17 | AZT;CAX;CAZ;CFT;CP;CPE; CRM;GM;LVX;P/T;TO;T/S | aminoglycoside;fluoroquinolone; lactam; other | GM | aminoglycoside |
| 18 | AZT;CAX;CAZ;CFT;CP;CPE; CRM;ETP;GM;LVX;P/T;TO ;T/S | aminoglycoside;fluoroquinolone; lactam; other | TO | aminoglycoside |
| 19 | AZT;CAX;CAZ;CFT;CP;CPE; CRM;ETP;GM;LVX;P/T;TO ;T/S | aminoglycoside;fluoroquinolone; lactam; other | T/S | other |
| 20 | AZT;CAX;CAZ;CFT;CP;CPE; CRM;GM;LVX;P/T;TO;T/S | aminoglycoside;fluoroquinolone; lactam; other | TO | aminoglycoside |
| 21 | AZT;CAX;CAZ;CFT;CP;CPE; GM;LVX;P/T;TO;T/S | aminoglycoside;fluoroquinolone; lactam; other | T/S | other |
| 22 | AZT;CAX;CAZ;CFT;CP;GM;LVX; P/T;TO;T/S | aminoglycoside;fluoroquinolone; lactam; other | T/S | other |
| 23 | AZT;CAX;CAZ;CFT;CP;CPE; GM;LVX;P/T;TO;T/S | aminoglycoside;fluoroquinolone; lactam; other | GM | aminoglycoside |
| 24 | AZT;CAX;CAZ;CFT;CP;CPE; GM;LVX;P/T;TO;T/S | aminoglycoside;fluoroquinolone; lactam; other | GM | aminoglycoside |
| 25 | AZT;CAX;CAZ;CFT;CP;CPE; ETP;GM;LVX;P/T;TO;T/S | aminoglycoside;fluoroquinolone; lactam; other | TO | aminoglycoside |
| 26 | AZT;CAX;CAZ;CFT;CP;CPE; ETP;GM;LVX;TO;T/S | aminoglycoside;fluoroquinolone; lactam; other | TO | aminoglycoside |
| 27 | AZT;CAX;CAZ;CFT;CP;CPE; GM;LVX;P/T;TO;T/S | aminoglycoside;fluoroquinolone; lactam; other | CPE | lactam |

(continued)

| No | sign_phenos | sign_phenos_class | best_pheno | best_pheno_ class |
|---|---|---|---|---|
| 28 | AZT;CAX;CAZ;CFT;CPE;GM; LVX;P/T;TO | aminoglycoside;fluoroquinolone;lactam | CPE | lactam |
| 29 | AZT;CAX;CAZ;CFT;CPE;GM; LVX;P/T;TO;T/S | aminoglycoside;fluoroquinolone; lactam; other | CPE | lactam |
| 30 | AZT;CAZ;CFT;CP;CPE;GM;LVX; P/T;TO;T/S | aminoglycoside;fluoroquinolone; lactam; other | CPE | lactam |
| 31 | AZT;CAX;CAZ;CPE;GM;LVX; P/T;TO;T/S | aminoglycoside;fluoroquinolone; lactam; other | CPE | lactam |
| 32 | AZT;CAX;CAZ;CPE;GM;LVX; P/T;TO;T/S | aminoglycoside;fluoroquinolone; lactam; other | CPE | lactam |
| 33 | AZT;CAX;CAZ;CPE;GM;LVX; P/T;TO;T/S | aminoglycoside;fluoroquinolone; lactam; other | CPE | lactam |
| 34 | AZT;CAX;CAZ;CPE;GM;LVX; P/T;TO;T/S | aminoglycoside;fluoroquinolone; lactam; other | CPE | lactam |
| 35 | AZT;CAX;CAZ;CPE;GM;LVX; P/T;TO;T/S | aminoglycoside;fluoroquinolone; lactam; other | CPE | lactam |
| 36 | AZT;CAX;CAZ;CPE;GM;LVX; P/T;TO;T/S | aminoglycoside;fluoroquinolone; lactam; other | CPE | lactam |
| 37 | AZT;CAX;CAZ;CPE;GM;LVX; P/T;TO;T/S | aminoglycoside;fluoroquinolone; lactam; other | CPE | lactam |
| 38 | CP;CPE;GM;LVX;TO;T/S | aminoglycoside;fluoroquinolone; lactam; other | CPE | lactam |
| 39 | CP;CPE;GM;LVX;TO;T/S | aminoglycoside;fluoroquinolone; lactam; other | CPE | lactam |
| 40 | AZT;CAX;CAZ;CFT;CRM;GM; P/T;TO;T/S | aminoglycoside;lactam;other | T/S | other |
| 41 | AZT;CAX;CAZ;CFT;CP;CPE; ETP;GM;LVX;P/T;TO;T/S | aminoglycoside;fluoroquinolone; lactam; other | CPE | lactam |
| 42 | CP;CPE;GM;LVX;TO;T/S | aminoglycoside;fluoroquinolone; lactam; other | CPE | lactam |
| 43 | AZT;CAX;CAZ;CPE;GM;LVX; P/T;TO;T/S | aminoglycoside;fluoroquinolone; lactam; other | CPE | lactam |
| 44 | AZT;CAX;CAZ;CFT;CP;GM;LVX; P/T;TO;T/S | aminoglycoside;fluoroquinolone; lactam; other | T/S | other |
| 45 | CAX;CAZ;CPE;GM;LVX;P/T;TO; T/S | aminoglycoside;fluoroquinolone; lactam; other | CPE | lactam |
| 46 | CP;CPE;GM;LVX;TO;T/S | aminoglycoside;fluoroquinolone; lactam; other | CPE | lactam |
| 47 | AZT;CAX;CAZ;CPE;GM;LVX; P/T;TO;T/S | aminoglycoside;fluoroquinolone; lactam; other | CPE | lactam |
| 48 | AZT;CAX;CAZ;CPE;GM;LVX; P/T;TO;T/S | aminoglycoside;fluoroquinolone; lactam; other | CPE | lactam |

(continued)

| No | sign_phenos | sign_phenos_class | best_ pheno | best_pheno_ class |
|---|---|---|---|---|
| 49 | AZT;CAX;CAZ;CPE;GM;LVX; P/T;TO;T/S | aminoglycoside;fluoroquinolone; lactam; other | CPE | lactam |
| 50 | AZT;CAX;CAZ;CPE;GM;LVX; P/T;TO;T/S | aminoglycoside;fluoroquinolone; lactam; other | CPE | lactam |

Table 5c: Data for Enterobacter, particularly Enterobacter cloacae (continued)

| No. | num_aminoglycoside | num_fluoroquinolone | num_lactam | num_other |
|---|---|---|---|---|
| 1 | 2 | 2 | 8 | 1 |
| 2 | 2 | 2 | 7 | 1 |
| 3 | 2 | 2 | 7 | 1 |
| 4 | 2 | 2 | 7 | 1 |
| 5 | 2 | 2 | 7 | 1 |
| 6 | 2 | 2 | 7 | 1 |
| 7 | 2 | 2 | 5 | 1 |
| 8 | 2 | 2 | 5 | 1 |
| 9 | 2 | 2 | 6 | 1 |
| 10 | 2 | 2 | 7 | 1 |
| 11 | 2 | 2 | 5 | 1 |
| 12 | 2 | 2 | 6 | 1 |
| 13 | 2 | 2 | 5 | 1 |
| 14 | 2 | 2 | 8 | 1 |
| 15 | 2 | 2 | 8 | 1 |
| 16 | 2 | 2 | 8 | 1 |
| 17 | 2 | 2 | 7 | 1 |
| 18 | 2 | 2 | 8 | 1 |
| 19 | 2 | 2 | 8 | 1 |
| 20 | 2 | 2 | 7 | 1 |
| 21 | 2 | 2 | 6 | 1 |
| 22 | 2 | 2 | 5 | 1 |
| 23 | 2 | 2 | 6 | 1 |
| 24 | 2 | 2 | 6 | 1 |
| 25 | 2 | 2 | 7 | 1 |
| 26 | 2 | 2 | 6 | 1 |
| 27 | 2 | 2 | 6 | 1 |
| 28 | 2 | 1 | 6 | 0 |
| 29 | 2 | 1 | 6 | 1 |
| 30 | 2 | 2 | 5 | 1 |

(continued)

| No. | num_aminoglycoside | num_fluoroquinolone | num_lactam | num_other |
|-----|--------------------|--------------------|-----------|-----------|
| 31 | 2 | 1 | 5 | 1 |
| 32 | 2 | 1 | 5 | 1 |
| 33 | 2 | 1 | 5 | 1 |
| 34 | 2 | 1 | 5 | 1 |
| 35 | 2 | 1 | 5 | 1 |
| 36 | 2 | 1 | 5 | 1 |
| 37 | 2 | 1 | 5 | 1 |
| 38 | 2 | 2 | 1 | 1 |
| 39 | 2 | 2 | 1 | 1 |
| 40 | 2 | 0 | 6 | 1 |
| 41 | 2 | 2 | 7 | 1 |
| 42 | 2 | 2 | 1 | 1 |
| 43 | 2 | 1 | 5 | 1 |
| 44 | 2 | 2 | 5 | 1 |
| 45 | 2 | 1 | 4 | 1 |
| 46 | 2 | 2 | 1 | 1 |
| 47 | 2 | 1 | 5 | 1 |
| 48 | 2 | 1 | 5 | 1 |
| 49 | 2 | 1 | 5 | 1 |
| 50 | 2 | 1 | 5 | 1 |

Table 5d: Data for Enterobacter, particularly Enterobacter cloacae (continued)

| No. | AZT_pv_adj | CAX_pv_adj | CAZ_pv_adj | CFT_pv_adj | CP_pv_adj |
|-----|-----------|-----------|-----------|-----------|-----------|
| 1 | 3.58E-10 | 7.39E-09 | 2.80E-11 | 3.62E-09 | 5.27E-08 |
| 2 | 4.06E-06 | 7.65E-06 | 8.35E-07 | 8.00E-07 | 3.72E-07 |
| 3 | 4.06E-06 | 7.65E-06 | 8.35E-07 | 8.00E-07 | 3.72E-07 |
| 4 | 4.06E-06 | 7.65E-06 | 8.35E-07 | 8.00E-07 | 3.72E-07 |
| 5 | 8.70E-07 | 8.75E-06 | 5.21E-07 | 5.80E-07 | 1.18E-06 |
| 6 | 1.92E-12 | 2.08E-13 | 5.48E-12 | 1.92E-12 | 4.74E-09 |
| 7 | 2.20E-05 | 4.05E-05 | 4.94E-06 | 4.85E-06 | 7.75E-07 |
| 8 | 2.20E-05 | 4.05E-05 | 4.94E-06 | 4.85E-06 | 7.75E-07 |
| 9 | 6.62E-06 | 1.21E-05 | 1.34E-06 | 1.27E-06 | 3.72E-07 |
| 10 | 3.70E-06 | 6.73E-06 | 7.48E-07 | 7.08E-07 | 2.65E-06 |
| 11 | 3.46E-05 | 6.07E-05 | 7.99E-06 | 7.84E-06 | 7.16E-07 |
| 12 | 6.62E-06 | 1.21E-05 | 1.34E-06 | 1.27E-06 | 3.14E-06 |
| 13 | 2.20E-05 | 4.05E-05 | 4.94E-06 | 4.85E-06 | 7.75E-07 |
| 14 | 2.41E-09 | 3.66E-10 | 2.89E-10 | 1.33E-09 | 1.31E-11 |

(continued)

| No. | AZT_pv_adj | CAX_pv_adj | CAZ_pv_adj | CFT_pv_adj | CP_pv_adj |
|-----|-----------|-----------|-----------|-----------|-----------|
| 15 | 6.79E-08 | 4.49E-08 | 1.13E-08 | 3.99E-08 | 4.19E-12 |
| 16 | 3.51E-09 | 1.67E-09 | 8.09E-10 | 8.35E-10 | 5.65E-12 |
| 17 | 3.11E-11 | 6.47E-11 | 7.01E-12 | 5.47E-11 | 1.53E-06 |
| 18 | 4.36E-12 | 6.39E-12 | 5.85E-13 | 5.48E-12 | 5.70E-07 |
| 19 | 6.79E-08 | 4.49E-08 | 1.13E-08 | 3.99E-08 | 3.61E-11 |
| 20 | 5.39E-09 | 1.23E-08 | 5.84E-09 | 6.70E-09 | 5.19E-07 |
| 21 | 3.36E-07 | 5.25E-07 | 5.19E-07 | 3.74E-06 | 3.91E-05 |
| 22 | 4.53E-07 | 8.07E-07 | 7.17E-07 | 6.01E-06 | 7.16E-05 |
| 23 | 1.62E-07 | 3.02E-07 | 2.83E-08 | 5.65E-08 | 9.46E-05 |
| 24 | 8.58E-09 | 1.75E-08 | 5.92E-08 | 2.01E-07 | 7.82E-07 |
| 25 | 5.28E-06 | 5.19E-07 | 1.32E-06 | 7.50E-07 | 1.27E-05 |
| 26 | 3.71E-05 | 2.06E-06 | 1.56E-05 | 9.73E-06 | 1.34E-04 |
| 27 | 2.41E-04 | 8.91E-05 | 8.91E-05 | 4.87E-04 | 5.72E-04 |
| 28 | 2.12E-03 | 7.75E-04 | 1.61E-03 | 5.63E-03 | 2.33E-02 |
| 29 | 3.60E-03 | 1.44E-03 | 1.46E-03 | 6.84E-03 | 1.58E-02 |
| 30 | 1.63E-03 | 1.30E-02 | 1.26E-03 | 9.88E-03 | 1.03E-03 |
| 31 | 8.73E-03 | 1.90E-03 | 3.60E-03 | 1.54E-02 | 2.18E-02 |
| 32 | 8.73E-03 | 1.90E-03 | 3.60E-03 | 1.54E-02 | 2.18E-02 |
| 33 | 8.73E-03 | 1.90E-03 | 3.60E-03 | 1.54E-02 | 2.18E-02 |
| 34 | 8.73E-03 | 1.90E-03 | 3.60E-03 | 1.54E-02 | 2.18E-02 |
| 35 | 8.73E-03 | 1.90E-03 | 3.60E-03 | 1.54E-02 | 2.18E-02 |
| 36 | 8.73E-03 | 1.90E-03 | 3.60E-03 | 1.54E-02 | 2.18E-02 |
| 37 | 9.06E-03 | 2.62E-03 | 4.92E-03 | 1.99E-02 | 2.22E-02 |
| 38 | 3.58E-02 | 1.04E-02 | 1.75E-02 | 5.31E-02 | 2.20E-05 |
| 39 | 5.33E-02 | 1.78E-02 | 2.85E-02 | 7.63E-02 | 6.85E-05 |
| 40 | 8.52E-05 | 3.90E-05 | 6.01E-06 | 2.49E-05 | 1.29E-02 |
| 41 | 4.11E-06 | 7.41E-04 | 4.26E-05 | 1.08E-03 | 6.16E-05 |
| 42 | 4.35E-02 | 1.36E-02 | 2.23E-02 | 6.48E-02 | 2.37E-05 |
| 43 | 8.79E-03 | 2.56E-03 | 4.80E-03 | 1.96E-02 | 2.95E-02 |
| 44 | 5.44E-03 | 1.03E-03 | 6.23E-04 | 1.01E-03 | 1.45E-04 |
| 45 | 1.49E-02 | 4.69E-03 | 8.56E-03 | 3.09E-02 | 3.86E-02 |
| 46 | 2.22E-01 | 6.69E-02 | 1.35E-01 | 3.01E-01 | 6.26E-04 |
| 47 | 8.87E-03 | 1.93E-03 | 3.69E-03 | 1.56E-02 | 3.05E-02 |
| 48 | 8.87E-03 | 1.93E-03 | 3.69E-03 | 1.56E-02 | 3.05E-02 |
| 49 | 8.87E-03 | 1.93E-03 | 3.69E-03 | 1.56E-02 | 3.05E-02 |
| 50 | 8.87E-03 | 1.93E-03 | 3.69E-03 | 1.56E-02 | 3.05E-02 |

Table 5e: Data for Enterobacter, particularly Enterobacter cloacae (continued)

| No. | CPE_pv_adj | CRM_pv_adj | ETP_pv_adj | GM_pv_adj |
|---|---|---|---|---|
| 1 | 2.76E-09 | 1.20E-03 | 1.29E-04 | 3.48E-19 |
| 2 | 2.95E-05 | 2.74E-03 | 5.44E-02 | 5.95E-17 |
| 3 | 2.95E-05 | 2.74E-03 | 5.44E-02 | 5.95E-17 |
| 4 | 2.95E-05 | 2.74E-03 | 5.44E-02 | 5.95E-17 |
| 5 | 5.40E-05 | 2.87E-03 | 9.20E-02 | 6.93E-16 |
| 6 | 3.27E-08 | 3.20E-02 | 6.76E-03 | 1.07E-19 |
| 7 | 6.14E-05 | 1.06E-02 | 5.77E-02 | 2.51E-16 |
| 8 | 6.14E-05 | 1.06E-02 | 5.77E-02 | 2.51E-16 |
| 9 | 5.81E-05 | 2.77E-03 | 7.62E-02 | 4.00E-16 |
| 10 | 5.02E-05 | 3.99E-03 | 3.88E-02 | 2.55E-15 |
| 11 | 5.72E-05 | 1.10E-02 | 5.64E-02 | 1.59E-16 |
| 12 | 5.81E-05 | 6.94E-03 | 4.04E-02 | 4.15E-15 |
| 13 | 1.93E-05 | 1.06E-02 | 5.77E-02 | 2.51E-16 |
| 14 | 5.33E-09 | 5.60E-04 | 8.61E-04 | 9.13E-23 |
| 15 | 1.62E-07 | 1.89E-03 | 7.13E-04 | 4.13E-22 |
| 16 | 1.55E-08 | 1.01E-03 | 1.04E-03 | 4.49E-22 |
| 17 | 5.31E-08 | 9.22E-03 | 1.04E-02 | 1.03E-21 |
| 18 | 1.37E-08 | 1.77E-03 | 7.24E-03 | 1.52E-21 |
| 19 | 3.31E-08 | 1.89E-03 | 6.74E-03 | 5.26E-21 |
| 20 | 5.79E-05 | 2.43E-03 | 5.85E-02 | 1.07E-14 |
| 21 | 8.87E-03 | 1.21E-02 | 2.44E-01 | 2.97E-08 |
| 22 | 1.76E-02 | 1.63E-02 | 2.41E-01 | 7.76E-08 |
| 23 | 2.82E-06 | 7.01E-02 | 5.22E-02 | 2.31E-14 |
| 24 | 3.31E-04 | 2.06E-02 | 1.04E-02 | 3.85E-13 |
| 25 | 1.65E-04 | 1.14E-01 | 1.77E-03 | 5.27E-08 |
| 26 | 1.92E-04 | 5.27E-02 | 1.45E-03 | 1.24E-08 |
| 27 | 1.84E-09 | 3.12E-02 | 9.47E-02 | 3.86E-04 |
| 28 | 3.38E-09 | 2.11E-01 | 1.66E-01 | 6.74E-04 |
| 29 | 5.84E-09 | 6.96E-02 | 2.65E-01 | 9.71E-04 |
| 30 | 8.81E-09 | 1.75E-01 | 1.27E-01 | 2.57E-04 |
| 31 | 1.29E-08 | 8.58E-02 | 3.28E-01 | 1.48E-03 |
| 32 | 1.29E-08 | 8.58E-02 | 3.28E-01 | 1.48E-03 |
| 33 | 1.29E-08 | 8.58E-02 | 3.28E-01 | 1.48E-03 |
| 34 | 1.29E-08 | 8.58E-02 | 3.28E-01 | 1.48E-03 |
| 35 | 1.29E-08 | 8.58E-02 | 3.28E-01 | 1.48E-03 |
| 36 | 1.29E-08 | 8.58E-02 | 3.28E-01 | 1.48E-03 |
| 37 | 2.23E-08 | 8.52E-02 | 3.28E-01 | 1.51E-03 |

(continued)

| No. | CPE_pv_adj | CRM_pv_adj | ETP_pv_adj | GM_pv_adj |
|---|---|---|---|---|
| 38 | 2.39E-08 | 4.38E-01 | 5.64E-02 | 4.16E-03 |
| 39 | 2.39E-08 | 4.38E-01 | 5.64E-02 | 4.16E-03 |
| 40 | 1.69E-01 | 6.82E-04 | 7.60E-01 | 2.98E-04 |
| 41 | 3.02E-08 | 3.74E-02 | 3.81E-04 | 1.38E-04 |
| 42 | 4.40E-08 | 3.77E-01 | 5.72E-02 | 4.38E-03 |
| 43 | 4.44E-08 | 6.75E-02 | 3.31E-01 | 2.17E-03 |
| 44 | 2.31E-02 | 4.84E-02 | 6.10E-02 | 1.15E-06 |
| 45 | 5.15E-08 | 8.55E-02 | 2.60E-01 | 3.19E-03 |
| 46 | 5.15E-08 | 7.80E-02 | 4.73E-01 | 1.13E-03 |
| 47 | 5.31E-08 | 1.08E-01 | 2.67E-01 | 2.37E-03 |
| 48 | 5.31E-08 | 1.08E-01 | 2.67E-01 | 2.37E-03 |
| 49 | 5.31E-08 | 1.08E-01 | 2.67E-01 | 2.37E-03 |
| 50 | 5.31E-08 | 1.08E-01 | 2.67E-01 | 2.37E-03 |

Table 5f: Data for Enterobacter, particularly Enterobacter cloacae (continued)

| No. | LVX_pv_adj | P/T_pv_adj | TO_pv_adj | T/S_pv_adj |
|---|---|---|---|---|
| 1 | 8.81E-09 | 1.52E-04 | 2.06E-19 | 5.49E-27 |
| 2 | 2.74E-08 | 6.34E-03 | 2.69E-20 | 5.83E-25 |
| 3 | 2.74E-08 | 6.34E-03 | 2.69E-20 | 5.83E-25 |
| 4 | 2.74E-08 | 6.34E-03 | 2.69E-20 | 5.83E-25 |
| 5 | 5.07E-07 | 6.25E-03 | 9.93E-19 | 1.25E-24 |
| 6 | 1.21E-11 | 4.05E-05 | 5.70E-21 | 2.32E-24 |
| 7 | 5.15E-08 | 1.58E-02 | 1.59E-19 | 2.46E-24 |
| 8 | 5.15E-08 | 1.58E-02 | 1.59E-19 | 2.46E-24 |
| 9 | 7.06E-08 | 1.10E-02 | 1.73E-19 | 2.46E-24 |
| 10 | 3.37E-07 | 8.08E-03 | 1.06E-18 | 1.59E-23 |
| 11 | 4.09E-08 | 2.07E-02 | 9.23E-20 | 1.59E-23 |
| 12 | 3.72E-07 | 1.10E-02 | 1.95E-18 | 2.83E-23 |
| 13 | 5.15E-08 | 1.58E-02 | 1.59E-19 | 2.83E-23 |
| 14 | 1.31E-11 | 2.13E-07 | 5.43E-22 | 1.85E-22 |
| 15 | 4.19E-12 | 8.40E-07 | 8.04E-22 | 2.17E-22 |
| 16 | 5.65E-12 | 5.70E-07 | 2.63E-21 | 1.03E-21 |
| 17 | 1.00E-08 | 4.60E-04 | 1.44E-21 | 4.11E-18 |
| 18 | 2.49E-09 | 1.68E-04 | 1.49E-21 | 4.99E-18 |
| 19 | 4.19E-12 | 8.40E-07 | 7.92E-21 | 2.34E-21 |
| 20 | 6.44E-09 | 2.28E-04 | 7.17E-16 | 1.02E-15 |
| 21 | 8.28E-05 | 6.44E-04 | 3.80E-10 | 1.05E-15 |

(continued)

| No. | LVX_pv_adj | P/T_pv_adj | TO_pv_adj | T/S_pv_adj |
|-----|-----------|-----------|-----------|-----------|
| 22 | 1.82E-04 | 9.24E-04 | 1.96E-09 | 4.35E-15 |
| 23 | 1.55E-05 | 1.40E-04 | 3.69E-14 | 6.50E-11 |
| 24 | 7.82E-07 | 1.61E-04 | 8.30E-12 | 1.21E-11 |
| 25 | 3.34E-06 | 3.34E-03 | 5.47E-11 | 4.91E-08 |
| 26 | 6.31E-06 | 1.70E-02 | 9.07E-10 | 5.12E-08 |
| 27 | 2.79E-05 | 2.12E-03 | 1.68E-04 | 4.44E-04 |
| 28 | 2.35E-03 | 8.21E-03 | 2.41E-03 | 3.78E-02 |
| 29 | 1.41E-03 | 2.25E-03 | 9.05E-04 | 1.74E-03 |
| 30 | 1.97E-05 | 1.77E-03 | 8.04E-05 | 5.50E-04 |
| 31 | 2.08E-03 | 5.50E-03 | 1.36E-03 | 3.40E-03 |
| 32 | 2.08E-03 | 5.50E-03 | 1.36E-03 | 3.40E-03 |
| 33 | 2.08E-03 | 5.50E-03 | 1.36E-03 | 3.40E-03 |
| 34 | 2.08E-03 | 5.50E-03 | 1.36E-03 | 3.40E-03 |
| 35 | 2.08E-03 | 5.50E-03 | 1.36E-03 | 3.40E-03 |
| 36 | 2.08E-03 | 5.50E-03 | 1.36E-03 | 3.40E-03 |
| 37 | 2.10E-03 | 7.39E-03 | 1.42E-03 | 3.42E-03 |
| 38 | 1.40E-06 | 4.00E-02 | 2.39E-03 | 8.45E-04 |
| 39 | 5.74E-06 | 7.03E-02 | 2.39E-03 | 8.45E-04 |
| 40 | 1.29E-02 | 8.60E-03 | 1.87E-05 | 2.77E-08 |
| 41 | 6.73E-06 | 8.68E-06 | 4.49E-05 | 2.53E-04 |
| 42 | 1.52E-06 | 4.87E-02 | 2.44E-03 | 8.72E-04 |
| 43 | 3.07E-03 | 5.47E-03 | 1.99E-03 | 2.25E-03 |
| 44 | 1.45E-04 | 6.88E-03 | 4.69E-06 | 5.12E-08 |
| 45 | 3.22E-03 | 9.52E-03 | 2.86E-03 | 3.22E-03 |
| 46 | 6.11E-05 | 1.82E-01 | 2.53E-04 | 3.05E-04 |
| 47 | 3.31E-03 | 5.73E-03 | 2.11E-03 | 5.16E-03 |
| 48 | 3.31E-03 | 5.73E-03 | 2.11E-03 | 5.16E-03 |
| 49 | 3.31E-03 | 5.73E-03 | 2.11E-03 | 5.16E-03 |
| 50 | 3.31E-03 | 5.73E-03 | 2.11E-03 | 5.16E-03 |

Table 5g: Data for Enterobacter, particularly Enterobacter cloacae (continued)

| No. | Locus.Tag | Gene. Symbol | GenBank.Accession | Chromosome |
|-----|-----------|--------------|-------------------|------------|
| 1 | ETAE_p041 | folP | YP_003297635 | plasmid pEIB202 |
| 2 | ECL_A197 | | YP_003602692 | plasmid pECL_A |
| 3 | APECO1_O1R60 | terY1 | YP_001481413 | plasmid pAPEC-O1-R |
| 4 | APECO1_O1R58 | terY2 | YP_001481411 | plasmid pAPEC-O1-R |
| 5 | ECL_A215 | | YP_003602710 | plasmid pECL_A |

(continued)

| No. | Locus.Tag | Gene. Symbol | GenBank.Accession | Chromosome |
|---|---|---|---|---|
| 6 | ECL_A245 | | YP_003602738 | plasmid pECL_A |
| 7 | ECL_A211 | | YP_003602706 | plasmid pECL_A |
| 8 | ECL_A213 | | YP_003602708 | plasmid pECL_A |
| 9 | CSAG_04328 | | ZP_04559059 | |
| 10 | CSAG_04340 | | ZP_04559071 | |
| 11 | APECO1_O1R72 | terD | YP_001481425 | plasmid pAPEC-O1-R |
| 12 | ESA_01791 | | YP_001437881 | |
| 13 | ECL_A210 | | YP_003602705 | plasmid pECL_A |
| 14 | HMPREF9551_04611 | | ZP_07191964 | |
| 15 | ECL_03822 | merP | YP_003614305 | |
| 16 | Salmoentericaenterica_ 010100000105 | | ZP_02702049 | |
| 17 | S2707 | parA | NP_838057 | |
| 18 | ECL_03815 | | YP_003614298 | |
| 19 | HMPREF9538_01300 | | ZP_08303641 | |
| 20 | ECL_00479 | | YP_003610994 | |
| 21 | SFxv_1737 | | ADA73942 | |
| 22 | EcDH1_ 2078 | | ACX39734 | |
| 23 | HMPREF9347_01086 | | ZP_07208640 | |
| 24 | HMPREF9530_03894 | | ZP_07153755 | |
| 25 | ECL_00391 | | YP_003610906 | |
| 26 | EcolE2_01002790 | | ZP_00728858 | |
| 27 | ENC_13410 | | CBK85143 | |
| 28 | ENC_03420 | | CBK84384 | |
| 29 | ENC_31170 | | CBK86512 | |
| 30 | HMPREF9086_ 2435 | | ZP_08498173 | |
| 31 | ENC_11900 | | CBK85029 | |
| 32 | ENC_02650 | | CBK84321 | |
| 33 | ENC_02700 | | CBK84325 | |
| 34 | HMPREF9086_4455 | amsH2 | ZP_08500191 | |
| 35 | HMPREF9086_ 4454 | yccY2 | ZP_08500190 | |
| 36 | HMPREF9086_2100 | yjdF | ZP_08497838 | |
| 37 | HMPREF9086_ 4460 | ligA2 | ZP_08500196 | |
| 38 | ENC_28850 | | CBK86326 | |
| 39 | HMPREF9086_3524 | gatAX | ZP_08499260 | |
| 40 | Entcl_2219 | | YP_003941757 | |
| 41 | Entcl_2207 | | YP_003941745 | |
| 42 | ENTCAN_03923 | | ZP_03284103 | |
| 43 | ECL_01132 | | YP_003611642 | |

(continued)

| No. | Locus.Tag | Gene. Symbol | GenBank.Accession | Chromosome |
|---|---|---|---|---|
| 44 | CSAG_04209 | | ZP_04558940 | |
| 45 | Entcl_0777 | | YP_003940336 | |
| 46 | HMPREF9086_0855 | | ZP_08496597 | |
| 47 | ENC_21030 | | CBK85719 | |
| 48 | ENC_20990 | | CBK85716 | |
| 49 | ENC_21000 | | CBK85717 | |
| 50 | ENC_21040 | | CBK85720 | |

Table 5h: Data for Enterobacter, particularly Enterobacter cloacae (continued)

| No. | Start.Coord | End.Coord | Strand | Scaffold.ID |
|---|---|---|---|---|
| 1 | 32101 | 32916 | - | 646311959 |
| 2 | 142295 | 142936 | - | 646564617 |
| 3 | 68792 | 69511 | - | 640753093 |
| 4 | 67490 | 68131 | - | 640753093 |
| 5 | 156077 | 157318 | + | 646564617 |
| 6 | 176747 | 177670 | - | 646564617 |
| 7 | 153338 | 154378 | + | 646564617 |
| 8 | 155073 | 155648 | + | 646564617 |
| 9 | 377133 | 378173 | - | 646206736 |
| 10 | 388284 | 389441 | + | 646206736 |
| 11 | 80263 | 80841 | + | 640753093 |
| 12 | 1731242 | 1732198 | - | 640753067 |
| 13 | 152860 | 153315 | + | 646564617 |
| 14 | 97 | 531 | - | 648293053 |
| 15 | 3908948 | 3909223 | + | 646564624 |
| 16 | 16271 | 16636 | - | 641778223 |
| 17 | 2602155 | 2602892 | - | 637000219 |
| 18 | 3903410 | 3903712 | + | 646564624 |
| 19 | 6404 | 6640 | + | 651333085 |
| 20 | 482851 | 484329 | - | 646564624 |
| 21 | 1636971 | 1637258 | - | 646862363 |
| 22 | 2232606 | 2232845 | + | 646862383 |
| 23 | 2726 | 5692 | - | 648294279 |
| 24 | 1918 | 3231 | - | 648290583 |
| 25 | 409807 | 410283 | + | 646564624 |
| 26 | 14395 | 15375 | + | 638358747 |
| 27 | 1397946 | 1398188 | - | 650378029 |

(continued)

| No. | Start.Coord | End.Coord | Strand | Scaffold.ID |
|-----|-------------|-----------|--------|-------------|
| 28 | 360699 | 361856 | + | 650378029 |
| 29 | 3151035 | 3151685 | - | 650378029 |
| 30 | 192614 | 193519 | + | 651335147 |
| 31 | 1250898 | 1252163 | + | 650378029 |
| 32 | 274155 | 276332 | - | 650378029 |
| 33 | 280790 | 281458 | - | 650378029 |
| 34 | 111547 | 112680 | - | 651335177 |
| 35 | 111116 | 111559 | - | 651335177 |
| 36 | 33364 | 34074 | + | 651335138 |
| 37 | 117314 | 119053 | - | 651335177 |
| 38 | 2904606 | 2905577 | - | 650378029 |
| 39 | 8440 | 9756 | - | 651335166 |
| 40 | 2349542 | 2349967 | + | 649633020 |
| 41 | 2334593 | 2335594 | - | 649633020 |
| 42 | 28272 | 29567 | - | 643006662 |
| 43 | 1168855 | 1169388 | + | 646564624 |
| 44 | 254005 | 255187 | - | 646206736 |
| 45 | 812618 | 813094 | + | 649633020 |
| 46 | 13947 | 15560 | - | 651335119 |
| 47 | 2148896 | 2151391 | + | 650378029 |
| 48 | 2145171 | 2145458 | + | 650378029 |
| 49 | 2145486 | 2146736 | + | 650378029 |
| 50 | 2151395 | 2152267 | - | 650378029 |

Table 6a: Data for Enterobacter, particularly Enterobacter cloacae

| No. | Scaffold.External. Accession | Scaffold.Name | best_pv |
|-----|------------------------------|---------------|---------|
| 1 | NC_013509 | Edwardsiella tarda EIB202 plasmid pEIB202: NC_013509 | 5.49490970435242e-27 |
| 2 | NC_014107 | Enterobacter cloacae subsp. cloacae ATCC 13047 plasmid pECL_A: NC_014107 | 5.83313344212793e-25 |
| 3 | NC_009838 | Escherichia coli APEC O1 plasmid pAPEC-O1-R: NC_009838 | 5.83313344212793e-25 |
| 4 | NC_009838 | Escherichia coli APEC O1 plasmid pAPEC-O1-R: NC_009838 | 5.83313344212793e-25 |
| 5 | NC_014107 | Enterobacter cloacae subsp. cloacae ATCC 13047 plasmid pECL_A: NC_014107 | 1.25162242820201e-24 |
| 6 | NC_014107 | Enterobacter cloacae subsp. cloacae ATCC 13047 plasmid pECL_A: NC_014107 | 2.31689992231266e-24 |
| 7 | NC_014107 | Enterobacter cloacae subsp. cloacae ATCC 13047 plasmid pECL_A: NC_014107 | 2.46438038993034e-24 |

(continued)

| No. | Scaffold.External. Accession | Scaffold.Name | best_pv |
|---|---|---|---|
| 8 | NC_014107 | Enterobacter cloacae subsp. cloacae ATCC 13047 plasmid pECL_A: NC_014107 | 2.46438038993034e-24 |
| 9 | NZ_GG657370 | Citrobacter sp. 30_2 genomic scaffold supercont1.5: NZ_GG657370 | 2.46438038993034e-24 |
| 10 | NZ_GG657370 | Citrobacter sp. 30_2 genomic scaffold supercont1.5: NZ_GG657370 | 1.59357817134779e-23 |
| 11 | NC_009838 | Escherichia coli APEC O1 plasmid pAPEC-O1-R: NC_009838 | 1.59357817134779e-23 |
| 12 | NC_009778 | Enterobacter sakazakii ATCC BAA-894: NC_009778 | 2.83245228449161e-23 |
| 13 | NC_014107 | Enterobacter cloacae subsp. cloacae ATCC 13047 plasmid pECL_A: NC_014107 | 2.83245228449161e-23 |
| 14 | NZ_ADUD01000537 | Escherichia coli MS 196-1 E_coli196-1-1.0_Cont1482.1: NZ_ADUD01000537 | 9.1281268279348e-23 |
| 15 | NC_014121 | Enterobacter cloacae subsp. cloacae ATCC 13047 chromosome: NC_014121 | 2.16502306009163e-22 |
| 16 | NZ_ABFH01000001 | Salmonella enterica subsp. enterica serovar Virchow str. SL491, unfinished sequence: NZ_ABFH01000001 | 4.49434330590708e-22 |
| 17 | NC_004741 | Shigella flexneri 2a str. 2457T: NC_004741 | 1.02679325501045e-21 |
| 18 | NC_014121 | Enterobacter cloacae subsp. cloacae ATCC 13047 chromosome: NC_014121 | 1.4942758061424e-21 |
| 19 | NZ_AFB001000168 | Klebsiella sp. MS 92-3 K_spMS92-3-1.0_Cont398.10: NZ_AFB001000168 | 2.33876952186319e-21 |
| 20 | NC_014121 | Enterobacter cloacae subsp. cloacae ATCC 13047 chromosome: NC_014121 | 7.16913512003583e-16 |
| 21 | CP001383 | Shigella flexneri 2002017: CP001383 | 1.0532279235518e-15 |
| 22 | CP001637 | Escherichia coli DH1: CP001637 | 4.34714801916322e-15 |
| 23 | NZ_ADWT01000006 | Escherichia coli MS 124-1 E_coliMS124-1-1.0.1_Cont5.1: NZ_ADWT01000006 | 2.30933700130366e-14 |
| 24 | NZ_ADTR01000359 | Escherichia coli MS 21-1 E_coli21-1-1.0_Cont669.1: NZ_ADTR01000359 | 3.84987338572512e-13 |
| 25 | NC_014121 | Enterobacter cloacae subsp. cloacae ATCC 13047 chromosome: NC_014121 | 5.47488441522503e-11 |
| 26 | NZ_AAJV01000019 | Escherichia coli E22, unfinished sequence: NZ_AAJV01000019 | 9.06569667406974e-10 |
| 27 | FP929040 | Enterobacter cloacae subsp. cloacae NCTC 9394 draft genome.: FP929040 | 1.83557061686134e-09 |
| 28 | FP929040 | Enterobacter cloacae subsp. cloacae NCTC 9394 draft genome.: FP929040 | 3.37768924887661e-09 |
| 29 | FP929040 | Enterobacter cloacae subsp. cloacae NCTC 9394 draft genome.: FP929040 | 5.84222468117604e-09 |
| 30 | NZ_AFHR01000043 | Enterobacter hormaechei ATCC 49162 contig00043: NZ_AFHR01000043 | 8.80996353751187e-09 |

(continued)

| No. | Scaffold.External. Accession | Scaffold.Name | best_pv |
|---|---|---|---|
| 31 | FP929040 | Enterobacter cloacae subsp. cloacae NCTC 9394 draft genome.: FP929040 | 1.29388450812614e-08 |
| 32 | FP929040 | Enterobacter cloacae subsp. cloacae NCTC 9394 draft genome.: FP929040 | 1.29388450812614e-08 |
| 33 | FP929040 | Enterobacter cloacae subsp. cloacae NCTC 9394 draft genome.: FP929040 | 1.29388450812614e-08 |
| 34 | NZ_AFHR01000073 | Enterobacter hormaechei ATCC 49162 contig00073: NZ_AFHR01000073 | 1.29388450812614e-08 |
| 35 | NZ_AFHR01000073 | Enterobacter hormaechei ATCC 49162 contig00073: NZ_AFHR01000073 | 1.29388450812614e-08 |
| 36 | NZ_AFHR01000034 | Enterobacter hormaechei ATCC 49162 contig00034: NZ_AFHR01000034 | 1.29388450812614e-08 |
| 37 | NZ_AFHR01000073 | Enterobacter hormaechei ATCC 49162 contig00073: NZ_AFHR01000073 | 2.23051874499664e-08 |
| 38 | FP929040 | Enterobacter cloacae subsp. cloacae NCTC 9394 draft genome.: FP929040 | 2.39066831640169e-08 |
| 39 | NZ_AFHR01000062 | Enterobacter hormaechei ATCC 49162 contig00062: NZ_AFHR01000062 | 2.39066831640169e-08 |
| 40 | NC_014618 | Enterobacter cloacae SCF1 chromosome: NC_014618 | 2.76892470483105e-08 |
| 41 | NC_014618 | Enterobacter cloacae SCF1 chromosome: NC_014618 | 3.01843352491008e-08 |
| 42 | NZ_ABWM01000064 | Enterobacter cancerogenus ATCC 35316, unfinished sequence: NZ_ABWM01000064 | 4.40355882800235e-08 |
| 43 | NC_014121 | Enterobacter cloacae subsp. cloacae ATCC 13047 chromosome: NC_014121 | 4.44394973624704e-08 |
| 44 | NZ_GG657370 | Citrobacter sp. 30_2 genomic scaffold supercont1.5: NZ_GG657370 | 5.11952538533182e-08 |
| 45 | NC_014618 | Enterobacter cloacae SCF1 chromosome: NC_014618 | 5.14762193286233e-08 |
| 46 | NZ_AFHR01000015 | Enterobacter hormaechei ATCC 49162 contig00015: NZ_AFHR01000015 | 5.14762193286233e-08 |
| 47 | FP929040 | Enterobacter cloacae subsp. cloacae NCTC 9394 draft genome.: FP929040 | 5.31046635969439e-08 |
| 48 | FP929040 | Enterobacter cloacae subsp. cloacae NCTC 9394 draft genome.: FP929040 | 5.31046635969439e-08 |
| 49 | FP929040 | Enterobacter cloacae subsp. cloacae NCTC 9394 draft genome.: FP929040 | 5.31046635969439e-08 |
| 50 | FP929040 | Enterobacter cloacae subsp. cloacae NCTC 9394 draft genome.: FP929040 | 5.31046635969439e-08 |

Table 6b: Data for Enterobacter, particularly Enterobacter cloacae (continued)

| No. | sign_phenos | sign_phenos_class | best_pheno | best_pheno_class |
|---|---|---|---|---|
| 1 | AZT;CAX;CAZ;CFT;CP;CPE; CRM;ETP;GM;LVX;P/T;TO;T/S | aminoglycoside;fluoroquinolone; lactam; other | T/S | other |

(continued)

| No. | sign_phenos | sign_phenos_class | best_pheno | best_pheno_ class |
|---|---|---|---|---|
| 2 | AZT;CAX;CAZ;CFT;CP;CPE; CRM;GM;LVX;P/T;TO;T/S | aminoglycoside;fluoroquinolone; lactam; other | T/S | other |
| 3 | AZT;CAX;CAZ;CFT;CP;CPE; CRM;GM;LVX;P/T;TO;T/S | aminoglycoside;fluoroquinolone; lactam; other | T/S | other |
| 4 | AZT;CAX;CAZ;CFT;CP;CPE; CRM;GM;LVX;P/T;TO;T/S | aminoglycoside;fluoroquinolone; lactam; other | T/S | other |
| 5 | AZT;CAX;CAZ;CFT;CP;CPE; CRM;GM;LVX;P/T;TO;T/S | aminoglycoside;fluoroquinolone; lactam; other | T/S | other |
| 6 | AZT;CAX;CAZ;CFT;CP;CPE; ETP;GM;LVX;P/T;TO;T/S | aminoglycoside;fluoroquinolone; lactam; other | T/S | other |
| 7 | AZT;CAX;CAZ;CFT;CP;CPE; GM;LVX;TO;T/S | aminoglycoside;fluoroquinolone; lactam; other | T/S | other |
| 8 | AZT;CAX;CAZ;CFT;CP;CPE; GM;LVX;TO;T/S | aminoglycoside;fluoroquinolone; lactam; other | T/S | other |
| 9 | AZT;CAX;CAZ;CFT;CP;CPE; CRM;GM;LVX;TO;T/S | aminoglycoside;fluoroquinolone; lactam; other | T/S | other |
| 10 | AZT;CAX;CAZ;CFT;CP;CPE; CRM;GM;LVX;P/T;TO;T/S | aminoglycoside;fluoroquinolone; lactam; other | T/S | other |
| 11 | AZT;CAX;CAZ;CFT;CP;CPE; GM;LVX;TO;T/S | aminoglycoside;fluoroquinolone; lactam; other | T/S | other |
| 12 | AZT;CAX;CAZ;CFT;CP;CPE; CRM;GM;LVX;TO;T/S | aminoglycoside;fluoroquinolone; lactam; other | T/S | other |
| 13 | AZT;CAX;CAZ;CFT;CP;CPE; GM;LVX;TO;T/S | aminoglycoside;fluoroquinolone; lactam; other | T/S | other |
| 14 | AZT;CAX;CAZ;CFT;CP;CPE; CRM;ETP;GM;LVX;P/T;TO;T/S | aminoglycoside;fluoroquinolone; lactam; other | GM | aminoglycosid e |
| 15 | AZT;CAX;CAZ;CFT;CP;CPE; CRM;ETP;GM;LVX;P/T;TO;T/S | aminoglycoside;fluoroquinolone; lactam; other | T/S | other |
| 16 | AZT;CAX;CAZ;CFT;CP;CPE; CRM;ETP;GM;LVX;P/T;TO;T/S | aminoglycoside;fluoroquinolone; lactam; other | GM | aminoglycosid e |
| 17 | AZT;CAX;CAZ;CFT;CP;CPE; CRM;GM;LVX;P/T;TO;T/S | aminoglycoside;fluoroquinolone; lactam; other | GM | aminoglycosid e |
| 18 | AZT;CAX;CAZ;CFT;CP;CPE; CRM;ETP;GM;LVX;P/T;TO;T/S | aminoglycoside;fluoroquinolone; lactam; other | TO | aminoglycosid e |
| 19 | AZT;CAX;CAZ;CFT;CP;CPE; CRM;ETP;GM;LVX;P/T;TO;T/S | aminoglycoside;fluoroquinolone; lactam; other | T/S | other |
| 20 | AZT;CAX;CAZ;CFT;CP;CPE; CRM;GM;LVX;P/T;TO;T/S | aminoglycoside;fluoroquinolone; lactam; other | TO | aminoglycosid e |
| 21 | AZT;CAX;CAZ;CFT;CP;CPE; GM;LVX;P/T;TO;T/S | aminoglycoside;fluoroquinolone; lactam; other | T/S | other |
| 22 | AZT;CAX;CAZ;CFT;CP;GM; LVX;P/T;TO;T/S | aminoglycoside;fluoroquinolone; lactam; other | T/S | other |
| 23 | AZT;CAX;CAZ;CFT;CP;CPE; GM;LVX;P/T;TO;T/S | aminoglycoside;fluoroquinolone; lactam; other | GM | aminoglycosid e |

(continued)

| No. | sign_phenos | sign_phenos_class | best_pheno | best_pheno_ class |
|-----|-------------|-------------------|------------|-------------------|
| 24 | AZT;CAX;CAZ;CFT;CP;CPE; GM;LVX;P/T;TO;T/S | aminoglycoside;fluoroquinolone; lactam; other | GM | aminoglycosid e |
| 25 | AZT;CAX;CAZ;CFT;CP;CPE; ETP;GM;LVX;P/T;TO;T/S | aminoglycoside;fluoroquinolone; lactam; other | TO | aminoglycosid e |
| 26 | AZT;CAX;CAZ;CFT;CP;CPE; ETP;GM;LVX;TO;T/S | aminoglycoside;fluoroquinolone; lactam; other | TO | aminoglycosid e |
| 27 | AZT;CAX;CAZ;CFT;CP;CPE; GM;LVX;P/T;TO;T/S | aminoglycoside;fluoroquinolone; lactam; other | CPE | lactam |
| 28 | AZT;CAX;CAZ;CFT;CPE;GM; LVX;P/T;TO | aminoglycoside;fluoroquinolone;lactam | CPE | lactam |
| 29 | AZT;CAX;CAZ;CFT;CPE;GM; LVX;P/T;TO;T/S | aminoglycoside;fluoroquinolone; lactam; other | CPE | lactam |
| 30 | AZT;CAZ;CFT;CP;CPE;GM; LVX;P/T;TO;T/S | aminoglycoside;fluoroquinolone; lactam; other | CPE | lactam |
| 31 | AZT;CAX;CAZ;CPE;GM;LVX; P/T;TO;T/S | aminoglycoside;fluoroquinolone; lactam; other | CPE | lactam |
| 32 | AZT;CAX;CAZ;CPE;GM;LVX; P/T;TO;T/S | aminoglycoside;fluoroquinolone; lactam; other | CPE | lactam |
| 33 | AZT;CAX;CAZ;CPE;GM;LVX; P/T;TO;T/S | aminoglycoside;fluoroquinolone; lactam; other | CPE | lactam |
| 34 | AZT;CAX;CAZ;CPE;GM;LVX; P/T;TO;T/S | aminoglycoside;fluoroquinolone; lactam; other | CPE | lactam |
| 35 | AZT;CAX;CAZ;CPE;GM;LVX; P/T;TO;T/S | aminoglycoside;fluoroquinolone; lactam; other | CPE | lactam |
| 36 | AZT;CAX;CAZ;CPE;GM;LVX; P/T;TO;T/S | aminoglycoside;fluoroquinolone; lactam; other | CPE | lactam |
| 37 | AZT;CAX;CAZ;CPE;GM;LVX; P/T;TO;T/S | aminoglycoside;fluoroquinolone; lactam; other | CPE | lactam |
| 38 | CP;CPE;GM;LVX;TO;T/S | aminoglycoside;fluoroquinolone; lactam; other | CPE | lactam |
| 39 | CP;CPE;GM;LVX;TO;T/S | aminoglycoside;fluoroquinolone; lactam; other | CPE | lactam |
| 40 | AZT;CAX;CAZ;CFT;CRM;GM; P/T;TO;T/S | aminoglycoside;lactam;other | T/S | other |
| 41 | AZT;CAX;CAZ;CFT;CP;CPE; ETP;GM;LVX;P/T;TO;T/S | aminoglycoside;fluoroquinolone; lactam; other | CPE | lactam |
| 42 | CP;CPE;GM;LVX;TO;T/S | aminoglycoside;fluoroquinolone; lactam; other | CPE | lactam |
| 43 | AZT;CAX;CAZ;CPE;GM;LVX; P/T;TO;T/S | aminoglycoside;fluoroquinolone; lactam; other | CPE | lactam |
| 44 | AZT;CAX;CAZ;CFT;CP;GM; LVX;P/T;TO;T/S | aminoglycoside;fluoroquinolone; lactam; other | T/S | other |

73

(continued)

| No. | sign_phenos | sign_phenos_class | best_ pheno | best_pheno_ class |
|---|---|---|---|---|
| 45 | CAX;CAZ;CPE;GM;LVX;P/T; TO;T/S | aminoglycoside;fluoroquinolone; lactam; other | CPE | lactam |
| 46 | CP;CPE;GM;LVX;TO;T/S | aminoglycoside;fluoroquinolone; lactam; other | CPE | lactam |
| 47 | AZT;CAX;CAZ;CPE;GM;LVX; P/T;TO;T/S | aminoglycoside;fluoroquinolone; lactam; other | CPE | lactam |
| 48 | AZT;CAX;CAZ;CPE;GM;LVX; P/T;TO;T/S | aminoglycoside;fluoroquinolone; lactam; other | CPE | lactam |
| 49 | AZT;CAX;CAZ;CPE;GM;LVX; P/T;TO;T/S | aminoglycoside;fluoroquinolone; lactam; other | CPE | lactam |
| 50 | AZT;CAX;CAZ;CPE;GM;LVX; P/T;TO;T/S | aminoglycoside;fluoroquinolone; lactam; other | CPE | lactam |

Table 6c: Data for Enterobacter, particularly Enterobacter cloacae (continued)

| No. | num_aminoglycoside | num_fluoroquinolone | num_lactam | num_other |
|---|---|---|---|---|
| 1 | 2 | 2 | 8 | 1 |
| 2 | 2 | 2 | 7 | 1 |
| 3 | 2 | 2 | 7 | 1 |
| 4 | 2 | 2 | 7 | 1 |
| 5 | 2 | 2 | 7 | 1 |
| 6 | 2 | 2 | 7 | 1 |
| 7 | 2 | 2 | 5 | 1 |
| 8 | 2 | 2 | 5 | 1 |
| 9 | 2 | 2 | 6 | 1 |
| 10 | 2 | 2 | 7 | 1 |
| 11 | 2 | 2 | 5 | 1 |
| 12 | 2 | 2 | 6 | 1 |
| 13 | 2 | 2 | 5 | 1 |
| 14 | 2 | 2 | 8 | 1 |
| 15 | 2 | 2 | 8 | 1 |
| 16 | 2 | 2 | 8 | 1 |
| 17 | 2 | 2 | 7 | 1 |
| 18 | 2 | 2 | 8 | 1 |
| 19 | 2 | 2 | 8 | 1 |
| 20 | 2 | 2 | 7 | 1 |
| 21 | 2 | 2 | 6 | 1 |
| 22 | 2 | 2 | 5 | 1 |
| 23 | 2 | 2 | 6 | 1 |
| 24 | 2 | 2 | 6 | 1 |

(continued)

| No. | num_aminoglycoside | num_fluoroquinolone | num_lactam | num_other |
|-----|--------------------|--------------------|------------|-----------|
| 25 | 2 | 2 | 7 | 1 |
| 26 | 2 | 2 | 6 | 1 |
| 27 | 2 | 2 | 6 | 1 |
| 28 | 2 | 1 | 6 | 0 |
| 29 | 2 | 1 | 6 | 1 |
| 30 | 2 | 2 | 5 | 1 |
| 31 | 2 | 1 | 5 | 1 |
| 32 | 2 | 1 | 5 | 1 |
| 33 | 2 | 1 | 5 | 1 |
| 34 | 2 | 1 | 5 | 1 |
| 35 | 2 | 1 | 5 | 1 |
| 36 | 2 | 1 | 5 | 1 |
| 37 | 2 | 1 | 5 | 1 |
| 38 | 2 | 2 | 1 | 1 |
| 39 | 2 | 2 | 1 | 1 |
| 40 | 2 | 0 | 6 | 1 |
| 41 | 2 | 2 | 7 | 1 |
| 42 | 2 | 2 | 1 | 1 |
| 43 | 2 | 1 | 5 | 1 |
| 44 | 2 | 2 | 5 | 1 |
| 45 | 2 | 1 | 4 | 1 |
| 46 | 2 | 2 | 1 | 1 |
| 47 | 2 | 1 | 5 | 1 |
| 48 | 2 | 1 | 5 | 1 |
| 49 | 2 | 1 | 5 | 1 |
| 50 | 2 | 1 | 5 | 1 |

Table 6d: Data for Enterobacter, particularly Enterobacter cloacae (continued)

| No. | AZT_pv_adj | CAX_pv_adj | CAZ_pv_adj | CFT_pv_adj | CP_pv_adj |
|-----|------------|------------|------------|------------|-----------|
| 1 | 3.58E-10 | 7.39E-09 | 2.80E-11 | 3.62E-09 | 5.27E-08 |
| 2 | 4.06E-06 | 7.65E-06 | 8.35E-07 | 8.00E-07 | 3.72E-07 |
| 3 | 4.06E-06 | 7.65E-06 | 8.35E-07 | 8.00E-07 | 3.72E-07 |
| 4 | 4.06E-06 | 7.65E-06 | 8.35E-07 | 8.00E-07 | 3.72E-07 |
| 5 | 8.70E-07 | 8.75E-06 | 5.21E-07 | 5.80E-07 | 1.18E-06 |
| 6 | 1.92E-12 | 2.08E-13 | 5.48E-12 | 1.92E-12 | 4.74E-09 |
| 7 | 2.20E-05 | 4.05E-05 | 4.94E-06 | 4.85E-06 | 7.75E-07 |
| 8 | 2.20E-05 | 4.05E-05 | 4.94E-06 | 4.85E-06 | 7.75E-07 |

(continued)

| No. | AZT_pv_adj | CAX_pv_adj | CAZ_pv_adj | CFT_pv_adj | CP_pv_adj |
|---|---|---|---|---|---|
| 9 | 6.62E-06 | 1.21E-05 | 1.34E-06 | 1.27E-06 | 3.72E-07 |
| 10 | 3.70E-06 | 6.73E-06 | 7.48E-07 | 7.08E-07 | 2.65E-06 |
| 11 | 3.46E-05 | 6.07E-05 | 7.99E-06 | 7.84E-06 | 7.16E-07 |
| 12 | 6.62E-06 | 1.21E-05 | 1.34E-06 | 1.27E-06 | 3.14E-06 |
| 13 | 2.20E-05 | 4.05E-05 | 4.94E-06 | 4.85E-06 | 7.75E-07 |
| 14 | 2.41E-09 | 3.66E-10 | 2.89E-10 | 1.33E-09 | 1.31E-11 |
| 15 | 6.79E-08 | 4.49E-08 | 1.13E-08 | 3.99E-08 | 4.19E-12 |
| 16 | 3.51E-09 | 1.67E-09 | 8.09E-10 | 8.35E-10 | 5.65E-12 |
| 17 | 3.11E-11 | 6.47E-11 | 7.01E-12 | 5.47E-11 | 1.53E-06 |
| 18 | 4.36E-12 | 6.39E-12 | 5.85E-13 | 5.48E-12 | 5.70E-07 |
| 19 | 6.79E-08 | 4.49E-08 | 1.13E-08 | 3.99E-08 | 3.61E-11 |
| 20 | 5.39E-09 | 1.23E-08 | 5.84E-09 | 6.70E-09 | 5.19E-07 |
| 21 | 3.36E-07 | 5.25E-07 | 5.19E-07 | 3.74E-06 | 3.91E-05 |
| 22 | 4.53E-07 | 8.07E-07 | 7.17E-07 | 6.01E-06 | 7.16E-05 |
| 23 | 1.62E-07 | 3.02E-07 | 2.83E-08 | 5.65E-08 | 9.46E-05 |
| 24 | 8.58E-09 | 1.75E-08 | 5.92E-08 | 2.01E-07 | 7.82E-07 |
| 25 | 5.28E-06 | 5.19E-07 | 1.32E-06 | 7.50E-07 | 1.27E-05 |
| 26 | 3.71E-05 | 2.06E-06 | 1.56E-05 | 9.73E-06 | 1.34E-04 |
| 27 | 2.41E-04 | 8.91E-05 | 8.91E-05 | 4.87E-04 | 5.72E-04 |
| 28 | 2.12E-03 | 7.75E-04 | 1.61E-03 | 5.63E-03 | 2.33E-02 |
| 29 | 3.60E-03 | 1.44E-03 | 1.46E-03 | 6.84E-03 | 1.58E-02 |
| 30 | 1.63E-03 | 1.30E-02 | 1.26E-03 | 9.88E-03 | 1.03E-03 |
| 31 | 8.73E-03 | 1.90E-03 | 3.60E-03 | 1.54E-02 | 2.18E-02 |
| 32 | 8.73E-03 | 1.90E-03 | 3.60E-03 | 1.54E-02 | 2.18E-02 |
| 33 | 8.73E-03 | 1.90E-03 | 3.60E-03 | 1.54E-02 | 2.18E-02 |
| 34 | 8.73E-03 | 1.90E-03 | 3.60E-03 | 1.54E-02 | 2.18E-02 |
| 35 | 8.73E-03 | 1.90E-03 | 3.60E-03 | 1.54E-02 | 2.18E-02 |
| 36 | 8.73E-03 | 1.90E-03 | 3.60E-03 | 1.54E-02 | 2.18E-02 |
| 37 | 9.06E-03 | 2.62E-03 | 4.92E-03 | 1.99E-02 | 2.22E-02 |
| 38 | 3.58E-02 | 1.04E-02 | 1.75E-02 | 5.31E-02 | 2.20E-05 |
| 39 | 5.33E-02 | 1.78E-02 | 2.85E-02 | 7.63E-02 | 6.85E-05 |
| 40 | 8.52E-05 | 3.90E-05 | 6.01E-06 | 2.49E-05 | 1.29E-02 |
| 41 | 4.11E-06 | 7.41E-04 | 4.26E-05 | 1.08E-03 | 6.16E-05 |
| 42 | 4.35E-02 | 1.36E-02 | 2.23E-02 | 6.48E-02 | 2.37E-05 |
| 43 | 8.79E-03 | 2.56E-03 | 4.80E-03 | 1.96E-02 | 2.95E-02 |
| 44 | 5.44E-03 | 1.03E-03 | 6.23E-04 | 1.01E-03 | 1.45E-04 |
| 45 | 1.49E-02 | 4.69E-03 | 8.56E-03 | 3.09E-02 | 3.86E-02 |
| 46 | 2.22E-01 | 6.69E-02 | 1.35E-01 | 3.01E-01 | 6.26E-04 |

(continued)

| No. | AZT_pv_adj | CAX_pv_adj | CAZ_pv_adj | CFT_pv_adj | CP_pv_adj |
|---|---|---|---|---|---|
| 47 | 8.87E-03 | 1.93E-03 | 3.69E-03 | 1.56E-02 | 3.05E-02 |
| 48 | 8.87E-03 | 1.93E-03 | 3.69E-03 | 1.56E-02 | 3.05E-02 |
| 49 | 8.87E-03 | 1.93E-03 | 3.69E-03 | 1.56E-02 | 3.05E-02 |
| 50 | 8.87E-03 | 1.93E-03 | 3.69E-03 | 1.56E-02 | 3.05E-02 |

Table 6e: Data for Enterobacter, particularly Enterobacter cloacae (continued)

| No. | CPE_pv_adj | CRM_pv_adj | ETP_pv_adj | GM_pv_adj |
|---|---|---|---|---|
| 1 | 2.76E-09 | 1.20E-03 | 1.29E-04 | 3.48E-19 |
| 2 | 2.95E-05 | 2.74E-03 | 5.44E-02 | 5.95E-17 |
| 3 | 2.95E-05 | 2.74E-03 | 5.44E-02 | 5.95E-17 |
| 4 | 2.95E-05 | 2.74E-03 | 5.44E-02 | 5.95E-17 |
| 5 | 5.40E-05 | 2.87E-03 | 9.20E-02 | 6.93E-16 |
| 6 | 3.27E-08 | 3.20E-02 | 6.76E-03 | 1.07E-19 |
| 7 | 6.14E-05 | 1.06E-02 | 5.77E-02 | 2.51E-16 |
| 8 | 6.14E-05 | 1.06E-02 | 5.77E-02 | 2.51E-16 |
| 9 | 5.81E-05 | 2.77E-03 | 7.62E-02 | 4.00E-16 |
| 10 | 5.02E-05 | 3.99E-03 | 3.88E-02 | 2.55E-15 |
| 11 | 5.72E-05 | 1.10E-02 | 5.64E-02 | 1.59E-16 |
| 12 | 5.81E-05 | 6.94E-03 | 4.04E-02 | 4.15E-15 |
| 13 | 1.93E-05 | 1.06E-02 | 5.77E-02 | 2.51E-16 |
| 14 | 5.33E-09 | 5.60E-04 | 8.61E-04 | 9.13E-23 |
| 15 | 1.62E-07 | 1.89E-03 | 7.13E-04 | 4.13E-22 |
| 16 | 1.55E-08 | 1.01E-03 | 1.04E-03 | 4.49E-22 |
| 17 | 5.31E-08 | 9.22E-03 | 1.04E-02 | 1.03E-21 |
| 18 | 1.37E-08 | 1.77E-03 | 7.24E-03 | 1.52E-21 |
| 19 | 3.31E-08 | 1.89E-03 | 6.74E-03 | 5.26E-21 |
| 20 | 5.79E-05 | 2.43E-03 | 5.85E-02 | 1.07E-14 |
| 21 | 8.87E-03 | 1.21E-02 | 2.44E-01 | 2.97E-08 |
| 22 | 1.76E-02 | 1.63E-02 | 2.41E-01 | 7.76E-08 |
| 23 | 2.82E-06 | 7.01E-02 | 5.22E-02 | 2.31E-14 |
| 24 | 3.31E-04 | 2.06E-02 | 1.04E-02 | 3.85E-13 |
| 25 | 1.65E-04 | 1.14E-01 | 1.77E-03 | 5.27E-08 |
| 26 | 1.92E-04 | 5.27E-02 | 1.45E-03 | 1.24E-08 |
| 27 | 1.84E-09 | 3.12E-02 | 9.47E-02 | 3.86E-04 |
| 28 | 3.38E-09 | 2.11E-01 | 1.66E-01 | 6.74E-04 |
| 29 | 5.84E-09 | 6.96E-02 | 2.65E-01 | 9.71E-04 |
| 30 | 8.81E-09 | 1.75E-01 | 1.27E-01 | 2.57E-04 |

(continued)

| No. | CPE_pv_adj | CRM_pv_adj | ETP_pv_adj | GM_pv_adj |
|-----|-----------|-----------|-----------|-----------|
| 31 | 1.29E-08 | 8.58E-02 | 3.28E-01 | 1.48E-03 |
| 32 | 1.29E-08 | 8.58E-02 | 3.28E-01 | 1.48E-03 |
| 33 | 1.29E-08 | 8.58E-02 | 3.28E-01 | 1.48E-03 |
| 34 | 1.29E-08 | 8.58E-02 | 3.28E-01 | 1.48E-03 |
| 35 | 1.29E-08 | 8.58E-02 | 3.28E-01 | 1.48E-03 |
| 36 | 1.29E-08 | 8.58E-02 | 3.28E-01 | 1.48E-03 |
| 37 | 2.23E-08 | 8.52E-02 | 3.28E-01 | 1.51E-03 |
| 38 | 2.39E-08 | 4.38E-01 | 5.64E-02 | 4.16E-03 |
| 39 | 2.39E-08 | 4.38E-01 | 5.64E-02 | 4.16E-03 |
| 40 | 1.69E-01 | 6.82E-04 | 7.60E-01 | 2.98E-04 |
| 41 | 3.02E-08 | 3.74E-02 | 3.81E-04 | 1.38E-04 |
| 42 | 4.40E-08 | 3.77E-01 | 5.72E-02 | 4.38E-03 |
| 43 | 4.44E-08 | 6.75E-02 | 3.31E-01 | 2.17E-03 |
| 44 | 2.31E-02 | 4.84E-02 | 6.10E-02 | 1.15E-06 |
| 45 | 5.15E-08 | 8.55E-02 | 2.60E-01 | 3.19E-03 |
| 46 | 5.15E-08 | 7.80E-02 | 4.73E-01 | 1.13E-03 |
| 47 | 5.31E-08 | 1.08E-01 | 2.67E-01 | 2.37E-03 |
| 48 | 5.31E-08 | 1.08E-01 | 2.67E-01 | 2.37E-03 |
| 49 | 5.31E-08 | 1.08E-01 | 2.67E-01 | 2.37E-03 |
| 50 | 5.31E-08 | 1.08E-01 | 2.67E-01 | 2.37E-03 |

Table 6f: Data for Enterobacter, particularly Enterobacter cloacae (continued)

| No. | LVX_pv_adj | P/T_pv_adj | TO_pv_adj | T/S_pv_adj |
|-----|-----------|-----------|-----------|-----------|
| 1 | 8.81E-09 | 1.52E-04 | 2.06E-19 | 5.49E-27 |
| 2 | 2.74E-08 | 6.34E-03 | 2.69E-20 | 5.83E-25 |
| 3 | 2.74E-08 | 6.34E-03 | 2.69E-20 | 5.83E-25 |
| 4 | 2.74E-08 | 6.34E-03 | 2.69E-20 | 5.83E-25 |
| 5 | 5.07E-07 | 6.25E-03 | 9.93E-19 | 1.25E-24 |
| 6 | 1.21E-11 | 4.05E-05 | 5.70E-21 | 2.32E-24 |
| 7 | 5.15E-08 | 1.58E-02 | 1.59E-19 | 2.46E-24 |
| 8 | 5.15E-08 | 1.58E-02 | 1.59E-19 | 2.46E-24 |
| 9 | 7.06E-08 | 1.10E-02 | 1.73E-19 | 2.46E-24 |
| 10 | 3.37E-07 | 8.08E-03 | 1.06E-18 | 1.59E-23 |
| 11 | 4.09E-08 | 2.07E-02 | 9.23E-20 | 1.59E-23 |
| 12 | 3.72E-07 | 1.10E-02 | 1.95E-18 | 2.83E-23 |
| 13 | 5.15E-08 | 1.58E-02 | 1.59E-19 | 2.83E-23 |
| 14 | 1.31E-11 | 2.13E-07 | 5.43E-22 | 1.85E-22 |

(continued)

| No. | LVX_pv_adj | P/T_pv_adj | TO_pv_adj | T/S_pv_adj |
|---|---|---|---|---|
| 15 | 4.19E-12 | 8.40E-07 | 8.04E-22 | 2.17E-22 |
| 16 | 5.65E-12 | 5.70E-07 | 2.63E-21 | 1.03E-21 |
| 17 | 1.00E-08 | 4.60E-04 | 1.44E-21 | 4.11E-18 |
| 18 | 2.49E-09 | 1.68E-04 | 1.49E-21 | 4.99E-18 |
| 19 | 4.19E-12 | 8.40E-07 | 7.92E-21 | 2.34E-21 |
| 20 | 6.44E-09 | 2.28E-04 | 7.17E-16 | 1.02E-15 |
| 21 | 8.28E-05 | 6.44E-04 | 3.80E-10 | 1.05E-15 |
| 22 | 1.82E-04 | 9.24E-04 | 1.96E-09 | 4.35E-15 |
| 23 | 1.55E-05 | 1.40E-04 | 3.69E-14 | 6.50E-11 |
| 24 | 7.82E-07 | 1.61E-04 | 8.30E-12 | 1.21E-11 |
| 25 | 3.34E-06 | 3.34E-03 | 5.47E-11 | 4.91E-08 |
| 26 | 6.31E-06 | 1.70E-02 | 9.07E-10 | 5.12E-08 |
| 27 | 2.79E-05 | 2.12E-03 | 1.68E-04 | 4.44E-04 |
| 28 | 2.35E-03 | 8.21E-03 | 2.41E-03 | 3.78E-02 |
| 29 | 1.41E-03 | 2.25E-03 | 9.05E-04 | 1.74E-03 |
| 30 | 1.97E-05 | 1.77E-03 | 8.04E-05 | 5.50E-04 |
| 31 | 2.08E-03 | 5.50E-03 | 1.36E-03 | 3.40E-03 |
| 32 | 2.08E-03 | 5.50E-03 | 1.36E-03 | 3.40E-03 |
| 33 | 2.08E-03 | 5.50E-03 | 1.36E-03 | 3.40E-03 |
| 34 | 2.08E-03 | 5.50E-03 | 1.36E-03 | 3.40E-03 |
| 35 | 2.08E-03 | 5.50E-03 | 1.36E-03 | 3.40E-03 |
| 36 | 2.08E-03 | 5.50E-03 | 1.36E-03 | 3.40E-03 |
| 37 | 2.10E-03 | 7.39E-03 | 1.42E-03 | 3.42E-03 |
| 38 | 1.40E-06 | 4.00E-02 | 2.39E-03 | 8.45E-04 |
| 39 | 5.74E-06 | 7.03E-02 | 2.39E-03 | 8.45E-04 |
| 40 | 1.29E-02 | 8.60E-03 | 1.87E-05 | 2.77E-08 |
| 41 | 6.73E-06 | 8.68E-06 | 4.49E-05 | 2.53E-04 |
| 42 | 1.52E-06 | 4.87E-02 | 2.44E-03 | 8.72E-04 |
| 43 | 3.07E-03 | 5.47E-03 | 1.99E-03 | 2.25E-03 |
| 44 | 1.45E-04 | 6.88E-03 | 4.69E-06 | 5.12E-08 |
| 45 | 3.22E-03 | 9.52E-03 | 2.86E-03 | 3.22E-03 |
| 46 | 6.11E-05 | 1.82E-01 | 2.53E-04 | 3.05E-04 |
| 47 | 3.31E-03 | 5.73E-03 | 2.11E-03 | 5.16E-03 |
| 48 | 3.31E-03 | 5.73E-03 | 2.11E-03 | 5.16E-03 |
| 49 | 3.31E-03 | 5.73E-03 | 2.11E-03 | 5.16E-03 |
| 50 | 3.31E-03 | 5.73E-03 | 2.11E-03 | 5.16E-03 |

Table 6g: Data for Enterobacter, particularly Enterobacter cloacae (continued)

| No. | Locus.Tag | Gene. Symbol | GenBank.Accession | Chromosome |
|---|---|---|---|---|
| 1 | ETAE_p041 | folP | YP_003297635 | plasmid pEIB202 |
| 2 | ECL_A197 | | YP_003602692 | plasmid pECL_A |
| 3 | APECO1_O1R60 | terY1 | YP_001481413 | plasmid pAPEC-O1-R |
| 4 | APECO1_O1R58 | terY2 | YP_001481411 | plasmid pAPEC-O1-R |
| 5 | ECL_A215 | | YP_003602710 | plasmid pECL_A |
| 6 | ECL_A245 | | YP_003602738 | plasmid pECL_A |
| 7 | ECL_A211 | | YP_003602706 | plasmid pECL_A |
| 8 | ECL_A213 | | YP_003602708 | plasmid pECL_A |
| 9 | CSAG_04328 | | ZP_04559059 | |
| 10 | CSAG_04340 | | ZP_04559071 | |
| 11 | APEC01_O1R72 | terD | YP_001481425 | plasmid pAPEC-O1-R |
| 12 | ESA_01791 | | YP_001437881 | |
| 13 | ECL_A210 | | YP_003602705 | plasmid pECL_A |
| 14 | HMPREF9551_04611 | | ZP_07191964 | |
| 15 | ECL_03822 | merP | YP_003614305 | |
| 16 | Salmoentericaenterica_010100000105 | | ZP_02702049 | |
| 17 | S2707 | parA | NP_838057 | |
| 18 | ECL_03815 | | YP_003614298 | |
| 19 | HMPREF9538_01300 | | ZP_08303641 | |
| 20 | ECL_00479 | | YP_003610994 | |
| 21 | SFxv_1737 | | ADA73942 | |
| 22 | EcDH1_2078 | | ACX39734 | |
| 23 | HMPREF9347_01086 | | ZP_07208640 | |
| 24 | HMPREF9530_03894 | | ZP_07153755 | |
| 25 | ECL_00391 | | YP_003610906 | |
| 26 | EcolE2_01002790 | | ZP_00728858 | |
| 27 | ENC_13410 | | CBK85143 | |
| 28 | ENC_03420 | | CBK84384 | |
| 29 | ENC_31170 | | CBK86512 | |
| 30 | HMPREF9086_2435 | | ZP_08498173 | |
| 31 | ENC_11900 | | CBK85029 | |
| 32 | ENC_02650 | | CBK84321 | |
| 33 | ENC_02700 | | CBK84325 | |
| 34 | HMPREF9086_4455 | amsH2 | ZP_08500191 | |
| 35 | HMPREF9086_4454 | yccY2 | ZP_08500190 | |
| 36 | HMPREF9086_2100 | yjdF | ZP_08497838 | |
| 37 | HMPREF9086_4460 | ligA2 | ZP_08500196 | |

(continued)

| No. | Locus.Tag | Gene. Symbol | GenBank.Accession | Chromosome |
|---|---|---|---|---|
| 38 | ENC_28850 | | CBK86326 | |
| 39 | HMPREF9086_3524 | gatAX | ZP_08499260 | |
| 40 | Entcl_2219 | | YP_003941757 | |
| 41 | Entcl_2207 | | YP_003941745 | |
| 42 | ENTCAN_03923 | | ZP_03284103 | |
| 43 | ECL_01132 | | YP_003611642 | |
| 44 | CSAG_04209 | | ZP_04558940 | |
| 45 | Entcl_0777 | | YP_003940336 | |
| 46 | HMPREF9086_0855 | | ZP_08496597 | |
| 47 | ENC_21030 | | CBK85719 | |
| 48 | ENC_20990 | | CBK85716 | |
| 49 | ENC_21000 | | CBK85717 | |
| 50 | ENC_21040 | | CBK85720 | |

Table 6h: Data for Enterobacter, particularly Enterobacter cloacae (continued)

| No. | Start.Coord | End.Coord | Strand | Scaffold.ID |
|---|---|---|---|---|
| 1 | 32101 | 32916 | - | 646311959 |
| 2 | 142295 | 142936 | - | 646564617 |
| 3 | 68792 | 69511 | - | 640753093 |
| 4 | 67490 | 68131 | - | 640753093 |
| 5 | 156077 | 157318 | + | 646564617 |
| 6 | 176747 | 177670 | - | 646564617 |
| 7 | 153338 | 154378 | + | 646564617 |
| 8 | 155073 | 155648 | + | 646564617 |
| 9 | 377133 | 378173 | - | 646206736 |
| 10 | 388284 | 389441 | + | 646206736 |
| 11 | 80263 | 80841 | + | 640753093 |
| 12 | 1731242 | 1732198 | - | 640753067 |
| 13 | 152860 | 153315 | + | 646564617 |
| 14 | 97 | 531 | - | 648293053 |
| 15 | 3908948 | 3909223 | + | 646564624 |
| 16 | 16271 | 16636 | - | 641778223 |
| 17 | 2602155 | 2602892 | - | 637000219 |
| 18 | 3903410 | 3903712 | + | 646564624 |
| 19 | 6404 | 6640 | + | 651333085 |
| 20 | 482851 | 484329 | - | 646564624 |
| 21 | 1636971 | 1637258 | - | 646862363 |

(continued)

| No. | Start.Coord | End.Coord | Strand | Scaffold.ID |
|-----|-------------|-----------|--------|-------------|
| 22 | 2232606 | 2232845 | + | 646862383 |
| 23 | 2726 | 5692 | - | 648294279 |
| 24 | 1918 | 3231 | - | 648290583 |
| 25 | 409807 | 410283 | + | 646564624 |
| 26 | 14395 | 15375 | + | 638358747 |
| 27 | 1397946 | 1398188 | - | 650378029 |
| 28 | 360699 | 361856 | + | 650378029 |
| 29 | 3151035 | 3151685 | - | 650378029 |
| 30 | 192614 | 193519 | + | 651335147 |
| 31 | 1250898 | 1252163 | + | 650378029 |
| 32 | 274155 | 276332 | - | 650378029 |
| 33 | 280790 | 281458 | - | 650378029 |
| 34 | 111547 | 112680 | - | 651335177 |
| 35 | 111116 | 111559 | - | 651335177 |
| 36 | 33364 | 34074 | + | 651335138 |
| 37 | 117314 | 119053 | - | 651335177 |
| 38 | 2904606 | 2905577 | - | 650378029 |
| 39 | 8440 | 9756 | - | 651335166 |
| 40 | 2349542 | 2349967 | + | 649633020 |
| 41 | 2334593 | 2335594 | - | 649633020 |
| 42 | 28272 | 29567 | - | 643006662 |
| 43 | 1168855 | 1169388 | + | 646564624 |
| 44 | 254005 | 255187 | - | 646206736 |
| 45 | 812618 | 813094 | + | 649633020 |
| 46 | 13947 | 15560 | - | 651335119 |
| 47 | 2148896 | 2151391 | + | 650378029 |
| 48 | 2145171 | 2145458 | + | 650378029 |
| 49 | 2145486 | 2146736 | + | 650378029 |
| 50 | 2151395 | 2152267 | - | 650378029 |

Example 4: Escherichia, particularly E. coli

The procedure was carried out as in Example 1, except that the following microorganisms were used:

Bacterial Strains

The inventors selected 1144 E.coli strains from the microbiology strain collection at Siemens Healthcare Diagnostics (West Sacramento, CA) for susceptibility testing and whole genome sequencing.

From MetaRef, 318 centroids of E.coli were used as reference sequences.

The results for E.coli are shown in Tables 7 (corresponding to Table 1) and 8 (corresponding to Table 2).

Table 7a: Data for Escherichia, particularly E.coli

| No. | Scaffold.External. Accession | Scaffold.Name | best_pv |
|---|---|---|---|
| 1 | NC_011083 | Salmonella enterica subsp. enterica serovar Heidelberg str. SL476: NC_011083 | 3.5041948925527e-207 |
| 2 | NZ_ AAJT01000012 | Escherichia coli B7A, unfinished sequence: NZ_ AAJT01000012 | 1.62252621621877e-119 |
| 3 | NZ_ ADWV01000045 | Escherichia coli MS 107-1 E_ coliMS107-1-1.0.1_Cont44.1: NZ_ADWV01000045 | 6.24750842070913e-97 |
| 4 | NC_009651 | Klebsiella pneumoniae subsp. pneumoniae MGH 78578 plasmid pKPN5: NC_009651 | 8.77735582031733e-96 |
| 5 | NZ_ ADUB01000261 | Escherichia coli MS 175-1 E_coli175-1-1.0_Cont542.3: NZ_ADUB01000261 | 1.23709746311649e-80 |
| 6 | NC_011092 | Salmonella enterica subsp. enterica serovar Schwarzengrund str. CVM19633 plasmid pCVM19633_110: NC_011092 | 2.41688550175243e-75 |
| 7 | NZ_ ADWV01000056 | Escherichia coli MS 107-1 E_ coliMS107-1-1.0.1_Cont55.1: NZ_ADWV01000056 | 7.68091736117399e-72 |
| 8 | NZ_ AAJX01000089 | Escherichia coli B171, unfinished sequence: NZ_ AAJX01000089 | 1.57772112100981e-71 |
| 9 | NC_011743 | Escherichia fergusonii ATCC 35469 plasmid pEFER: NC_011743 | 1.0118246517332e-69 |
| 10 | NC_013509 | Edwardsiella tarda EIB202 plasmid pEIB202: NC_ 013509 | 1.23255842888535e-68 |
| 11 | NZ_GG749218 | Escherichia coli FVEC1412 genomic scaffold supercont1.13: NZ_GG749218 | 6.77313303746994e-68 |
| 12 | NC_013509 | Edwardsiella tarda EIB202 plasmid pEIB202: NC_ 013509 | 1.94619564613577e-64 |
| 13 | NZ_GG749218 | Escherichia coli FVEC1412 genomic scaffold supercont1.13: NZ_GG749218 | 1.86737834552034e-63 |
| 14 | NC_013717 | Citrobacter rodentium ICC168 plasmid pCROD1: NC_ 013717 | 7.42780365363135e-62 |
| 15 | NC_013717 | Citrobacter rodentium ICC168 plasmid pCROD1: NC_ 013717 | 3.07751376659308e-61 |
| 16 | NZ_ ADWS01000089 | Escherichia coli MS 145-7 E_ coliMS145-7-1.0.1_Cont88.1: NZ_ADWS01000089 | 1.7351795845908e-60 |
| 17 | NC_011751 | Escherichia coli UMN026: NC_011751 | 2.44317906034667e-60 |
| 18 | NC_011751 | Escherichia coli UMN026: NC_011751 | 6.73588199342071e-60 |
| 19 | NZ_ ADWV01000056 | Escherichia coli MS 107-1 E_ coliMS107-1-1.0.1_Cont55.1: NZ_ADWV01000056 | 2.62943019143703e-59 |
| 20 | NC_010488 | Escherichia coli SECEC SMS-3-5 plasmid pSMS35_130: NC_010488 | 3.46702381924878e-47 |
| 21 | NZ_ ADTM01000311 | Escherichia coli MS 182-1 E_coli182-1-1.0_Cont460.3: NZ_ADTM01000311 | 4.65308035729244e-46 |

(continued)

| No. | Scaffold.External. Accession | Scaffold.Name | best_pv |
|---|---|---|---|
| 22 | NZ_ADWS01000095 | Escherichia coli MS 145-7 E_coliMS145-7-1.0.1_Cont94.1: NZ_ADWS01000095 | 7.71451120848483e-46 |
| 23 | NZ_ADTM01000311 | Escherichia coli MS 182-1 Ecoli182-1-1.0_Cont460.3: NZ_ADTM01000311 | 1.08551755499227e-44 |
| 24 | NC_009650 | Klebsiella pneumoniae subsp. pneumoniae MGH 78578 plasmid pKPN4: NC_009650 | 4.89761264283562e-44 |
| 25 | NZ_ADWV01000017 | Escherichia coli MS 107-1 E_coliMS107-1-1.0.1_Cont16.1: NZ_ADWV01000017 | 6.97693050178087e-44 |
| 26 | NC_009650 | Klebsiella pneumoniae subsp. pneumoniae MGH 78578 plasmid pKPN4: NC_009650 | 9.68294892967391e-44 |
| 27 | NZ_GG665811 | Escherichia sp. 1_1_43 genomic scaffold supercont1.1: NZ_GG665811 | 3.5843867496487e-43 |
| 28 | NZ_ADTL01000051 | Escherichia coli MS 115-1 E_coli115-1-1.0 Cont158.2: NZ_ADTL01000051 | 3.5843867496487e-43 |
| 29 | AP011957 | Salmonella enterica subsp. enterica serovar Typhimurium str. T000240 DNA: AP011957 | 5.53805522527039e-43 |
| 30 | NC_011282 | Klebsiella pneumoniae 342 plasmid pKP187: NC_011282 | 2.71226651272988e-42 |
| 31 | NZ_ABHR01000011 | Escherichia coli O157:H7 str. EC4401, unfinished sequence: NZ_ABHR01000011 | 8.45082785112866e-42 |
| 32 | NC_010488 | Escherichia coli SECEC SMS-3-5 plasmid pSMS35_130: NC_010488 | 1.02749291231853e-41 |
| 33 | NZ_AFBO01000013 | Klebsiella sp. MS 92-3 K_spMS92-3-1.0_Cont24.1: NZ_AFBO01000013 | 1.46909169086738e-41 |
| 34 | CP002797 | Escherichia coli NA114: CP002797 | 2.43588122712712e-41 |
| 35 | NZ_ABHK01000002 | Escherichia coli O157:H7 str. EC4206, unfinished sequence: NZ_ABHK01000002 | 2.60702932643171e-41 |
| 36 | NZ_GG657366 | Citrobacter sp. 30_2 genomic scaffold supercont1.1: NZ_GG657366 | 2.60702932643171e-41 |
| 37 | NC_011353 | Escherichia coli O157:H7 str. EC4115: NC_011353 | 2.60702932643171e-41 |
| 38 | NZ_ABWL01000012 | Citrobacter youngae ATCC 29220 C_sp-1.0_Cont2.3: NZ_ABWL01000012 | 3.21133372679738e-41 |
| 39 | AP009378 | Escherichia coli SE15 DNA: AP009378 | 3.68640118720819e-41 |
| 40 | NZ_ACXN01000074 | Escherichia coli O157:H7 str. FRIK966: NZ_ACXN01000074 | 5.75695041652081e-41 |
| 41 | NC_007384 | Shigella sonnei Ss046: NC_007384 | 6.75951367822551e-41 |
| 42 | NZ_ABWL01000012 | Citrobacter youngae ATCC 29220 C_sp-1.0_Cont2.3: NZ_ABWL01000012 | 8.12681193093067e-41 |
| 43 | NZ_ADTL01000083 | Escherichia coli MS 115-1 E_coli115-1-1.0_Cont264.1: NZ_ADTL01000083 | 8.12681193093067e-41 |
| 44 | AC_000091 | Escherichia coli W3110 DNA: AC_000091 | 8.12681193093067e-41 |

(continued)

| No. | Scaffold.External. Accession | Scaffold.Name | best_pv |
|---|---|---|---|
| 45 | NZ_ ADUD01000537 | Escherichia coli MS 196-1 E_coli196-1-1.0_Cont1482.1: NZ_ADUD01000537 | 1.06521634399457e-40 |
| 46 | NC_011083 | Salmonella enterica subsp. enterica serovar Heidelberg str. SL476: NC_011083 | 1.84998224240006e-40 |
| 47 | CP001383 | Shigella flexneri 2002017: CP001383 | 1.89942261631873e-40 |
| 48 | NZ_ ADTR01000134 | Escherichia coli MS 21-1 E_coli21-1-1.0_Cont296.1: NZ_ADTR01000134 | 3.35939290103889e-40 |
| 49 | NC_009650 | Klebsiella pneumoniae subsp. pneumoniae MGH 78578 plasmid pKPN4: NC_009650 | 3.76989142366575e-40 |
| 50 | NC_004851 | Shigella flexneri 2a str. 301 plasmid pCP301: NC_004851 | 8.68449218790606e-39 |

Table 7b: Data for Escherichia, particularly E.coli (continued)

| No. | sign_phenos | sign_phenos_class | best_ pheno | best_pheno_ class |
|---|---|---|---|---|
| 1 | AM;A/S;AUG;CAX;CP;CRM;GM; LVX;TO;T/S | aminoglycoside;fluoroquinolone; lactam;other | AM | lactam |
| 2 | AM;A/S;AUG;AZT;CAX;CFT;CP; CRM;GM;LVX;TO;T/S | aminoglycoside;fluoroquinolone; lactam;other | T/S | other |
| 3 | AM;A/S;AUG;AZT;CAX;CFT;CP; CRM;GM;LVX;TO;T/S | aminoglycoside;fluoroquinolone; lactam;other | T/S | other |
| 4 | AM;A/S;AUG;AZT;CAX;CFT;CP; CRM;GM;LVX;TO;T/S | aminoglycoside;fluoroquinolone; lactam;other | T/S | other |
| 5 | AM;A/S;AUG;CRM | lactam | AM | lactam |
| 6 | AM;A/S;AUG | lactam | AM | lactam |
| 7 | AM;A/S;AUG;AZT;CAX;CFT;CP; CRM;GM;LVX;TO;T/S | aminoglycoside;fluoroquinolone; lactam;other | T/S | other |
| 8 | AM;A/S;AUG;AZT;CAX;CFT;CP; CRM;GM;LVX;TO;T/S | aminoglycoside;fluoroquinolone; lactam;other | T/S | other |
| 9 | AM;A/S;AUG;AZT;CAX;CFT;CP; CRM;GM;LVX;TO;T/S | aminoglycoside;fluoroquinolone; lactam;other | T/S | other |
| 10 | AM;A/S;AUG;AZT;CAX;CFT;CP; CRM;GM;LVX;TO;T/S | aminoglycoside;fluoroquinolone; lactam;other | T/S | other |
| 11 | AM;A/S;AUG;AZT;CAX;CFT;CP; CRM;GM;LVX;TO;T/S | aminoglycoside;fluoroquinolone; lactam;other | T/S | other |
| 12 | AM;A/S;AUG;AZT;CAX;CFT;CP; CRM;GM;LVX;TO;T/S | aminoglycoside;fluoroquinolone; lactam;other | T/S | other |
| 13 | AM;A/S;AUG;AZT;CAX;CFT;CP; CRM;GM;LVX;TO;T/S | aminoglycoside;fluoroquinolone; lactam;other | T/S | other |
| 14 | AM;A/S;AUG;AZT;CAX;CFT;CP; CRM;GM;LVX;TO;T/S | aminoglycoside;fluoroquinolone; lactam;other | LVX | fluoroquinolone |
| 15 | AM;A/S;AUG;AZT;CAX;CFT;CP; CRM;GM;LVX;TO;T/S | aminoglycoside;fluoroquinolone; lactam;other | LVX | fluoroquinolone |

(continued)

| No. | sign_phenos | sign_phenos_class | best_pheno | best_pheno_ class |
|---|---|---|---|---|
| 16 | AM;A/S;AUG;AZT;CAX;CFT;CP; CRM;GM;LVX;TO;T/S | aminoglycoside;fluoroquinolone; lactam;other | T/S | other |
| 17 | AM;A/S;AUG;AZT;CAX;CFT;CP; CRM;GM;LVX;TO;T/S | aminoglycoside;fluoroquinolone; lactam;other | T/S | other |
| 18 | AM;A/S;AUG;AZT;CAX;CFT;CP; CRM;GM;LVX;TO;T/S | aminoglycoside;fluoroquinolone; lactam;other | T/S | other |
| 19 | AM;A/S;AUG;AZT;CAX;CFT;CP; CRM;GM;LVX;TO;T/S | aminoglycoside;fluoroquinolone; lactam;other | T/S | other |
| 20 | AM;A/S;AUG;AZT;CAX;CFT;CP; CRM;GM;LVX;TO;T/S | aminoglycoside;fluoroquinolone; lactam;other | T/S | other |
| 21 | AM;A/S;AUG;AZT;CAX;CFT;CP; CRM;GM;LVX;TO;T/S | aminoglycoside;fluoroquinolone; lactam;other | CAX | lactam |
| 22 | AM;A/S;AUG;AZT;CAX;CFT;CP; CRM;GM;LVX;TO;T/S | aminoglycoside;fluoroquinolone; lactam;other | T/S | other |
| 23 | AM;A/S;AUG;AZT;CAX;CFT;CP; CRM;GM;LVX;TO;T/S | aminoglycoside;fluoroquinolone; lactam;other | AM | lactam |
| 24 | AM;A/S;AUG;CAX;CFT;CP;CRM; GM;LVX;TO;T/S | aminoglycoside;fluoroquinolone; lactam;other | T/S | other |
| 25 | AM;A/S;AUG;AZT;CAX;CFT;CP; CRM;GM;LVX;T/S | aminoglycoside;fluoroquinolone; lactam;other | T/S | other |
| 26 | AM;A/S;AUG;AZT;CAX;CFT;CP; CRM;GM;TO;T/S | aminoglycoside;fluoroquinolone; lactam;other | T/S | other |
| 27 | AM;AUG;AZT;CAX;CFT;CP;CRM; GM;LVX;TO;T/S | aminoglycoside;fluoroquinolone; lactam;other | LVX | fluoroquinolone |
| 28 | AM;AUG;AZT;CAX;CFT;CP;CRM; GM;LVX;TO;T/S | aminoglycoside;fluoroquinolone; lactam;other | LVX | fluoroquinolone |
| 29 | AM;A/S;AUG;CAX;CFT;CP;CRM; GM;LVX;T/S | aminoglycoside;fluoroquinolone; lactam;other | T/S | other |
| 30 | AM;A/S;AUG;AZT;CAX;CFT;CP; CRM;GM;LVX;TO;T/S | aminoglycoside;fluoroquinolone; lactam;other | LVX | fluoroquinolone |
| 31 | AM;A/S;AUG;AZT;CAX;CFT;CP; CRM;GM;LVX;TO;T/S | aminoglycoside;fluoroquinolone; lactam;other | CP | fluoroquinolone |
| 32 | AM;A/S;AUG;AZT;CAX;CFT;CP; CRM;GM;LVX;TO;T/S | aminoglycoside;fluoroquinolone; lactam;other | LVX | fluoroquinolone |
| 33 | AM;A/S;AUG;AZT;CAX;CFT;CP; CRM;GM;LVX;TO;T/S | aminoglycoside;fluoroquinolone; lactam;other | LVX | fluoroquinolone |
| 34 | AM;A/S;AUG;AZT;CAX;CFT;CP; CRM;GM;LVX;TO;T/S | aminoglycoside;fluoroquinolone; lactam;other | LVX | fluoroquinolone |
| 35 | AM;A/S;AUG;AZT;CAX;CFT;CP; CRM;GM;LVX;TO;T/S | aminoglycoside;fluoroquinolone; lactam;other | LVX | fluoroquinolone |
| 36 | AM;A/S;AUG;AZT;CAX;CFT;CP; CRM;GM;LVX;TO;T/S | aminoglycoside;fluoroquinolone; lactam;other | LVX | fluoroquinolone |
| 37 | AM;A/S;AUG;AZT;CAX;CFT;CP; CRM;GM;LVX;TO;T/S | aminoglycoside;fluoroquinolone; lactam;other | LVX | fluoroquinolone |

(continued)

| No. | sign_phenos | sign_phenos_class | best_pheno | best_pheno_ class |
|---|---|---|---|---|
| 38 | AM;A/S;AUG;AZT;CAX;CFT;CP; CRM;GM;LVX;TO;T/S | aminoglycoside;fluoroquinolone; lactam;other | LVX | fluoroquinolone |
| 39 | AM;A/S;AUG;AZT;CAX;CFT;CP; CRM;GM;LVX;TO;T/S | aminoglycoside;fluoroquinolone; lactam;other | LVX | fluoroquinolone |
| 40 | AM;A/S;AUG;AZT;CAX;CFT;CP; CRM;GM;LVX;TO;T/S | aminoglycoside;fluoroquinolone; lactam;other | CP | fluoroquinolone |
| 41 | AM;A/S;AUG;AZT;CAX;CFT;CP; CRM;GM;LVX;TO;T/S | aminoglycoside;fluoroquinolone; lactam;other | LVX | fluoroquinolone |
| 42 | AM;A/S;AUG;AZT;CAX;CFT;CP; CRM;GM;LVX;TO;T/S | aminoglycoside;fluoroquinolone; lactam;other | LVX | fluoroquinolone |
| 43 | AM;A/S;AUG;AZT;CAX;CFT;CP; CRM;GM;LVX;TO;T/S | aminoglycoside;fluoroquinolone; lactam;other | LVX | fluoroquinolone |
| 44 | AM;A/S;AUG;AZT;CAX;CFT;CP; CRM;GM;LVX;TO;T/S | aminoglycoside;fluoroquinolone; lactam;other | LVX | fluoroquinolone |
| 45 | AM;A/S;AUG;AZT;CAX;CFT;CP; CRM;GM;T/S | aminoglycoside;fluoroquinolone; lactam;other | T/S | other |
| 46 | AM;A/S;AUG;AZT;CAX;CFT;CP; CRM;GM;LVX;TO;T/S | aminoglycoside;fluoroquinolone; lactam;other | LVX | fluoroquinolone |
| 47 | AM;A/S;AUG;AZT;CAX;CFT;CP; CRM;GM;LVX;TO;T/S | aminoglycoside;fluoroquinolone; lactam;other | LVX | fluoroquinolone |
| 48 | AM;A/S;AUG;AZT;CAX;CFT;CP; CRM;GM;LVX;TO;T/S | aminoglycoside;fluoroquinolone; lactam;other | LVX | fluoroquinolone |
| 49 | AM;A/S;AUG;T/S | lactam;other | T/S | other |
| 50 | AM;A/S;AUG;AZT;CAX;CFT;CP; CRM;GM;LVX;TO;T/S | aminoglycoside;fluoroquinolone; lactam;other | LVX | fluoroquinolone |

Table 7c: Data for Escherichia, particularly E.coli (continued)

| No. | num_aminoglycoside | num_fluoroquinolone | num_lactam | num_other |
|---|---|---|---|---|
| 1 | 2 | 2 | 5 | 1 |
| 2 | 2 | 2 | 7 | 1 |
| 3 | 2 | 2 | 7 | 1 |
| 4 | 2 | 2 | 7 | 1 |
| 5 | 0 | 0 | 4 | 0 |
| 6 | 0 | 0 | 3 | 0 |
| 7 | 2 | 2 | 7 | 1 |
| 8 | 2 | 2 | 7 | 1 |
| 9 | 2 | 2 | 7 | 1 |
| 10 | 2 | 2 | 7 | 1 |
| 11 | 2 | 2 | 7 | 1 |
| 12 | 2 | 2 | 7 | 1 |

(continued)

| No. | num_aminoglycoside | num_fluoroquinolone | num_lactam | num_other |
|-----|--------------------|--------------------|-----------|-----------|
| 13 | 2 | 2 | 7 | 1 |
| 14 | 2 | 2 | 7 | 1 |
| 15 | 2 | 2 | 7 | 1 |
| 16 | 2 | 2 | 7 | 1 |
| 17 | 2 | 2 | 7 | 1 |
| 18 | 2 | 2 | 7 | 1 |
| 19 | 2 | 2 | 7 | 1 |
| 20 | 2 | 2 | 7 | 1 |
| 21 | 2 | 2 | 7 | 1 |
| 22 | 2 | 2 | 7 | 1 |
| 23 | 2 | 2 | 7 | 1 |
| 24 | 2 | 2 | 6 | 1 |
| 25 | 1 | 2 | 7 | 1 |
| 26 | 2 | 1 | 7 | 1 |
| 27 | 2 | 2 | 6 | 1 |
| 28 | 2 | 2 | 6 | 1 |
| 29 | 1 | 2 | 6 | 1 |
| 30 | 2 | 2 | 7 | 1 |
| 31 | 2 | 2 | 7 | 1 |
| 32 | 2 | 2 | 7 | 1 |
| 33 | 2 | 2 | 7 | 1 |
| 34 | 2 | 2 | 7 | 1 |
| 35 | 2 | 2 | 7 | 1 |
| 36 | 2 | 2 | 7 | 1 |
| 37 | 2 | 2 | 7 | 1 |
| 38 | 2 | 2 | 7 | 1 |
| 39 | 2 | 2 | 7 | 1 |
| 40 | 2 | 2 | 7 | 1 |
| 41 | 2 | 2 | 7 | 1 |
| 42 | 2 | 2 | 7 | 1 |
| 43 | 2 | 2 | 7 | 1 |
| 44 | 2 | 2 | 7 | 1 |
| 45 | 1 | 1 | 7 | 1 |
| 46 | 2 | 2 | 7 | 1 |
| 47 | 2 | 2 | 7 | 1 |
| 48 | 2 | 2 | 7 | 1 |
| 49 | 0 | 0 | 3 | 1 |
| 50 | 2 | 2 | 7 | 1 |

Table 7d: Data for Escherichia, particularly E.coli (continued)

| No. | AM_pv_adj | A/S_pv_adj | AUG_pv_adj | AZT_pv_adj |
|-----|-----------|------------|------------|------------|
| 1 | 3.50E-207 | 1.39E-175 | 2.23E-54 | 1.94E-02 |
| 2 | 3.12E-58 | 4.85E-48 | 1.56E-20 | 2.24E-14 |
| 3 | 2.85E-46 | 1.23E-39 | 2.49E-17 | 7.44E-15 |
| 4 | 2.62E-45 | 3.93E-39 | 1.12E-17 | 7.82E-14 |
| 5 | 1.24E-80 | 9.93E-72 | 2.04E-21 | 1.77E-02 |
| 6 | 2.42E-75 | 1.11E-66 | 8.58E-21 | 6.08E-02 |
| 7 | 1.01E-26 | 2.48E-22 | 5.21E-09 | 2.11E-04 |
| 8 | 4.88E-38 | 1.20E-35 | 3.67E-16 | 6.67E-10 |
| 9 | 2.35E-59 | 7.04E-49 | 2.73E-24 | 1.89E-06 |
| 10 | 2.35E-59 | 7.04E-49 | 1.31E-23 | 5.63E-06 |
| 11 | 1.84E-24 | 1.82E-23 | 1.33E-10 | 1.05E-06 |
| 12 | 2.51E-64 | 6.77E-56 | 3.88E-22 | 9.19E-04 |
| 13 | 2.79E-25 | 9.09E-23 | 1.32E-08 | 1.48E-03 |
| 14 | 2.94E-45 | 2.43E-34 | 1.15E-22 | 2.63E-14 |
| 15 | 1.58E-45 | 1.72E-34 | 1.75E-22 | 1.91E-14 |
| 16 | 3.26E-28 | 1.85E-21 | 2.00E-08 | 1.16E-05 |
| 17 | 1.44E-20 | 2.94E-18 | 5.11E-07 | 1.25E-04 |
| 18 | 6.63E-29 | 5.38E-22 | 1.16E-08 | 1.11E-05 |
| 19 | 6.41E-30 | 1.52E-22 | 9.58E-09 | 6.45E-06 |
| 20 | 4.62E-35 | 3.20E-30 | 5.19E-17 | 2.18E-07 |
| 21 | 1.82E-09 | 8.62E-11 | 2.50E-17 | 1.74E-39 |
| 22 | 3.88E-27 | 4.06E-27 | 4.62E-12 | 9.38E-06 |
| 23 | 1.09E-44 | 2.09E-39 | 1.05E-16 | 2.10E-06 |
| 24 | 2.75E-28 | 8.80E-22 | 3.79E-08 | 1.79E-02 |
| 25 | 6.10E-28 | 3.56E-20 | 2.25E-09 | 8.28E-04 |
| 26 | 5.97E-23 | 1.19E-15 | 1.39E-06 | 1.28E-03 |
| 27 | 4.17E-03 | 3.66E-02 | 4.90E-07 | 1.65E-14 |
| 28 | 4.17E-03 | 3.66E-02 | 4.90E-07 | 1.65E-14 |
| 29 | 1.37E-28 | 1.45E-21 | 8.41E-09 | 2.36E-02 |
| 30 | 5.88E-10 | 1.33E-07 | 1.85E-09 | 7.58E-11 |
| 31 | 1.14E-08 | 2.28E-05 | 5.66E-08 | 3.43E-16 |
| 32 | 1.96E-09 | 3.67E-07 | 4.28E-09 | 1.44E-10 |
| 33 | 2.88E-09 | 5.08E-07 | 4.50E-09 | 1.51E-10 |
| 34 | 1.07E-10 | 2.85E-08 | 1.31E-06 | 4.19E-06 |
| 35 | 4.32E-08 | 6.04E-05 | 1.49E-07 | 4.44E-16 |
| 36 | 8.33E-08 | 9.79E-05 | 2.97E-07 | 4.44E-16 |
| 37 | 8.33E-08 | 9.79E-05 | 2.97E-07 | 4.44E-16 |

(continued)

| No. | AM_pv_adj | A/S_pv_adj | AUG_pv_adj | AZT_pv_adj |
|-----|-----------|------------|------------|------------|
| 38 | 3.07E-08 | 4.70E-05 | 1.02E-07 | 7.64E-16 |
| 39 | 4.14E-12 | 7.23E-10 | 1.20E-05 | 1.17E-03 |
| 40 | 1.57E-07 | 1.55E-04 | 4.17E-07 | 7.80E-16 |
| 41 | 2.15E-07 | 1.95E-04 | 5.61E-07 | 8.13E-16 |
| 42 | 1.55E-07 | 1.54E-04 | 3.95E-07 | 8.67E-16 |
| 43 | 1.55E-07 | 1.54E-04 | 3.95E-07 | 8.67E-16 |
| 44 | 1.55E-07 | 1.54E-04 | 3.95E-07 | 8.67E-16 |
| 45 | 3.13E-28 | 1.97E-20 | 2.60E-09 | 3.55E-04 |
| 46 | 1.16E-10 | 3.41E-08 | 3.47E-07 | 2.45E-06 |
| 47 | 2.72E-09 | 3.18E-07 | 2.44E-07 | 1.12E-10 |
| 48 | 2.66E-09 | 8.84E-08 | 1.21E-05 | 1.64E-08 |
| 49 | 4.47E-24 | 6.61E-18 | 2.77E-06 | 2.45E-01 |
| 50 | 2.16E-06 | 8.47E-04 | 1.36E-06 | 7.02E-16 |

Table 7e: Data for Escherichia, particularly E.coli (continued)

| No. | CAX_pv_adj | CFT_pv_adj | CP_pv_adj | CRM_pv_adj |
|-----|------------|------------|-----------|------------|
| 1 | 9.08E-03 | 5.54E-02 | 2.29E-17 | 2.63E-05 |
| 2 | 4.55E-21 | 9.31E-20 | 3.14E-43 | 1.15E-19 |
| 3 | 4.33E-19 | 1.87E-17 | 1.53E-32 | 5.07E-17 |
| 4 | 6.85E-18 | 2.28E-16 | 6.99E-31 | 7.62E-16 |
| 5 | 1.28E-02 | 7.14E-02 | 5.78E-01 | 3.21E-04 |
| 6 | 1.41E-01 | 3.53E-01 | 2.23E-01 | 3.75E-02 |
| 7 | 2.25E-06 | 3.51E-06 | 1.44E-38 | 1.55E-08 |
| 8 | 3.18E-15 | 1.35E-14 | 4.48E-11 | 1.70E-12 |
| 9 | 2.34E-05 | 5.15E-06 | 1.74E-15 | 7.72E-10 |
| 10 | 5.80E-05 | 9.14E-06 | 6.46E-15 | 2.18E-09 |
| 11 | 6.82E-10 | 3.64E-09 | 1.69E-49 | 1.37E-10 |
| 12 | 5.59E-03 | 1.88E-03 | 1.12E-09 | 2.39E-05 |
| 13 | 9.17E-05 | 2.37E-04 | 1.28E-36 | 6.73E-06 |
| 14 | 8.04E-16 | 5.19E-14 | 7.03E-61 | 9.05E-21 |
| 15 | 5.55E-16 | 2.31E-14 | 2.47E-60 | 6.03E-21 |
| 16 | 5.30E-07 | 2.10E-06 | 1.38E-39 | 8.69E-08 |
| 17 | 1.67E-06 | 3.95E-06 | 1.23E-38 | 5.25E-08 |
| 18 | 4.67E-07 | 1.87E-06 | 6.39E-39 | 5.20E-08 |
| 19 | 4.17E-07 | 1.68E-06 | 2.84E-39 | 4.28E-08 |
| 20 | 5.95E-11 | 4.86E-10 | 4.79E-07 | 1.46E-09 |
| 21 | 4.65E-46 | 9.26E-45 | 3.20E-19 | 1.06E-36 |

(continued)

| No. | CAX_pv_adj | CFT_pv_adj | CP_pv_adj | CRM_pv_adj |
|-----|-----------|-----------|-----------|-----------|
| 22 | 5.55E-08 | 2.77E-08 | 8.59E-09 | 1.64E-09 |
| 23 | 4.89E-09 | 9.33E-07 | 3.24E-25 | 8.32E-08 |
| 24 | 8.50E-03 | 9.80E-03 | 5.28E-05 | 4.13E-03 |
| 25 | 4.82E-05 | 7.11E-05 | 5.83E-03 | 5.89E-04 |
| 26 | 1.31E-04 | 1.77E-04 | 7.59E-03 | 2.84E-03 |
| 27 | 2.32E-15 | 2.75E-17 | 6.79E-43 | 2.56E-16 |
| 28 | 2.32E-15 | 2.75E-17 | 6.79E-43 | 2.56E-16 |
| 29 | 3.66E-03 | 4.47E-03 | 4.75E-03 | 2.48E-03 |
| 30 | 6.25E-11 | 6.54E-10 | 4.02E-42 | 2.80E-12 |
| 31 | 6.60E-17 | 2.67E-17 | 8.45E-42 | 2.23E-17 |
| 32 | 7.29E-11 | 7.76E-10 | 1.57E-41 | 5.31E-12 |
| 33 | 7.91E-11 | 8.46E-10 | 2.18E-41 | 1.70E-11 |
| 34 | 4.29E-06 | 1.52E-06 | 1.28E-40 | 1.80E-07 |
| 35 | 1.31E-16 | 5.36E-17 | 3.96E-41 | 4.26E-17 |
| 36 | 1.31E-16 | 5.36E-17 | 3.96E-41 | 4.26E-17 |
| 37 | 1.31E-16 | 5.36E-17 | 3.96E-41 | 4.26E-17 |
| 38 | 1.43E-16 | 5.77E-17 | 4.56E-41 | 1.17E-17 |
| 39 | 6.46E-05 | 1.74E-04 | 2.25E-40 | 1.80E-06 |
| 40 | 2.55E-16 | 1.06E-16 | 5.76E-41 | 7.39E-17 |
| 41 | 2.58E-16 | 1.06E-16 | 1.05E-40 | 7.84E-17 |
| 42 | 2.68E-16 | 1.10E-16 | 1.19E-40 | 2.23E-17 |
| 43 | 2.68E-16 | 1.10E-16 | 1.19E-40 | 2.23E-17 |
| 44 | 2.68E-16 | 1.10E-16 | 1.19E-40 | 2.23E-17 |
| 45 | 1.90E-05 | 2.89E-05 | 6.00E-03 | 1.99E-04 |
| 46 | 1.84E-06 | 5.25E-07 | 1.05E-39 | 6.76E-08 |
| 47 | 8.60E-10 | 4.43E-09 | 9.49E-40 | 8.85E-14 |
| 48 | 2.36E-11 | 1.27E-10 | 4.92E-38 | 2.22E-11 |
| 49 | 1.07E-01 | 1.14E-01 | 1.50E-02 | 6.49E-02 |
| 50 | 4.02E-16 | 1.67E-16 | 1.34E-38 | 7.42E-18 |

Table 7f: Data for Escherichia, particularly E.coli (continued)

| No. | GM_pv_adj | LVX_pv_adj | TO_pv_adj | T/S_pv_adj |
|-----|-----------|-----------|-----------|-----------|
| 1 | 2.40E-14 | 2.96E-16 | 6.56E-10 | 5.59E-55 |
| 2 | 9.02E-31 | 5.84E-43 | 3.02E-26 | 1.62E-119 |
| 3 | 3.09E-28 | 1.25E-33 | 1.52E-27 | 6.25E-97 |
| 4 | 1.19E-27 | 5.56E-32 | 5.30E-27 | 8.78E-96 |
| 5 | 5.50E-01 | 4.74E-01 | 9.96E-02 | 3.77E-02 |

(continued)

| No. | GM_pv_adj | LVX_pv_adj | TO_pv_adj | T/S_pv_adj |
|---|---|---|---|---|
| 6 | 8.08E-01 | 2.88E-01 | 8.73E-01 | 1.21E-01 |
| 7 | 2.33E-13 | 1.57E-38 | 6.37E-15 | 7.68E-72 |
| 8 | 4.17E-11 | 5.22E-11 | 1.79E-08 | 1.58E-71 |
| 9 | 7.23E-11 | 6.17E-14 | 8.00E-05 | 1.01E-69 |
| 10 | 2.80E-10 | 1.39E-13 | 8.00E-05 | 1.23E-68 |
| 11 | 1.18E-18 | 2.49E-49 | 2.67E-22 | 6.77E-68 |
| 12 | 7.65E-07 | 1.10E-08 | 7.07E-03 | 1.95E-64 |
| 13 | 1.21E-16 | 1.92E-37 | 4.13E-18 | 1.87E-63 |
| 14 | 4.86E-26 | 7.43E-62 | 5.25E-32 | 2.58E-39 |
| 15 | 2.72E-26 | 3.08E-61 | 2.92E-32 | 4.30E-39 |
| 16 | 1.39E-16 | 1.68E-39 | 1.59E-17 | 1.74E-60 |
| 17 | 5.78E-18 | 2.01E-39 | 1.91E-19 | 2.44E-60 |
| 18 | 1.02E-16 | 7.87E-39 | 1.17E-17 | 6.74E-60 |
| 19 | 7.38E-17 | 3.56E-39 | 8.56E-18 | 2.63E-59 |
| 20 | 6.73E-07 | 9.82E-07 | 5.94E-07 | 3.47E-47 |
| 21 | 5.34E-12 | 1.45E-19 | 7.00E-09 | 2.74E-06 |
| 22 | 3.89E-07 | 1.14E-08 | 2.09E-05 | 7.71E-46 |
| 23 | 6.04E-13 | 2.10E-24 | 3.47E-12 | 1.02E-37 |
| 24 | 8.52E-06 | 1.56E-04 | 7.54E-03 | 4.90E-44 |
| 25 | 9.39E-05 | 9.80E-03 | 1.28E-02 | 6.98E-44 |
| 26 | 2.16E-04 | 1.65E-02 | 9.49E-03 | 9.68E-44 |
| 27 | 1.81E-12 | 3.58E-43 | 2.05E-13 | 2.13E-06 |
| 28 | 1.81E-12 | 3.58E-43 | 2.05E-13 | 2.13E-06 |
| 29 | 1.08E-04 | 8.15E-03 | 5.44E-02 | 5.54E-43 |
| 30 | 1.81E-12 | 2.71E-42 | 4.67E-11 | 6.64E-17 |
| 31 | 3.65E-19 | 9.37E-42 | 2.80E-17 | 3.20E-18 |
| 32 | 3.61E-12 | 1.03E-41 | 9.37E-11 | 2.18E-16 |
| 33 | 3.86E-12 | 1.47E-41 | 9.73E-11 | 2.45E-16 |
| 34 | 1.03E-13 | 2.44E-41 | 2.22E-16 | 4.82E-04 |
| 35 | 4.79E-19 | 2.61E-41 | 5.83E-17 | 9.95E-18 |
| 36 | 4.79E-19 | 2.61E-41 | 5.83E-17 | 3.03E-17 |
| 37 | 4.79E-19 | 2.61E-41 | 5.83E-17 | 3.03E-17 |
| 38 | 8.47E-19 | 3.21E-41 | 6.04E-17 | 3.36E-18 |
| 39 | 3.56E-12 | 3.69E-41 | 8.59E-15 | 1.04E-04 |
| 40 | 8.74E-19 | 6.20E-41 | 6.42E-17 | 5.21E-17 |
| 41 | 9.35E-19 | 6.76E-41 | 6.96E-17 | 8.31E-17 |
| 42 | 1.03E-18 | 8.13E-41 | 1.27E-16 | 2.95E-17 |
| 43 | 1.03E-18 | 8.13E-41 | 1.27E-16 | 2.95E-17 |

(continued)

| No. | GM_pv_adj | LVX_pv_adj | TO_pv_adj | T/S_pv_adj |
|-----|-----------|------------|-----------|------------|
| 44 | 1.03E-18 | 8.13E-41 | 1.27E-16 | 2.95E-17 |
| 45 | 9.79E-05 | 1.29E-02 | 1.33E-02 | 1.07E-40 |
| 46 | 1.98E-13 | 1.85E-40 | 4.73E-16 | 4.17E-04 |
| 47 | 2.68E-16 | 1.90E-40 | 1.10E-17 | 2.80E-08 |
| 48 | 1.27E-13 | 3.36E-40 | 7.14E-14 | 3.41E-07 |
| 49 | 1.15E-02 | 2.48E-02 | 4.44E-01 | 3.77E-40 |
| 50 | 1.50E-18 | 8.68E-39 | 9.76E-17 | 2.56E-15 |

Table 7g: Data for Escherichia, particularly E.coli (continued)

| No. | Locus.Tag | Gene. Symbol | GenBank.Accession | Chromosome |
|-----|-----------|--------------|-------------------|------------|
| 1 | SeHA_C1580 | blaT | YP_002045448 | |
| 2 | EcolB7_01001258 | | ZP_00717086 | |
| 3 | HMPREF9345_ 05066 | | ZP_07100147 | |
| 4 | KPN_pKPN5p08201 | | YP_001338811 | plasmid pKPN5 |
| 5 | HMPREF9547_ 03104 | | ZP_07169558 | |
| 6 | SeSA_B0079 | | YP_002112958 | plasmid pCVM19633_110 |
| 7 | HMPREF9345_ 05232 | | ZP_07100311 | |
| 8 | EcolB_01004576 | | ZP_00708527 | |
| 9 | pEFER_0049 | | YP_002394596 | plasmid pEFER |
| 10 | ETAE_p040 | | YP_003297634 | plasmid pEIB202 |
| 11 | ECGG_04429 | | ZP_06648541 | |
| 12 | ETAE_p041 | folP | YP_003297635 | plasmid pEIB202 |
| 13 | ECGG_04434 | | ZP_06648545 | |
| 14 | ROD_p1051 | pemI | YP_003368450 | plasmid pCROD1 |
| 15 | ROD_p1061 | pemK | YP_003368451 | plasmid pCROD1 |
| 16 | HMPREF9348_ 05540 | | ZP_07692671 | |
| 17 | ECUMN_ 4816 | aadA | YP_002415408 | |
| 18 | ECUMN_ 4827 | mphA | YP_002415417 | |
| 19 | HMPREF9345_ 05223 | | ZP_07100302 | |
| 20 | EcSMS35_A0150 | | YP_001740030 | plasmid pSMS35_130 |
| 21 | HMPREF9548_ 03778 | | ZP_07141582 | |
| 22 | HMPREF9348_ 05561 | | ZP_07692692 | |
| 23 | HMPREF9548_ 03784 | | ZP_07141588 | |
| 24 | KPN_pKPN4p07064 | | YP_001338673 | plasmid pKPN4 |
| 25 | HMPREF9345_ 03525 | | ZP_07098656 | |

(continued)

| No. | Locus.Tag | Gene. Symbol | GenBank.Accession | Chromosome |
|---|---|---|---|---|
| 26 | KPN_pKPN4p07068 | merC | YP_001338677 | plasmid pKPN4 |
| 27 | ESCG_01010 | | ZP_ 04871321 | |
| 28 | HMPREF9540_ 00466 | | ZP_07133313 | |
| 29 | STMDT12_C39340 | | BAJ38877 | |
| 30 | KPK_A0022 | sopB | YP_002235674 | plasmid pKP187 |
| 31 | EcoliO157_010100014809 | | ZP_02780941 | |
| 32 | EcSMS35_A0072 | sopB | YP_001739960 | plasmid pSMS35_130 |
| 33 | HMPREF9538_00090 | | ZP_08302456 | |
| 34 | ECNA114_2444 | | AEG37278 | |
| 35 | EschericcoliO157_010100006568 | | ZP_02749755 | |
| 36 | CSAG_00128 | | ZP_04560798 | |
| 37 | ECH74115_0422 | mhpA | YP_002268988 | |
| 38 | CIT292_01335 | | ZP_03835561 | |
| 39 | ECSF_1165 | | BAI54705 | |
| 40 | EscherichiacoliO157EcO_010100009454 | | ZP_05948572 | |
| 41 | SSON_0329 | mhpD | YP_309341 | |
| 42 | CIT292_01336 | | ZP_03835562 | |
| 43 | HMPREF9540_00984 | | ZP_07133822 | |
| 44 | Missing locus_tag:AC_ 000091.cds340.373092..374105 | mhpE | AP_001004 | |
| 45 | HMPREF9551_04611 | | ZP_07191964 | |
| 46 | SeHA_C2638 | | YP_002046450 | |
| 47 | SFxv_0758 | | ADA73054 | |
| 48 | HMPREF9530_01431 | | ZP_07151340 | |
| 49 | KPN_pKPN4p07069 | merA | YP_001338678 | plasmid pKPN4 |
| 50 | CP0077 | | NP_858210 | plasmid pCP301 |

Table 7h: Data for Escherichia, particularly E.coli (continued)

| No. | Start.Coord | End.Coord | Strand | Scaffold.ID |
|---|---|---|---|---|
| 1 | 1525960 | 1526820 | + | 642555218 |
| 2 | 77515 | 77910 | + | 638358454 |
| 3 | 4715 | 5554 | - | 648294624 |
| 4 | 34908 | 35255 | - | 640753021 |
| 5 | 3248 | 3841 | + | 648291919 |
| 6 | 68933 | 70240 | + | 642555219 |
| 7 | 5252 | 5572 | - | 648294635 |

(continued)

| No. | Start.Coord | End.Coord | Strand | Scaffold.ID |
|-----|-------------|-----------|--------|-------------|
| 8 | 3555 | 4568 | + | 638359041 |
| 9 | 48658 | 49503 | + | 643692014 |
| 10 | 31237 | 32040 | - | 646311959 |
| 11 | 19278 | 19826 | + | 647536609 |
| 12 | 32101 | 32916 | - | 646311959 |
| 13 | 22618 | 23823 | + | 647536609 |
| 14 | 3339 | 3596 | + | 646311988 |
| 15 | 3529 | 3930 | + | 646311988 |
| 16 | 461 | 1045 | - | 649994953 |
| 17 | 4994882 | 4995670 | + | 644736335 |
| 18 | 5003053 | 5003958 | - | 644736335 |
| 19 | 1167 | 2405 | + | 648294635 |
| 20 | 118542 | 119474 | + | 641522668 |
| 21 | 4493 | 5755 | + | 648288593 |
| 22 | 209 | 661 | + | 649994959 |
| 23 | 8819 | 9535 | + | 648288593 |
| 24 | 23310 | 24614 | - | 640753020 |
| 25 | 1071 | 1307 | - | 648294596 |
| 26 | 25970 | 27664 | - | 640753020 |
| 27 | 241682 | 242032 | + | 646206991 |
| 28 | 192 | 443 | + | 648287910 |
| 29 | 4084530 | 4085237 | - | 651053007 |
| 30 | 12106 | 13071 | - | 643348532 |
| 31 | 123136 | 123969 | - | 641781714 |
| 32 | 53664 | 54635 | - | 641522668 |
| 33 | 5657 | 6832 | + | 651332930 |
| 34 | 2476906 | 2477910 | - | 651053098 |
| 35 | 369676 | 370620 | + | 641780393 |
| 36 | 146099 | 147763 | + | 646206732 |
| 37 | 430810 | 432474 | + | 643348548 |
| 38 | 25505 | 26449 | + | 643891503 |
| 39 | 1248345 | 1249289 | + | 646862305 |
| 40 | 20015 | 20965 | + | 645964942 |
| 41 | 356486 | 357301 | + | 640427102 |
| 42 | 26463 | 27344 | + | 643891503 |
| 43 | 7204 | 8217 | + | 648287942 |
| 44 | 373092 | 374105 | + | 637000000 |
| 45 | 97 | 531 | - | 648293053 |

(continued)

| No. | Start.Coord | End.Coord | Strand | Scaffold.ID |
|-----|-------------|-----------|--------|-------------|
| 46 | 2550781 | 2552010 | + | 642555218 |
| 47 | 734541 | 734921 | + | 646862363 |
| 48 | 8 | 643 | - | 648290358 |
| 49 | 27716 | 28177 | - | 640753020 |
| 50 | 61397 | 61579 | + | 637000224 |

Table 8a: Data for Escherichia, particularly E.coli

| No. | Scaffold.External. Accession | Scaffold.Name | best_pv |
|-----|------------------------------|---------------|---------|
| 1 | NC_011083 | Salmonella enterica subsp. enterica serovar Heidelberg str. SL476: NC_011083 | 3.5041948925527e-207 |
| 2 | NZ_ AAJT01000012 | Escherichia coli B7A, unfinished sequence: NZ_ AAJT01000012 | 1.62252621621877e-119 |
| 3 | NZ_ ADWV01000045 | Escherichia coli MS 107-1 E_coliMS107-1-1.0.1_Cont44.1: NZ_ADWV01000045 | 6.24750842070913e-97 |
| 4 | NC_009651 | Klebsiella pneumoniae subsp. pneumoniae MGH 78578 plasmid pKPN5: NC_009651 | 8.77735582031733e-96 |
| 5 | NZ_ ADWV01000056 | Escherichia coli MS 107-1 E_coliMS107-1-1.0.1_Cont55.1: NZ_ADWV01000056 | 7.68091736117399e-72 |
| 6 | NZ_ AAJX01000089 | Escherichia coli B171, unfinished sequence: NZ_ AAJX01000089 | 1.57772112100981e-71 |
| 7 | NC_011743 | Escherichia fergusonii ATCC 35469 plasmid pEFER: NC_ 011743 | 1.0118246517332e-69 |
| 8 | NC_013509 | Edwardsiella tarda EIB202 plasmid pEIB202: NC_013509 | 1.23255842888535e-68 |
| 9 | NZ_GG749218 | Escherichia coli FVEC1412 genomic scaffold supercont1.13: NZ_GG749218 | 6.77313303746994e-68 |
| 10 | NC_013509 | Edwardsiella tarda EIB202 plasmid pEIB202: NC_013509 | 1.94619564613577e-64 |
| 11 | NZ_GG749218 | Escherichia coli FVEC1412 genomic scaffold supercont1.13: NZ_GG749218 | 1.86737834552034e-63 |
| 12 | NC_013717 | Citrobacter rodentium ICC168 plasmid pCROD1: NC_013717 | 7.42780365363135e-62 |
| 13 | NC_013717 | Citrobacter rodentium ICC168 plasmid pCROD1: NC_013717 | 3.07751376659308e-61 |
| 14 | NZ_ ADWS01000089 | Escherichia coli MS 145-7 E_coliMS145-7-1.0.1_Cont88.1: NZ_ADWS01000089 | 1.7351795845908e-60 |
| 15 | NC_011751 | Escherichia coli UMN026: NC_011751 | 2.44317906034667e-60 |
| 16 | NC_011751 | Escherichia coli UMN026: NC_011751 | 6.73588199342071e-60 |
| 17 | NZ_ ADWV01000056 | Escherichia coli MS 107-1 E_coliMS107-1-1.0.1_Cont55.1: NZ_ADWV01000056 | 2.62943019143703e-59 |

(continued)

| No. | Scaffold.External.Accession | Scaffold.Name | best_pv |
|---|---|---|---|
| 18 | NC_010488 | Escherichia coli SECEC SMS-3-5 plasmid pSMS35_130: NC_010488 | 3.46702381924878e-47 |
| 19 | NZ_ADTM01000311 | Escherichia coli MS 182-1 E_coli182-1-1.0_Cont460.3: NZ_ADTM01000311 | 4.65308035729244e-46 |
| 20 | NZ_ADWS01000095 | Escherichia coli MS 145-7 E_coliMS145-7-1.0.1_Cont94.1: NZ_ADWS01000095 | 7.71451120848483e-46 |
| 21 | NZ_ADTM01000311 | Escherichia coli MS 182-1 E_coli182-1-1.0_Cont460.3: NZ_ADTM01000311 | 1.08551755499227e-44 |
| 22 | NC_009650 | Klebsiella pneumoniae subsp. pneumoniae MGH 78578 plasmid pKPN4: NC_009650 | 4.89761264283562e-44 |
| 23 | NZ_ADWV01000017 | Escherichia coli MS 107-1 E_coliMS107-1-1.0.1_Cont16.1: NZ_ADWV01000017 | 6.97693050178087e-44 |
| 24 | NC_009650 | Klebsiella pneumoniae subsp. pneumoniae MGH 78578 plasmid pKPN4: NC_009650 | 9.68294892967391e-44 |
| 25 | NZ_GG665811 | Escherichia sp. 1_1_43 genomic scaffold supercont1.1: NZ_GG665811 | 3.5843867496487e-43 |
| 26 | NZ_ADTL01000051 | Escherichia coli MS 115-1 E_coli115-1-1.0_Cont158.2: NZ_ADTL01000051 | 3.5843867496487e-43 |
| 27 | AP011957 | Salmonella enterica subsp. enterica serovar Typhimurium str. T000240 DNA: AP011957 | 5.53805522527039e-43 |
| 28 | NC_011282 | Klebsiella pneumoniae 342 plasmid pKP187: NC_011282 | 2.71226651272988e-42 |
| 29 | NZ_ABHR01000011 | Escherichia coli O157:H7 str. EC4401, unfinished sequence: NZ_ABHR01000011 | 8.45082785112866e-42 |
| 30 | NC_010488 | Escherichia coli SECEC SMS-3-5 plasmid pSMS35_130: NC_010488 | 1.02749291231853e-41 |
| 31 | NZ_AFBO01000013 | Klebsiella sp. MS 92-3 K_spMS92-3-1.0_Cont24.1: NZ_AFBO01000013 | 1.46909169086738e-41 |
| 32 | CP002797 | Escherichia coli NA114: CP002797 | 2.43588122712712e-41 |
| 33 | NZ_ABHK01000002 | Escherichia coli O157:H7 str. EC4206, unfinished sequence: NZ_ABHK01000002 | 2.60702932643171e-41 |
| 34 | NZ_GG657366 | Citrobacter sp. 30_2 genomic scaffold supercont1.1: NZ_GG657366 | 2.60702932643171e-41 |
| 35 | NC_011353 | Escherichia coli O157:H7 str. EC4115: NC_011353 | 2.60702932643171e-41 |
| 36 | NZ_ABWL01000012 | Citrobacter youngae ATCC 29220 C_sp-1.0_Cont2.3: NZ_ABWL01000012 | 3.21133372679738e-41 |
| 37 | AP009378 | Escherichia coli SE15 DNA: AP009378 | 3.68640118720819e-41 |
| 38 | NZ_ACXN01000074 | Escherichia coli O157:H7 str. FRIK966: NZ_ACXN01000074 | 5.75695041652081e-41 |
| 39 | NC_007384 | Shigella sonnei Ss046: NC_007384 | 6.75951367822551e-41 |

(continued)

| No. | Scaffold.External. Accession | Scaffold.Name | best_pv |
|---|---|---|---|
| 40 | NZ_ ABWL01000012 | Citrobacter youngae ATCC 29220 C_sp-1.0_Cont2.3: NZ_ ABWL01000012 | 8.12681193093067e-41 |
| 41 | NZ_ ADTL01000083 | Escherichia coli MS 115-1 E_coli115-1-1.0_Cont264.1: NZ_ ADTL01000083 | 8.12681193093067e-41 |
| 42 | AC_000091 | Escherichia coli W3110 DNA: AC_000091 | 8.12681193093067e-41 |
| 43 | NZ_ ADUD01000537 | Escherichia coli MS 196-1 E_coli196-1-1.0_Cont1482.1: NZ_ ADUD01000537 | 1.06521634399457e-40 |
| 44 | NC_011083 | Salmonella enterica subsp. enterica serovar Heidelberg str. SL476: NC_011083 | 1.84998224240006e-40 |
| 45 | CP001383 | Shigella flexneri 2002017: CP001383 | 1.89942261631873e-40 |
| 46 | NZ_ ADTR01000134 | Escherichia coli MS 21-1 E_coli21-1-1.0_Cont296.1: NZ_ ADTR01000134 | 3.35939290103889e-40 |
| 47 | NC_009650 | Klebsiella pneumoniae subsp. pneumoniae MGH 78578 plasmid pKPN4: NC_009650 | 3.76989142366575e-40 |
| 48 | NC_004851 | Shigella flexneri 2a str. 301 plasmid pCP301: NC_004851 | 8.68449218790606e-39 |
| 49 | NC_007607 | Shigella dysenteriae Sd197 plasmid pSD1_197: NC_007607 | 2.27746826869945e-38 |
| 50 | NZ_ ADTL01000280 | Escherichia coli MS 115-1 E_coli115-1-1.0_Cont677.5: NZ_ ADTL01000280 | 4.92053652141975e-38 |

Table 8b: Data for Escherichia, particularly E.coli (continued)

| No. | sign_phenos | sign_phenos_class | best_ pheno | best_pheno_ class |
|---|---|---|---|---|
| 1 | AM;A/S;AUG;CAX;CP;CRM;GM; LVX;TO;T/S | aminoglycoside;fluoroquinolone; lactam;other | AM | lactam |
| 2 | AM;A/S;AUG;AZT;CAX;CFT;CP; CRM;GM;LVX;TO;T/S | aminoglycoside;fluoroquinolone; lactam;other | T/S | other |
| 3 | AM;A/S;AUG;AZT;CAX;CFT;CP; CRM;GM;LVX;TO;T/S | aminoglycoside;fluoroquinolone; lactam;other | T/S | other |
| 4 | AM;A/S;AUG;AZT;CAX;CFT;CP; CRM;GM;LVX;TO;T/S | aminoglycoside;fluoroquinolone; lactam;other | T/S | other |
| 5 | AM;A/S;AUG;AZT;CAX;CFT;CP; CRM;GM;LVX;TO;T/S | aminoglycoside;fluoroquinolone; lactam;other | T/S | other |
| 6 | AM;A/S;AUG;AZT;CAX;CFT;CP; CRM;GM;LVX;TO;T/S | aminoglycoside;fluoroquinolone; lactam;other | T/S | other |
| 7 | AM;A/S;AUG;AZT;CAX;CFT;CP; CRM;GM;LVX;TO;T/S | aminoglycoside;fluoroquinolone; lactam;other | T/S | other |
| 8 | AM;A/S;AUG;AZT;CAX;CFT;CP; CRM;GM;LVX;TO;T/S | aminoglycoside;fluoroquinolone; lactam;other | T/S | other |

(continued)

| No. | sign_phenos | sign_phenos_class | best_ pheno | best_pheno_ class |
|-----|-------------|-------------------|-------------|-------------------|
| 9 | AM;A/S;AUG;AZT;CAX;CFT;CP; CRM;GM;LVX;TO;T/S | aminoglycoside;fluoroquinolone; lactam;other | T/S | other |
| 10 | AM;A/S;AUG;AZT;CAX;CFT;CP; CRM;GM;LVX;TO;T/S | aminoglycoside;fluoroquinolone; lactam;other | T/S | other |
| 11 | AM;A/S;AUG;AZT;CAX;CFT;CP; CRM;GM;LVX;TO;T/S | aminoglycoside;fluoroquinolone; lactam;other | T/S | other |
| 12 | AM;A/S;AUG;AZT;CAX;CFT;CP; CRM;GM;LVX;TO;T/S | aminoglycoside;fluoroquinolone; lactam;other | LVX | fluoroquinolone |
| 13 | AM;A/S;AUG;AZT;CAX;CFT;CP; CRM;GM;LVX;TO;T/S | aminoglycoside;fluoroquinolone; lactam;other | LVX | fluoroquinolone |
| 14 | AM;A/S;AUG;AZT;CAX;CFT;CP; CRM;GM;LVX;TO;T/S | aminoglycoside;fluoroquinolone; lactam;other | T/S | other |
| 15 | AM;A/S;AUG;AZT;CAX;CFT;CP; CRM;GM;LVX;TO;T/S | aminoglycoside;fluoroquinolone; lactam;other | T/S | other |
| 16 | AM;A/S;AUG;AZT;CAX;CFT;CP; CRM;GM;LVX;TO;T/S | aminoglycoside;fluoroquinolone; lactam;other | T/S | other |
| 17 | AM;A/S;AUG;AZT;CAX;CFT;CP; CRM;GM;LVX;TO;T/S | aminoglycoside;fluoroquinolone; lactam;other | T/S | other |
| 18 | AM;A/S;AUG;AZT;CAX;CFT;CP; CRM;GM;LVX;TO;T/S | aminoglycoside;fluoroquinolone; lactam;other | T/S | other |
| 19 | AM;A/S;AUG;AZT;CAX;CFT;CP; CRM;GM;LVX;TO;T/S | aminoglycoside;fluoroquinolone; lactam;other | CAX | lactam |
| 20 | AM;A/S;AUG;AZT;CAX;CFT;CP; CRM;GM;LVX;TO;T/S | aminoglycoside;fluoroquinolone; lactam;other | T/S | other |
| 21 | AM;A/S;AUG;AZT;CAX;CFT;CP; CRM;GM;LVX;TO;T/S | aminoglycoside;fluoroquinolone; lactam;other | AM | lactam |
| 22 | AM;A/S;AUG;CAX;CFT;CP;CRM; GM;LVX;TO;T/S | aminoglycoside;fluoroquinolone; lactam;other | T/S | other |
| 23 | AM;A/S;AUG;AZT;CAX;CFT;CP; CRM;GM;LVX;T/S | aminoglycoside;fluoroquinolone; lactam;other | T/S | other |
| 24 | AM;A/S;AUG;AZT;CAX;CFT;CP; CRM;GM;TO;T/S | aminoglycoside;fluoroquinolone; lactam;other | T/S | other |
| 25 | AM;AUG;AZT;CAX;CFT;CP;CRM; GM;LVX;TO;T/S | aminoglycoside;fluoroquinolone; lactam;other | LVX | fluoroquinolone |
| 26 | AM;AUG;AZT;CAX;CFT;CP;CRM; GM;LVX;TO;T/S | aminoglycoside;fluoroquinolone; lactam;other | LVX | fluoroquinolone |
| 27 | AM;A/S;AUG;CAX;CFT;CP;CRM; GM;LVX;T/S | aminoglycoside;fluoroquinolone; lactam;other | T/S | other |
| 28 | AM;A/S;AUG;AZT;CAX;CFT;CP; CRM;GM;LVX;TO;T/S | aminoglycoside;fluoroquinolone; lactam;other | LVX | fluoroquinolone |
| 29 | AM;A/S;AUG;AZT;CAX;CFT;CP; CRM;GM;LVX;TO;T/S | aminoglycoside;fluoroquinolone; lactam;other | CP | fluoroquinolone |
| 30 | AM;A/S;AUG;AZT;CAX;CFT;CP; CRM;GM;LVX;TO;T/S | aminoglycoside;fluoroquinolone; lactam;other | LVX | fluoroquinolone |

(continued)

| No. | sign_phenos | sign_phenos_class | best_pheno | best_pheno_ class |
|---|---|---|---|---|
| 31 | AM;A/S;AUG;AZT;CAX;CFT;CP; CRM;GM;LVX;TO;T/S | aminoglycoside;fluoroquinolone; lactam;other | LVX | fluoroquinolone |
| 32 | AM;A/S;AUG;AZT;CAX;CFT;CP; CRM;GM;LVX;TO;T/S | aminoglycoside;fluoroquinolone; lactam;other | LVX | fluoroquinolone |
| 33 | AM;A/S;AUG;AZT;CAX;CFT;CP; CRM;GM;LVX;TO;T/S | aminoglycoside;fluoroquinolone; lactam;other | LVX | fluoroquinolone |
| 34 | AM;A/S;AUG;AZT;CAX;CFT;CP; CRM;GM;LVX;TO;T/S | aminoglycoside;fluoroquinolone; lactam;other | LVX | fluoroquinolone |
| 35 | AM;A/S;AUG;AZT;CAX;CFT;CP; CRM;GM;LVX;TO;T/S | aminoglycoside;fluoroquinolone; lactam;other | LVX | fluoroquinolone |
| 36 | AM;A/S;AUG;AZT;CAX;CFT;CP; CRM;GM;LVX;TO;T/S | aminoglycoside;fluoroquinolone; lactam;other | LVX | fluoroquinolone |
| 37 | AM;A/S;AUG;AZT;CAX;CFT;CP; CRM;GM;LVX;TO;T/S | aminoglycoside;fluoroquinolone; lactam;other | LVX | fluoroquinolone |
| 38 | AM;A/S;AUG;AZT;CAX;CFT;CP; CRM;GM;LVX;TO;T/S | aminoglycoside;fluoroquinolone; lactam;other | CP | fluoroquinolone |
| 39 | AM;A/S;AUG;AZT;CAX;CFT;CP; CRM;GM;LVX;TO;T/S | aminoglycoside;fluoroquinolone; lactam;other | LVX | fluoroquinolone |
| 40 | AM;A/S;AUG;AZT;CAX;CFT;CP; CRM;GM;LVX;TO;T/S | aminoglycoside;fluoroquinolone; lactam;other | LVX | fluoroquinolone |
| 41 | AM;A/S;AUG;AZT;CAX;CFT;CP; CRM;GM;LVX;TO;T/S | aminoglycoside;fluoroquinolone; lactam;other | LVX | fluoroquinolone |
| 42 | AM;A/S;AUG;AZT;CAX;CFT;CP; CRM;GM;LVX;TO;T/S | aminoglycoside;fluoroquinolone; lactam;other | LVX | fluoroquinolone |
| 43 | AM;A/S;AUG;AZT;CAX;CFT;CP; CRM;GM;T/S | aminoglycoside;fluoroquinolone; lactam;other | T/S | other |
| 44 | AM;A/S;AUG;AZT;CAX;CFT;CP; CRM;GM;LVX;TO;T/S | aminoglycoside;fluoroquinolone; lactam;other | LVX | fluoroquinolone |
| 45 | AM;A/S;AUG;AZT;CAX;CFT;CP; CRM;GM;LVX;TO;T/S | aminoglycoside;fluoroquinolone; lactam;other | LVX | fluoroquinolone |
| 46 | AM;A/S;AUG;AZT;CAX;CFT;CP; CRM;GM;LVX;TO;T/S | aminoglycoside;fluoroquinolone; lactam;other | LVX | fluoroquinolone |
| 47 | AM;A/S;AUG;T/S | lactam;other | T/S | other |
| 48 | AM;A/S;AUG;AZT;CAX;CFT;CP; CRM;GM;LVX;TO;T/S | aminoglycoside;fluoroquinolone; lactam;other | LVX | fluoroquinolone |
| 49 | AM;A/S;AUG;AZT;CAX;CFT;CP; CRM;GM;LVX;TO;T/S | aminoglycoside;fluoroquinolone; lactam;other | LVX | fluoroquinolone |
| 50 | AM;A/S;AUG;AZT;CAX;CFT;CP; CRM;GM;LVX;TO;T/S | aminoglycoside;fluoroquinolone; lactam;other | LVX | fluoroquinolone |

Table 8c: Data for Escherichia, particularly E.coli (continued)

| No. | num_aminoglycoside | num_fluoroquinolone | num_lactam | num_other |
|---|---|---|---|---|
| 1 | 2 | 2 | 5 | 1 |

(continued)

| No. | num_aminoglycoside | num_fluoroquinolone | num_lactam | num_other |
|-----|--------------------|--------------------|------------|-----------|
| 2 | 2 | 2 | 7 | 1 |
| 3 | 2 | 2 | 7 | 1 |
| 4 | 2 | 2 | 7 | 1 |
| 5 | 2 | 2 | 7 | 1 |
| 6 | 2 | 2 | 7 | 1 |
| 7 | 2 | 2 | 7 | 1 |
| 8 | 2 | 2 | 7 | 1 |
| 9 | 2 | 2 | 7 | 1 |
| 10 | 2 | 2 | 7 | 1 |
| 11 | 2 | 2 | 7 | 1 |
| 12 | 2 | 2 | 7 | 1 |
| 13 | 2 | 2 | 7 | 1 |
| 14 | 2 | 2 | 7 | 1 |
| 15 | 2 | 2 | 7 | 1 |
| 16 | 2 | 2 | 7 | 1 |
| 17 | 2 | 2 | 7 | 1 |
| 18 | 2 | 2 | 7 | 1 |
| 19 | 2 | 2 | 7 | 1 |
| 20 | 2 | 2 | 7 | 1 |
| 21 | 2 | 2 | 7 | 1 |
| 22 | 2 | 2 | 6 | 1 |
| 23 | 1 | 2 | 7 | 1 |
| 24 | 2 | 1 | 7 | 1 |
| 25 | 2 | 2 | 6 | 1 |
| 26 | 2 | 2 | 6 | 1 |
| 27 | 1 | 2 | 6 | 1 |
| 28 | 2 | 2 | 7 | 1 |
| 29 | 2 | 2 | 7 | 1 |
| 30 | 2 | 2 | 7 | 1 |
| 31 | 2 | 2 | 7 | 1 |
| 32 | 2 | 2 | 7 | 1 |
| 33 | 2 | 2 | 7 | 1 |
| 34 | 2 | 2 | 7 | 1 |
| 35 | 2 | 2 | 7 | 1 |
| 36 | 2 | 2 | 7 | 1 |
| 37 | 2 | 2 | 7 | 1 |
| 38 | 2 | 2 | 7 | 1 |
| 39 | 2 | 2 | 7 | 1 |

(continued)

| No. | num_aminoglycoside | num_fluoroquinolone | num_lactam | num_other |
|-----|-----|-----|-----|-----|
| 40 | 2 | 2 | 7 | 1 |
| 41 | 2 | 2 | 7 | 1 |
| 42 | 2 | 2 | 7 | 1 |
| 43 | 1 | 1 | 7 | 1 |
| 44 | 2 | 2 | 7 | 1 |
| 45 | 2 | 2 | 7 | 1 |
| 46 | 2 | 2 | 7 | 1 |
| 47 | 0 | 0 | 3 | 1 |
| 48 | 2 | 2 | 7 | 1 |
| 49 | 2 | 2 | 7 | 1 |
| 50 | 2 | 2 | 7 | 1 |

Table 8d: Data for Escherichia, particularly E.coli (continued)

| No. | AM_pv_adj | A/S_pv_adj | AUG_pv_adj | AZT_pv_adj |
|-----|-----|-----|-----|-----|
| 1 | 3.50E-207 | 1.39E-175 | 2.23E-54 | 1.94E-02 |
| 2 | 3.12E-58 | 4.85E-48 | 1.56E-20 | 2.24E-14 |
| 3 | 2.85E-46 | 1.23E-39 | 2.49E-17 | 7.44E-15 |
| 4 | 2.62E-45 | 3.93E-39 | 1.12E-17 | 7.82E-14 |
| 5 | 1.01E-26 | 2.48E-22 | 5.21E-09 | 2.11E-04 |
| 6 | 4.88E-38 | 1.20E-35 | 3.67E-16 | 6.67E-10 |
| 7 | 2.35E-59 | 7.04E-49 | 2.73E-24 | 1.89E-06 |
| 8 | 2.35E-59 | 7.04E-49 | 1.31E-23 | 5.63E-06 |
| 9 | 1.84E-24 | 1.82E-23 | 1.33E-10 | 1.05E-06 |
| 10 | 2.51E-64 | 6.77E-56 | 3.88E-22 | 9.19E-04 |
| 11 | 2.79E-25 | 9.09E-23 | 1.32E-08 | 1.48E-03 |
| 12 | 2.94E-45 | 2.43E-34 | 1.15E-22 | 2.63E-14 |
| 13 | 1.58E-45 | 1.72E-34 | 1.75E-22 | 1.91E-14 |
| 14 | 3.26E-28 | 1.85E-21 | 2.00E-08 | 1.16E-05 |
| 15 | 1.44E-20 | 2.94E-18 | 5.11E-07 | 1.25E-04 |
| 16 | 6.63E-29 | 5.38E-22 | 1.16E-08 | 1.11E-05 |
| 17 | 6.41E-30 | 1.52E-22 | 9.58E-09 | 6.45E-06 |
| 18 | 4.62E-35 | 3.20E-30 | 5.19E-17 | 2.18E-07 |
| 19 | 1.82E-09 | 8.62E-11 | 2.50E-17 | 1.74E-39 |
| 20 | 3.88E-27 | 4.06E-27 | 4.62E-12 | 9.38E-06 |
| 21 | 1.09E-44 | 2.09E-39 | 1.05E-16 | 2.10E-06 |
| 22 | 2.75E-28 | 8.80E-22 | 3.79E-08 | 1.79E-02 |
| 23 | 6.10E-28 | 3.56E-20 | 2.25E-09 | 8.28E-04 |

(continued)

| No. | AM_pv_adj | A/S_pv_adj | AUG_pv_adj | AZT_pv_adj |
|---|---|---|---|---|
| 24 | 5.97E-23 | 1.19E-15 | 1.39E-06 | 1.28E-03 |
| 25 | 4.17E-03 | 3.66E-02 | 4.90E-07 | 1.65E-14 |
| 26 | 4.17E-03 | 3.66E-02 | 4.90E-07 | 1.65E-14 |
| 27 | 1.37E-28 | 1.45E-21 | 8.41E-09 | 2.36E-02 |
| 28 | 5.88E-10 | 1.33E-07 | 1.85E-09 | 7.58E-11 |
| 29 | 1.14E-08 | 2.28E-05 | 5.66E-08 | 3.43E-16 |
| 30 | 1.96E-09 | 3.67E-07 | 4.28E-09 | 1.44E-10 |
| 31 | 2.88E-09 | 5.08E-07 | 4.50E-09 | 1.51E-10 |
| 32 | 1.07E-10 | 2.85E-08 | 1.31E-06 | 4.19E-06 |
| 33 | 4.32E-08 | 6.04E-05 | 1.49E-07 | 4.44E-16 |
| 34 | 8.33E-08 | 9.79E-05 | 2.97E-07 | 4.44E-16 |
| 35 | 8.33E-08 | 9.79E-05 | 2.97E-07 | 4.44E-16 |
| 36 | 3.07E-08 | 4.70E-05 | 1.02E-07 | 7.64E-16 |
| 37 | 4.14E-12 | 7.23E-10 | 1.20E-05 | 1.17E-03 |
| 38 | 1.57E-07 | 1.55E-04 | 4.17E-07 | 7.80E-16 |
| 39 | 2.15E-07 | 1.95E-04 | 5.61E-07 | 8.13E-16 |
| 40 | 1.55E-07 | 1.54E-04 | 3.95E-07 | 8.67E-16 |
| 41 | 1.55E-07 | 1.54E-04 | 3.95E-07 | 8.67E-16 |
| 42 | 1.55E-07 | 1.54E-04 | 3.95E-07 | 8.67E-16 |
| 43 | 3.13E-28 | 1.97E-20 | 2.60E-09 | 3.55E-04 |
| 44 | 1.16E-10 | 3.41E-08 | 3.47E-07 | 2.45E-06 |
| 45 | 2.72E-09 | 3.18E-07 | 2.44E-07 | 1.12E-10 |
| 46 | 2.66E-09 | 8.84E-08 | 1.21E-05 | 1.64E-08 |
| 47 | 4.47E-24 | 6.61E-18 | 2.77E-06 | 2.45E-01 |
| 48 | 2.16E-06 | 8.47E-04 | 1.36E-06 | 7.02E-16 |
| 49 | 4.99E-06 | 1.53E-03 | 2.46E-06 | 1.26E-15 |
| 50 | 1.11E-05 | 1.86E-03 | 1.74E-06 | 1.63E-14 |

Table 8e: Data for Escherichia, particularly E.coli (continued)

| No. | CAX_pv_adj | CFT_pv_adj | CP_pv_adj | CRM_pv_adj |
|---|---|---|---|---|
| 1 | 9.08E-03 | 5.54E-02 | 2.29E-17 | 2.63E-05 |
| 2 | 4.55E-21 | 9.31E-20 | 3.14E-43 | 1.15E-19 |
| 3 | 4.33E-19 | 1.87E-17 | 1.53E-32 | 5.07E-17 |
| 4 | 6.85E-18 | 2.28E-16 | 6.99E-31 | 7.62E-16 |
| 5 | 2.25E-06 | 3.51E-06 | 1.44E-38 | 1.55E-08 |
| 6 | 3.18E-15 | 1.35E-14 | 4.48E-11 | 1.70E-12 |
| 7 | 2.34E-05 | 5.15E-06 | 1.74E-15 | 7.72E-10 |

(continued)

| No. | CAX_pv_adj | CFT_pv_adj | CP_pv_adj | CRM_pv_adj |
|---|---|---|---|---|
| 8 | 5.80E-05 | 9.14E-06 | 6.46E-15 | 2.18E-09 |
| 9 | 6.82E-10 | 3.64E-09 | 1.69E-49 | 1.37E-10 |
| 10 | 5.59E-03 | 1.88E-03 | 1.12E-09 | 2.39E-05 |
| 11 | 9.17E-05 | 2.37E-04 | 1.28E-36 | 6.73E-06 |
| 12 | 8.04E-16 | 5.19E-14 | 7.03E-61 | 9.05E-21 |
| 13 | 5.55E-16 | 2.31E-14 | 2.47E-60 | 6.03E-21 |
| 14 | 5.30E-07 | 2.10E-06 | 1.38E-39 | 8.69E-08 |
| 15 | 1.67E-06 | 3.95E-06 | 1.23E-38 | 5.25E-08 |
| 16 | 4.67E-07 | 1.87E-06 | 6.39E-39 | 5.20E-08 |
| 17 | 4.17E-07 | 1.68E-06 | 2.84E-39 | 4.28E-08 |
| 18 | 5.95E-11 | 4.86E-10 | 4.79E-07 | 1.46E-09 |
| 19 | 4.65E-46 | 9.26E-45 | 3.20E-19 | 1.06E-36 |
| 20 | 5.55E-08 | 2.77E-08 | 8.59E-09 | 1.64E-09 |
| 21 | 4.89E-09 | 9.33E-07 | 3.24E-25 | 8.32E-08 |
| 22 | 8.50E-03 | 9.80E-03 | 5.28E-05 | 4.13E-03 |
| 23 | 4.82E-05 | 7.11E-05 | 5.83E-03 | 5.89E-04 |
| 24 | 1.31E-04 | 1.77E-04 | 7.59E-03 | 2.84E-03 |
| 25 | 2.32E-15 | 2.75E-17 | 6.79E-43 | 2.56E-16 |
| 26 | 2.32E-15 | 2.75E-17 | 6.79E-43 | 2.56E-16 |
| 27 | 3.66E-03 | 4.47E-03 | 4.75E-03 | 2.48E-03 |
| 28 | 6.25E-11 | 6.54E-10 | 4.02E-42 | 2.80E-12 |
| 29 | 6.60E-17 | 2.67E-17 | 8.45E-42 | 2.23E-17 |
| 30 | 7.29E-11 | 7.76E-10 | 1.57E-41 | 5.31E-12 |
| 31 | 7.91E-11 | 8.46E-10 | 2.18E-41 | 1.70E-11 |
| 32 | 4.29E-06 | 1.52E-06 | 1.28E-40 | 1.80E-07 |
| 33 | 1.31E-16 | 5.36E-17 | 3.96E-41 | 4.26E-17 |
| 34 | 1.31E-16 | 5.36E-17 | 3.96E-41 | 4.26E-17 |
| 35 | 1.31E-16 | 5.36E-17 | 3.96E-41 | 4.26E-17 |
| 36 | 1.43E-16 | 5.77E-17 | 4.56E-41 | 1.17E-17 |
| 37 | 6.46E-05 | 1.74E-04 | 2.25E-40 | 1.80E-06 |
| 38 | 2.55E-16 | 1.06E-16 | 5.76E-41 | 7.39E-17 |
| 39 | 2.58E-16 | 1.06E-16 | 1.05E-40 | 7.84E-17 |
| 40 | 2.68E-16 | 1.10E-16 | 1.19E-40 | 2.23E-17 |
| 41 | 2.68E-16 | 1.10E-16 | 1.19E-40 | 2.23E-17 |
| 42 | 2.68E-16 | 1.10E-16 | 1.19E-40 | 2.23E-17 |
| 43 | 1.90E-05 | 2.89E-05 | 6.00E-03 | 1.99E-04 |
| 44 | 1.84E-06 | 5.25E-07 | 1.05E-39 | 6.76E-08 |
| 45 | 8.60E-10 | 4.43E-09 | 9.49E-40 | 8.85E-14 |

(continued)

| No. | CAX_pv_adj | CFT_pv_adj | CP_pv_adj | CRM_pv_adj |
|---|---|---|---|---|
| 46 | 2.36E-11 | 1.27E-10 | 4.92E-38 | 2.22E-11 |
| 47 | 1.07E-01 | 1.14E-01 | 1.50E-02 | 6.49E-02 |
| 48 | 4.02E-16 | 1.67E-16 | 1.34E-38 | 7.42E-18 |
| 49 | 4.46E-16 | 1.95E-16 | 3.53E-38 | 1.42E-17 |
| 50 | 4.97E-15 | 2.04E-15 | 7.83E-38 | 2.27E-17 |

Table 8f: Data for Escherichia, particularly E.coli (continued)

| No. | GM_pv_adj | LVX_pv_adj | TO_pv_adj | T/S_pv_adj |
|---|---|---|---|---|
| 1 | 2.40E-14 | 2.96E-16 | 6.56E-10 | 5.59E-55 |
| 2 | 9.02E-31 | 5.84E-43 | 3.02E-26 | 1.62E-119 |
| 3 | 3.09E-28 | 1.25E-33 | 1.52E-27 | 6.25E-97 |
| 4 | 1.19E-27 | 5.56E-32 | 5.30E-27 | 8.78E-96 |
| 5 | 2.33E-13 | 1.57E-38 | 6.37E-15 | 7.68E-72 |
| 6 | 4.17E-11 | 5.22E-11 | 1.79E-08 | 1.58E-71 |
| 7 | 7.23E-11 | 6.17E-14 | 8.00E-05 | 1.01E-69 |
| 8 | 2.80E-10 | 1.39E-13 | 8.00E-05 | 1.23E-68 |
| 9 | 1.18E-18 | 2.49E-49 | 2.67E-22 | 6.77E-68 |
| 10 | 7.65E-07 | 1.10E-08 | 7.07E-03 | 1.95E-64 |
| 11 | 1.21E-16 | 1.92E-37 | 4.13E-18 | 1.87E-63 |
| 12 | 4.86E-26 | 7.43E-62 | 5.25E-32 | 2.58E-39 |
| 13 | 2.72E-26 | 3.08E-61 | 2.92E-32 | 4.30E-39 |
| 14 | 1.39E-16 | 1.68E-39 | 1.59E-17 | 1.74E-60 |
| 15 | 5.78E-18 | 2.01E-39 | 1.91E-19 | 2.44E-60 |
| 16 | 1.02E-16 | 7.87E-39 | 1.17E-17 | 6.74E-60 |
| 17 | 7.38E-17 | 3.56E-39 | 8.56E-18 | 2.63E-59 |
| 18 | 6.73E-07 | 9.82E-07 | 5.94E-07 | 3.47E-47 |
| 19 | 5.34E-12 | 1.45E-19 | 7.00E-09 | 2.74E-06 |
| 20 | 3.89E-07 | 1.14E-08 | 2.09E-05 | 7.71E-46 |
| 21 | 6.04E-13 | 2.10E-24 | 3.47E-12 | 1.02E-37 |
| 22 | 8.52E-06 | 1.56E-04 | 7.54E-03 | 4.90E-44 |
| 23 | 9.39E-05 | 9.80E-03 | 1.28E-02 | 6.98E-44 |
| 24 | 2.16E-04 | 1.65E-02 | 9.49E-03 | 9.68E-44 |
| 25 | 1.81E-12 | 3.58E-43 | 2.05E-13 | 2.13E-06 |
| 26 | 1.81E-12 | 3.58E-43 | 2.05E-13 | 2.13E-06 |
| 27 | 1.08E-04 | 8.15E-03 | 5.44E-02 | 5.54E-43 |
| 28 | 1.81E-12 | 2.71E-42 | 4.67E-11 | 6.64E-17 |
| 29 | 3.65E-19 | 9.37E-42 | 2.80E-17 | 3.20E-18 |

(continued)

| No. | GM_pv_adj | LVX_pv_adj | TO_pv_adj | T/S_pv_adj |
|---|---|---|---|---|
| 30 | 3.61E-12 | 1.03E-41 | 9.37E-11 | 2.18E-16 |
| 31 | 3.86E-12 | 1.47E-41 | 9.73E-11 | 2.45E-16 |
| 32 | 1.03E-13 | 2.44E-41 | 2.22E-16 | 4.82E-04 |
| 33 | 4.79E-19 | 2.61E-41 | 5.83E-17 | 9.95E-18 |
| 34 | 4.79E-19 | 2.61E-41 | 5.83E-17 | 3.03E-17 |
| 35 | 4.79E-19 | 2.61E-41 | 5.83E-17 | 3.03E-17 |
| 36 | 8.47E-19 | 3.21E-41 | 6.04E-17 | 3.36E-18 |
| 37 | 3.56E-12 | 3.69E-41 | 8.59E-15 | 1.04E-04 |
| 38 | 8.74E-19 | 6.20E-41 | 6.42E-17 | 5.21E-17 |
| 39 | 9.35E-19 | 6.76E-41 | 6.96E-17 | 8.31E-17 |
| 40 | 1.03E-18 | 8.13E-41 | 1.27E-16 | 2.95E-17 |
| 41 | 1.03E-18 | 8.13E-41 | 1.27E-16 | 2.95E-17 |
| 42 | 1.03E-18 | 8.13E-41 | 1.27E-16 | 2.95E-17 |
| 43 | 9.79E-05 | 1.29E-02 | 1.33E-02 | 1.07E-40 |
| 44 | 1.98E-13 | 1.85E-40 | 4.73E-16 | 4.17E-04 |
| 45 | 2.68E-16 | 1.90E-40 | 1.10E-17 | 2.80E-08 |
| 46 | 1.27E-13 | 3.36E-40 | 7.14E-14 | 3.41E-07 |
| 47 | 1.15E-02 | 2.48E-02 | 4.44E-01 | 3.77E-40 |
| 48 | 1.50E-18 | 8.68E-39 | 9.76E-17 | 2.56E-15 |
| 49 | 3.08E-18 | 2.28E-38 | 1.98E-16 | 1.91E-14 |
| 50 | 2.46E-16 | 4.92E-38 | 8.76E-15 | 4.31E-13 |

Table 8g: Data for Escherichia, particularly E.coli (continued)

| No. | Locus.Tag | Gene. Symbol | GenBank.Accession | Chromosome |
|---|---|---|---|---|
| 1 | SeHA_C1580 | blaT | YP_002045448 | |
| 2 | EcolB7_01001258 | | ZP_00717086 | |
| 3 | HMPREF9345_ 05066 | | ZP_07100147 | |
| 4 | KPN_pKPN5p08201 | | YP_001338811 | plasmid pKPN5 |
| 5 | HMPREF9345_ 05232 | | ZP_07100311 | |
| 6 | EcolB_01004576 | | ZP_00708527 | |
| 7 | pEFER_0049 | | YP_002394596 | plasmid pEFER |
| 8 | ETAE_p040 | | YP_003297634 | plasmid pEIB202 |
| 9 | ECGG_04429 | | ZP_06648541 | |
| 10 | ETAE_p041 | folP | YP_003297635 | plasmid pEIB202 |

(continued)

| No. | Locus.Tag | Gene. Symbol | GenBank.Accession | Chromosome |
|---|---|---|---|---|
| 11 | ECGG_04434 | | ZP_06648545 | |
| 12 | ROD_p1051 | pemI | YP_003368450 | plasmid pCROD1 |
| 13 | ROD_p1061 | pemK | YP_003368451 | plasmid pCROD1 |
| 14 | HMPREF9348_ 05540 | | ZP_07692671 | |
| 15 | ECUMN_ 4816 | aadA | YP_002415408 | |
| 16 | ECUMN_ 4827 | mphA | YP_002415417 | |
| 17 | HMPREF9345_ 05223 | | ZP_07100302 | |
| 18 | EcSMS35_A0150 | | YP_001740030 | plasmid pSMS35_130 |
| 19 | HMPREF9548_ 03778 | | ZP_07141582 | |
| 20 | HMPREF9348_ 05561 | | ZP_07692692 | |
| 21 | HMPREF9548_ 03784 | | ZP_07141588 | |
| 22 | KPN_pKPN4p07064 | | YP_001338673 | plasmid pKPN4 |
| 23 | HMPREF9345_ 03525 | | ZP_07098656 | |
| 24 | KPN_pKPN4p07068 | merC | YP_001338677 | plasmid pKPN4 |
| 25 | ESCG_01010 | | ZP_ 04871321 | |
| 26 | HMPREF9540_ 00466 | | ZP_07133313 | |
| 27 | STMDT12_C39340 | | BAJ38877 | |
| 28 | KPK_A0022 | sopB | YP_002235674 | plasmid pKP187 |
| 29 | EcoliO157_010100014809 | | ZP_02780941 | |
| 30 | EcSMS35_A0072 | sopB | YP_001739960 | plasmid pSMS35_130 |
| 31 | HMPREF9538_00090 | | ZP_08302456 | |
| 32 | ECNA114_2444 | | AEG37278 | |
| 33 | EschericcoliO157_010100006568 | | ZP_02749755 | |
| 34 | CSAG_00128 | | ZP_04560798 | |
| 35 | ECH74115_0422 | mhpA | YP_002268988 | |
| 36 | CIT292_01335 | | ZP_03835561 | |
| 37 | ECSF_1165 | | BAI54705 | |
| 38 | EscherichiacoliO157EcO_010100009454 | | ZP_05948572 | |
| 39 | SSON_0329 | mhpD | YP_309341 | |
| 40 | CIT292_01336 | | ZP_03835562 | |
| 41 | HMPREF9540_00984 | | ZP_07133822 | |

(continued)

| No. | Locus.Tag | Gene. Symbol | GenBank.Accession | Chromosome |
|---|---|---|---|---|
| 42 | Missing locus_tag:AC_000091.cds340.373092.. 374105 | mhpE | AP_001004 | |
| 43 | HMPREF9551_04611 | | ZP_07191964 | |
| 44 | SeHA_C2638 | | YP_002046450 | |
| 45 | SFxv_0758 | | ADA73054 | |
| 46 | HMPREF9530_01431 | | ZP_07151340 | |
| 47 | KPN_pKPN4p07069 | merA | YP_001338678 | plasmid pKPN4 |
| 48 | CP0077 | | NP_858210 | plasmid pCP301 |
| 49 | SDY_P083 | | YP_406084 | plasmid pSD1_197 |
| 50 | HMPREF9540_ 03398 | | ZP_07136184 | |

Table 8h: Data for Escherichia, particularly E.coli (continued)

| No. | Start.Coord | End.Coord | Strand | Scaffold.ID |
|---|---|---|---|---|
| 1 | 1525960 | 1526820 | + | 642555218 |
| 2 | 77515 | 77910 | + | 638358454 |
| 3 | 4715 | 5554 | - | 648294624 |
| 4 | 34908 | 35255 | - | 640753021 |
| 5 | 5252 | 5572 | - | 648294635 |
| 6 | 3555 | 4568 | + | 638359041 |
| 7 | 48658 | 49503 | + | 643692014 |
| 8 | 31237 | 32040 | - | 646311959 |
| 9 | 19278 | 19826 | + | 647536609 |
| 10 | 32101 | 32916 | - | 646311959 |
| 11 | 22618 | 23823 | + | 647536609 |
| 12 | 3339 | 3596 | + | 646311988 |
| 13 | 3529 | 3930 | + | 646311988 |
| 14 | 461 | 1045 | - | 649994953 |
| 15 | 4994882 | 4995670 | + | 644736335 |
| 16 | 5003053 | 5003958 | - | 644736335 |
| 17 | 1167 | 2405 | + | 648294635 |
| 18 | 118542 | 119474 | + | 641522668 |
| 19 | 4493 | 5755 | + | 648288593 |
| 20 | 209 | 661 | + | 649994959 |
| 21 | 8819 | 9535 | + | 648288593 |
| 22 | 23310 | 24614 | - | 640753020 |

(continued)

| No. | Start.Coord | End.Coord | Strand | Scaffold.ID |
|---|---|---|---|---|
| 23 | 1071 | 1307 | - | 648294596 |
| 24 | 25970 | 27664 | - | 640753020 |
| 25 | 241682 | 242032 | + | 646206991 |
| 26 | 192 | 443 | + | 648287910 |
| 27 | 4084530 | 4085237 | - | 651053007 |
| 28 | 12106 | 13071 | - | 643348532 |
| 29 | 123136 | 123969 | - | 641781714 |
| 30 | 53664 | 54635 | - | 641522668 |
| 31 | 5657 | 6832 | + | 651332930 |
| 32 | 2476906 | 2477910 | - | 651053098 |
| 33 | 369676 | 370620 | + | 641780393 |
| 34 | 146099 | 147763 | + | 646206732 |
| 35 | 430810 | 432474 | + | 643348548 |
| 36 | 25505 | 26449 | + | 643891503 |
| 37 | 1248345 | 1249289 | + | 646862305 |
| 38 | 20015 | 20965 | + | 645964942 |
| 39 | 356486 | 357301 | + | 640427102 |
| 40 | 26463 | 27344 | + | 643891503 |
| 41 | 7204 | 8217 | + | 648287942 |
| 42 | 373092 | 374105 | + | 637000000 |
| 43 | 97 | 531 | - | 648293053 |
| 44 | 2550781 | 2552010 | + | 642555218 |
| 45 | 734541 | 734921 | + | 646862363 |
| 46 | 8 | 643 | - | 648290358 |
| 47 | 27716 | 28177 | - | 640753020 |
| 48 | 61397 | 61579 | + | 637000224 |
| 49 | 69343 | 69615 | - | 640427105 |
| 50 | 3415 | 4356 | - | 648288139 |

Examples 5 and 6: Klebsiella, particularly K. oxytoca and K. pneumoniae

The procedure was carried out as in Example 1, except that the following microorganisms were used:

Bacterial Strains

The inventors selected 1568 Klebsiella strains, particularly 1179 for Klebsiella pneumonia and 389 for Klebsiella oxytoca, from the microbiology strain collection at Siemens Healthcare Diagnostics (West Sacramento, CA) for susceptibility testing and whole genome sequencing.

From MetaRef, 1640 centroids of Klebsiella species were used as reference sequences for K. oxytoca, and 1641 centroids of K. pneumoniae were uses as reference sequences of K. pneumoniae.

The results for K. oxytoca are shown in Tables 9 (corresponding to Table 1) and 10 (corresponding to Table 2), and the results for K. pneumonia are shown in Tables 11 (corresponding to Table 1) and 12 (corresponding to Table 2).

Table 9a: Data for Klebsiella, particularly K. oxytoca

| No. | Scaffold.External. Accession | Scaffold.Name | best_pv |
|---|---|---|---|
| 1 | NC_011283 | Klebsiella pneumoniae 342: NC_011283 | 7.24545119859105e-19 |
| 2 | NZ_GG745508 | Klebsiella sp. 1_1_55 genomic scaffold supercont1.1: NZ_GG745508 | 7.24545119859105e-19 |
| 3 | NC_011283 | Klebsiella pneumoniae 342: NC_011283 | 1.17347446332913e-17 |
| 4 | NC_015663 | Enterobacter aerogenes KCTC 2190 chromosome: NC_015663 | 9.82145388713083e-17 |
| 5 | NC_013850 | Klebsiella variicola At-22 chromosome: NC_013850 | 4.06768464186916e-16 |
| 6 | NZ_ACZD01000230 | Klebsiella pneumoniae subsp. rhinoscleromatis ATCC 13884 contig00265: NZ_ACZD01000230 | 4.06768464186916e-16 |
| 7 | NC_013850 | Klebsiella variicola At-22 chromosome: NC_013850 | 6.05465029985694e-16 |
| 8 | CP002910 | Klebsiella pneumoniae KCTC 2242: CP002910 | 6.05465029985694e-16 |
| 9 | CP002910 | Klebsiella pneumoniae KCTC 2242: CP002910 | 6.05465029985694e-16 |
| 10 | NZ_GG745509 | Klebsiella sp. 1_1_55 genomic scaffold supercont1.2: NZ_GG745509 | 6.05465029985694e-16 |
| 11 | NZ_GG745508 | Klebsiella sp. 1_1_55 genomic scaffold supercont1.1: NZ_GG745508 | 6.05465029985694e-16 |
| 12 | NC_011283 | Klebsiella pneumoniae 342: NC_011283 | 6.05465029985694e-16 |
| 13 | NC_013850 | Klebsiella variicola At-22 chromosome: NC_013850 | 6.05465029985694e-16 |
| 14 | NC_013850 | Klebsiella variicola At-22 chromosome: NC_013850 | 6.05465029985694e-16 |
| 15 | NC_013850 | Klebsiella variicola At-22 chromosome: NC_013850 | 6.05465029985694e-16 |
| 16 | NC_013850 | Klebsiella variicola At-22 chromosome: NC_013850 | 6.05465029985694e-16 |
| 17 | NC_013850 | Klebsiella variicola At-22 chromosome: NC_013850 | 6.05465029985694e-16 |
| 18 | NZ_GG745512 | Klebsiella sp. 1_1_55 genomic scaffold supercont1.5: NZ_GG745512 | 6.05465029985694e-16 |
| 19 | NC_013850 | Klebsiella variicola At-22 chromosome: NC_013850 | 6.05465029985694e-16 |
| 20 | NC_013850 | Klebsiella variicola At-22 chromosome: NC_013850 | 6.05465029985694e-16 |
| 21 | NZ_GG745508 | Klebsiella sp. 1_1_55 genomic scaffold supercont1.1: NZ_GG745508 | 6.05465029985694e-16 |

(continued)

| No. | Scaffold.External. Accession | Scaffold.Name | best_pv |
|---|---|---|---|
| 22 | NC_009648 | Klebsiella pneumoniae subsp. pneumoniae MGH 78578: NC_009648 | 6.05465029985694e-16 |
| 23 | NC_013850 | Klebsiella variicola At-22 chromosome: NC_013850 | 6.05465029985694e-16 |
| 24 | NC_013850 | Klebsiella variicola At-22 chromosome: NC_013850 | 6.05465029985694e-16 |
| 25 | NC_012731 | Klebsiella pneumoniae NTUH-K2044: NC_012731 | 6.05465029985694e-16 |
| 26 | NC_013850 | Klebsiella variicola At-22 chromosome: NC_013850 | 6.05465029985694e-16 |
| 27 | NC_013850 | Klebsiella variicola At-22 chromosome: NC_013850 | 6.05465029985694e-16 |
| 28 | NZ_GG745508 | Klebsiella sp. 1_1_55 genomic scaffold supercont1.1: NZ_GG745508 | 6.05465029985694e-16 |
| 29 | NC_013850 | Klebsiella variicola At-22 chromosome: NC_013850 | 6.05465029985694e-16 |
| 30 | NC_013850 | Klebsiella variicola At-22 chromosome: NC_013850 | 6.05465029985694e-16 |
| 31 | NC_013850 | Klebsiella variicola At-22 chromosome: NC_013850 | 6.05465029985694e-16 |
| 32 | NZ_GG745508 | Klebsiella sp. 1_1_55 genomic scaffold supercont1.1: NZ_GG745508 | 6.05465029985694e-16 |
| 33 | NZ_GG745508 | Klebsiella sp. 1_1_55 genomic scaffold supercont1.1: NZ_GG745508 | 6.05465029985694e-16 |
| 34 | NC_012731 | Klebsiella pneumoniae NTUH-K2044: NC_012731 | 6.05465029985694e-16 |
| 35 | CP002910 | Klebsiella pneumoniae KCTC 2242: CP002910 | 6.05465029985694e-16 |
| 36 | NC_011283 | Klebsiella pneumoniae 342: NC_011283 | 6.05465029985694e-16 |
| 37 | NZ_AFBO01000503 | Klebsiella sp. MS 92-3 K_spMS92-3-1.0_Cont988.13: NZ_AFBO01000503 | 6.05465029985694e-16 |
| 38 | NZ_GG745509 | Klebsiella sp. 1_1_55 genomic scaffold supercont1.2: NZ_GG745509 | 6.05465029985694e-16 |
| 39 | NZ_GG745509 | Klebsiella sp. 1_1_55 genomic scaffold supercont1.2: NZ_GG745509 | 6.05465029985694e-16 |
| 40 | NC_013850 | Klebsiella variicola At-22 chromosome: NC_013850 | 6.05465029985694e-16 |
| 41 | NC_013850 | Klebsiella variicola At-22 chromosome: NC_013850 | 6.05465029985694e-16 |
| 42 | NC_013850 | Klebsiella variicola At-22 chromosome: NC_013850 | 6.05465029985694e-16 |
| 43 | NC_009648 | Klebsiella pneumoniae subsp. pneumoniae MGH 78578: NC_009648 | 6.05465029985694e-16 |

(continued)

| No. | Scaffold.External. Accession | Scaffold.Name | best_pv |
|---|---|---|---|
| 44 | NZ_GG745509 | Klebsiella sp. 1_1_55 genomic scaffold supercont1.2: NZ_GG745509 | 6.05465029985694e-16 |
| 45 | NC_013850 | Klebsiella variicola At-22 chromosome: NC_013850 | 6.05465029985694e-16 |
| 46 | NC_013850 | Klebsiella variicola At-22 chromosome: NC_013850 | 6.05465029985694e-16 |
| 47 | NZ_AFBO01000685 | Klebsiella sp. MS 92-3 K_spMS92-3-1.0_Cont1649.3: NZ_AFBO01000685 | 6.05465029985694e-16 |
| 48 | CP002910 | Klebsiella pneumoniae KCTC 2242: CP002910 | 6.05465029985694e-16 |
| 49 | NC_013850 | Klebsiella variicola At-22 chromosome: NC_013850 | 6.05465029985694e-16 |
| 50 | NC_013850 | Klebsiella variicola At-22 chromosome: NC_013850 | 6.05465029985694e-16 |

Table 9b: Data for Klebsiella, particularly K. oxytoca (continued)

| No. | sign_phenos | sign_phenos_class | best_pheno | best_pheno_class |
|---|---|---|---|---|
| 1 | A/S;AUG;CP;CRM;LVX;P/T | fluoroquinolone;lactam | A/S | lactam |
| 2 | A/S;AUG;CP;CRM;LVX;P/T | fluoroquinolone;lactam | A/S | lactam |
| 3 | A/S;AUG;CRM;LVX;P/T | fluoroquinolone;lactam | A/S | lactam |
| 4 | A/S;AUG;CP;CRM;LVX;P/T | fluoroquinolone;lactam | A/S | lactam |
| 5 | A/S;CP;LVX | fluoroquinolone;lactam | A/S | lactam |
| 6 | A/S;AUG;CP;CRM;LVX;P/T | fluoroquinolone;lactam | A/S | lactam |
| 7 | A/S;AUG;CP;CRM;LVX | fluoroquinolone;lactam | A/S | lactam |
| 8 | A/S;AUG;CP;CRM;LVX | fluoroquinolone;lactam | A/S | lactam |
| 9 | A/S;CRM;LVX | fluoroquinolone;lactam | A/S | lactam |
| 10 | A/S;AUG;CP;LVX | fluoroquinolone;lactam | A/S | lactam |
| 11 | A/S;AUG;CP;CRM;LVX | fluoroquinolone;lactam | A/S | lactam |
| 12 | A/S;AUG;CP;CRM;LVX | fluoroquinolone;lactam | A/S | lactam |
| 13 | A/S;AUG;CP;CRM;LVX | fluoroquinolone;lactam | A/S | lactam |
| 14 | A/S;AUG;CP;CRM;LVX | fluoroquinolone;lactam | A/S | lactam |
| 15 | A/S;CP;LVX | fluoroquinolone;lactam | A/S | lactam |
| 16 | A/S;AUG;CP;CRM;LVX;P/T | fluoroquinolone;lactam | A/S | lactam |
| 17 | A/S;AUG;CP;CRM;LVX | fluoroquinolone;lactam | A/S | lactam |
| 18 | A/S;AUG;CP;CRM;LVX | fluoroquinolone;lactam | A/S | lactam |
| 19 | A/S;AUG;CP;CRM;LVX | fluoroquinolone;lactam | A/S | lactam |
| 20 | A/S;AUG;CP;CRM;LVX | fluoroquinolone;lactam | A/S | lactam |
| 21 | A/S;AUG;CP;CRM;LVX | fluoroquinolone;lactam | A/S | lactam |
| 22 | A/S;AUG;CP;CRM;LVX | fluoroquinolone;lactam | A/S | lactam |

(continued)

| No. | sign_phenos | sign_phenos_class | best_pheno | best_pheno_class |
|---|---|---|---|---|
| 23 | A/S;AUG;CP;CRM;LVX | fluoroquinolone;lactam | A/S | lactam |
| 24 | A/S;AUG;CP;CRM;LVX | fluoroquinolone;lactam | A/S | lactam |
| 25 | A/S;AUG;CP;CRM;LVX | fluoroquinolone;lactam | A/S | lactam |
| 26 | A/S;AUG;CP;CRM;LVX | fluoroquinolone;lactam | A/S | lactam |
| 27 | A/S;AUG;CP;CRM;LVX | fluoroquinolone;lactam | A/S | lactam |
| 28 | A/S;AUG;CP;CRM;LVX | fluoroquinolone;lactam | A/S | lactam |
| 29 | A/S;AUG;CP;CRM;LVX | fluoroquinolone;lactam | A/S | lactam |
| 30 | A/S;AUG;CP;CRM;LVX | fluoroquinolone;lactam | A/S | lactam |
| 31 | A/S;AUG;CP;CRM;LVX | fluoroquinolone;lactam | A/S | lactam |
| 32 | A/S;AUG;CP;CRM;LVX | fluoroquinolone;lactam | A/S | lactam |
| 33 | A/S;AUG;CP;CRM;LVX | fluoroquinolone;lactam | A/S | lactam |
| 34 | A/S;AUG;CP;CRM;LVX | fluoroquinolone;lactam | A/S | lactam |
| 35 | A/S;AUG;CP;CRM;LVX | fluoroquinolone;lactam | A/S | lactam |
| 36 | A/S;AUG;CP;CRM;LVX | fluoroquinolone;lactam | A/S | lactam |
| 37 | A/S;AUG;CP;CRM;LVX | fluoroquinolone;lactam | A/S | lactam |
| 38 | A/S;AUG;CP;CRM;LVX | fluoroquinolone;lactam | A/S | lactam |
| 39 | A/S;AUG;CP;CRM;LVX | fluoroquinolone;lactam | A/S | lactam |
| 40 | A/S;CRM;LVX | fluoroquinolone;lactam | A/S | lactam |
| 41 | A/S;AUG;CP;CRM;LVX | fluoroquinolone;lactam | A/S | lactam |
| 42 | A/S;AUG;CP;CRM;LVX | fluoroquinolone;lactam | A/S | lactam |
| 43 | A/S;AUG;CP;CRM;LVX | fluoroquinolone;lactam | A/S | lactam |
| 44 | A/S;CRM;LVX | fluoroquinolone;lactam | A/S | lactam |
| 45 | A/S;AUG;CP;CRM;LVX | fluoroquinolone;lactam | A/S | lactam |
| 46 | A/S;AUG;CP;CRM;LVX | fluoroquinolone;lactam | A/S | lactam |
| 47 | A/S;CP;LVX | fluoroquinolone;lactam | A/S | lactam |
| 48 | A/S;CRM;LVX | fluoroquinolone;lactam | A/S | lactam |
| 49 | A/S;AUG;CP;CRM;LVX | fluoroquinolone;lactam | A/S | lactam |
| 50 | A/S;CRM;LVX | fluoroquinolone;lactam | A/S | lactam |

Table 9c: Data for Klebsiella, particularly K. oxytoca (continued)

| No. | num_fluoroquinolone | num_lactam |
|---|---|---|
| 1 | 2 | 4 |
| 2 | 2 | 4 |
| 3 | 1 | 4 |
| 4 | 2 | 4 |
| 5 | 2 | 1 |
| 6 | 2 | 4 |

(continued)

| No. | num_fluoroquinolone | num_lactam |
|---|---|---|
| 7 | 2 | 3 |
| 8 | 2 | 3 |
| 9 | 1 | 2 |
| 10 | 2 | 2 |
| 11 | 2 | 3 |
| 12 | 2 | 3 |
| 13 | 2 | 3 |
| 14 | 2 | 3 |
| 15 | 2 | 1 |
| 16 | 2 | 4 |
| 17 | 2 | 3 |
| 18 | 2 | 3 |
| 19 | 2 | 3 |
| 20 | 2 | 3 |
| 21 | 2 | 3 |
| 22 | 2 | 3 |
| 23 | 2 | 3 |
| 24 | 2 | 3 |
| 25 | 2 | 3 |
| 26 | 2 | 3 |
| 27 | 2 | 3 |
| 28 | 2 | 3 |
| 29 | 2 | 3 |
| 30 | 2 | 3 |
| 31 | 2 | 3 |
| 32 | 2 | 3 |
| 33 | 2 | 3 |
| 34 | 2 | 3 |
| 35 | 2 | 3 |
| 36 | 2 | 3 |
| 37 | 2 | 3 |
| 38 | 2 | 3 |
| 39 | 2 | 3 |
| 40 | 1 | 2 |
| 41 | 2 | 3 |
| 42 | 2 | 3 |
| 43 | 2 | 3 |
| 44 | 1 | 2 |

(continued)

| No. | num_fluoroquinolone | num_lactam |
|-----|---------------------|------------|
| 45 | 2 | 3 |
| 46 | 2 | 3 |
| 47 | 2 | 1 |
| 48 | 1 | 2 |
| 49 | 2 | 3 |
| 50 | 1 | 2 |

Table 9d: Data for Klebsiella, particularly K. oxytoca (continued)

| No. | A/S_pv_adj | AUG_pv_adj | AZT_pv_adj | CAX_pv_adj | CFT_pv_adj |
|-----|-----------|-----------|-----------|-----------|-----------|
| 1 | 7.25E-19 | 1.72E-03 | 1.02E-01 | 7.63E-02 | 1.14E-01 |
| 2 | 7.25E-19 | 1.72E-03 | 1.02E-01 | 7.63E-02 | 1.14E-01 |
| 3 | 1.17E-17 | 2.11E-03 | 1.25E-02 | 1.67E-02 | 3.76E-02 |
| 4 | 9.82E-17 | 1.48E-03 | 1.97E-02 | 1.37E-02 | 6.22E-02 |
| 5 | 4.07E-16 | 1.01E-02 | 2.64E-02 | 3.46E-02 | 3.40E-02 |
| 6 | 4.07E-16 | 2.92E-04 | 3.51E-02 | 2.48E-02 | 4.69E-02 |
| 7 | 6.05E-16 | 7.34E-03 | 3.46E-02 | 2.50E-02 | 2.46E-02 |
| 8 | 6.05E-16 | 7.34E-03 | 3.46E-02 | 2.50E-02 | 2.46E-02 |
| 9 | 6.05E-16 | 1.93E-02 | 7.20E-02 | 7.25E-02 | 5.85E-02 |
| 10 | 6.05E-16 | 9.84E-03 | 4.51E-02 | 3.38E-02 | 2.50E-02 |
| 11 | 6.05E-16 | 7.34E-03 | 3.46E-02 | 2.50E-02 | 2.46E-02 |
| 12 | 6.05E-16 | 7.34E-03 | 3.46E-02 | 2.50E-02 | 2.46E-02 |
| 13 | 6.05E-16 | 7.34E-03 | 3.46E-02 | 2.50E-02 | 2.46E-02 |
| 14 | 6.05E-16 | 7.34E-03 | 3.46E-02 | 2.50E-02 | 2.46E-02 |
| 15 | 6.05E-16 | 1.01E-02 | 4.61E-02 | 3.45E-02 | 3.46E-02 |
| 16 | 6.05E-16 | 5.13E-03 | 2.58E-02 | 1.89E-02 | 1.75E-02 |
| 17 | 6.05E-16 | 7.34E-03 | 3.46E-02 | 2.50E-02 | 2.46E-02 |
| 18 | 6.05E-16 | 7.34E-03 | 3.46E-02 | 2.50E-02 | 2.46E-02 |
| 19 | 6.05E-16 | 7.34E-03 | 3.46E-02 | 2.50E-02 | 2.46E-02 |
| 20 | 6.05E-16 | 7.34E-03 | 3.46E-02 | 2.50E-02 | 2.46E-02 |
| 21 | 6.05E-16 | 7.34E-03 | 3.46E-02 | 2.50E-02 | 2.46E-02 |
| 22 | 6.05E-16 | 7.34E-03 | 3.46E-02 | 2.50E-02 | 2.46E-02 |
| 23 | 6.05E-16 | 7.34E-03 | 3.46E-02 | 2.50E-02 | 2.46E-02 |
| 24 | 6.05E-16 | 7.34E-03 | 3.46E-02 | 2.50E-02 | 2.46E-02 |
| 25 | 6.05E-16 | 5.42E-03 | 3.46E-02 | 1.98E-02 | 2.46E-02 |
| 26 | 6.05E-16 | 7.34E-03 | 3.46E-02 | 2.50E-02 | 2.46E-02 |
| 27 | 6.05E-16 | 7.34E-03 | 3.46E-02 | 2.50E-02 | 2.46E-02 |
| 28 | 6.05E-16 | 7.34E-03 | 3.46E-02 | 2.50E-02 | 2.46E-02 |

(continued)

| No. | A/S_pv_adj | AUG_pv_adj | AZT_pv_adj | CAX_pv_adj | CFT_pv_adj |
|---|---|---|---|---|---|
| 29 | 6.05E-16 | 7.34E-03 | 3.46E-02 | 2.50E-02 | 2.46E-02 |
| 30 | 6.05E-16 | 7.34E-03 | 3.46E-02 | 2.50E-02 | 2.46E-02 |
| 31 | 6.05E-16 | 7.34E-03 | 3.46E-02 | 2.50E-02 | 2.46E-02 |
| 32 | 6.05E-16 | 7.34E-03 | 3.46E-02 | 2.50E-02 | 2.46E-02 |
| 33 | 6.05E-16 | 7.34E-03 | 3.46E-02 | 2.50E-02 | 2.46E-02 |
| 34 | 6.05E-16 | 7.34E-03 | 3.46E-02 | 2.50E-02 | 2.46E-02 |
| 35 | 6.05E-16 | 7.34E-03 | 3.46E-02 | 2.50E-02 | 2.46E-02 |
| 36 | 6.05E-16 | 7.34E-03 | 3.46E-02 | 2.50E-02 | 2.46E-02 |
| 37 | 6.05E-16 | 7.34E-03 | 3.46E-02 | 2.50E-02 | 2.46E-02 |
| 38 | 6.05E-16 | 7.34E-03 | 3.46E-02 | 2.50E-02 | 2.46E-02 |
| 39 | 6.05E-16 | 7.34E-03 | 3.46E-02 | 2.50E-02 | 2.46E-02 |
| 40 | 6.05E-16 | 1.96E-02 | 7.24E-02 | 9.30E-02 | 5.85E-02 |
| 41 | 6.05E-16 | 7.42E-03 | 3.51E-02 | 2.53E-02 | 2.48E-02 |
| 42 | 6.05E-16 | 7.34E-03 | 3.46E-02 | 2.50E-02 | 2.46E-02 |
| 43 | 6.05E-16 | 7.42E-03 | 3.51E-02 | 2.53E-02 | 2.48E-02 |
| 44 | 6.05E-16 | 1.96E-02 | 7.24E-02 | 9.30E-02 | 5.85E-02 |
| 45 | 6.05E-16 | 7.34E-03 | 3.46E-02 | 2.50E-02 | 2.46E-02 |
| 46 | 6.05E-16 | 7.42E-03 | 3.51E-02 | 2.53E-02 | 2.48E-02 |
| 47 | 6.05E-16 | 1.84E-02 | 7.73E-02 | 5.74E-02 | 4.41E-02 |
| 48 | 6.05E-16 | 1.96E-02 | 7.24E-02 | 9.30E-02 | 5.85E-02 |
| 49 | 6.05E-16 | 7.34E-03 | 3.46E-02 | 2.50E-02 | 2.46E-02 |
| 50 | 6.05E-16 | 1.96E-02 | 7.24E-02 | 9.30E-02 | 5.85E-02 |

Table 9e: Data for Klebsiella, particularly K. oxytoca (continued)

| No. | CP_pv_adj | CRM_pv_adj | LVX_pv_adj | P/T_pv_adj |
|---|---|---|---|---|
| 1 | 4.47E-03 | 3.22E-03 | 3.33E-05 | 4.21E-03 |
| 2 | 4.47E-03 | 3.22E-03 | 3.33E-05 | 4.21E-03 |
| 3 | 4.19E-02 | 3.50E-03 | 2.38E-03 | 7.73E-03 |
| 4 | 2.12E-04 | 4.16E-03 | 5.03E-07 | 3.38E-03 |
| 5 | 2.11E-03 | 1.38E-02 | 6.28E-05 | 1.93E-02 |
| 6 | 6.71E-04 | 1.62E-03 | 8.24E-07 | 2.85E-03 |
| 7 | 1.30E-03 | 7.85E-03 | 5.53E-05 | 1.37E-02 |
| 8 | 1.30E-03 | 7.85E-03 | 5.53E-05 | 1.37E-02 |
| 9 | 6.62E-02 | 2.94E-03 | 1.50E-03 | 3.78E-02 |
| 10 | 1.98E-03 | 1.03E-02 | 5.68E-05 | 1.89E-02 |
| 11 | 1.30E-03 | 7.85E-03 | 5.53E-05 | 1.37E-02 |
| 12 | 1.30E-03 | 7.85E-03 | 5.53E-05 | 1.37E-02 |

(continued)

| No. | CP_pv_adj | CRM_pv_adj | LVX_pv_adj | P/T_pv_adj |
|---|---|---|---|---|
| 13 | 1.30E-03 | 7.85E-03 | 5.53E-05 | 1.37E-02 |
| 14 | 1.30E-03 | 7.85E-03 | 5.53E-05 | 1.37E-02 |
| 15 | 1.26E-03 | 1.04E-02 | 3.08E-05 | 1.93E-02 |
| 16 | 7.58E-04 | 6.15E-03 | 5.93E-05 | 9.93E-03 |
| 17 | 1.30E-03 | 7.85E-03 | 5.53E-05 | 1.37E-02 |
| 18 | 1.30E-03 | 7.85E-03 | 5.53E-05 | 1.37E-02 |
| 19 | 1.30E-03 | 7.85E-03 | 5.53E-05 | 1.37E-02 |
| 20 | 1.30E-03 | 7.85E-03 | 5.53E-05 | 1.37E-02 |
| 21 | 1.30E-03 | 7.85E-03 | 5.53E-05 | 1.37E-02 |
| 22 | 1.30E-03 | 7.85E-03 | 5.53E-05 | 1.37E-02 |
| 23 | 1.30E-03 | 7.85E-03 | 5.53E-05 | 1.37E-02 |
| 24 | 1.30E-03 | 7.85E-03 | 5.53E-05 | 1.37E-02 |
| 25 | 3.39E-03 | 6.15E-03 | 1.55E-04 | 1.03E-02 |
| 26 | 1.30E-03 | 7.85E-03 | 5.53E-05 | 1.37E-02 |
| 27 | 1.30E-03 | 7.85E-03 | 5.53E-05 | 1.37E-02 |
| 28 | 1.30E-03 | 7.85E-03 | 5.53E-05 | 1.37E-02 |
| 29 | 1.30E-03 | 7.85E-03 | 5.53E-05 | 1.37E-02 |
| 30 | 1.30E-03 | 7.85E-03 | 5.53E-05 | 1.37E-02 |
| 31 | 1.30E-03 | 7.85E-03 | 5.53E-05 | 1.37E-02 |
| 32 | 1.30E-03 | 7.85E-03 | 5.53E-05 | 1.37E-02 |
| 33 | 1.30E-03 | 7.85E-03 | 5.53E-05 | 1.37E-02 |
| 34 | 1.30E-03 | 7.85E-03 | 5.53E-05 | 1.37E-02 |
| 35 | 1.30E-03 | 7.85E-03 | 5.53E-05 | 1.37E-02 |
| 36 | 1.30E-03 | 7.85E-03 | 5.53E-05 | 1.37E-02 |
| 37 | 1.30E-03 | 7.85E-03 | 5.53E-05 | 1.37E-02 |
| 38 | 1.30E-03 | 7.85E-03 | 5.53E-05 | 1.37E-02 |
| 39 | 1.30E-03 | 7.85E-03 | 5.53E-05 | 1.37E-02 |
| 40 | 9.00E-02 | 4.08E-03 | 1.50E-03 | 4.97E-02 |
| 41 | 1.97E-03 | 7.93E-03 | 5.53E-05 | 1.39E-02 |
| 42 | 1.30E-03 | 7.85E-03 | 5.53E-05 | 1.37E-02 |
| 43 | 1.97E-03 | 7.93E-03 | 5.53E-05 | 1.39E-02 |
| 44 | 9.00E-02 | 4.08E-03 | 1.50E-03 | 4.97E-02 |
| 45 | 1.30E-03 | 7.85E-03 | 5.53E-05 | 1.37E-02 |
| 46 | 1.97E-03 | 7.93E-03 | 5.53E-05 | 1.39E-02 |
| 47 | 7.43E-03 | 1.01E-02 | 2.47E-04 | 3.31E-02 |
| 48 | 9.00E-02 | 4.08E-03 | 1.50E-03 | 4.97E-02 |
| 49 | 1.30E-03 | 7.85E-03 | 5.53E-05 | 1.37E-02 |
| 50 | 9.00E-02 | 4.08E-03 | 1.50E-03 | 4.97E-02 |

Table 9f: Data for Klebsiella, particularly K. oxytoca (continued)

| No. | Locus.Tag | Gene. Symbol | GenBank.Accession | Chromosome |
|---|---|---|---|---|
| 1 | KPK_2590 | | YP_002238421 | |
| 2 | HMPREF0485_ 01216 | | ZP_06548816 | |
| 3 | KPK_0292 | | YP_002236174 | |
| 4 | EAE_11985 | | YP_004592590 | |
| 5 | Kvar_2784 | | YP_003439702 | |
| 6 | HMPREF0484_ 4675 | add | ZP_06017656 | |
| 7 | Kvar_2381 | | YP_003439305 | |
| 8 | KPN2242_ 04295 | | AEJ96779 | |
| 9 | KPN2242_ 08680 | | AEJ97652 | |
| 10 | HMPREF0485_01916 | | ZP_06549516 | |
| 11 | HMPREF0485_ 01247 | | ZP_06548847 | |
| 12 | KPK_2622 | | YP_002238453 | |
| 13 | Kvar_2568 | | YP_003439490 | |
| 14 | Kvar_0816 | | YP_003437758 | |
| 15 | Kvar_4643 | | YP_003441547 | |
| 16 | Kvar_3771 | | YP_003440683 | |
| 17 | Kvar_1596 | | YP_003438533 | |
| 18 | HMPREF0485_03998 | | ZP_06551594 | |
| 19 | Kvar_2140 | | YP_003439069 | |
| 20 | Kvar_3380 | | YP_003440292 | |
| 21 | HMPREF0485_00446 | | ZP_06548046 | |
| 22 | KPN_00275 | | YP_001333958 | |
| 23 | Kvar_1589 | | YP_003438526 | |
| 24 | Kvar_1856 | | YP_003438789 | |
| 25 | KP1_3016 | | YP_002919724 | |
| 26 | Kvar_5099 | | YP_003442002 | |
| 27 | Kvar_0479 | | YP_003437421 | |
| 28 | HMPREF0485_ 00450 | | ZP_06548050 | |
| 29 | Kvar_1586 | | YP_003438523 | |
| 30 | Kvar_1598 | | YP_003438535 | |
| 31 | Kvar_1597 | | YP_003438534 | |
| 32 | HMPREF0485_00443 | | ZP_06548043 | |
| 33 | HMPREF0485_ 00453 | | ZP_06548053 | |
| 34 | KP1_2851 | | YP_002919569 | |
| 35 | KPN2242_ 01120 | | AEJ96149 | |
| 36 | KPK_2936 | | YP_002238762 | |
| 37 | HMPREF9538_03901 | | ZP_08306208 | |

(continued)

| No. | Locus.Tag | Gene. Symbol | GenBank.Accession | Chromosome |
|---|---|---|---|---|
| 38 | HMPREF0485_02477 | | ZP_06550077 | |
| 39 | HMPREF0485_01831 | | ZP_06549431 | |
| 40 | Kvar_3258 | | YP_003440171 | |
| 41 | Kvar_3395 | | YP_003440307 | |
| 42 | Kvar_3210 | | YP_003440127 | |
| 43 | KPN_00277 | | YP_001333960 | |
| 44 | HMPREF0485_ 02605 | | ZP_06550204 | |
| 45 | Kvar_1604 | | YP_003438541 | |
| 46 | Kvar_4545 | | YP_003441451 | |
| 47 | HMPREF9538_05113 | | ZP_08307402 | |
| 48 | KPN2242_ 08695 | | AEJ97655 | |
| 49 | Kvar_4742 | | YP_003441646 | |
| 50 | Kvar_3261 | | YP_003440174 | |

Table 9g: Data for Klebsiella, particularly K. oxytoca (continued)

| No. | Start.Coord | End.Coord | Strand | Scaffold.ID |
|---|---|---|---|---|
| 1 | 2648871 | 2649476 | + | 643348533 |
| 2 | 1355295 | 1356167 | - | 647536389 |
| 3 | 321413 | 321955 | - | 643348533 |
| 4 | 2576463 | 2577677 | - | 650716122 |
| 5 | 2889784 | 2890686 | - | 646312028 |
| 6 | 12390 | 13076 | + | 647009977 |
| 7 | 2489613 | 2491040 | + | 646312028 |
| 8 | 925860 | 926771 | - | 651053122 |
| 9 | 1837699 | 1838718 | + | 651053122 |
| 10 | 237063 | 237326 | + | 647536390 |
| 11 | 1387256 | 1388314 | + | 647536389 |
| 12 | 2680912 | 2681727 | + | 643348533 |
| 13 | 2679911 | 2680687 | + | 646312028 |
| 14 | 851698 | 852411 | + | 646312028 |
| 15 | 4934923 | 4936932 | + | 646312028 |
| 16 | 3964905 | 3966425 | + | 646312028 |
| 17 | 1721712 | 1722182 | - | 646312028 |
| 18 | 140912 | 141796 | + | 647536393 |
| 19 | 2255010 | 2255996 | + | 646312028 |
| 20 | 3519306 | 3520322 | - | 646312028 |
| 21 | 523538 | 524377 | - | 647536389 |

(continued)

| No. | Start.Coord | End.Coord | Strand | Scaffold.ID |
|-----|-------------|-----------|--------|-------------|
| 22 | 311624 | 312142 | + | 640753018 |
| 23 | 1716249 | 1716779 | + | 646312028 |
| 24 | 1971206 | 1973212 | - | 646312028 |
| 25 | 2878963 | 2879583 | + | 646564530 |
| 26 | 5455245 | 5456423 | - | 646312028 |
| 27 | 509672 | 510736 | + | 646312028 |
| 28 | 528513 | 529175 | - | 647536389 |
| 29 | 1711303 | 1712709 | - | 646312028 |
| 30 | 1723564 | 1724937 | - | 646312028 |
| 31 | 1722169 | 1723554 | - | 646312028 |
| 32 | 520902 | 521159 | - | 647536389 |
| 33 | 531062 | 532510 | - | 647536389 |
| 34 | 2717504 | 2718439 | + | 646564530 |
| 35 | 230663 | 231895 | - | 651053122 |
| 36 | 2983904 | 2984626 | + | 643348533 |
| 37 | 15395 | 16450 | + | 651333420 |
| 38 | 872347 | 873972 | + | 647536390 |
| 39 | 149922 | 150884 | - | 647536390 |
| 40 | 3387706 | 3389424 | - | 646312028 |
| 41 | 3535340 | 3535786 | + | 646312028 |
| 42 | 3340882 | 3341376 | - | 646312028 |
| 43 | 312969 | 315452 | + | 640753018 |
| 44 | 1009891 | 1010898 | + | 647536390 |
| 45 | 1730820 | 1734335 | + | 646312028 |
| 46 | 4822901 | 4823620 | + | 646312028 |
| 47 | 52 | 390 | - | 651333602 |
| 48 | 1839612 | 1840562 | + | 651053122 |
| 49 | 5039066 | 5040004 | + | 646312028 |
| 50 | 3391540 | 3392502 | + | 646312028 |

Table 10a: Data for Klebsiella, particularly K. oxytoca

| No. | Scaffold.External.Accession | Scaffold.Name | best_pv |
|-----|------------------------------|---------------|---------|
| 1 | NC_011283 | Klebsiella pneumoniae 342: NC_011283 | 7.24545119859105e-19 |
| 2 | NZ_GG745508 | Klebsiella sp. 1_1_55 genomic scaffold supercont1.1: NZ_GG745508 | 7.24545119859105e-19 |
| 3 | NC_015663 | Enterobacter aerogenes KCTC 2190 chromosome: NC_015663 | 9.82145388713083e-17 |

(continued)

| No. | Scaffold.External. Accession | Scaffold.Name | best_pv |
|---|---|---|---|
| 4 | NC_013850 | Klebsiella variicola At-22 chromosome: NC_013850 | 4.06768464186916e-16 |
| 5 | NZ_ACZD01000230 | Klebsiella pneumoniae subsp. rhinoscleromatis ATCC 13884 contig00265: NZ_ACZD01000230 | 4.06768464186916e-16 |
| 6 | NC_013850 | Klebsiella variicola At-22 chromosome: NC_013850 | 6.05465029985694e-16 |
| 7 | CP002910 | Klebsiella pneumoniae KCTC 2242: CP002910 | 6.05465029985694e-16 |
| 8 | NZ_GG745509 | Klebsiella sp. 1_1_55 genomic scaffold supercont1.2: NZ_GG745509 | 6.05465029985694e-16 |
| 9 | NZ_GG745508 | Klebsiella sp. 1_1_55 genomic scaffold supercont1.1: NZ_GG745508 | 6.05465029985694e-16 |
| 10 | NC_011283 | Klebsiella pneumoniae 342: NC_011283 | 6.05465029985694e-16 |
| 11 | NC_013850 | Klebsiella variicola At-22 chromosome: NC_013850 | 6.05465029985694e-16 |
| 12 | NC_013850 | Klebsiella variicola At-22 chromosome: NC_013850 | 6.05465029985694e-16 |
| 13 | NC_013850 | Klebsiella variicola At-22 chromosome: NC_013850 | 6.05465029985694e-16 |
| 14 | NC_013850 | Klebsiella variicola At-22 chromosome: NC_013850 | 6.05465029985694e-16 |
| 15 | NC_013850 | Klebsiella variicola At-22 chromosome: NC_013850 | 6.05465029985694e-16 |
| 16 | NZ_GG745512 | Klebsiella sp. 1_1_55 genomic scaffold supercont1.5: NZ_GG745512 | 6.05465029985694e-16 |
| 17 | NC_013850 | Klebsiella variicola At-22 chromosome: NC_013850 | 6.05465029985694e-16 |
| 18 | NC_013850 | Klebsiella variicola At-22 chromosome: NC_013850 | 6.05465029985694e-16 |
| 19 | NZ_GG745508 | Klebsiella sp. 1_1_55 genomic scaffold supercont1.1: NZ_GG745508 | 6.05465029985694e-16 |
| 20 | NC_009648 | Klebsiella pneumoniae subsp. pneumoniae MGH 78578: NC_009648 | 6.05465029985694e-16 |
| 21 | NC_013850 | Klebsiella variicola At-22 chromosome: NC_013850 | 6.05465029985694e-16 |
| 22 | NC_013850 | Klebsiella variicola At-22 chromosome: NC_013850 | 6.05465029985694e-16 |
| 23 | NC_012731 | Klebsiella pneumoniae NTUH-K2044: NC_012731 | 6.05465029985694e-16 |
| 24 | NC_013850 | Klebsiella variicola At-22 chromosome: NC_013850 | 6.05465029985694e-16 |
| 25 | NC_013850 | Klebsiella variicola At-22 chromosome: NC_013850 | 6.05465029985694e-16 |

(continued)

| No. | Scaffold.External. Accession | Scaffold.Name | best_pv |
|---|---|---|---|
| 26 | NZ_GG745508 | Klebsiella sp. 1_1_55 genomic scaffold supercont1.1: NZ_GG745508 | 6.05465029985694e-16 |
| 27 | NC_013850 | Klebsiella variicola At-22 chromosome: NC_013850 | 6.05465029985694e-16 |
| 28 | NC_013850 | Klebsiella variicola At-22 chromosome: NC_013850 | 6.05465029985694e-16 |
| 29 | NC_013850 | Klebsiella variicola At-22 chromosome: NC_013850 | 6.05465029985694e-16 |
| 30 | NZ_GG745508 | Klebsiella sp. 1_1_55 genomic scaffold supercont1.1: NZ_GG745508 | 6.05465029985694e-16 |
| 31 | NZ_GG745508 | Klebsiella sp. 1_1_55 genomic scaffold supercont1.1: NZ_GG745508 | 6.05465029985694e-16 |
| 32 | NC_012731 | Klebsiella pneumoniae NTUH-K2044: NC_012731 | 6.05465029985694e-16 |
| 33 | CP002910 | Klebsiella pneumoniae KCTC 2242: CP002910 | 6.05465029985694e-16 |
| 34 | NC_011283 | Klebsiella pneumoniae 342: NC_011283 | 6.05465029985694e-16 |
| 35 | NZ_AFBO01000503 | Klebsiella sp. MS 92-3 K_spMS92-3-1.0_Cont988.13: NZ_AFBO01000503 | 6.05465029985694e-16 |
| 36 | NZ_GG745509 | Klebsiella sp. 1_1_55 genomic scaffold supercont1.2: NZ_GG745509 | 6.05465029985694e-16 |
| 37 | NZ_GG745509 | Klebsiella sp. 1_1_55 genomic scaffold supercont1.2: NZ_GG745509 | 6.05465029985694e-16 |
| 38 | NC_013850 | Klebsiella variicola At-22 chromosome: NC_013850 | 6.05465029985694e-16 |
| 39 | NC_013850 | Klebsiella variicola At-22 chromosome: NC_013850 | 6.05465029985694e-16 |
| 40 | NC_009648 | Klebsiella pneumoniae subsp. pneumoniae MGH 78578: NC_009648 | 6.05465029985694e-16 |
| 41 | NC_013850 | Klebsiella variicola At-22 chromosome: NC_013850 | 6.05465029985694e-16 |
| 42 | NC_013850 | Klebsiella variicola At-22 chromosome: NC_013850 | 6.05465029985694e-16 |
| 43 | NZ_AFBO01000685 | Klebsiella sp. MS 92-3 K_spMS92-3-1.0_Cont1649.3: NZ_AFBO01000685 | 6.05465029985694e-16 |
| 44 | NC_013850 | Klebsiella variicola At-22 chromosome: NC_013850 | 6.05465029985694e-16 |
| 45 | NC_013850 | Klebsiella variicola At-22 chromosome: NC_013850 | 6.05465029985694e-16 |
| 46 | NC_013850 | Klebsiella variicola At-22 chromosome: NC_013850 | 6.05465029985694e-16 |
| 47 | NC_009648 | Klebsiella pneumoniae subsp. pneumoniae MGH 78578: NC_009648 | 6.05465029985694e-16 |

(continued)

| No. | Scaffold.External. Accession | Scaffold.Name | best_pv |
|---|---|---|---|
| 48 | NC_011283 | Klebsiella pneumoniae 342: NC_011283 | 6.05465029985694e-16 |
| 49 | NC_014500 | Dickeya dadantii 3937 chromosome: NC_014500 | 6.05465029985694e-16 |
| 50 | NC_011283 | Klebsiella pneumoniae 342: NC_011283 | 6.05465029985694e-16 |

Table 10b: Data for Klebsiella, particularly K. oxytoca (continued)

| No. | sign_phenos | sign_phenos_class | best_pheno | best_pheno_class |
|---|---|---|---|---|
| 1 | A/S;AUG;CP;CRM;LVX;P/T | fluoroquinolone;lactam | A/S | lactam |
| 2 | A/S;AUG;CP;CRM;LVX;P/T | fluoroquinolone;lactam | A/S | lactam |
| 3 | A/S;AUG;CP;CRM;LVX;P/T | fluoroquinolone;lactam | A/S | lactam |
| 4 | A/S;CP;LVX | fluoroquinolone;lactam | A/S | lactam |
| 5 | A/S;AUG;CP;CRM;LVX;P/T | fluoroquinolone;lactam | A/S | lactam |
| 6 | A/S;AUG;CP;CRM;LVX | fluoroquinolone;lactam | A/S | lactam |
| 7 | A/S;AUG;CP;CRM;LVX | fluoroquinolone;lactam | A/S | lactam |
| 8 | A/S;AUG;CP;LVX | fluoroquinolone;lactam | A/S | lactam |
| 9 | A/S;AUG;CP;CRM;LVX | fluoroquinolone;lactam | A/S | lactam |
| 10 | A/S;AUG;CP;CRM;LVX | fluoroquinolone;lactam | A/S | lactam |
| 11 | A/S;AUG;CP;CRM;LVX | fluoroquinolone;lactam | A/S | lactam |
| 12 | A/S;AUG;CP;CRM;LVX | fluoroquinolone;lactam | A/S | lactam |
| 13 | A/S;CP;LVX | fluoroquinolone;lactam | A/S | lactam |
| 14 | A/S;AUG;CP;CRM;LVX;P/T | fluoroquinolone;lactam | A/S | lactam |
| 15 | A/S;AUG;CP;CRM;LVX | fluoroquinolone;lactam | A/S | lactam |
| 16 | A/S;AUG;CP;CRM;LVX | fluoroquinolone;lactam | A/S | lactam |
| 17 | A/S;AUG;CP;CRM;LVX | fluoroquinolone;lactam | A/S | lactam |
| 18 | A/S;AUG;CP;CRM;LVX | fluoroquinolone;lactam | A/S | lactam |
| 19 | A/S;AUG;CP;CRM;LVX | fluoroquinolone;lactam | A/S | lactam |
| 20 | A/S;AUG;CP;CRM;LVX | fluoroquinolone;lactam | A/S | lactam |
| 21 | A/S;AUG;CP;CRM;LVX | fluoroquinolone;lactam | A/S | lactam |
| 22 | A/S;AUG;CP;CRM;LVX | fluoroquinolone;lactam | A/S | lactam |
| 23 | A/S;AUG;CP;CRM;LVX | fluoroquinolone;lactam | A/S | lactam |
| 24 | A/S;AUG;CP;CRM;LVX | fluoroquinolone;lactam | A/S | lactam |
| 25 | A/S;AUG;CP;CRM;LVX | fluoroquinolone;lactam | A/S | lactam |
| 26 | A/S;AUG;CP;CRM;LVX | fluoroquinolone;lactam | A/S | lactam |
| 27 | A/S;AUG;CP;CRM;LVX | fluoroquinolone;lactam | A/S | lactam |
| 28 | A/S;AUG;CP;CRM;LVX | fluoroquinolone;lactam | A/S | lactam |
| 29 | A/S;AUG;CP;CRM;LVX | fluoroquinolone;lactam | A/S | lactam |

(continued)

| No. | sign_phenos | sign_phenos_class | best_pheno | best_pheno_class |
|---|---|---|---|---|
| 30 | A/S;AUG;CP;CRM;LVX | fluoroquinolone;lactam | A/S | lactam |
| 31 | A/S;AUG;CP;CRM;LVX | fluoroquinolone;lactam | A/S | lactam |
| 32 | A/S;AUG;CP;CRM;LVX | fluoroquinolone;lactam | A/S | lactam |
| 33 | A/S;AUG;CP;CRM;LVX | fluoroquinolone;lactam | A/S | lactam |
| 34 | A/S;AUG;CP;CRM;LVX | fluoroquinolone;lactam | A/S | lactam |
| 35 | A/S;AUG;CP;CRM;LVX | fluoroquinolone;lactam | A/S | lactam |
| 36 | A/S;AUG;CP;CRM;LVX | fluoroquinolone;lactam | A/S | lactam |
| 37 | A/S;AUG;CP;CRM;LVX | fluoroquinolone;lactam | A/S | lactam |
| 38 | A/S;AUG;CP;CRM;LVX | fluoroquinolone;lactam | A/S | lactam |
| 39 | A/S;AUG;CP;CRM;LVX | fluoroquinolone;lactam | A/S | lactam |
| 40 | A/S;AUG;CP;CRM;LVX | fluoroquinolone;lactam | A/S | lactam |
| 41 | A/S;AUG;CP;CRM;LVX | fluoroquinolone;lactam | A/S | lactam |
| 42 | A/S;AUG;CP;CRM;LVX | fluoroquinolone;lactam | A/S | lactam |
| 43 | A/S;CP;LVX | fluoroquinolone;lactam | A/S | lactam |
| 44 | A/S;AUG;CP;CRM;LVX | fluoroquinolone;lactam | A/S | lactam |
| 45 | A/S;AUG;CP;CRM;LVX | fluoroquinolone;lactam | A/S | lactam |
| 46 | A/S;AUG;CP;CRM;LVX | fluoroquinolone;lactam | A/S | lactam |
| 47 | A/S;AUG;CP;CRM;LVX | fluoroquinolone;lactam | A/S | lactam |
| 48 | A/S;AUG;CP;CRM;LVX | fluoroquinolone;lactam | A/S | lactam |
| 49 | A/S;AUG;CP;CRM;LVX | fluoroquinolone;lactam | A/S | lactam |
| 50 | A/S;AUG;CP;CRM;LVX | fluoroquinolone;lactam | A/S | lactam |

Table 10c: Data for Klebsiella, particularly K. oxytoca (continued)

| No. | num_fluoroquinolone | num_lactam |
|---|---|---|
| 1 | 2 | 4 |
| 2 | 2 | 4 |
| 3 | 2 | 4 |
| 4 | 2 | 1 |
| 5 | 2 | 4 |
| 6 | 2 | 3 |
| 7 | 2 | 3 |
| 8 | 2 | 2 |
| 9 | 2 | 3 |
| 10 | 2 | 3 |
| 11 | 2 | 3 |
| 12 | 2 | 3 |
| 13 | 2 | 1 |

(continued)

| No. | num_fluoroquinolone | num_lactam |
|-----|---------------------|------------|
| 14 | 2 | 4 |
| 15 | 2 | 3 |
| 16 | 2 | 3 |
| 17 | 2 | 3 |
| 18 | 2 | 3 |
| 19 | 2 | 3 |
| 20 | 2 | 3 |
| 21 | 2 | 3 |
| 22 | 2 | 3 |
| 23 | 2 | 3 |
| 24 | 2 | 3 |
| 25 | 2 | 3 |
| 26 | 2 | 3 |
| 27 | 2 | 3 |
| 28 | 2 | 3 |
| 29 | 2 | 3 |
| 30 | 2 | 3 |
| 31 | 2 | 3 |
| 32 | 2 | 3 |
| 33 | 2 | 3 |
| 34 | 2 | 3 |
| 35 | 2 | 3 |
| 36 | 2 | 3 |
| 37 | 2 | 3 |
| 38 | 2 | 3 |
| 39 | 2 | 3 |
| 40 | 2 | 3 |
| 41 | 2 | 3 |
| 42 | 2 | 3 |
| 43 | 2 | 1 |
| 44 | 2 | 3 |
| 45 | 2 | 3 |
| 46 | 2 | 3 |
| 47 | 2 | 3 |
| 48 | 2 | 3 |
| 49 | 2 | 3 |
| 50 | 2 | 3 |

Table 10d: Data for Klebsiella, particularly K. oxytoca (continued)

| No. | A/S_pv_adj | AUG_pv_adj | AZT_pv_adj | CAX_pv_adj | CFT_pv_adj |
|-----|-----------|-----------|-----------|-----------|-----------|
| 1 | 7.25E-19 | 1.72E-03 | 1.02E-01 | 7.63E-02 | 1.14E-01 |
| 2 | 7.25E-19 | 1.72E-03 | 1.02E-01 | 7.63E-02 | 1.14E-01 |
| 3 | 9.82E-17 | 1.48E-03 | 1.97E-02 | 1.37E-02 | 6.22E-02 |
| 4 | 4.07E-16 | 1.01E-02 | 2.64E-02 | 3.46E-02 | 3.40E-02 |
| 5 | 4.07E-16 | 2.92E-04 | 3.51E-02 | 2.48E-02 | 4.69E-02 |
| 6 | 6.05E-16 | 7.34E-03 | 3.46E-02 | 2.50E-02 | 2.46E-02 |
| 7 | 6.05E-16 | 7.34E-03 | 3.46E-02 | 2.50E-02 | 2.46E-02 |
| 8 | 6.05E-16 | 9.84E-03 | 4.51E-02 | 3.38E-02 | 2.50E-02 |
| 9 | 6.05E-16 | 7.34E-03 | 3.46E-02 | 2.50E-02 | 2.46E-02 |
| 10 | 6.05E-16 | 7.34E-03 | 3.46E-02 | 2.50E-02 | 2.46E-02 |
| 11 | 6.05E-16 | 7.34E-03 | 3.46E-02 | 2.50E-02 | 2.46E-02 |
| 12 | 6.05E-16 | 7.34E-03 | 3.46E-02 | 2.50E-02 | 2.46E-02 |
| 13 | 6.05E-16 | 1.01E-02 | 4.61E-02 | 3.45E-02 | 3.46E-02 |
| 14 | 6.05E-16 | 5.13E-03 | 2.58E-02 | 1.89E-02 | 1.75E-02 |
| 15 | 6.05E-16 | 7.34E-03 | 3.46E-02 | 2.50E-02 | 2.46E-02 |
| 16 | 6.05E-16 | 7.34E-03 | 3.46E-02 | 2.50E-02 | 2.46E-02 |
| 17 | 6.05E-16 | 7.34E-03 | 3.46E-02 | 2.50E-02 | 2.46E-02 |
| 18 | 6.05E-16 | 7.34E-03 | 3.46E-02 | 2.50E-02 | 2.46E-02 |
| 19 | 6.05E-16 | 7.34E-03 | 3.46E-02 | 2.50E-02 | 2.46E-02 |
| 20 | 6.05E-16 | 7.34E-03 | 3.46E-02 | 2.50E-02 | 2.46E-02 |
| 21 | 6.05E-16 | 7.34E-03 | 3.46E-02 | 2.50E-02 | 2.46E-02 |
| 22 | 6.05E-16 | 7.34E-03 | 3.46E-02 | 2.50E-02 | 2.46E-02 |
| 23 | 6.05E-16 | 5.42E-03 | 3.46E-02 | 1.98E-02 | 2.46E-02 |
| 24 | 6.05E-16 | 7.34E-03 | 3.46E-02 | 2.50E-02 | 2.46E-02 |
| 25 | 6.05E-16 | 7.34E-03 | 3.46E-02 | 2.50E-02 | 2.46E-02 |
| 26 | 6.05E-16 | 7.34E-03 | 3.46E-02 | 2.50E-02 | 2.46E-02 |
| 27 | 6.05E-16 | 7.34E-03 | 3.46E-02 | 2.50E-02 | 2.46E-02 |
| 28 | 6.05E-16 | 7.34E-03 | 3.46E-02 | 2.50E-02 | 2.46E-02 |
| 29 | 6.05E-16 | 7.34E-03 | 3.46E-02 | 2.50E-02 | 2.46E-02 |
| 30 | 6.05E-16 | 7.34E-03 | 3.46E-02 | 2.50E-02 | 2.46E-02 |
| 31 | 6.05E-16 | 7.34E-03 | 3.46E-02 | 2.50E-02 | 2.46E-02 |
| 32 | 6.05E-16 | 7.34E-03 | 3.46E-02 | 2.50E-02 | 2.46E-02 |
| 33 | 6.05E-16 | 7.34E-03 | 3.46E-02 | 2.50E-02 | 2.46E-02 |
| 34 | 6.05E-16 | 7.34E-03 | 3.46E-02 | 2.50E-02 | 2.46E-02 |
| 35 | 6.05E-16 | 7.34E-03 | 3.46E-02 | 2.50E-02 | 2.46E-02 |
| 36 | 6.05E-16 | 7.34E-03 | 3.46E-02 | 2.50E-02 | 2.46E-02 |
| 37 | 6.05E-16 | 7.34E-03 | 3.46E-02 | 2.50E-02 | 2.46E-02 |

(continued)

| No. | A/S_pv_adj | AUG_pv_adj | AZT_pv_adj | CAX_pv_adj | CFT_pv_adj |
|---|---|---|---|---|---|
| 38 | 6.05E-16 | 7.42E-03 | 3.51E-02 | 2.53E-02 | 2.48E-02 |
| 39 | 6.05E-16 | 7.34E-03 | 3.46E-02 | 2.50E-02 | 2.46E-02 |
| 40 | 6.05E-16 | 7.42E-03 | 3.51E-02 | 2.53E-02 | 2.48E-02 |
| 41 | 6.05E-16 | 7.34E-03 | 3.46E-02 | 2.50E-02 | 2.46E-02 |
| 42 | 6.05E-16 | 7.42E-03 | 3.51E-02 | 2.53E-02 | 2.48E-02 |
| 43 | 6.05E-16 | 1.84E-02 | 7.73E-02 | 5.74E-02 | 4.41E-02 |
| 44 | 6.05E-16 | 7.34E-03 | 3.46E-02 | 2.50E-02 | 2.46E-02 |
| 45 | 6.05E-16 | 7.34E-03 | 3.46E-02 | 2.50E-02 | 2.46E-02 |
| 46 | 6.05E-16 | 7.34E-03 | 3.46E-02 | 2.50E-02 | 2.46E-02 |
| 47 | 6.05E-16 | 7.34E-03 | 3.46E-02 | 2.50E-02 | 2.46E-02 |
| 48 | 6.05E-16 | 7.42E-03 | 3.51E-02 | 2.53E-02 | 2.48E-02 |
| 49 | 6.05E-16 | 7.34E-03 | 3.46E-02 | 2.50E-02 | 2.46E-02 |
| 50 | 6.05E-16 | 7.34E-03 | 3.46E-02 | 2.50E-02 | 2.46E-02 |

Table 10e: Data for Klebsiella, particularly K. oxytoca (continued)

| No. | CP_pv_adj | CRM_pv_adj | LVX_pv_adj | P/T_pv_adj |
|---|---|---|---|---|
| 1 | 4.47E-03 | 3.22E-03 | 3.33E-05 | 4.21E-03 |
| 2 | 4.47E-03 | 3.22E-03 | 3.33E-05 | 4.21E-03 |
| 3 | 2.12E-04 | 4.16E-03 | 5.03E-07 | 3.38E-03 |
| 4 | 2.11E-03 | 1.38E-02 | 6.28E-05 | 1.93E-02 |
| 5 | 6.71E-04 | 1.62E-03 | 8.24E-07 | 2.85E-03 |
| 6 | 1.30E-03 | 7.85E-03 | 5.53E-05 | 1.37E-02 |
| 7 | 1.30E-03 | 7.85E-03 | 5.53E-05 | 1.37E-02 |
| 8 | 1.98E-03 | 1.03E-02 | 5.68E-05 | 1.89E-02 |
| 9 | 1.30E-03 | 7.85E-03 | 5.53E-05 | 1.37E-02 |
| 10 | 1.30E-03 | 7.85E-03 | 5.53E-05 | 1.37E-02 |
| 11 | 1.30E-03 | 7.85E-03 | 5.53E-05 | 1.37E-02 |
| 12 | 1.30E-03 | 7.85E-03 | 5.53E-05 | 1.37E-02 |
| 13 | 1.26E-03 | 1.04E-02 | 3.08E-05 | 1.93E-02 |
| 14 | 7.58E-04 | 6.15E-03 | 5.93E-05 | 9.93E-03 |
| 15 | 1.30E-03 | 7.85E-03 | 5.53E-05 | 1.37E-02 |
| 16 | 1.30E-03 | 7.85E-03 | 5.53E-05 | 1.37E-02 |
| 17 | 1.30E-03 | 7.85E-03 | 5.53E-05 | 1.37E-02 |
| 18 | 1.30E-03 | 7.85E-03 | 5.53E-05 | 1.37E-02 |
| 19 | 1.30E-03 | 7.85E-03 | 5.53E-05 | 1.37E-02 |
| 20 | 1.30E-03 | 7.85E-03 | 5.53E-05 | 1.37E-02 |
| 21 | 1.30E-03 | 7.85E-03 | 5.53E-05 | 1.37E-02 |

(continued)

| No. | CP_pv_adj | CRM_pv_adj | LVX_pv_adj | P/T_pv_adj |
|-----|-----------|------------|------------|------------|
| 22 | 1.30E-03 | 7.85E-03 | 5.53E-05 | 1.37E-02 |
| 23 | 3.39E-03 | 6.15E-03 | 1.55E-04 | 1.03E-02 |
| 24 | 1.30E-03 | 7.85E-03 | 5.53E-05 | 1.37E-02 |
| 25 | 1.30E-03 | 7.85E-03 | 5.53E-05 | 1.37E-02 |
| 26 | 1.30E-03 | 7.85E-03 | 5.53E-05 | 1.37E-02 |
| 27 | 1.30E-03 | 7.85E-03 | 5.53E-05 | 1.37E-02 |
| 28 | 1.30E-03 | 7.85E-03 | 5.53E-05 | 1.37E-02 |
| 29 | 1.30E-03 | 7.85E-03 | 5.53E-05 | 1.37E-02 |
| 30 | 1.30E-03 | 7.85E-03 | 5.53E-05 | 1.37E-02 |
| 31 | 1.30E-03 | 7.85E-03 | 5.53E-05 | 1.37E-02 |
| 32 | 1.30E-03 | 7.85E-03 | 5.53E-05 | 1.37E-02 |
| 33 | 1.30E-03 | 7.85E-03 | 5.53E-05 | 1.37E-02 |
| 34 | 1.30E-03 | 7.85E-03 | 5.53E-05 | 1.37E-02 |
| 35 | 1.30E-03 | 7.85E-03 | 5.53E-05 | 1.37E-02 |
| 36 | 1.30E-03 | 7.85E-03 | 5.53E-05 | 1.37E-02 |
| 37 | 1.30E-03 | 7.85E-03 | 5.53E-05 | 1.37E-02 |
| 38 | 1.97E-03 | 7.93E-03 | 5.53E-05 | 1.39E-02 |
| 39 | 1.30E-03 | 7.85E-03 | 5.53E-05 | 1.37E-02 |
| 40 | 1.97E-03 | 7.93E-03 | 5.53E-05 | 1.39E-02 |
| 41 | 1.30E-03 | 7.85E-03 | 5.53E-05 | 1.37E-02 |
| 42 | 1.97E-03 | 7.93E-03 | 5.53E-05 | 1.39E-02 |
| 43 | 7.43E-03 | 1.01E-02 | 2.47E-04 | 3.31E-02 |
| 44 | 1.30E-03 | 7.85E-03 | 5.53E-05 | 1.37E-02 |
| 45 | 1.30E-03 | 7.85E-03 | 5.53E-05 | 1.37E-02 |
| 46 | 1.30E-03 | 7.85E-03 | 5.53E-05 | 1.37E-02 |
| 47 | 1.30E-03 | 7.85E-03 | 5.53E-05 | 1.37E-02 |
| 48 | 1.97E-03 | 4.47E-03 | 5.53E-05 | 1.39E-02 |
| 49 | 1.30E-03 | 7.85E-03 | 5.53E-05 | 1.37E-02 |
| 50 | 1.30E-03 | 7.85E-03 | 5.53E-05 | 1.37E-02 |

Table 10f: Data for Klebsiella, particularly K. oxytoca (continued)

| No. | Locus.Tag | Gene. Symbol | GenBank.Accession | Chromosome |
|-----|-----------|--------------|-------------------|------------|
| 1 | KPK_2590 | | YP_002238421 | |
| 2 | HMPREF0485_ 01216 | | ZP_06548816 | |
| 3 | EAE_11985 | | YP_004592590 | |
| 4 | Kvar_2784 | | YP_003439702 | |
| 5 | HMPREF0484_ 4675 | add | ZP_06017656 | |

(continued)

| No. | Locus.Tag | Gene. Symbol | GenBank.Accession | Chromosome |
|-----|-----------|--------------|-------------------|------------|
| 6 | Kvar_2381 | | YP_003439305 | |
| 7 | KPN2242_ 04295 | | AEJ96779 | |
| 8 | HMPREF0485_ 01916 | | ZP_06549516 | |
| 9 | HMPREF0485_ 01247 | | ZP_06548847 | |
| 10 | KPK_2622 | | YP_002238453 | |
| 11 | Kvar_2568 | | YP_003439490 | |
| 12 | Kvar_0816 | | YP_003437758 | |
| 13 | Kvar_4643 | | YP_003441547 | |
| 14 | Kvar_3771 | | YP_003440683 | |
| 15 | Kvar_1596 | | YP_003438533 | |
| 16 | HMPREF0485_03998 | | ZP_06551594 | |
| 17 | Kvar_2140 | | YP_003439069 | |
| 18 | Kvar_3380 | | YP_003440292 | |
| 19 | HMPREF0485_ 00446 | | ZP_06548046 | |
| 20 | KPN_00275 | | YP_001333958 | |
| 21 | Kvar_1589 | | YP_003438526 | |
| 22 | Kvar_1856 | | YP_003438789 | |
| 23 | KP1_3016 | | YP_002919724 | |
| 24 | Kvar_5099 | | YP_003442002 | |
| 25 | Kvar_0479 | | YP_003437421 | |
| 26 | HMPREF0485_ 00450 | | ZP_06548050 | |
| 27 | Kvar_1586 | | YP_003438523 | |
| 28 | Kvar_1598 | | YP_003438535 | |
| 29 | Kvar_1597 | | YP_003438534 | |
| 30 | HMPREF0485_ 00443 | | ZP_06548043 | |
| 31 | HMPREF0485_00453 | | ZP_06548053 | |
| 32 | KP1_2851 | | YP_002919569 | |
| 33 | KPN2242_ 01120 | | AEJ96149 | |
| 34 | KPK_2936 | | YP_002238762 | |
| 35 | HMPREF9538_03901 | | ZP_08306208 | |
| 36 | HMPREF0485_02477 | | ZP_06550077 | |
| 37 | HMPREF0485_ 01831 | | ZP_06549431 | |
| 38 | Kvar_3395 | | YP_003440307 | |
| 39 | Kvar_3210 | | YP_003440127 | |
| 40 | KPN_00277 | | YP_001333960 | |
| 41 | Kvar_1604 | | YP_003438541 | |
| 42 | Kvar_4545 | | YP_003441451 | |
| 43 | HMPREF9538_05113 | | ZP_08307402 | |

(continued)

| No. | Locus.Tag | Gene. Symbol | GenBank.Accession | Chromosome |
|-----|-----------|--------------|-------------------|------------|
| 44 | Kvar_4742 | | YP_003441646 | |
| 45 | Kvar_2570 | | YP_003439492 | |
| 46 | Kvar_2569 | | YP_003439491 | |
| 47 | KPN_04505 | melA | YP_001338127 | |
| 48 | KPK_1698 | nifA | YP_002237549 | |
| 49 | Dda3937_02155 | nifH | YP_003884784 | |
| 50 | KPK_1712 | nifK | YP_002237563 | |

Table 10g: Data for Klebsiella, particularly K. oxytoca (continued)

| No. | Start.Coord | End.Coord | Strand | Scaffold.ID |
|-----|-------------|-----------|--------|-------------|
| 1 | 2648871 | 2649476 | + | 643348533 |
| 2 | 1355295 | 1356167 | - | 647536389 |
| 3 | 2576463 | 2577677 | - | 650716122 |
| 4 | 2889784 | 2890686 | - | 646312028 |
| 5 | 12390 | 13076 | + | 647009977 |
| 6 | 2489613 | 2491040 | + | 646312028 |
| 7 | 925860 | 926771 | - | 651053122 |
| 8 | 237063 | 237326 | + | 647536390 |
| 9 | 1387256 | 1388314 | + | 647536389 |
| 10 | 2680912 | 2681727 | + | 643348533 |
| 11 | 2679911 | 2680687 | + | 646312028 |
| 12 | 851698 | 852411 | + | 646312028 |
| 13 | 4934923 | 4936932 | + | 646312028 |
| 14 | 3964905 | 3966425 | + | 646312028 |
| 15 | 1721712 | 1722182 | - | 646312028 |
| 16 | 140912 | 141796 | + | 647536393 |
| 17 | 2255010 | 2255996 | + | 646312028 |
| 18 | 3519306 | 3520322 | - | 646312028 |
| 19 | 523538 | 524377 | - | 647536389 |
| 20 | 311624 | 312142 | + | 640753018 |
| 21 | 1716249 | 1716779 | + | 646312028 |
| 22 | 1971206 | 1973212 | - | 646312028 |
| 23 | 2878963 | 2879583 | + | 646564530 |
| 24 | 5455245 | 5456423 | - | 646312028 |
| 25 | 509672 | 510736 | + | 646312028 |
| 26 | 528513 | 529175 | - | 647536389 |
| 27 | 1711303 | 1712709 | - | 646312028 |

(continued)

| No. | Start.Coord | End.Coord | Strand | Scaffold.ID |
|---|---|---|---|---|
| 28 | 1723564 | 1724937 | - | 646312028 |
| 29 | 1722169 | 1723554 | - | 646312028 |
| 30 | 520902 | 521159 | - | 647536389 |
| 31 | 531062 | 532510 | - | 647536389 |
| 32 | 2717504 | 2718439 | + | 646564530 |
| 33 | 230663 | 231895 | - | 651053122 |
| 34 | 2983904 | 2984626 | + | 643348533 |
| 35 | 15395 | 16450 | + | 651333420 |
| 36 | 872347 | 873972 | + | 647536390 |
| 37 | 149922 | 150884 | - | 647536390 |
| 38 | 3535340 | 3535786 | + | 646312028 |
| 39 | 3340882 | 3341376 | - | 646312028 |
| 40 | 312969 | 315452 | + | 640753018 |
| 41 | 1730820 | 1734335 | + | 646312028 |
| 42 | 4822901 | 4823620 | + | 646312028 |
| 43 | 52 | 390 | - | 651333602 |
| 44 | 5039066 | 5040004 | + | 646312028 |
| 45 | 2681431 | 2682066 | + | 646312028 |
| 46 | 2680692 | 2681420 | + | 646312028 |
| 47 | 4930190 | 4931542 | + | 640753018 |
| 48 | 1752865 | 1754439 | - | 643348533 |
| 49 | 4321869 | 4322750 | - | 648028072 |
| 50 | 1766440 | 1768002 | - | 643348533 |

Table 11a: Data for Klebsiella, particularly K. pneumoniae

| No. | Scaffold.External. Accession | Scaffold.Name | best_pv |
|---|---|---|---|
| 1 | NC_007384 | Shigella sonnei Ss046: NC_007384 | 1.21395823682916e-119 |
| 2 | NC_011083 | Salmonella enterica subsp. enterica serovar Heidelberg str. SL476: NC_011083 | 1.70517984237646e-115 |
| 3 | NZ_ADWV01000045 | Escherichia coli MS 107-1 E_coliMS107-1-1.0.1_Cont44.1: NZ_ADWV01000045 | 3.12333368944279e-110 |
| 4 | NC_009651 | Klebsiella pneumoniae subsp. pneumoniae MGH 78578 plasmid pKPN5: NC_009651 | 1.8490215255217e-103 |
| 5 | NZ_ABFH01000001 | Salmonella enterica subsp. enterica serovar Virchow str. SL491, unfinished sequence: NZ_ABFH01000001 | 4.16211077482132e-97 |
| 6 | CP002910 | Klebsiella pneumoniae KCTC 2242: CP002910 | 1.34397014479151e-80 |
| 7 | CP002910 | Klebsiella pneumoniae KCTC 2242: CP002910 | 1.21342816034335e-71 |

(continued)

| No. | Scaffold.External. Accession | Scaffold.Name | best_pv |
|---|---|---|---|
| 8 | NZ_ ACZD01000070 | Klebsiella pneumoniae subsp. rhinoscleromatis ATCC 13884 contig00070: NZ_ACZD01000070 | 4.42089689687353e-61 |
| 9 | NC_013850 | Klebsiella variicola At-22 chromosome: NC_013850 | 8.41004168548543e-61 |
| 10 | NC_011282 | Klebsiella pneumoniae 342 plasmid pKP187: NC_011282 | 8.41004168548543e-61 |
| 11 | NZ_ ACZD01000070 | Klebsiella pneumoniae subsp. rhinoscleromatis ATCC 13884 contig00070: NZ_ACZD01000070 | 8.41004168548543e-61 |
| 12 | NZ_ ADTP01000323 | Escherichia coli MS 69-1 E_coli69-1-1.0_Cont630.1: NZ_ADTP01000323 | 4.94539115664213e-60 |
| 13 | NC_009793 | Citrobacter koseri ATCC BAA-895 plasmid pCKO3: NC_009793 | 5.71878021049123e-59 |
| 14 | NZ_ ACZD01000005 | Klebsiella pneumoniae subsp. rhinoscleromatis ATCC 13884 contig00005: NZ_ACZD01000005 | 1.22977045856913e-58 |
| 15 | NZ_ ACZD01000127 | Klebsiella pneumoniae subsp. rhinoscleromatis ATCC 13884 contig00129: NZ_ACZD01000127 | 1.49068550300511e-58 |
| 16 | NZ_GG745515 | Klebsiella sp. 1_1_55 genomic scaffold supercont1.8: NZ_GG745515 | 7.09566707309011e-58 |
| 17 | NZ_ AAJX01000089 | Escherichia coli B171, unfinished sequence: NZ_AAJX01000089 | 1.65409147045657e-56 |
| 18 | NZ_ ACZD01000005 | Klebsiella pneumoniae subsp. rhinoscleromatis ATCC 13884 contig00005: NZ_ACZD01000005 | 7.54090407593199e-55 |
| 19 | NZ_ AFBO01000042 | Klebsiella sp. MS 92-3 K_spMS92-3-1.0_Cont91.1: NZ_AFBO01000042 | 2.8348333685311e-54 |
| 20 | NC_009649 | Klebsiella pneumoniae subsp. pneumoniae MGH 78578 plasmid pKPN3: NC_009649 | 2.8348333685311e-54 |
| 21 | CP002910 | Klebsiella pneumoniae KCTC 2242: CP002910 | 2.83530891190408e-54 |
| 22 | NC_009649 | Klebsiella pneumoniae subsp. pneumoniae MGH 78578 plasmid pKPN3: NC_009649 | 4.89541334527925e-54 |
| 23 | NZ_ AFBO01000042 | Klebsiella sp. MS 92-3 K_spMS92-3-1.0_Cont91.1: NZ_AFBO01000042 | 2.18592863711471e-53 |
| 24 | NC_009649 | Klebsiella pneumoniae subsp. pneumoniae MGH 78578 plasmid pKPN3: NC_009649 | 2.18592863711471e-53 |
| 25 | NZ_ ACZD01000127 | Klebsiella pneumoniae subsp. rhinoscleromatis ATCC 13884 contig00129: NZ_ACZD01000127 | 1.08576020331202e-52 |
| 26 | NZ_ ACZD01000127 | Klebsiella pneumoniae subsp. rhinoscleromatis ATCC 13884 contig00129: NZ_ACZD01000127 | 1.37521017661703e-52 |
| 27 | NZ_ ACZD01000127 | Klebsiella pneumoniae subsp. rhinoscleromatis ATCC 13884 contig00129: NZ_ACZD01000127 | 2.1362800317976e-52 |
| 28 | NC_014121 | Enterobacter cloacae subsp. cloacae ATCC 13047 chromosome: NC_ 014121 | 2.21381524453565e-51 |
| 29 | CP002910 | Klebsiella pneumoniae KCTC 2242: CP002910 | 6.53846908249994e-51 |

(continued)

| No. | Scaffold.External. Accession | Scaffold.Name | best_pv |
|---|---|---|---|
| 30 | NZ_GG745512 | Klebsiella sp. 1_1_55 genomic scaffold supercont1.5: NZ_GG745512 | 2.78992804201171e-50 |
| 31 | NZ_ CAAZ01000154 | Salmonella enterica subsp. enterica serovar Typhi str. E98-3139, unfinished sequence: NZ_CAAZ01000154 | 4.10919139010377e-50 |
| 32 | NZ_ ACZD01000127 | Klebsiella pneumoniae subsp. rhinoscleromatis ATCC 13884 contig00129: NZ_ACZD01000127 | 6.10670380866266e-50 |
| 33 | NZ_ ACZD01000127 | Klebsiella pneumoniae subsp. rhinoscleromatis ATCC 13884 contig00129: NZ_ACZD01000127 | 1.00996840886331e-49 |
| 34 | CP002910 | Klebsiella pneumoniae KCTC 2242: CP002910 | 1.33344542353295e-48 |
| 35 | CP002910 | Klebsiella pneumoniae KCTC 2242: CP002910 | 3.00269800428115e-48 |
| 36 | CP002910 | Klebsiella pneumoniae KCTC 2242: CP002910 | 3.13126971411145e-48 |
| 37 | NZ_ ACZD01000127 | Klebsiella pneumoniae subsp. rhinoscleromatis ATCC 13884 contig00129: NZ_ACZD01000127 | 3.13126971411145e-48 |
| 38 | NC_014121 | Enterobacter cloacae subsp. cloacae ATCC 13047 chromosome: NC_ 014121 | 3.79149056816214e-48 |
| 39 | NZ_ AFBO01000625 | Klebsiella sp. MS 92-3 K_spMS92-3-1.0_Cont1175.2: NZ_AFBO01000625 | 2.5975548233422e-47 |
| 40 | NZ_ ACZD01000127 | Klebsiella pneumoniae subsp. rhinoscleromatis ATCC 13884 contig00129: NZ_ACZD01000127 | 1.05391699509183e-46 |
| 41 | NC_011092 | Salmonella enterica subsp. enterica serovar Schwarzengrund str. CVM19633 plasmid pCVM19633_110: NC_011092 | 4.42178242563847e-46 |
| 42 | NC_013850 | Klebsiella variicola At-22 chromosome: NC_013850 | 6.65483065085068e-46 |
| 43 | NC_012912 | Dickeya zeae Ech1591: NC_012912 | 1.05262166415392e-45 |
| 44 | NC_013509 | Edwardsiella tarda EIB202 plasmid pEIB202: NC_ 013509 | 1.24074600175825e-45 |
| 45 | AP011957 | Salmonella enterica subsp. enterica serovar Typhimurium str. T000240 DNA: AP011957 | 2.46270503642371e-45 |
| 46 | NC_006856 | Salmonella enterica subsp. enterica serovar Choleraesuis str. SC-B67 | 9.02631418377704e-45 |
| | | plasmid pSC138: NC_006856 | |
| 47 | AP011957 | Salmonella enterica subsp. enterica serovar Typhimurium str. T000240 DNA: AP011957 | 3.37821430101762e-44 |
| 48 | NZ_ AFBO01000232 | Klebsiella sp. MS 92-3 K_spMS92-3-1.0_Cont505.1: NZ_AFBO01000232 | 4.87973844618098e-43 |
| 49 | NC_004631 | Salmonella enterica subsp. enterica serovar Typhi Ty2: NC_004631 | 1.54269244019591e-42 |
| 50 | NC_006625 | Klebsiella pneumoniae NTUH-K2044 plasmid pK2044: NC_006625 | 1.89874529968327e-42 |

Table 11b: Data for Klebsiella, particularly K. pneumoniae (continued)

| No. | sign_phenos | sign_phenos_class | best_pheno | best_pheno_ class |
|---|---|---|---|---|
| 1 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 2 | A/S;AUG;AZT;CAX;CAZ;CFT;CP; CPE;CRM; ETP;GM;LVX;P/T;TO;T/S | aminoglycoside;fluoroquinolone; lactam;other | AUG | lactam |
| 3 | A/S;AUG;AZT;CAX;CAZ;CFT;CP; CPE;CRM; ETP;GM;LVX;P/T;TO;T/S | aminoglycoside;fluoroquinolone; lactam;other | T/S | other |
| 4 | A/S;AUG;AZT;CAX;CAZ;CFT;CP; CPE;CRM; ETP;GM;LVX;P/T;TO;T/S | aminoglycoside;fluoroquinolone; lactam;other | T/S | other |
| 5 | A/S;AUG;AZT;CAX;CAZ;CFT;CP; CPE;CRM; ETP;GM;LVX;P/T;TO;T/S | aminoglycoside;fluoroquinolone; lactam;other | T/S | other |
| 6 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 7 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 8 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 9 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 10 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 11 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 12 | A/S;AUG;AZT;CAX;CAZ;CFT;CP; CPE;CRM; ETP;GM;LVX;P/T;TO;T/S | aminoglycoside;fluoroquinolone; lactam;other | CAZ | lactam |
| 13 | A/S;AUG;AZT;CAX;CAZ;CFT;CP; CPE;CRM; ETP;GM;LVX;P/T;TO;T/S | aminoglycoside;fluoroquinolone; lactam;other | AUG | lactam |
| 14 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 15 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 16 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 17 | A/S;AUG;AZT;CAX;CAZ;CFT;CP; CPE;CRM; ETP;GM;LVX;P/T;TO;T/S | aminoglycoside;fluoroquinolone; lactam;other | T/S | other |
| 18 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 19 | A/S;AUG;AZT;CAX;CAZ;CFT;CP; CPE;CRM; ETP;GM;LVX;P/T;TO;T/S | aminoglycoside;fluoroquinolone; lactam;other | P/T | lactam |
| 20 | A/S;AUG;AZT;CAX;CAZ;CFT;CP; CPE;CRM; ETP;GM;LVX;P/T;TO;T/S | aminoglycoside;fluoroquinolone; lactam;other | P/T | lactam |
| 21 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 22 | A/S;AUG;AZT;CAX;CAZ;CFT;CP; CPE;CRM; ETP;GM;LVX;P/T;TO;T/S | aminoglycoside;fluoroquinolone; lactam;other | P/T | lactam |

(continued)

| No. | sign_phenos | sign_phenos_class | best_pheno | best_pheno_ class |
|---|---|---|---|---|
| 23 | A/S;AUG;AZT;CAX;CAZ;CFT;CP; CPE;CRM; ETP;GM;LVX;P/T;TO;T/S | aminoglycoside;fluoroquinolone; lactam;other | P/T | lactam |
| 24 | A/S;AUG;AZT;CAX;CAZ;CFT;CP; CPE;CRM; ETP;GM;LVX;P/T;TO;T/S | aminoglycoside;fluoroquinolone; lactam;other | P/T | lactam |
| 25 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 26 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 27 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 28 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 29 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 30 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 31 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 32 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 33 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 34 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 35 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 36 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 37 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 38 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 39 | A/S;AUG;AZT;CAX;CAZ;CFT;CP; CPE;CRM; GM;LVX;P/T;TO;T/S | aminoglycoside;fluoroquinolone; lactam;other | T/S | other |
| 40 | A/S;AUG;AZT;CAX;CAZ;CFT;CP; CPE;CRM; ETP;GM;LVX;P/T;TO;T/S | aminoglycoside;fluoroquinolone; lactam;other | CP | fluoroquinolon e |
| 41 | A/S;AUG;AZT;CAX;CAZ;CFT;CP; CPE;CRM; ETP;GM;LVX;P/T;TO;T/S | aminoglycoside;fluoroquinolone; lactam;other | AUG | lactam |
| 42 | AUG;AZT;CAX;CAZ;CFT;CP;CPE; CRM;ETP; LVX;P/T;TO;T/S | aminoglycoside;fluoroquinolone; lactam;other | ETP | lactam |
| 43 | AUG;AZT;CAX;CAZ;CFT;CP;CPE; CRM;ETP; LVX;P/T;TO;T/S | aminoglycoside;fluoroquinolone; lactam;other | ETP | lactam |
| 44 | A/S;AUG;AZT;CAX;CAZ;CFT;CP; CPE;CRM; ETP;GM;LVX;P/T;TO;T/S | aminoglycoside;fluoroquinolone; lactam;other | T/S | other |

(continued)

| No. | sign_phenos | sign_phenos_class | best_pheno | best_pheno_ class |
|---|---|---|---|---|
| 45 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 46 | A/S;AUG;AZT;CAX;CAZ;CFT;CP; CPE;CRM; ETP;GM;LVX;P/T;TO;T/S | aminoglycoside;fluoroquinolone; lactam;other | TO | aminoglycoside |
| 47 | A/S;AUG;AZT;CAX;CAZ;CFT;CP; CPE;CRM; ETP;GM;LVX;P/T;TO;T/S | aminoglycoside;fluoroquinolone; lactam;other | AUG | lactam |
| 48 | A/S;AUG;AZT;CAX;CAZ;CFT;CP; CPE;CRM; ETP;GM;LVX;P/T;TO;T/S | aminoglycoside;fluoroquinolone; lactam;other | T/S | other |
| 49 | A/S;AUG;AZT;CAX;CAZ;CFT;CP; CPE;CRM; ETP;GM;LVX;P/T;TO;T/S | aminoglycoside;fluoroquinolone; lactam;other | ETP | lactam |
| 50 | A/S;AUG;AZT;CAX;CAZ;CFT;CP; CPE;CRM; ETP;GM;LVX;P/T;TO;T/S | aminoglycoside;fluoroquinolone; lactam;other | T/S | other |

Table 11c: Data for Klebsiella, particularly K. pneumoniae (continued)

| No. | num_aminoglycoside | num_fluoroquinolone | num_lactam | num_other |
|---|---|---|---|---|
| 1 | 2 | 2 | 10 | 1 |
| 2 | 2 | 2 | 10 | 1 |
| 3 | 2 | 2 | 10 | 1 |
| 4 | 2 | 2 | 10 | 1 |
| 5 | 2 | 2 | 10 | 1 |
| 6 | 2 | 2 | 10 | 1 |
| 7 | 2 | 2 | 10 | 1 |
| 8 | 2 | 2 | 10 | 1 |
| 9 | 2 | 2 | 10 | 1 |
| 10 | 2 | 2 | 10 | 1 |
| 11 | 2 | 2 | 10 | 1 |
| 12 | 2 | 2 | 10 | 1 |
| 13 | 2 | 2 | 10 | 1 |
| 14 | 2 | 2 | 10 | 1 |
| 15 | 2 | 2 | 10 | 1 |
| 16 | 2 | 2 | 10 | 1 |
| 17 | 2 | 2 | 10 | 1 |
| 18 | 2 | 2 | 10 | 1 |
| 19 | 2 | 2 | 10 | 1 |
| 20 | 2 | 2 | 10 | 1 |
| 21 | 2 | 2 | 10 | 1 |
| 22 | 2 | 2 | 10 | 1 |
| 23 | 2 | 2 | 10 | 1 |
| 24 | 2 | 2 | 10 | 1 |

(continued)

| No. | num_aminoglycoside | num_fluoroquinolone | num_lactam | num_other |
|---|---|---|---|---|
| 25 | 2 | 2 | 10 | 1 |
| 26 | 2 | 2 | 10 | 1 |
| 27 | 2 | 2 | 10 | 1 |
| 28 | 2 | 2 | 10 | 1 |
| 29 | 2 | 2 | 10 | 1 |
| 30 | 2 | 2 | 10 | 1 |
| 31 | 2 | 2 | 10 | 1 |
| 32 | 2 | 2 | 10 | 1 |
| 33 | 2 | 2 | 10 | 1 |
| 34 | 2 | 2 | 10 | 1 |
| 35 | 2 | 2 | 10 | 1 |
| 36 | 2 | 2 | 10 | 1 |
| 37 | 2 | 2 | 10 | 1 |
| 38 | 2 | 2 | 10 | 1 |
| 39 | 2 | 2 | 9 | 1 |
| 40 | 2 | 2 | 10 | 1 |
| 41 | 2 | 2 | 10 | 1 |
| 42 | 1 | 2 | 9 | 1 |
| 43 | 1 | 2 | 9 | 1 |
| 44 | 2 | 2 | 10 | 1 |
| 45 | 2 | 2 | 10 | 1 |
| 46 | 2 | 2 | 10 | 1 |
| 47 | 2 | 2 | 10 | 1 |
| 48 | 2 | 2 | 10 | 1 |
| 49 | 2 | 2 | 10 | 1 |
| 50 | 2 | 2 | 10 | 1 |

Table 11d: Data for Klebsiella, particularly K. pneumoniae (continued)

| No. | A/S_pv_adj | AUG_pv_adj | AZT_pv_adj | CAX_pv_adj | CAZ_pv_adj |
|---|---|---|---|---|---|
| 1 | 1.28E-76 | 1.24E-105 | 4.06E-95 | 3.39E-95 | 5.99E-93 |
| 2 | 1.18E-98 | 1.71E-115 | 9.09E-91 | 6.55E-88 | 1.66E-96 |
| 3 | 1.08E-81 | 5.85E-86 | 2.76E-94 | 4.16E-97 | 6.84E-100 |
| 4 | 5.55E-76 | 3.05E-84 | 1.38E-91 | 2.64E-96 | 5.33E-94 |
| 5 | 1.22E-70 | 3.41E-71 | 1.41E-80 | 1.50E-83 | 6.22E-90 |
| 6 | 7.70E-21 | 1.03E-41 | 8.20E-48 | 2.50E-44 | 2.69E-46 |
| 7 | 8.54E-16 | 4.34E-29 | 4.54E-38 | 5.50E-34 | 1.34E-34 |
| 8 | 2.62E-12 | 1.74E-24 | 1.49E-26 | 2.74E-25 | 2.84E-24 |

(continued)

| No. | A/S_pv_adj | AUG_pv_adj | AZT_pv_adj | CAX_pv_adj | CAZ_pv_adj |
|-----|-----------|-----------|-----------|-----------|-----------|
| 9 | 5.20E-12 | 2.50E-24 | 3.69E-26 | 7.31E-25 | 7.66E-24 |
| 10 | 5.20E-12 | 2.50E-24 | 3.69E-26 | 7.31E-25 | 7.66E-24 |
| 11 | 5.20E-12 | 2.50E-24 | 3.69E-26 | 7.31E-25 | 7.66E-24 |
| 12 | 3.32E-43 | 1.75E-59 | 1.48E-54 | 9.28E-55 | 4.95E-60 |
| 13 | 2.41E-43 | 5.72E-59 | 4.20E-53 | 2.96E-53 | 2.14E-58 |
| 14 | 2.47E-16 | 8.57E-27 | 1.49E-34 | 1.96E-31 | 8.83E-33 |
| 15 | 1.72E-35 | 6.40E-55 | 1.25E-44 | 4.12E-43 | 1.61E-46 |
| 16 | 3.04E-18 | 5.19E-32 | 4.83E-36 | 2.23E-35 | 1.91E-32 |
| 17 | 1.02E-47 | 9.19E-45 | 1.85E-48 | 9.17E-53 | 6.18E-56 |
| 18 | 3.51E-17 | 3.04E-23 | 8.29E-30 | 1.21E-27 | 9.29E-29 |
| 19 | 1.03E-40 | 7.90E-52 | 1.41E-37 | 4.81E-39 | 9.97E-38 |
| 20 | 1.03E-40 | 7.90E-52 | 1.41E-37 | 4.81E-39 | 9.97E-38 |
| 21 | 8.56E-34 | 4.84E-53 | 8.75E-43 | 2.41E-41 | 9.43E-45 |
| 22 | 5.13E-42 | 1.10E-52 | 3.02E-38 | 9.55E-40 | 3.15E-38 |
| 23 | 1.11E-41 | 3.84E-52 | 1.00E-37 | 3.11E-39 | 8.03E-38 |
| 24 | 1.11E-41 | 3.84E-52 | 1.00E-37 | 3.11E-39 | 8.03E-38 |
| 25 | 8.85E-33 | 1.54E-51 | 2.83E-42 | 1.24E-40 | 2.88E-44 |
| 26 | 1.96E-32 | 2.54E-52 | 2.98E-42 | 1.41E-40 | 3.55E-44 |
| 27 | 5.12E-32 | 6.70E-50 | 1.84E-40 | 2.34E-38 | 1.33E-42 |
| 28 | 1.65E-30 | 3.00E-47 | 1.96E-38 | 1.37E-36 | 4.94E-39 |
| 29 | 4.43E-31 | 1.45E-47 | 2.29E-39 | 1.06E-37 | 3.63E-40 |
| 30 | 6.99E-12 | 2.37E-26 | 2.77E-24 | 5.28E-24 | 2.55E-26 |
| 31 | 2.78E-31 | 7.62E-46 | 1.11E-38 | 5.40E-37 | 1.95E-39 |
| 32 | 9.62E-32 | 1.45E-47 | 2.29E-39 | 1.06E-37 | 3.63E-40 |
| 33 | 2.27E-31 | 2.28E-47 | 3.20E-39 | 1.66E-37 | 5.77E-40 |
| 34 | 9.74E-28 | 9.40E-43 | 1.44E-35 | 7.30E-34 | 3.24E-36 |
| 35 | 1.57E-28 | 2.77E-42 | 6.21E-36 | 1.59E-34 | 2.14E-36 |
| 36 | 3.41E-27 | 2.34E-43 | 7.21E-36 | 4.53E-33 | 9.07E-37 |
| 37 | 3.41E-27 | 2.34E-43 | 7.21E-36 | 4.53E-33 | 9.07E-37 |
| 38 | 2.00E-30 | 7.61E-47 | 1.00E-38 | 1.06E-37 | 3.63E-40 |
| 39 | 8.29E-47 | 2.48E-33 | 3.67E-37 | 5.57E-42 | 2.74E-43 |
| 40 | 1.53E-27 | 6.97E-43 | 2.91E-36 | 2.70E-35 | 5.96E-38 |
| 41 | 1.75E-44 | 4.42E-46 | 2.16E-37 | 1.21E-36 | 1.12E-42 |
| 42 | 4.51E-02 | 4.66E-12 | 9.77E-17 | 3.13E-16 | 9.90E-15 |
| 43 | 5.37E-02 | 5.73E-12 | 1.27E-16 | 6.11E-16 | 1.15E-14 |
| 44 | 6.65E-31 | 4.17E-32 | 1.40E-25 | 3.69E-27 | 1.78E-29 |
| 45 | 6.15E-34 | 5.78E-41 | 2.29E-36 | 9.94E-38 | 1.48E-38 |
| 46 | 3.01E-32 | 3.12E-38 | 7.22E-37 | 3.96E-36 | 6.00E-37 |

(continued)

| No. | A/S_pv_adj | AUG_pv_adj | AZT_pv_adj | CAX_pv_adj | CAZ_pv_adj |
|-----|-----------|-----------|-----------|-----------|-----------|
| 47 | 4.35E-41 | 3.38E-44 | 1.49E-30 | 1.42E-33 | 1.82E-29 |
| 48 | 6.41E-31 | 1.63E-41 | 3.24E-33 | 2.26E-36 | 1.97E-31 |
| 49 | 2.54E-11 | 2.61E-18 | 8.71E-21 | 1.23E-18 | 1.87E-19 |
| 50 | 2.38E-23 | 3.76E-29 | 2.21E-27 | 4.97E-29 | 1.37E-29 |

Table 11e: Data for Klebsiella, particularly K. pneumoniae (continued)

| No. | CFT_pv_adj | CP_pv_adj | CPE_pv_adj | CRM_pv_adj | ETP_pv_adj |
|-----|-----------|-----------|-----------|-----------|-----------|
| 1 | 2.17E-99 | 2.40E-85 | 2.31E-68 | 1.21E-82 | 1.87E-58 |
| 2 | 2.52E-83 | 1.28E-61 | 4.61E-66 | 7.83E-57 | 8.20E-51 |
| 3 | 3.93E-93 | 2.17E-58 | 2.89E-43 | 3.32E-73 | 3.54E-24 |
| 4 | 1.52E-90 | 4.25E-60 | 1.75E-43 | 2.59E-71 | 1.63E-23 |
| 5 | 3.61E-77 | 1.76E-47 | 1.47E-36 | 5.14E-62 | 6.31E-21 |
| 6 | 2.84E-46 | 1.53E-54 | 2.89E-63 | 1.06E-36 | 1.34E-80 |
| 7 | 8.43E-36 | 1.96E-43 | 3.06E-55 | 3.95E-29 | 1.21E-71 |
| 8 | 5.55E-27 | 2.63E-33 | 6.90E-40 | 1.01E-20 | 4.42E-61 |
| 9 | 1.58E-26 | 5.49E-33 | 1.32E-39 | 2.40E-20 | 8.41E-61 |
| 10 | 1.58E-26 | 5.49E-33 | 1.32E-39 | 2.40E-20 | 8.41E-61 |
| 11 | 1.58E-26 | 5.49E-33 | 1.32E-39 | 4.98E-21 | 8.41E-61 |
| 12 | 4.39E-54 | 4.96E-60 | 1.80E-51 | 7.75E-50 | 7.02E-53 |
| 13 | 1.26E-52 | 1.35E-58 | 1.72E-50 | 4.57E-49 | 3.71E-52 |
| 14 | 1.28E-31 | 2.00E-30 | 6.27E-42 | 1.20E-29 | 1.23E-58 |
| 15 | 2.05E-41 | 2.56E-52 | 7.76E-46 | 3.74E-32 | 5.20E-48 |
| 16 | 1.52E-36 | 2.10E-37 | 5.58E-48 | 5.62E-28 | 7.10E-58 |
| 17 | 9.26E-49 | 4.16E-22 | 1.22E-19 | 9.42E-36 | 5.55E-05 |
| 18 | 6.27E-28 | 1.02E-25 | 4.83E-36 | 7.62E-29 | 7.54E-55 |
| 19 | 9.92E-40 | 2.65E-39 | 2.28E-41 | 4.71E-28 | 1.10E-32 |
| 20 | 9.92E-40 | 2.65E-39 | 2.28E-41 | 4.71E-28 | 1.10E-32 |
| 21 | 1.47E-40 | 1.18E-49 | 1.74E-45 | 2.18E-32 | 1.46E-46 |
| 22 | 2.00E-40 | 6.22E-40 | 7.04E-42 | 1.08E-28 | 5.10E-33 |
| 23 | 6.77E-40 | 2.32E-39 | 3.78E-41 | 2.22E-28 | 3.70E-32 |
| 24 | 6.77E-40 | 2.32E-39 | 3.78E-41 | 2.22E-28 | 3.70E-32 |
| 25 | 4.69E-40 | 3.62E-49 | 1.06E-44 | 1.70E-33 | 2.11E-45 |
| 26 | 4.89E-40 | 1.97E-49 | 1.88E-45 | 2.34E-32 | 8.59E-46 |
| 27 | 5.94E-39 | 1.13E-47 | 2.46E-43 | 3.44E-33 | 1.93E-49 |
| 28 | 3.32E-36 | 2.21E-51 | 1.87E-44 | 1.56E-31 | 1.83E-41 |
| 29 | 2.69E-37 | 6.54E-51 | 4.48E-44 | 2.19E-30 | 3.44E-41 |
| 30 | 1.39E-24 | 6.75E-31 | 1.12E-35 | 5.11E-15 | 2.79E-50 |

(continued)

| No. | CFT_pv_adj | CP_pv_adj | CPE_pv_adj | CRM_pv_adj | ETP_pv_adj |
|---|---|---|---|---|---|
| 31 | 1.34E-36 | 4.11E-50 | 1.67E-43 | 1.45E-31 | 8.85E-41 |
| 32 | 2.69E-37 | 6.11E-50 | 4.48E-44 | 2.19E-30 | 3.44E-41 |
| 33 | 6.74E-37 | 1.01E-49 | 6.91E-44 | 1.07E-30 | 4.69E-41 |
| 34 | 3.28E-34 | 1.33E-48 | 1.41E-40 | 1.52E-30 | 7.40E-43 |
| 35 | 3.72E-36 | 3.00E-48 | 1.67E-40 | 8.05E-31 | 2.56E-43 |
| 36 | 1.04E-34 | 3.13E-48 | 5.88E-41 | 1.06E-29 | 3.84E-43 |
| 37 | 1.04E-34 | 3.13E-48 | 5.88E-41 | 1.06E-29 | 3.84E-43 |
| 38 | 2.69E-37 | 3.79E-48 | 4.21E-43 | 9.89E-32 | 5.95E-40 |
| 39 | 2.84E-38 | 3.78E-14 | 1.15E-05 | 9.70E-31 | 9.25E-01 |
| 40 | 1.88E-36 | 1.05E-46 | 3.80E-41 | 1.01E-30 | 2.77E-43 |
| 41 | 4.01E-36 | 1.78E-19 | 2.93E-16 | 1.27E-21 | 2.46E-10 |
| 42 | 5.45E-16 | 4.08E-18 | 6.25E-28 | 7.56E-08 | 6.65E-46 |
| 43 | 1.13E-15 | 5.57E-18 | 9.82E-28 | 1.16E-07 | 1.05E-45 |
| 44 | 2.55E-25 | 6.58E-14 | 7.47E-22 | 3.89E-16 | 2.18E-20 |
| 45 | 5.14E-35 | 2.41E-27 | 5.02E-15 | 4.61E-29 | 2.22E-12 |
| 46 | 1.40E-36 | 2.78E-33 | 1.06E-29 | 5.12E-30 | 2.25E-28 |
| 47 | 3.46E-32 | 1.18E-32 | 1.62E-32 | 2.49E-29 | 1.62E-26 |
| 48 | 6.74E-36 | 1.36E-34 | 2.38E-34 | 1.27E-25 | 6.79E-29 |
| 49 | 9.64E-20 | 3.40E-22 | 1.36E-30 | 8.87E-18 | 1.54E-42 |
| 50 | 1.19E-26 | 1.97E-18 | 9.42E-19 | 1.46E-19 | 1.52E-12 |

Table 11f: Data for Klebsiella, particularly K. pneumoniae (continued)

| No. | GM_pv_adj | LVX_pv_adj | P/T_pv_adj | TO_pv_adj | T/S_pv_adj |
|---|---|---|---|---|---|
| 1 | 6.24E-60 | 3.97E-80 | 7.18E-96 | 1.21E-119 | 1.12E-106 |
| 2 | 6.10E-67 | 3.76E-55 | 1.20E-67 | 2.77E-105 | 3.59E-83 |
| 3 | 6.00E-68 | 3.14E-50 | 5.58E-70 | 1.37E-91 | 3.12E-110 |
| 4 | 3.06E-63 | 4.39E-52 | 7.17E-70 | 6.65E-91 | 1.85E-103 |
| 5 | 5.29E-59 | 6.80E-39 | 4.00E-51 | 1.40E-82 | 4.16E-97 |
| 6 | 5.64E-09 | 3.19E-59 | 3.01E-52 | 7.11E-48 | 1.50E-31 |
| 7 | 1.61E-05 | 6.12E-48 | 2.20E-40 | 1.45E-34 | 2.10E-20 |
| 8 | 1.57E-06 | 6.85E-37 | 6.06E-35 | 1.79E-29 | 2.02E-18 |
| 9 | 2.46E-06 | 1.41E-36 | 1.30E-34 | 3.62E-29 | 2.62E-18 |
| 10 | 2.46E-06 | 1.41E-36 | 1.30E-34 | 3.62E-29 | 2.62E-18 |
| 11 | 2.46E-06 | 1.41E-36 | 1.30E-34 | 3.62E-29 | 2.62E-18 |
| 12 | 2.30E-25 | 1.32E-53 | 6.01E-52 | 5.28E-59 | 5.07E-39 |
| 13 | 1.61E-25 | 2.14E-52 | 1.19E-50 | 1.22E-58 | 6.30E-38 |
| 14 | 6.89E-05 | 6.38E-32 | 9.66E-29 | 7.19E-31 | 3.05E-15 |

(continued)

| No. | GM_pv_adj | LVX_pv_adj | P/T_pv_adj | TO_pv_adj | T/S_pv_adj |
|---|---|---|---|---|---|
| 15 | 1.55E-23 | 1.89E-48 | 1.36E-48 | 1.49E-58 | 1.23E-46 |
| 16 | 7.14E-10 | 1.99E-38 | 1.30E-34 | 9.13E-37 | 1.47E-21 |
| 17 | 6.27E-40 | 9.96E-18 | 4.98E-31 | 1.50E-43 | 1.65E-56 |
| 18 | 7.35E-05 | 4.57E-28 | 1.61E-25 | 2.68E-27 | 2.50E-15 |
| 19 | 1.16E-19 | 5.28E-36 | 2.83E-54 | 2.59E-42 | 9.74E-48 |
| 20 | 1.16E-19 | 5.28E-36 | 2.83E-54 | 2.59E-42 | 9.74E-48 |
| 21 | 6.44E-22 | 5.93E-45 | 3.28E-47 | 2.84E-54 | 2.84E-46 |
| 22 | 6.19E-20 | 1.49E-36 | 4.90E-54 | 5.19E-43 | 1.61E-47 |
| 23 | 4.80E-20 | 5.67E-36 | 2.19E-53 | 1.90E-42 | 7.35E-49 |
| 24 | 1.78E-19 | 5.67E-36 | 2.19E-53 | 1.90E-42 | 6.83E-48 |
| 25 | 1.71E-21 | 3.92E-44 | 2.20E-46 | 1.09E-52 | 8.26E-45 |
| 26 | 1.49E-20 | 1.39E-44 | 1.02E-46 | 1.38E-52 | 9.52E-46 |
| 27 | 9.23E-19 | 4.68E-44 | 1.13E-48 | 2.14E-52 | 7.18E-42 |
| 28 | 1.04E-18 | 2.43E-47 | 2.68E-42 | 8.73E-48 | 1.22E-43 |
| 29 | 1.81E-19 | 6.48E-47 | 5.36E-42 | 7.69E-49 | 5.28E-43 |
| 30 | 1.67E-04 | 1.19E-33 | 1.48E-27 | 1.33E-24 | 2.01E-19 |
| 31 | 2.71E-19 | 3.53E-46 | 2.81E-41 | 2.57E-47 | 3.11E-43 |
| 32 | 3.53E-20 | 6.26E-46 | 5.36E-42 | 7.69E-49 | 5.57E-44 |
| 33 | 4.08E-20 | 9.51E-46 | 1.44E-41 | 1.11E-48 | 8.48E-44 |
| 34 | 9.00E-16 | 3.30E-45 | 1.53E-41 | 3.99E-46 | 1.50E-39 |
| 35 | 4.69E-14 | 4.82E-45 | 1.45E-39 | 3.68E-47 | 5.85E-39 |
| 36 | 6.72E-15 | 8.68E-45 | 4.34E-42 | 8.00E-45 | 1.33E-38 |
| 37 | 6.72E-15 | 8.68E-45 | 4.34E-42 | 8.00E-45 | 1.33E-38 |
| 38 | 4.79E-19 | 4.19E-45 | 5.36E-42 | 3.42E-47 | 5.28E-43 |
| 39 | 1.94E-40 | 1.06E-10 | 6.27E-21 | 4.23E-35 | 2.60E-47 |
| 40 | 1.53E-15 | 3.13E-43 | 2.72E-42 | 3.02E-45 | 1.22E-39 |
| 41 | 9.94E-38 | 3.55E-16 | 4.46E-22 | 7.61E-44 | 1.24E-41 |
| 42 | 2.34E-02 | 4.47E-21 | 3.44E-16 | 3.10E-15 | 2.24E-09 |
| 43 | 2.39E-02 | 1.02E-20 | 4.32E-16 | 6.38E-15 | 3.86E-09 |
| 44 | 3.44E-22 | 5.51E-13 | 6.56E-18 | 4.39E-24 | 1.24E-45 |
| 45 | 1.36E-21 | 1.14E-24 | 2.38E-33 | 2.46E-45 | 1.53E-29 |
| 46 | 1.36E-19 | 4.39E-32 | 1.03E-35 | 9.03E-45 | 5.01E-44 |
| 47 | 1.18E-14 | 5.29E-30 | 3.76E-40 | 3.55E-44 | 5.56E-38 |
| 48 | 4.91E-17 | 8.23E-33 | 1.06E-40 | 6.11E-39 | 4.88E-43 |
| 49 | 1.35E-04 | 2.25E-22 | 1.78E-19 | 5.28E-22 | 2.63E-12 |
| 50 | 8.68E-26 | 1.45E-15 | 1.73E-22 | 6.68E-34 | 1.90E-42 |

Table 11g: Data for Klebsiella, particularly K. pneumoniae (continued)

| No. | Locus.Tag | Gene. Symbol | GenBank.Accession | Chromosome |
|-----|-----------|--------------|-------------------|------------|
| 1 | SSON_3896 | aadA | YP_312670 | |
| 2 | SeHA_C1580 | blaT | YP_002045448 | |
| 3 | HMPREF9345_05066 | | ZP_07100147 | |
| 4 | KPN_pKPN5p08201 | | YP_001338811 | plasmid pKPN5 |
| 5 | Salmoentericaenterica_010100000245 | | ZP_02702077 | |
| 6 | KPN2242_ 07235 | | AEJ97365 | |
| 7 | KPN2242_ 00750 | | AEJ96075 | |
| 8 | HMPREF0484_1390 | mgtA | ZP_06014374 | |
| 9 | Kvar_3592 | | YP_003440504 | |
| 10 | KPK_A0103 | | YP_002235751 | plasmid pKP187 |
| 11 | HMPREF0484_ 1392 | pacL | ZP_06014376 | |
| 12 | HMPREF9534_04427 | | ZP_07188952 | |
| 13 | CKO_pCKO3p06155 | | YP_001456633 | plasmid pCKO3 |
| 14 | HMPREF0484_ 0044 | vmtV | ZP_06013029 | |
| 15 | HMPREF0484_ 2697 | | ZP_06015679 | |
| 16 | HMPREF0485_ 04762 | | ZP_06552358 | |
| 17 | EcolB_01004576 | | ZP_00708527 | |
| 18 | HMPREF0484_ 0046 | | ZP_06013031 | |
| 19 | HMPREF9538_00440 | | ZP_08302804 | |
| 20 | KPN_pKPN3p05893 | ptxE | YP_001338502 | plasmid pKPN3 |
| 21 | KPN2242_ 07425 | | AEJ97403 | |
| 22 | KPN_pKPN3p05890 | ptxB | YP_001338499 | plasmid pKPN3 |
| 23 | HMPREF9538_00443 | | ZP_08302807 | |
| 24 | KPN_pKPN3p05891 | ptxC | YP_001338500 | plasmid pKPN3 |
| 25 | HMPREF0484_2686 | | ZP_06015668 | |
| 26 | HMPREF0484_ 2687 | gpw | ZP_06015669 | |
| 27 | HMPREF0484_ 2684 | | ZP_06015666 | |
| 28 | ECL_04426 | | YP_003614904 | |
| 29 | KPN2242_07415 | | AEJ97401 | |
| 30 | HMPREF0485_ 03923 | | ZP_06551519 | |
| 31 | Salmonellentericaenterica_010100016432 | | ZP_03412648 | |
| 32 | HMPREF0484_ 2698 | | ZP_06015680 | |
| 33 | HMPREF0484_ 2694 | fii | ZP_06015676 | |
| 34 | KPN2242_ 07345 | | AEJ97387 | |
| 35 | KPN2242_ 07330 | | AEJ97384 | |
| 36 | KPN2242_ 07325 | | AEJ97383 | |
| 37 | HMPREF0484_ 2676 | | ZP_06015658 | |

(continued)

| No. | Locus.Tag | Gene. Symbol | GenBank.Accession | Chromosome |
|-----|-----------|--------------|-------------------|------------|
| 38 | ECL_04434 | | YP_003614912 | |
| 39 | HMPREF9538_04737 | | ZP_08307036 | |
| 40 | HMPREF0484_ 2673 | | ZP_06015655 | |
| 41 | SeSA_B0079 | | YP_002112958 | plasmid pCVM19633_110 |
| 42 | Kvar_2527 | | YP_003439449 | |
| 43 | Dd1591_2579 | | YP_003004893 | |
| 44 | ETAE_p041 | folP | YP_003297635 | plasmid pEIB202 |
| 45 | STMDT12_C39340 | | BAJ38877 | |
| 46 | SC026 | aph | YP_209331 | plasmid pSC138 |
| 47 | STMDT12_C39170 | | BAJ38860 | |
| 48 | HMPREF9538_01820 | | ZP_08304144 | |
| 49 | t1924 | exo | NP_805691 | |
| 50 | pK2044_00525 | | YP_001687935 | plasmid pK2044 |

Table 11h: Data for Klebsiella, particularly K. pneumoniae (continued)

| No. | Start.Coord | End.Coord | Strand | Scaffold.ID |
|-----|-------------|-----------|--------|-------------|
| 1 | 4110458 | 4111246 | - | 640427102 |
| 2 | 1525960 | 1526820 | + | 642555218 |
| 3 | 4715 | 5554 | - | 648294624 |
| 4 | 34908 | 35255 | - | 640753021 |
| 5 | 36757 | 37062 | + | 641778223 |
| 6 | 1535907 | 1536932 | - | 651053122 |
| 7 | 159235 | 160521 | - | 651053122 |
| 8 | 2865 | 3848 | - | 647009817 |
| 9 | 3765814 | 3768501 | - | 646312028 |
| 10 | 67987 | 70674 | + | 643348532 |
| 11 | 4691 | 5551 | - | 647009817 |
| 12 | 2120 | 2368 | + | 648289812 |
| 13 | 8632 | 8931 | + | 640753081 |
| 14 | 12911 | 13663 | + | 647009752 |
| 15 | 20402 | 22975 | + | 647009874 |
| 16 | 93128 | 94558 | + | 647536396 |
| 17 | 3555 | 4568 | + | 638359041 |
| 18 | 14573 | 15328 | + | 647009752 |
| 19 | 933 | 1943 | - | 651332959 |
| 20 | 38514 | 39383 | + | 640753019 |
| 21 | 1563447 | 1563746 | + | 651053122 |

(continued)

| No. | Start.Coord | End.Coord | Strand | Scaffold.ID |
|-----|-------------|-----------|--------|-------------|
| 22 | 35802 | 36665 | + | 640753019 |
| 23 | 3648 | 4475 | - | 651332959 |
| 24 | 36662 | 37501 | + | 640753019 |
| 25 | 11204 | 11776 | + | 647009874 |
| 26 | 11773 | 12132 | + | 647009874 |
| 27 | 10265 | 10696 | + | 647009874 |
| 28 | 4537618 | 4539336 | + | 646564624 |
| 29 | 1561697 | 1562869 | + | 651053122 |
| 30 | 51251 | 51370 | - | 647536393 |
| 31 | 154 | 357 | - | 643042634 |
| 32 | 23022 | 23267 | + | 647009874 |
| 33 | 19408 | 19923 | + | 647009874 |
| 34 | 1552497 | 1552712 | + | 651053122 |
| 35 | 1551079 | 1551729 | + | 651053122 |
| 36 | 1550023 | 1551075 | + | 651053122 |
| 37 | 6165 | 7229 | + | 647009874 |
| 38 | 4543715 | 4546123 | - | 646564624 |
| 39 | 1555 | 2055 | - | 651333542 |
| 40 | 2380 | 3411 | - | 647009874 |
| 41 | 68933 | 70240 | + | 642555219 |
| 42 | 2637299 | 2637790 | + | 646312028 |
| 43 | 2932956 | 2933891 | + | 644736389 |
| 44 | 32101 | 32916 | - | 646311959 |
| 45 | 4084530 | 4085237 | - | 651053007 |
| 46 | 21137 | 21952 | - | 637000354 |
| 47 | 4067283 | 4067942 | + | 651053007 |
| 48 | 2070 | 2927 | - | 651333149 |
| 49 | 1967759 | 1968439 | + | 637000205 |
| 50 | 78317 | 81286 | - | 646564501 |

Table 12a: Data for Klebsiella, particularly K. pneumoniae

| No. | Scaffold.External.Accession | Scaffold.Name | best_pv |
|-----|------------------------------|---------------|---------|
| 1 | NC_007384 | Shigella sonnei Ss046: NC_007384 | 1.21395823682916e-119 |
| 2 | NC_011083 | Salmonella enterica subsp. enterica serovar Heidelberg str. SL476: NC_011083 | 1.70517984237646e-115 |
| 3 | NZ_ADWV01000045 | Escherichia coli MS 107-1 E_coliMS107-1-1.0.1_Cont44.1: NZ_ADWV01000045 | 3.12333368944279e-110 |

(continued)

| No. | Scaffold.External. Accession | Scaffold.Name | best_pv |
|---|---|---|---|
| 4 | NC_009651 | Klebsiella pneumoniae subsp. pneumoniae MGH 78578 plasmid pKPN5: NC_009651 | 1.8490215255217e-103 |
| 5 | NZ_ABFH01000001 | Salmonella enterica subsp. enterica serovar Virchow str. SL491, unfinished sequence: NZ_ABFH01000001 | 4.16211077482132e-97 |
| 6 | CP002910 | Klebsiella pneumoniae KCTC 2242: CP002910 | 1.34397014479151e-80 |
| 7 | CP002910 | Klebsiella pneumoniae KCTC 2242: CP002910 | 1.21342816034335e-71 |
| 8 | NZ_ACZD01000070 | Klebsiella pneumoniae subsp. rhinoscleromatis ATCC 13884 contig00070: NZ_ACZD01000070 | 4.42089689687353e-61 |
| 9 | NC_013850 | Klebsiella variicola At-22 chromosome: NC_013850 | 8.41004168548543e-61 |
| 10 | NC_011282 | Klebsiella pneumoniae 342 plasmid pKP187: NC_011282 | 8.41004168548543e-61 |
| 11 | NZ_ACZD01000070 | Klebsiella pneumoniae subsp. rhinoscleromatis ATCC 13884 contig00070: NZ_ACZD01000070 | 8.41004168548543e-61 |
| 12 | NZ_ADTP01000323 | Escherichia coli MS 69-1 E_coli69-1-1.0_Cont630.1: NZ_ADTP01000323 | 4.94539115664213e-60 |
| 13 | NC_009793 | Citrobacter koseri ATCC BAA-895 plasmid pCKO3: NC_009793 | 5.71878021049123e-59 |
| 14 | NZ_ACZD01000005 | Klebsiella pneumoniae subsp. rhinoscleromatis ATCC 13884 contig00005: NZ_ACZD01000005 | 1.22977045856913e-58 |
| 15 | NZ_ACZD01000127 | Klebsiella pneumoniae subsp. rhinoscleromatis ATCC 13884 contig00129: NZ_ACZD01000127 | 1.49068550300511e-58 |
| 16 | NZ_GG745515 | Klebsiella sp. 1_1_55 genomic scaffold supercont1.8: NZ_GG745515 | 7.09566707309011e-58 |
| 17 | NZ_AAJX01000089 | Escherichia coli B171, unfinished sequence: NZ_AAJX01000089 | 1.65409147045657e-56 |
| 18 | NZ_ACZD01000005 | Klebsiella pneumoniae subsp. rhinoscleromatis ATCC 13884 contig00005: NZ_ACZD01000005 | 7.54090407593199e-55 |
| 19 | NZ_AFBO01000042 | Klebsiella sp. MS 92-3 K_spMS92-3-1.0_Cont91.1: NZ_AFBO01000042 | 2.8348333685311e-54 |
| 20 | NC_009649 | Klebsiella pneumoniae subsp. pneumoniae MGH 78578 plasmid pKPN3: NC_009649 | 2.8348333685311e-54 |
| 21 | CP002910 | Klebsiella pneumoniae KCTC 2242: CP002910 | 2.83530891190408e-54 |
| 22 | NC_009649 | Klebsiella pneumoniae subsp. pneumoniae MGH 78578 plasmid pKPN3: NC_009649 | 4.89541334527925e-54 |
| 23 | NZ_AFBO01000042 | Klebsiella sp. MS 92-3 K_spMS92-3-1.0_Cont91.1: NZ_AFBO01000042 | 2.18592863711471e-53 |
| 24 | NC_009649 | Klebsiella pneumoniae subsp. pneumoniae MGH 78578 plasmid pKPN3: NC_009649 | 2.18592863711471e-53 |
| 25 | NZ_ACZD01000127 | Klebsiella pneumoniae subsp. rhinoscleromatis ATCC 13884 contig00129: NZ_ACZD01000127 | 1.08576020331202e-52 |
| 26 | NZ_ACZD01000127 | Klebsiella pneumoniae subsp. rhinoscleromatis ATCC 13884 contig00129: NZ_ACZD01000127 | 1.37521017661703e-52 |

(continued)

| No. | Scaffold.External. Accession | Scaffold.Name | best_pv |
|---|---|---|---|
| 27 | NZ_ ACZD01000127 | Klebsiella pneumoniae subsp. rhinoscleromatis ATCC 13884 contig00129: NZ_ACZD01000127 | 2.1362800317976e-52 |
| 28 | NC_014121 | Enterobacter cloacae subsp. cloacae ATCC 13047 chromosome: NC_014121 | 2.21381524453565e-51 |
| 29 | CP002910 | Klebsiella pneumoniae KCTC 2242: CP002910 | 6.53846908249994e-51 |
| 30 | NZ_GG745512 | Klebsiella sp. 1_1_55 genomic scaffold supercont1.5: NZ_GG745512 | 2.78992804201171e-50 |
| 31 | NZ_ CAAZ01000154 | Salmonella enterica subsp. enterica serovar Typhi str. E98-3139, unfinished sequence: NZ_CAAZ01000154 | 4.10919139010377e-50 |
| 32 | NZ_ ACZD01000127 | Klebsiella pneumoniae subsp. rhinoscleromatis ATCC 13884 contig00129: NZ_ACZD01000127 | 6.10670380866266e-50 |
| 33 | NZ_ ACZD01000127 | Klebsiella pneumoniae subsp. rhinoscleromatis ATCC 13884 contig00129: NZ_ACZD01000127 | 1.00996840886331e-49 |
| 34 | CP002910 | Klebsiella pneumoniae KCTC 2242: CP002910 | 1.33344542353295e-48 |
| 35 | CP002910 | Klebsiella pneumoniae KCTC 2242: CP002910 | 3.00269800428115e-48 |
| 36 | CP002910 | Klebsiella pneumoniae KCTC 2242: CP002910 | 3.13126971411145e-48 |
| 37 | NZ_ ACZD01000127 | Klebsiella pneumoniae subsp. rhinoscleromatis ATCC 13884 contig00129: NZ_ACZD01000127 | 3.13126971411145e-48 |
| 38 | NC_014121 | Enterobacter cloacae subsp. cloacae ATCC 13047 chromosome: NC_014121 | 3.79149056816214e-48 |
| 39 | NZ_ AFBO01000625 | Klebsiella sp. MS 92-3 K_spMS92-3-1.0_Cont1175.2: NZ_AFBO01000625 | 2.5975548233422e-47 |
| 40 | NZ_ ACZD01000127 | Klebsiella pneumoniae subsp. rhinoscleromatis ATCC 13884 contig00129: NZ_ACZD01000127 | 1.05391699509183e-46 |
| 41 | NC_011092 | Salmonella enterica subsp. enterica serovar Schwarzengrund str. CVM19633 plasmid pCVM19633_110: NC_011092 | 4.42178242563847e-46 |
| 42 | NC_013850 | Klebsiella variicola At-22 chromosome: NC_013850 | 6.65483065085068e-46 |
| 43 | NC_012912 | Dickeya zeae Ech1591: NC_012912 | 1.05262166415392e-45 |
| 44 | NC_013509 | Edwardsiella tarda EIB202 plasmid pEIB202: NC_ 013509 | 1.24074600175825e-45 |
| 45 | AP011957 | Salmonella enterica subsp. enterica serovar Typhimurium str. | 2.46270503642371e-45 |
| | | T000240 DNA: AP011957 | |
| 46 | NC_006856 | Salmonella enterica subsp. enterica serovar Choleraesuis str. SC-B67 plasmid pSC138: NC_006856 | 9.02631418377704e-45 |
| 47 | AP011957 | Salmonella enterica subsp. enterica serovar Typhimurium str. T000240 DNA: AP011957 | 3.37821430101762e-44 |
| 48 | NZ_ AFBO01000232 | Klebsiella sp. MS 92-3 K_spMS92-3-1.0_Cont505.1: NZ_AFBO01000232 | 4.87973844618098e-43 |
| 49 | NC_004631 | Salmonella enterica subsp. enterica serovar Typhi Ty2: NC_004631 | 1.54269244019591e-42 |

(continued)

| No. | Scaffold.External. Accession | Scaffold.Name | best_pv |
|---|---|---|---|
| 50 | NC_006625 | Klebsiella pneumoniae NTUH-K2044 plasmid pK2044: NC_006625 | 1.89874529968327e-42 |

Table 12b: Data for Klebsiella, particularly K. pneumoniae (continued)

| No. | sign_phenos | sign_phenos_class | best_pheno | best_pheno_class |
|---|---|---|---|---|
| 1 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 2 | A/S;AUG;AZT;CAX;CAZ;CFT;CP; CPE; CRM;ETP;GM;LVX;P/T;TO; T/S | aminoglycoside;fluoroquinolone; lactam;other | AUG | lactam |
| 3 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 4 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 5 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 6 | A/S;AUG;AZT;CAX;CAZ;CFT;CP; CPE; <br> CRM;ETP;GM;LVX;P/T;TO;T/S | aminoglycoside;fluoroquinolone; lactam;other | ETP | lactam |
| 7 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 8 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 9 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 10 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 11 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 12 | A/S;AUG;AZT;CAX;CAZ;CFT;CP; CPE; CRM;ETP;GM;LVX;P/T;TO; T/S | aminoglycoside;fluoroquinolone; lactam;other | CAZ | lactam |
| 13 | A/S;AUG;AZT;CAX;CAZ;CFT;CP; CPE; CRM;ETP;GM;LVX;P/T;TO; T/S | aminoglycoside;fluoroquinolone; lactam;other | AUG | lactam |

(continued)

| No. | sign_phenos | sign_phenos_class | best_pheno | best_pheno_ class |
|---|---|---|---|---|
| 14 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 15 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 16 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 17 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 18 | A/S;AUG;AZT;CAX;CAZ;CFT;CP; CPE; CRM;ETP;GM;LVX;P/T;TO;T/S | aminoglycoside;fluoroquinolone; lactam;other | ETP | lactam |
| 19 | A/S;AUG;AZT;CAX;CAZ;CFT;CP; CPE; CRM;ETP;GM;LVX;P/T;TO; T/S | aminoglycoside;fluoroquinolone; lactam;other | P/T | lactam |
| 20 | A/S;AUG;AZT;CAX;CAZ;CFT;CP; CPE; CRM;ETP;GM;LVX;P/T;TO; T/S | aminoglycoside;fluoroquinolone; lactam;other | P/T | lactam |
| 21 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 22 | A/S;AUG;AZT;CAX;CAZ;CFT;CP; CPE; CRM;ETP;GM;LVX;P/T;TO; T/S | aminoglycoside;fluoroquinolone; lactam;other | P/T | lactam |
| 23 | A/S;AUG;AZT;CAX;CAZ;CFT;CP; CPE; CRM;ETP;GM;LVX;P/T;TO; T/S | aminoglycoside;fluoroquinolone; lactam;other | P/T | lactam |
| 24 | A/S;AUG;AZT;CAX;CAZ;CFT;CP; CPE; CRM;ETP;GM;LVX;P/T;TO; T/S | aminoglycoside;fluoroquinolone; lactam;other | P/T | lactam |
| 25 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 26 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 27 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 28 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |

(continued)

| No. | sign_phenos | sign_phenos_class | best_pheno | best_pheno_ class |
|---|---|---|---|---|
| 29 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 30 | A/S;AUG;AZT;CAX;CAZ;CFT;CP; CPE; | aminoglycoside;fluoroquinolone; lactam;other | ETP | lactam |
|  | CRM;ETP;GM;LVX;P/T;TO;T/S |  |  |  |
| 31 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 32 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 33 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 34 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 35 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 36 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 37 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 38 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 39 | A/S;AUG;AZT;CAX;CAZ;CFT;CP; CPE; CRM;GM;LVX;P/T;TO;T/S | aminoglycoside;fluoroquinolone; lactam;other | T/S | other |
| 40 | A/S;AUG;AZT;CAX;CAZ;CFT;CP; CPE; CRM;ETP;GM;LVX;P/T;TO; T/S | aminoglycoside;fluoroquinolone; lactam;other | CP | fluoroquinolo ne |
| 41 | A/S;AUG;AZT;CAX;CAZ;CFT;CP; CPE; CRM;ETP;GM;LVX;P/T;TO; T/S | aminoglycoside;fluoroquinolone; lactam;other | AUG | lactam |
| 42 | AUG;AZT;CAX;CAZ;CFT;CP;CPE; CRM; | aminoglycoside;fluoroquinolone; lactam;other | ETP | lactam |
|  | ETP;LVX;P/T;TO;T/S |  |  |  |
| 43 | AUG;AZT;CAX;CAZ;CFT;CP;CPE; CRM; ETP;LVX;P/T;TO;T/S | aminoglycoside;fluoroquinolone; lactam;other | ETP | lactam |

(continued)

| No. | sign_phenos | sign_phenos_class | best_ pheno | best_pheno_ class |
|---|---|---|---|---|
| 44 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 45 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 46 | A/S;AUG;AZT;CAX;CAZ;CFT;CP; CPE; CRM;ETP;GM;LVX;P/T;TO; T/S | aminoglycoside;fluoroquinolone; lactam;other | TO | aminoglycosid e |
| 47 | A/S;AUG;AZT;CAX;CAZ;CFT;CP; CPE; CRM;ETP;GM;LVX;P/T;TO; T/S | aminoglycoside;fluoroquinolone; lactam;other | AUG | lactam |
| 48 | A/S;AUG;AZT;CAX;CAZ;CFT;CP; CPE; CRM;ETP;GM;LVX;P/T;TO; T/S | aminoglycoside;fluoroquinolone; lactam;other | T/S | other |
| 49 | A/S;AUG;AZT;CAX;CAZ;CFT;CP; CPE; CRM;ETP;GM;LVX;P/T;TO; T/S | aminoglycoside;fluoroquinolone; lactam;other | ETP | lactam |
| 50 | A/S;AUG;AZT;CAX;CAZ;CFT;CP; CPE; CRM;ETP;GM;LVX;P/T;TO; T/S | aminoglycoside;fluoroquinolone; lactam;other | T/S | other |

Table 12c: Data for Klebsiella, particularly K. pneumoniae (continued)

| No. | num_aminoglycoside | num_fluoroquinolone | num_lactam | num_other |
|---|---|---|---|---|
| 1 | 2 | 2 | 10 | 1 |
| 2 | 2 | 2 | 10 | 1 |
| 3 | 2 | 2 | 10 | 1 |
| 4 | 2 | 2 | 10 | 1 |
| 5 | 2 | 2 | 10 | 1 |
| 6 | 2 | 2 | 10 | 1 |
| 7 | 2 | 2 | 10 | 1 |
| 8 | 2 | 2 | 10 | 1 |
| 9 | 2 | 2 | 10 | 1 |
| 10 | 2 | 2 | 10 | 1 |
| 11 | 2 | 2 | 10 | 1 |
| 12 | 2 | 2 | 10 | 1 |
| 13 | 2 | 2 | 10 | 1 |
| 14 | 2 | 2 | 10 | 1 |
| 15 | 2 | 2 | 10 | 1 |
| 16 | 2 | 2 | 10 | 1 |
| 17 | 2 | 2 | 10 | 1 |

(continued)

| No. | num_aminoglycoside | num_fluoroquinolone | num_lactam | num_other |
|---|---|---|---|---|
| 18 | 2 | 2 | 10 | 1 |
| 19 | 2 | 2 | 10 | 1 |
| 20 | 2 | 2 | 10 | 1 |
| 21 | 2 | 2 | 10 | 1 |
| 22 | 2 | 2 | 10 | 1 |
| 23 | 2 | 2 | 10 | 1 |
| 24 | 2 | 2 | 10 | 1 |
| 25 | 2 | 2 | 10 | 1 |
| 26 | 2 | 2 | 10 | 1 |
| 27 | 2 | 2 | 10 | 1 |
| 28 | 2 | 2 | 10 | 1 |
| 29 | 2 | 2 | 10 | 1 |
| 30 | 2 | 2 | 10 | 1 |
| 31 | 2 | 2 | 10 | 1 |
| 32 | 2 | 2 | 10 | 1 |
| 33 | 2 | 2 | 10 | 1 |
| 34 | 2 | 2 | 10 | 1 |
| 35 | 2 | 2 | 10 | 1 |
| 36 | 2 | 2 | 10 | 1 |
| 37 | 2 | 2 | 10 | 1 |
| 38 | 2 | 2 | 10 | 1 |
| 39 | 2 | 2 | 9 | 1 |
| 40 | 2 | 2 | 10 | 1 |
| 41 | 2 | 2 | 10 | 1 |
| 42 | 1 | 2 | 9 | 1 |
| 43 | 1 | 2 | 9 | 1 |
| 44 | 2 | 2 | 10 | 1 |
| 45 | 2 | 2 | 10 | 1 |
| 46 | 2 | 2 | 10 | 1 |
| 47 | 2 | 2 | 10 | 1 |
| 48 | 2 | 2 | 10 | 1 |
| 49 | 2 | 2 | 10 | 1 |
| 50 | 2 | 2 | 10 | 1 |

Table 12d: Data for Klebsiella, particularly K. pneumoniae (continued)

| No. | A/S_pv_adj | AUG_pv_adj | AZT_pv_adj | CAX_pv_adj | CAZ_pv_adj |
|---|---|---|---|---|---|
| 1 | 1.28E-76 | 1.24E-105 | 4.06E-95 | 3.39E-95 | 5.99E-93 |

(continued)

| No. | A/S_pv_adj | AUG_pv_adj | AZT_pv_adj | CAX_pv_adj | CAZ_pv_adj |
|-----|-----------|-----------|-----------|-----------|-----------|
| 2 | 1.18E-98 | 1.71E-115 | 9.09E-91 | 6.55E-88 | 1.66E-96 |
| 3 | 1.08E-81 | 5.85E-86 | 2.76E-94 | 4.16E-97 | 6.84E-100 |
| 4 | 5.55E-76 | 3.05E-84 | 1.38E-91 | 2.64E-96 | 5.33E-94 |
| 5 | 1.22E-70 | 3.41E-71 | 1.41E-80 | 1.50E-83 | 6.22E-90 |
| 6 | 7.70E-21 | 1.03E-41 | 8.20E-48 | 2.50E-44 | 2.69E-46 |
| 7 | 8.54E-16 | 4.34E-29 | 4.54E-38 | 5.50E-34 | 1.34E-34 |
| 8 | 2.62E-12 | 1.74E-24 | 1.49E-26 | 2.74E-25 | 2.84E-24 |
| 9 | 5.20E-12 | 2.50E-24 | 3.69E-26 | 7.31E-25 | 7.66E-24 |
| 10 | 5.20E-12 | 2.50E-24 | 3.69E-26 | 7.31E-25 | 7.66E-24 |
| 11 | 5.20E-12 | 2.50E-24 | 3.69E-26 | 7.31E-25 | 7.66E-24 |
| 12 | 3.32E-43 | 1.75E-59 | 1.48E-54 | 9.28E-55 | 4.95E-60 |
| 13 | 2.41E-43 | 5.72E-59 | 4.20E-53 | 2.96E-53 | 2.14E-58 |
| 14 | 2.47E-16 | 8.57E-27 | 1.49E-34 | 1.96E-31 | 8.83E-33 |
| 15 | 1.72E-35 | 6.40E-55 | 1.25E-44 | 4.12E-43 | 1.61E-46 |
| 16 | 3.04E-18 | 5.19E-32 | 4.83E-36 | 2.23E-35 | 1.91E-32 |
| 17 | 1.02E-47 | 9.19E-45 | 1.85E-48 | 9.17E-53 | 6.18E-56 |
| 18 | 3.51E-17 | 3.04E-23 | 8.29E-30 | 1.21E-27 | 9.29E-29 |
| 19 | 1.03E-40 | 7.90E-52 | 1.41E-37 | 4.81E-39 | 9.97E-38 |
| 20 | 1.03E-40 | 7.90E-52 | 1.41E-37 | 4.81E-39 | 9.97E-38 |
| 21 | 8.56E-34 | 4.84E-53 | 8.75E-43 | 2.41E-41 | 9.43E-45 |
| 22 | 5.13E-42 | 1.10E-52 | 3.02E-38 | 9.55E-40 | 3.15E-38 |
| 23 | 1.11E-41 | 3.84E-52 | 1.00E-37 | 3.11E-39 | 8.03E-38 |
| 24 | 1.11E-41 | 3.84E-52 | 1.00E-37 | 3.11E-39 | 8.03E-38 |
| 25 | 8.85E-33 | 1.54E-51 | 2.83E-42 | 1.24E-40 | 2.88E-44 |
| 26 | 1.96E-32 | 2.54E-52 | 2.98E-42 | 1.41E-40 | 3.55E-44 |
| 27 | 5.12E-32 | 6.70E-50 | 1.84E-40 | 2.34E-38 | 1.33E-42 |
| 28 | 1.65E-30 | 3.00E-47 | 1.96E-38 | 1.37E-36 | 4.94E-39 |
| 29 | 4.43E-31 | 1.45E-47 | 2.29E-39 | 1.06E-37 | 3.63E-40 |
| 30 | 6.99E-12 | 2.37E-26 | 2.77E-24 | 5.28E-24 | 2.55E-26 |
| 31 | 2.78E-31 | 7.62E-46 | 1.11E-38 | 5.40E-37 | 1.95E-39 |
| 32 | 9.62E-32 | 1.45E-47 | 2.29E-39 | 1.06E-37 | 3.63E-40 |
| 33 | 2.27E-31 | 2.28E-47 | 3.20E-39 | 1.66E-37 | 5.77E-40 |
| 34 | 9.74E-28 | 9.40E-43 | 1.44E-35 | 7.30E-34 | 3.24E-36 |
| 35 | 1.57E-28 | 2.77E-42 | 6.21E-36 | 1.59E-34 | 2.14E-36 |
| 36 | 3.41E-27 | 2.34E-43 | 7.21E-36 | 4.53E-33 | 9.07E-37 |
| 37 | 3.41E-27 | 2.34E-43 | 7.21E-36 | 4.53E-33 | 9.07E-37 |
| 38 | 2.00E-30 | 7.61E-47 | 1.00E-38 | 1.06E-37 | 3.63E-40 |
| 39 | 8.29E-47 | 2.48E-33 | 3.67E-37 | 5.57E-42 | 2.74E-43 |

(continued)

| No. | A/S_pv_adj | AUG_pv_adj | AZT_pv_adj | CAX_pv_adj | CAZ_pv_adj |
|---|---|---|---|---|---|
| 40 | 1.53E-27 | 6.97E-43 | 2.91E-36 | 2.70E-35 | 5.96E-38 |
| 41 | 1.75E-44 | 4.42E-46 | 2.16E-37 | 1.21E-36 | 1.12E-42 |
| 42 | 4.51E-02 | 4.66E-12 | 9.77E-17 | 3.13E-16 | 9.90E-15 |
| 43 | 5.37E-02 | 5.73E-12 | 1.27E-16 | 6.11E-16 | 1.15E-14 |
| 44 | 6.65E-31 | 4.17E-32 | 1.40E-25 | 3.69E-27 | 1.78E-29 |
| 45 | 6.15E-34 | 5.78E-41 | 2.29E-36 | 9.94E-38 | 1.48E-38 |
| 46 | 3.01E-32 | 3.12E-38 | 7.22E-37 | 3.96E-36 | 6.00E-37 |
| 47 | 4.35E-41 | 3.38E-44 | 1.49E-30 | 1.42E-33 | 1.82E-29 |
| 48 | 6.41E-31 | 1.63E-41 | 3.24E-33 | 2.26E-36 | 1.97E-31 |
| 49 | 2.54E-11 | 2.61E-18 | 8.71E-21 | 1.23E-18 | 1.87E-19 |
| 50 | 2.38E-23 | 3.76E-29 | 2.21E-27 | 4.97E-29 | 1.37E-29 |

Table 12e: Data for Klebsiella, particularly K. pneumoniae (continued)

| No. | CFT_pv_adj | CP_pv_adj | CPE_pv_adj | CRM_pv_adj | ETP_pv_adj |
|---|---|---|---|---|---|
| 1 | 2.17E-99 | 2.40E-85 | 2.31E-68 | 1.21E-82 | 1.87E-58 |
| 2 | 2.52E-83 | 1.28E-61 | 4.61E-66 | 7.83E-57 | 8.20E-51 |
| 3 | 3.93E-93 | 2.17E-58 | 2.89E-43 | 3.32E-73 | 3.54E-24 |
| 4 | 1.52E-90 | 4.25E-60 | 1.75E-43 | 2.59E-71 | 1.63E-23 |
| 5 | 3.61E-77 | 1.76E-47 | 1.47E-36 | 5.14E-62 | 6.31E-21 |
| 6 | 2.84E-46 | 1.53E-54 | 2.89E-63 | 1.06E-36 | 1.34E-80 |
| 7 | 8.43E-36 | 1.96E-43 | 3.06E-55 | 3.95E-29 | 1.21E-71 |
| 8 | 5.55E-27 | 2.63E-33 | 6.90E-40 | 1.01E-20 | 4.42E-61 |
| 9 | 1.58E-26 | 5.49E-33 | 1.32E-39 | 2.40E-20 | 8.41E-61 |
| 10 | 1.58E-26 | 5.49E-33 | 1.32E-39 | 2.40E-20 | 8.41E-61 |
| 11 | 1.58E-26 | 5.49E-33 | 1.32E-39 | 4.98E-21 | 8.41E-61 |
| 12 | 4.39E-54 | 4.96E-60 | 1.80E-51 | 7.75E-50 | 7.02E-53 |
| 13 | 1.26E-52 | 1.35E-58 | 1.72E-50 | 4.57E-49 | 3.71E-52 |
| 14 | 1.28E-31 | 2.00E-30 | 6.27E-42 | 1.20E-29 | 1.23E-58 |
| 15 | 2.05E-41 | 2.56E-52 | 7.76E-46 | 3.74E-32 | 5.20E-48 |
| 16 | 1.52E-36 | 2.10E-37 | 5.58E-48 | 5.62E-28 | 7.10E-58 |
| 17 | 9.26E-49 | 4.16E-22 | 1.22E-19 | 9.42E-36 | 5.55E-05 |
| 18 | 6.27E-28 | 1.02E-25 | 4.83E-36 | 7.62E-29 | 7.54E-55 |
| 19 | 9.92E-40 | 2.65E-39 | 2.28E-41 | 4.71E-28 | 1.10E-32 |
| 20 | 9.92E-40 | 2.65E-39 | 2.28E-41 | 4.71E-28 | 1.10E-32 |
| 21 | 1.47E-40 | 1.18E-49 | 1.74E-45 | 2.18E-32 | 1.46E-46 |
| 22 | 2.00E-40 | 6.22E-40 | 7.04E-42 | 1.08E-28 | 5.10E-33 |
| 23 | 6.77E-40 | 2.32E-39 | 3.78E-41 | 2.22E-28 | 3.70E-32 |

(continued)

| No. | CFT_pv_adj | CP_pv_adj | CPE_pv_adj | CRM_pv_adj | ETP_pv_adj |
|---|---|---|---|---|---|
| 24 | 6.77E-40 | 2.32E-39 | 3.78E-41 | 2.22E-28 | 3.70E-32 |
| 25 | 4.69E-40 | 3.62E-49 | 1.06E-44 | 1.70E-33 | 2.11E-45 |
| 26 | 4.89E-40 | 1.97E-49 | 1.88E-45 | 2.34E-32 | 8.59E-46 |
| 27 | 5.94E-39 | 1.13E-47 | 2.46E-43 | 3.44E-33 | 1.93E-49 |
| 28 | 3.32E-36 | 2.21E-51 | 1.87E-44 | 1.56E-31 | 1.83E-41 |
| 29 | 2.69E-37 | 6.54E-51 | 4.48E-44 | 2.19E-30 | 3.44E-41 |
| 30 | 1.39E-24 | 6.75E-31 | 1.12E-35 | 5.11E-15 | 2.79E-50 |
| 31 | 1.34E-36 | 4.11E-50 | 1.67E-43 | 1.45E-31 | 8.85E-41 |
| 32 | 2.69E-37 | 6.11E-50 | 4.48E-44 | 2.19E-30 | 3.44E-41 |
| 33 | 6.74E-37 | 1.01E-49 | 6.91E-44 | 1.07E-30 | 4.69E-41 |
| 34 | 3.28E-34 | 1.33E-48 | 1.41E-40 | 1.52E-30 | 7.40E-43 |
| 35 | 3.72E-36 | 3.00E-48 | 1.67E-40 | 8.05E-31 | 2.56E-43 |
| 36 | 1.04E-34 | 3.13E-48 | 5.88E-41 | 1.06E-29 | 3.84E-43 |
| 37 | 1.04E-34 | 3.13E-48 | 5.88E-41 | 1.06E-29 | 3.84E-43 |
| 38 | 2.69E-37 | 3.79E-48 | 4.21E-43 | 9.89E-32 | 5.95E-40 |
| 39 | 2.84E-38 | 3.78E-14 | 1.15E-05 | 9.70E-31 | 9.25E-01 |
| 40 | 1.88E-36 | 1.05E-46 | 3.80E-41 | 1.01E-30 | 2.77E-43 |
| 41 | 4.01E-36 | 1.78E-19 | 2.93E-16 | 1.27E-21 | 2.46E-10 |
| 42 | 5.45E-16 | 4.08E-18 | 6.25E-28 | 7.56E-08 | 6.65E-46 |
| 43 | 1.13E-15 | 5.57E-18 | 9.82E-28 | 1.16E-07 | 1.05E-45 |
| 44 | 2.55E-25 | 6.58E-14 | 7.47E-22 | 3.89E-16 | 2.18E-20 |
| 45 | 5.14E-35 | 2.41E-27 | 5.02E-15 | 4.61E-29 | 2.22E-12 |
| 46 | 1.40E-36 | 2.78E-33 | 1.06E-29 | 5.12E-30 | 2.25E-28 |
| 47 | 3.46E-32 | 1.18E-32 | 1.62E-32 | 2.49E-29 | 1.62E-26 |
| 48 | 6.74E-36 | 1.36E-34 | 2.38E-34 | 1.27E-25 | 6.79E-29 |
| 49 | 9.64E-20 | 3.40E-22 | 1.36E-30 | 8.87E-18 | 1.54E-42 |
| 50 | 1.19E-26 | 1.97E-18 | 9.42E-19 | 1.46E-19 | 1.52E-12 |

Table 12f: Data for Klebsiella, particularly K. pneumoniae (continued)

| No. | GM_pv_adj | LVX_pv_adj | P/T_pv_adj | TO_pv_adj | T/S_pv_adj |
|---|---|---|---|---|---|
| 1 | 6.24E-60 | 3.97E-80 | 7.18E-96 | 1.21E-119 | 1.12E-106 |
| 2 | 6.10E-67 | 3.76E-55 | 1.20E-67 | 2.77E-105 | 3.59E-83 |
| 3 | 6.00E-68 | 3.14E-50 | 5.58E-70 | 1.37E-91 | 3.12E-110 |
| 4 | 3.06E-63 | 4.39E-52 | 7.17E-70 | 6.65E-91 | 1.85E-103 |
| 5 | 5.29E-59 | 6.80E-39 | 4.00E-51 | 1.40E-82 | 4.16E-97 |
| 6 | 5.64E-09 | 3.19E-59 | 3.01E-52 | 7.11E-48 | 1.50E-31 |
| 7 | 1.61E-05 | 6.12E-48 | 2.20E-40 | 1.45E-34 | 2.10E-20 |

(continued)

| No. | GM_pv_adj | LVX_pv_adj | P/T_pv_adj | TO_pv_adj | T/S_pv_adj |
|---|---|---|---|---|---|
| 8 | 1.57E-06 | 6.85E-37 | 6.06E-35 | 1.79E-29 | 2.02E-18 |
| 9 | 2.46E-06 | 1.41E-36 | 1.30E-34 | 3.62E-29 | 2.62E-18 |
| 10 | 2.46E-06 | 1.41E-36 | 1.30E-34 | 3.62E-29 | 2.62E-18 |
| 11 | 2.46E-06 | 1.41E-36 | 1.30E-34 | 3.62E-29 | 2.62E-18 |
| 12 | 2.30E-25 | 1.32E-53 | 6.01E-52 | 5.28E-59 | 5.07E-39 |
| 13 | 1.61E-25 | 2.14E-52 | 1.19E-50 | 1.22E-58 | 6.30E-38 |
| 14 | 6.89E-05 | 6.38E-32 | 9.66E-29 | 7.19E-31 | 3.05E-15 |
| 15 | 1.55E-23 | 1.89E-48 | 1.36E-48 | 1.49E-58 | 1.23E-46 |
| 16 | 7.14E-10 | 1.99E-38 | 1.30E-34 | 9.13E-37 | 1.47E-21 |
| 17 | 6.27E-40 | 9.96E-18 | 4.98E-31 | 1.50E-43 | 1.65E-56 |
| 18 | 7.35E-05 | 4.57E-28 | 1.61E-25 | 2.68E-27 | 2.50E-15 |
| 19 | 1.16E-19 | 5.28E-36 | 2.83E-54 | 2.59E-42 | 9.74E-48 |
| 20 | 1.16E-19 | 5.28E-36 | 2.83E-54 | 2.59E-42 | 9.74E-48 |
| 21 | 6.44E-22 | 5.93E-45 | 3.28E-47 | 2.84E-54 | 2.84E-46 |
| 22 | 6.19E-20 | 1.49E-36 | 4.90E-54 | 5.19E-43 | 1.61E-47 |
| 23 | 4.80E-20 | 5.67E-36 | 2.19E-53 | 1.90E-42 | 7.35E-49 |
| 24 | 1.78E-19 | 5.67E-36 | 2.19E-53 | 1.90E-42 | 6.83E-48 |
| 25 | 1.71E-21 | 3.92E-44 | 2.20E-46 | 1.09E-52 | 8.26E-45 |
| 26 | 1.49E-20 | 1.39E-44 | 1.02E-46 | 1.38E-52 | 9.52E-46 |
| 27 | 9.23E-19 | 4.68E-44 | 1.13E-48 | 2.14E-52 | 7.18E-42 |
| 28 | 1.04E-18 | 2.43E-47 | 2.68E-42 | 8.73E-48 | 1.22E-43 |
| 29 | 1.81E-19 | 6.48E-47 | 5.36E-42 | 7.69E-49 | 5.28E-43 |
| 30 | 1.67E-04 | 1.19E-33 | 1.48E-27 | 1.33E-24 | 2.01E-19 |
| 31 | 2.71E-19 | 3.53E-46 | 2.81E-41 | 2.57E-47 | 3.11E-43 |
| 32 | 3.53E-20 | 6.26E-46 | 5.36E-42 | 7.69E-49 | 5.57E-44 |
| 33 | 4.08E-20 | 9.51E-46 | 1.44E-41 | 1.11E-48 | 8.48E-44 |
| 34 | 9.00E-16 | 3.30E-45 | 1.53E-41 | 3.99E-46 | 1.50E-39 |
| 35 | 4.69E-14 | 4.82E-45 | 1.45E-39 | 3.68E-47 | 5.85E-39 |
| 36 | 6.72E-15 | 8.68E-45 | 4.34E-42 | 8.00E-45 | 1.33E-38 |
| 37 | 6.72E-15 | 8.68E-45 | 4.34E-42 | 8.00E-45 | 1.33E-38 |
| 38 | 4.79E-19 | 4.19E-45 | 5.36E-42 | 3.42E-47 | 5.28E-43 |
| 39 | 1.94E-40 | 1.06E-10 | 6.27E-21 | 4.23E-35 | 2.60E-47 |
| 40 | 1.53E-15 | 3.13E-43 | 2.72E-42 | 3.02E-45 | 1.22E-39 |
| 41 | 9.94E-38 | 3.55E-16 | 4.46E-22 | 7.61E-44 | 1.24E-41 |
| 42 | 2.34E-02 | 4.47E-21 | 3.44E-16 | 3.10E-15 | 2.24E-09 |
| 43 | 2.39E-02 | 1.02E-20 | 4.32E-16 | 6.38E-15 | 3.86E-09 |
| 44 | 3.44E-22 | 5.51E-13 | 6.56E-18 | 4.39E-24 | 1.24E-45 |
| 45 | 1.36E-21 | 1.14E-24 | 2.38E-33 | 2.46E-45 | 1.53E-29 |

(continued)

| No. | GM_pv_adj | LVX_pv_adj | P/T_pv_adj | TO_pv_adj | T/S_pv_adj |
|---|---|---|---|---|---|
| 46 | 1.36E-19 | 4.39E-32 | 1.03E-35 | 9.03E-45 | 5.01E-44 |
| 47 | 1.18E-14 | 5.29E-30 | 3.76E-40 | 3.55E-44 | 5.56E-38 |
| 48 | 4.91E-17 | 8.23E-33 | 1.06E-40 | 6.11E-39 | 4.88E-43 |
| 49 | 1.35E-04 | 2.25E-22 | 1.78E-19 | 5.28E-22 | 2.63E-12 |
| 50 | 8.68E-26 | 1.45E-15 | 1.73E-22 | 6.68E-34 | 1.90E-42 |

Table 12g: Data for Klebsiella, particularly K. pneumoniae (continued)

| No. | Locus.Tag | Gene. Symbol | GenBank.Accession | Chromosome |
|---|---|---|---|---|
| 1 | SSON_3896 | aadA | YP_312670 | |
| 2 | SeHA_C1580 | blaT | YP_002045448 | |
| 3 | HMPREF9345_ 05066 | | ZP_07100147 | |
| 4 | KPN_pKPN5p08201 | | YP_001338811 | plasmid pKPN5 |
| 5 | Salmoentericaenterica_010100000245 | | ZP_02702077 | |
| 6 | KPN2242_ 07235 | | AEJ97365 | |
| 7 | KPN2242_ 00750 | | AEJ96075 | |
| 8 | HMPREF0484_1390 | mgtA | ZP_06014374 | |
| 9 | Kvar_3592 | | YP_003440504 | |
| 10 | KPK_A0103 | | YP_002235751 | plasmid pKP187 |
| 11 | HMPREF0484_ 1392 | pacL | ZP_06014376 | |
| 12 | HMPREF9534_04427 | | ZP_07188952 | |
| 13 | CK0_pCK03p06155 | | YP_001456633 | plasmid pCKO3 |
| 14 | HMPREF0484_ 0044 | vmtV | ZP_0601302 9 | |
| 15 | HMPREF0484_ 2697 | | ZP_06015679 | |
| 16 | HMPREF0485_ 04762 | | ZP_06552358 | |
| 17 | EcolB_01004576 | | ZP_00708527 | |
| 18 | HMPREF0484_ 0046 | | ZP_06013031 | |
| 19 | HMPREF9538_00440 | | ZP_08302804 | |
| 20 | KPN_pKPN3p05893 | ptxE | YP_001338502 | plasmid pKPN3 |
| 21 | KPN2242_ 07425 | | AEJ97403 | |
| 22 | KPN_pKPN3p05890 | ptxB | YP_001338499 | plasmid pKPN3 |
| 23 | HMPREF9538_00443 | | ZP_08302807 | |
| 24 | KPN_pKPN3p05891 | ptxC | YP_001338500 | plasmid pKPN3 |
| 25 | HMPREF0484_ 2686 | | ZP_06015668 | |
| 26 | HMPREF0484_ 2687 | gpw | ZP_06015669 | |
| 27 | HMPREF0484_ 2684 | | ZP_06015666 | |
| 28 | ECL_04426 | | YP_003614904 | |
| 29 | KPN2242_07415 | | AEJ97401 | |

(continued)

| No. | Locus.Tag | Gene. Symbol | GenBank.Accession | Chromosome |
|-----|-----------|--------------|-------------------|------------|
| 30 | HMPREF0485_ 03923 | | ZP_06551519 | |
| 31 | Salmonellentericaenterica_010100016432 | | ZP_03412648 | |
| 32 | HMPREF0484_ 2698 | | ZP_06015680 | |
| 33 | HMPREF0484_ 2694 | fii | ZP_06015676 | |
| 34 | KPN2242_ 07345 | | AEJ97387 | |
| 35 | KPN2242_ 07330 | | AEJ97384 | |
| 36 | KPN2242_ 07325 | | AEJ97383 | |
| 37 | HMPREF0484_ 2676 | | ZP_06015658 | |
| 38 | ECL_04434 | | YP_003614912 | |
| 39 | HMPREF9538_04737 | | ZP_08307036 | |
| 40 | HMPREF0484_ 2673 | | ZP_06015655 | |
| 41 | SeSA_B0079 | | YP_002112958 | plasmid pCVM19633_110 |
| 42 | Kvar_2527 | | YP_003439449 | |
| 43 | Dd1591_2579 | | YP_003004893 | |
| 44 | ETAE_p041 | folP | YP_003297635 | plasmid pEIB202 |
| 45 | STMDT12_C3934 0 | | BAJ38877 | |
| 46 | SC026 | aph | YP_209331 | plasmid pSC138 |
| 47 | STMDT12_C39170 | | BAJ38860 | |
| 48 | HMPREF9538_01820 | | ZP_08304144 | |
| 49 | t1924 | exo | NP_805691 | |
| 50 | pK2044_00525 | | YP_001687935 | plasmid pK2044 |

Table 12h: Data for Klebsiella, particularly K. pneumoniae (continued)

| No. | Start.Coord | End.Coord | Strand | Scaffold.ID |
|-----|-------------|-----------|--------|-------------|
| 1 | 4110458 | 4111246 | - | 640427102 |
| 2 | 1525960 | 1526820 | + | 642555218 |
| 3 | 4715 | 5554 | - | 648294624 |
| 4 | 34908 | 35255 | - | 640753021 |
| 5 | 36757 | 37062 | + | 641778223 |
| 6 | 1535907 | 1536932 | - | 651053122 |
| 7 | 159235 | 160521 | - | 651053122 |
| 8 | 2865 | 3848 | - | 647009817 |
| 9 | 3765814 | 3768501 | - | 646312028 |
| 10 | 67987 | 70674 | + | 643348532 |
| 11 | 4691 | 5551 | - | 647009817 |
| 12 | 2120 | 2368 | + | 648289812 |
| 13 | 8632 | 8931 | + | 640753081 |

(continued)

| No. | Start.Coord | End.Coord | Strand | Scaffold.ID |
|-----|-------------|-----------|--------|-------------|
| 14 | 12911 | 13663 | + | 647009752 |
| 15 | 20402 | 22975 | + | 647009874 |
| 16 | 93128 | 94558 | + | 647536396 |
| 17 | 3555 | 4568 | + | 638359041 |
| 18 | 14573 | 15328 | + | 647009752 |
| 19 | 933 | 1943 | - | 651332959 |
| 20 | 38514 | 39383 | + | 640753019 |
| 21 | 1563447 | 1563746 | + | 651053122 |
| 22 | 35802 | 36665 | + | 640753019 |
| 23 | 3648 | 4475 | - | 651332959 |
| 24 | 36662 | 37501 | + | 640753019 |
| 25 | 11204 | 11776 | + | 647009874 |
| 26 | 11773 | 12132 | + | 647009874 |
| 27 | 10265 | 10696 | + | 647009874 |
| 28 | 4537618 | 4539336 | + | 646564624 |
| 29 | 1561697 | 1562869 | + | 651053122 |
| 30 | 51251 | 51370 | - | 647536393 |
| 31 | 154 | 357 | - | 643042634 |
| 32 | 23022 | 23267 | + | 647009874 |
| 33 | 19408 | 19923 | + | 647009874 |
| 34 | 1552497 | 1552712 | + | 651053122 |
| 35 | 1551079 | 1551729 | + | 651053122 |
| 36 | 1550023 | 1551075 | + | 651053122 |
| 37 | 6165 | 7229 | + | 647009874 |
| 38 | 4543715 | 4546123 | - | 646564624 |
| 39 | 1555 | 2055 | - | 651333542 |
| 40 | 2380 | 3411 | - | 647009874 |
| 41 | 68933 | 70240 | + | 642555219 |
| 42 | 2637299 | 2637790 | + | 646312028 |
| 43 | 2932956 | 2933891 | + | 644736389 |
| 44 | 32101 | 32916 | - | 646311959 |
| 45 | 4084530 | 4085237 | - | 651053007 |
| 46 | 21137 | 21952 | - | 637000354 |
| 47 | 4067283 | 4067942 | + | 651053007 |
| 48 | 2070 | 2927 | - | 651333149 |
| 49 | 1967759 | 1968439 | + | 637000205 |
| 50 | 78317 | 81286 | - | 646564501 |

Example 7: Proteus, particularly Proteus mirabilis, Proteus penneri and/or Proteus vulgaris

The procedure was carried out as in Example 1, except that the following microorganisms were used:

Bacterial Strains

The inventors selected 582 Proteus strains from the microbiology strain collection at Siemens Healthcare Diagnostics (West Sacramento, CA) for susceptibility testing and whole genome sequencing.
From MetaRef, 2318 centroids of Proteus species were used as reference sequences.
The results for Proteus are shown in Tables 13 (corresponding to Table 1) and 14 (corresponding to Table 2).

Table 13a: Data for Proteus species

| No. | Scaffold.External. Accession | Scaffold.Name | best_pv |
|---|---|---|---|
| 1 | NZ_ABVP01000020 | Proteus penneri ATCC 35198 P_penneri-1.1.1_Cont338: NZ_ABVP01000020 | 9.1377556355866e-62 |
| 2 | NZ_ABVP01000025 | Proteus penneri ATCC 35198 P_penneri-1.1.1_Cont344: NZ_ABVP01000025 | 9.1377556355866e-62 |
| 3 | NZ_ABVP01000025 | Proteus penneri ATCC 35198 P_penneri-1.1.1_Cont344: NZ_ABVP01000025 | 9.1377556355866e-62 |
| 4 | NZ_ABVP01000023 | Proteus penneri ATCC 35198 P_penneri-1.1.1_Cont336: NZ_ABVP01000023 | 9.1377556355866e-62 |
| 5 | NZ_ABVP01000026 | Proteus penneri ATCC 35198 P_penneri-1.1.1_Cont348: NZ_ABVP01000026 | 9.1377556355866e-62 |
| 6 | NZ_ABVP01000019 | Proteus penneri ATCC 35198 P_penneri-1.1.1_Cont6.1: NZ_ABVP01000019 | 9.1377556355866e-62 |
| 7 | NZ_ABVP01000021 | Proteus penneri ATCC 35198 P_penneri-1.1.1_Cont356: NZ_ABVP01000021 | 9.1377556355866e-62 |
| 8 | NC_010554 | Proteus mirabilis HI4320: NC_010554 | 9.1377556355866e-62 |
| 9 | NZ_ABVP01000022 | Proteus penneri ATCC 35198 P_penneri-1.1.1_Cont341: NZ_ABVP01000022 | 9.1377556355866e-62 |
| 10 | NZ_ABVP01000026 | Proteus penneri ATCC 35198 P_penneri-1.1.1_Cont348: NZ_ABVP01000026 | 9.1377556355866e-62 |
| 11 | NZ_ABVP01000026 | Proteus penneri ATCC 35198 P_penneri-1.1.1_Cont348: NZ_ABVP01000026 | 9.1377556355866e-62 |
| 12 | NZ_ABVP01000014 | Proteus penneri ATCC 35198 P_penneri-1.1.1_Cont350: NZ_ABVP01000014 | 9.1377556355866e-62 |
| 13 | NZ_ABVP01000026 | Proteus penneri ATCC 35198 P_penneri-1.1.1_Cont348: NZ_ABVP01000026 | 9.1377556355866e-62 |
| 14 | NZ_ABVP01000025 | Proteus penneri ATCC 35198 P_penneri-1.1.1_Cont344: NZ_ABVP01000025 | 9.1377556355866e-62 |
| 15 | NZ_ABVP01000025 | Proteus penneri ATCC 35198 P_penneri-1.1.1_Cont344: NZ_ABVP01000025 | 9.1377556355866e-62 |
| 16 | NC_010554 | Proteus mirabilis HI4320: NC_010554 | 9.1377556355866e-62 |
| 17 | NZ_ABVP01000022 | Proteus penneri ATCC 35198 P_penneri-1.1.1_Cont341: NZ_ABVP01000022 | 9.1377556355866e-62 |
| 18 | NZ_ ACLE01000064 | Proteus mirabilis ATCC 29906 contig00088: NZ_ACLE01000064 | 9.1377556355866e-62 |
| 19 | NZ_ABVP01000026 | Proteus penneri ATCC 35198 P_penneri-1.1.1_Cont348: NZ_ABVP01000026 | 9.1377556355866e-62 |

(continued)

| No. | Scaffold.External. Accession | Scaffold.Name | best_pv |
|-----|------------------------------|---------------|---------|
| 20 | NZ_ABVP01000026 | Proteus penneri ATCC 35198 P_penneri-1.1.1_Cont348: NZ_ABVP01000026 | 9.1377556355866e-62 |
| 21 | NZ_ABVP01000026 | Proteus penneri ATCC 35198 P_penneri-1.1.1_Cont348: NZ_ABVP01000026 | 9.1377556355866e-62 |
| 22 | NZ_ABVP01000022 | Proteus penneri ATCC 35198 P_penneri-1.1.1_Cont341: NZ_ABVP01000022 | 9.1377556355866e-62 |
| 23 | NZ_ABVP01000025 | Proteus penneri ATCC 35198 P_penneri-1.1.1_Cont344: NZ_ABVP01000025 | 9.1377556355866e-62 |
| 24 | NZ_ABVP01000025 | Proteus penneri ATCC 35198 P_penneri-1.1.1_Cont344: NZ_ABVP01000025 | 9.1377556355866e-62 |
| 25 | NZ_ABVP01000025 | Proteus penneri ATCC 35198 P_penneri-1.1.1_Cont344: NZ_ABVP01000025 | 9.1377556355866e-62 |
| 26 | NZ_ABVP01000025 | Proteus penneri ATCC 35198 P_penneri-1.1.1_Cont344: NZ_ABVP01000025 | 9.1377556355866e-62 |
| 27 | NZ_ABVP01000025 | Proteus penneri ATCC 35198 P_penneri-1.1.1_Cont344: NZ_ABVP01000025 | 9.1377556355866e-62 |
| 28 | NZ_ABVP01000025 | Proteus penneri ATCC 35198 P_penneri-1.1.1_Cont344: NZ_ABVP01000025 | 9.1377556355866e-62 |
| 29 | NC_010554 | Proteus mirabilis HI4320: NC_010554 | 9.1377556355866e-62 |
| 30 | NZ_ABVP01000021 | Proteus penneri ATCC 35198 P_penneri-1.1.1_Cont356: NZ_ABVP01000021 | 9.1377556355866e-62 |
| 31 | NZ_ABVP01000020 | Proteus penneri ATCC 35198 P_penneri-1.1.1_Cont338: NZ_ABVP01000020 | 9.1377556355866e-62 |
| 32 | NZ_ABVP01000025 | Proteus penneri ATCC 35198 P_penneri-1.1.1_Cont344: NZ_ABVP01000025 | 9.1377556355866e-62 |
| 33 | NZ_ABVP01000025 | Proteus penneri ATCC 35198 P_penneri-1.1.1_Cont344: NZ_ABVP01000025 | 9.1377556355866e-62 |
| 34 | NZ_ABVP01000023 | Proteus penneri ATCC 35198 P_penneri-1.1.1_Cont336: NZ_ABVP01000023 | 9.1377556355866e-62 |
| 35 | NZ_ABVP01000022 | Proteus penneri ATCC 35198 P_penneri-1.1.1_Cont341: NZ_ABVP01000022 | 9.1377556355866e-62 |
| 36 | NZ_ABVP01000025 | Proteus penneri ATCC 35198 P_penneri-1.1.1_Cont344: NZ_ABVP01000025 | 9.1377556355866e-62 |
| 37 | NZ_ABVP01000024 | Proteus penneri ATCC 35198 P_penneri-1.1.1-Cont334: NZ_ABVP01000024 | 9.1377556355866e-62 |
| 38 | NZ_ABVP01000025 | Proteus penneri ATCC 35198 P_penneri-1.1.1_Cont344: NZ_ABVP01000025 | 9.1377556355866e-62 |
| 39 | NC_010554 | Proteus mirabilis HI4320: NC_010554 | 9.1377556355866e-62 |
| 40 | NZ_ABVP01000019 | Proteus penneri ATCC 35198 P_penneri-1.1.1_Cont6.1: NZ_ABVP01000019 | 9.1377556355866e-62 |
| 41 | NZ_ABVP01000019 | Proteus penneri ATCC 35198 P_penneri-1.1.1_Cont6.1: NZ_ABVP01000019 | 9.1377556355866e-62 |
| 42 | NZ_ABVP01000025 | Proteus penneri ATCC 35198 P_penneri-1.1.1_Cont344: NZ_ABVP01000025 | 9.1377556355866e-62 |

(continued)

| No. | Scaffold.External. Accession | Scaffold.Name | best_pv |
|---|---|---|---|
| 43 | NZ_ABVP01000020 | Proteus penneri ATCC 35198 P_penneri-1.1.1_Cont338: NZ_ABVP01000020 | 9.1377556355866e-62 |
| 44 | NZ_ABVP01000023 | Proteus penneri ATCC 35198 P_penneri-1.1.1_Cont336: NZ_ABVP01000023 | 9.1377556355866e-62 |
| 45 | NZ_ABVP01000026 | Proteus penneri ATCC 35198 P_penneri-1.1.1_Cont348: NZ_ABVP01000026 | 9.1377556355866e-62 |
| 46 | NZ_ABVP01000025 | Proteus penneri ATCC 35198 P_penneri-1.1.1_Cont344: NZ_ABVP01000025 | 9.1377556355866e-62 |
| 47 | NZ_ABVP01000021 | Proteus penneri ATCC 35198 P_penneri-1.1.1_Cont356: NZ_ABVP01000021 | 9.1377556355866e-62 |
| 48 | NZ_ ACLE01000079 | Proteus mirabilis ATCC 29906 contig00105: NZ_ ACLE01000079 | 9.1377556355866e-62 |
| 49 | NC_010554 | Proteus mirabilis HI4320: NC_010554 | 9.1377556355866e-62 |
| 50 | NZ_ABVP01000023 | Proteus penneri ATCC 35198 P_penneri-1.1.1_Cont336: NZ_ABVP01000023 | 9.1377556355866e-62 |

Table 13b: Data for Proteus species (continued)

| No. | sign_phenos | sign_phenos_class | best_pheno | best_pheno_class |
|---|---|---|---|---|
| 1 | AM;CAX;CP;CRM;GM;LVX;TO;T/S | aminoglycoside;fluoroquinolone;lactam;other | CRM | lactam |
| 2 | AM;CAX;CP;CRM;GM;LVX;TO;T/S | aminoglycoside;fluoroquinolone;lactam;other | CRM | lactam |
| 3 | AM;CAX;CP;CRM;GM;LVX;TO;T/S | aminoglycoside;fluoroquinolone;lactam;other | CRM | lactam |
| 4 | AM;CAX;CP;CRM;GM;LVX;TO;T/S | aminoglycoside;fluoroquinolone;lactam;other | CRM | lactam |
| 5 | AM;CAX;CP;CRM;GM;LVX;TO;T/S | aminoglycoside;fluoroquinolone;lactam;other | CRM | lactam |
| 6 | AM;CAX;CP;CRM;GM;LVX;TO;T/S | aminoglycoside;fluoroquinolone;lactam;other | CRM | lactam |
| 7 | AM;CAX;CP;CRM;GM;LVX;TO;T/S | aminoglycoside;fluoroquinolone;lactam;other | CRM | lactam |
| 8 | AM;CAX;CP;CRM;GM;LVX;TO;T/S | aminoglycoside;fluoroquinolone;lactam;other | CRM | lactam |
| 9 | AM;CAX;CP;CRM;GM;LVX;TO;T/S | aminoglycoside;fluoroquinolone;lactam;other | CRM | lactam |
| 10 | AM;CAX;CP;CRM;GM;LVX;TO;T/S | aminoglycoside;fluoroquinolone;lactam;other | CRM | lactam |
| 11 | AM;CAX;CP;CRM;GM;LVX;TO;T/S | aminoglycoside;fluoroquinolone;lactam;other | CRM | lactam |
| 12 | AM;CAX;CP;CRM;GM;LVX;TO;T/S | aminoglycoside;fluoroquinolone;lactam;other | CRM | lactam |

(continued)

| No. | sign_phenos | sign_phenos_class | best_pheno | best_pheno_class |
|---|---|---|---|---|
| 13 | AM;CAX;CP;CRM;GM;LVX;TO;T/S | aminoglycoside;fluoroquinolone;lactam;other | CRM | lactam |
| 14 | AM;CAX;CP;CRM;GM;LVX;TO;T/S | aminoglycoside;fluoroquinolone;lactam;other | CRM | lactam |
| 15 | AM;CAX;CP;CRM;GM;LVX;TO;T/S | aminoglycoside;fluoroquinolone;lactam;other | CRM | lactam |
| 16 | AM;CAX;CP;CRM;GM;LVX;TO;T/S | aminoglycoside;fluoroquinolone;lactam;other | CRM | lactam |
| 17 | AM;CAX;CP;CRM;GM;LVX;TO;T/S | aminoglycoside;fluoroquinolone;lactam;other | CRM | lactam |
| 18 | AM;CAX;CP;CRM;GM;LVX;TO;T/S | aminoglycoside;fluoroquinolone;lactam;other | CRM | lactam |
| 19 | AM;CAX;CP;CRM;GM;LVX;TO;T/S | aminoglycoside;fluoroquinolone;lactam;other | CRM | lactam |
| 20 | AM;CAX;CP;CRM;GM;LVX;TO;T/S | aminoglycoside;fluoroquinolone;lactam;other | CRM | lactam |
| 21 | AM;CAX;CP;CRM;GM;LVX;TO;T/S | aminoglycoside;fluoroquinolone;lactam;other | CRM | lactam |
| 22 | AM;CAX;CP;CRM;GM;LVX;TO;T/S | aminoglycoside;fluoroquinolone;lactam;other | CRM | lactam |
| 23 | AM;CAX;CP;CRM;GM;LVX;TO;T/S | aminoglycoside;fluoroquinolone;lactam;other | CRM | lactam |
| 24 | AM;CAX;CP;CRM;GM;LVX;TO;T/S | aminoglycoside;fluoroquinolone;lactam;other | CRM | lactam |
| 25 | AM;CAX;CP;CRM;GM;LVX;TO;T/S | aminoglycoside;fluoroquinolone;lactam;other | CRM | lactam |
| 26 | AM;CAX;CP;CRM;GM;LVX;TO;T/S | aminoglycoside;fluoroquinolone;lactam;other | CRM | lactam |
| 27 | AM;CAX;CP;CRM;GM;LVX;TO;T/S | aminoglycoside;fluoroquinolone;lactam;other | CRM | lactam |
| 28 | AM;CAX;CP;CRM;GM;LVX;TO;T/S | aminoglycoside;fluoroquinolone;lactam;other | CRM | lactam |
| 29 | AM;CAX;CP;CRM;GM;LVX;TO;T/S | aminoglycoside;fluoroquinolone;lactam;other | CRM | lactam |
| 30 | AM;CAX;CP;CRM;GM;LVX;TO;T/S | aminoglycoside;fluoroquinolone;lactam;other | CRM | lactam |
| 31 | AM;CAX;CP;CRM;GM;LVX;TO;T/S | aminoglycoside;fluoroquinolone;lactam;other | CRM | lactam |
| 32 | AM;CAX;CP;CRM;GM;LVX;TO;T/S | aminoglycoside;fluoroquinolone;lactam;other | CRM | lactam |
| 33 | AM;CAX;CP;CRM;GM;LVX;TO;T/S | aminoglycoside;fluoroquinolone;lactam;other | CRM | lactam |
| 34 | AM;CAX;CP;CRM;GM;LVX;TO;T/S | aminoglycoside;fluoroquinolone;lactam;other | CRM | lactam |

(continued)

| No. | sign_phenos | sign_phenos_class | best_pheno | best_pheno_class |
|---|---|---|---|---|
| 35 | AM;CAX;CP;CRM;GM;LVX;TO;T/S | aminoglycoside;fluoroquinolone;lactam;other | CRM | lactam |
| 36 | AM;CAX;CP;CRM;GM;LVX;TO;T/S | aminoglycoside;fluoroquinolone;lactam;other | CRM | lactam |
| 37 | AM;CAX;CP;CRM;GM;LVX;TO;T/S | aminoglycoside;fluoroquinolone;lactam;other | CRM | lactam |
| 38 | AM;CAX;CP;CRM;GM;LVX;TO;T/S | aminoglycoside;fluoroquinolone;lactam;other | CRM | lactam |
| 39 | AM;CAX;CP;CRM;GM;LVX;TO;T/S | aminoglycoside;fluoroquinolone;lactam;other | CRM | lactam |
| 40 | AM;CAX;CP;CRM;GM;LVX;TO;T/S | aminoglycoside;fluoroquinolone;lactam;other | CRM | lactam |
| 41 | AM;CAX;CP;CRM;GM;LVX;TO;T/S | aminoglycoside;fluoroquinolone;lactam;other | CRM | lactam |
| 42 | AM;CAX;CP;CRM;GM;LVX;TO;T/S | aminoglycoside;fluoroquinolone;lactam;other | CRM | lactam |
| 43 | AM;CAX;CP;CRM;GM;LVX;TO;T/S | aminoglycoside;fluoroquinolone;lactam;other | CRM | lactam |
| 44 | AM;CAX;CP;CRM;GM;LVX;TO;T/S | aminoglycoside;fluoroquinolone;lactam;other | CRM | lactam |
| 45 | AM;CAX;CP;CRM;GM;LVX;TO;T/S | aminoglycoside;fluoroquinolone;lactam;other | CRM | lactam |
| 46 | AM;CAX;CP;CRM;GM;LVX;TO;T/S | aminoglycoside;fluoroquinolone;lactam;other | CRM | lactam |
| 47 | AM;CAX;CP;CRM;GM;LVX;TO;T/S | aminoglycoside;fluoroquinolone;lactam;other | CRM | lactam |
| 48 | AM;CAX;CP;CRM;GM;LVX;TO;T/S | aminoglycoside;fluoroquinolone;lactam;other | CRM | lactam |
| 49 | AM;CAX;CP;CRM;GM;LVX;TO;T/S | aminoglycoside;fluoroquinolone;lactam;other | CRM | lactam |
| 50 | AM;CAX;CP;CRM;GM;LVX;TO;T/S | aminoglycoside;fluoroquinolone;lactam;other | CRM | lactam |

Table 13c: Data for Proteus species (continued)

| No. | num_aminoglycoside | num_fluoroquinolone | num_lactam | num_other |
|---|---|---|---|---|
| 1 | 2 | 2 | 3 | 1 |
| 2 | 2 | 2 | 3 | 1 |
| 3 | 2 | 2 | 3 | 1 |
| 4 | 2 | 2 | 3 | 1 |
| 5 | 2 | 2 | 3 | 1 |
| 6 | 2 | 2 | 3 | 1 |
| 7 | 2 | 2 | 3 | 1 |

(continued)

| No. | num_aminoglycoside | num_fluoroquinolone | num_lactam | num_other |
|-----|--------------------|--------------------|------------|-----------|
| 8 | 2 | 2 | 3 | 1 |
| 9 | 2 | 2 | 3 | 1 |
| 10 | 2 | 2 | 3 | 1 |
| 11 | 2 | 2 | 3 | 1 |
| 12 | 2 | 2 | 3 | 1 |
| 13 | 2 | 2 | 3 | 1 |
| 14 | 2 | 2 | 3 | 1 |
| 15 | 2 | 2 | 3 | 1 |
| 16 | 2 | 2 | 3 | 1 |
| 17 | 2 | 2 | 3 | 1 |
| 18 | 2 | 2 | 3 | 1 |
| 19 | 2 | 2 | 3 | 1 |
| 20 | 2 | 2 | 3 | 1 |
| 21 | 2 | 2 | 3 | 1 |
| 22 | 2 | 2 | 3 | 1 |
| 23 | 2 | 2 | 3 | 1 |
| 24 | 2 | 2 | 3 | 1 |
| 25 | 2 | 2 | 3 | 1 |
| 26 | 2 | 2 | 3 | 1 |
| 27 | 2 | 2 | 3 | 1 |
| 28 | 2 | 2 | 3 | 1 |
| 29 | 2 | 2 | 3 | 1 |
| 30 | 2 | 2 | 3 | 1 |
| 31 | 2 | 2 | 3 | 1 |
| 32 | 2 | 2 | 3 | 1 |
| 33 | 2 | 2 | 3 | 1 |
| 34 | 2 | 2 | 3 | 1 |
| 35 | 2 | 2 | 3 | 1 |
| 36 | 2 | 2 | 3 | 1 |
| 37 | 2 | 2 | 3 | 1 |
| 38 | 2 | 2 | 3 | 1 |
| 39 | 2 | 2 | 3 | 1 |
| 40 | 2 | 2 | 3 | 1 |
| 41 | 2 | 2 | 3 | 1 |
| 42 | 2 | 2 | 3 | 1 |
| 43 | 2 | 2 | 3 | 1 |
| 44 | 2 | 2 | 3 | 1 |
| 45 | 2 | 2 | 3 | 1 |

(continued)

| No. | num_aminoglycoside | num_fluoroquinolone | num_lactam | num_other |
|---|---|---|---|---|
| 46 | 2 | 2 | 3 | 1 |
| 47 | 2 | 2 | 3 | 1 |
| 48 | 2 | 2 | 3 | 1 |
| 49 | 2 | 2 | 3 | 1 |
| 50 | 2 | 2 | 3 | 1 |

Table 13d: Data for Proteus species (continued)

| No. | AM_pv_adj | A/S_pv_adj | CAX_pv_adj | CP_pv_adj | CRM_pv_adj |
|---|---|---|---|---|---|
| 1 | 3.94E-36 | 1 | 1.38E-16 | 1.03E-08 | 9.14E-62 |
| 2 | 3.94E-36 | 1 | 1.38E-16 | 1.03E-08 | 9.14E-62 |
| 3 | 3.94E-36 | 1 | 1.38E-16 | 1.03E-08 | 9.14E-62 |
| 4 | 3.94E-36 | 1 | 1.38E-16 | 1.03E-08 | 9.14E-62 |
| 5 | 3.94E-36 | 1 | 1.38E-16 | 1.03E-08 | 9.14E-62 |
| 6 | 3.94E-36 | 1 | 1.38E-16 | 1.03E-08 | 9.14E-62 |
| 7 | 3.94E-36 | 1 | 1.38E-16 | 1.03E-08 | 9.14E-62 |
| 8 | 5.45E-37 | 9.36E-01 | 4.09E-17 | 7.21E-08 | 9.14E-62 |
| 9 | 3.94E-36 | 1 | 1.38E-16 | 1.03E-08 | 9.14E-62 |
| 10 | 3.94E-36 | 1 | 1.38E-16 | 1.03E-08 | 9.14E-62 |
| 11 | 3.94E-36 | 1 | 1.38E-16 | 1.03E-08 | 9.14E-62 |
| 12 | 3.94E-36 | 1 | 1.38E-16 | 1.03E-08 | 9.14E-62 |
| 13 | 3.94E-36 | 1 | 1.38E-16 | 1.03E-08 | 9.14E-62 |
| 14 | 3.94E-36 | 1 | 1.38E-16 | 1.03E-08 | 9.14E-62 |
| 15 | 3.94E-36 | 1 | 1.38E-16 | 1.03E-08 | 9.14E-62 |
| 16 | 3.94E-36 | 1 | 1.38E-16 | 1.03E-08 | 9.14E-62 |
| 17 | 3.94E-36 | 1 | 1.38E-16 | 1.03E-08 | 9.14E-62 |
| 18 | 3.94E-36 | 1 | 1.38E-16 | 1.03E-08 | 9.14E-62 |
| 19 | 3.94E-36 | 1 | 1.38E-16 | 1.03E-08 | 9.14E-62 |
| 20 | 3.94E-36 | 1 | 1.38E-16 | 1.03E-08 | 9.14E-62 |
| 21 | 1.07E-35 | 9.36E-01 | 8.67E-17 | 1.70E-08 | 9.14E-62 |
| 22 | 3.94E-36 | 1 | 1.38E-16 | 1.03E-08 | 9.14E-62 |
| 23 | 3.94E-36 | 1 | 1.38E-16 | 1.03E-08 | 9.14E-62 |
| 24 | 3.94E-36 | 1 | 1.38E-16 | 1.03E-08 | 9.14E-62 |
| 25 | 3.94E-36 | 1 | 1.38E-16 | 1.03E-08 | 9.14E-62 |
| 26 | 3.94E-36 | 1 | 1.38E-16 | 1.03E-08 | 9.14E-62 |
| 27 | 3.94E-36 | 1 | 1.38E-16 | 1.03E-08 | 9.14E-62 |
| 28 | 3.94E-36 | 1 | 1.38E-16 | 1.03E-08 | 9.14E-62 |
| 29 | 3.94E-36 | 1 | 1.38E-16 | 1.03E-08 | 9.14E-62 |

(continued)

| No. | AM_pv_adj | A/S_pv_adj | CAX_pv_adj | CP_pv_adj | CRM_pv_adj |
|---|---|---|---|---|---|
| 30 | 3.94E-36 | 1 | 1.38E-16 | 1.03E-08 | 9.14E-62 |
| 31 | 3.94E-36 | 1 | 1.38E-16 | 1.03E-08 | 9.14E-62 |
| 32 | 3.94E-36 | 1 | 1.38E-16 | 1.03E-08 | 9.14E-62 |
| 33 | 3.94E-36 | 1 | 1.38E-16 | 1.03E-08 | 9.14E-62 |
| 34 | 3.94E-36 | 1 | 1.38E-16 | 1.03E-08 | 9.14E-62 |
| 35 | 3.94E-36 | 1 | 1.38E-16 | 1.03E-08 | 9.14E-62 |
| 36 | 3.94E-36 | 1 | 1.38E-16 | 1.03E-08 | 9.14E-62 |
| 37 | 3.94E-36 | 1 | 1.38E-16 | 1.03E-08 | 9.14E-62 |
| 38 | 3.94E-36 | 1 | 1.38E-16 | 1.03E-08 | 9.14E-62 |
| 39 | 5.45E-37 | 9.36E-01 | 4.09E-17 | 6.06E-09 | 9.14E-62 |
| 40 | 3.94E-36 | 1 | 1.38E-16 | 1.03E-08 | 9.14E-62 |
| 41 | 3.94E-36 | 1 | 1.38E-16 | 1.03E-08 | 9.14E-62 |
| 42 | 3.94E-36 | 1 | 1.38E-16 | 1.03E-08 | 9.14E-62 |
| 43 | 3.94E-36 | 1 | 1.38E-16 | 1.03E-08 | 9.14E-62 |
| 44 | 3.94E-36 | 1 | 1.38E-16 | 1.03E-08 | 9.14E-62 |
| 45 | 3.94E-36 | 1 | 1.38E-16 | 1.03E-08 | 9.14E-62 |
| 46 | 3.94E-36 | 1 | 1.38E-16 | 1.03E-08 | 9.14E-62 |
| 47 | 1.07E-35 | 9.36E-01 | 8.67E-17 | 1.70E-08 | 9.14E-62 |
| 48 | 6.31E-38 | 5.69E-01 | 1.13E-16 | 4.44E-07 | 9.14E-62 |
| 49 | 1.56E-36 | 8.45E-01 | 2.42E-17 | 1.26E-07 | 9.14E-62 |
| 50 | 3.94E-36 | 1 | 1.38E-16 | 1.03E-08 | 9.14E-62 |

Table 13e: Data for Proteus species (continued)

| No. | GM_pv_adj | LVX_pv_adj | TO_pv_adj | T/S_pv_adj |
|---|---|---|---|---|
| 1 | 3.39E-06 | 1.43E-06 | 6.08E-05 | 2.82E-06 |
| 2 | 3.39E-06 | 1.43E-06 | 6.08E-05 | 2.82E-06 |
| 3 | 3.39E-06 | 1.43E-06 | 6.08E-05 | 2.82E-06 |
| 4 | 3.39E-06 | 1.43E-06 | 6.08E-05 | 2.82E-06 |
| 5 | 3.39E-06 | 1.43E-06 | 6.08E-05 | 2.82E-06 |
| 6 | 3.39E-06 | 1.43E-06 | 6.08E-05 | 2.82E-06 |
| 7 | 3.39E-06 | 1.43E-06 | 6.08E-05 | 2.82E-06 |
| 8 | 4.54E-05 | 4.64E-06 | 3.83E-05 | 1.26E-05 |
| 9 | 3.39E-06 | 1.43E-06 | 6.08E-05 | 2.82E-06 |
| 10 | 3.39E-06 | 1.43E-06 | 6.08E-05 | 2.82E-06 |
| 11 | 3.39E-06 | 1.43E-06 | 6.08E-05 | 2.82E-06 |
| 12 | 3.39E-06 | 1.43E-06 | 6.08E-05 | 2.82E-06 |
| 13 | 3.39E-06 | 1.43E-06 | 6.08E-05 | 2.82E-06 |

(continued)

| No. | GM_pv_adj | LVX_pv_adj | TO_pv_adj | T/S_pv_adj |
|-----|-----------|------------|-----------|------------|
| 14 | 3.39E-06 | 1.43E-06 | 6.08E-05 | 2.82E-06 |
| 15 | 3.39E-06 | 1.43E-06 | 6.08E-05 | 2.82E-06 |
| 16 | 3.39E-06 | 1.43E-06 | 6.08E-05 | 2.82E-06 |
| 17 | 3.39E-06 | 1.43E-06 | 6.08E-05 | 2.82E-06 |
| 18 | 3.39E-06 | 1.43E-06 | 6.08E-05 | 2.82E-06 |
| 19 | 3.39E-06 | 1.43E-06 | 6.08E-05 | 2.82E-06 |
| 20 | 3.39E-06 | 1.43E-06 | 6.08E-05 | 2.82E-06 |
| 21 | 5.45E-06 | 1.43E-06 | 6.08E-05 | 4.40E-06 |
| 22 | 3.39E-06 | 1.43E-06 | 6.08E-05 | 2.82E-06 |
| 23 | 3.39E-06 | 1.43E-06 | 6.08E-05 | 2.82E-06 |
| 24 | 3.39E-06 | 1.43E-06 | 6.08E-05 | 2.82E-06 |
| 25 | 3.39E-06 | 1.43E-06 | 6.08E-05 | 2.82E-06 |
| 26 | 3.39E-06 | 1.43E-06 | 6.08E-05 | 2.82E-06 |
| 27 | 3.39E-06 | 1.43E-06 | 6.08E-05 | 2.82E-06 |
| 28 | 3.39E-06 | 1.43E-06 | 6.08E-05 | 2.82E-06 |
| 29 | 3.39E-06 | 1.43E-06 | 6.08E-05 | 2.82E-06 |
| 30 | 3.39E-06 | 1.43E-06 | 6.08E-05 | 2.82E-06 |
| 31 | 3.39E-06 | 1.43E-06 | 6.08E-05 | 2.82E-06 |
| 32 | 3.39E-06 | 1.43E-06 | 6.08E-05 | 2.82E-06 |
| 33 | 3.39E-06 | 1.43E-06 | 6.08E-05 | 2.82E-06 |
| 34 | 3.39E-06 | 1.43E-06 | 6.08E-05 | 2.82E-06 |
| 35 | 3.39E-06 | 1.43E-06 | 6.08E-05 | 2.82E-06 |
| 36 | 3.39E-06 | 1.43E-06 | 6.08E-05 | 2.82E-06 |
| 37 | 3.39E-06 | 1.43E-06 | 6.08E-05 | 2.82E-06 |
| 38 | 3.39E-06 | 1.43E-06 | 6.08E-05 | 2.82E-06 |
| 39 | 4.54E-05 | 8.81E-07 | 4.61E-04 | 1.26E-05 |
| 40 | 3.39E-06 | 1.43E-06 | 6.08E-05 | 2.82E-06 |
| 41 | 3.39E-06 | 1.43E-06 | 6.08E-05 | 2.82E-06 |
| 42 | 3.39E-06 | 1.43E-06 | 6.08E-05 | 2.82E-06 |
| 43 | 3.39E-06 | 1.43E-06 | 6.08E-05 | 2.82E-06 |
| 44 | 3.39E-06 | 1.43E-06 | 6.08E-05 | 2.82E-06 |
| 45 | 3.39E-06 | 1.43E-06 | 6.08E-05 | 2.82E-06 |
| 46 | 3.39E-06 | 1.43E-06 | 6.08E-05 | 2.82E-06 |
| 47 | 5.45E-06 | 1.43E-06 | 6.08E-05 | 4.40E-06 |
| 48 | 1.60E-03 | 2.08E-05 | 4.47E-04 | 7.97E-06 |
| 49 | 3.39E-06 | 7.10E-06 | 6.19E-05 | 2.82E-06 |
| 50 | 3.39E-06 | 1.43E-06 | 6.08E-05 | 2.82E-06 |

Table 13f: Data for Proteus species (continued)

| No. | Locus.Tag | Gene.Symbol | GenBank.Accession | Chromosome |
|---|---|---|---|---|
| 1 | PROPEN_00658 | | ZP_03802316 | |
| 2 | PROPEN_02821 | | ZP_03804437 | |
| 3 | PROPEN_02837 | | ZP_03804453 | |
| 4 | PROPEN_01744 | | ZP_03803381 | |
| 5 | PROPEN_04772 | | ZP_03806369 | |
| 6 | PROPEN_00566 | | ZP_03802226 | |
| 7 | PROPEN_00937 | | ZP_03802594 | |
| 8 | PMI0230 | | YP_002150011 | |
| 9 | PROPEN_01382 | | ZP_03803029 | |
| 10 | PROPEN_04308 | | ZP_03805908 | |
| 11 | PROPEN_03726 | | ZP_03805332 | |
| 12 | PROPEN_00113 | | ZP_03801788 | |
| 13 | PROPEN_04501 | | ZP_03806101 | |
| 14 | PROPEN_02952 | | ZP_03804567 | |
| 15 | PROPEN_03365 | | ZP_03804978 | |
| 16 | PMI3610 | | YP_002153285 | |
| 17 | PROPEN_01429 | | ZP_03803076 | |
| 18 | HMPREF0693_ 2570 | | ZP_03841679 | |
| 19 | PROPEN_04448 | | ZP_03806048 | |
| 20 | PROPEN_04169 | | ZP_03805774 | |
| 21 | PROPEN_04170 | | ZP_03805775 | |
| 22 | PROPEN_01358 | | ZP_03803005 | |
| 23 | PROPEN_02831 | | ZP_03804447 | |
| 24 | PROPEN_03253 | | ZP_03804866 | |
| 25 | PROPEN_03536 | | ZP_03805144 | |
| 26 | PROPEN_03368 | | ZP_03804981 | |
| 27 | PROPEN_03235 | | ZP_03804848 | |
| 28 | PROPEN_03484 | | ZP_03805093 | |
| 29 | PMI3521 | | YP_002153198 | |
| 30 | PROPEN_00908 | | ZP_03802565 | |
| 31 | PROPEN_00654 | | ZP_03802312 | |
| 32 | PROPEN_03299 | | ZP_03804912 | |
| 33 | PROPEN_03648 | | ZP_03805254 | |
| 34 | PROPEN_01652 | | ZP_03803297 | |
| 35 | PROPEN_01314 | | ZP_03802961 | |
| 36 | PROPEN_02647 | | ZP_03804265 | |
| 37 | PROPEN_02468 | | ZP_03804091 | |

(continued)

| No. | Locus.Tag | Gene.Symbol | GenBank.Accession | Chromosome |
|---|---|---|---|---|
| 38 | PROPEN_03281 | | ZP_03804894 | |
| 39 | PMI0234 | | YP_002150015 | |
| 40 | PROPEN_00544 | | ZP_03802209 | |
| 41 | PROPEN_00500 | | ZP_03802168 | |
| 42 | PROPEN_02930 | | ZP_03804546 | |
| 43 | PROPEN_00758 | | ZP_03802416 | |
| 44 | PROPEN_01810 | | ZP_03803447 | |
| 45 | PROPEN_04133 | | ZP_03805738 | |
| 46 | PROPEN_02599 | | ZP_03804222 | |
| 47 | PROPEN_01149 | | ZP_03802800 | |
| 48 | HMPREF0693_3224 | | ZP_03842333 | |
| 49 | PMI3023 | | YP_002152722 | |
| 50 | PROPEN_01623 | | ZP_03803268 | |

Table 13g: Data for Proteus species (continued)

| No. | Start.Coord | End.Coord | Strand | Scaffold.ID |
|---|---|---|---|---|
| 1 | 42080 | 42412 | + | 643889140 |
| 2 | 185454 | 186779 | - | 643889145 |
| 3 | 201544 | 202038 | + | 643889145 |
| 4 | 122639 | 124333 | - | 643889143 |
| 5 | 853119 | 853544 | + | 643889146 |
| 6 | 57621 | 58505 | - | 643889139 |
| 7 | 44265 | 45014 | + | 643889141 |
| 8 | 273673 | 274467 | + | 642555123 |
| 9 | 115768 | 116316 | - | 643889142 |
| 10 | 495434 | 496444 | - | 643889146 |
| 11 | 53270 | 53875 | - | 643889146 |
| 12 | 28708 | 29421 | - | 643889134 |
| 13 | 639163 | 640215 | - | 643889146 |
| 14 | 285467 | 286066 | - | 643889145 |
| 15 | 601755 | 602153 | - | 643889145 |
| 16 | 3948188 | 3948604 | - | 642555123 |
| 17 | 160792 | 161340 | + | 643889142 |
| 18 | 122510 | 123433 | - | 643905827 |
| 19 | 593048 | 593632 | - | 643889146 |
| 20 | 390159 | 391736 | + | 643889146 |
| 21 | 391736 | 392329 | + | 643889146 |

(continued)

| No. | Start.Coord | End.Coord | Strand | Scaffold.ID |
|-----|-------------|-----------|--------|-------------|
| 22 | 99554 | 100063 | - | 643889142 |
| 23 | 195059 | 197509 | - | 643889145 |
| 24 | 511254 | 511508 | - | 643889145 |
| 25 | 711617 | 713044 | + | 643889145 |
| 26 | 603518 | 605086 | - | 643889145 |
| 27 | 500249 | 500962 | + | 643889145 |
| 28 | 676951 | 677898 | - | 643889145 |
| 29 | 3840767 | 3841663 | - | 642555123 |
| 30 | 23566 | 24027 | - | 643889141 |
| 31 | 39429 | 39599 | + | 643889140 |
| 32 | 548639 | 549616 | - | 643889145 |
| 33 | 797158 | 797820 | + | 643889145 |
| 34 | 48570 | 49238 | - | 643889143 |
| 35 | 63033 | 63515 | + | 643889142 |
| 36 | 56690 | 57196 | - | 643889145 |
| 37 | 284519 | 287461 | + | 643889144 |
| 38 | 535246 | 536289 | + | 643889145 |
| 39 | 279605 | 281008 | + | 642555123 |
| 40 | 44077 | 44439 | - | 643889139 |
| 41 | 12698 | 14710 | - | 643889139 |
| 42 | 268387 | 269874 | - | 643889145 |
| 43 | 106241 | 106837 | - | 643889140 |
| 44 | 175312 | 176142 | + | 643889143 |
| 45 | 362809 | 363438 | + | 643889146 |
| 46 | 23652 | 24185 | + | 643889145 |
| 47 | 200043 | 201293 | - | 643889141 |
| 48 | 37235 | 38317 | - | 643905842 |
| 49 | 3320488 | 3321087 | + | 642555123 |
| 50 | 30478 | 31134 | + | 643889143 |

Table 14a: Data for Proteus species

| No. | Scaffold.External. Accession | Scaffold.Name | best_pv |
|-----|------------------------------|---------------|---------|
| 1 | NZ_ABVP01000020 | Proteus penneri ATCC 35198 P_penneri-1.1.1_Cont338: NZ_ABVP01000020 | 9.1377556355866e-62 |
| 2 | NZ_ABVP01000025 | Proteus penneri ATCC 35198 P_penneri-1.1.1_Cont344: NZ_ABVP01000025 | 9.1377556355866e-62 |
| 3 | NZ_ABVP01000025 | Proteus penneri ATCC 35198 P_penneri-1.1.1_Cont344: NZ_ABVP01000025 | 9.1377556355866e-62 |

(continued)

| No. | Scaffold.External. Accession | Scaffold.Name | best_pv |
|---|---|---|---|
| 4 | NZ_ABVP01000023 | Proteus penneri ATCC 35198 P_penneri-1.1.1_Cont336: NZ_ABVP01000023 | 9.1377556355866e-62 |
| 5 | NZ_ABVP01000026 | Proteus penneri ATCC 35198 P_penneri-1.1.1_Cont348: NZ_ABVP01000026 | 9.1377556355866e-62 |
| 6 | NZ_ABVP01000019 | Proteus penneri ATCC 35198 P_penneri-1.1.1_Cont6.1: NZ_ABVP01000019 | 9.1377556355866e-62 |
| 7 | NZ_ABVP01000021 | Proteus penneri ATCC 35198 P_penneri-1.1.1_Cont356: NZ_ABVP01000021 | 9.1377556355866e-62 |
| 8 | NC_010554 | Proteus mirabilis HI4320: NC_010554 | 9.1377556355866e-62 |
| 9 | NZ_ABVP01000022 | Proteus penneri ATCC 35198 P_penneri-1.1.1_Cont341: NZ_ABVP01000022 | 9.1377556355866e-62 |
| 10 | NZ_ABVP01000026 | Proteus penneri ATCC 35198 P_penneri-1.1.1_Cont348: NZ_ABVP01000026 | 9.1377556355866e-62 |
| 11 | NZ_ABVP01000026 | Proteus penneri ATCC 35198 P_penneri-1.1.1_Cont348: NZ_ABVP01000026 | 9.1377556355866e-62 |
| 12 | NZ_ABVP01000014 | Proteus penneri ATCC 35198 P_penneri-1.1.1-Cont350: NZ_ABVP01000014 | 9.1377556355866e-62 |
| 13 | NZ_ABVP01000026 | Proteus penneri ATCC 35198 P_penneri-1.1.1_Cont348: NZ_ABVP01000026 | 9.1377556355866e-62 |
| 14 | NZ_ABVP01000025 | Proteus penneri ATCC 35198 P_penneri-1.1.1_Cont344: NZ_ABVP01000025 | 9.1377556355866e-62 |
| 15 | NZ_ABVP01000025 | Proteus penneri ATCC 35198 P_penneri-1.1.1_Cont344: NZ_ABVP01000025 | 9.1377556355866e-62 |
| 16 | NC_010554 | Proteus mirabilis HI4320: NC_010554 | 9.1377556355866e-62 |
| 17 | NZ_ABVP01000022 | Proteus penneri ATCC 35198 P_penneri-1.1.1_Cont341: NZ_ABVP01000022 | 9.1377556355866e-62 |
| 18 | NZ_ACLE01000064 | Proteus mirabilis ATCC 29906 contig00088: NZ_ACLE01000064 | 9.1377556355866e-62 |
| 19 | NZ_ABVP01000026 | Proteus penneri ATCC 35198 P_penneri-1.1.1_Cont348: NZ_ABVP01000026 | 9.1377556355866e-62 |
| 20 | NZ_ABVP01000026 | Proteus penneri ATCC 35198 P_penneri-1.1.1_Cont348: NZ_ABVP01000026 | 9.1377556355866e-62 |
| 21 | NZ_ABVP01000026 | Proteus penneri ATCC 35198 P_penneri-1.1.1_Cont348: NZ_ABVP01000026 | 9.1377556355866e-62 |
| 22 | NZ_ABVP01000022 | Proteus penneri ATCC 35198 P_penneri-1.1.1_Cont341: NZ_ABVP01000022 | 9.1377556355866e-62 |
| 23 | NZ_ABVP01000025 | Proteus penneri ATCC 35198 P_penneri-1.1.1_Cont344: NZ_ABVP01000025 | 9.1377556355866e-62 |
| 24 | NZ_ABVP01000025 | Proteus penneri ATCC 35198 P_penneri-1.1.1_Cont344: NZ_ABVP01000025 | 9.1377556355866e-62 |
| 25 | NZ_ABVP01000025 | Proteus penneri ATCC 35198 P_penneri-1.1.1_Cont344: NZ_ABVP01000025 | 9.1377556355866e-62 |
| 26 | NZ_ABVP01000025 | Proteus penneri ATCC 35198 P_penneri-1.1.1_Cont344: NZ_ABVP01000025 | 9.1377556355866e-62 |

(continued)

| No. | Scaffold.External. Accession | Scaffold.Name | best_pv |
|-----|------------------------------|---------------|---------|
| 27 | NZ_ABVP01000025 | Proteus penneri ATCC 35198 P_penneri-1.1.1_Cont344: NZ_ABVP01000025 | 9.1377556355866e-62 |
| 28 | NZ_ABVP01000025 | Proteus penneri ATCC 35198 P_penneri-1.1.1_Cont344: NZ_ABVP01000025 | 9.1377556355866e-62 |
| 29 | NC_010554 | Proteus mirabilis HI4320: NC_010554 | 9.1377556355866e-62 |
| 30 | NZ_ABVP01000021 | Proteus penneri ATCC 35198 P_penneri-1.1.1_Cont356: NZ_ABVP01000021 | 9.1377556355866e-62 |
| 31 | NZ_ABVP01000020 | Proteus penneri ATCC 35198 P_penneri-1.1.1_Cont338: NZ_ABVP01000020 | 9.1377556355866e-62 |
| 32 | NZ_ABVP01000025 | Proteus penneri ATCC 35198 P_penneri-1.1.1_Cont344: NZ_ABVP01000025 | 9.1377556355866e-62 |
| 33 | NZ_ABVP01000025 | Proteus penneri ATCC 35198 P_penneri-1.1.1_Cont344: NZ_ABVP01000025 | 9.1377556355866e-62 |
| 34 | NZ_ABVP01000023 | Proteus penneri ATCC 35198 P_penneri-1.1.1_Cont336: NZ_ABVP01000023 | 9.1377556355866e-62 |
| 35 | NZ_ABVP01000022 | Proteus penneri ATCC 35198 P_penneri-1.1.1_Cont341: NZ_ABVP01000022 | 9.1377556355866e-62 |
| 36 | NZ_ABVP01000025 | Proteus penneri ATCC 35198 P_penneri-1.1.1_Cont344: NZ_ABVP01000025 | 9.1377556355866e-62 |
| 37 | NZ_ABVP01000024 | Proteus penneri ATCC 35198 P_penneri-1.1.1_Cont334: NZ_ABVP01000024 | 9.1377556355866e-62 |
| 38 | NZ_ABVP01000025 | Proteus penneri ATCC 35198 P_penneri-1.1.1_Cont344: NZ_ABVP01000025 | 9.1377556355866e-62 |
| 39 | NC_010554 | Proteus mirabilis HI4320: NC_010554 | 9.1377556355866e-62 |
| 40 | NZ_ABVP01000019 | Proteus penneri ATCC 35198 P_penneri-1.1.1_Cont6.1: NZ_ABVP01000019 | 9.1377556355866e-62 |
| 41 | NZ_ABVP01000019 | Proteus penneri ATCC 35198 P_penneri-1.1.1_Cont6.1: NZ_ABVP01000019 | 9.1377556355866e-62 |
| 42 | NZ_ABVP01000025 | Proteus penneri ATCC 35198 P_penneri-1.1.1_Cont344: NZ_ABVP01000025 | 9.1377556355866e-62 |
| 43 | NZ_ABVP01000020 | Proteus penneri ATCC 35198 P_penneri-1.1.1_Cont338: NZ_ABVP01000020 | 9.1377556355866e-62 |
| 44 | NZ_ABVP01000023 | Proteus penneri ATCC 35198 P_penneri-1.1.1_Cont336: NZ_ABVP01000023 | 9.1377556355866e-62 |
| 45 | NZ_ABVP01000026 | Proteus penneri ATCC 35198 P_penneri-1.1.1_Cont348: NZ_ABVP01000026 | 9.1377556355866e-62 |
| 46 | NZ_ABVP01000025 | Proteus penneri ATCC 35198 P_penneri-1.1.1_Cont344: NZ_ABVP01000025 | 9.1377556355866e-62 |
| 47 | NZ_ABVP01000021 | Proteus penneri ATCC 35198 P_penneri-1.1.1_Cont356: NZ_ABVP01000021 | 9.1377556355866e-62 |
| 48 | NZ_ACLE01000079 | Proteus mirabilis ATCC 29906 contig00105: NZ_ACLE01000079 | 9.1377556355866e-62 |
| 49 | NC_010554 | Proteus mirabilis HI4320: NC_010554 | 9.1377556355866e-62 |

(continued)

| No. | Scaffold.External. Accession | Scaffold.Name | best_pv |
|---|---|---|---|
| 50 | NZ_ABVP01000023 | Proteus penneri ATCC 35198 P_penneri-1.1.1_Cont336: NZ_ABVP01000023 | 9.1377556355866e-62 |

Table 14b: Data for Proteus species (continued)

| No. | sign_phenos | sign_phenos_class | best_pheno | best_pheno_class |
|---|---|---|---|---|
| 1 | AM;CAX;CP;CRM;GM;LVX;TO;T/S | aminoglycoside;fluoroquinolone;lactam;other | CRM | lactam |
| 2 | AM;CAX;CP;CRM;GM;LVX;TO;T/S | aminoglycoside;fluoroquinolone;lactam;other | CRM | lactam |
| 3 | AM;CAX;CP;CRM;GM;LVX;TO;T/S | aminoglycoside;fluoroquinolone;lactam;other | CRM | lactam |
| 4 | AM;CAX;CP;CRM;GM;LVX;TO;T/S | aminoglycoside;fluoroquinolone;lactam;other | CRM | lactam |
| 5 | AM;CAX;CP;CRM;GM;LVX;TO;T/S | aminoglycoside;fluoroquinolone;lactam;other | CRM | lactam |
| 6 | AM;CAX;CP;CRM;GM;LVX;TO;T/S | aminoglycoside;fluoroquinolone;lactam;other | CRM | lactam |
| 7 | AM;CAX;CP;CRM;GM;LVX;TO;T/S | aminoglycoside;fluoroquinolone;lactam;other | CRM | lactam |
| 8 | AM;CAX;CP;CRM;GM;LVX;TO;T/S | aminoglycoside;fluoroquinolone;lactam;other | CRM | lactam |
| 9 | AM;CAX;CP;CRM;GM;LVX;TO;T/S | aminoglycoside;fluoroquinolone;lactam;other | CRM | lactam |
| 10 | AM;CAX;CP;CRM;GM;LVX;TO;T/S | aminoglycoside;fluoroquinolone;lactam;other | CRM | lactam |
| 11 | AM;CAX;CP;CRM;GM;LVX;TO;T/S | aminoglycoside;fluoroquinolone;lactam;other | CRM | lactam |
| 12 | AM;CAX;CP;CRM;GM;LVX;TO;T/S | aminoglycoside;fluoroquinolone;lactam;other | CRM | lactam |
| 13 | AM;CAX;CP;CRM;GM;LVX;TO;T/S | aminoglycoside;fluoroquinolone;lactam;other | CRM | lactam |
| 14 | AM;CAX;CP;CRM;GM;LVX;TO;T/S | aminoglycoside;fluoroquinolone;lactam;other | CRM | lactam |
| 15 | AM;CAX;CP;CRM;GM;LVX;TO;T/S | aminoglycoside;fluoroquinolone;lactam;other | CRM | lactam |
| 16 | AM;CAX;CP;CRM;GM;LVX;TO;T/S | aminoglycoside;fluoroquinolone;lactam;other | CRM | lactam |
| 17 | AM;CAX;CP;CRM;GM;LVX;TO;T/S | aminoglycoside;fluoroquinolone;lactam;other | CRM | lactam |
| 18 | AM;CAX;CP;CRM;GM;LVX;TO;T/S | aminoglycoside;fluoroquinolone;lactam;other | CRM | lactam |
| 19 | AM;CAX;CP;CRM;GM;LVX;TO;T/S | aminoglycoside;fluoroquinolone;lactam;other | CRM | lactam |

(continued)

| No. | sign_phenos | sign_phenos_class | best_pheno | best_pheno_class |
|---|---|---|---|---|
| 20 | AM;CAX;CP;CRM;GM;LVX;TO;T/S | aminoglycoside;fluoroquinolone;lactam;other | CRM | lactam |
| 21 | AM;CAX;CP;CRM;GM;LVX;TO;T/S | aminoglycoside;fluoroquinolone;lactam;other | CRM | lactam |
| 22 | AM;CAX;CP;CRM;GM;LVX;TO;T/S | aminoglycoside;fluoroquinolone;lactam;other | CRM | lactam |
| 23 | AM;CAX;CP;CRM;GM;LVX;TO;T/S | aminoglycoside;fluoroquinolone;lactam;other | CRM | lactam |
| 24 | AM;CAX;CP;CRM;GM;LVX;TO;T/S | aminoglycoside;fluoroquinolone;lactam;other | CRM | lactam |
| 25 | AM;CAX;CP;CRM;GM;LVX;TO;T/S | aminoglycoside;fluoroquinolone;lactam;other | CRM | lactam |
| 26 | AM;CAX;CP;CRM;GM;LVX;TO;T/S | aminoglycoside;fluoroquinolone;lactam;other | CRM | lactam |
| 27 | AM;CAX;CP;CRM;GM;LVX;TO;T/S | aminoglycoside;fluoroquinolone;lactam;other | CRM | lactam |
| 28 | AM;CAX;CP;CRM;GM;LVX;TO;T/S | aminoglycoside;fluoroquinolone;lactam;other | CRM | lactam |
| 29 | AM;CAX;CP;CRM;GM;LVX;TO;T/S | aminoglycoside;fluoroquinolone;lactam;other | CRM | lactam |
| 30 | AM;CAX;CP;CRM;GM;LVX;TO;T/S | aminoglycoside;fluoroquinolone;lactam;other | CRM | lactam |
| 31 | AM;CAX;CP;CRM;GM;LVX;TO;T/S | aminoglycoside;fluoroquinolone;lactam;other | CRM | lactam |
| 32 | AM;CAX;CP;CRM;GM;LVX;TO;T/S | aminoglycoside;fluoroquinolone;lactam;other | CRM | lactam |
| 33 | AM;CAX;CP;CRM;GM;LVX;TO;T/S | aminoglycoside;fluoroquinolone;lactam;other | CRM | lactam |
| 34 | AM;CAX;CP;CRM;GM;LVX;TO;T/S | aminoglycoside;fluoroquinolone;lactam;other | CRM | lactam |
| 35 | AM;CAX;CP;CRM;GM;LVX;TO;T/S | aminoglycoside;fluoroquinolone;lactam;other | CRM | lactam |
| 36 | AM;CAX;CP;CRM;GM;LVX;TO;T/S | aminoglycoside;fluoroquinolone;lactam;other | CRM | lactam |
| 37 | AM;CAX;CP;CRM;GM;LVX;TO;T/S | aminoglycoside;fluoroquinolone;lactam;other | CRM | lactam |
| 38 | AM;CAX;CP;CRM;GM;LVX;TO;T/S | aminoglycoside;fluoroquinolone;lactam;other | CRM | lactam |
| 39 | AM;CAX;CP;CRM;GM;LVX;TO;T/S | aminoglycoside;fluoroquinolone;lactam;other | CRM | lactam |
| 40 | AM;CAX;CP;CRM;GM;LVX;TO;T/S | aminoglycoside;fluoroquinolone;lactam;other | CRM | lactam |
| 41 | AM;CAX;CP;CRM;GM;LVX;TO;T/S | aminoglycoside;fluoroquinolone;lactam;other | CRM | lactam |

(continued)

| No. | sign_phenos | sign_phenos_class | best_pheno | best_pheno_class |
|-----|-------------|-------------------|------------|------------------|
| 42 | AM;CAX;CP;CRM;GM;LVX;TO;T/S | aminoglycoside;fluoroquinolone;lactam;other | CRM | lactam |
| 43 | AM;CAX;CP;CRM;GM;LVX;TO;T/S | aminoglycoside;fluoroquinolone;lactam;other | CRM | lactam |
| 44 | AM;CAX;CP;CRM;GM;LVX;TO;T/S | aminoglycoside;fluoroquinolone;lactam;other | CRM | lactam |
| 45 | AM;CAX;CP;CRM;GM;LVX;TO;T/S | aminoglycoside;fluoroquinolone;lactam;other | CRM | lactam |
| 46 | AM;CAX;CP;CRM;GM;LVX;TO;T/S | aminoglycoside;fluoroquinolone;lactam;other | CRM | lactam |
| 47 | AM;CAX;CP;CRM;GM;LVX;TO;T/S | aminoglycoside;fluoroquinolone;lactam;other | CRM | lactam |
| 48 | AM;CAX;CP;CRM;GM;LVX;TO;T/S | aminoglycoside;fluoroquinolone;lactam;other | CRM | lactam |
| 49 | AM;CAX;CP;CRM;GM;LVX;TO;T/S | aminoglycoside;fluoroquinolone;lactam;other | CRM | lactam |
| 50 | AM;CAX;CP;CRM;GM;LVX;TO;T/S | aminoglycoside;fluoroquinolone;lactam;other | CRM | lactam |

Table 14c: Data for Proteus species (continued)

| No. | num_aminoglycoside | num_fluoroquinolone | num_lactam | num_other |
|-----|--------------------|--------------------|-----------|-----------|
| 1 | 2 | 2 | 3 | 1 |
| 2 | 2 | 2 | 3 | 1 |
| 3 | 2 | 2 | 3 | 1 |
| 4 | 2 | 2 | 3 | 1 |
| 5 | 2 | 2 | 3 | 1 |
| 6 | 2 | 2 | 3 | 1 |
| 7 | 2 | 2 | 3 | 1 |
| 8 | 2 | 2 | 3 | 1 |
| 9 | 2 | 2 | 3 | 1 |
| 10 | 2 | 2 | 3 | 1 |
| 11 | 2 | 2 | 3 | 1 |
| 12 | 2 | 2 | 3 | 1 |
| 13 | 2 | 2 | 3 | 1 |
| 14 | 2 | 2 | 3 | 1 |
| 15 | 2 | 2 | 3 | 1 |
| 16 | 2 | 2 | 3 | 1 |
| 17 | 2 | 2 | 3 | 1 |
| 18 | 2 | 2 | 3 | 1 |

(continued)

| No. | num_aminoglycoside | num_fluoroquinolone | num_lactam | num_other |
|-----|--------------------|--------------------|------------|-----------|
| 19 | 2 | 2 | 3 | 1 |
| 20 | 2 | 2 | 3 | 1 |
| 21 | 2 | 2 | 3 | 1 |
| 22 | 2 | 2 | 3 | 1 |
| 23 | 2 | 2 | 3 | 1 |
| 24 | 2 | 2 | 3 | 1 |
| 25 | 2 | 2 | 3 | 1 |
| 26 | 2 | 2 | 3 | 1 |
| 27 | 2 | 2 | 3 | 1 |
| 28 | 2 | 2 | 3 | 1 |
| 29 | 2 | 2 | 3 | 1 |
| 30 | 2 | 2 | 3 | 1 |
| 31 | 2 | 2 | 3 | 1 |
| 32 | 2 | 2 | 3 | 1 |
| 33 | 2 | 2 | 3 | 1 |
| 34 | 2 | 2 | 3 | 1 |
| 35 | 2 | 2 | 3 | 1 |
| 36 | 2 | 2 | 3 | 1 |
| 37 | 2 | 2 | 3 | 1 |
| 38 | 2 | 2 | 3 | 1 |
| 39 | 2 | 2 | 3 | 1 |
| 40 | 2 | 2 | 3 | 1 |
| 41 | 2 | 2 | 3 | 1 |
| 42 | 2 | 2 | 3 | 1 |
| 43 | 2 | 2 | 3 | 1 |
| 44 | 2 | 2 | 3 | 1 |
| 45 | 2 | 2 | 3 | 1 |
| 46 | 2 | 2 | 3 | 1 |
| 47 | 2 | 2 | 3 | 1 |
| 48 | 2 | 2 | 3 | 1 |
| 49 | 2 | 2 | 3 | 1 |
| 50 | 2 | 2 | 3 | 1 |

Table 14d: Data for Proteus species (continued)

| No. | AM_pv_adj | A/S_pv_adj | CAX_pv_adj | CP_pv_adj | CRM_pv_adj |
|-----|-----------|------------|------------|-----------|------------|
| 1 | 3.94E-36 | 1 | 1.38E-16 | 1.03E-08 | 9.14E-62 |
| 2 | 3.94E-36 | 1 | 1.38E-16 | 1.03E-08 | 9.14E-62 |

(continued)

| No. | AM_pv_adj | A/S_pv_adj | CAX_pv_adj | CP_pv_adj | CRM_pv_adj |
|-----|-----------|------------|------------|-----------|------------|
| 3 | 3.94E-36 | 1 | 1.38E-16 | 1.03E-08 | 9.14E-62 |
| 4 | 3.94E-36 | 1 | 1.38E-16 | 1.03E-08 | 9.14E-62 |
| 5 | 3.94E-36 | 1 | 1.38E-16 | 1.03E-08 | 9.14E-62 |
| 6 | 3.94E-36 | 1 | 1.38E-16 | 1.03E-08 | 9.14E-62 |
| 7 | 3.94E-36 | 1 | 1.38E-16 | 1.03E-08 | 9.14E-62 |
| 8 | 5.45E-37 | 9.36E-01 | 4.09E-17 | 7.21E-08 | 9.14E-62 |
| 9 | 3.94E-36 | 1 | 1.38E-16 | 1.03E-08 | 9.14E-62 |
| 10 | 3.94E-36 | 1 | 1.38E-16 | 1.03E-08 | 9.14E-62 |
| 11 | 3.94E-36 | 1 | 1.38E-16 | 1.03E-08 | 9.14E-62 |
| 12 | 3.94E-36 | 1 | 1.38E-16 | 1.03E-08 | 9.14E-62 |
| 13 | 3.94E-36 | 1 | 1.38E-16 | 1.03E-08 | 9.14E-62 |
| 14 | 3.94E-36 | 1 | 1.38E-16 | 1.03E-08 | 9.14E-62 |
| 15 | 3.94E-36 | 1 | 1.38E-16 | 1.03E-08 | 9.14E-62 |
| 16 | 3.94E-36 | 1 | 1.38E-16 | 1.03E-08 | 9.14E-62 |
| 17 | 3.94E-36 | 1 | 1.38E-16 | 1.03E-08 | 9.14E-62 |
| 18 | 3.94E-36 | 1 | 1.38E-16 | 1.03E-08 | 9.14E-62 |
| 19 | 3.94E-36 | 1 | 1.38E-16 | 1.03E-08 | 9.14E-62 |
| 20 | 3.94E-36 | 1 | 1.38E-16 | 1.03E-08 | 9.14E-62 |
| 21 | 1.07E-35 | 9.36E-01 | 8.67E-17 | 1.70E-08 | 9.14E-62 |
| 22 | 3.94E-36 | 1 | 1.38E-16 | 1.03E-08 | 9.14E-62 |
| 23 | 3.94E-36 | 1 | 1.38E-16 | 1.03E-08 | 9.14E-62 |
| 24 | 3.94E-36 | 1 | 1.38E-16 | 1.03E-08 | 9.14E-62 |
| 25 | 3.94E-36 | 1 | 1.38E-16 | 1.03E-08 | 9.14E-62 |
| 26 | 3.94E-36 | 1 | 1.38E-16 | 1.03E-08 | 9.14E-62 |
| 27 | 3.94E-36 | 1 | 1.38E-16 | 1.03E-08 | 9.14E-62 |
| 28 | 3.94E-36 | 1 | 1.38E-16 | 1.03E-08 | 9.14E-62 |
| 29 | 3.94E-36 | 1 | 1.38E-16 | 1.03E-08 | 9.14E-62 |
| 30 | 3.94E-36 | 1 | 1.38E-16 | 1.03E-08 | 9.14E-62 |
| 31 | 3.94E-36 | 1 | 1.38E-16 | 1.03E-08 | 9.14E-62 |
| 32 | 3.94E-36 | 1 | 1.38E-16 | 1.03E-08 | 9.14E-62 |
| 33 | 3.94E-36 | 1 | 1.38E-16 | 1.03E-08 | 9.14E-62 |
| 34 | 3.94E-36 | 1 | 1.38E-16 | 1.03E-08 | 9.14E-62 |
| 35 | 3.94E-36 | 1 | 1.38E-16 | 1.03E-08 | 9.14E-62 |
| 36 | 3.94E-36 | 1 | 1.38E-16 | 1.03E-08 | 9.14E-62 |
| 37 | 3.94E-36 | 1 | 1.38E-16 | 1.03E-08 | 9.14E-62 |
| 38 | 3.94E-36 | 1 | 1.38E-16 | 1.03E-08 | 9.14E-62 |
| 39 | 5.45E-37 | 9.36E-01 | 4.09E-17 | 6.06E-09 | 9.14E-62 |
| 40 | 3.94E-36 | 1 | 1.38E-16 | 1.03E-08 | 9.14E-62 |

(continued)

| No. | AM_pv_adj | A/S_pv_adj | CAX_pv_adj | CP_pv_adj | CRM_pv_adj |
|-----|-----------|------------|------------|-----------|------------|
| 41 | 3.94E-36 | 1 | 1.38E-16 | 1.03E-08 | 9.14E-62 |
| 42 | 3.94E-36 | 1 | 1.38E-16 | 1.03E-08 | 9.14E-62 |
| 43 | 3.94E-36 | 1 | 1.38E-16 | 1.03E-08 | 9.14E-62 |
| 44 | 3.94E-36 | 1 | 1.38E-16 | 1.03E-08 | 9.14E-62 |
| 45 | 3.94E-36 | 1 | 1.38E-16 | 1.03E-08 | 9.14E-62 |
| 46 | 3.94E-36 | 1 | 1.38E-16 | 1.03E-08 | 9.14E-62 |
| 47 | 1.07E-35 | 9.36E-01 | 8.67E-17 | 1.70E-08 | 9.14E-62 |
| 48 | 6.31E-38 | 5.69E-01 | 1.13E-16 | 4.44E-07 | 9.14E-62 |
| 49 | 1.56E-36 | 8.45E-01 | 2.42E-17 | 1.26E-07 | 9.14E-62 |
| 50 | 3.94E-36 | 1 | 1.38E-16 | 1.03E-08 | 9.14E-62 |

Table 14e: Data for Proteus species (continued)

| No. | GM_pv_adj | LVX_pv_adj | TO_pv_adj | T/S_pv_adj |
|-----|-----------|------------|-----------|------------|
| 1 | 3.39E-06 | 1.43E-06 | 6.08E-05 | 2.82E-06 |
| 2 | 3.39E-06 | 1.43E-06 | 6.08E-05 | 2.82E-06 |
| 3 | 3.39E-06 | 1.43E-06 | 6.08E-05 | 2.82E-06 |
| 4 | 3.39E-06 | 1.43E-06 | 6.08E-05 | 2.82E-06 |
| 5 | 3.39E-06 | 1.43E-06 | 6.08E-05 | 2.82E-06 |
| 6 | 3.39E-06 | 1.43E-06 | 6.08E-05 | 2.82E-06 |
| 7 | 3.39E-06 | 1.43E-06 | 6.08E-05 | 2.82E-06 |
| 8 | 4.54E-05 | 4.64E-06 | 3.83E-05 | 1.26E-05 |
| 9 | 3.39E-06 | 1.43E-06 | 6.08E-05 | 2.82E-06 |
| 10 | 3.39E-06 | 1.43E-06 | 6.08E-05 | 2.82E-06 |
| 11 | 3.39E-06 | 1.43E-06 | 6.08E-05 | 2.82E-06 |
| 12 | 3.39E-06 | 1.43E-06 | 6.08E-05 | 2.82E-06 |
| 13 | 3.39E-06 | 1.43E-06 | 6.08E-05 | 2.82E-06 |
| 14 | 3.39E-06 | 1.43E-06 | 6.08E-05 | 2.82E-06 |
| 15 | 3.39E-06 | 1.43E-06 | 6.08E-05 | 2.82E-06 |
| 16 | 3.39E-06 | 1.43E-06 | 6.08E-05 | 2.82E-06 |
| 17 | 3.39E-06 | 1.43E-06 | 6.08E-05 | 2.82E-06 |
| 18 | 3.39E-06 | 1.43E-06 | 6.08E-05 | 2.82E-06 |
| 19 | 3.39E-06 | 1.43E-06 | 6.08E-05 | 2.82E-06 |
| 20 | 3.39E-06 | 1.43E-06 | 6.08E-05 | 2.82E-06 |
| 21 | 5.45E-06 | 1.43E-06 | 6.08E-05 | 4.40E-06 |
| 22 | 3.39E-06 | 1.43E-06 | 6.08E-05 | 2.82E-06 |
| 23 | 3.39E-06 | 1.43E-06 | 6.08E-05 | 2.82E-06 |
| 24 | 3.39E-06 | 1.43E-06 | 6.08E-05 | 2.82E-06 |

(continued)

| No. | GM_pv_adj | LVX_pv_adj | TO_pv_adj | T/S_pv_adj |
|---|---|---|---|---|
| 25 | 3.39E-06 | 1.43E-06 | 6.08E-05 | 2.82E-06 |
| 26 | 3.39E-06 | 1.43E-06 | 6.08E-05 | 2.82E-06 |
| 27 | 3.39E-06 | 1.43E-06 | 6.08E-05 | 2.82E-06 |
| 28 | 3.39E-06 | 1.43E-06 | 6.08E-05 | 2.82E-06 |
| 29 | 3.39E-06 | 1.43E-06 | 6.08E-05 | 2.82E-06 |
| 30 | 3.39E-06 | 1.43E-06 | 6.08E-05 | 2.82E-06 |
| 31 | 3.39E-06 | 1.43E-06 | 6.08E-05 | 2.82E-06 |
| 32 | 3.39E-06 | 1.43E-06 | 6.08E-05 | 2.82E-06 |
| 33 | 3.39E-06 | 1.43E-06 | 6.08E-05 | 2.82E-06 |
| 34 | 3.39E-06 | 1.43E-06 | 6.08E-05 | 2.82E-06 |
| 35 | 3.39E-06 | 1.43E-06 | 6.08E-05 | 2.82E-06 |
| 36 | 3.39E-06 | 1.43E-06 | 6.08E-05 | 2.82E-06 |
| 37 | 3.39E-06 | 1.43E-06 | 6.08E-05 | 2.82E-06 |
| 38 | 3.39E-06 | 1.43E-06 | 6.08E-05 | 2.82E-06 |
| 39 | 4.54E-05 | 8.81E-07 | 4.61E-04 | 1.26E-05 |
| 40 | 3.39E-06 | 1.43E-06 | 6.08E-05 | 2.82E-06 |
| 41 | 3.39E-06 | 1.43E-06 | 6.08E-05 | 2.82E-06 |
| 42 | 3.39E-06 | 1.43E-06 | 6.08E-05 | 2.82E-06 |
| 43 | 3.39E-06 | 1.43E-06 | 6.08E-05 | 2.82E-06 |
| 44 | 3.39E-06 | 1.43E-06 | 6.08E-05 | 2.82E-06 |
| 45 | 3.39E-06 | 1.43E-06 | 6.08E-05 | 2.82E-06 |
| 46 | 3.39E-06 | 1.43E-06 | 6.08E-05 | 2.82E-06 |
| 47 | 5.45E-06 | 1.43E-06 | 6.08E-05 | 4.40E-06 |
| 48 | 1.60E-03 | 2.08E-05 | 4.47E-04 | 7.97E-06 |
| 49 | 3.39E-06 | 7.10E-06 | 6.19E-05 | 2.82E-06 |
| 50 | 3.39E-06 | 1.43E-06 | 6.08E-05 | 2.82E-06 |

Table 14f: Data for Proteus species (continued)

| No. | Locus.Tag | Gene. Symbol | GenBank.Accession | Chromosome |
|---|---|---|---|---|
| 1 | PROPEN_00658 | | ZP_03802316 | |
| 2 | PROPEN_02821 | | ZP_03804437 | |
| 3 | PROPEN_02837 | | ZP_03804453 | |
| 4 | PROPEN_01744 | | ZP_03803381 | |
| 5 | PROPEN_04772 | | ZP_03806369 | |
| 6 | PROPEN_00566 | | ZP_03802226 | |
| 7 | PROPEN_00937 | | ZP_03802594 | |
| 8 | PMI0230 | | YP_002150011 | |

(continued)

| No. | Locus.Tag | Gene. Symbol | GenBank.Accession | Chromosome |
|-----|-----------|--------------|-------------------|------------|
| 9 | PROPEN_01382 | | ZP_03803029 | |
| 10 | PROPEN_04308 | | ZP_03805908 | |
| 11 | PROPEN_03726 | | ZP_03805332 | |
| 12 | PROPEN_00113 | | ZP_03801788 | |
| 13 | PROPEN_04501 | | ZP_03806101 | |
| 14 | PROPEN_02952 | | ZP_03804567 | |
| 15 | PROPEN_03365 | | ZP_03804978 | |
| 16 | PMI3610 | | YP_002153285 | |
| 17 | PROPEN_01429 | | ZP_03803076 | |
| 18 | HMPREF0693_ 2570 | | ZP_03841679 | |
| 19 | PROPEN_04448 | | ZP_03806048 | |
| 20 | PROPEN_04169 | | ZP_03805774 | |
| 21 | PROPEN_04170 | | ZP_03805775 | |
| 22 | PROPEN_01358 | | ZP_03803005 | |
| 23 | PROPEN_02831 | | ZP_03804447 | |
| 24 | PROPEN_03253 | | ZP_03804866 | |
| 25 | PROPEN_03536 | | ZP_03805144 | |
| 26 | PROPEN_03368 | | ZP_03804981 | |
| 27 | PROPEN_03235 | | ZP_03804848 | |
| 28 | PROPEN_03484 | | ZP_03805093 | |
| 29 | PMI3521 | | YP_002153198 | |
| 30 | PROPEN_00908 | | ZP_03802565 | |
| 31 | PROPEN_00654 | | ZP_03802312 | |
| 32 | PROPEN_03299 | | ZP_03804912 | |
| 33 | PROPEN_03648 | | ZP_03805254 | |
| 34 | PROPEN_01652 | | ZP_03803297 | |
| 35 | PROPEN_01314 | | ZP_03802961 | |
| 36 | PROPEN_02647 | | ZP_03804265 | |
| 37 | PROPEN_02468 | | ZP_03804091 | |
| 38 | PROPEN_03281 | | ZP_03804894 | |
| 39 | PMI0234 | | YP_002150015 | |
| 40 | PROPEN_00544 | | ZP_03802209 | |
| 41 | PROPEN_00500 | | ZP_03802168 | |
| 42 | PROPEN_02930 | | ZP_03804546 | |
| 43 | PROPEN_00758 | | ZP_03802416 | |
| 44 | PROPEN_01810 | | ZP_03803447 | |
| 45 | PROPEN_04133 | | ZP_03805738 | |
| 46 | PROPEN_02599 | | ZP_03804222 | |

(continued)

| No. | Locus.Tag | Gene. Symbol | GenBank.Accession | Chromosome |
|-----|-----------|--------------|-------------------|------------|
| 47 | PROPEN_01149 | | ZP_03802800 | |
| 48 | HMPREF0693_3224 | | ZP_03842333 | |
| 49 | PMI3023 | | YP_002152722 | |
| 50 | PROPEN_01623 | | ZP_03803268 | |

Table 14g: Data for Proteus species (continued)

| No. | Start.Coord | End.Coord | Strand | Scaffold.ID |
|-----|-------------|-----------|--------|-------------|
| 1 | 42080 | 42412 | + | 643889140 |
| 2 | 185454 | 186779 | - | 643889145 |
| 3 | 201544 | 202038 | + | 643889145 |
| 4 | 122639 | 124333 | - | 643889143 |
| 5 | 853119 | 853544 | + | 643889146 |
| 6 | 57621 | 58505 | - | 643889139 |
| 7 | 44265 | 45014 | + | 643889141 |
| 8 | 273673 | 274467 | + | 642555123 |
| 9 | 115768 | 116316 | - | 643889142 |
| 10 | 495434 | 496444 | - | 643889146 |
| 11 | 53270 | 53875 | - | 643889146 |
| 12 | 28708 | 29421 | - | 643889134 |
| 13 | 639163 | 640215 | - | 643889146 |
| 14 | 285467 | 286066 | - | 643889145 |
| 15 | 601755 | 602153 | - | 643889145 |
| 16 | 3948188 | 3948604 | - | 642555123 |
| 17 | 160792 | 161340 | + | 643889142 |
| 18 | 122510 | 123433 | - | 643905827 |
| 19 | 593048 | 593632 | - | 643889146 |
| 20 | 390159 | 391736 | + | 643889146 |
| 21 | 391736 | 392329 | + | 643889146 |
| 22 | 99554 | 100063 | - | 643889142 |
| 23 | 195059 | 197509 | - | 643889145 |
| 24 | 511254 | 511508 | - | 643889145 |
| 25 | 711617 | 713044 | + | 643889145 |
| 26 | 603518 | 605086 | - | 643889145 |
| 27 | 500249 | 500962 | + | 643889145 |
| 28 | 676951 | 677898 | - | 643889145 |
| 29 | 3840767 | 3841663 | - | 642555123 |
| 30 | 23566 | 24027 | - | 643889141 |

(continued)

| No. | Start.Coord | End.Coord | Strand | Scaffold.ID |
|-----|-------------|-----------|--------|-------------|
| 31 | 39429 | 39599 | + | 643889140 |
| 32 | 548639 | 549616 | - | 643889145 |
| 33 | 797158 | 797820 | + | 643889145 |
| 34 | 48570 | 49238 | - | 643889143 |
| 35 | 63033 | 63515 | + | 643889142 |
| 36 | 56690 | 57196 | - | 643889145 |
| 37 | 284519 | 287461 | + | 643889144 |
| 38 | 535246 | 536289 | + | 643889145 |
| 39 | 279605 | 281008 | + | 642555123 |
| 40 | 44077 | 44439 | - | 643889139 |
| 41 | 12698 | 14710 | - | 643889139 |
| 42 | 268387 | 269874 | - | 643889145 |
| 43 | 106241 | 106837 | - | 643889140 |
| 44 | 175312 | 176142 | + | 643889143 |
| 45 | 362809 | 363438 | + | 643889146 |
| 46 | 23652 | 24185 | + | 643889145 |
| 47 | 200043 | 201293 | - | 643889141 |
| 48 | 37235 | 38317 | - | 643905842 |
| 49 | 3320488 | 3321087 | + | 642555123 |
| 50 | 30478 | 31134 | + | 643889143 |

Example 8: Pseudomonas, particularly P. aeruginosa

The procedure was carried out as in Example 1, except that the following microorganisms were used:

Bacterial Strains

The inventors selected 1042 Pseudomonas strains, particularly Pseudomonas aeruginosa, from the microbiology strain collection at Siemens Healthcare Diagnostics (West Sacramento, CA) for susceptibility testing and whole genome sequencing.
From MetaRef, 640 centroids of Pseudomonas aeruginosa were used as reference sequences.
The results for P. aeruginosa are shown in Tables 15 (corresponding to Table 1) and 16 (corresponding to Table 2).

Table 15a: Data for Pseudomonas, particularly Pseudomonas aeruginosa

| No. | Scaffold.External.Accession | Scaffold.Name | best_pv |
|-----|-----------------------------|---------------|---------|
| 1 | NC_009656 | Pseudomonas aeruginosa PA7: NC_009656 | 4.52939243363163e-78 |
| 2 | NC_009656 | Pseudomonas aeruginosa PA7: NC_ 009656 | 2.75541175953672e-58 |
| 3 | NZ_AEEX01000100 | Pseudomonas aeruginosa 39016 contig00116: NZ_AEEX01000100 | 4.23483908607088e-28 |
| 4 | NC_010501 | Pseudomonas putida W619: NC_010501 | 2.9087092265635e-26 |
| 5 | NC_009656 | Pseudomonas aeruginosa PA7: NC_009656 | 5.61705273260437e-22 |

(continued)

| No. | Scaffold.External. Accession | Scaffold.Name | best_pv |
|---|---|---|---|
| 6 | NC_015740 | Pseudomonas stutzeri ATCC 17588 = LMG 11199 chromosome: NC_015740 | 5.61705273260437e-22 |
| 7 | NC_015733 | Pseudomonas putida S16 chromosome: NC_015733 | 1.91188334380809e-20 |
| 8 | NZ_AEEX01000148 | Pseudomonas aeruginosa 39016 contig00164: NZ_AEEX01000148 | 4.25099609715652e-20 |
| 9 | NZ_AAKV01000030 | Pseudomonas aeruginosa C3719, unfinished sequence: NZ_AAKV01000030 | 2.79764535555464e-19 |
| 10 | NZ_AEEX01000098 | Pseudomonas aeruginosa 39016 contig00114: NZ_AEEX01000098 | 4.15595600170243e-19 |
| 11 | NZ_AEEX01000163 | Pseudomonas aeruginosa 39016 contig00179: NZ_AEEX01000163 | 4.72113166087411e-19 |
| 12 | NZ_AAKW01000042 | Pseudomonas aeruginosa 2192, unfinished sequence: NZ_AAKW01000042 | 2.33370938002383e-17 |
| 13 | NZ_AEEX01000009 | Pseudomonas aeruginosa 39016 contig00011: NZ_AEEX01000009 | 3.60022886576205e-17 |
| 14 | NZ_AAKW01000075 | Pseudomonas aeruginosa 2192, unfinished sequence: NZ_AAKW01000075 | 1.13322970340506e-14 |
| 15 | NC_009656 | Pseudomonas aeruginosa PA7: NC_009656 | 2.33016029331653e-14 |
| 16 | NC_009656 | Pseudomonas aeruginosa PA7: NC_009656 | 2.33822187391836e-14 |
| 17 | NZ_AEEX01000098 | Pseudomonas aeruginosa 39016 contig00114: NZ_AEEX01000098 | 2.91419614855849e-14 |
| 18 | NC_009656 | Pseudomonas aeruginosa PA7: NC_009656 | 5.5018212149287e-14 |
| 19 | NC_010501 | Pseudomonas putida W619: NC_010501 | 6.06970436387873e-14 |
| 20 | NC_009656 | Pseudomonas aeruginosa PA7: NC_009656 | 7.75377932078369e-14 |
| 21 | NZ_AAKW01000022 | Pseudomonas aeruginosa 2192, unfinished sequence: NZ_AAKW01000022 | 3.34337634494896e-13 |
| 22 | NZ_ABKZ01000250 | Pseudomonas aeruginosa PAb1 NZ_ABKZ01000250: NZ_ABKZ01000250 | 6.17495854397665e-13 |
| 23 | NC_008463 | Pseudomonas aeruginosa UCBPP-PA14: NC_008463 | 6.42896969501816e-13 |
| 24 | NZ_AAKW01000022 | Pseudomonas aeruginosa 2192, unfinished sequence: NZ_AAKW01000022 | 1.20774339924326e-12 |
| 25 | NZ_AAQW01000001 | Pseudomonas aeruginosa PACS2, unfinished sequence: NZ_AAQW01000001 | 1.22221957191107e-12 |
| 26 | NC_011770 | Pseudomonas aeruginosa LESB58: NC_011770 | 1.79185026742242e-12 |
| 27 | NZ_AAQW01000001 | Pseudomonas aeruginosa PACS2, unfinished sequence: NZ_AAQW01000001 | 2.12378785336506e-12 |
| 28 | NZ_AAKW01000022 | Pseudomonas aeruginosa 2192, unfinished sequence: NZ_AAKW01000022 | 2.4812070369021e-12 |
| 29 | NC_009656 | Pseudomonas aeruginosa PA7: NC_009656 | 3.74499379826048e-12 |
| 30 | NZ_AEEX01000148 | Pseudomonas aeruginosa 39016 contig00164: NZ_AEEX01000148 | 4.42748938639645e-12 |

(continued)

| No. | Scaffold.External. Accession | Scaffold.Name | best_pv |
|-----|------------------------------|---------------|---------|
| 31 | NC_008463 | Pseudomonas aeruginosa UCBPP-PA14: NC_ 008463 | 5.32658560222593e-12 |
| 32 | NZ_AAQW01000001 | Pseudomonas aeruginosa PACS2, unfinished sequence: NZ_AAQW01000001 | 5.99725940121888e-12 |
| 33 | NC_009656 | Pseudomonas aeruginosa PA7: NC_ 009656 | 7.28057447109574e-12 |
| 34 | NC_009656 | Pseudomonas aeruginosa PA7: NC_009656 | 7.48144363663932e-12 |
| 35 | NZ_AAKW01000022 | Pseudomonas aeruginosa 2192, unfinished sequence: NZ_AAKW01000022 | 9.72376870484031e-12 |
| 36 | NZ_AEEX01000085 | Pseudomonas aeruginosa 39016 contig00101: NZ_ AEEX01000085 | 9.72376870484031e-12 |
| 37 | NZ_AAQW01000001 | Pseudomonas aeruginosa PACS2, unfinished sequence: NZ_AAQW01000001 | 9.72376870484031e-12 |
| 38 | NZ_AAQW01000001 | Pseudomonas aeruginosa PACS2, unfinished sequence: NZ_AAQW01000001 | 9.72376870484031e-12 |
| 39 | NZ_AAKW01000022 | Pseudomonas aeruginosa 2192, unfinished sequence: NZ_AAKW01000022 | 9.73896479060769e-12 |
| 40 | NC_011770 | Pseudomonas aeruginosa LESB58: NC_011770 | 9.73896479060769e-12 |
| 41 | CP002622 | Pseudomonas stutzeri DSM 4166: CP002622 | 9.73896479060769e-12 |
| 42 | NZ_AAQW01000001 | Pseudomonas aeruginosa PACS2, unfinished sequence: NZ_AAQW01000001 | 9.73896479060769e-12 |
| 43 | NZ_AEEX01000149 | Pseudomonas aeruginosa 39016 contig00165: NZ_ AEEX01000149 | 1.05759735249337e-11 |
| 44 | NZ_AEEX01000149 | Pseudomonas aeruginosa 39016 contig00165: NZ_ AEEX01000149 | 1.05759735249337e-11 |
| 45 | NC_008463 | Pseudomonas aeruginosa UCBPP-PA14: NC_ 008463 | 1.05759735249337e-11 |
| 46 | NZ_AAKW01000024 | Pseudomonas aeruginosa 2192, unfinished sequence: NZ_AAKW01000024 | 1.13412272447338e-11 |
| 47 | NZ_AAKW01000022 | Pseudomonas aeruginosa 2192, unfinished sequence: NZ_AAKW01000022 | 1.64096469464127e-11 |
| 48 | NC_012560 | Azotobacter vinelandii DJ: NC_012560 | 1.85224764530357e-11 |
| 49 | NZ_AEEX01000149 | Pseudomonas aeruginosa 39016 contig00165: NZ_ AEEX01000149 | 1.86265894165925e-11 |
| 50 | NC_009656 | Pseudomonas aeruginosa PA7: NC_ 009656 | 1.8721278417616e-11 |

Table 15b: Data for Pseudomonas, particularly Pseudomonas aeruginosa (continued)

| No. | sign_phenos | sign_phenos_class | best_ pheno | best_pheno_class |
|-----|-------------|-------------------|-------------|------------------|
| 1 | AZT;CAZ;CP;CPE;GM;IMP;LVX; MER;P/T;TO | aminoglycoside;fluoroquinolone; lactam | TO | aminoglycoside |
| 2 | AZT;CAZ;CP;CPE;GM;IMP;LVX; MER;P/T;TO | aminoglycoside;fluoroquinolone; lactam | TO | aminoglycoside |

(continued)

| No. | sign_phenos | sign_phenos_class | best_pheno | best_pheno_class |
|---|---|---|---|---|
| 3 | CP;CPE;GM;LVX;P/T;TO | aminoglycoside;fluoroquinolone; lactam | TO | aminoglycoside |
| 4 | CP;CPE;GM;LVX;P/T;TO | aminoglycoside;fluoroquinolone; lactam | TO | aminoglycoside |
| 5 | CP;GM;LVX;TO | aminoglycoside;fluoroquinolone | TO | aminoglycoside |
| 6 | CP;GM;LVX;TO | aminoglycoside;fluoroquinolone | TO | aminoglycoside |
| 7 | CP;GM;LVX;TO | aminoglycoside;fluoroquinolone | TO | aminoglycoside |
| 8 | CP;GM;LVX;MER;P/T;TO | aminoglycoside;fluoroquinolone; lactam | TO | aminoglycoside |
| 9 | CAZ;CP;CPE;GM;LVX;MER;P/T; TO | aminoglycoside;fluoroquinolone; lactam | TO | aminoglycoside |
| 10 | CP;GM;TO | aminoglycoside;fluoroquinolone | TO | aminoglycoside |
| 11 | AZT;CAZ;CP;CPE;GM;LVX;P/T; TO | aminoglycoside;fluoroquinolone; lactam | TO | aminoglycoside |
| 12 | CAZ;CP;CPE;GM;LVX;P/T;TO | aminoglycoside;fluoroquinolone; lactam | TO | aminoglycoside |
| 13 | AZT;CAZ;CP;CPE;GM;LVX;P/T; TO | aminoglycoside;fluoroquinolone; lactam | TO | aminoglycoside |
| 14 | CP;GM;LVX;TO | aminoglycoside;fluoroquinolone | TO | aminoglycoside |
| 15 | CAZ;CP;CPE;GM;LVX;P/T;TO | aminoglycoside;fluoroquinolone; lactam | TO | aminoglycoside |
| 16 | CP;GM;IMP;LVX;MER;TO | aminoglycoside;fluoroquinolone; lactam | TO | aminoglycoside |
| 17 | CP;GM;TO | aminoglycoside;fluoroquinolone | TO | aminoglycoside |
| 18 | CAZ;CP;CPE;GM;LVX;P/T;TO | aminoglycoside;fluoroquinolone; lactam | TO | aminoglycoside |
| 19 | CP;GM;LVX;TO | aminoglycoside;fluoroquinolone | TO | aminoglycoside |
| 20 | CP;GM;LVX;TO | aminoglycoside;fluoroquinolone | TO | aminoglycoside |
| 21 | CAZ;CP;GM;MER;P/T;TO | aminoglycoside;fluoroquinolone; lactam | TO | aminoglycoside |
| 22 | CP;GM;IMP;LVX;MER;TO | aminoglycoside;fluoroquinolone; lactam | TO | aminoglycoside |
| 23 | CP;GM;TO | aminoglycoside;fluoroquinolone | TO | aminoglycoside |
| 24 | CAZ;CP;GM;MER;P/T;TO | aminoglycoside;fluoroquinolone; lactam | TO | aminoglycoside |
| 25 | CAZ;CP;GM;LVX;MER;P/T;TO | aminoglycoside;fluoroquinolone; lactam | TO | aminoglycoside |
| 26 | CAZ;CP;GM;LVX;MER;P/T;TO | aminoglycoside;fluoroquinolone; lactam | TO | aminoglycoside |
| 27 | CAZ;CP;GM;LVX;MER;P/T;TO | aminoglycoside;fluoroquinolone; lactam | TO | aminoglycoside |
| 28 | CP;GM;TO | aminoglycoside;fluoroquinolone | TO | aminoglycoside |

(continued)

| No. | sign_phenos | sign_phenos_class | best_pheno | best_pheno_class |
|---|---|---|---|---|
| 29 | CP;CPE;GM;LVX;P/T;TO | aminoglycoside;fluoroquinolone; lactam | TO | aminoglycoside |
| 30 | CP;GM;IMP;LVX;MER;TO | aminoglycoside;fluoroquinolone; lactam | TO | aminoglycoside |
| 31 | AZT;CAZ;CP;CPE;GM;LVX;P/T; TO | aminoglycoside;fluoroquinolone; lactam | TO | aminoglycoside |
| 32 | CAZ;CP;GM;LVX;MER;TO | aminoglycoside;fluoroquinolone; lactam | TO | aminoglycoside |
| 33 | CP;GM;IMP;LVX;MER;TO | aminoglycoside;fluoroquinolone; lactam | TO | aminoglycoside |
| 34 | CAZ;CP;CPE;GM;P/T;TO | aminoglycoside;fluoroquinolone; lactam | TO | aminoglycoside |
| 35 | CAZ;CP;GM;LVX;MER;P/T;TO | aminoglycoside;fluoroquinolone; lactam | TO | aminoglycoside |
| 36 | CAZ;CP;GM;LVX;MER;P/T;TO | aminoglycoside;fluoroquinolone; lactam | TO | aminoglycoside |
| 37 | CAZ;CP;GM;LVX;MER;P/T;TO | aminoglycoside;fluoroquinolone; lactam | TO | aminoglycoside |
| 38 | CAZ;CP;GM;LVX;MER;TO | aminoglycoside;fluoroquinolone; lactam | TO | aminoglycoside |
| 39 | CAZ;CP;GM;LVX;MER;P/T;TO | aminoglycoside;fluoroquinolone; lactam | TO | aminoglycoside |
| 40 | CAZ;CP;GM;LVX;MER;TO | aminoglycoside;fluoroquinolone; lactam | TO | aminoglycoside |
| 41 | CAZ;CP;GM;LVX;MER;P/T;TO | aminoglycoside;fluoroquinolone; lactam | TO | aminoglycoside |
| 42 | CAZ;CP;GM;LVX;MER;P/T;TO | aminoglycoside;fluoroquinolone; lactam | TO | aminoglycoside |
| 43 | CP;GM;IMP;LVX;TO | aminoglycoside;fluoroquinolone; lactam | TO | aminoglycoside |
| 44 | CP;GM;IMP;LVX;MER;TO | aminoglycoside;fluoroquinolone; lactam | TO | aminoglycoside |
| 45 | CP;GM;IMP;LVX;MER;TO | aminoglycoside;fluoroquinolone; lactam | TO | aminoglycoside |
| 46 | CP;GM;IMP;LVX;MER;TO | aminoglycoside;fluoroquinolone; lactam | TO | aminoglycoside |
| 47 | CAZ;CP;GM;LVX;MER;TO | aminoglycoside;fluoroquinolone; lactam | TO | aminoglycoside |
| 48 | CP;GM;IMP;LVX;MER;TO | aminoglycoside;fluoroquinolone; lactam | TO | aminoglycoside |
| 49 | CP;GM;IMP;LVX;MER;TO | aminoglycoside;fluoroquinolone; lactam | TO | aminoglycoside |
| 50 | CP;GM;IMP;LVX;TO | aminoglycoside;fluoroquinolone; lactam | TO | aminoglycoside |

Table 15c: Data for Pseudomonas, particularly Pseudomonas aeruginosa (continued)

| No. | num_aminoglycoside | num_fluoroquinolone | num_lactam |
|-----|--------------------|--------------------|------------|
| 1 | 2 | 2 | 6 |
| 2 | 2 | 2 | 6 |
| 3 | 2 | 2 | 2 |
| 4 | 2 | 2 | 2 |
| 5 | 2 | 2 | 0 |
| 6 | 2 | 2 | 0 |
| 7 | 2 | 2 | 0 |
| 8 | 2 | 2 | 2 |
| 9 | 2 | 2 | 4 |
| 10 | 2 | 1 | 0 |
| 11 | 2 | 2 | 4 |
| 12 | 2 | 2 | 3 |
| 13 | 2 | 2 | 4 |
| 14 | 2 | 2 | 0 |
| 15 | 2 | 2 | 3 |
| 16 | 2 | 2 | 2 |
| 17 | 2 | 1 | 0 |
| 18 | 2 | 2 | 3 |
| 19 | 2 | 2 | 0 |
| 20 | 2 | 2 | 0 |
| 21 | 2 | 1 | 3 |
| 22 | 2 | 2 | 2 |
| 23 | 2 | 1 | 0 |
| 24 | 2 | 1 | 3 |
| 25 | 2 | 2 | 3 |
| 26 | 2 | 2 | 3 |
| 27 | 2 | 2 | 3 |
| 28 | 2 | 1 | 0 |
| 29 | 2 | 2 | 2 |
| 30 | 2 | 2 | 2 |
| 31 | 2 | 2 | 4 |
| 32 | 2 | 2 | 2 |
| 33 | 2 | 2 | 2 |
| 34 | 2 | 1 | 3 |
| 35 | 2 | 2 | 3 |
| 36 | 2 | 2 | 3 |
| 37 | 2 | 2 | 3 |

(continued)

| No. | num_aminoglycoside | num_fluoroquinolone | num_lactam |
|---|---|---|---|
| 38 | 2 | 2 | 2 |
| 39 | 2 | 2 | 3 |
| 40 | 2 | 2 | 2 |
| 41 | 2 | 2 | 3 |
| 42 | 2 | 2 | 3 |
| 43 | 2 | 2 | 1 |
| 44 | 2 | 2 | 2 |
| 45 | 2 | 2 | 2 |
| 46 | 2 | 2 | 2 |
| 47 | 2 | 2 | 2 |
| 48 | 2 | 2 | 2 |
| 49 | 2 | 2 | 2 |
| 50 | 2 | 2 | 1 |

Table 15d: Data for Pseudomonas, particularly Pseudomonas aeruginosa (continued)

| No. | AZT_pv_adj | CAZ_pv_adj | CP_pv_adj | CPE_pv_adj | GM_pv_adj |
|---|---|---|---|---|---|
| 1 | 4.68E-03 | 2.31E-09 | 4.27E-32 | 5.83E-17 | 1.14E-56 |
| 2 | 1.88E-03 | 1.86E-08 | 3.37E-28 | 4.30E-16 | 2.05E-42 |
| 3 | 4.88E-01 | 1.79E-02 | 4.13E-10 | 4.41E-04 | 7.91E-18 |
| 4 | 5.97E-01 | 1.23E-02 | 2.07E-09 | 2.52E-04 | 9.24E-17 |
| 5 | 5.49E-01 | 5.49E-01 | 9.07E-06 | 1.26E-01 | 1.33E-11 |
| 6 | 5.49E-01 | 5.49E-01 | 9.07E-06 | 1.26E-01 | 1.33E-11 |
| 7 | 6.66E-01 | 3.70E-01 | 9.33E-05 | 1.41E-01 | 5.53E-11 |
| 8 | 9.33E-01 | 6.81E-02 | 1.30E-09 | 3.20E-02 | 9.74E-12 |
| 9 | 2.57E-01 | 3.98E-05 | 1.63E-09 | 1.56E-07 | 6.24E-16 |
| 10 | 7.47E-01 | 4.36E-01 | 2.70E-04 | 2.27E-01 | 5.68E-10 |
| 11 | 4.31E-03 | 4.22E-08 | 3.91E-13 | 2.77E-09 | 7.84E-16 |
| 12 | 3.06E-02 | 6.75E-05 | 1.29E-09 | 9.32E-06 | 1.77E-14 |
| 13 | 2.11E-03 | 3.40E-07 | 3.34E-10 | 7.91E-07 | 4.31E-14 |
| 14 | 6.90E-01 | 3.88E-02 | 8.36E-06 | 2.05E-02 | 1.97E-09 |
| 15 | 1.08E-01 | 8.48E-04 | 2.11E-07 | 1.99E-04 | 4.46E-13 |
| 16 | 8.07E-01 | 4.16E-02 | 3.85E-08 | 3.59E-02 | 1.19E-09 |
| 17 | 6.52E-01 | 4.40E-01 | 1.90E-04 | 5.44E-01 | 1.07E-07 |
| 18 | 1.20E-02 | 1.84E-06 | 1.64E-08 | 1.64E-07 | 4.99E-11 |
| 19 | 4.08E-01 | 1.28E-02 | 2.37E-05 | 3.15E-02 | 2.20E-09 |
| 20 | 9.48E-01 | 6.78E-02 | 9.58E-07 | 3.17E-02 | 1.32E-09 |
| 21 | 5.47E-01 | 9.24E-03 | 2.24E-04 | 5.63E-02 | 4.24E-08 |

(continued)

| No. | AZT_pv_adj | CAZ_pv_adj | CP_pv_adj | CPE_pv_adj | GM_pv_adj |
|---|---|---|---|---|---|
| 22 | 8.08E-01 | 1.43E-01 | 2.42E-06 | 2.48E-01 | 1.01E-07 |
| 23 | 8.44E-01 | 2.48E-01 | 4.61E-04 | 3.25E-01 | 9.75E-07 |
| 24 | 4.14E-01 | 4.37E-03 | 1.78E-04 | 5.65E-02 | 1.49E-07 |
| 25 | 3.25E-01 | 1.51E-03 | 6.15E-05 | 2.63E-02 | 9.67E-08 |
| 26 | 6.54E-01 | 5.11E-03 | 1.38E-05 | 1.82E-02 | 5.63E-08 |
| 27 | 1.63E-01 | 1.65E-04 | 7.86E-05 | 1.59E-02 | 1.34E-09 |
| 28 | 7.69E-01 | 2.48E-01 | 7.44E-04 | 3.25E-01 | 2.80E-06 |
| 29 | 4.44E-01 | 6.54E-02 | 4.51E-05 | 8.97E-06 | 1.56E-07 |
| 30 | 9.82E-01 | 1.63E-01 | 3.28E-06 | 2.73E-01 | 2.89E-08 |
| 31 | 7.11E-03 | 2.08E-05 | 1.73E-08 | 1.31E-06 | 7.90E-09 |
| 32 | 4.78E-01 | 7.49E-03 | 4.49E-05 | 2.57E-02 | 2.96E-07 |
| 33 | 6.55E-01 | 1.81E-01 | 8.24E-06 | 2.97E-01 | 2.97E-07 |
| 34 | 5.00E-01 | 5.68E-03 | 8.53E-03 | 7.20E-03 | 1.63E-11 |
| 35 | 4.77E-01 | 4.27E-03 | 6.00E-05 | 2.17E-02 | 9.67E-08 |
| 36 | 4.77E-01 | 4.27E-03 | 6.00E-05 | 2.17E-02 | 9.67E-08 |
| 37 | 4.77E-01 | 4.27E-03 | 6.00E-05 | 2.17E-02 | 9.67E-08 |
| 38 | 5.46E-01 | 9.06E-03 | 6.00E-05 | 3.01E-02 | 2.03E-07 |
| 39 | 4.78E-01 | 4.34E-03 | 1.28E-04 | 2.20E-02 | 1.35E-07 |
| 40 | 5.47E-01 | 9.22E-03 | 7.62E-05 | 3.04E-02 | 2.83E-07 |
| 41 | 4.78E-01 | 4.34E-03 | 1.28E-04 | 2.20E-02 | 1.35E-07 |
| 42 | 4.78E-01 | 4.34E-03 | 1.28E-04 | 2.20E-02 | 1.35E-07 |
| 43 | 1 | 1.81E-01 | 8.55E-06 | 2.01E-01 | 4.71E-07 |
| 44 | 9.49E-01 | 1.43E-01 | 2.47E-06 | 2.48E-01 | 2.19E-07 |
| 45 | 1 | 1.12E-01 | 4.65E-06 | 2.01E-01 | 2.19E-07 |
| 46 | 9.82E-01 | 4.90E-02 | 6.77E-07 | 7.55E-02 | 2.07E-07 |
| 47 | 4.78E-01 | 7.49E-03 | 7.54E-05 | 2.57E-02 | 2.96E-07 |
| 48 | 9.49E-01 | 1.44E-01 | 6.20E-06 | 2.48E-01 | 4.67E-07 |
| 49 | 9.82E-01 | 1.43E-01 | 2.41E-06 | 1.62E-01 | 6.60E-07 |
| 50 | 7.30E-01 | 2.71E-01 | 1.97E-05 | 4.14E-01 | 1.33E-06 |

Table 15e: Data for Pseudomonas, particularly Pseudomonas aeruginosa (continued)

| No. | IMP_pv_adj | LVX_pv_adj | MER_pv_adj | P/T_pv_adj | TO_pv_adj |
|---|---|---|---|---|---|
| 1 | 1,35E-04 | 9,50E-27 | 2,29E-10 | 3,68E-18 | 4,53E-78 |
| 2 | 3,88E-03 | 4,29E-23 | 1,88E-05 | 3,33E-17 | 2,76E-58 |
| 3 | 4,33E-01 | 1,56E-06 | 1,57E-01 | 7,34E-04 | 4,23E-28 |
| 4 | 3,93E-01 | 4,01E-06 | 1,33E-01 | 9,25E-04 | 2,91E-26 |
| 5 | 2,74E-01 | 1,86E-03 | 8,61E-02 | 3,50E-01 | 5,62E-22 |

(continued)

| No. | IMP_pv_adj | LVX_pv_adj | MER_pv_adj | P/T_pv_adj | TO_pv_adj |
|---|---|---|---|---|---|
| 6 | 2,74E-01 | 1,86E-03 | 8,61E-02 | 3,50E-01 | 5,62E-22 |
| 7 | 5,97E-01 | 7,47E-03 | 1,01E-01 | 4,09E-01 | 1,91E-20 |
| 8 | 4,04E-02 | 2,96E-08 | 8,12E-03 | 3,01E-03 | 4,25E-20 |
| 9 | 4,48E-01 | 4,75E-07 | 8,35E-03 | 1,95E-05 | 2,80E-19 |
| 10 | 8,37E-01 | 1,48E-02 | 2,27E-01 | 4,37E-01 | 4,16E-19 |
| 11 | 1 | 7,77E-10 | 2,74E-02 | 2,06E-06 | 4,72E-19 |
| 12 | 8,04E-01 | 2,34E-06 | 1,05E-01 | 5,83E-04 | 2,33E-17 |
| 13 | 1 | 1,18E-06 | 1,82E-01 | 5,92E-05 | 3,60E-17 |
| 14 | 2,13E-02 | 2,85E-04 | 4,99E-01 | 1,04E-02 | 1,13E-14 |
| 15 | 4,16E-01 | 1,60E-05 | 4,41E-01 | 1,42E-03 | 2,33E-14 |
| 16 | 1,11E-03 | 7,44E-05 | 3,68E-04 | 1,33E-02 | 2,34E-14 |
| 17 | 4,55E-01 | 3,37E-02 | 3,01E-01 | 5,40E-01 | 2,91E-14 |
| 18 | 9,48E-01 | 9,19E-07 | 1,68E-01 | 5,53E-05 | 5,50E-14 |
| 19 | 1 | 1,24E-03 | 2,34E-01 | 1,09E-01 | 6,07E-14 |
| 20 | 1,05E-02 | 1,87E-04 | 2,63E-01 | 3,01E-02 | 7,75E-14 |
| 21 | 1,13E-01 | 1,21E-02 | 5,24E-03 | 6,95E-03 | 3,34E-13 |
| 22 | 2,50E-03 | 3,35E-04 | 2,39E-03 | 1,14E-01 | 6,17E-13 |
| 23 | 9,76E-02 | 4,12E-02 | 1,60E-01 | 4,20E-01 | 6,43E-13 |
| 24 | 1,75E-01 | 1,21E-02 | 8,59E-03 | 4,17E-03 | 1,21E-12 |
| 25 | 1,31E-01 | 7,32E-03 | 6,68E-03 | 1,51E-03 | 1,22E-12 |
| 26 | 9,53E-02 | 5,07E-03 | 6,50E-03 | 9,57E-03 | 1,79E-12 |
| 27 | 1,89E-02 | 8,78E-03 | 1,36E-04 | 3,10E-04 | 2,12E-12 |
| 28 | 1,14E-01 | 4,77E-02 | 1,85E-01 | 4,19E-01 | 2,48E-12 |
| 29 | 3,21E-02 | 1,60E-05 | 3,06E-01 | 2,70E-04 | 3,74E-12 |
| 30 | 4,18E-03 | 1,07E-03 | 6,72E-03 | 1,01E-01 | 4,43E-12 |
| 31 | 8,38E-01 | 7,16E-05 | 2,64E-01 | 1,39E-03 | 5,33E-12 |
| 32 | 6,86E-02 | 3,50E-03 | 3,93E-03 | 1,13E-02 | 6,00E-12 |
| 33 | 5,18E-03 | 1,07E-03 | 8,59E-03 | 1,13E-01 | 7,28E-12 |
| 34 | 9,58E-01 | 1,06E-02 | 1,47E-01 | 6,26E-03 | 7,48E-12 |
| 35 | 8,10E-02 | 4,28E-03 | 4,05E-03 | 8,08E-03 | 9,72E-12 |
| 36 | 8,10E-02 | 4,28E-03 | 4,05E-03 | 8,08E-03 | 9,72E-12 |
| 37 | 8,10E-02 | 4,28E-03 | 4,05E-03 | 8,08E-03 | 9,72E-12 |
| 38 | 8,10E-02 | 4,28E-03 | 4,05E-03 | 1,56E-02 | 9,72E-12 |
| 39 | 8,13E-02 | 7,24E-03 | 5,04E-03 | 9,57E-03 | 9,74E-12 |
| 40 | 8,13E-02 | 5,09E-03 | 5,04E-03 | 1,83E-02 | 9,74E-12 |
| 41 | 8,13E-02 | 7,24E-03 | 5,04E-03 | 9,57E-03 | 9,74E-12 |
| 42 | 8,13E-02 | 7,24E-03 | 5,04E-03 | 9,57E-03 | 9,74E-12 |
| 43 | 4,13E-03 | 1,98E-03 | 1,07E-02 | 1,29E-01 | 1,06E-11 |

(continued)

| No. | IMP_pv_adj | LVX_pv_adj | MER_pv_adj | P/T_pv_adj | TO_pv_adj |
|---|---|---|---|---|---|
| 44 | 2,49E-03 | 1,32E-03 | 4,07E-03 | 1,01E-01 | 1,06E-11 |
| 45 | 2,49E-03 | 1,98E-03 | 4,07E-03 | 7,86E-02 | 1,06E-11 |
| 46 | 6,61E-03 | 1,62E-03 | 6,66E-03 | 3,47E-02 | 1,13E-11 |
| 47 | 6,86E-02 | 5,04E-03 | 3,93E-03 | 1,56E-02 | 1,64E-11 |
| 48 | 1,92E-03 | 1,62E-03 | 5,24E-03 | 1,28E-01 | 1,85E-11 |
| 49 | 3,22E-03 | 1,08E-03 | 6,76E-03 | 7,83E-02 | 1,86E-11 |
| 50 | 6,52E-03 | 4,34E-03 | 1,32E-02 | 2,40E-01 | 1,87E-11 |

Table 15f: Data for Pseudomonas, particularly Pseudomonas aeruginosa (continued)

| No. | Locus.Tag | Gene. Symbol | GenBank.Accession | Chromosome |
|---|---|---|---|---|
| 1 | PSPA7_5342 | | YP_001350673 | |
| 2 | PSPA7_5335 | | YP_001350666 | |
| 3 | PA39016_001160000 | | ZP_07794022 | |
| 4 | PputW619_2322 | | YP_001749191 | |
| 5 | PSPA7_0102 | | YP_001345498 | |
| 6 | PSTAB_1238 | merR | YP_004713608 | |
| 7 | PPS_5233 | | YP_004704640 | |
| 8 | PA39016_001640030 | | ZP_07794960 | |
| 9 | PaerC_01001712 | | ZP_00968897 | |
| 10 | PA39016_001140033 | | ZP_07794005 | |
| 11 | PA39016_001790028 | | ZP_07795388 | |
| 12 | Paer2_01003738 | | ZP_00973073 | |
| 13 | PA39016_000110208 | | ZP_07792174 | |
| 14 | Paer2_01005870 | | ZP_00970967 | |
| 15 | PSPA7_0117 | | YP_001345513 | |
| 16 | PSPA7_3727 | | YP_001349081 | |
| 17 | PA39016_001140034 | | ZP_07794006 | |
| 18 | PSPA7_5254 | | YP_001350586 | |
| 19 | PputW619_2321 | | YP_001749190 | |
| 20 | PSPA7_ 4822 | | YP_001350163 | |
| 21 | Paer2_01002125 | | ZP_00974743 | |
| 22 | PaerPAb_010100012576 | | ZP_06878458 | |
| 23 | PA14_15470 | merP | YP_789375 | |
| 24 | Paer2_01002118 | | ZP_00974736 | |
| 25 | PaerPA_01003088 | | ZP_01365958 | |
| 26 | PLES_26911 | | YP_002440283 | |
| 27 | PaerPA_01003092 | | ZP_01365962 | |

(continued)

| No. | Locus.Tag | Gene. Symbol | GenBank.Accession | Chromosome |
|---|---|---|---|---|
| 28 | Paer2_01002142 | | ZP_00974760 | |
| 29 | PSPA7_0357 | | YP_001345752 | |
| 30 | PA39016_001640027 | | ZP_07794957 | |
| 31 | PA14_49520 | pyoS3A | YP_792115 | |
| 32 | PaerPA_01003114 | | ZP_01365983 | |
| 33 | PSPA7_3718 | | YP_001349072 | |
| 34 | PSPA7_0113 | merP1 | YP_001345509 | |
| 35 | Paer2_01002136 | | ZP_00974754 | |
| 36 | PA39016_001010047 | | ZP_07793693 | |
| 37 | PaerPA_01003095 | | ZP_01365965 | |
| 38 | PaerPA_01003132 | | ZP_01366001 | |
| 39 | Paer2_01002121 | | ZP_00974739 | |
| 40 | PLES_26591 | | YP_002440252 | |
| 41 | PSTAA_1280 | | AEA83190 | |
| 42 | PaerPA_01003094 | | ZP_01365964 | |
| 43 | PA39016_001650006 | | ZP_07794967 | |
| 44 | PA39016_001650001 | | ZP_07794962 | |
| 45 | PA14_30900 | | YP_790630 | |
| 46 | Paer2_01002387 | | ZP_00974497 | |
| 47 | Paer2_01002180 | | ZP_00974798 | |
| 48 | Avin_35690 | trbE | YP_002800691 | |
| 49 | PA39016_001650003 | | ZP_07794964 | |
| 50 | PSPA7_3698 | | YP_001349052 | |

Table 15g: Data for Pseudomonas, particularly Pseudomonas aeruginosa (continued)

| No. | Start.Coord | End.Coord | Strand | Scaffold.ID |
|---|---|---|---|---|
| 1 | 5509888 | 5510901 | + | 640753026 |
| 2 | 5497616 | 5498380 | - | 640753026 |
| 3 | 269 | 829 | + | 649999021 |
| 4 | 2600850 | 2601410 | - | 641522670 |
| 5 | 112316 | 112732 | + | 640753026 |
| 6 | 1298354 | 1298788 | + | 650716170 |
| 7 | 5930458 | 5930823 | - | 650716164 |
| 8 | 29403 | 29642 | - | 649999069 |
| 9 | 9109 | 10122 | - | 638362473 |
| 10 | 35241 | 35606 | - | 649999019 |
| 11 | 25607 | 28270 | + | 649999084 |

(continued)

| No. | Start.Coord | End.Coord | Strand | Scaffold.ID |
|-----|-------------|-----------|--------|-------------|
| 12 | 18240 | 19688 | - | 638362609 |
| 13 | 212874 | 213821 | - | 649998930 |
| 14 | 22370 | 34666 | + | 638362642 |
| 15 | 122819 | 123664 | + | 640753026 |
| 16 | 3864574 | 3865185 | - | 640753026 |
| 17 | 35624 | 37309 | - | 649999019 |
| 18 | 5409448 | 5411001 | - | 640753026 |
| 19 | 2597880 | 2600846 | - | 641522670 |
| 20 | 4961575 | 4962753 | - | 640753026 |
| 21 | 167495 | 167935 | + | 638362589 |
| 22 | 28 | 241 | - | 647002430 |
| 23 | 1310460 | 1310735 | - | 639279323 |
| 24 | 161059 | 161934 | + | 638362589 |
| 25 | 3500513 | 3501292 | + | 638372930 |
| 26 | 2885013 | 2885837 | - | 643348637 |
| 27 | 3505406 | 3506134 | + | 638372930 |
| 28 | 182786 | 184474 | + | 638362589 |
| 29 | 366452 | 367480 | - | 640753026 |
| 30 | 26408 | 27688 | - | 649999069 |
| 31 | 4403159 | 4405534 | - | 639279323 |
| 32 | 3528459 | 3529049 | + | 638372930 |
| 33 | 3855983 | 3856195 | - | 640753026 |
| 34 | 121405 | 121680 | - | 640753026 |
| 35 | 179158 | 179895 | + | 638362589 |
| 36 | 48194 | 50227 | + | 649999006 |
| 37 | 3507452 | 3509473 | + | 638372930 |
| 38 | 3547764 | 3548102 | + | 638372930 |
| 39 | 163874 | 164434 | + | 638362589 |
| 40 | 2856655 | 2857038 | - | 643348637 |
| 41 | 1367161 | 1367601 | - | 651053081 |
| 42 | 3506732 | 3507172 | + | 638372930 |
| 43 | 6653 | 8650 | - | 649999070 |
| 44 | 2001 | 4451 | - | 649999070 |
| 45 | 2683111 | 2683836 | - | 639279323 |
| 46 | 138340 | 138654 | - | 638362591 |
| 47 | 214185 | 216386 | + | 638362589 |
| 48 | 3640230 | 3642680 | - | 643692060 |
| 49 | 4730 | 5116 | - | 649999070 |

(continued)

| No. | Start.Coord | End.Coord | Strand | Scaffold.ID |
|---|---|---|---|---|
| 50 | 3833901 | 3834893 | - | 640753026 |

Table 16a: Data for Pseudomonas, particularly Pseudomonas aeruginosa

| No. | Scaffold.External. Accession | Scaffold.Name | best_pv |
|---|---|---|---|
| 1 | NC_009656 | Pseudomonas aeruginosa PA7: NC_009656 | 4.52939243363163e-78 |
| 2 | NC_009656 | Pseudomonas aeruginosa PA7: NC_ 009656 | 2.75541175953672e-58 |
| 3 | NZ_AEEX01000100 | Pseudomonas aeruginosa 39016 contig00116: NZ_ AEEX01000100 | 4.23483908607088e-28 |
| 4 | NC_010501 | Pseudomonas putida W619: NC_010501 | 2.9087092265635e-26 |
| 5 | NC_009656 | Pseudomonas aeruginosa PA7: NC_009656 | 5.61705273260437e-22 |
| 6 | NC_015740 | Pseudomonas stutzeri ATCC 17588 = LMG 11199 chromosome: NC_015740 | 5.61705273260437e-22 |
| 7 | NC_015733 | Pseudomonas putida S16 chromosome: NC_015733 | 1.91188334380809e-20 |
| 8 | NZ_AEEX01000148 | Pseudomonas aeruginosa 39016 contig00164: NZ_ AEEX01000148 | 4.25099609715652e-20 |
| 9 | NZ_AAKV01000030 | Pseudomonas aeruginosa C3719, unfinished sequence: NZ_AAKV01000030 | 2.79764535555464e-19 |
| 10 | NZ_AEEX01000098 | Pseudomonas aeruginosa 39016 contig00114: NZ_ AEEX01000098 | 4.15595600170243e-19 |
| 11 | NZ_AEEX01000163 | Pseudomonas aeruginosa 39016 contig00179: NZ_ AEEX01000163 | 4.72113166087411e-19 |
| 12 | NZ_AAKW01000042 | Pseudomonas aeruginosa 2192, unfinished sequence: NZ_AAKW01000042 | 2.33370938002383e-17 |
| 13 | NZ_AEEX01000009 | Pseudomonas aeruginosa 39016 contig00011: NZ_ AEEX01000009 | 3.60022886576205e-17 |
| 14 | NZ_AAKW01000075 | Pseudomonas aeruginosa 2192, unfinished sequence: NZ_AAKW01000075 | 1.13322970340506e-14 |
| 15 | NC_009656 | Pseudomonas aeruginosa PA7: NC_ 009656 | 2.33016029331653e-14 |
| 16 | NC_009656 | Pseudomonas aeruginosa PA7: NC_009656 | 2.33822187391836e-14 |
| 17 | NZ_AEEX01000098 | Pseudomonas aeruginosa 39016 contig00114: NZ_ AEEX01000098 | 2.91419614855849e-14 |
| 18 | NC_009656 | Pseudomonas aeruginosa PA7: NC_009656 | 5.5018212149287e-14 |
| 19 | NC_010501 | Pseudomonas putida W619: NC_010501 | 6.06970436387873e-14 |
| 20 | NC_009656 | Pseudomonas aeruginosa PA7: NC_ 009656 | 7.75377932078369e-14 |
| 21 | NZ_AAKW01000022 | Pseudomonas aeruginosa 2192, unfinished sequence: NZ_AAKW01000022 | 3.34337634494896e-13 |
| 22 | NZ_ABKZ01000250 | Pseudomonas aeruginosa PAb1 NZ_ ABKZ01000250: NZ_ABKZ01000250 | 6.17495854397665e-13 |
| 23 | NC_008463 | Pseudomonas aeruginosa UCBPP-PA14: NC_ 008463 | 6.42896969501816e-13 |

(continued)

| No. | Scaffold.External. Accession | Scaffold.Name | best_pv |
|---|---|---|---|
| 24 | NZ_AAKW01000022 | Pseudomonas aeruginosa 2192, unfinished sequence: NZ_AAKW01000022 | 1.20774339924326e-12 |
| 25 | NZ_AAQW01000001 | Pseudomonas aeruginosa PACS2, unfinished sequence: NZ_AAQW01000001 | 1.22221957191107e-12 |
| 26 | NC_011770 | Pseudomonas aeruginosa LESB58: NC_011770 | 1.79185026742242e-12 |
| 27 | NZ_AAQW01000001 | Pseudomonas aeruginosa PACS2, unfinished sequence: NZ_AAQW01000001 | 2.12378785336506e-12 |
| 28 | NZ_AAKW01000022 | Pseudomonas aeruginosa 2192, unfinished sequence: NZ_AAKW01000022 | 2.4812070369021e-12 |
| 29 | NC_009656 | Pseudomonas aeruginosa PA7: NC_009656 | 3.74499379826048e-12 |
| 30 | NZ_AEEX01000148 | Pseudomonas aeruginosa 39016 contig00164: NZ_AEEX01000148 | 4.42748938639645e-12 |
| 31 | NC_008463 | Pseudomonas aeruginosa UCBPP-PA14: NC_008463 | 5.32658560222593e-12 |
| 32 | NZ_AAQW01000001 | Pseudomonas aeruginosa PACS2, unfinished sequence: NZ_AAQW01000001 | 5.99725940121888e-12 |
| 33 | NC_009656 | Pseudomonas aeruginosa PA7: NC_ 009656 | 7.28057447109574e-12 |
| 34 | NC_009656 | Pseudomonas aeruginosa PA7: NC_009656 | 7.48144363663932e-12 |
| 35 | NZ_AAKW01000022 | Pseudomonas aeruginosa 2192, unfinished sequence: NZ_AAKW01000022 | 9.72376870484031e-12 |
| 36 | NZ_AEEX01000085 | Pseudomonas aeruginosa 39016 contig00101: NZ_AEEX01000085 | 9.72376870484031e-12 |
| 37 | NZ_AAQW01000001 | Pseudomonas aeruginosa PACS2, unfinished sequence: NZ_AAQW01000001 | 9.72376870484031e-12 |
| 38 | NZ_AAQW01000001 | Pseudomonas aeruginosa PACS2, unfinished sequence: NZ_AAQW01000001 | 9.72376870484031e-12 |
| 39 | NZ_AAKW01000022 | Pseudomonas aeruginosa 2192, unfinished sequence: NZ_AAKW01000022 | 9.73896479060769e-12 |
| 40 | NC_011770 | Pseudomonas aeruginosa LESB58: NC_011770 | 9.73896479060769e-12 |
| 41 | CP002622 | Pseudomonas stutzeri DSM 4166: CP002622 | 9.73896479060769e-12 |
| 42 | NZ_AAQW01000001 | Pseudomonas aeruginosa PACS2, unfinished sequence: NZ_AAQW01000001 | 9.73896479060769e-12 |
| 43 | NZ_AEEX01000149 | Pseudomonas aeruginosa 39016 contig00165: NZ_AEEX01000149 | 1.05759735249337e-11 |
| 44 | NZ_AEEX01000149 | Pseudomonas aeruginosa 39016 contig00165: NZ_AEEX01000149 | 1.05759735249337e-11 |
| 45 | NC_008463 | Pseudomonas aeruginosa UCBPP-PA14: NC_008463 | 1.05759735249337e-11 |
| 46 | NZ_AAKW01000024 | Pseudomonas aeruginosa 2192, unfinished sequence: NZ_AAKW01000024 | 1.13412272447338e-11 |
| 47 | NZ_AAKW01000022 | Pseudomonas aeruginosa 2192, unfinished sequence: NZ_AAKW01000022 | 1.64096469464127e-11 |

(continued)

| No. | Scaffold.External. Accession | Scaffold.Name | best_pv |
|---|---|---|---|
| 48 | NC_012560 | Azotobacter vinelandii DJ: NC_012560 | 1.85224764530357e-11 |
| 49 | NZ_AEEX01000149 | Pseudomonas aeruginosa 39016 contig00165: NZ_AEEX01000149 | 1.86265894165925e-11 |
| 50 | NC_009656 | Pseudomonas aeruginosa PA7: NC_ 009656 | 1.8721278417616e-11 |

Table 16b: Data for Pseudomonas, particularly Pseudomonas aeruginosa (continued)

| No. | sign_phenos | sign_phenos_class | best_pheno | best_pheno_class |
|---|---|---|---|---|
| 1 | AZT;CAZ;CP;CPE;GM;IMP;LVX;MER;P/T;TO | aminoglycoside;fluoroquinolone;lactam | TO | aminoglycoside |
| 2 | AZT;CAZ;CP;CPE;GM;IMP;LVX;MER;P/T;TO | aminoglycoside;fluoroquinolone;lactam | TO | aminoglycoside |
| 3 | CP;CPE;GM;LVX;P/T;TO | aminoglycoside;fluoroquinolone;lactam | TO | aminoglycoside |
| 4 | CP;CPE;GM;LVX;P/T;TO | aminoglycoside;fluoroquinolone;lactam | TO | aminoglycoside |
| 5 | CP;GM;LVX;TO | aminoglycoside;fluoroquinolone | TO | aminoglycoside |
| 6 | CP;GM;LVX;TO | aminoglycoside;fluoroquinolone | TO | aminoglycoside |
| 7 | CP;GM;LVX;TO | aminoglycoside;fluoroquinolone | TO | aminoglycoside |
| 8 | CP;GM;LVX;MER;P/T;TO | aminoglycoside;fluoroquinolone;lactam | TO | aminoglycoside |
| 9 | CAZ;CP;CPE;GM;LVX;MER;P/T;TO | aminoglycoside;fluoroquinolone;lactam | TO | aminoglycoside |
| 10 | CP;GM;TO | aminoglycoside;fluoroquinolone | TO | aminoglycoside |
| 11 | AZT;CAZ;CP;CPE;GM;LVX;P/T;TO | aminoglycoside;fluoroquinolone;lactam | TO | aminoglycoside |
| 12 | CAZ;CP;CPE;GM;LVX;P/T;TO | aminoglycoside;fluoroquinolone;lactam | TO | aminoglycoside |
| 13 | AZT;CAZ;CP;CPE;GM;LVX;P/T;TO | aminoglycoside;fluoroquinolone;lactam | TO | aminoglycoside |
| 14 | CP;GM;LVX;TO | aminoglycoside;fluoroquinolone | TO | aminoglycoside |
| 15 | CAZ;CP;CPE;GM;LVX;P/T;TO | aminoglycoside;fluoroquinolone;lactam | TO | aminoglycoside |
| 16 | CP;GM;IMP;LVX;MER;TO | aminoglycoside;fluoroquinolone;lactam | TO | aminoglycoside |
| 17 | CP;GM;TO | aminoglycoside;fluoroquinolone | TO | aminoglycoside |
| 18 | CAZ;CP;CPE;GM;LVX;P/T;TO | aminoglycoside;fluoroquinolone;lactam | TO | aminoglycoside |
| 19 | CP;GM;LVX;TO | aminoglycoside;fluoroquinolone | TO | aminoglycoside |
| 20 | CP;GM;LVX;TO | aminoglycoside;fluoroquinolone | TO | aminoglycoside |

(continued)

| No. | sign_phenos | sign_phenos_class | best_pheno | best_pheno_class |
|---|---|---|---|---|
| 21 | CAZ;CP;GM;MER;P/T;TO | aminoglycoside;fluoroquinolone; lactam | TO | aminoglycoside |
| 22 | CP;GM;IMP;LVX;MER;TO | aminoglycoside;fluoroquinolone; lactam | TO | aminoglycoside |
| 23 | CP;GM;TO | aminoglycoside;fluoroquinolone | TO | aminoglycoside |
| 24 | CAZ;CP;GM;MER;P/T;TO | aminoglycoside;fluoroquinolone; lactam | TO | aminoglycoside |
| 25 | CAZ;CP;GM;LVX;MER;P/T;TO | aminoglycoside;fluoroquinolone; lactam | TO | aminoglycoside |
| 26 | CAZ;CP;GM;LVX;MER;P/T;TO | aminoglycoside;fluoroquinolone; lactam | TO | aminoglycoside |
| 27 | CAZ;CP;GM;LVX;MER;P/T;TO | aminoglycoside;fluoroquinolone; lactam | TO | aminoglycoside |
| 28 | CP;GM;TO | aminoglycoside;fluoroquinolone | TO | aminoglycoside |
| 29 | CP;CPE;GM;LVX;P/T;TO | aminoglycoside;fluoroquinolone; lactam | TO | aminoglycoside |
| 30 | CP;GM;IMP;LVX;MER;TO | aminoglycoside;fluoroquinolone; lactam | TO | aminoglycoside |
| 31 | AZT;CAZ;CP;CPE;GM;LVX;P/T; TO | aminoglycoside;fluoroquinolone; lactam | TO | aminoglycoside |
| 32 | CAZ;CP;GM;LVX;MER;TO | aminoglycoside;fluoroquinolone; lactam | TO | aminoglycoside |
| 33 | CP;GM;IMP;LVX;MER;TO | aminoglycoside;fluoroquinolone; lactam | TO | aminoglycoside |
| 34 | CAZ;CP;CPE;GM;P/T;TO | aminoglycoside;fluoroquinolone; lactam | TO | aminoglycoside |
| 35 | CAZ;CP;GM;LVX;MER;P/T;TO | aminoglycoside;fluoroquinolone; lactam | TO | aminoglycoside |
| 36 | CAZ;CP;GM;LVX;MER;P/T;TO | aminoglycoside;fluoroquinolone; lactam | TO | aminoglycoside |
| 37 | CAZ;CP;GM;LVX;MER;P/T;TO | aminoglycoside;fluoroquinolone; lactam | TO | aminoglycoside |
| 38 | CAZ;CP;GM;LVX;MER;TO | aminoglycoside;fluoroquinolone; lactam | TO | aminoglycoside |
| 39 | CAZ;CP;GM;LVX;MER;P/T;TO | aminoglycoside;fluoroquinolone; lactam | TO | aminoglycoside |
| 40 | CAZ;CP;GM;LVX;MER;TO | aminoglycoside;fluoroquinolone; lactam | TO | aminoglycoside |
| 41 | CAZ;CP;GM;LVX;MER;P/T;TO | aminoglycoside;fluoroquinolone; lactam | TO | aminoglycoside |
| 42 | CAZ;CP;GM;LVX;MER;P/T;TO | aminoglycoside;fluoroquinolone; lactam | TO | aminoglycoside |

(continued)

| No. | sign_phenos | sign_phenos_class | best_pheno | best_pheno_class |
|---|---|---|---|---|
| 43 | CP;GM;IMP;LVX;TO | aminoglycoside;fluoroquinolone; lactam | TO | aminoglycoside |
| 44 | CP;GM;IMP;LVX;MER;TO | aminoglycoside;fluoroquinolone; lactam | TO | aminoglycoside |
| 45 | CP;GM;IMP;LVX;MER;TO | aminoglycoside;fluoroquinolone; lactam | TO | aminoglycoside |
| 46 | CP;GM;IMP;LVX;MER;TO | aminoglycoside;fluoroquinolone; lactam | TO | aminoglycoside |
| 47 | CAZ;CP;GM;LVX;MER;TO | aminoglycoside;fluoroquinolone; lactam | TO | aminoglycoside |
| 48 | CP;GM;IMP;LVX;MER;TO | aminoglycoside;fluoroquinolone; lactam | TO | aminoglycoside |
| 49 | CP;GM;IMP;LVX;MER;TO | aminoglycoside;fluoroquinolone; lactam | TO | aminoglycoside |
| 50 | CP;GM;IMP;LVX;TO | aminoglycoside;fluoroquinolone; lactam | TO | aminoglycoside |

Table 16c: Data for Pseudomonas, particularly Pseudomonas aeruginosa (continued)

| No. | num_aminoglycoside | num_fluoroquinolone | num_lactam |
|---|---|---|---|
| 1 | 2 | 2 | 6 |
| 2 | 2 | 2 | 6 |
| 3 | 2 | 2 | 2 |
| 4 | 2 | 2 | 2 |
| 5 | 2 | 2 | 0 |
| 6 | 2 | 2 | 0 |
| 7 | 2 | 2 | 0 |
| 8 | 2 | 2 | 2 |
| 9 | 2 | 2 | 4 |
| 10 | 2 | 1 | 0 |
| 11 | 2 | 2 | 4 |
| 12 | 2 | 2 | 3 |
| 13 | 2 | 2 | 4 |
| 14 | 2 | 2 | 0 |
| 15 | 2 | 2 | 3 |
| 16 | 2 | 2 | 2 |
| 17 | 2 | 1 | 0 |
| 18 | 2 | 2 | 3 |
| 19 | 2 | 2 | 0 |
| 20 | 2 | 2 | 0 |

(continued)

| No. | num_aminoglycoside | num_fluoroquinolone | num_lactam |
|---|---|---|---|
| 21 | 2 | 1 | 3 |
| 22 | 2 | 2 | 2 |
| 23 | 2 | 1 | 0 |
| 24 | 2 | 1 | 3 |
| 25 | 2 | 2 | 3 |
| 26 | 2 | 2 | 3 |
| 27 | 2 | 2 | 3 |
| 28 | 2 | 1 | 0 |
| 29 | 2 | 2 | 2 |
| 30 | 2 | 2 | 2 |
| 31 | 2 | 2 | 4 |
| 32 | 2 | 2 | 2 |
| 33 | 2 | 2 | 2 |
| 34 | 2 | 1 | 3 |
| 35 | 2 | 2 | 3 |
| 36 | 2 | 2 | 3 |
| 37 | 2 | 2 | 3 |
| 38 | 2 | 2 | 2 |
| 39 | 2 | 2 | 3 |
| 40 | 2 | 2 | 2 |
| 41 | 2 | 2 | 3 |
| 42 | 2 | 2 | 3 |
| 43 | 2 | 2 | 1 |
| 44 | 2 | 2 | 2 |
| 45 | 2 | 2 | 2 |
| 46 | 2 | 2 | 2 |
| 47 | 2 | 2 | 2 |
| 48 | 2 | 2 | 2 |
| 49 | 2 | 2 | 2 |
| 50 | 2 | 2 | 1 |

Table 16d: Data for Pseudomonas, particularly Pseudomonas aeruginosa (continued)

| No. | AZT_pv_adj | CAZ_pv_adj | CP_pv_adj | CPE_pv_adj | GM_pv_adj |
|---|---|---|---|---|---|
| 1 | 4.68E-03 | 2.31E-09 | 4.27E-32 | 5.83E-17 | 1.14E-56 |
| 2 | 1.88E-03 | 1.86E-08 | 3.37E-28 | 4.30E-16 | 2.05E-42 |
| 3 | 4.88E-01 | 1.79E-02 | 4.13E-10 | 4.41E-04 | 7.91E-18 |
| 4 | 5.97E-01 | 1.23E-02 | 2.07E-09 | 2.52E-04 | 9.24E-17 |

(continued)

| No. | AZT_pv_adj | CAZ_pv_adj | CP_pv_adj | CPE_pv_adj | GM_pv_adj |
|---|---|---|---|---|---|
| 5 | 5.49E-01 | 5.49E-01 | 9.07E-06 | 1.26E-01 | 1.33E-11 |
| 6 | 5.49E-01 | 5.49E-01 | 9.07E-06 | 1.26E-01 | 1.33E-11 |
| 7 | 6.66E-01 | 3.70E-01 | 9.33E-05 | 1.41E-01 | 5.53E-11 |
| 8 | 9.33E-01 | 6.81E-02 | 1.30E-09 | 3.20E-02 | 9.74E-12 |
| 9 | 2.57E-01 | 3.98E-05 | 1.63E-09 | 1.56E-07 | 6.24E-16 |
| 10 | 7.47E-01 | 4.36E-01 | 2.70E-04 | 2.27E-01 | 5.68E-10 |
| 11 | 4.31E-03 | 4.22E-08 | 3.91E-13 | 2.77E-09 | 7.84E-16 |
| 12 | 3.06E-02 | 6.75E-05 | 1.29E-09 | 9.32E-06 | 1.77E-14 |
| 13 | 2.11E-03 | 3.40E-07 | 3.34E-10 | 7.91E-07 | 4.31E-14 |
| 14 | 6.90E-01 | 3.88E-02 | 8.36E-06 | 2.05E-02 | 1.97E-09 |
| 15 | 1.08E-01 | 8.48E-04 | 2.11E-07 | 1.99E-04 | 4.46E-13 |
| 16 | 8.07E-01 | 4.16E-02 | 3.85E-08 | 3.59E-02 | 1.19E-09 |
| 17 | 6.52E-01 | 4.40E-01 | 1.90E-04 | 5.44E-01 | 1.07E-07 |
| 18 | 1.20E-02 | 1.84E-06 | 1.64E-08 | 1.64E-07 | 4.99E-11 |
| 19 | 4.08E-01 | 1.28E-02 | 2.37E-05 | 3.15E-02 | 2.20E-09 |
| 20 | 9.48E-01 | 6.78E-02 | 9.58E-07 | 3.17E-02 | 1.32E-09 |
| 21 | 5.47E-01 | 9.24E-03 | 2.24E-04 | 5.63E-02 | 4.24E-08 |
| 22 | 8.08E-01 | 1.43E-01 | 2.42E-06 | 2.48E-01 | 1.01E-07 |
| 23 | 8.44E-01 | 2.48E-01 | 4.61E-04 | 3.25E-01 | 9.75E-07 |
| 24 | 4.14E-01 | 4.37E-03 | 1.78E-04 | 5.65E-02 | 1.49E-07 |
| 25 | 3.25E-01 | 1.51E-03 | 6.15E-05 | 2.63E-02 | 9.67E-08 |
| 26 | 6.54E-01 | 5.11E-03 | 1.38E-05 | 1.82E-02 | 5.63E-08 |
| 27 | 1.63E-01 | 1.65E-04 | 7.86E-05 | 1.59E-02 | 1.34E-09 |
| 28 | 7.69E-01 | 2.48E-01 | 7.44E-04 | 3.25E-01 | 2.80E-06 |
| 29 | 4.44E-01 | 6.54E-02 | 4.51E-05 | 8.97E-06 | 1.56E-07 |
| 30 | 9.82E-01 | 1.63E-01 | 3.28E-06 | 2.73E-01 | 2.89E-08 |
| 31 | 7.11E-03 | 2.08E-05 | 1.73E-08 | 1.31E-06 | 7.90E-09 |
| 32 | 4.78E-01 | 7.49E-03 | 4.49E-05 | 2.57E-02 | 2.96E-07 |
| 33 | 6.55E-01 | 1.81E-01 | 8.24E-06 | 2.97E-01 | 2.97E-07 |
| 34 | 5.00E-01 | 5.68E-03 | 8.53E-03 | 7.20E-03 | 1.63E-11 |
| 35 | 4.77E-01 | 4.27E-03 | 6.00E-05 | 2.17E-02 | 9.67E-08 |
| 36 | 4.77E-01 | 4.27E-03 | 6.00E-05 | 2.17E-02 | 9.67E-08 |
| 37 | 4.77E-01 | 4.27E-03 | 6.00E-05 | 2.17E-02 | 9.67E-08 |
| 38 | 5.46E-01 | 9.06E-03 | 6.00E-05 | 3.01E-02 | 2.03E-07 |
| 39 | 4.78E-01 | 4.34E-03 | 1.28E-04 | 2.20E-02 | 1.35E-07 |
| 40 | 5.47E-01 | 9.22E-03 | 7.62E-05 | 3.04E-02 | 2.83E-07 |
| 41 | 4.78E-01 | 4.34E-03 | 1.28E-04 | 2.20E-02 | 1.35E-07 |
| 42 | 4.78E-01 | 4.34E-03 | 1.28E-04 | 2.20E-02 | 1.35E-07 |

(continued)

| No. | AZT_pv_adj | CAZ_pv_adj | CP_pv_adj | CPE_pv_adj | GM_pv_adj |
|---|---|---|---|---|---|
| 43 | 1 | 1.81E-01 | 8.55E-06 | 2.01E-01 | 4.71E-07 |
| 44 | 9.49E-01 | 1.43E-01 | 2.47E-06 | 2.48E-01 | 2.19E-07 |
| 45 | 1 | 1.12E-01 | 4.65E-06 | 2.01E-01 | 2.19E-07 |
| 46 | 9.82E-01 | 4.90E-02 | 6.77E-07 | 7.55E-02 | 2.07E-07 |
| 47 | 4.78E-01 | 7.49E-03 | 7.54E-05 | 2.57E-02 | 2.96E-07 |
| 48 | 9.49E-01 | 1.44E-01 | 6.20E-06 | 2.48E-01 | 4.67E-07 |
| 49 | 9.82E-01 | 1.43E-01 | 2.41E-06 | 1.62E-01 | 6.60E-07 |
| 50 | 7.30E-01 | 2.71E-01 | 1.97E-05 | 4.14E-01 | 1.33E-06 |

Table 16e: Data for Pseudomonas, particularly Pseudomonas aeruginosa (continued)

| No. | IMP_pv_adj | LVX_pv_adj | MER_pv_adj | P/T_pv_adj | TO_pv_adj |
|---|---|---|---|---|---|
| 1 | 1.35E-04 | 9.50E-27 | 2.29E-10 | 3.68E-18 | 4.53E-78 |
| 2 | 3.88E-03 | 4.29E-23 | 1.88E-05 | 3.33E-17 | 2.76E-58 |
| 3 | 4.33E-01 | 1.56E-06 | 1.57E-01 | 7.34E-04 | 4.23E-28 |
| 4 | 3.93E-01 | 4.01E-06 | 1.33E-01 | 9.25E-04 | 2.91E-26 |
| 5 | 2.74E-01 | 1.86E-03 | 8.61E-02 | 3.50E-01 | 5.62E-22 |
| 6 | 2.74E-01 | 1.86E-03 | 8.61E-02 | 3.50E-01 | 5.62E-22 |
| 7 | 5.97E-01 | 7.47E-03 | 1.01E-01 | 4.09E-01 | 1.91E-20 |
| 8 | 4.04E-02 | 2.96E-08 | 8.12E-03 | 3.01E-03 | 4.25E-20 |
| 9 | 4.48E-01 | 4.75E-07 | 8.35E-03 | 1.95E-05 | 2.80E-19 |
| 10 | 8.37E-01 | 1.48E-02 | 2 .27E-01 | 4.37E-01 | 4.16E-19 |
| 11 | 1 | 7.77E-10 | 2.74E-02 | 2.06E-06 | 4.72E-19 |
| 12 | 8.04E-01 | 2.34E-06 | 1.05E-01 | 5.83E-04 | 2.33E-17 |
| 13 | 1 | 1.18E-06 | 1.82E-01 | 5.92E-05 | 3.60E-17 |
| 14 | 2.13E-02 | 2.85E-04 | 4.99E-01 | 1.04E-02 | 1.13E-14 |
| 15 | 4.16E-01 | 1.60E-05 | 4.41E-01 | 1.42E-03 | 2.33E-14 |
| 16 | 1.11E-03 | 7.44E-05 | 3.68E-04 | 1.33E-02 | 2.34E-14 |
| 17 | 4.55E-01 | 3.37E-02 | 3.01E-01 | 5.40E-01 | 2.91E-14 |
| 18 | 9.48E-01 | 9.19E-07 | 1.68E-01 | 5.53E-05 | 5.50E-14 |
| 19 | 1 | 1.24E-03 | 2.34E-01 | 1.09E-01 | 6.07E-14 |
| 20 | 1.05E-02 | 1.87E-04 | 2.63E-01 | 3.01E-02 | 7.75E-14 |
| 21 | 1.13E-01 | 1.21E-02 | 5.24E-03 | 6.95E-03 | 3.34E-13 |
| 22 | 2.50E-03 | 3.35E-04 | 2.39E-03 | 1.14E-01 | 6.17E-13 |
| 23 | 9.76E-02 | 4.12E-02 | 1.60E-01 | 4.20E-01 | 6.43E-13 |
| 24 | 1.75E-01 | 1.21E-02 | 8.59E-03 | 4.17E-03 | 1.21E-12 |
| 25 | 1.31E-01 | 7.32E-03 | 6.68E-03 | 1.51E-03 | 1.22E-12 |
| 26 | 9.53E-02 | 5.07E-03 | 6.50E-03 | 9.57E-03 | 1.79E-12 |

(continued)

| No. | IMP_pv_adj | LVX_pv_adj | MER_pv_adj | P/T_pv_adj | TO_pv_adj |
|---|---|---|---|---|---|
| 27 | 1.89E-02 | 8.78E-03 | 1.36E-04 | 3.10E-04 | 2.12E-12 |
| 28 | 1.14E-01 | 4.77E-02 | 1.85E-01 | 4.19E-01 | 2.48E-12 |
| 29 | 3.21E-02 | 1.60E-05 | 3.06E-01 | 2.70E-04 | 3.74E-12 |
| 30 | 4.18E-03 | 1.07E-03 | 6.72E-03 | 1.01E-01 | 4.43E-12 |
| 31 | 8.38E-01 | 7.16E-05 | 2.64E-01 | 1.39E-03 | 5.33E-12 |
| 32 | 6.86E-02 | 3.50E-03 | 3.93E-03 | 1.13E-02 | 6.00E-12 |
| 33 | 5.18E-03 | 1.07E-03 | 8.59E-03 | 1.13E-01 | 7.28E-12 |
| 34 | 9.58E-01 | 1.06E-02 | 1.47E-01 | 6.26E-03 | 7.48E-12 |
| 35 | 8.10E-02 | 4.28E-03 | 4.05E-03 | 8.08E-03 | 9.72E-12 |
| 36 | 8.10E-02 | 4.28E-03 | 4.05E-03 | 8.08E-03 | 9.72E-12 |
| 37 | 8.10E-02 | 4.28E-03 | 4.05E-03 | 8.08E-03 | 9.72E-12 |
| 38 | 8.10E-02 | 4.28E-03 | 4.05E-03 | 1.56E-02 | 9.72E-12 |
| 39 | 8.13E-02 | 7.24E-03 | 5.04E-03 | 9.57E-03 | 9.74E-12 |
| 40 | 8.13E-02 | 5.09E-03 | 5.04E-03 | 1.83E-02 | 9.74E-12 |
| 41 | 8.13E-02 | 7.24E-03 | 5.04E-03 | 9.57E-03 | 9.74E-12 |
| 42 | 8.13E-02 | 7.24E-03 | 5.04E-03 | 9.57E-03 | 9.74E-12 |
| 43 | 4.13E-03 | 1.98E-03 | 1.07E-02 | 1.29E-01 | 1.06E-11 |
| 44 | 2.49E-03 | 1.32E-03 | 4.07E-03 | 1.01E-01 | 1.06E-11 |
| 45 | 2.49E-03 | 1.98E-03 | 4.07E-03 | 7.86E-02 | 1.06E-11 |
| 46 | 6.61E-03 | 1.62E-03 | 6.66E-03 | 3.47E-02 | 1.13E-11 |
| 47 | 6.86E-02 | 5.04E-03 | 3.93E-03 | 1.56E-02 | 1.64E-11 |
| 48 | 1.92E-03 | 1.62E-03 | 5.24E-03 | 1.28E-01 | 1.85E-11 |
| 49 | 3.22E-03 | 1.08E-03 | 6.76E-03 | 7.83E-02 | 1.86E-11 |
| 50 | 6.52E-03 | 4.34E-03 | 1.32E-02 | 2.40E-01 | 1.87E-11 |

Table 16f: Data for Pseudomonas, particularly Pseudomonas aeruginosa (continued)

| No. | Locus.Tag | Gene. Symbol | GenBank.Accession | Chromosome |
|---|---|---|---|---|
| 1 | PSPA7_5342 | | YP_001350673 | |
| 2 | PSPA7_5335 | | YP_001350666 | |
| 3 | PA39016_001160000 | | ZP_07794022 | |
| 4 | PputW619_2322 | | YP_001749191 | |
| 5 | PSPA7_0102 | | YP_001345498 | |
| 6 | PSTAB_1238 | merR | YP_004713608 | |
| 7 | PPS_5233 | | YP_004704640 | |
| 8 | PA39016_001640030 | | ZP_07794960 | |
| 9 | PaerC_01001712 | | ZP_00968897 | |
| 10 | PA39016_001140033 | | ZP_07794005 | |

(continued)

| No. | Locus.Tag | Gene. Symbol | GenBank.Accession | Chromosome |
|-----|-----------|--------------|-------------------|------------|
| 11 | PA39016_001790028 | | ZP_07795388 | |
| 12 | Paer2_01003738 | | ZP_00973073 | |
| 13 | PA39016_000110208 | | ZP_07792174 | |
| 14 | Paer2_01005870 | | ZP_00970967 | |
| 15 | PSPA7_0117 | | YP_001345513 | |
| 16 | PSPA7_3727 | | YP_001349081 | |
| 17 | PA39016_001140034 | | ZP_07794006 | |
| 18 | PSPA7_5254 | | YP_001350586 | |
| 19 | PputW619_2321 | | YP_001749190 | |
| 20 | PSPA7_ 4822 | | YP_001350163 | |
| 21 | Paer2_01002125 | | ZP_00974743 | |
| 22 | PaerPAb_010100012576 | | ZP_06878458 | |
| 23 | PA14_15470 | merP | YP_789375 | |
| 24 | Paer2_01002118 | | ZP_00974736 | |
| 25 | PaerPA_01003088 | | ZP_01365958 | |
| 26 | PLES_26911 | | YP_002440283 | |
| 27 | PaerPA_01003092 | | ZP_01365962 | |
| 28 | Paer2_01002142 | | ZP_00974760 | |
| 29 | PSPA7_0357 | | YP_001345752 | |
| 30 | PA39016_001640027 | | ZP_07794957 | |
| 31 | PA14_49520 | pyoS3A | YP_792115 | |
| 32 | PaerPA_01003114 | | ZP_01365983 | |
| 33 | PSPA7_3718 | | YP_001349072 | |
| 34 | PSPA7_0113 | merP1 | YP_001345509 | |
| 35 | Paer2_01002136 | | ZP_00974754 | |
| 36 | PA39016_001010047 | | ZP_07793693 | |
| 37 | PaerPA_01003095 | | ZP_01365965 | |
| 38 | PaerPA_01003132 | | ZP_01366001 | |
| 39 | Paer2_01002121 | | ZP_00974739 | |
| 40 | PLES_26591 | | YP_002440252 | |
| 41 | PSTAA_1280 | | AEA83190 | |
| 42 | PaerPA_01003094 | | ZP_01365964 | |
| 43 | PA39016_001650006 | | ZP_07794967 | |
| 44 | PA39016_001650001 | | ZP_07794962 | |
| 45 | PA14_30900 | | YP_790630 | |
| 46 | Paer2_01002387 | | ZP_00974497 | |
| 47 | Paer2_01002180 | | ZP_00974798 | |
| 48 | Avin_35690 | trbE | YP_002800691 | |

(continued)

| No. | Locus.Tag | Gene. Symbol | GenBank.Accession | Chromosome |
|---|---|---|---|---|
| 49 | PA39016_001650003 | | ZP_07794964 | |
| 50 | PSPA7_3698 | | YP_001349052 | |

Table 16g: Data for Pseudomonas, particularly Pseudomonas aeruginosa (continued)

| No. | Start.Coord | End.Coord | Strand | Scaffold.ID |
|---|---|---|---|---|
| 1 | 5509888 | 5510901 | + | 640753026 |
| 2 | 5497616 | 5498380 | - | 640753026 |
| 3 | 269 | 829 | + | 649999021 |
| 4 | 2600850 | 2601410 | - | 641522670 |
| 5 | 112316 | 112732 | + | 640753026 |
| 6 | 1298354 | 1298788 | + | 650716170 |
| 7 | 5930458 | 5930823 | - | 650716164 |
| 8 | 29403 | 29642 | - | 649999069 |
| 9 | 9109 | 10122 | - | 638362473 |
| 10 | 35241 | 35606 | - | 649999019 |
| 11 | 25607 | 28270 | + | 649999084 |
| 12 | 18240 | 19688 | - | 638362609 |
| 13 | 212874 | 213821 | - | 649998930 |
| 14 | 22370 | 34666 | + | 638362642 |
| 15 | 122819 | 123664 | + | 640753026 |
| 16 | 3864574 | 3865185 | - | 640753026 |
| 17 | 35624 | 37309 | - | 649999019 |
| 18 | 5409448 | 5411001 | - | 640753026 |
| 19 | 2597880 | 2600846 | - | 641522670 |
| 20 | 4961575 | 4962753 | - | 640753026 |
| 21 | 167495 | 167935 | + | 638362589 |
| 22 | 28 | 241 | - | 647002430 |
| 23 | 1310460 | 1310735 | - | 639279323 |
| 24 | 161059 | 161934 | + | 638362589 |
| 25 | 3500513 | 3501292 | + | 638372930 |
| 26 | 2885013 | 2885837 | - | 643348637 |
| 27 | 3505406 | 3506134 | + | 638372930 |
| 28 | 182786 | 184474 | + | 638362589 |
| 29 | 366452 | 367480 | - | 640753026 |
| 30 | 26408 | 27688 | - | 649999069 |
| 31 | 4403159 | 4405534 | - | 639279323 |
| 32 | 3528459 | 3529049 | + | 638372930 |

(continued)

| No. | Start.Coord | End.Coord | Strand | Scaffold.ID |
|---|---|---|---|---|
| 33 | 3855983 | 3856195 | - | 640753026 |
| 34 | 121405 | 121680 | - | 640753026 |
| 35 | 179158 | 179895 | + | 638362589 |
| 36 | 48194 | 50227 | + | 649999006 |
| 37 | 3507452 | 3509473 | + | 638372930 |
| 38 | 3547764 | 3548102 | + | 638372930 |
| 39 | 163874 | 164434 | + | 638362589 |
| 40 | 2856655 | 2857038 | - | 643348637 |
| 41 | 1367161 | 1367601 | - | 651053081 |
| 42 | 3506732 | 3507172 | + | 638372930 |
| 43 | 6653 | 8650 | - | 649999070 |
| 44 | 2001 | 4451 | - | 649999070 |
| 45 | 2683111 | 2683836 | - | 639279323 |
| 46 | 138340 | 138654 | - | 638362591 |
| 47 | 214185 | 216386 | + | 638362589 |
| 48 | 3640230 | 3642680 | - | 643692060 |
| 49 | 4730 | 5116 | - | 649999070 |
| 50 | 3833901 | 3834893 | - | 640753026 |

Example 9: Salmonella species

The procedure was carried out as in Example 1, except that the following microorganisms were used:

Bacterial Strains

The inventors selected 634 Salmonella strains, particularly from Salmonella_choleraesuis, Salmonella_dublin, Salmonella_enterica_ssp_arizonae, Salmonella_enterica_ssp_diarizoniae, Salmonella_enteritidis, Salmonella_gallinarum, Salmonella_Group_A, Salmonella_Group_B, Salmonella_Group_C, Salmonella_Group_D, Salmonella_heidelberg, Salmonella_miami, Salmonella_newport, Salmonella_panama, Salmonella_parahaemolyticus_A, Salmonella_paratyphi_A, Salmonella_paratyphi_B, Salmonella_pullorum, Salmonella_senfienberg, Salmonella_species, Salmonella_species_Lac_--,_ONPG_+, Salmonella_species_Lac_+,_ONPG_+, Salmonella_subgenus_I, Salmonella_subgenus_II, Salmonella_subgenus_IV, Salmonella_subgroup_I_Suc+, Salmonella_tennessee, and Salmonella_typhi, from the microbiology strain collection at Siemens Healthcare Diagnostics (West Sacramento, CA) for susceptibility testing and whole genome sequencing.

From MetaRef, 713 centroids of Salmonella species were used as reference sequences.

The results for Salmonella species are shown in Tables 17 (corresponding to Table 1) and 18 (corresponding to Table 2).

Table 17a: Data for Salmonella species

| No. | Scaffold.External.Accession | Scaffold.Name | best_pv |
|---|---|---|---|
| 1 | NC_011083 | Salmonella enterica subsp. enterica serovar Heidelberg str. SL476: NC_011083 | 1.09817680047592e-59 |

(continued)

| No. | Scaffold.External. Accession | Scaffold.Name | best_pv |
|---|---|---|---|
| 2 | NC_011092 | Salmonella enterica subsp. enterica serovar Schwarzengrund str. CVM19633 plasmid pCVM19633_110: NC_011092 | 3.48405564494782e-42 |
| 3 | NZ_ADUB01000261 | Escherichia coli MS 175-1 E_coli175-1-1.0_Cont542.3: NZ_ADUB01000261 | 2.50701590062067e-24 |
| 4 | NC_009651 | Klebsiella pneumoniae subsp. pneumoniae MGH 78578 plasmid pKPN5: NC_009651 | 3.64875434048827e-22 |
| 5 | NC_013509 | Edwardsiella tarda EIB202 plasmid pEIB202: NC_013509 | 1.44445847616457e-21 |
| 6 | NZ_ABFH01000001 | Salmonella enterica subsp. enterica serovar Virchow str. SL491, unfinished sequence: NZ_ABFH01000001 | 3.48310596778991e-21 |
| 7 | NC_011743 | Escherichia fergusonii ATCC 35469 plasmid pEFER: NC_011743 | 3.48310596778991e-21 |
| 8 | NZ_ADWV01000045 | Escherichia coli MS 107-1 E_coliMS107-1-1.0.1_Cont44.1: NZ_ADWV01000045 | 1.74745925214862e-20 |
| 9 | NC_013509 | Edwardsiella tarda EIB202 plasmid pEIB202: NC_013509 | 3.3130773442785e-20 |
| 10 | NZ_AAJX01000089 | Escherichia coli B171, unfinished sequence: NZ_AAJX01000089 | 5.485221922018e-20 |
| 11 | NZ_AFB001000625 | Klebsiella sp. MS 92-3 K_spMS92-3-1.0_Cont1175.2: NZ_AFBO01000625 | 7.41649956052229e-17 |
| 12 | NZ_ADTM01000311 | Escherichia coli MS 182-1 E_coli182-1-1.0_Cont460.3: NZ_ADTM01000311 | 8.87457605302667e-17 |
| 13 | CP001383 | Shigella flexneri 2002017: CP001383 | 5.28841759700461e-14 |
| 14 | NC_007384 | Shigella sonnei Ss046: NC_007384 | 5.8995130852235e-14 |
| 15 | NZ_ADUD01000537 | Escherichia coli MS 196-1 E_coli196-1-1.0_Cont1482.1: NZ_ADUD01000537 | 1.65276555063276e-13 |
| 16 | NZ_ABFH01000001 | Salmonella enterica subsp. enterica serovar Virchow str. SL491, unfinished sequence: NZ_ABFH01000001 | 1.65276555063276e-13 |
| 17 | NC_009650 | Klebsiella pneumoniae subsp. pneumoniae MGH 78578 plasmid pKPN4: NC_009650 | 3.61473686772869e-13 |
| 18 | NZ_ABFH01000001 | Salmonella enterica subsp. enterica serovar Virchow str. SL491, unfinished sequence: NZ_ABFH01000001 | 5.18796174101329e-13 |
| 19 | NZ_ADWV01000017 | Escherichia coli MS 107-1 E_coliMS107-1-1.0.1_Cont16.1: NZ_ADWV01000017 | 3.37689513091676e-12 |
| 20 | NZ_ADWT01000152 | Escherichia coli MS 124-1 E_coliMS124-1-1.0.1_Cont151.1: NZ_ADWT01000152 | 5.32683257781776e-12 |
| 21 | CP001383 | Shigella flexneri 2002017: CP001383 | 9.59412420701724e-12 |
| 22 | NC_011081 | Salmonella enterica subsp. enterica serovar Heidelberg str. SL476 plasmid pSL476_91: NC_011081 | 1.80214883490127e-11 |
| 23 | AP011957 | Salmonella enterica subsp. enterica serovar Typhimurium str. T000240 DNA: AP011957 | 2.02729069966733e-11 |
| 24 | NC_009650 | Klebsiella pneumoniae subsp. pneumoniae MGH 78578 plasmid pKPN4: NC_009650 | 2.02729069966733e-11 |

(continued)

| No. | Scaffold.External. Accession | Scaffold.Name | best_pv |
|---|---|---|---|
| 25 | NC_011092 | Salmonella enterica subsp. enterica serovar Schwarzengrund str. CVM19633 plasmid pCVM19633_110: NC_011092 | 3.62500325966221e-11 |
| 26 | NZ_ABKX01000001 | Escherichia albertii TW07627, unfinished sequence: NZ_ABKX01000001 | 5.50212585421897e-11 |
| 27 | CP002490 | Salmonella enterica subsp. enterica serovar Typhimurium str. 4/74 plasmid TY474p3: CP002490 | 1.68214594408478e-10 |
| 28 | NC_012124 | Salmonella enterica subsp. enterica serovar Paratyphi C strain | 8.57828076933948e-10 |
| | | RKS4594 plasmid pSPCV: NC_012124 | |
| 29 | NC_007385 | Shigella sonnei Ss046 plasmid pSS_046: NC_007385 | 1.06330274297554e-09 |
| 30 | NC_004851 | Shigella flexneri 2a str. 301 plasmid pCP301: NC_004851 | 2.2269317280314e-09 |
| 31 | NC_003277 | Salmonella typhimurium LT2 plasmid pSLT: NC_003277 | 2.2269317280314e-09 |
| 32 | CP001362 | Salmonella enterica subsp. enterica serovar Typhimurium str. 14028S plasmid: CP001362 | 1.72272938882188e-08 |
| 33 | NZ_ABKX01000001 | Escherichia albertii TW07627, unfinished sequence: NZ_ABKX01000001 | 1.8184047282463e-08 |
| 34 | NC_012124 | Salmonella enterica subsp. enterica serovar Paratyphi C strain RKS4594 plasmid pSPCV: NC_012124 | 3.5232749457209e-08 |
| 35 | NC_012125 | Salmonella enterica subsp. enterica serovar Paratyphi C strain RKS4594: NC_012125 | 4.21301623299358e-08 |
| 36 | CP002487 | Salmonella enterica subsp. enterica serovar Typhimurium str. 4/74: CP002487 | 5.49621728459414e-08 |
| 37 | NZ_ABEL01000010 | Salmonella enterica subsp. enterica serovar Heidelberg str. SL486, unfinished sequence: NZ_ABEL01000010 | 9.94168564125988e-08 |
| 38 | NC_012124 | Salmonella enterica subsp. enterica serovar Paratyphi C strain RKS4594 plasmid pSPCV: NC_012124 | 1.81471391611537e-07 |
| 39 | NC_012125 | Salmonella enterica subsp. enterica serovar Paratyphi C strain RKS4594: NC_012125 | 1.9714526298817e-07 |
| 40 | NC_003197 | Salmonella typhimurium LT2: NC_003197 | 2.16264005553646e-07 |
| 41 | CP002488 | Salmonella enterica subsp. enterica serovar Typhimurium str. 4/74 plasmid TY474p1: CP002488 | 2.16264005553646e-07 |
| 42 | NZ_ABAM01000046 | Salmonella enterica subsp. enterica serovar Saintpaul str. SARA23, unfinished sequence: NZ_ABAM01000046 | 2.29779633234294e-07 |
| 43 | NC_010102 | Salmonella enterica subsp. enterica serovar Paratyphi B str. SPB7: NC_010102 | 3.05541326329248e-07 |
| 44 | AP011957 | Salmonella enterica subsp. enterica serovar Typhimurium str. T000240 DNA: AP011957 | 3.48861961356893e-07 |
| 45 | NC_010067 | Salmonella enterica subsp. arizonae serovar 62:z4,z23:--: NC_ 010067 | 4.35325104483177e-07 |
| 46 | NC_010067 | Salmonella enterica subsp. arizonae serovar 62:z4,z23:--: NC_ 010067 | 4.35325104483177e-07 |
| 47 | NC_015761 | Salmonella bongori NCTC 12419: NC_015761 | 4.35325104483177e-07 |

(continued)

| No. | Scaffold.External. Accession | Scaffold.Name | best_pv |
|---|---|---|---|
| 48 | NZ_ ABEL01000010 | Salmonella enterica subsp. enterica serovar Heidelberg str. SL486, unfinished sequence: NZ_ABEL01000010 | 7.61833017744037e-07 |
| 49 | NC_012125 | Salmonella enterica subsp. enterica serovar Paratyphi C strain RKS4594: NC_012125 | 8.71713912669417e-07 |
| 50 | NZ_ ABEJ01000032 | Salmonella enterica subsp. enterica serovar Schwarzengrund str. SL480, unfinished sequence: NZ_ ABEJ01000032 | 8.71713912669417e-07 |

Table 17b: Data for Salmonella species (continued)

| No. | sign_phenos | sign_phenos_class | best_pheno | best_pheno_class |
|---|---|---|---|---|
| 1 | AM;A/S | lactam | AM | lactam |
| 2 | AM;A/S | lactam | AM | lactam |
| 3 | AM;A/S | lactam | AM | lactam |
| 4 | AM;A/S | lactam | A/S | lactam |
| 5 | AM;A/S | lactam | AM | lactam |
| 6 | AM;A/S | lactam | A/S | lactam |
| 7 | AM;A/S | lactam | AM | lactam |
| 8 | AM;A/S | lactam | A/S | lactam |
| 9 | AM;A/S | lactam | AM | lactam |
| 10 | AM;A/S | lactam | A/S | lactam |
| 11 | AM;A/S | lactam | A/S | lactam |
| 12 | AM;A/S | lactam | A/S | lactam |
| 13 | AM;A/S | lactam | AM | lactam |
| 14 | AM;A/S | lactam | A/S | lactam |
| 15 | AM;A/S | lactam | A/S | lactam |
| 16 | AM;A/S | lactam | A/S | lactam |
| 17 | AM;A/S | lactam | A/S | lactam |
| 18 | AM;A/S | lactam | A/S | lactam |
| 19 | AM;A/S | lactam | A/S | lactam |
| 20 | AM;A/S | lactam | AM | lactam |
| 21 | AM;A/S | lactam | AM | lactam |
| 22 | AM;A/S | lactam | AM | lactam |
| 23 | AM;A/S | lactam | A/S | lactam |
| 24 | AM;A/S | lactam | A/S | lactam |
| 25 | AM;A/S | lactam | A/S | lactam |
| 26 | AM;A/S | lactam | AM | lactam |
| 27 | AM;A/S | lactam | AM | lactam |
| 28 | AM;A/S | lactam | AM | lactam |

(continued)

| No. | sign_phenos | sign_phenos_class | best_pheno | best_pheno_class |
|---|---|---|---|---|
| 29 | AM;A/S | lactam | AM | lactam |
| 30 | AM;A/S | lactam | AM | lactam |
| 31 | AM;A/S | lactam | AM | lactam |
| 32 | AM;A/S | lactam | AM | lactam |
| 33 | AM;A/S | lactam | AM | lactam |
| 34 | AM;A/S | lactam | AM | lactam |
| 35 | AM;A/S | lactam | AM | lactam |
| 36 | AM;A/S | lactam | AM | lactam |
| 37 | AM;A/S | lactam | A/S | lactam |
| 38 | AM;A/S | lactam | AM | lactam |
| 39 | AM;A/S | lactam | AM | lactam |
| 40 | AM;A/S | lactam | AM | lactam |
| 41 | AM;A/S | lactam | AM | lactam |
| 42 | AM;A/S | lactam | A/S | lactam |
| 43 | AM;A/S | lactam | AM | lactam |
| 44 | AM;A/S | lactam | A/S | lactam |
| 45 | AM;A/S | lactam | A/S | lactam |
| 46 | AM;A/S | lactam | A/S | lactam |
| 47 | AM;A/S | lactam | A/S | lactam |
| 48 | AM;A/S | lactam | A/S | lactam |
| 49 | AM;A/S | lactam | A/S | lactam |
| 50 | AM;A/S | lactam | A/S | lactam |

Table 17c: Data for Salmonella species (continued)

| No. | num_lactam | AM_pv_adj | A/S_pv_adj |
|---|---|---|---|
| 1 | 2 | 1.10E-59 | 1.70E-55 |
| 2 | 2 | 3.48E-42 | 1.81E-40 |
| 3 | 2 | 2.51E-24 | 2.30E-22 |
| 4 | 2 | 1.08E-21 | 3.65E-22 |
| 5 | 2 | 1.44E-21 | 6.88E-20 |
| 6 | 2 | 1.05E-20 | 3.48E-21 |
| 7 | 2 | 3.48E-21 | 1.05E-20 |
| 8 | 2 | 5.22E-20 | 1.75E-20 |
| 9 | 2 | 3.31E-20 | 1.15E-19 |
| 10 | 2 | 1.65E-19 | 5.49E-20 |
| 11 | 2 | 1.74E-16 | 7.42E-17 |
| 12 | 2 | 2.98E-16 | 8.87E-17 |

(continued)

| No. | num_lactam | AM_pv_adj | A/S_pv_adj |
|-----|-----------|-----------|------------|
| 13 | 2 | 5.29E-14 | 1.45E-13 |
| 14 | 2 | 1.45E-13 | 5.90E-14 |
| 15 | 2 | 3.62E-13 | 1.65E-13 |
| 16 | 2 | 3.62E-13 | 1.65E-13 |
| 17 | 2 | 6.38E-13 | 3.61E-13 |
| 18 | 2 | 1.10E-12 | 5.19E-13 |
| 19 | 2 | 6.91E-12 | 3.38E-12 |
| 20 | 2 | 5.33E-12 | 3.21E-11 |
| 21 | 2 | 9.59E-12 | 8.71E-10 |
| 22 | 2 | 1.80E-11 | 6.37E-10 |
| 23 | 2 | 3.64E-11 | 2.03E-11 |
| 24 | 2 | 3.64E-11 | 2.03E-11 |
| 25 | 2 | 6.67E-11 | 3.63E-11 |
| 26 | 2 | 5.50E-11 | 4.78E-09 |
| 27 | 2 | 1.68E-10 | 1.06E-09 |
| 28 | 2 | 8.58E-10 | 2.16E-08 |
| 29 | 2 | 1.06E-09 | 2.10E-08 |
| 30 | 2 | 2.23E-09 | 3.77E-08 |
| 31 | 2 | 2.23E-09 | 3.77E-08 |
| 32 | 2 | 1.72E-08 | 2.02E-07 |
| 33 | 2 | 1.82E-08 | 1.76E-06 |
| 34 | 2 | 3.52E-08 | 8.59E-08 |
| 35 | 2 | 4.21E-08 | 6.33E-08 |
| 36 | 2 | 5.50E-08 | 7.64E-08 |
| 37 | 2 | 1.40E-07 | 9.94E-08 |
| 38 | 2 | 1.81E-07 | 3.88E-07 |
| 39 | 2 | 1.97E-07 | 2.71E-07 |
| 40 | 2 | 2.16E-07 | 2.91E-07 |
| 41 | 2 | 2.16E-07 | 7.75E-07 |
| 42 | 2 | 7.51E-07 | 2.30E-07 |
| 43 | 2 | 3.06E-07 | 8.87E-07 |
| 44 | 2 | 2.22E-06 | 3.49E-07 |
| 45 | 2 | 1.28E-06 | 4.35E-07 |
| 46 | 2 | 1.28E-06 | 4.35E-07 |
| 47 | 2 | 1.25E-06 | 4.35E-07 |
| 48 | 2 | 2.83E-06 | 7.62E-07 |
| 49 | 2 | 1.23E-06 | 8.72E-07 |
| 50 | 2 | 4.30E-06 | 8.72E-07 |

Table 17d: Data for Salmonella species (continued)

| No. | Locus.Tag | Gene. Symbol | GenBank.Accession | Chromosome |
|---|---|---|---|---|
| 1 | SeHA_C1580 | blaT | YP_002045448 | |
| 2 | SeSA_B0079 | | YP_002112958 | plasmid pCVM19633_110 |
| 3 | HMPREF9547_ 03104 | | ZP_07169558 | |
| 4 | KPN_pKPN5p08201 | | YP_001338811 | plasmid pKPN5 |
| 5 | ETAE_p041 | folP | YP_003297635 | plasmid pEIB202 |
| 6 | Salmoentericaenterica_010100000245 | | ZP_02702077 | |
| 7 | pEFER_0049 | | YP_002394596 | plasmid pEFER |
| 8 | HMPREF9345_ 05066 | | ZP_07100147 | |
| 9 | ETAE_p040 | | YP_003297634 | plasmid pEIB202 |
| 10 | EcolB_01004576 | | ZP_00708527 | |
| 11 | HMPREF9538_04737 | | ZP_08307036 | |
| 12 | HMPREF9548_ 03784 | | ZP_07141588 | |
| 13 | SFxv_1142 | | ADA73400 | |
| 14 | SSON_3896 | aadA | YP_312670 | |
| 15 | HMPREF9551_04611 | | ZP_07191964 | |
| 16 | Salmoentericaenterica_010100000130 | | ZP_02702054 | |
| 17 | KPN_pKPN4p07064 | | YP_001338673 | plasmid pKPN4 |
| 18 | Salmoentericaenterica_010100000105 | | ZP_02702049 | |
| 19 | HMPREF9345_03525 | | ZP_07098656 | |
| 20 | HMPREF9347_ 06051 | | ZP_07213485 | |
| 21 | SFxv_1141 | | ADA73399 | |
| 22 | SeHA_A0031 | | YP_002043869 | plasmid pSL476_91 |
| 23 | STMDT12_C39390 | | BAJ38882 | |
| 24 | KPN_pKPN4p07068 | merC | YP_001338677 | plasmid pKPN4 |
| 25 | SeSA_B0082 | | YP_002112961 | plasmid pCVM19633_110 |
| 26 | ESCAB7627_ 0644 | | ZP_02900823 | |
| 27 | STM474_p303 | repA | ADX20580 | plasmid TY474p3 |
| 28 | SPC_p013 | rsdB | YP_002635587 | plasmid pSPCV |
| 29 | SSO_P190 | traD | YP_313445 | plasmid pSS_046 |
| 30 | CP0245 | mvpA | NP_858378 | plasmid pCP301 |
| 31 | PSLT105 | trbH | NP_490589 | plasmid pSLT |
| 32 | STM14_5622 | traD | ACY86531 | |
| 33 | ESCAB7627_ 0650 | | ZP_02900555 | |
| 34 | SPC_p042 | traX | YP_002635616 | plasmid pSPCV |
| 35 | SPC_2707 | | YP_002638248 | |
| 36 | STM474_1038 | | ADX16744 | |
| 37 | Senterienterica_010100019467 | | ZP_02668762 | |

(continued)

| No. | Locus.Tag | Gene. Symbol | GenBank.Accession | Chromosome |
|---|---|---|---|---|
| 38 | SPC_p041 | finO | YP_002635615 | plasmid pSPCV |
| 39 | SPC_2708 | | YP_002638249 | |
| 40 | STM1046 | | NP_460021 | |
| 41 | STM474_p1093 | ccdB | ADX20463 | plasmid TY474p1 |
| 42 | Sentent_010100022581 | | ZP_02341659 | |
| 43 | SPAB_05276 | | YP_001591385 | |
| 44 | STMDT12_C10780 | | BAJ36021 | |
| 45 | SARI_01515 | | YP_001570553 | |
| 46 | SARI_01514 | | YP_001570552 | |
| 47 | SBG_1292 | malR | YP_004730162 | |
| 48 | Senterienterica_010100019192 | | ZP_02668707 | |
| 49 | SPC_0340 | | YP_002635966 | |
| 50 | Senteenterica_010100025269 | | ZP_02664569 | |

Table 17e: Data for Salmonella species (continued)

| No. | Start.Coord | End.Coord | Strand | Scaffold.ID |
|---|---|---|---|---|
| 1 | 1525960 | 1526820 | + | 642555218 |
| 2 | 68933 | 70240 | + | 642555219 |
| 3 | 3248 | 3841 | + | 648291919 |
| 4 | 34908 | 35255 | - | 640753021 |
| 5 | 32101 | 32916 | - | 646311959 |
| 6 | 36757 | 37062 | + | 641778223 |
| 7 | 48658 | 49503 | + | 643692014 |
| 8 | 4715 | 5554 | - | 648294624 |
| 9 | 31237 | 32040 | - | 646311959 |
| 10 | 3555 | 4568 | + | 638359041 |
| 11 | 1555 | 2055 | - | 651333542 |
| 12 | 8819 | 9535 | + | 648288593 |
| 13 | 1108351 | 1108854 | - | 646862363 |
| 14 | 4110458 | 4111246 | - | 640427102 |
| 15 | 97 | 531 | - | 648293053 |
| 16 | 19257 | 19691 | + | 641778223 |
| 17 | 23310 | 24614 | - | 640753020 |
| 18 | 16271 | 16636 | - | 641778223 |
| 19 | 1071 | 1307 | - | 648294596 |
| 20 | 812 | 1663 | - | 648294425 |
| 21 | 1107342 | 1107968 | + | 646862363 |

(continued)

| No. | Start.Coord | End.Coord | Strand | Scaffold.ID |
|-----|-------------|-----------|--------|-------------|
| 22 | 16416 | 17195 | + | 642555216 |
| 23 | 4088051 | 4088326 | - | 651053007 |
| 24 | 25970 | 27664 | - | 640753020 |
| 25 | 71061 | 71411 | - | 642555219 |
| 26 | 233492 | 234697 | - | 641784575 |
| 27 | 1988 | 2827 | - | 650378006 |
| 28 | 10060 | 10842 | - | 643692029 |
| 29 | 166425 | 168626 | + | 640427103 |
| 30 | 198117 | 198515 | - | 637000224 |
| 31 | 86566 | 87285 | + | 637000134 |
| 32 | 84297 | 86459 | + | 646862361 |
| 33 | 238788 | 239996 | - | 641784575 |
| 34 | 28413 | 29153 | - | 643692029 |
| 35 | 2745099 | 2745596 | - | 643692030 |
| 36 | 1088568 | 1089272 | + | 650378003 |
| 37 | 62341 | 62874 | - | 641777794 |
| 38 | 27795 | 28358 | - | 643692029 |
| 39 | 2745695 | 2746027 | - | 643692030 |
| 40 | 1131381 | 1132118 | + | 637000122 |
| 41 | 73221 | 73526 | - | 650378004 |
| 42 | 23575 | 23910 | - | 641746440 |
| 43 | 4445787 | 4448009 | + | 641228523 |
| 44 | 1141208 | 1141399 | - | 651053007 |
| 45 | 1476854 | 1478026 | + | 641228515 |
| 46 | 1475252 | 1476844 | + | 641228515 |
| 47 | 1414087 | 1415115 | + | 650716177 |
| 48 | 26832 | 28046 | + | 641777794 |
| 49 | 367421 | 368839 | + | 643692030 |
| 50 | 35858 | 36535 | - | 641777693 |

Table 18a: Data for Salmonella species

| No. | Scaffold.External. Accession | Scaffold.Name | best_pv |
|-----|------------------------------|---------------|---------|
| 1 | NC_011083 | Salmonella enterica subsp. enterica serovar Heidelberg str. SL476: NC_011083 | 1.09817680047592e-59 |
| 2 | NC_011092 | Salmonella enterica subsp. enterica serovar Schwarzengrund str. CVM19633 plasmid pCVM19633_110: NC_011092 | 3.48405564494782e-42 |

(continued)

| No. | Scaffold.External. Accession | Scaffold.Name | best_pv |
|---|---|---|---|
| 3 | NZ_ ADUB01000261 | Escherichia coli MS 175-1 E_coli175-1-1.0_Cont542.3: NZ_ADUB01000261 | 2.50701590062067e-24 |
| 4 | NC_009651 | Klebsiella pneumoniae subsp. pneumoniae MGH 78578 plasmid pKPN5: NC_009651 | 3.64875434048827e-22 |
| 5 | NC_013509 | Edwardsiella tarda EIB202 plasmid pEIB202: NC_013509 | 1.44445847616457e-21 |
| 6 | NZ_ ABFH01000001 | Salmonella enterica subsp. enterica serovar Virchow str. SL491, unfinished sequence: NZ_ABFH01000001 | 3.48310596778991e-21 |
| 7 | NC_011743 | Escherichia fergusonii ATCC 35469 plasmid pEFER: NC_ 011743 | 3.48310596778991e-21 |
| 8 | NZ_ ADWV01000045 | Escherichia coli MS 107-1 E_coliMS107-1-1.0.1_Cont44.1: NZ_ADWV01000045 | 1.74745925214862e-20 |
| 9 | NC_013509 | Edwardsiella tarda EIB202 plasmid pEIB202: NC_013509 | 3.3130773442785e-20 |
| 10 | NZ_ AAJX01000089 | Escherichia coli B171, unfinished sequence: NZ_ AAJX01000089 | 5.485221922018e-20 |
| 11 | NZ_ AFBO01000625 | Klebsiella sp. MS 92-3 K_spMS92-3-1.0_Cont1175.2: NZ_ AFBO01000625 | 7.41649956052229e-17 |
| 12 | NZ_ ADTM01000311 | Escherichia coli MS 182-1 E_coli182-1-1.0_Cont460.3: NZ_ADTM01000311 | 8.87457605302667e-17 |
| 13 | CP001383 | Shigella flexneri 2002017: CP001383 | 5.28841759700461e-14 |
| 14 | NC_007384 | Shigella sonnei Ss046: NC_007384 | 5.8995130852235e-14 |
| 15 | NZ_ ADUD01000537 | Escherichia coli MS 196-1 E_coli196-1-1.0_Cont1482.1: NZ_ADUD01000537 | 1.65276555063276e-13 |
| 16 | NZ_ ABFH01000001 | Salmonella enterica subsp. enterica serovar Virchow str. SL491, unfinished sequence: NZ_ABFH01000001 | 1.65276555063276e-13 |
| 17 | NC_009650 | Klebsiella pneumoniae subsp. pneumoniae MGH 78578 plasmid pKPN4: NC_009650 | 3.61473686772869e-13 |
| 18 | NZ_ ABFH01000001 | Salmonella enterica subsp. enterica serovar Virchow str. SL491, unfinished sequence: NZ_ABFH01000001 | 5.18796174101329e-13 |
| 19 | NZ_ ADWV01000017 | Escherichia coli MS 107-1 E_coliMS107-1-1.0.1_Cont16.1: NZ_ADWV01000017 | 3.37689513091676e-12 |
| 20 | NZ_ ADWT01000152 | Escherichia coli MS 124-1 E_ coliMS124-1-1.0.1_Cont151.1: NZ_ADWT01000152 | 5.32683257781776e-12 |
| 21 | CP001383 | Shigella flexneri 2002017: CP001383 | 9.59412420701724e-12 |
| 22 | NC_011081 | Salmonella enterica subsp. enterica serovar Heidelberg str. SL476 plasmid pSL476_91: NC_011081 | 1.80214883490127e-11 |
| 23 | AP011957 | Salmonella enterica subsp. enterica serovar Typhimurium str. T000240 DNA: AP011957 | 2.02729069966733e-11 |
| 24 | NC_009650 | Klebsiella pneumoniae subsp. pneumoniae MGH 78578 plasmid pKPN4: NC_009650 | 2.02729069966733e-11 |
| 25 | NC_011092 | Salmonella enterica subsp. enterica serovar Schwarzengrund str. CVM19633 plasmid pCVM19633_110: NC_011092 | 3.62500325966221e-11 |

(continued)

| No. | Scaffold.External. Accession | Scaffold.Name | best_pv |
|---|---|---|---|
| 26 | NZ_ABKX01000001 | Escherichia albertii TW07627, unfinished sequence: NZ_ABKX01000001 | 5.50212585421897e-11 |
| 27 | CP002490 | Salmonella enterica subsp. enterica serovar Typhimurium str. 4/74 plasmid TY474p3: CP002490 | 1.68214594408478e-10 |
| 28 | NC_012124 | Salmonella enterica subsp. enterica serovar Paratyphi C strain RKS4594 plasmid pSPCV: NC_012124 | 8.57828076933948e-10 |
| 29 | NC_007385 | Shigella sonnei Ss046 plasmid pSS_046: NC_007385 | 1.06330274297554e-09 |
| 30 | NC_004851 | Shigella flexneri 2a str. 301 plasmid pCP301: NC_004851 | 2.2269317280314e-09 |
| 31 | NC_003277 | Salmonella typhimurium LT2 plasmid pSLT: NC_003277 | 2.2269317280314e-09 |
| 32 | CP001362 | Salmonella enterica subsp. enterica serovar Typhimurium str. 14028S plasmid: CP001362 | 1.72272938882188e-08 |
| 33 | NZ_ABKX01000001 | Escherichia albertii TW07627, unfinished sequence: NZ_ABKX01000001 | 1.8184047282463e-08 |
| 34 | NC_012124 | Salmonella enterica subsp. enterica serovar Paratyphi C strain RKS4594 plasmid pSPCV: NC_012124 | 3.5232749457209e-08 |
| 35 | NC_012125 | Salmonella enterica subsp. enterica serovar Paratyphi C strain RKS4594: NC_012125 | 4.21301623299358e-08 |
| 36 | CP002487 | Salmonella enterica subsp. enterica serovar Typhimurium str. 4/74: CP002487 | 5.49621728459414e-08 |
| 37 | NZ_ABEL01000010 | Salmonella enterica subsp. enterica serovar Heidelberg str. SL486, unfinished sequence: NZ_ABEL01000010 | 9.94168564125988e-08 |
| 38 | NC_012124 | Salmonella enterica subsp. enterica serovar Paratyphi C strain RKS4594 plasmid pSPCV: NC_012124 | 1.81471391611537e-07 |
| 39 | NC_012125 | Salmonella enterica subsp. enterica serovar Paratyphi C strain RKS4594: NC_012125 | 1.9714526298817e-07 |
| 40 | NC_003197 | Salmonella typhimurium LT2: NC_003197 | 2.16264005553646e-07 |
| 41 | CP002488 | Salmonella enterica subsp. enterica serovar Typhimurium str. 4/74 plasmid TY474p1: CP002488 | 2.16264005553646e-07 |
| 42 | NZ_ABAM01000046 | Salmonella enterica subsp. enterica serovar Saintpaul str. SARA23, unfinished sequence: NZ_ABAM01000046 | 2.29779633234294e-07 |
| 43 | NC_010102 | Salmonella enterica subsp. enterica serovar Paratyphi B str. SPB7: NC_010102 | 3.05541326329248e-07 |
| 44 | AP011957 | Salmonella enterica subsp. enterica serovar Typhimurium str. T000240 DNA: AP011957 | 3.48861961356893e-07 |
| 45 | NC_010067 | Salmonella enterica subsp. arizonae serovar 62:z4,z23:--: NC_010067 | 4.35325104483177e-07 |
| 46 | NC_010067 | Salmonella enterica subsp. arizonae serovar 62:z4,z23:--: NC_010067 | 4.35325104483177e-07 |
| 47 | NC_015761 | Salmonella bongori NCTC 12419: NC_015761 | 4.35325104483177e-07 |
| 48 | NZ_ABEL01000010 | Salmonella enterica subsp. enterica serovar Heidelberg str. SL486, unfinished sequence: NZ_ABEL01000010 | 7.61833017744037e-07 |
| 49 | NC_012125 | Salmonella enterica subsp. enterica serovar Paratyphi C strain RKS4594: NC_012125 | 8.71713912669417e-07 |

(continued)

| No. | Scaffold.External. Accession | Scaffold.Name | best_pv |
|---|---|---|---|
| 50 | NZ_ ABEJ01000032 | Salmonella enterica subsp. enterica serovar Schwarzengrund str. SL480, unfinished sequence: NZ_ ABEJ01000032 | 8.71713912669417e-07 |

Table 18b: Data for Salmonella species (continued)

| No. | sign_phenos | sign_phenos_class | best_pheno | best_pheno_class |
|---|---|---|---|---|
| 1 | AM;A/S | lactam | AM | lactam |
| 2 | AM;A/S | lactam | AM | lactam |
| 3 | AM;A/S | lactam | AM | lactam |
| 4 | AM;A/S | lactam | A/S | lactam |
| 5 | AM;A/S | lactam | AM | lactam |
| 6 | AM;A/S | lactam | A/S | lactam |
| 7 | AM;A/S | lactam | AM | lactam |
| 8 | AM;A/S | lactam | A/S | lactam |
| 9 | AM;A/S | lactam | AM | lactam |
| 10 | AM;A/S | lactam | A/S | lactam |
| 11 | AM;A/S | lactam | A/S | lactam |
| 12 | AM;A/S | lactam | A/S | lactam |
| 13 | AM;A/S | lactam | AM | lactam |
| 14 | AM;A/S | lactam | A/S | lactam |
| 15 | AM;A/S | lactam | A/S | lactam |
| 16 | AM;A/S | lactam | A/S | lactam |
| 17 | AM;A/S | lactam | A/S | lactam |
| 18 | AM;A/S | lactam | A/S | lactam |
| 19 | AM;A/S | lactam | A/S | lactam |
| 20 | AM;A/S | lactam | AM | lactam |
| 21 | AM;A/S | lactam | AM | lactam |
| 22 | AM;A/S | lactam | AM | lactam |
| 23 | AM;A/S | lactam | A/S | lactam |
| 24 | AM;A/S | lactam | A/S | lactam |
| 25 | AM;A/S | lactam | A/S | lactam |
| 26 | AM;A/S | lactam | AM | lactam |
| 27 | AM;A/S | lactam | AM | lactam |
| 28 | AM;A/S | lactam | AM | lactam |
| 29 | AM;A/S | lactam | AM | lactam |
| 30 | AM;A/S | lactam | AM | lactam |
| 31 | AM;A/S | lactam | AM | lactam |

(continued)

| No. | sign_phenos | sign_phenos_class | best_pheno | best_pheno_class |
|---|---|---|---|---|
| 32 | AM;A/S | lactam | AM | lactam |
| 33 | AM;A/S | lactam | AM | lactam |
| 34 | AM;A/S | lactam | AM | lactam |
| 35 | AM;A/S | lactam | AM | lactam |
| 36 | AM;A/S | lactam | AM | lactam |
| 37 | AM;A/S | lactam | A/S | lactam |
| 38 | AM;A/S | lactam | AM | lactam |
| 39 | AM;A/S | lactam | AM | lactam |
| 40 | AM;A/S | lactam | AM | lactam |
| 41 | AM;A/S | lactam | AM | lactam |
| 42 | AM;A/S | lactam | A/S | lactam |
| 43 | AM;A/S | lactam | AM | lactam |
| 44 | AM;A/S | lactam | A/S | lactam |
| 45 | AM;A/S | lactam | A/S | lactam |
| 46 | AM;A/S | lactam | A/S | lactam |
| 47 | AM;A/S | lactam | A/S | lactam |
| 48 | AM;A/S | lactam | A/S | lactam |
| 49 | AM;A/S | lactam | A/S | lactam |
| 50 | AM;A/S | lactam | A/S | lactam |

Table 18c: Data for Salmonella species (continued)

| No. | num_lactam | AM_pv_adj | A/S_pv_adj |
|---|---|---|---|
| 1 | 2 | 1.10E-59 | 1.70E-55 |
| 2 | 2 | 3.48E-42 | 1.81E-40 |
| 3 | 2 | 2.51E-24 | 2.30E-22 |
| 4 | 2 | 1.08E-21 | 3.65E-22 |
| 5 | 2 | 1.44E-21 | 6.88E-20 |
| 6 | 2 | 1.05E-20 | 3.48E-21 |
| 7 | 2 | 3.48E-21 | 1.05E-20 |
| 8 | 2 | 5.22E-20 | 1.75E-20 |
| 9 | 2 | 3.31E-20 | 1.15E-19 |
| 10 | 2 | 1.65E-19 | 5.49E-20 |
| 11 | 2 | 1.74E-16 | 7.42E-17 |
| 12 | 2 | 2.98E-16 | 8.87E-17 |
| 13 | 2 | 5.29E-14 | 1.45E-13 |
| 14 | 2 | 1.45E-13 | 5.90E-14 |
| 15 | 2 | 3.62E-13 | 1.65E-13 |

(continued)

| No. | num_lactam | AM_pv_adj | A/S_pv_adj |
|-----|-----------|-----------|------------|
| 16 | 2 | 3.62E-13 | 1.65E-13 |
| 17 | 2 | 6.38E-13 | 3.61E-13 |
| 18 | 2 | 1.10E-12 | 5.19E-13 |
| 19 | 2 | 6.91E-12 | 3.38E-12 |
| 20 | 2 | 5.33E-12 | 3.21E-11 |
| 21 | 2 | 9.59E-12 | 8.71E-10 |
| 22 | 2 | 1.80E-11 | 6.37E-10 |
| 23 | 2 | 3.64E-11 | 2.03E-11 |
| 24 | 2 | 3.64E-11 | 2.03E-11 |
| 25 | 2 | 6.67E-11 | 3.63E-11 |
| 26 | 2 | 5.50E-11 | 4.78E-09 |
| 27 | 2 | 1.68E-10 | 1.06E-09 |
| 28 | 2 | 8.58E-10 | 2.16E-08 |
| 29 | 2 | 1.06E-09 | 2.10E-08 |
| 30 | 2 | 2.23E-09 | 3.77E-08 |
| 31 | 2 | 2.23E-09 | 3.77E-08 |
| 32 | 2 | 1.72E-08 | 2.02E-07 |
| 33 | 2 | 1.82E-08 | 1.76E-06 |
| 34 | 2 | 3.52E-08 | 8.59E-08 |
| 35 | 2 | 4.21E-08 | 6.33E-08 |
| 36 | 2 | 5.50E-08 | 7.64E-08 |
| 37 | 2 | 1.40E-07 | 9.94E-08 |
| 38 | 2 | 1.81E-07 | 3.88E-07 |
| 39 | 2 | 1.97E-07 | 2.71E-07 |
| 40 | 2 | 2.16E-07 | 2.91E-07 |
| 41 | 2 | 2.16E-07 | 7.75E-07 |
| 42 | 2 | 7.51E-07 | 2.30E-07 |
| 43 | 2 | 3.06E-07 | 8.87E-07 |
| 44 | 2 | 2.22E-06 | 3.49E-07 |
| 45 | 2 | 1.28E-06 | 4.35E-07 |
| 46 | 2 | 1.28E-06 | 4.35E-07 |
| 47 | 2 | 1.25E-06 | 4.35E-07 |
| 48 | 2 | 2.83E-06 | 7.62E-07 |
| 49 | 2 | 1.23E-06 | 8.72E-07 |
| 50 | 2 | 4.30E-06 | 8.72E-07 |

Table 18d: Data for Salmonella species (continued)

| No. | Locus.Tag | Gene.Symbol | GenBank.Accession | Chromosome |
|---|---|---|---|---|
| 1 | SeHA_C1580 | blaT | YP_002045448 | |
| 2 | SeSA_B0079 | | YP_002112958 | plasmid pCVM19633_110 |
| 3 | HMPREF9547_ 03104 | | ZP_07169558 | |
| 4 | KPN_pKPN5p08201 | | YP_001338811 | plasmid pKPN5 |
| 5 | ETAE_p041 | folP | YP_003297635 | plasmid pEIB202 |
| 6 | Salmoentericaenterica_010100000245 | | ZP_02702077 | |
| 7 | pEFER_0049 | | YP_002394596 | plasmid pEFER |
| 8 | HMPREF9345_ 05066 | | ZP_07100147 | |
| 9 | ETAE_p040 | | YP_003297634 | plasmid pEIB202 |
| 10 | EcolB_01004576 | | ZP_00708527 | |
| 11 | HMPREF9538_04737 | | ZP_08307036 | |
| 12 | HMPREF9548_ 03784 | | ZP_07141588 | |
| 13 | SFxv_1142 | | ADA73400 | |
| 14 | SSON_3896 | aadA | YP_312670 | |
| 15 | HMPREF9551_04611 | | ZP_07191964 | |
| 16 | Salmoentericaenterica_010100000130 | | ZP_02702054 | |
| 17 | KPN_pKPN4p07064 | | YP_001338673 | plasmid pKPN4 |
| 18 | Salmoentericaenterica_010100000105 | | ZP_02702049 | |
| 19 | HMPREF9345_ 03525 | | ZP_07098656 | |
| 20 | HMPREF9347_06051 | | ZP_07213485 | |
| 21 | SFxv_1141 | | ADA73399 | |
| 22 | SeHA_A0031 | | YP_002043869 | plasmid pSL476_91 |
| 23 | STMDT12_C39390 | | BAJ38882 | |
| 24 | KPN_pKPN4p07068 | merC | YP_001338677 | plasmid pKPN4 |
| 25 | SeSA_B0082 | | YP_002112961 | plasmid pCVM19633_110 |
| 26 | ESCAB7627_ 0644 | | ZP_02900823 | |
| 27 | STM474_p303 | repA | ADX20580 | plasmid TY474p3 |
| 28 | SPC_p013 | rsdB | YP_002635587 | plasmid pSPCV |
| 29 | SSO_P190 | traD | YP_313445 | plasmid pSS_046 |
| 30 | CP0245 | mvpA | NP_858378 | plasmid pCP301 |
| 31 | PSLT105 | trbH | NP_490589 | plasmid pSLT |
| 32 | STM14_5622 | traD | ACY86531 | |
| 33 | ESCAB7627_ 0650 | | ZP_02900555 | |
| 34 | SPC_p042 | traX | YP_002635616 | plasmid pSPCV |
| 35 | SPC_2707 | | YP_002638248 | |
| 36 | STM474_1038 | | ADX16744 | |

(continued)

| No. | Locus.Tag | Gene.Symbol | GenBank.Accession | Chromosome |
|---|---|---|---|---|
| 37 | Senterienterica_010100019467 | | ZP_02668762 | |
| 38 | SPC_p041 | finO | YP_002635615 | plasmid pSPCV |
| 39 | SPC_2708 | | YP_002638249 | |
| 40 | STM1046 | | NP_460021 | |
| 41 | STM474_p1093 | ccdB | ADX20463 | plasmid TY474p1 |
| 42 | Sentent_010100022581 | | ZP_02341659 | |
| 43 | SPAB_05276 | | YP_001591385 | |
| 44 | STMDT12_C10780 | | BAJ36021 | |
| 45 | SARI_01515 | | YP_001570553 | |
| 46 | SARI_01514 | | YP_001570552 | |
| 47 | SBG_1292 | malR | YP_004730162 | |
| 48 | Senterienterica_010100019192 | | ZP_02668707 | |
| 49 | SPC_0340 | | YP_002635966 | |
| 50 | Senteenterica_010100025269 | | ZP_02664569 | |

Table 18e: Data for Salmonella species (continued)

| No. | Start.Coord | End.Coord | Strand | Scaffold.ID |
|---|---|---|---|---|
| 1 | 1525960 | 1526820 | + | 642555218 |
| 2 | 68933 | 70240 | + | 642555219 |
| 3 | 3248 | 3841 | + | 648291919 |
| 4 | 34908 | 35255 | - | 640753021 |
| 5 | 32101 | 32916 | - | 646311959 |
| 6 | 36757 | 37062 | + | 641778223 |
| 7 | 48658 | 49503 | + | 643692014 |
| 8 | 4715 | 5554 | - | 648294624 |
| 9 | 31237 | 32040 | - | 646311959 |
| 10 | 3555 | 4568 | + | 638359041 |
| 11 | 1555 | 2055 | - | 651333542 |
| 12 | 8819 | 9535 | + | 648288593 |
| 13 | 1108351 | 1108854 | - | 646862363 |
| 14 | 4110458 | 4111246 | - | 640427102 |
| 15 | 97 | 531 | - | 648293053 |
| 16 | 19257 | 19691 | + | 641778223 |
| 17 | 23310 | 24614 | - | 640753020 |
| 18 | 16271 | 16636 | - | 641778223 |
| 19 | 1071 | 1307 | - | 648294596 |
| 20 | 812 | 1663 | - | 648294425 |

(continued)

| No. | Start.Coord | End.Coord | Strand | Scaffold.ID |
|-----|-------------|-----------|--------|-------------|
| 21 | 1107342 | 1107968 | + | 646862363 |
| 22 | 16416 | 17195 | + | 642555216 |
| 23 | 4088051 | 4088326 | - | 651053007 |
| 24 | 25970 | 27664 | - | 640753020 |
| 25 | 71061 | 71411 | - | 642555219 |
| 26 | 233492 | 234697 | - | 641784575 |
| 27 | 1988 | 2827 | - | 650378006 |
| 28 | 10060 | 10842 | - | 643692029 |
| 29 | 166425 | 168626 | + | 640427103 |
| 30 | 198117 | 198515 | - | 637000224 |
| 31 | 86566 | 87285 | + | 637000134 |
| 32 | 84297 | 86459 | + | 646862361 |
| 33 | 238788 | 239996 | - | 641784575 |
| 34 | 28413 | 29153 | - | 643692029 |
| 35 | 2745099 | 2745596 | - | 643692030 |
| 36 | 1088568 | 1089272 | + | 650378003 |
| 37 | 62341 | 62874 | - | 641777794 |
| 38 | 27795 | 28358 | - | 643692029 |
| 39 | 2745695 | 2746027 | - | 643692030 |
| 40 | 1131381 | 1132118 | + | 637000122 |
| 41 | 73221 | 73526 | - | 650378004 |
| 42 | 23575 | 23910 | - | 641746440 |
| 43 | 4445787 | 4448009 | + | 641228523 |
| 44 | 1141208 | 1141399 | - | 651053007 |
| 45 | 1476854 | 1478026 | + | 641228515 |
| 46 | 1475252 | 1476844 | + | 641228515 |
| 47 | 1414087 | 1415115 | + | 650716177 |
| 48 | 26832 | 28046 | + | 641777794 |
| 49 | 367421 | 368839 | + | 643692030 |
| 50 | 35858 | 36535 | - | 641777693 |

Example 10: Serratia, particularly Serratia marcescens

The procedure was carried out as in Example 1, except that the following microorganisms were used:

Bacterial Strains

The inventors selected 437 Serratia strains from the microbiology strain collection at Siemens Healthcare Diagnostics (West Sacramento, CA) for susceptibility testing and whole genome sequencing.
From MetaRef, 1671 centroids of Serratia species were used as reference sequences.
The results for Serratia are shown in Tables 19 (corresponding to Table 1) and 20 (corresponding to Table 2).

Table 19a: Data for Serratia, particularly Serratia marcescens

| No. | Scaffold.External. Accession | Scaffold.Name | best_pv |
|---|---|---|---|
| 1 | NC_009832 | Serratia proteamaculans 568: NC_009832 | 3.70422109249874e-06 |
| 2 | NZ_ADBY01000058 | Serratia odorifera DSM 4582 contig00061: NZ_ADBY01000058 | 6.00624466630552e-06 |
| 3 | NZ_ADBY01000056 | Serratia odorifera DSM 4582 contig00059: NZ_ADBY01000056 | 6.00624466630552e-06 |
| 4 | NZ_ADBY01000031 | Serratia odorifera DSM 4582 contig00034: NZ_ADBY01000031 | 8.61505367610747e-06 |
| 5 | NZ_GL636422 | Serratia symbiotica str. Tucson genomic scaffold contig09350: NZ_GL636422 | 8.61505367610747e-06 |
| 6 | NZ_ADBX01000013 | Serratia odorifera 4Rx13 SODm: NZ_ADBX01000013 | 8.61505367610747e-06 |
| 7 | NZ_ADBX01000004 | Serratia odorifera 4Rx13 SODd: NZ_ADBX01000004 | 1.33354783010227e-05 |
| 8 | NC_009832 | Serratia proteamaculans 568: NC_009832 | 1.37889112422256e-05 |
| 9 | NZ_ADBX01000010 | Serratia odorifera 4Rx13 SODj: NZ_ADBX01000010 | 1.55998636550055e-05 |
| 10 | NZ_ADBX01000013 | Serratia odorifera 4Rx13 SODm: NZ_ADBX01000013 | 1.55998636550055e-05 |
| 11 | NZ_GL636105 | Serratia symbiotica str. Tucson genomic scaffold scaffold00209: NZ_GL636105 | 1.55998636550055e-05 |
| 12 | NC_014837 | Pantoea sp. At-9b chromosome: NC_014837 | 1.55998636550055e-05 |
| 13 | NZ_GL636364 | Serratia symbiotica str. Tucson genomic scaffold contig09039: NZ_GL636364 | 1.55998636550055e-05 |
| 14 | NC_009832 | Serratia proteamaculans 568: NC_009832 | 1.55998636550055e-05 |
| 15 | NZ_ADBY01000048 | Serratia odorifera DSM 4582 contig00051: NZ_ADBY01000048 | 2.17438866604963e-05 |
| 16 | NZ_ADBX01000002 | Serratia odorifera 4Rx13 SODb: NZ_ADBX01000002 | 2.94809945975299e-05 |
| 17 | NZ_ADBY01000031 | Serratia odorifera DSM 4582 contig00034: NZ_ADBY01000031 | 3.13750499443641e-05 |
| 18 | NZ_ADBY01000045 | Serratia odorifera DSM 4582 contig00048: NZ_ADBY01000045 | 3.13750499443641e-05 |
| 19 | NZ_ADBY01000031 | Serratia odorifera DSM 4582 contig00034: NZ_ADBY01000031 | 3.13750499443641e-05 |
| 20 | NZ_ADBY01000058 | Serratia odorifera DSM 4582 contig00061: NZ_ADBY01000058 | 3.13750499443641e-05 |
| 21 | NZ_ADBX01000002 | Serratia odorifera 4Rx13 SODb: NZ_ADBX01000002 | 3.43966250408489e-05 |
| 22 | NZ_ADBY01000050 | Serratia odorifera DSM 4582 contig00053: NZ_ADBY01000050 | 3.66332257629745e-05 |
| 23 | NZ_ADBY01000013 | Serratia odorifera DSM 4582 contig00016: NZ_ADBY01000013 | 3.7147650177074e-05 |

(continued)

| No. | Scaffold.External. Accession | Scaffold.Name | best_pv |
|---|---|---|---|
| 24 | NZ_ADBY01000056 | Serratia odorifera DSM 4582 contig00059: NZ_ADBY01000056 | 3.7147650177074e-05 |
| 25 | NZ_ADBY01000026 | Serratia odorifera DSM 4582 contig00029: NZ_ADBY01000026 | 3.7147650177074e-05 |
| 26 | NZ_ADBX01000001 | Serratia odorifera 4Rx13 SODa: NZ_ADBX01000001 | 3.77977323028027e-05 |
| 27 | NZ_ADBY01000057 | Serratia odorifera DSM 4582 contig00060: NZ_ADBY01000057 | 4.06693999812307e-05 |
| 28 | NC_009832 | Serratia proteamaculans 568: NC_009832 | 4.06693999812307e-05 |
| 29 | NZ_ADBY01000057 | Serratia odorifera DSM 4582 contig00060: NZ_ADBY01000057 | 4.06693999812307e-05 |
| 30 | NZ_ADBX01000013 | Serratia odorifera 4Rx13 SODm: NZ_ADBX01000013 | 4.06693999812307e-05 |
| 31 | NZ_ADBY01000031 | Serratia odorifera DSM 4582 contig00034: NZ_ADBY01000031 | 4.20000578456372e-05 |
| 32 | NZ_ADBX01000002 | Serratia odorifera 4Rx13 SODb: NZ_ADBX01000002 | 5.02288669357547e-05 |
| 33 | NZ_ADBX01000002 | Serratia odorifera 4Rx13 SODb: NZ_ADBX01000002 | 5.02288669357547e-05 |
| 34 | NZ_ADBX01000003 | Serratia odorifera 4Rx13 SODc: NZ_ADBX01000003 | 5.02288669357547e-05 |
| 35 | NC_009832 | Serratia proteamaculans 568: NC_009832 | 5.02288669357547e-05 |
| 36 | NC_009832 | Serratia proteamaculans 568: NC_009832 | 5.02288669357547e-05 |
| 37 | NC_009832 | Serratia proteamaculans 568: NC_009832 | 5.02288669357547e-05 |
| 38 | NZ_ADBX01000004 | Serratia odorifera 4Rx13 SODd: NZ_ADBX01000004 | 5.02288669357547e-05 |
| 39 | NZ_GG657374 | Citrobacter sp. 30_2 genomic scaffold supercont1.9: NZ_GG657374 | 5.02288669357547e-05 |
| 40 | NC_009832 | Serratia proteamaculans 568: NC_009832 | 5.02288669357547e-05 |
| 41 | NC_009832 | Serratia proteamaculans 568: NC_009832 | 5.02288669357547e-05 |
| 42 | NC_009832 | Serratia proteamaculans 568: NC_009832 | 5.02288669357547e-05 |
| 43 | NZ_ADBX01000001 | Serratia odorifera 4Rx13 SODa: NZ_ADBX01000001 | 5.02288669357547e-05 |
| 44 | NZ_ADBX01000004 | Serratia odorifera 4Rx13 SODd: NZ_ADBX01000004 | 5.02288669357547e-05 |
| 45 | NZ_ADBX01000002 | Serratia odorifera 4Rx13 SODb: NZ_ADBX01000002 | 5.02288669357547e-05 |
| 46 | NZ_ADBY01000056 | Serratia odorifera DSM 4582 contig00059: NZ_ADBY01000056 | 5.02288669357547e-05 |
| 47 | NZ_ADBY01000032 | Serratia odorifera DSM 4582 contig00035: NZ_ADBY01000032 | 5.02288669357547e-05 |
| 48 | NZ_ADBY01000013 | Serratia odorifera DSM 4582 contig00016: NZ_ADBY01000013 | 5.88044402302405e-05 |

(continued)

| No. | Scaffold.External. Accession | Scaffold.Name | best_pv |
|---|---|---|---|
| 49 | NC_009832 | Serratia proteamaculans 568: NC_009832 | 6.62251962114689e-05 |
| 50 | NC_009832 | Serratia proteamaculans 568: NC_009832 | 7.15986854143193e-05 |

Table 19b: Data for Serratia, particularly Serratia marcescens (continued)

| No. | sign_phenos | sign_phenos_class | best_pheno | best_pheno_class |
|---|---|---|---|---|
| 1 | AZT;CAX;CAZ;CFT;CP;GM;LVX;P/T;TO;T/S | aminoglycoside;fluoroquinolone;lactam;other | LVX | fluoroquinolone |
| 2 | CP;LVX;T/S | fluoroquinolone;other | CP | fluoroquinolone |
| 3 | AZT;CAX;CP;LVX;T/S | fluoroquinolone;lactam;other | LVX | fluoroquinolone |
| 4 | AZT;CAX;CFT;CP;LVX;P/T;T/S | fluoroquinolone;lactam;other | LVX | fluoroquinolone |
| 5 | CAX;CAZ;CFT;CP;LVX;P/T | fluoroquinolone;lactam | CP | fluoroquinolone |
| 6 | AZT;CAX;CAZ;CFT;CP;LVX;P/T | fluoroquinolone;lactam | CP | fluoroquinolone |
| 7 | CAX;CFT;CP;LVX | fluoroquinolone;lactam | LVX | fluoroquinolone |
| 8 | CAX;CFT;CP;LVX;T/S | fluoroquinolone;lactam;other | LVX | fluoroquinolone |
| 9 | CAX;CFT;CP;LVX;TO | aminoglycoside;fluoroquinolone;lactam | LVX | fluoroquinolone |
| 10 | AZT;CAX;CAZ;CFT;CP;LVX;P/T | fluoroquinolone;lactam | CP | fluoroquinolone |
| 11 | AZT;CAX;CAZ;CFT;CP;P/T | fluoroquinolone;lactam | CAX | lactam |
| 12 | CAX;CFT;CP;GM;LVX;P/T;TO;T/S | aminoglycoside;fluoroquinolone;lactam;other | LVX | fluoroquinolone |
| 13 | AZT;CAX;CAZ;CFT;CP;P/T | fluoroquinolone;lactam | CAX | lactam |
| 14 | CP;LVX | fluoroquinolone | LVX | fluoroquinolone |
| 15 | AZT;CAX;CFT;CP;GM;LVX;P/T;TO;T/S | aminoglycoside;fluoroquinolone;lactam;other | LVX | fluoroquinolone |
| 16 | AZT;CAX;CFT;CP;GM;LVX;P/T;TO;T/S | aminoglycoside;fluoroquinolone;lactam;other | LVX | fluoroquinolone |
| 17 | AZT;CAX;CFT;CP;LVX;P/T;T/S | fluoroquinolone;lactam;other | LVX | fluoroquinolone |
| 18 | CAX;CFT;CP;GM;LVX;P/T;TO;T/S | aminoglycoside;fluoroquinolone;lactam;other | LVX | fluoroquinolone |
| 19 | AZT;CAX;CFT;CP;LVX;P/T;T/S | fluoroquinolone;lactam;other | LVX | fluoroquinolone |
| 20 | AZT;CAX;CFT;CP;GM;LVX;P/T;TO;T/S | aminoglycoside;fluoroquinolone;lactam;other | LVX | fluoroquinolone |
| 21 | CAX;CP;LVX;T/S | fluoroquinolone;lactam;other | LVX | fluoroquinolone |
| 22 | AZT;CAX;CFT;CP;GM;LVX;P/T;TO;T/S | aminoglycoside;fluoroquinolone;lactam;other | LVX | fluoroquinolone |
| 23 | CAX;CFT;CP;GM;LVX;P/T;TO;T/S | aminoglycoside;fluoroquinolone;lactam;other | CP | fluoroquinolone |
| 24 | AZT;CAX;CAZ;CFT;P/T | lactam | CAZ | lactam |

(continued)

| No. | sign_phenos | sign_phenos_class | best_pheno | best_pheno_class |
|---|---|---|---|---|
| 25 | CAX;CFT;CP;GM;LVX;P/T | aminoglycoside;fluoroquinolone;lactam | LVX | fluoroquinolone |
| 26 | AZT;CAX;CFT;CP;GM;LVX;P/T;TO;T/S | aminoglycoside;fluoroquinolone;lactam;other | LVX | fluoroquinolone |
| 27 | AZT;CAZ;CFT;CP;TO | aminoglycoside;fluoroquinolone;lactam | CP | fluoroquinolone |
| 28 | CAX;CP;LVX | fluoroquinolone;lactam | LVX | fluoroquinolone |
| 29 | AZT;CAZ;CFT;CP;TO | aminoglycoside;fluoroquinolone;lactam | CP | fluoroquinolone |
| 30 | AZT;CAZ;CFT;CP;TO | aminoglycoside;fluoroquinolone;lactam | CP | fluoroquinolone |
| 31 | CAX;CFT;CP;LVX;P/T;T/S | fluoroquinolone;lactam;other | P/T | lactam |
| 32 | AZT;CAX;CFT;CP;GM;LVX;P/T;TO;T/S | aminoglycoside;fluoroquinolone;lactam;other | LVX | fluoroquinolone |
| 33 | CAX;CFT;CP;LVX;T/S | fluoroquinolone;lactam;other | LVX | fluoroquinolone |
| 34 | CAX;CFT;CP;LVX;T/S | fluoroquinolone;lactam;other | LVX | fluoroquinolone |
| 35 | AZT;CAX;CFT;CP;GM;LVX;P/T;TO;T/S | aminoglycoside;fluoroquinolone;lactam;other | LVX | fluoroquinolone |
| 36 | AZT;CAX;CFT;CP;LVX;T/S | fluoroquinolone;lactam;other | CP | fluoroquinolone |
| 37 | AZT;CAX;CFT;CP;GM;LVX;P/T;TO;T/S | aminoglycoside;fluoroquinolone;lactam;other | LVX | fluoroquinolone |
| 38 | CAX;CFT;CP;LVX;P/T;T/S | fluoroquinolone;lactam;other | LVX | fluoroquinolone |
| 39 | CP;GM;LVX;TO;T/S | aminoglycoside;fluoroquinolone;other | LVX | fluoroquinolone |
| 40 | CAX;CFT;CP;LVX;P/T;T/S | fluoroquinolone;lactam;other | LVX | fluoroquinolone |
| 41 | AZT;CAX;CFT;CP;GM;LVX;P/T;TO;T/S | aminoglycoside;fluoroquinolone;lactam;other | LVX | fluoroquinolone |
| 42 | AZT;CAX;CFT;CP;GM;LVX;P/T;TO;T/S | aminoglycoside;fluoroquinolone;lactam;other | LVX | fluoroquinolone |
| 43 | CP;LVX;P/T | fluoroquinolone;lactam | LVX | fluoroquinolone |
| 44 | CAX;CFT;CP;LVX;T/S | fluoroquinolone;lactam;other | LVX | fluoroquinolone |
| 45 | AZT;CAX;CFT;CP;GM;LVX;P/T;TO;T/S | aminoglycoside;fluoroquinolone;lactam;other | LVX | fluoroquinolone |
| 46 | AZT;CAX;CAZ | lactam | CAZ | lactam |
| 47 | AZT;CAX;CFT;CP;GM;LVX;TO;T/S | aminoglycoside;fluoroquinolone;lactam;other | LVX | fluoroquinolone |
| 48 | CAX;CFT;CP;LVX;P/T | fluoroquinolone;lactam | LVX | fluoroquinolone |
| 49 | CAX;CFT;CP;LVX | fluoroquinolone;lactam | LVX | fluoroquinolone |
| 50 | AZT;CAX;CFT;CP;GM;LVX;P/T;TO;T/S | aminoglycoside;fluoroquinolone;lactam;other | LVX | fluoroquinolone |

Table 19c: Data for Serratia, particularly Serratia marcescens (continued)

| No. | num_aminoglycoside | num_fluoroquinolone | num_lactam | num_other |
|---|---|---|---|---|
| 1 | 2 | 2 | 5 | 1 |
| 2 | 0 | 2 | 0 | 1 |
| 3 | 0 | 2 | 2 | 1 |
| 4 | 0 | 2 | 4 | 1 |
| 5 | 0 | 2 | 4 | 0 |
| 6 | 0 | 2 | 5 | 0 |
| 7 | 0 | 2 | 2 | 0 |
| 8 | 0 | 2 | 2 | 1 |
| 9 | 1 | 2 | 2 | 0 |
| 10 | 0 | 2 | 5 | 0 |
| 11 | 0 | 1 | 5 | 0 |
| 12 | 2 | 2 | 3 | 1 |
| 13 | 0 | 1 | 5 | 0 |
| 14 | 0 | 2 | 0 | 0 |
| 15 | 2 | 2 | 4 | 1 |
| 16 | 2 | 2 | 4 | 1 |
| 17 | 0 | 2 | 4 | 1 |
| 18 | 2 | 2 | 3 | 1 |
| 19 | 0 | 2 | 4 | 1 |
| 20 | 2 | 2 | 4 | 1 |
| 21 | 0 | 2 | 1 | 1 |
| 22 | 2 | 2 | 4 | 1 |
| 23 | 2 | 2 | 3 | 1 |
| 24 | 0 | 0 | 5 | 0 |
| 25 | 1 | 2 | 3 | 0 |
| 26 | 2 | 2 | 4 | 1 |
| 27 | 1 | 1 | 3 | 0 |
| 28 | 0 | 2 | 1 | 0 |
| 29 | 1 | 1 | 3 | 0 |
| 30 | 1 | 1 | 3 | 0 |
| 31 | 0 | 2 | 3 | 1 |
| 32 | 2 | 2 | 4 | 1 |
| 33 | 0 | 2 | 2 | 1 |
| 34 | 0 | 2 | 2 | 1 |
| 35 | 2 | 2 | 4 | 1 |
| 36 | 0 | 2 | 3 | 1 |
| 37 | 2 | 2 | 4 | 1 |
| 38 | 0 | 2 | 3 | 1 |

(continued)

| No. | num_aminoglycoside | num_fluoroquinolone | num_lactam | num_other |
|-----|--------------------|--------------------|------------|-----------|
| 39 | 2 | 2 | 0 | 1 |
| 40 | 0 | 2 | 3 | 1 |
| 41 | 2 | 2 | 4 | 1 |
| 42 | 2 | 2 | 4 | 1 |
| 43 | 0 | 2 | 1 | 0 |
| 44 | 0 | 2 | 2 | 1 |
| 45 | 2 | 2 | 4 | 1 |
| 46 | 0 | 0 | 3 | 0 |
| 47 | 2 | 2 | 3 | 1 |
| 48 | 0 | 2 | 3 | 0 |
| 49 | 0 | 2 | 2 | 0 |
| 50 | 2 | 2 | 4 | 1 |

Table 19d: Data for Serratia, particularly Serratia marcescens (continued)

| No. | AZT_pv_adj | CAX_pv_adj | CAZ_pv_adj | CFT_pv_adj | CP_pv_adj |
|-----|-----------|-----------|-----------|-----------|-----------|
| 1 | 4.82E-04 | 5.24E-04 | 6.41E-03 | 7.29E-04 | 2.27E-05 |
| 2 | 1.58E-01 | 1.39E-01 | 2.26E-01 | 3.68E-02 | 6.01E-06 |
| 3 | 6.77E-04 | 8.30E-03 | 2.51E-02 | 1.65E-02 | 2.17E-05 |
| 4 | 8.92E-03 | 3.01E-03 | 3.16E-01 | 7.90E-03 | 2.95E-05 |
| 5 | 1.60E-02 | 4.77E-04 | 5.25E-03 | 1.36E-04 | 8.62E-06 |
| 6 | 1.22E-03 | 1.22E-03 | 1.24E-03 | 4.08E-04 | 8.62E-06 |
| 7 | 3.82E-02 | 3.55E-03 | 4.51E-02 | 2.17E-03 | 7.86E-05 |
| 8 | 1.48E-01 | 5.09E-03 | 6.93E-02 | 4.93E-03 | 1.10E-04 |
| 9 | 4.53E-02 | 5.02E-03 | 3.12E-01 | 2.64E-03 | 5.13E-04 |
| 10 | 6.43E-04 | 1.61E-04 | 7.67E-04 | 8.45E-05 | 1.56E-05 |
| 11 | 3.67E-05 | 1.56E-05 | 1.56E-05 | 5.02E-05 | 6.54E-03 |
| 12 | 1.44E-02 | 6.36E-03 | 4.83E-02 | 3.41E-03 | 2.36E-04 |
| 13 | 3.67E-05 | 1.56E-05 | 1.56E-05 | 5.02E-05 | 6.54E-03 |
| 14 | 4.07E-01 | 5.36E-02 | 7.62E-01 | 4.93E-02 | 5.86E-04 |
| 15 | 1.11E-03 | 7.67E-04 | 4.28E-02 | 1.04E-03 | 4.94E-05 |
| 16 | 6.43E-04 | 1.24E-03 | 2.38E-02 | 2.68E-03 | 1.82E-04 |
| 17 | 4.26E-03 | 1.84E-03 | 6.64E-02 | 4.93E-03 | 1.22E-04 |
| 18 | 1.73E-02 | 1.76E-04 | 2.04E-02 | 5.24E-04 | 6.30E-04 |
| 19 | 4.26E-03 | 1.84E-03 | 6.64E-02 | 4.93E-03 | 1.22E-04 |
| 20 | 9.20E-04 | 1.82E-04 | 2.34E-02 | 1.48E-04 | 1.69E-04 |
| 21 | 2.19E-02 | 6.70E-03 | 1 | 2.46E-02 | 7.82E-04 |
| 22 | 1.55E-03 | 4.30E-04 | 6.08E-02 | 5.90E-04 | 1.52E-04 |

(continued)

| No. | AZT_pv_adj | CAX_pv_adj | CAZ_pv_adj | CFT_pv_adj | CP_pv_adj |
|-----|-----------|-----------|-----------|-----------|-----------|
| 23 | 4.85E-02 | 7.20E-04 | 1.01E-01 | 6.90E-04 | 3.71E-05 |
| 24 | 2.28E-03 | 2.41E-03 | 3.71E-05 | 4.23E-03 | 1.66E-01 |
| 25 | 1.01E-02 | 3.27E-04 | 5.44E-02 | 4.95E-04 | 7.62E-04 |
| 26 | 8.18E-04 | 3.20E-04 | 4.45E-02 | 3.27E-04 | 1.53E-04 |
| 27 | 5.89E-04 | 1.07E-02 | 1.19E-03 | 3.35E-03 | 4.07E-05 |
| 28 | 2.82E-02 | 6.08E-03 | 2.88E-01 | 4.51E-02 | 1.10E-04 |
| 29 | 5.89E-04 | 1.07E-02 | 1.19E-03 | 3.35E-03 | 4.07E-05 |
| 30 | 5.89E-04 | 1.07E-02 | 1.19E-03 | 3.35E-03 | 4.07E-05 |
| 31 | 5.92E-02 | 3.15E-04 | 6.50E-01 | 1.11E-03 | 6.89E-03 |
| 32 | 1.66E-03 | 6.30E-04 | 8.28E-02 | 6.43E-04 | 3.33E-04 |
| 33 | 5.14E-02 | 1.60E-03 | 5.77E-02 | 2.64E-03 | 1.17E-03 |
| 34 | 5.14E-02 | 1.60E-03 | 5.77E-02 | 2.64E-03 | 1.17E-03 |
| 35 | 1.17E-03 | 3.15E-04 | 5.90E-02 | 3.27E-04 | 3.15E-04 |
| 36 | 1.07E-03 | 3.99E-03 | 2.05E-02 | 2.84E-03 | 5.02E-05 |
| 37 | 1.16E-03 | 5.89E-04 | 5.88E-02 | 6.07E-04 | 2.32E-04 |
| 38 | 4.11E-02 | 1.55E-03 | 5.76E-02 | 2.12E-03 | 1.15E-03 |
| 39 | 2.08E-02 | 2.04E-02 | 2.72E-01 | 3.09E-02 | 3.33E-04 |
| 40 | 5.14E-02 | 1.13E-03 | 5.80E-02 | 1.53E-03 | 1.50E-03 |
| 41 | 1.17E-03 | 3.15E-04 | 5.90E-02 | 3.27E-04 | 3.15E-04 |
| 42 | 4.30E-03 | 1.43E-03 | 1.74E-01 | 2.47E-03 | 9.38E-04 |
| 43 | 8.63E-02 | 1.24E-02 | 2.31E-01 | 1.12E-02 | 7.34E-04 |
| 44 | 5.14E-02 | 2.07E-03 | 5.80E-02 | 2.73E-03 | 1.50E-03 |
| 45 | 1.17E-03 | 3.15E-04 | 5.90E-02 | 3.27E-04 | 3.15E-04 |
| 46 | 2.22E-03 | 6.95E-03 | 5.02E-05 | 1.63E-02 | 4.21E-01 |
| 47 | 7.04E-03 | 6.21E-03 | 3.82E-02 | 3.31E-03 | 1.04E-04 |
| 48 | 3.16E-01 | 1.58E-03 | 5.58E-01 | 1.57E-03 | 7.78E-04 |
| 49 | 2.46E-02 | 8.70E-03 | 2.31E-01 | 5.07E-03 | 1.80E-03 |
| 50 | 1.55E-03 | 7.82E-04 | 5.97E-02 | 8.10E-04 | 3.15E-04 |

Table 19e: Data for Serratia, particularly Serratia marcescens (continued)

| No. | GM_pv_adj | LVX_pv_adj | P/T_pv_adj | TO_pv_adj | T/S_pv_adj |
|-----|-----------|-----------|-----------|-----------|-----------|
| 1 | 7.29E-04 | 3.70E-06 | 4.19E-03 | 5.65E-04 | 2.79E-04 |
| 2 | 3.92E-02 | 8.62E-06 | 5.32E-01 | 5.08E-02 | 9.68E-03 |
| 3 | 3.60E-02 | 6.01E-06 | 4.96E-02 | 9.86E-02 | 1.55E-03 |
| 4 | 2.38E-01 | 8.62E-06 | 4.51E-04 | 1.33E-01 | 5.10E-03 |
| 5 | 2.87E-01 | 4.05E-04 | 5.63E-05 | 3.66E-02 | 2.11E-02 |
| 6 | 9.01E-01 | 8.10E-04 | 6.41E-03 | 4.07E-02 | 1.63E-01 |

(continued)

| No. | GM_pv_adj | LVX_pv_adj | P/T_pv_adj | TO_pv_adj | T/S_pv_adj |
|---|---|---|---|---|---|
| 7 | 3.66E-02 | 1.33E-05 | 1.49E-02 | 1.15E-02 | 2.81E-02 |
| 8 | 1.16E-01 | 1.38E-05 | 1.31E-02 | 6.43E-02 | 5.52E-03 |
| 9 | 1.25E-02 | 1.56E-05 | 5.88E-02 | 1.37E-03 | 3.00E-02 |
| 10 | 2.58E-01 | 1.22E-04 | 9.05E-05 | 4.31E-02 | 3.97E-02 |
| 11 | 2.06E-01 | 3.60E-01 | 3.67E-05 | 1.87E-01 | 3.72E-01 |
| 12 | 5.33E-03 | 1.56E-05 | 6.87E-03 | 3.46E-03 | 8.20E-03 |
| 13 | 2.06E-01 | 3.60E-01 | 3.67E-05 | 1.87E-01 | 3.72E-01 |
| 14 | 4.95E-02 | 1.56E-05 | 1.34E-01 | 1.43E-01 | 2.07E-02 |
| 15 | 2.56E-03 | 2.17E-05 | 2.35E-03 | 3.96E-03 | 4.94E-04 |
| 16 | 3.89E-04 | 2.95E-05 | 1.52E-03 | 3.51E-03 | 2.43E-03 |
| 17 | 1.48E-01 | 3.14E-05 | 2.32E-04 | 8.78E-02 | 5.89E-04 |
| 18 | 3.24E-03 | 3.14E-05 | 2.41E-03 | 4.32E-03 | 4.63E-03 |
| 19 | 1.48E-01 | 3.14E-05 | 2.32E-04 | 8.78E-02 | 5.89E-04 |
| 20 | 2.78E-03 | 3.14E-05 | 1.10E-03 | 2.42E-03 | 6.75E-04 |
| 21 | 2.77E-02 | 3.44E-05 | 5.49E-02 | 1.12E-01 | 1.40E-03 |
| 22 | 5.63E-03 | 3.66E-05 | 3.16E-03 | 5.49E-03 | 3.24E-03 |
| 23 | 3.72E-03 | 4.07E-05 | 1.76E-03 | 4.82E-03 | 3.60E-03 |
| 24 | 2.22E-01 | 1 | 9.74E-03 | 1.18E-01 | 2.81E-01 |
| 25 | 4.82E-03 | 3.71E-05 | 7.82E-04 | 2.06E-02 | 1.08E-02 |
| 26 | 1.24E-03 | 3.78E-05 | 1.36E-03 | 2.34E-03 | 1.55E-03 |
| 27 | 6.23E-01 | 1.02E-02 | 5.43E-02 | 8.38E-03 | 8.82E-02 |
| 28 | 5.36E-02 | 4.07E-05 | 4.63E-02 | 3.92E-02 | 1.38E-02 |
| 29 | 6.23E-01 | 1.02E-02 | 5.43E-02 | 8.38E-03 | 8.82E-02 |
| 30 | 6.23E-01 | 1.02E-02 | 5.43E-02 | 8.38E-03 | 8.82E-02 |
| 31 | 1.41E-01 | 7.82E-04 | 4.20E-05 | 3.04E-02 | 5.10E-03 |
| 32 | 2.77E-03 | 5.02E-05 | 1.37E-03 | 5.69E-03 | 2.21E-03 |
| 33 | 1.47E-02 | 5.02E-05 | 1.03E-02 | 3.27E-02 | 6.75E-03 |
| 34 | 1.47E-02 | 5.02E-05 | 1.03E-02 | 3.27E-02 | 6.75E-03 |
| 35 | 1.76E-03 | 5.02E-05 | 1.07E-03 | 4.09E-03 | 2.26E-03 |
| 36 | 2.27E-02 | 3.89E-04 | 4.16E-02 | 1.11E-02 | 6.71E-03 |
| 37 | 1.76E-03 | 5.02E-05 | 1.80E-03 | 3.17E-03 | 2.24E-03 |
| 38 | 1.48E-02 | 5.02E-05 | 8.05E-03 | 2.57E-02 | 6.70E-03 |
| 39 | 2.77E-03 | 5.02E-05 | 1.42E-02 | 2.41E-03 | 2.10E-04 |
| 40 | 1.48E-02 | 5.02E-05 | 5.52E-03 | 3.27E-02 | 6.89E-03 |
| 41 | 1.76E-03 | 5.02E-05 | 1.07E-03 | 4.09E-03 | 2.26E-03 |
| 42 | 1.31E-03 | 5.02E-05 | 5.93E-03 | 3.06E-03 | 2.21E-03 |
| 43 | 3.13E-02 | 5.02E-05 | 5.52E-03 | 1.02E-02 | 9.79E-02 |
| 44 | 1.48E-02 | 5.02E-05 | 1.06E-02 | 3.27E-02 | 6.89E-03 |

(continued)

| No. | GM_pv_adj | LVX_pv_adj | P/T_pv_adj | TO_pv_adj | T/S_pv_adj |
|-----|-----------|------------|------------|-----------|------------|
| 45 | 1.76E-03 | 5.02E-05 | 1.07E-03 | 4.09E-03 | 2.26E-03 |
| 46 | 2.06E-01 | 1 | 7.15E-02 | 3.33E-01 | 1.57E-01 |
| 47 | 9.28E-04 | 5.02E-05 | 6.47E-02 | 2.47E-03 | 1.76E-03 |
| 48 | 1.83E-02 | 5.88E-05 | 1.62E-03 | 1.34E-02 | 1.37E-02 |
| 49 | 9.13E-01 | 6.62E-05 | 6.12E-02 | 1.22E-01 | 1.54E-01 |
| 50 | 1.80E-03 | 7.16E-05 | 2.42E-03 | 4.20E-03 | 2.34E-03 |

Table 19f: Data for Serratia, particularly Serratia marcescens (continued)

| No. | Locus.Tag | Gene. Symbol | GenBank.Accession | Chromosome |
|-----|-----------|--------------|-------------------|------------|
| 1 | Spro_0657 | | YP_001476891 | |
| 2 | HMPREF0758_ 4528 | | ZP_06641192 | |
| 3 | HMPREF0758_ 4282 | wrbA | ZP_06640946 | |
| 4 | HMPREF0758_ 1761 | | ZP_06638425 | |
| 5 | SSYM_0272 | | ZP_08040542 | |
| 6 | SOD_m00050 | | ZP_06193534 | |
| 7 | SOD_d02380 | | ZP_06191491 | |
| 8 | Spro_3910 | | YP_001480133 | |
| 9 | SOD_j00160 | | ZP_06193066 | |
| 10 | SOD_m00140 | | ZP_06193543 | |
| 11 | SSYM_0779 | | ZP_08038782 | |
| 12 | Pat9b_2844 | | YP_004116700 | |
| 13 | SSYM_0215 | | ZP_08040491 | |
| 14 | Spro_1286 | | YP_001477518 | |
| 15 | HMPREF0758_3087 | paaY | ZP_06639751 | |
| 16 | SOD_b04490 | | ZP_06190513 | |
| 17 | HMPREF0758_ 1765 | | ZP_06638429 | |
| 18 | HMPREF0758_2630 | deoR | ZP_06639294 | |
| 19 | HMPREF0758_ 1762 | vtaK | ZP_06638426 | |
| 20 | HMPREF0758_ 4768 | yedI | ZP_06641432 | |
| 21 | SOD_b01290 | | ZP_06190194 | |
| 22 | HMPREF0758_ 3449 | mdtJ | ZP_06640113 | |
| 23 | HMPREF0758_ 0340 | | ZP_06637004 | |
| 24 | HMPREF0758_ 4122 | dnaC | ZP_06640786 | |
| 25 | HMPREF0758_ 1641 | pepE | ZP_06638305 | |
| 26 | SOD_a01780 | | ZP_06189226 | |
| 27 | HMPREF0758_ 4410 | | ZP_06641074 | |
| 28 | Spro_0998 | | YP_001477230 | |

(continued)

| No. | Locus.Tag | Gene. Symbol | GenBank.Accession | Chromosome |
|---|---|---|---|---|
| 29 | HMPREF0758_4409 | | ZP_06641073 | |
| 30 | SOD_m00010 | | ZP_06193530 | |
| 31 | HMPREF0758_1747 | | ZP_06638411 | |
| 32 | SOD_b01600 | | ZP_06190225 | |
| 33 | SOD_b01460 | | ZP_06190211 | |
| 34 | SOD_c02470 | | ZP_06190898 | |
| 35 | Spro_2856 | | YP_001479085 | |
| 36 | Spro_1467 | | YP_001477699 | |
| 37 | Spro_1011 | | YP_001477243 | |
| 38 | SOD_d01640 | | ZP_06191418 | |
| 39 | CSAG_04704 | | ZP_04558356 | |
| 40 | Spro_0906 | | YP_001477140 | |
| 41 | Spro_2853 | | YP_001479082 | |
| 42 | Spro_4574 | | YP_001480795 | |
| 43 | SOD_a08590 | | ZP_06189897 | |
| 44 | SOD_d03490 | | ZP_06191602 | |
| 45 | SOD_b05060 | | ZP_06190570 | |
| 46 | HMPREF0758_ 4124 | cpaE | ZP_06640788 | |
| 47 | HMPREF0758_ 1879 | yhcC | ZP_06638543 | |
| 48 | HMPREF0758_ 0325 | apbE | ZP_06636989 | |
| 49 | Spro_2867 | | YP_001479096 | |
| 50 | Spro_0904 | | YP_001477138 | |

Table 19g: Data for Serratia, particularly Serratia marcescens (continued)

| No. | Start.Coord | End.Coord | Strand | Scaffold.ID |
|---|---|---|---|---|
| 1 | 721013 | 721786 | + | 640753091 |
| 2 | 113063 | 113683 | + | 647012891 |
| 3 | 196203 | 196802 | + | 647012889 |
| 4 | 14537 | 15286 | + | 647012864 |
| 5 | 1 | 622 | + | 651285392 |
| 6 | 5289 | 5756 | - | 647012829 |
| 7 | 276617 | 277270 | - | 647012820 |
| 8 | 4328564 | 4329223 | + | 640753091 |
| 9 | 10640 | 11029 | - | 647012826 |
| 10 | 12102 | 12680 | - | 647012829 |
| 11 | 54235 | 55095 | - | 651285075 |
| 12 | 3117915 | 3119003 | - | 649633106 |

(continued)

| No. | Start.Coord | End.Coord | Strand | Scaffold.ID |
|---|---|---|---|---|
| 13 | 1 | 203 | + | 651285334 |
| 14 | 1415512 | 1416153 | - | 640753091 |
| 15 | 166028 | 166624 | - | 647012881 |
| 16 | 563304 | 564224 | - | 647012818 |
| 17 | 17164 | 19063 | + | 647012864 |
| 18 | 1577 | 1921 | + | 647012878 |
| 19 | 15286 | 15990 | + | 647012864 |
| 20 | 357014 | 357931 | - | 647012891 |
| 21 | 207219 | 208373 | - | 647012818 |
| 22 | 144943 | 145311 | + | 647012883 |
| 23 | 46700 | 47314 | - | 647012846 |
| 24 | 40618 | 41358 | - | 647012889 |
| 25 | 66823 | 67548 | + | 647012859 |
| 26 | 183381 | 184292 | + | 647012817 |
| 27 | 12440 | 13084 | - | 647012890 |
| 28 | 1101888 | 1102271 | + | 640753091 |
| 29 | 11210 | 12427 | - | 647012890 |
| 30 | 189 | 1409 | - | 647012829 |
| 31 | 1316 | 1822 | + | 647012864 |
| 32 | 243135 | 244313 | - | 647012818 |
| 33 | 227360 | 228310 | + | 647012818 |
| 34 | 276786 | 277940 | - | 647012819 |
| 35 | 3134756 | 3135724 | - | 640753091 |
| 36 | 1599118 | 1600077 | + | 640753091 |
| 37 | 1112389 | 1113570 | - | 640753091 |
| 38 | 196893 | 197789 | - | 647012820 |
| 39 | 17332 | 17544 | - | 646206740 |
| 40 | 1005118 | 1006293 | + | 640753091 |
| 41 | 3131862 | 3132830 | - | 640753091 |
| 42 | 5048349 | 5049131 | - | 640753091 |
| 43 | 927700 | 928224 | + | 647012817 |
| 44 | 395510 | 396043 | + | 647012820 |
| 45 | 616091 | 617041 | - | 647012818 |
| 46 | 42335 | 42577 | - | 647012889 |
| 47 | 107941 | 108870 | + | 647012865 |
| 48 | 30480 | 31511 | - | 647012846 |
| 49 | 3146974 | 3147780 | - | 640753091 |
| 50 | 1003277 | 1004080 | + | 640753091 |

Table 20a: Data for Serratia, particularly Serratia marcescens

| No. | Scaffold.External. Accession | Scaffold.Name | best_pv |
|---|---|---|---|
| 1 | NC_009832 | Serratia proteamaculans 568: NC_009832 | 3.70422109249874e-06 |
| 2 | NZ_ADBY01000058 | Serratia odorifera DSM 4582 contig00061: NZ_ADBY01000058 | 6.00624466630552e-06 |
| 3 | NZ_ADBY01000056 | Serratia odorifera DSM 4582 contig00059: NZ_ADBY01000056 | 6.00624466630552e-06 |
| 4 | NZ_ADBY01000031 | Serratia odorifera DSM 4582 contig00034: NZ_ADBY01000031 | 8.61505367610747e-06 |
| 5 | NZ_GL636422 | Serratia symbiotica str. Tucson genomic scaffold contig09350: NZ_GL636422 | 8.61505367610747e-06 |
| 6 | NZ_ADBX01000013 | Serratia odorifera 4Rx13 SODm: NZ_ADBX01000013 | 8.61505367610747e-06 |
| 7 | NZ_ADBX01000004 | Serratia odorifera 4Rx13 SODd: NZ_ADBX01000004 | 1.33354783010227e-05 |
| 8 | NC_009832 | Serratia proteamaculans 568: NC_009832 | 1.37889112422256e-05 |
| 9 | NZ_ADBX01000010 | Serratia odorifera 4Rx13 SODj: NZ_ADBX01000010 | 1.55998636550055e-05 |
| 10 | NZ_ADBX01000013 | Serratia odorifera 4Rx13 SODm: NZ_ADBX01000013 | 1.55998636550055e-05 |
| 11 | NZ_GL636105 | Serratia symbiotica str. Tucson genomic scaffold scaffold00209: NZ_GL636105 | 1.55998636550055e-05 |
| 12 | NC_014837 | Pantoea sp. At-9b chromosome: NC_014837 | 1.55998636550055e-05 |
| 13 | NZ_GL636364 | Serratia symbiotica str. Tucson genomic scaffold contig09039: NZ_GL636364 | 1.55998636550055e-05 |
| 14 | NC_009832 | Serratia proteamaculans 568: NC_009832 | 1.55998636550055e-05 |
| 15 | NZ_ADBY01000048 | Serratia odorifera DSM 4582 contig00051: NZ_ADBY01000048 | 2.17438866604963e-05 |
| 16 | NZ_ADBX01000002 | Serratia odorifera 4Rx13 SODb: NZ_ADBX01000002 | 2.94809945975299e-05 |
| 17 | NZ_ADBY01000031 | Serratia odorifera DSM 4582 contig00034: NZ_ADBY01000031 | 3.13750499443641e-05 |
| 18 | NZ_ADBY01000045 | Serratia odorifera DSM 4582 contig00048: NZ_ADBY01000045 | 3.13750499443641e-05 |
| 19 | NZ_ADBY01000031 | Serratia odorifera DSM 4582 contig00034: NZ_ADBY01000031 | 3.13750499443641e-05 |
| 20 | NZ_ADBY01000058 | Serratia odorifera DSM 4582 contig00061: NZ_ADBY01000058 | 3.13750499443641e-05 |
| 21 | NZ_ADBX01000002 | Serratia odorifera 4Rx13 SODb: NZ_ADBX01000002 | 3.43966250408489e-05 |
| 22 | NZ_ADBY01000050 | Serratia odorifera DSM 4582 contig00053: NZ_ADBY01000050 | 3.66332257629745e-05 |
| 23 | NZ_ADBY01000013 | Serratia odorifera DSM 4582 contig00016: NZ_ADBY01000013 | 3.7147650177074e-05 |

(continued)

| No. | Scaffold.External. Accession | Scaffold.Name | best_pv |
|---|---|---|---|
| 24 | NZ_ADBY01000056 | Serratia odorifera DSM 4582 contig00059: NZ_ADBY01000056 | 3.7147650177074e-05 |
| 25 | NZ_ADBY01000026 | Serratia odorifera DSM 4582 contig00029: NZ_ADBY01000026 | 3.7147650177074e-05 |
| 26 | NZ_ADBX01000001 | Serratia odorifera 4Rx13 SODa: NZ_ADBX01000001 | 3.77977323028027e-05 |
| 27 | NC_009832 | Serratia proteamaculans 568: NC_009832 | 4.06693999812307e-05 |
| 28 | NZ_ADBY01000031 | Serratia odorifera DSM 4582 contig00034: NZ_ADBY01000031 | 4.20000578456372e-05 |
| 29 | NZ_ADBX01000002 | Serratia odorifera 4Rx13 SODb: NZ_ADBX01000002 | 5.02288669357547e-05 |
| 30 | NZ_ADBX01000002 | Serratia odorifera 4Rx13 SODb: NZ_ADBX01000002 | 5.02288669357547e-05 |
| 31 | NZ_ADBX01000003 | Serratia odorifera 4Rx13 SODc: NZ_ADBX01000003 | 5.02288669357547e-05 |
| 32 | NC_009832 | Serratia proteamaculans 568: NC_009832 | 5.02288669357547e-05 |
| 33 | NC_009832 | Serratia proteamaculans 568: NC_009832 | 5.02288669357547e-05 |
| 34 | NC_009832 | Serratia proteamaculans 568: NC_009832 | 5.02288669357547e-05 |
| 35 | NZ_ADBX01000004 | Serratia odorifera 4Rx13 SODd: NZ_ADBX01000004 | 5.02288669357547e-05 |
| 36 | NZ_GG657374 | Citrobacter sp. 30_2 genomic scaffold supercont1.9: NZ_GG657374 | 5.02288669357547e-05 |
| 37 | NC_009832 | Serratia proteamaculans 568: NC_009832 | 5.02288669357547e-05 |
| 38 | NC_009832 | Serratia proteamaculans 568: NC_009832 | 5.02288669357547e-05 |
| 39 | NC_009832 | Serratia proteamaculans 568: NC_009832 | 5.02288669357547e-05 |
| 40 | NZ_ADBX01000001 | Serratia odorifera 4Rx13 SODa: NZ_ADBX01000001 | 5.02288669357547e-05 |
| 41 | NZ_ADBX01000004 | Serratia odorifera 4Rx13 SODd: NZ_ADBX01000004 | 5.02288669357547e-05 |
| 42 | NZ_ADBX01000002 | Serratia odorifera 4Rx13 SODb: NZ_ADBX01000002 | 5.02288669357547e-05 |
| 43 | NZ_ADBY01000032 | Serratia odorifera DSM 4582 contig00035: NZ_ADBY01000032 | 5.02288669357547e-05 |
| 44 | NZ_ADBY01000013 | Serratia odorifera DSM 4582 contig00016: NZ_ADBY01000013 | 5.88044402302405e-05 |
| 45 | NC_009832 | Serratia proteamaculans 568: NC_009832 | 6.62251962114689e-05 |
| 46 | NC_009832 | Serratia proteamaculans 568: NC_009832 | 7.15986854143193e-05 |
| 47 | NZ_ADBX01000002 | Serratia odorifera 4Rx13 SODb: NZ_ADBX01000002 | 7.1811098690049e-05 |
| 48 | NC_009832 | Serratia proteamaculans 568: NC_009832 | 7.1811098690049e-05 |
| 49 | NC_009832 | Serratia proteamaculans 568: NC_009832 | 7.1811098690049e-05 |
| 50 | NZ_ADBY01000036 | Serratia odorifera DSM 4582 contig00039: NZ_ADBY01000036 | 7.1811098690049e-05 |

Table 20b: Data for Serratia, particularly Serratia marcescens (continued)

| No. | sign_phenos | sign_phenos_class | best_pheno | best_pheno_class |
|---|---|---|---|---|
| 1 | AZT;CAX;CAZ;CFT;CP;GM;LVX;P/T;TO;T/S | aminoglycoside;fluoroquinolone;lactam;other | LVX | fluoroquinolone |
| 2 | CP;LVX;T/S | fluoroquinolone;other | CP | fluoroquinolone |
| 3 | AZT;CAX;CP;LVX;T/S | fluoroquinolone;lactam;other | LVX | fluoroquinolone |
| 4 | AZT;CAX;CFT;CP;LVX;P/T;T/S | fluoroquinolone;lactam;other | LVX | fluoroquinolone |
| 5 | CAX;CAZ;CFT;CP;LVX;P/T | fluoroquinolone;lactam | CP | fluoroquinolone |
| 6 | AZT;CAX;CAZ;CFT;CP;LVX;P/T | fluoroquinolone;lactam | CP | fluoroquinolone |
| 7 | CAX;CFT;CP;LVX | fluoroquinolone;lactam | LVX | fluoroquinolone |
| 8 | CAX;CFT;CP;LVX;T/S | fluoroquinolone;lactam;other | LVX | fluoroquinolone |
| 9 | CAX;CFT;CP;LVX;TO | aminoglycoside;fluoroquinolone;lactam | LVX | fluoroquinolone |
| 10 | AZT;CAX;CAZ;CFT;CP;LVX;P/T | fluoroquinolone;lactam | CP | fluoroquinolone |
| 11 | AZT;CAX;CAZ;CFT;CP;P/T | fluoroquinolone;lactam | CAX | lactam |
| 12 | CAX;CFT;CP;GM;LVX;P/T;TO;T/S | aminoglycoside;fluoroquinolone;lactam;other | LVX | fluoroquinolone |
| 13 | AZT;CAX;CAZ;CFT;CP;P/T | fluoroquinolone;lactam | CAX | lactam |
| 14 | CP;LVX | fluoroquinolone | LVX | fluoroquinolone |
| 15 | AZT;CAX;CFT;CP;GM;LVX;P/T;TO;T/S | aminoglycoside;fluoroquinolone;lactam;other | LVX | fluoroquinolone |
| 16 | AZT;CAX;CFT;CP;GM;LVX;P/T;TO;T/S | aminoglycoside;fluoroquinolone;lactam;other | LVX | fluoroquinolone |
| 17 | AZT;CAX;CFT;CP;LVX;P/T;T/S | fluoroquinolone;lactam;other | LVX | fluoroquinolone |
| 18 | CAX;CFT;CP;GM;LVX;P/T;TO;T/S | aminoglycoside;fluoroquinolone;lactam;other | LVX | fluoroquinolone |
| 19 | AZT;CAX;CFT;CP;LVX;P/T;T/S | fluoroquinolone;lactam;other | LVX | fluoroquinolone |
| 20 | AZT;CAX;CFT;CP;GM;LVX;P/T;TO;T/S | aminoglycoside;fluoroquinolone;lactam;other | LVX | fluoroquinolone |
| 21 | CAX;CP;LVX;T/S | fluoroquinolone;lactam;other | LVX | fluoroquinolone |
| 22 | AZT;CAX;CFT;CP;GM;LVX;P/T;TO;T/S | aminoglycoside;fluoroquinolone;lactam;other | LVX | fluoroquinolone |
| 23 | CAX;CFT;CP;GM;LVX;P/T;TO;T/S | aminoglycoside;fluoroquinolone;lactam;other | CP | fluoroquinolone |
| 24 | AZT;CAX;CAZ;CFT;P/T | lactam | CAZ | lactam |
| 25 | CAX;CFT;CP;GM;LVX;P/T | aminoglycoside;fluoroquinolone;lactam | LVX | fluoroquinolone |
| 26 | AZT;CAX;CFT;CP;GM;LVX;P/T;TO;T/S | aminoglycoside;fluoroquinolone;lactam;other | LVX | fluoroquinolone |
| 27 | CAX;CP;LVX | fluoroquinolone;lactam | LVX | fluoroquinolone |
| 28 | CAX;CFT;CP;LVX;P/T;T/S | fluoroquinolone;lactam;other | P/T | lactam |

(continued)

| No. | sign_phenos | sign_phenos_class | best_pheno | best_pheno_class |
|---|---|---|---|---|
| 29 | AZT;CAX;CFT;CP;GM;LVX; P/T;TO;T/S | aminoglycoside;fluoroquinolone; lactam;other | LVX | fluoroquinolone |
| 30 | CAX;CFT;CP;LVX;T/S | fluoroquinolone;lactam;other | LVX | fluoroquinolone |
| 31 | CAX;CFT;CP;LVX;T/S | fluoroquinolone;lactam;other | LVX | fluoroquinolone |
| 32 | AZT;CAX;CFT;CP;GM;LVX; P/T;TO;T/S | aminoglycoside;fluoroquinolone; lactam;other | LVX | fluoroquinolone |
| 33 | AZT;CAX;CFT;CP;LVX;T/S | fluoroquinolone;lactam;other | CP | fluoroquinolone |
| 34 | AZT;CAX;CFT;CP;GM;LVX; P/T;TO;T/S | aminoglycoside;fluoroquinolone; lactam;other | LVX | fluoroquinolone |
| 35 | CAX;CFT;CP;LVX;P/T;T/S | fluoroquinolone;lactam;other | LVX | fluoroquinolone |
| 36 | CP;GM;LVX;TO;T/S | aminoglycoside;fluoroquinolone; other | LVX | fluoroquinolone |
| 37 | CAX;CFT;CP;LVX;P/T;T/S | fluoroquinolone;lactam;other | LVX | fluoroquinolone |
| 38 | AZT;CAX;CFT;CP;GM;LVX; P/T;TO;T/S | aminoglycoside;fluoroquinolone; lactam;other | LVX | fluoroquinolone |
| 39 | AZT;CAX;CFT;CP;GM;LVX; P/T;TO;T/S | aminoglycoside;fluoroquinolone; lactam;other | LVX | fluoroquinolone |
| 40 | CP;LVX;P/T | fluoroquinolone;lactam | LVX | fluoroquinolone |
| 41 | CAX;CFT;CP;LVX;T/S | fluoroquinolone;lactam;other | LVX | fluoroquinolone |
| 42 | AZT;CAX;CFT;CP;GM;LVX; P/T;TO;T/S | aminoglycoside;fluoroquinolone; lactam;other | LVX | fluoroquinolone |
| 43 | AZT;CAX;CFT;CP;GM;LVX;TO; T/S | aminoglycoside;fluoroquinolone; lactam;other | LVX | fluoroquinolone |
| 44 | CAX;CFT;CP;LVX;P/T | fluoroquinolone;lactam | LVX | fluoroquinolone |
| 45 | CAX;CFT;CP;LVX | fluoroquinolone;lactam | LVX | fluoroquinolone |
| 46 | AZT;CAX;CFT;CP;GM;LVX; P/T;TO;T/S | aminoglycoside;fluoroquinolone; lactam;other | LVX | fluoroquinolone |
| 47 | AZT;CAX;CFT;CP;LVX;P/T;TO | aminoglycoside;fluoroquinolone; lactam | CP | fluoroquinolone |
| 48 | CAX;CFT;CP;LVX;T/S | fluoroquinolone;lactam;other | LVX | fluoroquinolone |
| 49 | CFT;CP;LVX | fluoroquinolone;lactam | LVX | fluoroquinolone |
| 50 | AZT;CAX;CFT;CP;GM;LVX;P/T | aminoglycoside;fluoroquinolone; lactam | CAX | lactam |

Table 20c: Data for Serratia, particularly Serratia marcescens (continued)

| No. | num_aminoglycoside | num_fluoroquinolone | num_lactam | num_other |
|---|---|---|---|---|
| 1 | 2 | 2 | 5 | 1 |
| 2 | 0 | 2 | 0 | 1 |
| 3 | 0 | 2 | 2 | 1 |
| 4 | 0 | 2 | 4 | 1 |
| 5 | 0 | 2 | 4 | 0 |

(continued)

| No. | num_aminoglycoside | num_fluoroquinolone | num_lactam | num_other |
|-----|-----|-----|-----|-----|
| 6 | 0 | 2 | 5 | 0 |
| 7 | 0 | 2 | 2 | 0 |
| 8 | 0 | 2 | 2 | 1 |
| 9 | 1 | 2 | 2 | 0 |
| 10 | 0 | 2 | 5 | 0 |
| 11 | 0 | 1 | 5 | 0 |
| 12 | 2 | 2 | 3 | 1 |
| 13 | 0 | 1 | 5 | 0 |
| 14 | 0 | 2 | 0 | 0 |
| 15 | 2 | 2 | 4 | 1 |
| 16 | 2 | 2 | 4 | 1 |
| 17 | 0 | 2 | 4 | 1 |
| 18 | 2 | 2 | 3 | 1 |
| 19 | 0 | 2 | 4 | 1 |
| 20 | 2 | 2 | 4 | 1 |
| 21 | 0 | 2 | 1 | 1 |
| 22 | 2 | 2 | 4 | 1 |
| 23 | 2 | 2 | 3 | 1 |
| 24 | 0 | 0 | 5 | 0 |
| 25 | 1 | 2 | 3 | 0 |
| 26 | 2 | 2 | 4 | 1 |
| 27 | 0 | 2 | 1 | 0 |
| 28 | 0 | 2 | 3 | 1 |
| 29 | 2 | 2 | 4 | 1 |
| 30 | 0 | 2 | 2 | 1 |
| 31 | 0 | 2 | 2 | 1 |
| 32 | 2 | 2 | 4 | 1 |
| 33 | 0 | 2 | 3 | 1 |
| 34 | 2 | 2 | 4 | 1 |
| 35 | 0 | 2 | 3 | 1 |
| 36 | 2 | 2 | 0 | 1 |
| 37 | 0 | 2 | 3 | 1 |
| 38 | 2 | 2 | 4 | 1 |
| 39 | 2 | 2 | 4 | 1 |
| 40 | 0 | 2 | 1 | 0 |
| 41 | 0 | 2 | 2 | 1 |
| 42 | 2 | 2 | 4 | 1 |
| 43 | 2 | 2 | 3 | 1 |

(continued)

| No. | num_aminoglycoside | num_fluoroquinolone | num_lactam | num_other |
|-----|-----|-----|-----|-----|
| 44 | 0 | 2 | 3 | 0 |
| 45 | 0 | 2 | 2 | 0 |
| 46 | 2 | 2 | 4 | 1 |
| 47 | 1 | 2 | 4 | 0 |
| 48 | 0 | 2 | 2 | 1 |
| 49 | 0 | 2 | 1 | 0 |
| 50 | 1 | 2 | 4 | 0 |

Table 20d: Data for Serratia, particularly Serratia marcescens (continued)

| No. | AZT_pv_adj | CAX_pv_adj | CAZ_pv_adj | CFT_pv_adj | CP_pv_adj |
|-----|-----|-----|-----|-----|-----|
| 1 | 4.82E-04 | 5.24E-04 | 6.41E-03 | 7.29E-04 | 2.27E-05 |
| 2 | 1.58E-01 | 1.39E-01 | 2.26E-01 | 3.68E-02 | 6.01E-06 |
| 3 | 6.77E-04 | 8.30E-03 | 2.51E-02 | 1.65E-02 | 2.17E-05 |
| 4 | 8.92E-03 | 3.01E-03 | 3.16E-01 | 7.90E-03 | 2.95E-05 |
| 5 | 1.60E-02 | 4.77E-04 | 5.25E-03 | 1.36E-04 | 8.62E-06 |
| 6 | 1.22E-03 | 1.22E-03 | 1.24E-03 | 4.08E-04 | 8.62E-06 |
| 7 | 3.82E-02 | 3.55E-03 | 4.51E-02 | 2.17E-03 | 7.86E-05 |
| 8 | 1.48E-01 | 5.09E-03 | 6.93E-02 | 4.93E-03 | 1.10E-04 |
| 9 | 4.53E-02 | 5.02E-03 | 3.12E-01 | 2.64E-03 | 5.13E-04 |
| 10 | 6.43E-04 | 1.61E-04 | 7.67E-04 | 8.45E-05 | 1.56E-05 |
| 11 | 3.67E-05 | 1.56E-05 | 1.56E-05 | 5.02E-05 | 6.54E-03 |
| 12 | 1.44E-02 | 6.36E-03 | 4.83E-02 | 3.41E-03 | 2.36E-04 |
| 13 | 3.67E-05 | 1.56E-05 | 1.56E-05 | 5.02E-05 | 6.54E-03 |
| 14 | 4.07E-01 | 5.36E-02 | 7.62E-01 | 4.93E-02 | 5.86E-04 |
| 15 | 1.11E-03 | 7.67E-04 | 4.28E-02 | 1.04E-03 | 4.94E-05 |
| 16 | 6.43E-04 | 1.24E-03 | 2.38E-02 | 2.68E-03 | 1.82E-04 |
| 17 | 4.26E-03 | 1.84E-03 | 6.64E-02 | 4.93E-03 | 1.22E-04 |
| 18 | 1.73E-02 | 1.76E-04 | 2.04E-02 | 5.24E-04 | 6.30E-04 |
| 19 | 4.26E-03 | 1.84E-03 | 6.64E-02 | 4.93E-03 | 1.22E-04 |
| 20 | 9.20E-04 | 1.82E-04 | 2.34E-02 | 1.48E-04 | 1.69E-04 |
| 21 | 2.19E-02 | 6.70E-03 | 1 | 2.46E-02 | 7.82E-04 |
| 22 | 1.55E-03 | 4.30E-04 | 6.08E-02 | 5.90E-04 | 1.52E-04 |
| 23 | 4.85E-02 | 7.20E-04 | 1.01E-01 | 6.90E-04 | 3.71E-05 |
| 24 | 2.28E-03 | 2.41E-03 | 3.71E-05 | 4.23E-03 | 1.66E-01 |
| 25 | 1.01E-02 | 3.27E-04 | 5.44E-02 | 4.95E-04 | 7.62E-04 |
| 26 | 8.18E-04 | 3.20E-04 | 4.45E-02 | 3.27E-04 | 1.53E-04 |
| 27 | 2.82E-02 | 6.08E-03 | 2.88E-01 | 4.51E-02 | 1.10E-04 |

(continued)

| No. | AZT_pv_adj | CAX_pv_adj | CAZ_pv_adj | CFT_pv_adj | CP_pv_adj |
|---|---|---|---|---|---|
| 28 | 5.92E-02 | 3.15E-04 | 6.50E-01 | 1.11E-03 | 6.89E-03 |
| 29 | 1.66E-03 | 6.30E-04 | 8.28E-02 | 6.43E-04 | 3.33E-04 |
| 30 | 5.14E-02 | 1.60E-03 | 5.77E-02 | 2.64E-03 | 1.17E-03 |
| 31 | 5.14E-02 | 1.60E-03 | 5.77E-02 | 2.64E-03 | 1.17E-03 |
| 32 | 1.17E-03 | 3.15E-04 | 5.90E-02 | 3.27E-04 | 3.15E-04 |
| 33 | 1.07E-03 | 3.99E-03 | 2.05E-02 | 2.84E-03 | 5.02E-05 |
| 34 | 1.16E-03 | 5.89E-04 | 5.88E-02 | 6.07E-04 | 2.32E-04 |
| 35 | 4.11E-02 | 1.55E-03 | 5.76E-02 | 2.12E-03 | 1.15E-03 |
| 36 | 2.08E-02 | 2.04E-02 | 2.72E-01 | 3.09E-02 | 3.33E-04 |
| 37 | 5.14E-02 | 1.13E-03 | 5.80E-02 | 1.53E-03 | 1.50E-03 |
| 38 | 1.17E-03 | 3.15E-04 | 5.90E-02 | 3.27E-04 | 3.15E-04 |
| 39 | 4.30E-03 | 1.43E-03 | 1.74E-01 | 2.47E-03 | 9.38E-04 |
| 40 | 8.63E-02 | 1.24E-02 | 2.31E-01 | 1.12E-02 | 7.34E-04 |
| 41 | 5.14E-02 | 2.07E-03 | 5.80E-02 | 2.73E-03 | 1.50E-03 |
| 42 | 1.17E-03 | 3.15E-04 | 5.90E-02 | 3.27E-04 | 3.15E-04 |
| 43 | 7.04E-03 | 6.21E-03 | 3.82E-02 | 3.31E-03 | 1.04E-04 |
| 44 | 3.16E-01 | 1.58E-03 | 5.58E-01 | 1.57E-03 | 7.78E-04 |
| 45 | 2.46E-02 | 8.70E-03 | 2.31E-01 | 5.07E-03 | 1.80E-03 |
| 46 | 1.55E-03 | 7.82E-04 | 5.97E-02 | 8.10E-04 | 3.15E-04 |
| 47 | 2.96E-04 | 2.61E-03 | 5.49E-02 | 3.46E-03 | 7.18E-05 |
| 48 | 5.20E-02 | 2.61E-03 | 5.87E-02 | 3.46E-03 | 1.53E-03 |
| 49 | 2.15E-01 | 1.36E-02 | 2.60E-01 | 9.12E-03 | 1.92E-04 |
| 50 | 2.93E-03 | 7.18E-05 | 2.88E-02 | 7.18E-05 | 1.15E-03 |

Table 20e: Data for Serratia, particularly Serratia marcescens (continued)

| No. | GM_pv_adj | LVX_pv_adj | P/T_pv_adj | TO_pv_adj | T/S_pv_adj |
|---|---|---|---|---|---|
| 1 | 7.29E-04 | 3.70E-06 | 4.19E-03 | 5.65E-04 | 2.79E-04 |
| 2 | 3.92E-02 | 8.62E-06 | 5.32E-01 | 5.08E-02 | 9.68E-03 |
| 3 | 3.60E-02 | 6.01E-06 | 4.96E-02 | 9.86E-02 | 1.55E-03 |
| 4 | 2.38E-01 | 8.62E-06 | 4.51E-04 | 1.33E-01 | 5.10E-03 |
| 5 | 2.87E-01 | 4.05E-04 | 5.63E-05 | 3.66E-02 | 2.11E-02 |
| 6 | 9.01E-01 | 8.10E-04 | 6.41E-03 | 4.07E-02 | 1.63E-01 |
| 7 | 3.66E-02 | 1.33E-05 | 1.49E-02 | 1.15E-02 | 2.81E-02 |
| 8 | 1.16E-01 | 1.38E-05 | 1.31E-02 | 6.43E-02 | 5.52E-03 |
| 9 | 1.25E-02 | 1.56E-05 | 5.88E-02 | 1.37E-03 | 3.00E-02 |
| 10 | 2.58E-01 | 1.22E-04 | 9.05E-05 | 4.31E-02 | 3.97E-02 |
| 11 | 2.06E-01 | 3.60E-01 | 3.67E-05 | 1.87E-01 | 3.72E-01 |

(continued)

| No. | GM_pv_adj | LVX_pv_adj | P/T_pv_adj | TO_pv_adj | T/S_pv_adj |
|-----|-----------|------------|------------|-----------|------------|
| 12 | 5.33E-03 | 1.56E-05 | 6.87E-03 | 3.46E-03 | 8.20E-03 |
| 13 | 2.06E-01 | 3.60E-01 | 3.67E-05 | 1.87E-01 | 3.72E-01 |
| 14 | 4.95E-02 | 1.56E-05 | 1.34E-01 | 1.43E-01 | 2.07E-02 |
| 15 | 2.56E-03 | 2.17E-05 | 2.35E-03 | 3.96E-03 | 4.94E-04 |
| 16 | 3.89E-04 | 2.95E-05 | 1.52E-03 | 3.51E-03 | 2.43E-03 |
| 17 | 1.48E-01 | 3.14E-05 | 2.32E-04 | 8.78E-02 | 5.89E-04 |
| 18 | 3.24E-03 | 3.14E-05 | 2.41E-03 | 4.32E-03 | 4.63E-03 |
| 19 | 1.48E-01 | 3.14E-05 | 2.32E-04 | 8.78E-02 | 5.89E-04 |
| 20 | 2.78E-03 | 3.14E-05 | 1.10E-03 | 2.42E-03 | 6.75E-04 |
| 21 | 2.77E-02 | 3.44E-05 | 5.49E-02 | 1.12E-01 | 1.40E-03 |
| 22 | 5.63E-03 | 3.66E-05 | 3.16E-03 | 5.49E-03 | 3.24E-03 |
| 23 | 3.72E-03 | 4.07E-05 | 1.76E-03 | 4.82E-03 | 3.60E-03 |
| 24 | 2.22E-01 | 1 | 9.74E-03 | 1.18E-01 | 2.81E-01 |
| 25 | 4.82E-03 | 3.71E-05 | 7.82E-04 | 2.06E-02 | 1.08E-02 |
| 26 | 1.24E-03 | 3.78E-05 | 1.36E-03 | 2.34E-03 | 1.55E-03 |
| 27 | 5.36E-02 | 4.07E-05 | 4.63E-02 | 3.92E-02 | 1.38E-02 |
| 28 | 1.41E-01 | 7.82E-04 | 4.20E-05 | 3.04E-02 | 5.10E-03 |
| 29 | 2.77E-03 | 5.02E-05 | 1.37E-03 | 5.69E-03 | 2.21E-03 |
| 30 | 1.47E-02 | 5.02E-05 | 1.03E-02 | 3.27E-02 | 6.75E-03 |
| 31 | 1.47E-02 | 5.02E-05 | 1.03E-02 | 3.27E-02 | 6.75E-03 |
| 32 | 1.76E-03 | 5.02E-05 | 1.07E-03 | 4.09E-03 | 2.26E-03 |
| 33 | 2.27E-02 | 3.89E-04 | 4.16E-02 | 1.11E-02 | 6.71E-03 |
| 34 | 1.76E-03 | 5.02E-05 | 1.80E-03 | 3.17E-03 | 2.24E-03 |
| 35 | 1.48E-02 | 5.02E-05 | 8.05E-03 | 2.57E-02 | 6.70E-03 |
| 36 | 2.77E-03 | 5.02E-05 | 1.42E-02 | 2.41E-03 | 2.10E-04 |
| 37 | 1.48E-02 | 5.02E-05 | 5.52E-03 | 3.27E-02 | 6.89E-03 |
| 38 | 1.76E-03 | 5.02E-05 | 1.07E-03 | 4.09E-03 | 2.26E-03 |
| 39 | 1.31E-03 | 5.02E-05 | 5.93E-03 | 3.06E-03 | 2.21E-03 |
| 40 | 3.13E-02 | 5.02E-05 | 5.52E-03 | 1.02E-02 | 9.79E-02 |
| 41 | 1.48E-02 | 5.02E-05 | 1.06E-02 | 3.27E-02 | 6.89E-03 |
| 42 | 1.76E-03 | 5.02E-05 | 1.07E-03 | 4.09E-03 | 2.26E-03 |
| 43 | 9.28E-04 | 5.02E-05 | 6.47E-02 | 2.47E-03 | 1.76E-03 |
| 44 | 1.83E-02 | 5.88E-05 | 1.62E-03 | 1.34E-02 | 1.37E-02 |
| 45 | 9.13E-01 | 6.62E-05 | 6.12E-02 | 1.22E-01 | 1.54E-01 |
| 46 | 1.80E-03 | 7.16E-05 | 2.42E-03 | 4.20E-03 | 2.34E-03 |
| 47 | 4.20E-02 | 8.45E-05 | 9.22E-03 | 6.72E-03 | 1.09E-02 |
| 48 | 1.50E-02 | 7.18E-05 | 1.34E-02 | 4.14E-02 | 9.77E-03 |
| 49 | 4.30E-02 | 7.18E-05 | 3.73E-02 | 2.25E-02 | 1.57E-02 |

(continued)

| No. | GM_pv_adj | LVX_pv_adj | P/T_pv_adj | TO_pv_adj | T/S_pv_adj |
|-----|-----------|------------|------------|-----------|------------|
| 50  | 4.68E-03  | 1.51E-04   | 1.15E-03   | 2.57E-02  | 1.67E-02   |

Table 20f: Data for Serratia, particularly Serratia marcescens (continued)

| No. | Locus.Tag | Gene. Symbol | GenBank.Accession | Chromosome |
|-----|-----------|--------------|-------------------|------------|
| 1   | Spro_0657 |              | YP_001476891      |            |
| 2   | HMPREF0758_ 4528 |       | ZP_06641192       |            |
| 3   | HMPREF0758_ 4282 | wrbA | ZP_06640946       |            |
| 4   | HMPREF0758_ 1761 |      | ZP_06638425       |            |
| 5   | SSYM_0272 |              | ZP_08040542       |            |
| 6   | SOD_m00050 |             | ZP_06193534       |            |
| 7   | SOD_d02380 |             | ZP_06191491       |            |
| 8   | Spro_3910 |              | YP_001480133      |            |
| 9   | SOD_j00160 |             | ZP_06193066       |            |
| 10  | SOD_m00140 |             | ZP_06193543       |            |
| 11  | SSYM_0779 |              | ZP_08038782       |            |
| 12  | Pat9b_2844 |             | YP_004116700      |            |
| 13  | SSYM_0215 |              | ZP_08040491       |            |
| 14  | Spro_1286 |              | YP_001477518      |            |
| 15  | HMPREF0758_3087 | paaY | ZP_06639751       |            |
| 16  | SOD_b04490 |             | ZP_06190513       |            |
| 17  | HMPREF0758_ 1765 |      | ZP_06638429       |            |
| 18  | HMPREF0758_2630 | deoR | ZP_06639294       |            |
| 19  | HMPREF0758_ 1762 | vtaK | ZP_06638426       |            |
| 20  | HMPREF0758_ 4768 | yedI | ZP_06641432       |            |
| 21  | SOD_b01290 |             | ZP_06190194       |            |
| 22  | HMPREF0758_ 3449 | mdtJ | ZP_06640113       |            |
| 23  | HMPREF0758_ 0340 |      | ZP_06637004       |            |
| 24  | HMPREF0758_ 4122 | dnaC | ZP_06640786       |            |
| 25  | HMPREF0758_ 1641 | pepE | ZP_06638305       |            |
| 26  | SOD_a01780 |             | ZP_06189226       |            |
| 27  | Spro_0998 |              | YP_001477230      |            |
| 28  | HMPREF0758_1747 |       | ZP_06638411       |            |
| 29  | SOD_b01600 |             | ZP_06190225       |            |
| 30  | SOD_b01460 |             | ZP_06190211       |            |
| 31  | SOD_c02470 |             | ZP_06190898       |            |
| 32  | Spro_2856 |              | YP_001479085      |            |
| 33  | Spro_1467 |              | YP_001477699      |            |

(continued)

| No. | Locus.Tag | Gene. Symbol | GenBank.Accession | Chromosome |
|---|---|---|---|---|
| 34 | Spro_1011 | | YP_001477243 | |
| 35 | SOD_d01640 | | ZP_06191418 | |
| 36 | CSAG_04704 | | ZP_04558356 | |
| 37 | Spro_0906 | | YP_001477140 | |
| 38 | Spro_2853 | | YP_001479082 | |
| 39 | Spro_4574 | | YP_001480795 | |
| 40 | SOD_a08590 | | ZP_06189897 | |
| 41 | SOD_d03490 | | ZP_06191602 | |
| 42 | SOD_b05060 | | ZP_06190570 | |
| 43 | HMPREF0758_1879 | yhcC | ZP_06638543 | |
| 44 | HMPREF0758_ 0325 | apbE | ZP_06636989 | |
| 45 | Spro_2867 | | YP_001479096 | |
| 46 | Spro_0904 | | YP_001477138 | |
| 47 | SOD_b01270 | | ZP_06190192 | |
| 48 | Spro_4454 | | YP_001480676 | |
| 49 | Spro_4262 | | YP_001480484 | |
| 50 | HMPREF0758_ 2167 | hrpB | ZP_06638831 | |

Table 20g: Data for Serratia, particularly Serratia marcescens (continued)

| No. | Start.Coord | End.Coord | Strand | Scaffold.ID |
|---|---|---|---|---|
| 1 | 721013 | 721786 | + | 640753091 |
| 2 | 113063 | 113683 | + | 647012891 |
| 3 | 196203 | 196802 | + | 647012889 |
| 4 | 14537 | 15286 | + | 647012864 |
| 5 | 1 | 622 | + | 651285392 |
| 6 | 5289 | 5756 | - | 647012829 |
| 7 | 276617 | 277270 | - | 647012820 |
| 8 | 4328564 | 4329223 | + | 640753091 |
| 9 | 10640 | 11029 | - | 647012826 |
| 10 | 12102 | 12680 | - | 647012829 |
| 11 | 54235 | 55095 | - | 651285075 |
| 12 | 3117915 | 3119003 | - | 649633106 |
| 13 | 1 | 203 | + | 651285334 |
| 14 | 1415512 | 1416153 | - | 640753091 |
| 15 | 166028 | 166624 | - | 647012881 |
| 16 | 563304 | 564224 | - | 647012818 |
| 17 | 17164 | 19063 | + | 647012864 |

(continued)

| No. | Start.Coord | End.Coord | Strand | Scaffold.ID |
|-----|-------------|-----------|--------|-------------|
| 18 | 1577 | 1921 | + | 647012878 |
| 19 | 15286 | 15990 | + | 647012864 |
| 20 | 357014 | 357931 | - | 647012891 |
| 21 | 207219 | 208373 | - | 647012818 |
| 22 | 144943 | 145311 | + | 647012883 |
| 23 | 46700 | 47314 | - | 647012846 |
| 24 | 40618 | 41358 | - | 647012889 |
| 25 | 66823 | 67548 | + | 647012859 |
| 26 | 183381 | 184292 | + | 647012817 |
| 27 | 1101888 | 1102271 | + | 640753091 |
| 28 | 1316 | 1822 | + | 647012864 |
| 29 | 243135 | 244313 | - | 647012818 |
| 30 | 227360 | 228310 | + | 647012818 |
| 31 | 276786 | 277940 | - | 647012819 |
| 32 | 3134756 | 3135724 | - | 640753091 |
| 33 | 1599118 | 1600077 | + | 640753091 |
| 34 | 1112389 | 1113570 | - | 640753091 |
| 35 | 196893 | 197789 | - | 647012820 |
| 36 | 17332 | 17544 | - | 646206740 |
| 37 | 1005118 | 1006293 | + | 640753091 |
| 38 | 3131862 | 3132830 | - | 640753091 |
| 39 | 5048349 | 5049131 | - | 640753091 |
| 40 | 927700 | 928224 | + | 647012817 |
| 41 | 395510 | 396043 | + | 647012820 |
| 42 | 616091 | 617041 | - | 647012818 |
| 43 | 107941 | 108870 | + | 647012865 |
| 44 | 30480 | 31511 | - | 647012846 |
| 45 | 3146974 | 3147780 | - | 640753091 |
| 46 | 1003277 | 1004080 | + | 640753091 |
| 47 | 202590 | 204044 | - | 647012818 |
| 48 | 4927194 | 4927727 | + | 640753091 |
| 49 | 4725710 | 4726606 | - | 640753091 |
| 50 | 70350 | 72779 | + | 647012869 |

Example 11: Shigella

The procedure was carried out as in Example 1, except that the following microorganisms were used:

Bacterial Strains

The inventors selected 442 Shigella strains, particularly Shigella boydii, Shigella dysenteriae, Shigella flexneri, Shigella sonnei and other Shigella species, from the microbiology strain collection at Siemens Healthcare Diagnostics (West Sacramento, CA) for susceptibility testing and whole genome sequencing.

From MetaRef, 730 centroids of Shigella species were used as reference sequences.

The results for Shigella species are shown in Tables 21 (corresponding to Table 1) and 22 (corresponding to Table 2).

Table 21a: Data for Shigella species

| No. | Scaffold.External. Accession | Scaffold.Name | best_pv |
|---|---|---|---|
| 1 | NZ_ ADUB01000093 | Escherichia coli MS 175-1 E_coli175-1-1.0_Cont166.1: NZ_ADUB01000093 | 6.86676649351252e-56 |
| 2 | NZ_ ADUD01000475 | Escherichia coli MS 196-1 E_coli196-1-1.0_Cont1343.1: NZ_ADUD01000475 | 4.14817893591733e-46 |
| 3 | NZ_ ADWT01000006 | Escherichia coli MS 124-1 E_coliMS124-1-1.0.1_Cont5.1: NZ_ADWT01000006 | 4.14817893591733e-46 |
| 4 | AP011957 | Salmonella enterica subsp. enterica serovar Typhimurium str. T000240 DNA: AP011957 | 1.24332752332433e-44 |
| 5 | CP001383 | Shigella flexneri 2002017: CP001383 | 5.69762827081762e-37 |
| 6 | NZ_ AAMJ01000001 | Shigella dysenteriae 1012, unfinished sequence: NZ_ AAMJ01000001 | 3.04086824044829e-34 |
| 7 | NZ_ ADUB01000227 | Escherichia coli MS 175-1 E_coli175-1-1.0_Cont422.2: NZ_ADUB01000227 | 3.04086824044829e-34 |
| 8 | NZ_ AAMJ01000001 | Shigella dysenteriae 1012, unfinished sequence: NZ_ AAMJ01000001 | 5.67507153238613e-34 |
| 9 | CP001383 | Shigella flexneri 2002017: CP001383 | 1.46728061376833e-26 |
| 10 | NZ_ ADUB01000365 | Escherichia coli MS 175-1 E_coli175-1-1.0_Cont823.2: NZ_ADUB01000365 | 4.97695865217724e-26 |
| 11 | NZ_ ADUB01000054 | Escherichia coli MS 175-1 E_coli175-1-1.0_Cont100.1: NZ_ADUB01000054 | 1.31351033204593e-24 |
| 12 | CP001383 | Shigella flexneri 2002017: CP001383 | 1.667006155646725e-24 |
| 13 | NC_013509 | Edwardsiella tarda EIB202 plasmid pEIB202: NC_013509 | 3.081692811393e-24 |
| 14 | NZ_ ABKX01000001 | Escherichia albertii TW07627, unfinished sequence: NZ_ ABKX01000001 | 3.45432516278107e-24 |
| 15 | NC_007384 | Shigella sonnei Ss046: NC_007384 | 4.84345172785563e-23 |
| 16 | CP001383 | Shigella flexneri 2002017: CP001383 | 1.59570448713613e-17 |
| 17 | NZ_ ADUT01000002 | Shigella dysenteriae 1617 gss1617.assembly.11: NZ_ ADUT01000002 | 4.66906657298136e-17 |
| 18 | NZ_ ABKX01000001 | Escherichia albertii TW07627, unfinished sequence: NZ_ ABKX01000001 | 5.88113462200155e-17 |
| 19 | NZ_ ADTR01000390 | Escherichia coli MS 21-1 E_coli21-1-1.0_Cont744.1: NZ_ ADTR01000390 | 7.05214682414966e-16 |
| 20 | NZ_ ADUT01000002 | Shigella dysenteriae 1617 gss1617.assembly.11: NZ_ ADUT01000002 | 3.49647006284355e-15 |
| 21 | NC_006856 | Salmonella enterica subsp. enterica serovar Choleraesuis str. SC-B67 plasmid pSC138: NC_006856 | 3.88428414470937e-13 |

(continued)

| No. | Scaffold.External. Accession | Scaffold.Name | best_pv |
|---|---|---|---|
| 22 | NC_011743 | Escherichia fergusonii ATCC 35469 plasmid pEFER: NC_011743 | 6.18164438611072e-13 |
| 23 | NZ_ADUT01000048 | Shigella dysenteriae 1617 gss1617.assembly.53: NZ_ADUT01000048 | 6.40162543140323e-12 |
| 24 | NZ_ADUT01000002 | Shigella dysenteriae 1617 gss1617.assembly.11: NZ_ADUT01000002 | 1.53869362793954e-11 |
| 25 | NC_011745 | Escherichia coli ED1a: NC_011745 | 2.16302499579491e-11 |
| 26 | NZ_ADUT01000002 | Shigella dysenteriae 1617 gss1617.assembly.11: NZ_ADUT01000002 | 2.30761086733495e-11 |
| 27 | NZ_ABKY02000005 | Escherichia coli O157:H7 str. TW14588, unfinished sequence: NZ_ABKY02000005 | 4.43701528503845e-11 |
| 28 | NZ_ADUT01000002 | Shigella dysenteriae 1617 gss1617.assembly.11: NZ_ADUT01000002 | 8.04473895167901e-11 |
| 29 | CP002729 | Escherichia coli UMNK88: CP002729 | 1.42165494676851e-10 |
| 30 | NC_011742 | Escherichia coli S88: NC_011742 | 2.05879614239143e-10 |
| 31 | NZ_ABAK01000021 | Salmonella enterica subsp. enterica serovar Kentucky str. CVM29188, unfinished sequence: NZ_ABAK01000021 | 2.13757750700881e-10 |
| 32 | NC_013509 | Edwardsiella tarda EIB202 plasmid pEIB202: NC_013509 | 4.04449595701974e-10 |
| 33 | NZ_ABHS01000025 | Escherichia coli O157:H7 str. EC4486, unfinished sequence: NZ_ABHS01000025 | 4.85503165975477e-10 |
| 34 | NC_012759 | Escherichia coli BW2952: NC_012759 | 4.85503165975477e-10 |
| 35 | NZ_ADUD01000718 | Escherichia coli MS 196-1 E_coli196-1-1.0_Cont2124.2: NZ_ADUD01000718 | 5.8673304681529e-10 |
| 36 | NC_002655 | Escherichia coli O157:H7 EDL933: NC_002655 | 7.67538561798816e-10 |
| 37 | CP001509 | Escherichia coli BL21(DE3): CP001509 | 8.69490265401296e-10 |
| 38 | FP929040 | Enterobacter cloacae subsp. cloacae NCTC 9394 draft genome.: FP929040 | 1.59741374269166e-09 |
| 39 | NZ_AFBO01000392 | Klebsiella sp. MS 92-3 K_spMS92-3-1.0_Cont773.2: NZ_AFBO01000392 | 1.59741374269166e-09 |
| 40 | NZ_ADTQ01000190 | Escherichia coli MS 187-1 E_coli187-1-1.0_Cont287.1: NZ_ADTQ01000190 | 1.59741374269166e-09 |
| 41 | NZ_ACZD01000015 | Klebsiella pneumoniae subsp. rhinoscleromatis ATCC 13884 contig00015: NZ_ACZD01000015 | 1.59741374269166e-09 |
| 42 | NC_007384 | Shigella sonnei Ss046: NC_007384 | 1.59741374269166e-09 |
| 43 | NZ_ADUT01000040 | Shigella dysenteriae 1617 gss1617.assembly.46: NZ_ADUT01000040 | 1.75588873952222e-09 |
| 44 | NZ_CAAZ01000109 | Salmonella enterica subsp. enterica serovar Typhi str. E98-3139, unfinished sequence: NZ_CAAZ01000109 | 4.20666708065652e-09 |
| 45 | NZ_AAMJ01000012 | Shigella dysenteriae 1012, unfinished sequence: NZ_AAMJ01000012 | 5.072437403203e-09 |
| 46 | NC_010658 | Shigella boydii CDC 3083-94: NC_010658 | 8.52438957707961e-09 |
| 47 | FN554766 | Escherichia coli 042: FN554766 | 8.7610944464379e-09 |

(continued)

| No. | Scaffold.External. Accession | Scaffold.Name | best_pv |
|---|---|---|---|
| 48 | NZ_ ADTL01000219 | Escherichia coli MS 115-1 E_coli115-1-1.0_Cont569.1: NZ_ADTL01000219 | 8.7610944464379e-09 |
| 49 | NZ_ ABJT01000012 | Escherichia coli O157:H7 str. EC4024, unfinished sequence: NZ_ABJT01000012 | 9.3358254121691e-09 |
| 50 | NZ_ AAJV01000016 | Escherichia coli E22, unfinished sequence: NZ_ AAJV01000016 | 1.17232056049228e-08 |

Table 21b: Data for Shigella species (continued)

| No. | sign_phenos | sign_phenos_class | best_pheno | best_pheno_class |
|---|---|---|---|---|
| 1 | AM;A/S;AUG;T/S | lactam;other | AUG | lactam |
| 2 | AM;A/S;AUG;T/S | lactam;other | AUG | lactam |
| 3 | AM;A/S;AUG;T/S | lactam;other | AUG | lactam |
| 4 | AM;A/S;AUG;T/S | lactam;other | AUG | lactam |
| 5 | AM;A/S;AUG | lactam | AUG | lactam |
| 6 | T/S | other | T/S | other |
| 7 | T/S | other | T/S | other |
| 8 | T/S | other | T/S | other |
| 9 | T/S | other | T/S | other |
| 10 | T/S | other | T/S | other |
| 11 | AM;A/S;AUG;T/S | lactam;other | AUG | lactam |
| 12 | AM;A/S;AUG;T/S | lactam;other | AUG | lactam |
| 13 | A/S;AUG;T/S | lactam;other | T/S | other |
| 14 | AM;A/S;AUG;T/S | lactam;other | AUG | lactam |
| 15 | AM;A/S;AUG;T/S | lactam;other | AUG | lactam |
| 16 | T/S | other | T/S | other |
| 17 | AM;A/S | lactam | A/S | lactam |
| 18 | AM;A/S;AUG | lactam | AUG | lactam |
| 19 | AM;A/S | lactam | A/S | lactam |
| 20 | AM;A/S | lactam | A/S | lactam |
| 21 | AM;A/S | lactam | A/S | lactam |
| 22 | T/S | other | T/S | other |
| 23 | AM;A/S;AUG | lactam | AUG | lactam |
| 24 | AM;A/S | lactam | A/S | lactam |
| 25 | AM;A/S;AUG;T/S | lactam;other | AM | lactam |
| 26 | AM;A/S | lactam | A/S | lactam |
| 27 | AM;A/S;AUG | lactam | A/S | lactam |
| 28 | AM;A/S | lactam | A/S | lactam |
| 29 | AUG | lactam | AUG | lactam |

(continued)

| No. | sign_phenos | sign_phenos_class | best_pheno | best_pheno_class |
|---|---|---|---|---|
| 30 | T/S | other | T/S | other |
| 31 | AM;A/S | lactam | A/S | lactam |
| 32 | T/S | other | T/S | other |
| 33 | AM;A/S | lactam | A/S | lactam |
| 34 | AM;A/S | lactam | AM | lactam |
| 35 | AM;A/S | lactam | A/S | lactam |
| 36 | AUG | lactam | AUG | lactam |
| 37 | AM;A/S | lactam | AM | lactam |
| 38 | AM;A/S | lactam | AM | lactam |
| 39 | AM;A/S | lactam | AM | lactam |
| 40 | AM;A/S | lactam | AM | lactam |
| 41 | AM;A/S | lactam | AM | lactam |
| 42 | AM;A/S | lactam | AM | lactam |
| 43 | AUG | lactam | AUG | lactam |
| 44 | AUG | lactam | AUG | lactam |
| 45 | AUG | lactam | AUG | lactam |
| 46 | AUG | lactam | AUG | lactam |
| 47 | AUG | lactam | AUG | lactam |
| 48 | AUG | lactam | AUG | lactam |
| 49 | AM;A/S | lactam | AM | lactam |
| 50 | AM;A/S;T/S | lactam;other | AM | lactam |

Table 21c: Data for Shigella species (continued)

| No. | num_lactam | num_other | AM_pv_adj | A/S_pv_adj | AUG_pv_adj | T/S_pv_adj |
|---|---|---|---|---|---|---|
| 1 | 3 | 1 | 2.50E-24 | 2.78E-27 | 6.87E-56 | 6.43E-03 |
| 2 | 3 | 1 | 3.96E-18 | 5.18E-21 | 4.15E-46 | 3.89E-03 |
| 3 | 3 | 1 | 1.61E-18 | 1.80E-21 | 4.15E-46 | 2.75E-03 |
| 4 | 3 | 1 | 1.48E-18 | 1.80E-21 | 1.24E-44 | 5.45E-03 |
| 5 | 3 | 0 | 6.38E-20 | 1.76E-12 | 5.70E-37 | 1.36E-02 |
| 6 | 0 | 1 | 4.19E-01 | 7.59E-01 | 1.18E-01 | 3.04E-34 |
| 7 | 0 | 1 | 4.19E-01 | 7.59E-01 | 1.18E-01 | 3.04E-34 |
| 8 | 0 | 1 | 4.14E-01 | 6.88E-01 | 1.42E-01 | 5.68E-34 |
| 9 | 0 | 1 | 5.86E-01 | 9.62E-01 | 1.89E-01 | 1.47E-26 |
| 10 | 0 | 1 | 6.64E-01 | 8.98E-01 | 3.47E-01 | 4.98E-26 |
| 11 | 3 | 1 | 1.30E-12 | 6.35E-09 | 1.31E-24 | 1.10E-04 |
| 12 | 3 | 1 | 8.43E-13 | 4.07E-09 | 1.67E-24 | 3.04E-05 |
| 13 | 2 | 1 | 5.59E-02 | 5.39E-03 | 8.83E-03 | 3.08E-24 |

(continued)

| No. | num_lactam | num_other | AM_pv_adj | A/S_pv_adj | AUG_pv_adj | T/S_pv_adj |
|-----|-----------|-----------|-----------|------------|------------|------------|
| 14 | 3 | 1 | 6.77E-13 | 3.67E-09 | 3.45E-24 | 4.57E-05 |
| 15 | 3 | 1 | 1.11E-11 | 1.11E-08 | 4.84E-23 | 3.29E-12 |
| 16 | 0 | 1 | 7.13E-01 | 9.56E-01 | 8.48E-02 | 1.60E-17 |
| 17 | 2 | 0 | 1.56E-14 | 4.67E-17 | 6.49E-01 | 1.06E-01 |
| 18 | 3 | 0 | 7.73E-05 | 2.34E-06 | 5.88E-17 | 9.61E-01 |
| 19 | 2 | 0 | 5.63E-14 | 7.05E-16 | 9.63E-01 | 3.40E-02 |
| 20 | 2 | 0 | 4.77E-14 | 3.50E-15 | 1 | 7.62E-02 |
| 21 | 2 | 0 | 3.45E-11 | 3.88E-13 | 6.47E-01 | 3.82E-02 |
| 22 | 0 | 1 | 5.80E-01 | 2.16E-01 | 2.14E-01 | 6.18E-13 |
| 23 | 3 | 0 | 5.45E-03 | 1.12E-03 | 6.40E-12 | 1.67E-01 |
| 24 | 2 | 0 | 2.67E-09 | 1.54E-11 | 4.21E-01 | 7.26E-02 |
| 25 | 3 | 1 | 2.16E-11 | 8.03E-10 | 4.80E-05 | 1.95E-03 |
| 26 | 2 | 0 | 3.72E-09 | 2.31E-11 | 5.37E-01 | 1.18E-01 |
| 27 | 3 | 0 | 2.93E-08 | 4.44E-11 | 1.86E-03 | 1.59E-01 |
| 28 | 2 | 0 | 7.34E-09 | 8.04E-11 | 6.55E-01 | 7.07E-02 |
| 29 | 1 | 0 | 2.46E-01 | 5.71E-02 | 1.42E-10 | 5.27E-01 |
| 30 | 0 | 1 | 1.01E-01 | 3.04E-02 | 5.99E-01 | 2.06E-10 |
| 31 | 2 | 0 | 7.76E-09 | 2.14E-10 | 3.71E-01 | 1.76E-01 |
| 32 | 0 | 1 | 6.37E-01 | 2.87E-01 | 2.49E-01 | 4.04E-10 |
| 33 | 2 | 0 | 5.22E-08 | 4.86E-10 | 2.71E-01 | 2.09E-01 |
| 34 | 2 | 0 | 4.86E-10 | 3.14E-08 | 5.20E-02 | 5.27E-01 |
| 35 | 2 | 0 | 1.90E-08 | 5.87E-10 | 4.20E-01 | 1.73E-01 |
| 36 | 1 | 0 | 1.02E-01 | 2.02E-02 | 7.68E-10 | 3.60E-01 |
| 37 | 2 | 0 | 8.69E-10 | 5.22E-08 | 6.38E-02 | 5.96E-01 |
| 38 | 2 | 0 | 1.60E-09 | 8.74E-08 | 6.39E-02 | 5.96E-01 |
| 39 | 2 | 0 | 1.60E-09 | 8.74E-08 | 6.39E-02 | 5.96E-01 |
| 40 | 2 | 0 | 1.60E-09 | 8.74E-08 | 6.39E-02 | 5.96E-01 |
| 41 | 2 | 0 | 1.60E-09 | 8.74E-08 | 6.39E-02 | 5.96E-01 |
| 42 | 2 | 0 | 1.60E-09 | 8.74E-08 | 6.39E-02 | 5.96E-01 |
| 43 | 1 | 0 | 4.98E-01 | 1.34E-01 | 1.76E-09 | 3.44E-01 |
| 44 | 1 | 0 | 5.00E-01 | 1.36E-01 | 4.21E-09 | 4.06E-01 |
| 45 | 1 | 0 | 3.20E-01 | 7.93E-02 | 5.07E-09 | 6.63E-01 |
| 46 | 1 | 0 | 1 | 5.81E-01 | 8.52E-09 | 4.31E-01 |
| 47 | 1 | 0 | 2.42E-01 | 8.26E-02 | 8.76E-09 | 5.20E-01 |
| 48 | 1 | 0 | 2.42E-01 | 8.26E-02 | 8.76E-09 | 5.20E-01 |
| 49 | 2 | 0 | 9.34E-09 | 1.80E-06 | 2.16E-01 | 7.92E-02 |
| 50 | 2 | 1 | 1.17E-08 | 4.60E-05 | 3.74E-02 | 7.92E-04 |

Table 21d: Data for Shigella species (continued)

| No. | Locus.Tag | Gene.Symbol | GenBank.Accession | Chromosome |
|---|---|---|---|---|
| 1 | HMPREF9547_01191 | | ZP_07167682 | |
| 2 | HMPREF9551_04221 | | ZP_07191576 | |
| 3 | HMPREF9347_01086 | | ZP_07208640 | |
| 4 | STMDT12_C39170 | | BAJ38860 | |
| 5 | SFxv_1168 | | ADA73425 | |
| 6 | Sdys1_01000012 | | ZP_00922750 | |
| 7 | HMPREF9547_ 02735 | | ZP_07169192 | |
| 8 | Sdys1_01000013 | | ZP_00922751 | |
| 9 | SFxv_414 6 | | ADA76106 | |
| 10 | HMPREF9547_ 04432 | | ZP_07170853 | |
| 11 | HMPREF9547_ 00721 | | ZP_07167228 | |
| 12 | SFxv_1141 | | ADA73399 | |
| 13 | ETAE_p041 | folP | YP_003297635 | plasmid pEIB202 |
| 14 | ESCAB7627 _ 0644 | | ZP_02900823 | |
| 15 | SSON_3896 | aadA | YP_312670 | |
| 16 | SFxv_4148 | | ADA76108 | |
| 17 | SD1617_0118 | | ZP_ 07678322 | |
| 18 | ESCAB7627_ 0650 | | ZP_02900555 | |
| 19 | HMPREF9530_04295 | | ZP_07154150 | |
| 20 | SD1617_0032 | | ZP_07678236 | |
| 21 | SC109 | ccgA2 | YP_209411 | plasmid pSC138 |
| 22 | pEFER_0049 | | YP_002394596 | plasmid pEFER |
| 23 | SD1617_4863 | | ZP_07682961 | |
| 24 | SD1617_0123 | | ZP_07678327 | |
| 25 | ECED1_1050 | | YP_002397074 | |
| 26 | SD1617_0043 | | ZP_07678247 | |
| 27 | ESCC014588_3315 | | ZP_03445386 | |
| 28 | SD1617_0122 | | ZP_07678326 | |
| 29 | UMNK88_1471 | | AEE56075 | |
| 30 | ECS88_2899 | rusA | YP_002392541 | |
| 31 | Sente_010100023234 | | ZP_02563153 | |
| 32 | ETAE_p040 | | YP_003297634 | plasmid pEIB202 |
| 33 | EcolO157_010100025333 | | ZP_02795604 | |
| 34 | BWG_3989 | fecR | YP_002929180 | |
| 35 | HMPREF9551_05588 | | ZP_07192915 | |
| 36 | Z1242 | dmsC | NP_286773 | |
| 37 | ECD_04153 | fecE | ACT45937 | |

(continued)

| No. | Locus.Tag | Gene.Symbol | GenBank.Accession | Chromosome |
|-----|-----------|-------------|-------------------|------------|
| 38 | ENC_36610 | | CBK86965 | |
| 39 | HMPREF9538_03067 | | ZP_08305382 | |
| 40 | HMPREF9550_02956 | | ZP_07146070 | |
| 41 | HMPREF0484_0234 | fecA | ZP_06013219 | |
| 42 | SSON_4460 | fecD | YP_313172 | |
| 43 | SD1617_4069 | | ZP_07682183 | |
| 44 | Salmonellentericaenterica_010100012935 | | ZP_03412076 | |
| 45 | Sdys1_01002088 | | ZP_00921058 | |
| 46 | SbBS512_E0266 | | YP_001879059 | |
| 47 | EC042_2952 | | CBG35781 | |
| 48 | HMPREF9540_02755 | | ZP_07135552 | |
| 49 | Escherichcoli0157_010100012850 | | ZP_03082702 | |
| 50 | EcolE2_01002516 | | ZP_00729149 | |

Table 21e: Data for Shigella species (continued)

| No. | Start.Coord | End.Coord | Strand | Scaffold.ID |
|-----|-------------|-----------|--------|-------------|
| 1 | 6987 | 7817 | + | 648291751 |
| 2 | 10102 | 10662 | - | 648292991 |
| 3 | 2726 | 5692 | - | 648294279 |
| 4 | 4067283 | 4067942 | + | 651053007 |
| 5 | 1128245 | 1129249 | + | 646862363 |
| 6 | 12375 | 12701 | - | 638365528 |
| 7 | 4692 | 5189 | - | 648291885 |
| 8 | 14252 | 14776 | + | 638365528 |
| 9 | 3950954 | 3952570 | - | 646862363 |
| 10 | 8898 | 9719 | - | 648292023 |
| 11 | 5423 | 6016 | - | 648291712 |
| 12 | 1107342 | 1107968 | + | 646862363 |
| 13 | 32101 | 32916 | - | 646311959 |
| 14 | 233492 | 234697 | - | 641784575 |
| 15 | 4110458 | 4111246 | - | 640427102 |
| 16 | 3954100 | 3955767 | - | 646862363 |
| 17 | 94539 | 95570 | + | 649994652 |
| 18 | 238788 | 239996 | - | 641784575 |
| 19 | 2693 | 3169 | + | 648290614 |
| 20 | 9270 | 9803 | - | 649994652 |
| 21 | 84616 | 85080 | - | 637000354 |

(continued)

| No. | Start.Coord | End.Coord | Strand | Scaffold.ID |
|---|---|---|---|---|
| 22 | 48658 | 49503 | + | 643692014 |
| 23 | 80678 | 81835 | - | 649994698 |
| 24 | 98900 | 99016 | - | 649994652 |
| 25 | 1066134 | 1066832 | + | 643348624 |
| 26 | 17832 | 18008 | + | 649994652 |
| 27 | 383590 | 384330 | - | 642987534 |
| 28 | 98413 | 98754 | - | 649994652 |
| 29 | 1420478 | 1421209 | - | 651053088 |
| 30 | 2863316 | 2863705 | - | 644736332 |
| 31 | 17544 | 17792 | + | 641746180 |
| 32 | 31237 | 32040 | - | 646311959 |
| 33 | 65402 | 65668 | - | 641781914 |
| 34 | 4453522 | 4454475 | - | 644736346 |
| 35 | 1327 | 1764 | - | 648293234 |
| 36 | 1164368 | 1165231 | + | 637000062 |
| 37 | 4424087 | 4424854 | - | 646862369 |
| 38 | 3698592 | 3699494 | - | 650378029 |
| 39 | 2039 | 3037 | - | 651333309 |
| 40 | 271 | 792 | + | 648290104 |
| 41 | 4245 | 6485 | + | 647009762 |
| 42 | 4731572 | 4732528 | - | 640427102 |
| 43 | 500702 | 501520 | + | 649994690 |
| 44 | 43535 | 45085 | + | 643042589 |
| 45 | 19505 | 20209 | - | 638365539 |
| 46 | 266116 | 266985 | - | 641522725 |
| 47 | 3140492 | 3141238 | - | 646862421 |
| 48 | 1421 | 1912 | - | 648288078 |
| 49 | 103370 | 103972 | + | 642792886 |
| 50 | 35676 | 36281 | - | 638358744 |

Table 22a: Data for Shigella species

| No. | Scaffold.External. Accession | Scaffold.Name | best_pv |
|---|---|---|---|
| 1 | NZ_ ADUB01000093 | Escherichia coli MS 175-1 E_coli175-1-1.0_Cont166.1: NZ_ADUB01000093 | 6.86676649351252e-56 |
| 2 | NZ_ ADUD01000475 | Escherichia coli MS 196-1 E_coli196-1-1.0_Cont1343.1: NZ_ADUD01000475 | 4.14817893591733e-46 |
| 3 | NZ_ ADWT01000006 | Escherichia coli MS 124-1 E_coliMS124-1-1.0.1_Cont5.1: NZ_ADWT01000006 | 4.14817893591733e-46 |

(continued)

| No. | Scaffold.External. Accession | Scaffold.Name | best_pv |
|---|---|---|---|
| 4 | AP011957 | Salmonella enterica subsp. enterica serovar Typhimurium str. T000240 DNA: AP011957 | 1.24332752332433e-44 |
| 5 | CP001383 | Shigella flexneri 2002017: CP001383 | 5.69762827081762e-37 |
| 6 | NZ_ AAMJ01000001 | Shigella dysenteriae 1012, unfinished sequence: NZ_ AAMJ01000001 | 3.04086824044829e-34 |
| 7 | NZ_ ADUB01000227 | Escherichia coli MS 175-1 E_coli175-1-1.0_Cont422.2: NZ_ADUB01000227 | 3.04086824044829e-34 |
| 8 | NZ_ AAMJ01000001 | Shigella dysenteriae 1012, unfinished sequence: NZ_ AAMJ01000001 | 5.67507153238613e-34 |
| 9 | CP001383 | Shigella flexneri 2002017: CP001383 | 1.46728061376833e-26 |
| 10 | NZ_ ADUB01000365 | Escherichia coli MS 175-1 E_coli175-1-1.0_Cont823.2: NZ_ADUB01000365 | 4.97695865217724e-26 |
| 11 | NZ_ ADUB01000054 | Escherichia coli MS 175-1 E_coli175-1-1.0_Cont100.1: NZ_ADUB01000054 | 1.31351033204593e-24 |
| 12 | CP001383 | Shigella flexneri 2002017: CP001383 | 1.66700615646725e-24 |
| 13 | NC_013509 | Edwardsiella tarda EIB202 plasmid pEIB202: NC_013509 | 3.081692811393e-24 |
| 14 | NZ_ ABKX01000001 | Escherichia albertii TW07627, unfinished sequence: NZ_ ABKX01000001 | 3.45432516278107e-24 |
| 15 | NC_007384 | Shigella sonnei Ss046: NC_007384 | 4.84345172785563e-23 |
| 16 | CP001383 | Shigella flexneri 2002017: CP001383 | 1.59570448713613e-17 |
| 17 | NZ_ ABKX01000001 | Escherichia albertii TW07627, unfinished sequence: NZ_ ABKX01000001 | 5.88113462200155e-17 |
| 18 | NC_011743 | Escherichia fergusonii ATCC 35469 plasmid pEFER: NC_ 011743 | 6.18164438611072e-13 |
| 19 | NZ_ ADUT01000048 | Shigella dysenteriae 1617 gss1617.assembly.53: NZ_ ADUT01000048 | 6.40162543140323e-12 |
| 20 | NC_011745 | Escherichia coli ED1a: NC_011745 | 2.16302499579491e-11 |
| 21 | NZ_ ABKY02000005 | Escherichia coli O157:H7 str. TW14588, unfinished sequence: NZ_ABKY02000005 | 4.43701528503845e-11 |
| 22 | NC_011742 | Escherichia coli S88: NC_011742 | 2.05879614239143e-10 |
| 23 | NC_013509 | Edwardsiella tarda EIB202 plasmid pEIB202: NC_013509 | 4.04449595701974e-10 |
| 24 | NZ_ AAJV01000016 | Escherichia coli E22, unfinished sequence: NZ_ AAJV01000016 | 1.17232056049228e-08 |
| 25 | NC_012731 | Klebsiella pneumoniae NTUH-K2044: NC_012731 | 1.74593394018129e-08 |
| 26 | NC_011740 | Escherichia fergusonii ATCC 35469: NC_011740 | 2.08793524887857e-08 |
| 27 | NC_011740 | Escherichia fergusonii ATCC 35469: NC_011740 | 3.5987076197447e-08 |
| 28 | NZ_GG749327 | Escherichia coli B354 genomic scaffold supercont1.2: NZ_ GG749327 | 3.5987076197447e-08 |
| 29 | NZ_ ADTP01000357 | Escherichia coli MS 69-1 E_coli69-1-1.0_Cont727.1: NZ_ ADTP01000357 | 3.5987076197447e-08 |
| 30 | NC_008258 | Shigella flexneri 5 str. 8401: NC_008258 | 3.5987076197447e-08 |

(continued)

| No. | Scaffold.External. Accession | Scaffold.Name | best_pv |
|---|---|---|---|
| 31 | NC_008258 | Shigella flexneri 5 str. 8401: NC_008258 | 3.6014669209108e-08 |
| 32 | NC_011093 | Salmonella enterica subsp. enterica serovar Schwarzengrund str. CVM19633 plasmid pCVM19633_4: NC_011093 | 4.21633659816666e-08 |
| 33 | NZ_ABWL01000002 | Citrobacter youngae ATCC 29220 C_sp-1.0_Cont0.2: NZ_ABWL01000002 | 5.87968902033994e-08 |
| 34 | NC_011740 | Escherichia fergusonii ATCC 35469: NC_011740 | 5.87968902033994e-08 |
| 35 | NZ_ADUT01000013 | Shigella dysenteriae 1617 gss1617.assembly.21: NZ_ADUT01000013 | 5.87968902033994e-08 |
| 36 | NC_010658 | Shigella boydii CDC 3083-94: NC_010658 | 5.87968902033994e-08 |
| 37 | NC_011740 | Escherichia fergusonii ATCC 35469: NC_011740 | 5.87968902033994e-08 |
| 38 | NZ_ABEJ01000032 | Salmonella enterica subsp. enterica serovar Schwarzengrund str. SL480, unfinished sequence: NZ_ABEJ01000032 | 6.12813910192695e-08 |
| 39 | NC_009345 | Shigella sonnei Ss046 plasmid pSS046_spA: NC_009345 | 7.76943438061442e-08 |
| 40 | NC_011740 | Escherichia fergusonii ATCC 35469: NC_011740 | 8.81464918585737e-08 |
| 41 | NZ_ABKX01000001 | Escherichia albertii TW07627, unfinished sequence: NZ_ABKX01000001 | 1.19080570281449e-07 |
| 42 | CP001671 | Escherichia coli ABU 83972: CP001671 | 1.50273530777206e-07 |
| 43 | CP001637 | Escherichia coli DH1: CP001637 | 2.0405961424353e-07 |
| 44 | NC_004741 | Shigella flexneri 2a str. 2457T: NC_004741 | 2.0405961424353e-07 |
| 45 | NZ_AAKB01000013 | Escherichia coli 53638, unfinished sequence: NZ_AAKB01000013 | 2.08917442901333e-07 |
| 46 | NZ_ADUL01000041 | Escherichia coli 2362-75 gec2362.assembly.47: NZ_ADUL01000041 | 2.54562372489639e-07 |
| 47 | NZ_ABKX01000001 | Escherichia albertii TW07627, unfinished sequence: NZ_ABKX01000001 | 3.82958267583033e-07 |
| 48 | NZ_ADTR01000289 | Escherichia coli MS 21-1 E_coli21-1-1.0_Cont515.2: NZ_ADTR01000289 | 3.97520776634592e-07 |
| 49 | NC_010658 | Shigella boydii CDC 3083-94: NC_010658 | 4.00202730594618e-07 |
| 50 | NZ_GG657366 | Citrobacter sp. 30_2 genomic scaffold supercont1.1: NZ_GG657366 | 4.18196454987758e-07 |

Table 22b: Data for Shigella species

| No. | sign_phenos | sign_phenos_class | best_pheno | best_pheno_class |
|---|---|---|---|---|
| 1 | AM;A/S;AUG;T/S | lactam;other | AUG | lactam |
| 2 | AM;A/S;AUG;T/S | lactam;other | AUG | lactam |
| 3 | AM;A/S;AUG;T/S | lactam;other | AUG | lactam |
| 4 | AM;A/S;AUG;T/S | lactam;other | AUG | lactam |
| 5 | AM;A/S;AUG | lactam | AUG | lactam |

(continued)

| No. | sign_phenos | sign_phenos_class | best_pheno | best_pheno_class |
|-----|-------------|-------------------|------------|------------------|
| 6 | T/S | other | T/S | other |
| 7 | T/S | other | T/S | other |
| 8 | T/S | other | T/S | other |
| 9 | T/S | other | T/S | other |
| 10 | T/S | other | T/S | other |
| 11 | AM;A/S;AUG;T/S | lactam;other | AUG | lactam |
| 12 | AM;A/S;AUG;T/S | lactam;other | AUG | lactam |
| 13 | A/S;AUG;T/S | lactam;other | T/S | other |
| 14 | AM;A/S;AUG;T/S | lactam;other | AUG | lactam |
| 15 | AM;A/S;AUG;T/S | lactam;other | AUG | lactam |
| 16 | T/S | other | T/S | other |
| 17 | AM;A/S;AUG | lactam | AUG | lactam |
| 18 | T/S | other | T/S | other |
| 19 | AM;A/S;AUG | lactam | AUG | lactam |
| 20 | AM;A/S;AUG;T/S | lactam;other | AM | lactam |
| 21 | AM;A/S;AUG | lactam | A/S | lactam |
| 22 | T/S | other | T/S | other |
| 23 | T/S | other | T/S | other |
| 24 | AM;A/S;T/S | lactam;other | AM | lactam |
| 25 | AM;A/S;T/S | lactam;other | AM | lactam |
| 26 | AM;A/S;AUG | lactam | AM | lactam |
| 27 | AM;A/S;T/S | lactam;other | AM | lactam |
| 28 | AM;A/S;T/S | lactam;other | AM | lactam |
| 29 | AM;A/S;T/S | lactam;other | AM | lactam |
| 30 | AM;A/S;T/S | lactam;other | AM | lactam |
| 31 | AM;A/S;AUG | lactam | AUG | lactam |
| 32 | AM;A/S;AUG | lactam | AM | lactam |
| 33 | AM;A/S;T/S | lactam;other | A/S | lactam |
| 34 | AM;A/S;T/S | lactam;other | A/S | lactam |
| 35 | AM;A/S;T/S | lactam;other | A/S | lactam |
| 36 | AM;A/S;T/S | lactam;other | A/S | lactam |
| 37 | AM;A/S;T/S | lactam;other | A/S | lactam |
| 38 | AM;A/S;AUG;T/S | lactam;other | T/S | other |
| 39 | AM;A/S;AUG;T/S | lactam;other | T/S | other |
| 40 | AM;A/S;AUG;T/S | lactam;other | AUG | lactam |
| 41 | AM;A/S;AUG | lactam | A/S | lactam |
| 42 | AM;A/S;T/S | lactam;other | AM | lactam |
| 43 | AM;A/S;AUG | lactam | A/S | lactam |

(continued)

| 10 | T/S | other | T/S | other |
|---|---|---|---|---|
| 44 | AM;A/S;AUG | lactam | A/S | lactam |
| 45 | AM;A/S;AUG | lactam | AM | lactam |
| 46 | AM;A/S;AUG;T/S | lactam;other | A/S | lactam |
| 47 | AM;A/S;AUG | lactam | A/S | lactam |
| 48 | AM;A/S;T/S | lactam;other | AM | lactam |
| 49 | AM;A/S;T/S | lactam;other | T/S | other |
| 50 | AM;A/S;AUG | lactam | AM | lactam |

Table 22c: Data for Shigella species

| No. | num_lactam | num_other | AM_pv_adj | A/S_pv_adj | AUG_pv_adj | T/S_pv_adj |
|---|---|---|---|---|---|---|
| 1 | 3 | 1 | 2.50E-24 | 2.78E-27 | 6.87E-56 | 6.43E-03 |
| 2 | 3 | 1 | 3.96E-18 | 5.18E-21 | 4.15E-46 | 3.89E-03 |
| 3 | 3 | 1 | 1.61E-18 | 1.80E-21 | 4.15E-46 | 2.75E-03 |
| 4 | 3 | 1 | 1.48E-18 | 1.80E-21 | 1.24E-44 | 5.45E-03 |
| 5 | 3 | 0 | 6.38E-20 | 1.76E-12 | 5.70E-37 | 1.36E-02 |
| 6 | 0 | 1 | 4.19E-01 | 7.59E-01 | 1.18E-01 | 3.04E-34 |
| 7 | 0 | 1 | 4.19E-01 | 7.59E-01 | 1.18E-01 | 3.04E-34 |
| 8 | 0 | 1 | 4.14E-01 | 6.88E-01 | 1.42E-01 | 5.68E-34 |
| 9 | 0 | 1 | 5.86E-01 | 9.62E-01 | 1.89E-01 | 1.47E-26 |
| 10 | 0 | 1 | 6.64E-01 | 8.98E-01 | 3.47E-01 | 4.98E-26 |
| 11 | 3 | 1 | 1.30E-12 | 6.35E-09 | 1.31E-24 | 1.10E-04 |
| 12 | 3 | 1 | 8.43E-13 | 4.07E-09 | 1.67E-24 | 3.04E-05 |
| 13 | 2 | 1 | 5.59E-02 | 5.39E-03 | 8.83E-03 | 3.08E-24 |
| 14 | 3 | 1 | 6.77E-13 | 3.67E-09 | 3.45E-24 | 4.57E-05 |
| 15 | 3 | 1 | 1.11E-11 | 1.11E-08 | 4.84E-23 | 3.29E-12 |
| 16 | 0 | 1 | 7.13E-01 | 9.56E-01 | 8.48E-02 | 1.60E-17 |
| 17 | 3 | 0 | 7.73E-05 | 2.34E-06 | 5.88E-17 | 9.61E-01 |
| 18 | 0 | 1 | 5.80E-01 | 2.16E-01 | 2.14E-01 | 6.18E-13 |
| 19 | 3 | 0 | 5.45E-03 | 1.12E-03 | 6.40E-12 | 1.67E-01 |
| 20 | 3 | 1 | 2.16E-11 | 8.03E-10 | 4.80E-05 | 1.95E-03 |
| 21 | 3 | 0 | 2.93E-08 | 4.44E-11 | 1.86E-03 | 1.59E-01 |
| 22 | 0 | 1 | 1.01E-01 | 3.04E-02 | 5.99E-01 | 2.06E-10 |
| 23 | 0 | 1 | 6.37E-01 | 2.87E-01 | 2.49E-01 | 4.04E-10 |
| 24 | 2 | 1 | 1.17E-08 | 4.60E-05 | 3.74E-02 | 7.92E-04 |
| 25 | 2 | 1 | 1.75E-08 | 3.59E-04 | 9.59E-02 | 5.74E-05 |
| 26 | 3 | 0 | 2.09E-08 | 6.13E-08 | 3.86E-05 | 4.21E-01 |
| 27 | 2 | 1 | 3.60E-08 | 2.62E-04 | 7.92E-02 | 9.64E-04 |

(continued)

| No. | num_lactam | num_other | AM_pv_adj | A/S_pv_adj | AUG_pv_adj | T/S_pv_adj |
|---|---|---|---|---|---|---|
| 28 | 2 | 1 | 3.60E-08 | 5.37E-04 | 7.92E-02 | 1.59E-04 |
| 29 | 2 | 1 | 3.60E-08 | 2.62E-04 | 7.92E-02 | 9.64E-04 |
| 30 | 2 | 1 | 3.60E-08 | 5.37E-04 | 7.92E-02 | 1.59E-04 |
| 31 | 3 | 0 | 6.06E-04 | 1.15E-05 | 3.60E-08 | 1.18E-02 |
| 32 | 3 | 0 | 4.22E-08 | 6.96E-07 | 1.35E-04 | 3.01E-02 |
| 33 | 2 | 1 | 2.70E-07 | 5.88E-08 | 1.78E-01 | 7.31E-04 |
| 34 | 2 | 1 | 2.70E-07 | 5.88E-08 | 1.78E-01 | 7.31E-04 |
| 35 | 2 | 1 | 2.70E-07 | 5.88E-08 | 1.78E-01 | 7.31E-04 |
| 36 | 2 | 1 | 2.70E-07 | 5.88E-08 | 1.78E-01 | 7.31E-04 |
| 37 | 2 | 1 | 2.70E-07 | 5.88E-08 | 1.78E-01 | 7.31E-04 |
| 38 | 3 | 1 | 3.43E-04 | 1.83E-03 | 7.05E-04 | 6.13E-08 |
| 39 | 3 | 1 | 8.13E-05 | 5.17E-04 | 3.23E-04 | 7.77E-08 |
| 40 | 3 | 1 | 2.18E-04 | 3.33E-06 | 8.81E-08 | 1.34E-03 |
| 41 | 3 | 0 | 5.77E-06 | 1.19E-07 | 6.46E-03 | 3.28E-01 |
| 42 | 2 | 1 | 1.50E-07 | 1.17E-03 | 6.39E-02 | 1.05E-04 |
| 43 | 3 | 0 | 1.24E-05 | 2.04E-07 | 6.60E-03 | 3.27E-01 |
| 44 | 3 | 0 | 1.24E-05 | 2.04E-07 | 6.60E-03 | 3.27E-01 |
| 45 | 3 | 0 | 2.09E-07 | 1.92E-06 | 4.75E-05 | 4.59E-01 |
| 46 | 3 | 1 | 1.84E-04 | 2.55E-07 | 1.33E-06 | 3.22E-03 |
| 47 | 3 | 0 | 1.32E-05 | 3.83E-07 | 8.66E-03 | 2.79E-01 |
| 48 | 2 | 1 | 3.98E-07 | 1.01E-05 | 1.08E-01 | 3.36E-03 |
| 49 | 2 | 1 | 3.82E-05 | 1.71E-03 | 2.98E-01 | 4.00E-07 |
| 50 | 3 | 0 | 4.18E-07 | 3.44E-06 | 2.44E-03 | 1.37E-01 |

Table 22d: Data for Shigella species

| No. | Locus.Tag | Gene.Symbol | GenBank.Accession | Chromosome |
|---|---|---|---|---|
| 1 | HMPREF9547_01191 | | ZP_07167682 | |
| 2 | HMPREF9551_04221 | | ZP_07191576 | |
| 3 | HMPREF9347_01086 | | ZP_07208640 | |
| 4 | STMDT12_C39170 | | BAJ38860 | |
| 5 | SFxv_1168 | | ADA73425 | |
| 6 | Sdys1_01000012 | | ZP_00922750 | |
| 7 | HMPREF9547_ 02735 | | ZP_07169192 | |
| 8 | Sdys1_01000013 | | ZP_00922751 | |
| 9 | SFxv_4146 | | ADA76106 | |
| 10 | HMPREF9547_04432 | | ZP_07170853 | |
| 11 | HMPREF9547_ 00721 | | ZP_07167228 | |

(continued)

| No. | Locus.Tag | Gene.Symbol | GenBank.Accession | Chromosome |
|-----|-----------|-------------|-------------------|------------|
| 12 | SFxv_1141 | | ADA73399 | |
| 13 | ETAE_p041 | folP | YP_003297635 | plasmid pEIB202 |
| 14 | ESCAB7627_0644 | | ZP_02900823 | |
| 15 | SSON_3896 | aadA | YP_312670 | |
| 16 | SFxv_4148 | | ADA76108 | |
| 17 | ESCAB7627_0650 | | ZP_02900555 | |
| 18 | pEFER 0049 | | YP_002394596 | plasmid pEFER |
| 19 | SD1617_4863 | | ZP_07682961 | |
| 20 | ECED1_1050 | | YP_002397074 | |
| 21 | ESCC014588_3315 | | ZP_03445386 | |
| 22 | ECS88_2899 | rusA | YP_002392541 | |
| 23 | ETAE_p040 | | YP_003297634 | plasmid pEIB202 |
| 24 | EcolE2_01002516 | | ZP_00729149 | |
| 25 | KP1_1529 | betB | YP_002918349 | |
| 26 | EFER_2751 | fhiA | YP_002383857 | |
| 27 | EFER_1964 | | YP_002383093 | |
| 28 | ECEG_03397 | | ZP_06652297 | |
| 29 | HMPREF9534_04860 | | ZP_07189724 | |
| 30 | SFV_0325 | betT | YP_687904 | |
| 31 | SFV_3136 | tsaC | YP_690497 | |
| 32 | SeSA_C0004 | | YP_002113008 | plasmid pCVM19633_4 |
| 33 | CIT292_00036 | | ZP_03834370 | |
| 34 | EFER_4022 | phoU | YP_002385049 | |
| 35 | SD1617_0888 | pstA | ZP_07679073 | |
| 36 | SbBS512_E4201 | pstC | YP_001882442 | |
| 37 | EFER_4026 | pstS | YP_002385053 | |
| 38 | Senteenterica_010100025269 | | ZP_02664569 | |
| 39 | SSON_PA02 | tetA | YP_001139953 | plasmid pSS046_spA |
| 40 | EFER_4455 | | YP_002385453 | |
| 41 | ESCAB7627_0589 | agaF | ZP_02900787 | |
| 42 | ECABU_c04070 | betA | ADN45010 | |
| 43 | EcDH1_0571 | | ACX38258 | |
| 44 | S3383 | agaV | NP_838641 | |
| 45 | Eco15_01002315 | | ZP_00735973 | |
| 46 | EC236275_0400 | | ZP_07778877 | |
| 47 | ESCAB7627_0593 | | ZP_02900381 | |
| 48 | HMPREF9530_03165 | | ZP_07153036 | |
| 49 | SbBS512_E1297 | | YP_001879929 | |

(continued)

| No. | Locus.Tag | Gene.Symbol | GenBank.Accession | Chromosome |
|-----|-----------|-------------|-------------------|------------|
| 50 | CSAG_02340 | | ZP_04563010 | |

Table 22e: Data for Shigella species

| No. | Start.Coord | End.Coord | Strand | Scaffold.ID |
|-----|-------------|-----------|--------|-------------|
| 1 | 6987 | 7817 | + | 648291751 |
| 2 | 10102 | 10662 | - | 648292991 |
| 3 | 2726 | 5692 | - | 648294279 |
| 4 | 4067283 | 4067942 | + | 651053007 |
| 5 | 1128245 | 1129249 | + | 646862363 |
| 6 | 12375 | 12701 | - | 638365528 |
| 7 | 4692 | 5189 | - | 648291885 |
| 8 | 14252 | 14776 | + | 638365528 |
| 9 | 3950954 | 3952570 | - | 646862363 |
| 10 | 8898 | 9719 | - | 648292023 |
| 11 | 5423 | 6016 | - | 648291712 |
| 12 | 1107342 | 1107968 | + | 646862363 |
| 13 | 32101 | 32916 | - | 646311959 |
| 14 | 233492 | 234697 | - | 641784575 |
| 15 | 4110458 | 4111246 | - | 640427102 |
| 16 | 3954100 | 3955767 | - | 646862363 |
| 17 | 238788 | 239996 | - | 641784575 |
| 18 | 48658 | 49503 | + | 643692014 |
| 19 | 80678 | 81835 | - | 649994698 |
| 20 | 1066134 | 1066832 | + | 643348624 |
| 21 | 383590 | 384330 | - | 642987534 |
| 22 | 2863316 | 2863705 | - | 644736332 |
| 23 | 31237 | 32040 | - | 646311959 |
| 24 | 35676 | 36281 | - | 638358744 |
| 25 | 1469088 | 1470560 | - | 646564530 |
| 26 | 2808563 | 2810692 | + | 643692013 |
| 27 | 2010341 | 2013862 | - | 643692013 |
| 28 | 167911 | 168516 | - | 647536674 |
| 29 | 4513 | 7977 | - | 648289846 |
| 30 | 342056 | 344089 | + | 637000590 |
| 31 | 3209986 | 3212835 | - | 637000590 |
| 32 | 2398 | 3933 | - | 642555220 |
| 33 | 7614 | 8387 | + | 643891493 |

(continued)

| No. | Start.Coord | End.Coord | Strand | Scaffold.ID |
|---|---|---|---|---|
| 34 | 4127070 | 4127795 | - | 643692013 |
| 35 | 217087 | 217971 | - | 649994663 |
| 36 | 3927571 | 3928530 | + | 641522725 |
| 37 | 4130610 | 4131650 | - | 643692013 |
| 38 | 35858 | 36535 | - | 641777693 |
| 39 | 1155 | 2429 | + | 640427123 |
| 40 | 4539896 | 4540645 | + | 643692013 |
| 41 | 182707 | 183141 | - | 641784575 |
| 42 | 425884 | 427554 | - | 648231700 |
| 43 | 602662 | 603063 | - | 646862383 |
| 44 | 3261857 | 3262366 | + | 637000219 |
| 45 | 39234 | 40157 | - | 638359205 |
| 46 | 26968 | 27516 | - | 649994608 |
| 47 | 185313 | 186593 | - | 641784575 |
| 48 | 5665 | 6162 | - | 648290513 |
| 49 | 1184368 | 1184799 | + | 641522725 |
| 50 | 2538496 | 2539857 | + | 646206732 |

Example 12: Staphylococcus, particularly S. aureus

The procedure was carried out as in Example 1, except for the following changes:

Bacterial Strains

The inventors selected 1001 specimens of S. aureus from the microbiology strain collection at Siemens Healthcare Diagnostics (West Sacramento, CA) for susceptibility testing and whole genome sequencing, of which 985 had an assembly, a unique Kiel NGS ID (NGS data and assembly ID, a unique resistance profile (no different resistance profiles with different outcomes, and at least one drug with non-missing resistance value, so that these were further analyzed.

From MetaRef, 754 centroids of S. aureus were used as reference sequences.

Resistance / susceptibility was determined for the following antibiotics as described below: Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

For testing, standard procedures were used, i.e. VITEK 2 system and AST cards (Biomerieux), Microscan system and AST panels (Beckmann Coulter).

DNA extraction

DNA extraction and purification was carried out using the MagAttract HMW DNA Kit (Qiagen) procedure with the following changes. After up to $2x10^9$ bacteria (1ml culture) were centrifuged in a 2 ml tube (10 min, 5000 x g) and the supernatant was discharged, it was again centrifuged 1 min and the sample was taken. The resulting pellet was dispersed in 160 $\mu$l P1, 20 $\mu$l lysozyme (100 mg/ml) and 4 $\mu$l lysostaphin were added and mixed, and the suspension was incubated at 37°C at 900 rpm for 30 mins in a thermal mixer. Afterwards 300 $\mu$l lysis buffer and proteinase K (30 $\mu$l) (both from the blood kit for Maxwell of Promega) were added and the whole again incubated for 30 mins at 56°C and 900 rpm. The samples as a whole ($\sim$ 510 $\mu$l) were then transferred to the Maxwell cartridges for further processing, using the Tissue LEV Total RNA Kit AS1220 or the XAS1220 Custom Kit (Promega).

Next generation sequencing and data analysis was carried out as in Example 1.

The results for S. aureus are shown in Tables 23 (corresponding to Table 1) and 24 (corresponding to Table 2).

Table 23a: Data for Staphylococcus, particularly S. aureus

| No. | Scaffold.External. Accession | Scaffold.Name | best_pv |
|---|---|---|---|
| 1 | NZ_ACJC01000132 | Staphylococcus epidermidis W23144: NZ_ACJC01000132 | 9.38479881072327e-227 |
| 2 | NZ_ADMU01000036 | Staphylococcus epidermidis M23864:W2(grey) contig00037: NZ_ADMU01000036 | 9.38479881072327e-227 |
| 3 | NZ_ADMU01000036 | Staphylococcus epidermidis M23864:W2(grey) contig00037: NZ_ADMU01000036 | 2.67435403500704e-225 |
| 4 | NZ_GG770515 | Staphylococcus aureus subsp. aureus EMRSA16 genomic scaffold supercont1.3: NZ_GG770515 | 1.77186061517335e-211 |
| 5 | NZ_ACHE01000064 | Staphylococcus epidermidis BCM-HMP0060: NZ_ACHE01000064 | 3.22333061264623e-210 |
| 6 | NZ_ACKJ01000008 | Staphylococcus aureus A9719: NZ_ACKJ01000008 | 4.28801157250818e-188 |
| 7 | FN433596 | Staphylococcus aureus subsp. aureus TW20: FN433596 | 6.72085159673615e-181 |
| 8 | FN433596 | Staphylococcus aureus subsp. aureus TW20: FN433596 | 6.72085159673615e-181 |
| 9 | NC_002976 | Staphylococcus epidermidis RP62A: NC_002976 | 6.72085159673615e-181 |
| 10 | NZ_ACKF01000035 | Staphylococcus aureus A6300: NZ_ACKF01000035 | 8.5426103876016e-179 |
| 11 | NC_002976 | Staphylococcus epidermidis RP62A: NC_002976 | 8.5426103876016e-179 |
| 12 | NC_002976 | Staphylococcus epidermidis RP62A: NC_002976 | 1.81714784068785e-175 |
| 13 | NZ_ADMU01000035 | Staphylococcus epidermidis M23864:W2(grey) contig00036: NZ_ADMU01000035 | 3.23410033711171e-175 |
| 14 | NC_002976 | Staphylococcus epidermidis RP62A: NC_002976 | 9.12700235375297e-175 |
| 15 | NC_002976 | Staphylococcus epidermidis RP62A: NC_002976 | 9.12700235375297e-175 |
| 16 | NC_002976 | Staphylococcus epidermidis RP62A: NC_002976 | 9.12700235375297e-175 |
| 17 | NC_002976 | Staphylococcus epidermidis RP62A: NC_002976 | 9.12700235375297e-175 |
| 18 | NC_002976 | Staphylococcus epidermidis RP62A: NC_002976 | 2.25944344923286e-173 |
| 19 | NZ_ACKF01000007 | Staphylococcus aureus A6300: NZ_ACKF01000007 | 2.40063417489507e-172 |
| 20 | NC_002976 | Staphylococcus epidermidis RP62A: NC_002976 | 2.59802993040457e-167 |

(continued)

| No. | Scaffold.External. Accession | Scaffold.Name | best_pv |
|-----|------------------------------|---------------|---------|
| 21 | NC_002976 | Staphylococcus epidermidis RP62A: NC_002976 | 3.12953724144125e-166 |
| 22 | NZ_ADJJ01000031 | Staphylococcus aureus A8796 cont1.31: NZ_ADJJ01000031 | 3.37208086862656e-166 |
| 23 | NC_002745 | Staphylococcus aureus subsp. aureus N315: NC_002745 | 3.37208086862656e-166 |
| 24 | NC_002976 | Staphylococcus epidermidis RP62A: NC_002976 | 7.17319474297637e-161 |
| 25 | NZ_ACHH01000128 | Staphylococcus aureus subsp. aureus TCH70: NZ_ACHH01000128 | 4.16856451727179e-152 |
| 26 | NZ_ACKC01000026 | Staphylococcus aureus A5937: NZ_ACKC01000026 | 1.58751373713434e-135 |
| 27 | NZ_ACKC01000026 | Staphylococcus aureus A5937: NZ_ACKC01000026 | 1.69633422486642e-135 |
| 28 | CP001844 | Staphylococcus aureus 04-02981: CP001844 | 1.77656315889944e-134 |
| 29 | FR714927 | Staphylococcus aureus subsp. aureus ECT-R 2: FR714927 | 4.06264970457926e-133 |
| 30 | CP001844 | Staphylococcus aureus 04-02981: CP001844 | 1.01251954267327e-130 |
| 31 | NC_007622 | Staphylococcus aureus RF122: NC_007622 | 4.84169534955158e-126 |
| 32 | NC_009632 | Staphylococcus aureus subsp. aureus JH1: NC_009632 | 6.20485118760467e-122 |
| 33 | NC_002976 | Staphylococcus epidermidis RP62A: NC_002976 | 5.75915090813908e-120 |
| 34 | NZ_ACY001000032 | Staphylococcus aureus A8117 cont1.32: NZ_ACY001000032 | 1.84672228386398e-116 |
| 35 | FR714927 | Staphylococcus aureus subsp. aureus ECT-R 2: FR714927 | 7.1946440374743e-116 |
| 36 | NZ_ACHD01000273 | Staphylococcus aureus subsp. aureus TCH130: NZ_ACHD01000273 | 4.22320321058421e-113 |
| 37 | NZ_ACKE01000012 | Staphylococcus aureus A6224: NZ_ACKE01000012 | 4.22320321058421e-113 |
| 38 | CP002114 | Staphylococcus aureus subsp. aureus JKD6159: CP002114 | 4.83609713640718e-112 |
| 39 | NZ_GG730131 | Staphylococcus aureus subsp. aureus C101 genomic scaffold super-cont1.12: NZ_GG730131 | 4.63942446669448e-110 |
| 40 | NZ_ACKF01000014 | Staphylococcus aureus A6300: NZ_ACKF01000014 | 5.76090691723747e-108 |
| 41 | NZ_ACKK01000039 | Staphylococcus aureus A9763: NZ_ACKK01000039 | 2.40583396327414e-107 |
| 42 | NZ_ACKE01000008 | Staphylococcus aureus A6224: NZ_ACKE01000008 | 2.99974766558167e-106 |

(continued)

| No. | Scaffold.External. Accession | Scaffold.Name | best_pv |
|---|---|---|---|
| 43 | NZ_ACKG01000031 | Staphylococcus aureus A8115: NZ_ ACKG01000031 | 1.57559858677166e-102 |
| 44 | NC_009632 | Staphylococcus aureus subsp. aureus JH1: NC_009632 | 1.0725409335823e-101 |
| 45 | NZ_ACY001000003 | Staphylococcus aureus A8117 cont1.3: NZ_ ACY001000003 | 1.0725409335823e-101 |
| 46 | FR714929 | Staphylococcus aureus subsp. aureus ECT-R 2 plasmid pLUH02: FR714929 | 5.26123840198017e-101 |
| 47 | CP001844 | Staphylococcus aureus 04-02981: CP001844 | 8.01600909282956e-101 |
| 48 | CP001844 | Staphylococcus aureus 04-02981: CP001844 | 1.78546333248495e-95 |
| 49 | NZ_ADJK01000020 | Staphylococcus aureus A8819 cont1.20: NZ_ ADJK01000020 | 1.80469278143707e-94 |
| 50 | NZ_GL545262 | Staphylococcus hominis subsp. hominis C80 genomic scaffold supercont1.11: NZ_ GL545262 | 2.82728623419544e-94 |

Table 23b: Data for Staphylococcus, particularly S. aureus (continued)

| No. | sign_phenos |
|---|---|
| 1 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 2 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 3 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 4 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 5 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 6 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tobramycin |
| 7 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tetracyclin;Tobramycin |
| 8 | Ampicillin;Ampicillin/Sulbactam;Cefepim;Cefotaxim;Cefuroxim;Ciprofloxacin; Clindamycin;Erythromycin;Imipenem;Levofloxacin;Moxifloxacin;Oxacillin;Penicillin G; Piperacillin/Tazobactam;Tetracyclin;Tobramycin |

(continued)

| No. | sign_phenos |
|---|---|
| 9 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 10 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 11 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 12 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 13 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 14 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 15 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 16 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 17 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 18 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 19 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 20 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 21 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 22 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 23 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |

(continued)

| No. | sign_phenos |
|---|---|
| 24 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 25 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 26 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 27 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 28 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 29 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 30 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 31 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 32 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tobramycin |
| 33 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 34 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tobramycin |
| 35 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 36 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 37 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 38 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |

(continued)

| No. | sign_phenos |
|---|---|
| 39 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 40 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 41 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 42 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 43 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 44 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 45 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 46 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 47 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 48 | Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 49 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |
| 50 | Ampicillin;Ampicillin/Sulbactam;Cefepim;Cefotaxim;Cefuroxim;Ciprofloxacin;Clindamycin;Erythromycin;Imipenem;Levofloxacin;Moxifloxacin;Oxacillin;Penicillin G;Piperacillin/Tazobactam;Tetracyclin;Tobramycin |

Table 23c: Data for Staphylococcus, particularly S. aureus (continued)

| No. | sign_phenos_class | best_pheno | best_pheno_class |
|---|---|---|---|
| 1 | aminoglycoside;fluoroquinolone;lactam;lincosamide;macrolide | Oxacillin | lactam |
| 2 | aminoglycoside;fluoroquinolone;lactam;lincosamide;macrolide | Oxacillin | lactam |
| 3 | aminoglycoside;fluoroquinolone;lactam;lincosamide;macrolide | Oxacillin | lactam |
| 4 | aminoglycoside;fluoroquinolone;lactam;lincosamide;macrolide | Oxacillin | lactam |
| 5 | aminoglycoside;fluoroquinolone;lactam;lincosamide;macrolide | Oxacillin | lactam |
| 6 | aminoglycoside;fluoroquinolone;lactam;lincosamide;macrolide | Moxifloxacin | fluoroquinolone |

(continued)

| No. | sign_phenos_class | best_pheno | best_pheno_class |
|---|---|---|---|
| 7 | aminoglycoside;fluoroquinolone;lactam;lincosamide;macrolide; tetracycline | Oxacillin | lactam |
| 8 | aminoglycoside;fluoroquinolone;lactam;lincosamide;macrolide; tetracycline | Oxacillin | lactam |
| 9 | aminoglycoside;fluoroquinolone;lactam;lincosamide;macrolide; tetracycline | Oxacillin | lactam |
| 10 | aminoglycoside;fluoroquinolone;lactam;lincosamide;macrolide; tetracycline | Oxacillin | lactam |
| 11 | aminoglycoside;fluoroquinolone;lactam;lincosamide;macrolide; tetracycline | Oxacillin | lactam |
| 12 | aminoglycoside;fluoroquinolone;lactam;lincosamide;macrolide; tetracycline | Oxacillin | lactam |
| 13 | aminoglycoside;fluoroquinolone;lactam;lincosamide;macrolide; tetracycline | Oxacillin | lactam |
| 14 | aminoglycoside;fluoroquinolone;lactam;lincosamide;macrolide; tetracycline | Oxacillin | lactam |
| 15 | aminoglycoside;fluoroquinolone;lactam;lincosamide;macrolide; tetracycline | Oxacillin | lactam |
| 16 | aminoglycoside;fluoroquinolone;lactam;lincosamide;macrolide; tetracycline | Oxacillin | lactam |
| 17 | aminoglycoside;fluoroquinolone;lactam;lincosamide;macrolide; tetracycline | Oxacillin | lactam |
| 18 | aminoglycoside;fluoroquinolone;lactam;lincosamide;macrolide; tetracycline | Oxacillin | lactam |
| 19 | aminoglycoside;fluoroquinolone;lactam;lincosamide;macrolide; tetracycline | Oxacillin | lactam |
| 20 | aminoglycoside;fluoroquinolone;lactam;lincosamide;macrolide; tetracycline | Moxifloxacin | fluoroquinolone |
| 21 | aminoglycoside;fluoroquinolone;lactam;lincosamide;macrolide; tetracycline | Moxifloxacin | fluoroquinolone |
| 22 | aminoglycoside;fluoroquinolone;lactam;lincosamide;macrolide; tetracycline | Moxifloxacin | fluoroquinolone |
| 23 | aminoglycoside;fluoroquinolone;lactam;lincosamide;macrolide; tetracycline | Moxifloxacin | fluoroquinolone |
| 24 | aminoglycoside;fluoroquinolone;lactam;lincosamide;macrolide; tetracycline | Moxifloxacin | fluoroquinolone |
| 25 | aminoglycoside;fluoroquinolone;lactam;lincosamide;macrolide; tetracycline | Moxifloxacin | fluoroquinolone |
| 26 | aminoglycoside;fluoroquinolone;lactam;lincosamide;macrolide; tetracycline | Moxifloxacin | fluoroquinolone |
| 27 | aminoglycoside;fluoroquinolone;lactam;lincosamide;macrolide; tetracycline | Moxifloxacin | fluoroquinolone |
| 28 | aminoglycoside;fluoroquinolone;lactam;lincosamide;macrolide; tetracycline | Moxifloxacin | fluoroquinolone |

(continued)

| No. | sign_phenos_class | best_pheno | best_pheno_class |
|---|---|---|---|
| 29 | aminoglycoside;fluoroquinolone;lactam;lincosamide;macrolide; tetracycline | Moxifloxacin | fluoroquinolone |
| 30 | aminoglycoside;fluoroquinolone;lactam;lincosamide;macrolide; tetracycline | Moxifloxacin | fluoroquinolone |
| 31 | aminoglycoside;fluoroquinolone;lactam;lincosamide;macrolide; tetracycline | Moxifloxacin | fluoroquinolone |
| 32 | aminoglycoside;fluoroquinolone;lactam;lincosamide;macrolide | Moxifloxacin | fluoroquinolone |
| 33 | aminoglycoside;fluoroquinolone;lactam;lincosamide;macrolide; tetracycline | Moxifloxacin | fluoroquinolone |
| 34 | aminoglycoside;fluoroquinolone;lactam;lincosamide;macrolide | Moxifloxacin | fluoroquinolone |
| 35 | aminoglycoside;fluoroquinolone;lactam;lincosamide;macrolide; tetracycline | Moxifloxacin | fluoroquinolone |
| 36 | aminoglycoside;fluoroquinolone;lactam;lincosamide;macrolide; tetracycline | Moxifloxacin | fluoroquinolone |
| 37 | aminoglycoside;fluoroquinolone;lactam;lincosamide;macrolide; tetracycline | Moxifloxacin | fluoroquinolone |
| 38 | aminoglycoside;fluoroquinolone;lactam;lincosamide;macrolide; tetracycline | Moxifloxacin | fluoroquinolone |
| 39 | aminoglycoside;fluoroquinolone;lactam;lincosamide;macrolide; tetracycline | Moxifloxacin | fluoroquinolone |
| 40 | aminoglycoside;fluoroquinolone;lactam;lincosamide;macrolide; tetracycline | Moxifloxacin | fluoroquinolone |
| 41 | aminoglycoside;fluoroquinolone;lactam;lincosamide;macrolide; tetracycline | Moxifloxacin | fluoroquinolone |
| 42 | aminoglycoside;fluoroquinolone;lactam;lincosamide;macrolide; tetracycline | Moxifloxacin | fluoroquinolone |
| 43 | aminoglycoside;fluoroquinolone;lactam;lincosamide;macrolide; tetracycline | Moxifloxacin | fluoroquinolone |
| 44 | aminoglycoside;fluoroquinolone;lactam;lincosamide;macrolide; tetracycline | Moxifloxacin | fluoroquinolone |
| 45 | aminoglycoside;fluoroquinolone;lactam;lincosamide;macrolide; tetracycline | Moxifloxacin | fluoroquinolone |
| 46 | aminoglycoside;fluoroquinolone;lactam;lincosamide;macrolide; tetracycline | Moxifloxacin | fluoroquinolone |
| 47 | aminoglycoside;fluoroquinolone;lactam;lincosamide;macrolide; tetracycline | Moxifloxacin | fluoroquinolone |
| 48 | aminoglycoside;fluoroquinolone;lactam;lincosamide;macrolide; tetracycline | Moxifloxacin | fluoroquinolone |
| 49 | aminoglycoside;fluoroquinolone;lactam;lincosamide;macrolide; tetracycline | Moxifloxacin | fluoroquinolone |
| 50 | aminoglycoside;fluoroquinolone;lactam;lincosamide;macrolide; tetracycline | Oxacillin | lactam |

Table 23d: Data for Staphylococcus, particularly S. aureus (continued)

| No. | num_aminoglycoside | num_fluoroquinolone | num_lactam | num_lincosamide | num_macrolide | num_tetracycline |
|---|---|---|---|---|---|---|
| 1 | 1 | 3 | 9 | 1 | 1 | 0 |
| 2 | 1 | 3 | 9 | 1 | 1 | 0 |
| 3 | 1 | 3 | 9 | 1 | 1 | 0 |
| 4 | 1 | 3 | 9 | 1 | 1 | 0 |
| 5 | 1 | 3 | 9 | 1 | 1 | 0 |
| 6 | 1 | 3 | 9 | 1 | 1 | 0 |
| 7 | 1 | 3 | 9 | 1 | 1 | 1 |
| 8 | 1 | 3 | 9 | 1 | 1 | 1 |
| 9 | 1 | 3 | 9 | 1 | 1 | 1 |
| 10 | 1 | 3 | 9 | 1 | 1 | 1 |
| 11 | 1 | 3 | 9 | 1 | 1 | 1 |
| 12 | 1 | 3 | 9 | 1 | 1 | 1 |
| 13 | 1 | 3 | 9 | 1 | 1 | 1 |
| 14 | 1 | 3 | 9 | 1 | 1 | 1 |
| 15 | 1 | 3 | 9 | 1 | 1 | 1 |
| 16 | 1 | 3 | 9 | 1 | 1 | 1 |
| 17 | 1 | 3 | 9 | 1 | 1 | 1 |
| 18 | 1 | 3 | 9 | 1 | 1 | 1 |
| 19 | 1 | 3 | 9 | 1 | 1 | 1 |
| 20 | 1 | 3 | 9 | 1 | 1 | 1 |
| 21 | 1 | 3 | 9 | 1 | 1 | 1 |
| 22 | 1 | 3 | 9 | 1 | 1 | 1 |
| 23 | 1 | 3 | 9 | 1 | 1 | 1 |
| 24 | 1 | 3 | 9 | 1 | 1 | 1 |
| 25 | 1 | 3 | 9 | 1 | 1 | 1 |
| 26 | 1 | 3 | 8 | 1 | 1 | 1 |
| 27 | 1 | 3 | 8 | 1 | 1 | 1 |
| 28 | 1 | 3 | 8 | 1 | 1 | 1 |
| 29 | 1 | 3 | 8 | 1 | 1 | 1 |
| 30 | 1 | 3 | 8 | 1 | 1 | 1 |
| 31 | 1 | 3 | 9 | 1 | 1 | 1 |
| 32 | 1 | 3 | 8 | 1 | 1 | 0 |
| 33 | 1 | 3 | 9 | 1 | 1 | 1 |
| 34 | 1 | 3 | 8 | 1 | 1 | 0 |
| 35 | 1 | 3 | 8 | 1 | 1 | 1 |
| 36 | 1 | 3 | 9 | 1 | 1 | 1 |
| 37 | 1 | 3 | 9 | 1 | 1 | 1 |

(continued)

| No. | num_aminoglycoside | num_fluoroquinolone | num_lactam | num_lincosamide | num_macrolide | num_tetracycline |
|---|---|---|---|---|---|---|
| 38 | 1 | 3 | 8 | 1 | 1 | 1 |
| 39 | 1 | 3 | 8 | 1 | 1 | 1 |
| 40 | 1 | 3 | 8 | 1 | 1 | 1 |
| 41 | 1 | 3 | 8 | 1 | 1 | 1 |
| 42 | 1 | 3 | 8 | 1 | 1 | 1 |
| 43 | 1 | 3 | 8 | 1 | 1 | 1 |
| 44 | 1 | 3 | 8 | 1 | 1 | 1 |
| 45 | 1 | 3 | 8 | 1 | 1 | 1 |
| 46 | 1 | 3 | 9 | 1 | 1 | 1 |
| 47 | 1 | 3 | 8 | 1 | 1 | 1 |
| 48 | 1 | 3 | 8 | 1 | 1 | 1 |
| 49 | 1 | 3 | 9 | 1 | 1 | 1 |
| 50 | 1 | 3 | 9 | 1 | 1 | 1 |

Table 23e: Data for Staphylococcus, particularly S. aureus (continued)

| No. | Ampicillin_pv_adj | Ampicillin/Sulbactam_pv_adj | Cefepim_pv_adj | Cefotaxim_pv_adj |
|---|---|---|---|---|
| 1 | 2.20E-08 | 5.96E-37 | 5.96E-37 | 5.96E-37 |
| 2 | 2.20E-08 | 5.96E-37 | 5.96E-37 | 5.96E-37 |
| 3 | 2.20E-08 | 5.96E-37 | 5.96E-37 | 5.96E-37 |
| 4 | 4.64E-08 | 4.55E-35 | 4.55E-35 | 4.55E-35 |
| 5 | 2.20E-08 | 2.16E-33 | 2.16E-33 | 2.16E-33 |
| 6 | 2.22E-09 | 1.64E-31 | 1.64E-31 | 1.64E-31 |
| 7 | 3.22E-07 | 2.72E-27 | 2.72E-27 | 2.72E-27 |
| 8 | 3.22E-07 | 2.72E-27 | 2.72E-27 | 2.72E-27 |
| 9 | 3.22E-07 | 2.72E-27 | 2.72E-27 | 2.72E-27 |
| 10 | 3.22E-07 | 2.72E-27 | 2.72E-27 | 2.72E-27 |
| 11 | 3.22E-07 | 2.72E-27 | 2.72E-27 | 2.72E-27 |
| 12 | 6.85E-07 | 2.72E-27 | 2.72E-27 | 2.72E-27 |
| 13 | 2.90E-07 | 1.05E-25 | 1.05E-25 | 1.05E-25 |
| 14 | 3.22E-07 | 2.72E-27 | 2.72E-27 | 2.72E-27 |
| 15 | 3.22E-07 | 2.72E-27 | 2.72E-27 | 2.72E-27 |
| 16 | 3.22E-07 | 2.72E-27 | 2.72E-27 | 2.72E-27 |
| 17 | 3.22E-07 | 2.72E-27 | 2.72E-27 | 2.72E-27 |
| 18 | 2.90E-07 | 1.05E-25 | 1.05E-25 | 1.05E-25 |
| 19 | 2.90E-07 | 1.05E-25 | 1.05E-25 | 1.05E-25 |
| 20 | 8.65E-04 | 1.18E-21 | 1.18E-21 | 1.18E-21 |
| 21 | 8.65E-04 | 1.18E-21 | 1.18E-21 | 1.18E-21 |

(continued)

| No. | Ampicillin_pv_adj | Ampicillin/Sulbactam_pv_adj | Cefepim_pv_adj | Cefotaxim_pv_adj |
|-----|-------------------|------------------------------|----------------|------------------|
| 22 | 3.21E-03 | 7.21E-21 | 7.21E-21 | 7.21E-21 |
| 23 | 3.21E-03 | 7.21E-21 | 7.21E-21 | 7.21E-21 |
| 24 | 1.55E-03 | 1.27E-19 | 1.27E-19 | 1.27E-19 |
| 25 | 1.83E-03 | 2.01E-19 | 2.01E-19 | 2.01E-19 |
| 26 | 1.26E-02 | 1.32E-20 | 1.32E-20 | 1.32E-20 |
| 27 | 1.18E-02 | 7.81E-20 | 7.81E-20 | 7.81E-20 |
| 28 | 1.18E-02 | 7.81E-20 | 7.81E-20 | 7.81E-20 |
| 29 | 1.26E-02 | 7.25E-19 | 7.25E-19 | 7.25E-19 |
| 30 | 3.28E-02 | 3.76E-18 | 3.76E-18 | 3.76E-18 |
| 31 | 4.05E-03 | 1.35E-15 | 1.35E-15 | 1.35E-15 |
| 32 | 1.24E-02 | 1.13E-19 | 1.13E-19 | 1.13E-19 |
| 33 | 1.11E-03 | 1.73E-15 | 1.73E-15 | 1.73E-15 |
| 34 | 1.27E-02 | 6.35E-19 | 6.35E-19 | 6.35E-19 |
| 35 | 2.32E-02 | 3.05E-13 | 3.05E-13 | 3.05E-13 |
| 36 | 7.10E-03 | 2.03E-18 | 2.03E-18 | 2.03E-18 |
| 37 | 7.10E-03 | 2.03E-18 | 2.03E-18 | 2.03E-18 |
| 38 | 1.24E-02 | 2.91E-15 | 2.91E-15 | 2.91E-15 |
| 39 | 3.37E-02 | 1.60E-16 | 1.60E-16 | 1.60E-16 |
| 40 | 5.70E-02 | 1.88E-12 | 1.88E-12 | 1.88E-12 |
| 41 | 1.15E-01 | 1.25E-15 | 1.25E-15 | 1.25E-15 |
| 42 | 3.84E-02 | 9.78E-11 | 9.78E-11 | 9.78E-11 |
| 43 | 3.56E-02 | 5.77E-17 | 5.77E-17 | 5.77E-17 |
| 44 | 3.37E-02 | 1.60E-16 | 1.60E-16 | 1.60E-16 |
| 45 | 3.37E-02 | 1.60E-16 | 1.60E-16 | 1.60E-16 |
| 46 | 2.90E-07 | 1.61E-15 | 1.61E-15 | 1.61E-15 |
| 47 | 7.69E-02 | 7.81E-20 | 7.81E-20 | 7.81E-20 |
| 48 | 8.07E-02 | 6.08E-12 | 6.08E-12 | 6.08E-12 |
| 49 | 2.90E-07 | 1.61E-15 | 1.61E-15 | 1.61E-15 |
| 50 | 3.04E-06 | 1.82E-17 | 1.82E-17 | 1.82E-17 |

Table 23f: Data for Staphylococcus, particularly S. aureus (continued)

| No. | Cefuroxim_pv_adj | Ciprofloxacin_pv_adj | Clindamycin_pv_adj | Erythromycin_pv_adj |
|-----|------------------|----------------------|--------------------|---------------------|
| 1 | 1.02E-172 | 4.82E-131 | 5.41E-111 | 1.04E-111 |
| 2 | 1.02E-172 | 4.82E-131 | 5.41E-111 | 1.04E-111 |
| 3 | 1.02E-172 | 3.02E-132 | 6.31E-112 | 7.39E-113 |
| 4 | 1.19E-170 | 1.00E-131 | 5.45E-114 | 1.72E-111 |
| 5 | 9.55E-169 | 1.65E-131 | 8.55E-113 | 3.44E-110 |

(continued)

| No. | Cefuroxim_pv_adj | Ciprofloxacin_pv_adj | Clindamycin_pv_adj | Erythromycin_pv_adj |
|---|---|---|---|---|
| 6 | 3.09E-155 | 2.03E-145 | 7.78E-102 | 4.54E-100 |
| 7 | 2.00E-140 | 4.08E-138 | 6.00E-125 | 2.44E-127 |
| 8 | 2.00E-140 | 5.95E-135 | 7.70E-123 | 2.44E-127 |
| 9 | 2.00E-140 | 4.08E-138 | 6.00E-125 | 2.44E-127 |
| 10 | 2.29E-138 | 2.11E-136 | 1.31E-123 | 4.65E-126 |
| 11 | 2.29E-138 | 3.02E-133 | 1.67E-121 | 4.65E-126 |
| 12 | 1.01E-138 | 1.36E-128 | 4.01E-120 | 1.01E-123 |
| 13 | 2.71E-145 | 1.43E-135 | 2.49E-122 | 2.94E-121 |
| 14 | 5.87E-142 | 6.84E-140 | 7.88E-125 | 3.61E-124 |
| 15 | 5.87E-142 | 6.84E-140 | 7.88E-125 | 3.61E-124 |
| 16 | 5.87E-142 | 6.84E-140 | 7.88E-125 | 3.61E-124 |
| 17 | 5.87E-142 | 6.84E-140 | 7.88E-125 | 3.61E-124 |
| 18 | 3.15E-140 | 6.84E-140 | 1.65E-123 | 1.01E-122 |
| 19 | 3.78E-139 | 5.24E-139 | 7.90E-123 | 4.58E-122 |
| 20 | 3.99E-125 | 5.65E-132 | 3.88E-136 | 8.83E-140 |
| 21 | 5.82E-123 | 4.09E-130 | 1.24E-134 | 2.39E-138 |
| 22 | 4.30E-124 | 5.65E-132 | 7.67E-139 | 1.30E-142 |
| 23 | 4.30E-124 | 5.65E-132 | 7.67E-139 | 1.30E-142 |
| 24 | 8.70E-122 | 4.09E-130 | 4.42E-135 | 5.01E-139 |
| 25 | 1.95E-112 | 3.48E-117 | 1.09E-121 | 8.26E-126 |
| 26 | 9.99E-102 | 2.00E-99 | 7.42E-94 | 1.88E-93 |
| 27 | 9.92E-101 | 1.25E-100 | 4.84E-95 | 2.65E-94 |
| 28 | 1.08E-99 | 6.62E-99 | 5.06E-95 | 1.80E-94 |
| 29 | 4.45E-100 | 1.46E-101 | 3.05E-98 | 1.82E-99 |
| 30 | 3.62E-99 | 6.76E-98 | 4.11E-89 | 2.04E-88 |
| 31 | 5.46E-82 | 8.46E-90 | 4.95E-69 | 2.56E-67 |
| 32 | 3.45E-93 | 1.68E-88 | 1.37E-90 | 2.27E-88 |
| 33 | 7.10E-92 | 7.29E-86 | 6.57E-78 | 1.16E-76 |
| 34 | 3.46E-90 | 5.20E-83 | 1.49E-80 | 4.89E-80 |
| 35 | 9.56E-73 | 1.94E-83 | 1.78E-63 | 2.45E-61 |
| 36 | 1.95E-83 | 3.50E-91 | 5.92E-79 | 2.14E-84 |
| 37 | 1.95E-83 | 3.50E-91 | 5.92E-79 | 2.14E-84 |
| 38 | 7.71E-76 | 4.08E-77 | 6.05E-58 | 6.49E-57 |
| 39 | 3.93E-89 | 4.20E-78 | 5.89E-71 | 3.43E-72 |
| 40 | 3.54E-69 | 3.19E-77 | 4.43E-58 | 3.86E-57 |
| 41 | 8.95E-84 | 1.49E-77 | 1.71E-74 | 1.87E-74 |
| 42 | 3.77E-75 | 8.95E-84 | 6.90E-85 | 9.85E-84 |
| 43 | 6.75E-81 | 1.60E-81 | 9.46E-72 | 9.56E-79 |

(continued)

| No. | Cefuroxim_pv_adj | Ciprofloxacin_pv_adj | Clindamycin_pv_adj | Erythromycin_pv_adj |
|-----|-----|-----|-----|-----|
| 44 | 3.58E-81 | 2.83E-81 | 9.63E-72 | 1.15E-78 |
| 45 | 4.14E-80 | 2.83E-81 | 1.29E-70 | 1.77E-77 |
| 46 | 7.59E-62 | 5.15E-79 | 7.94E-76 | 5.98E-76 |
| 47 | 6.11E-79 | 1.32E-73 | 5.43E-98 | 2.20E-97 |
| 48 | 1.16E-77 | 7.87E-68 | 1.31E-67 | 3.92E-67 |
| 49 | 5.08E-59 | 3.55E-74 | 3.25E-71 | 5.25E-71 |
| 50 | 4.13E-78 | 4.35E-52 | 2.56E-53 | 1.24E-51 |

Table 23g: Data for Staphylococcus, particularly S. aureus (continued)

| No. | Imipenem_pv_adj | Levofloxacin_pv_adj | Moxifloxacin_pv_adj | Oxacillin_pv_adj |
|-----|-----|-----|-----|-----|
| 1 | 2.22E-97 | 8.36E-31 | 1.85E-167 | 9.38E-227 |
| 2 | 2.22E-97 | 8.36E-31 | 1.85E-167 | 9.38E-227 |
| 3 | 2.22E-97 | 8.36E-31 | 1.24E-168 | 2.67E-225 |
| 4 | 5.09E-91 | 5.82E-30 | 1.90E-168 | 1.77E-211 |
| 5 | 2.50E-88 | 1.50E-28 | 3.37E-166 | 3.22E-210 |
| 6 | 1.02E-75 | 5.49E-34 | 4.29E-188 | 2.96E-187 |
| 7 | 5.60E-79 | 1.02E-27 | 1.24E-168 | 6.72E-181 |
| 8 | 5.60E-79 | 1.02E-27 | 8.38E-166 | 6.72E-181 |
| 9 | 5.60E-79 | 1.02E-27 | 1.24E-168 | 6.72E-181 |
| 10 | 5.60E-79 | 1.02E-27 | 9.67E-167 | 8.54E-179 |
| 11 | 7.66E-78 | 1.02E-27 | 6.64E-164 | 8.54E-179 |
| 12 | 5.60E-79 | 5.41E-27 | 4.22E-160 | 1.82E-175 |
| 13 | 1.11E-77 | 1.82E-28 | 7.77E-170 | 3.23E-175 |
| 14 | 5.16E-79 | 1.02E-27 | 1.02E-172 | 9.13E-175 |
| 15 | 5.16E-79 | 1.02E-27 | 1.02E-172 | 9.13E-175 |
| 16 | 5.16E-79 | 1.02E-27 | 1.02E-172 | 9.13E-175 |
| 17 | 5.16E-79 | 1.02E-27 | 1.02E-172 | 9.13E-175 |
| 18 | 1.11E-77 | 4.43E-26 | 5.10E-171 | 2.26E-173 |
| 19 | 1.11E-77 | 4.43E-26 | 4.94E-170 | 2.40E-172 |
| 20 | 2.49E-65 | 7.88E-28 | 2.60E-167 | 1.43E-155 |
| 21 | 7.09E-65 | 7.88E-28 | 3.13E-166 | 2.33E-153 |
| 22 | 1.38E-64 | 8.83E-27 | 3.37E-166 | 1.17E-154 |
| 23 | 1.38E-64 | 8.83E-27 | 3.37E-166 | 1.17E-154 |
| 24 | 6.20E-63 | 2.26E-24 | 7.17E-161 | 3.62E-151 |
| 25 | 1.34E-61 | 1.22E-26 | 4.17E-152 | 4.81E-142 |
| 26 | 6.28E-51 | 3.77E-23 | 1.59E-135 | 2.07E-120 |
| 27 | 6.51E-51 | 2.53E-22 | 1.70E-135 | 8.40E-122 |

(continued)

| No. | Imipenem_pv_adj | Levofloxacin_pv_adj | Moxifloxacin_pv_adj | Oxacillin_pv_adj |
|-----|-----------------|---------------------|---------------------|------------------|
| 28 | 6.51E-51 | 2.53E-22 | 1.78E-134 | 8.52E-120 |
| 29 | 5.53E-51 | 1.45E-21 | 4.06E-133 | 1.84E-119 |
| 30 | 8.78E-49 | 9.16E-21 | 1.01E-130 | 3.53E-117 |
| 31 | 8.13E-44 | 1.25E-24 | 4.84E-126 | 3.22E-102 |
| 32 | 2.98E-44 | 2.87E-19 | 6.20E-122 | 5.33E-111 |
| 33 | 6.56E-47 | 5.69E-23 | 5.76E-120 | 7.13E-108 |
| 34 | 4.99E-46 | 2.00E-18 | 1.85E-116 | 2.91E-105 |
| 35 | 1.79E-38 | 1.08E-20 | 7.19E-116 | 1.17E-92 |
| 36 | 4.65E-43 | 6.88E-21 | 4.22E-113 | 2.39E-97 |
| 37 | 4.65E-43 | 6.88E-21 | 4.22E-113 | 2.39E-97 |
| 38 | 1.60E-42 | 7.01E-24 | 4.84E-112 | 7.55E-91 |
| 39 | 1.54E-44 | 3.19E-20 | 4.64E-110 | 2.85E-100 |
| 40 | 4.20E-36 | 3.14E-19 | 5.76E-108 | 5.09E-88 |
| 41 | 1.65E-39 | 8.87E-19 | 2.41E-107 | 6.05E-97 |
| 42 | 8.79E-40 | 1.09E-14 | 3.00E-106 | 3.22E-94 |
| 43 | 1.21E-43 | 1.71E-19 | 1.58E-102 | 1.41E-92 |
| 44 | 2.98E-44 | 8.40E-19 | 1.07E-101 | 4.03E-92 |
| 45 | 8.13E-44 | 8.40E-19 | 1.07E-101 | 4.03E-92 |
| 46 | 2.94E-31 | 2.14E-17 | 5.26E-101 | 5.94E-79 |
| 47 | 1.67E-43 | 1.76E-21 | 8.02E-101 | 1.17E-94 |
| 48 | 2.19E-35 | 1.61E-13 | 1.79E-95 | 4.56E-88 |
| 49 | 4.69E-30 | 2.14E-17 | 1.80E-94 | 5.69E-74 |
| 50 | 3.32E-41 | 8.45E-15 | 1.32E-71 | 2.83E-94 |

Table 23h: Data for Staphylococcus, particularly S. aureus (continued)

| No. | Penicillin G_pv_adj | Piperacillin/Tazobactam_pv_adj | Tetracyclin_pv_adj | Tobramycin_pv_adj |
|-----|---------------------|--------------------------------|--------------------|-------------------|
| 1 | 1.37E-41 | 1.55E-21 | 1.22E-01 | 2.16E-15 |
| 2 | 1.37E-41 | 1.55E-21 | 1.22E-01 | 2.16E-15 |
| 3 | 1.51E-41 | 1.55E-21 | 9.63E-02 | 2.16E-15 |
| 4 | 3.46E-40 | 1.17E-19 | 2.75E-02 | 6.21E-16 |
| 5 | 6.83E-41 | 2.25E-18 | 3.70E-02 | 2.16E-15 |
| 6 | 3.80E-41 | 1.59E-17 | 1.27E-02 | 1.94E-13 |
| 7 | 6.95E-35 | 3.69E-14 | 5.90E-03 | 7.81E-15 |
| 8 | 6.95E-35 | 3.69E-14 | 5.90E-03 | 7.81E-15 |
| 9 | 6.95E-35 | 3.69E-14 | 5.90E-03 | 7.81E-15 |
| 10 | 1.50E-34 | 3.69E-14 | 8.03E-03 | 7.81E-15 |
| 11 | 1.50E-34 | 3.69E-14 | 8.03E-03 | 7.81E-15 |

(continued)

| No. | Penicillin G_pv_adj | Piperacillin/Tazobactam_pv_adj | Tetracyclin_pv_adj | Tobramycin_pv_adj |
|-----|---------------------|-------------------------------|--------------------|--------------------|
| 12 | 5.97E-34 | 3.69E-14 | 8.79E-03 | 7.81E-15 |
| 13 | 5.30E-34 | 5.46E-13 | 9.97E-04 | 9.71E-16 |
| 14 | 1.23E-34 | 3.69E-14 | 1.42E-03 | 7.81E-15 |
| 15 | 1.23E-34 | 3.69E-14 | 1.42E-03 | 7.81E-15 |
| 16 | 1.23E-34 | 3.69E-14 | 1.42E-03 | 7.81E-15 |
| 17 | 1.23E-34 | 3.69E-14 | 1.42E-03 | 7.81E-15 |
| 18 | 1.23E-34 | 5.46E-13 | 1.42E-03 | 3.02E-14 |
| 19 | 1.23E-34 | 5.46E-13 | 1.42E-03 | 3.02E-14 |
| 20 | 5.10E-21 | 3.04E-11 | 5.98E-04 | 7.15E-15 |
| 21 | 9.90E-21 | 3.04E-11 | 2.52E-04 | 7.15E-15 |
| 22 | 2.82E-20 | 9.29E-11 | 5.97E-04 | 2.24E-14 |
| 23 | 2.82E-20 | 9.29E-11 | 5.97E-04 | 2.24E-14 |
| 24 | 1.92E-20 | 4.28E-09 | 8.65E-04 | 4.98E-13 |
| 25 | 1.42E-21 | 6.78E-10 | 2.40E-03 | 6.71E-15 |
| 26 | 2.07E-10 | 4.04E-10 | 5.35E-05 | 4.17E-12 |
| 27 | 1.36E-10 | 1.16E-09 | 2.27E-05 | 1.33E-11 |
| 28 | 2.18E-10 | 1.16E-09 | 3.46E-05 | 1.33E-11 |
| 29 | 4.59E-14 | 1.16E-09 | 8.29E-05 | 7.56E-11 |
| 30 | 6.54E-10 | 2.97E-08 | 3.46E-05 | 2.92E-10 |
| 31 | 4.06E-09 | 7.73E-08 | 2.02E-05 | 2.25E-08 |
| 32 | 2.64E-11 | 3.31E-11 | 2.07E-02 | 6.23E-11 |
| 33 | 2.28E-19 | 2.60E-08 | 2.52E-06 | 9.88E-10 |
| 34 | 1.20E-07 | 3.78E-10 | 1.24E-02 | 9.10E-11 |
| 35 | 4.14E-07 | 1.59E-06 | 1.78E-06 | 6.53E-07 |
| 36 | 4.05E-13 | 7.74E-09 | 1.25E-04 | 1.01E-10 |
| 37 | 4.05E-13 | 7.74E-09 | 1.25E-04 | 1.01E-10 |
| 38 | 4.22E-06 | 1.49E-07 | 1.46E-05 | 5.29E-08 |
| 39 | 1.18E-06 | 8.70E-08 | 2.19E-04 | 7.09E-11 |
| 40 | 1.87E-06 | 1.59E-06 | 3.63E-05 | 1.92E-07 |
| 41 | 4.31E-06 | 6.90E-07 | 1.08E-04 | 2.74E-09 |
| 42 | 6.34E-11 | 4.80E-05 | 2.10E-05 | 6.87E-09 |
| 43 | 2.66E-09 | 4.09E-08 | 4.92E-04 | 1.94E-10 |
| 44 | 7.22E-09 | 8.70E-08 | 3.32E-04 | 6.07E-10 |
| 45 | 2.79E-09 | 8.70E-08 | 3.32E-04 | 6.07E-10 |
| 46 | 1.54E-26 | 8.34E-08 | 2.05E-05 | 1.24E-11 |
| 47 | 4.32E-03 | 1.16E-09 | 4.05E-06 | 1.33E-11 |
| 48 | 1.28E-06 | 1.86E-05 | 1.61E-03 | 1.89E-08 |
| 49 | 3.43E-27 | 8.34E-08 | 1.11E-03 | 1.24E-11 |

(continued)

| No. | Penicillin G_pv_adj | Piperacillin/Tazobactam_pv_adj | Tetracyclin_pv_adj | Tobramycin_pv_adj |
|---|---|---|---|---|
| 50 | 2.12E-15 | 1.05E-10 | 6.95E-03 | 1.62E-07 |

Table 23i: Data for Staphylococcus, particularly S. aureus (continued)

| No. | Locus.Tag | Gene. Symbol | GenBank.Accession | Chromosome |
|---|---|---|---|---|
| 1 | HMPREF0791_ 1752 | mecA | ZP_04797671 | |
| 2 | HMPREF0794_ 1488 | ugpQ | ZP_06614206 | |
| 3 | HMPREF0794_1485 | mecR | ZP_06614203 | |
| 4 | SIAG_00762 | | ZP_06819251 | |
| 5 | HMPREF0789_ 1682 | | ZP_04825874 | |
| 6 | SAMG_01950 | | ZP_05682863 | |
| 7 | Missing locus_tag:FN433596.cds78.83191..82796 | | CBI47961 | |
| 8 | SATW20_00761 | | CBI67631 | |
| 9 | SERP2515 | | YP_190057 | |
| 10 | SAIG_02579 | | ZP_05694555 | |
| 11 | SERP2519 | mecl | YP_190060 | |
| 12 | SERP2505 | | YP_190047 | |
| 13 | HMPREF0794_ 1451 | | ZP_06614169 | |
| 14 | SERP2490 | kdeP | YP_190033 | |
| 15 | SERP2487 | kdpA | YP_190030 | |
| 16 | SERP2485 | kdpC | YP_190028 | |
| 17 | SERP2489 | kdpD | YP_190032 | |
| 18 | SERP2486 | kdpB | YP_190029 | |
| 19 | SAIG_01884 | | ZP_05693385 | |
| 20 | SERP1347 | tnpA-2 | YP_188919 | |
| 21 | SERP1223 | tnpB-1 | YP_188797 | |
| 22 | SLAG_02844 | | ZP_06930609 | |
| 23 | SA0049 | ant(9) | NP_373289 | |
| 24 | SERP1342 | | YP_188914 | |
| 25 | HMPREF0773_2426 | radC | ZP_04829125 | |
| 26 | SAFG_01021 | | ZP_05703394 | |
| 27 | SAFG_01015 | | ZP_05703388 | |
| 28 | SA2981_2149 | | ADC38360 | |
| 29 | ECTR2_1640 | | CBX35020 | |
| 30 | SA2981_1770 | | ADC37981 | |
| 31 | SAB0169 | | YP_415679 | |
| 32 | SaurJH1_0467 | | YP_001315613 | |
| 33 | SERP1345 | tnpC-2 | YP_188917 | |

(continued)

| No. | Locus.Tag | Gene. Symbol | GenBank.Accession | Chromosome |
|---|---|---|---|---|
| 34 | SGAG_02626 | | ZP_06303506 | |
| 35 | ECTR2_ 2075 | | CBX35404 | |
| 36 | HMPREF0774_2643 | | ZP_04869332 | |
| 37 | SAHG_01730 | | ZP_05696261 | |
| 38 | SAA6159_00204 | coa | ADL22160 | |
| 39 | SARG_01832 | | ZP_06332611 | |
| 40 | SAIG_01373 | | ZP_05693621 | |
| 41 | SANG_00111 | | ZP_05682471 | |
| 42 | SAHG_02693 | | ZP_05696106 | |
| 43 | SAJG_00740 | | ZP_05692517 | |
| 44 | SaurJH1_0188 | | YP_001315337 | |
| 45 | SGAG_00714 | | ZP_06301594 | |
| 46 | ECTR2_2580 | | CBX35910 | plasmid pLUH02 |
| 47 | SA2981_0414 | lpl3 | ADC36625 | |
| 48 | SA2981_1977 | agrB | ADC38188 | |
| 49 | SMAG_02765 | | ZP_06817387 | |
| 50 | HMPREF0798_ 02109 | | ZP_07844597 | |

Table 23j: Data for Staphylococcus, particularly S. aureus (continued)

| No. | Start.Coord | End.Coord | Strand | Scaffold.ID |
|---|---|---|---|---|
| 1 | 1335 | 3341 | + | 645076738 |
| 2 | 7348 | 8091 | - | 647019109 |
| 3 | 3685 | 4599 | - | 647019109 |
| 4 | 3987 | 5336 | - | 647537018 |
| 5 | 1842 | 3491 | - | 645076234 |
| 6 | 89170 | 89277 | - | 645958292 |
| 7 | 82796 | 84130 | - | 646862418 |
| 8 | 81146 | 81214 | - | 646862418 |
| 9 | 2570266 | 2571330 | + | 637000098 |
| 10 | 6265 | 7374 | + | 645958144 |
| 11 | 2574428 | 2574799 | - | 637000098 |
| 12 | 2562107 | 2562424 | + | 637000098 |
| 13 | 8938 | 9252 | + | 647019108 |
| 14 | 2549489 | 2550184 | + | 637000098 |
| 15 | 2544901 | 2546577 | - | 637000098 |
| 16 | 2542288 | 2542845 | - | 637000098 |
| 17 | 2546845 | 2549514 | + | 637000098 |

(continued)

| No. | Start.Coord | End.Coord | Strand | Scaffold.ID |
|-----|-------------|-----------|--------|-------------|
| 18 | 2542861 | 2544882 | - | 637000098 |
| 19 | 76580 | 76657 | - | 645958116 |
| 20 | 1403752 | 1404837 | - | 637000098 |
| 21 | 1255425 | 1257317 | - | 637000098 |
| 22 | 4557 | 5288 | - | 647016972 |
| 23 | 56859 | 57641 | - | 637000072 |
| 24 | 1399547 | 1399606 | + | 637000098 |
| 25 | 4478 | 5164 | - | 645076413 |
| 26 | 277066 | 278082 | + | 645957989 |
| 27 | 272112 | 272231 | - | 645957989 |
| 28 | 2299803 | 2300126 | - | 646862390 |
| 29 | 1804139 | 1805368 | - | 651053129 |
| 30 | 1899111 | 1899692 | + | 646862390 |
| 31 | 209129 | 211012 | + | 637000460 |
| 32 | 487880 | 489091 | + | 640753011 |
| 33 | 1401479 | 1401856 | - | 637000098 |
| 34 | 245 | 686 | - | 647008393 |
| 35 | 2210164 | 2210319 | - | 651053129 |
| 36 | 16441 | 17124 | + | 645076159 |
| 37 | 85620 | 86303 | + | 645958072 |
| 38 | 242193 | 244169 | + | 648231718 |
| 39 | 177946 | 180372 | + | 647535981 |
| 40 | 38140 | 39216 | + | 645958123 |
| 41 | 77988 | 79490 | - | 645958383 |
| 42 | 5009 | 5929 | - | 645958068 |
| 43 | 78131 | 78853 | + | 645958189 |
| 44 | 221049 | 221729 | + | 640753011 |
| 45 | 44419 | 44616 | + | 647008364 |
| 46 | 12682 | 13458 | - | 651053131 |
| 47 | 456367 | 457155 | + | 646862390 |
| 48 | 2096829 | 2097392 | + | 646862390 |
| 49 | 14918 | 15697 | - | 647017059 |
| 50 | 54037 | 54601 | + | 651285048 |

Table 24a: Data for Staphylococcus, particularly S. aureus

| No. | Scaffold.External. Accession | Scaffold.Name | best_pv |
|---|---|---|---|
| 1 | NZ_ACJC01000132 | Staphylococcus epidermidis W23144: NZ_ACJC01000132 | 9.38479881072327e-227 |
| 2 | NZ_ADMU01000036 | Staphylococcus epidermidis M23864:W2(grey) contig00037: NZ_ADMU01000036 | 9.38479881072327e-227 |
| 3 | NZ_ADMU01000036 | Staphylococcus epidermidis M23864:W2(grey) contig00037: NZ_ADMU01000036 | 2.67435403500704e-225 |
| 4 | NZ_GG770515 | Staphylococcus aureus subsp. aureus EMRSA16 genomic scaffold supercont1.3: NZ_GG770515 | 1.77186061517335e-211 |
| 5 | NZ_ACHE01000064 | Staphylococcus epidermidis BCM-HMP0060: NZ_ACHE01000064 | 3.22333061264623e-210 |
| 6 | NZ_ACKJ01000008 | Staphylococcus aureus A9719: NZ_ACKJ01000008 | 4.28801157250818e-188 |
| 7 | FN433596 | Staphylococcus aureus subsp. aureus TW20: FN433596 | 6.72085159673615e-181 |
| 8 | FN433596 | Staphylococcus aureus subsp. aureus TW20: FN433596 | 6.72085159673615e-181 |
| 9 | NC_002976 | Staphylococcus epidermidis RP62A: NC_002976 | 6.72085159673615e-181 |
| 10 | NZ_ACKF01000035 | Staphylococcus aureus A6300: NZ_ACKF01000035 | 8.5426103876016e-179 |
| 11 | NC_002976 | Staphylococcus epidermidis RP62A: NC_002976 | 8.5426103876016e-179 |
| 12 | NC_002976 | Staphylococcus epidermidis RP62A: NC_002976 | 1.81714784068785e-175 |
| 13 | NZ_ADMU01000035 | Staphylococcus epidermidis M23864:W2(grey) contig00036: NZ_ADMU01000035 | 3.23410033711171e-175 |
| 14 | NC_002976 | Staphylococcus epidermidis RP62A: NC_002976 | 9.12700235375297e-175 |
| 15 | NC_002976 | Staphylococcus epidermidis RP62A: NC_002976 | 9.12700235375297e-175 |
| 16 | NC_002976 | Staphylococcus epidermidis RP62A: NC_002976 | 9.12700235375297e-175 |
| 17 | NC_002976 | Staphylococcus epidermidis RP62A: NC_002976 | 9.12700235375297e-175 |
| 18 | NC_002976 | Staphylococcus epidermidis RP62A: NC_002976 | 2.25944344923286e-173 |
| 19 | NZ_ACKF01000007 | Staphylococcus aureus A6300: NZ_ACKF01000007 | 2.40063417489507e-172 |
| 20 | NC_002976 | Staphylococcus epidermidis RP62A: NC_002976 | 2.59802993040457e-167 |
| 21 | NC_002976 | Staphylococcus epidermidis RP62A: NC_002976 | 3.12953724144125e-166 |

(continued)

| No. | Scaffold.External. Accession | Scaffold.Name | best_pv |
|---|---|---|---|
| 22 | NZ_ADJJ01000031 | Staphylococcus aureus A8796 cont1.31: NZ_ADJJ01000031 | 3.37208086862656e-166 |
| 23 | NC_002745 | Staphylococcus aureus subsp. aureus N315: NC_002745 | 3.37208086862656e-166 |
| 24 | NC_002976 | Staphylococcus epidermidis RP62A: NC_002976 | 7.17319474297637e-161 |
| 25 | NZ_ACHH01000128 | Staphylococcus aureus subsp. aureus TCH70: NZ_ACHH01000128 | 4.16856451727179e-152 |
| 26 | NZ_ACKC01000026 | Staphylococcus aureus A5937: NZ_ACKC01000026 | 1.58751373713434e-135 |
| 27 | NZ_ACKC01000026 | Staphylococcus aureus A5937: NZ_ACKC01000026 | 1.69633422486642e-135 |
| 28 | CP001844 | Staphylococcus aureus 04-02981: CP001844 | 1.77656315889944e-134 |
| 29 | FR714927 | Staphylococcus aureus subsp. aureus ECT-R 2: FR714927 | 4.06264970457926e-133 |
| 30 | CP001844 | Staphylococcus aureus 04-02981: CP001844 | 1.01251954267327e-130 |
| 31 | NC_007622 | Staphylococcus aureus RF122: NC_007622 | 4.84169534955158e-126 |
| 32 | NC_009632 | Staphylococcus aureus subsp. aureus JH1: NC_009632 | 6.20485118760467e-122 |
| 33 | NC_002976 | Staphylococcus epidermidis RP62A: NC_002976 | 5.75915090813908e-120 |
| 34 | NZ_ACY001000032 | Staphylococcus aureus A8117 cont1.32: NZ_ACY001000032 | 1.84672228386398e-116 |
| 35 | FR714927 | Staphylococcus aureus subsp. aureus ECT-R 2: FR714927 | 7.1946440374743e-116 |
| 36 | NZ_ACHD01000273 | Staphylococcus aureus subsp. aureus TCH130: NZ_ACHD01000273 | 4.22320321058421e-113 |
| 37 | NZ_ACKE01000012 | Staphylococcus aureus A6224: NZ_ACKE01000012 | 4.22320321058421e-113 |
| 38 | CP002114 | Staphylococcus aureus subsp. aureus JKD6159: CP002114 | 4.83609713640718e-112 |
| 39 | NZ_GG730131 | Staphylococcus aureus subsp. aureus C101 genomic scaffold super-cont1.12: NZ_GG730131 | 4.63942446669448e-110 |
| 40 | NZ_ACKF01000014 | Staphylococcus aureus A6300: NZ_ACKF01000014 | 5.76090691723747e-108 |
| 41 | NZ_ACKK01000039 | Staphylococcus aureus A9763: NZ_ACKK01000039 | 2.40583396327414e-107 |
| 42 | NZ_ACKE01000008 | Staphylococcus aureus A6224: NZ_ACKE01000008 | 2.99974766558167e-106 |
| 43 | NZ_ACKG01000031 | Staphylococcus aureus A8115: NZ_ACKG01000031 | 1.57559858677166e-102 |

(continued)

| No. | Scaffold.External. Accession | Scaffold.Name | best_pv |
|---|---|---|---|
| 44 | NC_009632 | Staphylococcus aureus subsp. aureus JH1: NC_009632 | 1.0725409335823e-101 |
| 45 | NZ_ACY001000003 | Staphylococcus aureus A8117 cont1.3: NZ_ACY001000003 | 1.0725409335823e-101 |
| 46 | FR714929 | Staphylococcus aureus subsp. aureus ECT-R 2 plasmid pLUH02: FR714929 | 5.26123840198017e-101 |
| 47 | CP001844 | Staphylococcus aureus 04-02981: CP001844 | 8.01600909282956e-101 |
| 48 | CP001844 | Staphylococcus aureus 04-02981: CP001844 | 1.78546333248495e-95 |
| 49 | NZ_ADJK01000020 | Staphylococcus aureus A8819 cont1.20: NZ_ADJK01000020 | 1.80469278143707e-94 |
| 50 | NZ_GL545262 | Staphylococcus hominis subsp. hominis C80 genomic scaffold supercont1.11: NZ_GL545262 | 2.82728623419544e-94 |

Table 24b: Data for Staphylococcus, particularly S. aureus (continued)

| No. | sign_phenos |
|---|---|
| 1 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 2 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 3 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 4 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 5 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 6 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tobramycin |
| 7 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 8 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 9 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |

(continued)

| No. | sign_phenos |
| --- | --- |
| 10 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 11 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 12 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 13 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 14 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 15 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 16 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 17 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 18 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 19 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 20 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 21 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 22 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 23 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 24 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |

(continued)

| No. | sign_phenos |
|---|---|
| 25 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 26 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 27 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 28 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 29 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 30 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 31 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 32 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tobramycin |
| 33 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 34 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tobramycin |
| 35 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 36 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 37 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 38 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 39 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |

(continued)

| No. | sign_phenos |
|---|---|
| 40 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 41 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 42 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 43 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 44 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 45 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 46 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 47 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 48 | Ampicillin/Sulbactam;Cefepim;Cefotaxim;Cefuroxim;Ciprofloxacin;Clindamycin; Erythromycin;Imipenem;Levofloxaci n;Moxifloxacin;Oxacillin;Penicillin G;Piperacillin/ Tazobactam;Tetracyclin;Tobramycin |
| 49 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |
| 50 | Ampicillin;Ampicillin/Sulbactam;Cefepim;Cefotaxim;Cefuroxim;Ciprofloxacin; Clindamycin;Erythromycin;Imipenem; Levofloxacin;Moxifloxacin;Oxacillin;Penicillin G; Piperacillin/Tazobactam;Tetracyclin;Tobramycin |

Table 24c: Data for Staphylococcus, particularly S. aureus (continued)

| No. | sign_phenos_class | best_pheno | best_pheno_class |
|---|---|---|---|
| 1 | aminoglycoside;fluoroquinolone;lactam;lincosamide;macrolide | Oxacillin | lactam |
| 2 | aminoglycoside;fluoroquinolone;lactam;lincosamide;macrolide | Oxacillin | lactam |
| 3 | aminoglycoside;fluoroquinolone;lactam;lincosamide;macrolide | Oxacillin | lactam |
| 4 | aminoglycoside;fluoroquinolone;lactam;lincosamide;macrolide | Oxacillin | lactam |
| 5 | aminoglycoside;fluoroquinolone;lactam;lincosamide;macrolide | Oxacillin | lactam |
| 6 | aminoglycoside;fluoroquinolone;lactam;lincosamide;macrolide | Moxifloxacin | fluoroquinolone |
| 7 | aminoglycoside;fluoroquinolone;lactam;lincosamide;macrolide; tetracycline | Oxacillin | lactam |

(continued)

| No. | sign_phenos_class | best_pheno | best_pheno_class |
|---|---|---|---|
| 8 | aminoglycoside;fluoroquinolone;lactam;lincosamide;macrolide; tetracycline | Oxacillin | lactam |
| 9 | aminoglycoside;fluoroquinolone;lactam;lincosamide;macrolide; tetracycline | Oxacillin | lactam |
| 10 | aminoglycoside;fluoroquinolone;lactam;lincosamide;macrolide; tetracycline | Oxacillin | lactam |
| 11 | aminoglycoside;fluoroquinolone;lactam;lincosamide;macrolide; tetracycline | Oxacillin | lactam |
| 12 | aminoglycoside;fluoroquinolone;lactam;lincosamide;macrolide; tetracycline | Oxacillin | lactam |
| 13 | aminoglycoside;fluoroquinolone;lactam;lincosamide;macrolide; tetracycline | Oxacillin | lactam |
| 14 | aminoglycoside;fluoroquinolone;lactam;lincosamide;macrolide; tetracycline | Oxacillin | lactam |
| 15 | aminoglycoside;fluoroquinolone;lactam;lincosamide;macrolide; tetracycline | Oxacillin | lactam |
| 16 | aminoglycoside;fluoroquinolone;lactam;lincosamide;macrolide; tetracycline | Oxacillin | lactam |
| 17 | aminoglycoside;fluoroquinolone;lactam;lincosamide;macrolide; tetracycline | Oxacillin | lactam |
| 18 | aminoglycoside;fluoroquinolone;lactam;lincosamide;macrolide; tetracycline | Oxacillin | lactam |
| 19 | aminoglycoside;fluoroquinolone;lactam;lincosamide;macrolide; tetracycline | Oxacillin | lactam |
| 20 | aminoglycoside;fluoroquinolone;lactam;lincosamide;macrolide; tetracycline | Moxifloxacin | fluoroquinolone |
| 21 | aminoglycoside;fluoroquinolone;lactam;lincosamide;macrolide; tetracycline | Moxifloxacin | fluoroquinolone |
| 22 | aminoglycoside;fluoroquinolone;lactam;lincosamide;macrolide; tetracycline | Moxifloxacin | fluoroquinolone |
| 23 | aminoglycoside;fluoroquinolone;lactam;lincosamide;macrolide; tetracycline | Moxifloxacin | fluoroquinolone |
| 24 | aminoglycoside;fluoroquinolone;lactam;lincosamide;macrolide; tetracycline | Moxifloxacin | fluoroquinolone |
| 25 | aminoglycoside;fluoroquinolone;lactam;lincosamide;macrolide; tetracycline | Moxifloxacin | fluoroquinolone |
| 26 | aminoglycoside;fluoroquinolone;lactam;lincosamide;macrolide; tetracycline | Moxifloxacin | fluoroquinolone |
| 27 | aminoglycoside;fluoroquinolone;lactam;lincosamide;macrolide; tetracycline | Moxifloxacin | fluoroquinolone |
| 28 | aminoglycoside;fluoroquinolone;lactam;lincosamide;macrolide; tetracycline | Moxifloxacin | fluoroquinolone |
| 29 | aminoglycoside;fluoroquinolone;lactam;lincosamide;macrolide; tetracycline | Moxifloxacin | fluoroquinolone |

(continued)

| No. | sign_phenos_class | best_pheno | best_pheno_class |
|---|---|---|---|
| 30 | aminoglycoside;fluoroquinolone;lactam;lincosamide;macrolide; tetracycline | Moxifloxacin | fluoroquinolone |
| 31 | aminoglycoside;fluoroquinolone;lactam;lincosamide;macrolide; tetracycline | Moxifloxacin | fluoroquinolone |
| 32 | aminoglycoside;fluoroquinolone;lactam;lincosamide;macrolide | Moxifloxacin | fluoroquinolone |
| 33 | aminoglycoside;fluoroquinolone;lactam;lincosamide;macrolide; tetracycline | Moxifloxacin | fluoroquinolone |
| 34 | aminoglycoside;fluoroquinolone;lactam;lincosamide;macrolide | Moxifloxacin | fluoroquinolone |
| 35 | aminoglycoside;fluoroquinolone;lactam;lincosamide;macrolide; tetracycline | Moxifloxacin | fluoroquinolone |
| 36 | aminoglycoside;fluoroquinolone;lactam;lincosamide;macrolide; tetracycline | Moxifloxacin | fluoroquinolone |
| 37 | aminoglycoside;fluoroquinolone;lactam;lincosamide;macrolide; tetracycline | Moxifloxacin | fluoroquinolone |
| 38 | aminoglycoside;fluoroquinolone;lactam;lincosamide;macrolide; tetracycline | Moxifloxacin | fluoroquinolone |
| 39 | aminoglycoside;fluoroquinolone;lactam;lincosamide;macrolide; tetracycline | Moxifloxacin | fluoroquinolone |
| 40 | aminoglycoside;fluoroquinolone;lactam;lincosamide;macrolide; tetracycline | Moxifloxacin | fluoroquinolone |
| 41 | aminoglycoside;fluoroquinolone;lactam;lincosamide;macrolide; tetracycline | Moxifloxacin | fluoroquinolone |
| 42 | aminoglycoside;fluoroquinolone;lactam;lincosamide;macrolide; tetracycline | Moxifloxacin | fluoroquinolone |
| 43 | aminoglycoside;fluoroquinolone;lactam;lincosamide;macrolide; tetracycline | Moxifloxacin | fluoroquinolone |
| 44 | aminoglycoside;fluoroquinolone;lactam;lincosamide;macrolide; tetracycline | Moxifloxacin | fluoroquinolone |
| 45 | aminoglycoside;fluoroquinolone;lactam;lincosamide;macrolide; tetracycline | Moxifloxacin | fluoroquinolone |
| 46 | aminoglycoside;fluoroquinolone;lactam;lincosamide;macrolide; tetracycline | Moxifloxacin | fluoroquinolone |
| 47 | aminoglycoside;fluoroquinolone;lactam;lincosamide;macrolide; tetracycline | Moxifloxacin | fluoroquinolone |
| 48 | aminoglycoside;fluoroquinolone;lactam;lincosamide;macrolide; tetracycline | Moxifloxacin | fluoroquinolone |
| 49 | aminoglycoside;fluoroquinolone;lactam;lincosamide;macrolide; tetracycline | Moxifloxacin | fluoroquinolone |
| 50 | aminoglycoside;fluoroquinolone;lactam;lincosamide;macrolide; tetracycline | Oxacillin | lactam |

Table 24d: Data for Staphylococcus, particularly S. aureus (continued)

| No. | num_aminoglycoside | num_fluoroquinolone | num_lactam | num_lincosamide | num_macrolide | num_tetracycline |
|---|---|---|---|---|---|---|
| 1 | 1 | 3 | 9 | 1 | 1 | 0 |
| 2 | 1 | 3 | 9 | 1 | 1 | 0 |
| 3 | 1 | 3 | 9 | 1 | 1 | 0 |
| 4 | 1 | 3 | 9 | 1 | 1 | 0 |
| 5 | 1 | 3 | 9 | 1 | 1 | 0 |
| 6 | 1 | 3 | 9 | 1 | 1 | 0 |
| 7 | 1 | 3 | 9 | 1 | 1 | 1 |
| 8 | 1 | 3 | 9 | 1 | 1 | 1 |
| 9 | 1 | 3 | 9 | 1 | 1 | 1 |
| 10 | 1 | 3 | 9 | 1 | 1 | 1 |
| 11 | 1 | 3 | 9 | 1 | 1 | 1 |
| 12 | 1 | 3 | 9 | 1 | 1 | 1 |
| 13 | 1 | 3 | 9 | 1 | 1 | 1 |
| 14 | 1 | 3 | 9 | 1 | 1 | 1 |
| 15 | 1 | 3 | 9 | 1 | 1 | 1 |
| 16 | 1 | 3 | 9 | 1 | 1 | 1 |
| 17 | 1 | 3 | 9 | 1 | 1 | 1 |
| 18 | 1 | 3 | 9 | 1 | 1 | 1 |
| 19 | 1 | 3 | 9 | 1 | 1 | 1 |
| 20 | 1 | 3 | 9 | 1 | 1 | 1 |
| 21 | 1 | 3 | 9 | 1 | 1 | 1 |
| 22 | 1 | 3 | 9 | 1 | 1 | 1 |
| 23 | 1 | 3 | 9 | 1 | 1 | 1 |
| 24 | 1 | 3 | 9 | 1 | 1 | 1 |
| 25 | 1 | 3 | 9 | 1 | 1 | 1 |
| 26 | 1 | 3 | 8 | 1 | 1 | 1 |
| 27 | 1 | 3 | 8 | 1 | 1 | 1 |
| 28 | 1 | 3 | 8 | 1 | 1 | 1 |
| 29 | 1 | 3 | 8 | 1 | 1 | 1 |
| 30 | 1 | 3 | 8 | 1 | 1 | 1 |
| 31 | 1 | 3 | 9 | 1 | 1 | 1 |
| 32 | 1 | 3 | 8 | 1 | 1 | 0 |
| 33 | 1 | 3 | 9 | 1 | 1 | 1 |
| 34 | 1 | 3 | 8 | 1 | 1 | 0 |
| 35 | 1 | 3 | 8 | 1 | 1 | 1 |
| 36 | 1 | 3 | 9 | 1 | 1 | 1 |
| 37 | 1 | 3 | 9 | 1 | 1 | 1 |

(continued)

| No. | num_aminoglycoside | num_fluoroquinolone | num_lactam | num_lincosamide | num_macrolide | num_tetracycline |
|---|---|---|---|---|---|---|
| 38 | 1 | 3 | 8 | 1 | 1 | 1 |
| 39 | 1 | 3 | 8 | 1 | 1 | 1 |
| 40 | 1 | 3 | 8 | 1 | 1 | 1 |
| 41 | 1 | 3 | 8 | 1 | 1 | 1 |
| 42 | 1 | 3 | 8 | 1 | 1 | 1 |
| 43 | 1 | 3 | 8 | 1 | 1 | 1 |
| 44 | 1 | 3 | 8 | 1 | 1 | 1 |
| 45 | 1 | 3 | 8 | 1 | 1 | 1 |
| 46 | 1 | 3 | 9 | 1 | 1 | 1 |
| 47 | 1 | 3 | 8 | 1 | 1 | 1 |
| 48 | 1 | 3 | 8 | 1 | 1 | 1 |
| 49 | 1 | 3 | 9 | 1 | 1 | 1 |
| 50 | 1 | 3 | 9 | 1 | 1 | 1 |

Table 24e: Data for Staphylococcus, particularly S. aureus (continued)

| No. | Ampicillin_pv_adj | Ampicillin/Sulbactam_pv_adj | Cefepim_pv_adj | Cefotaxim_pv_adj |
|---|---|---|---|---|
| 1 | 2.20E-08 | 5.96E-37 | 5.96E-37 | 5.96E-37 |
| 2 | 2.20E-08 | 5.96E-37 | 5.96E-37 | 5.96E-37 |
| 3 | 2.20E-08 | 5.96E-37 | 5.96E-37 | 5.96E-37 |
| 4 | 4.64E-08 | 4.55E-35 | 4.55E-35 | 4.55E-35 |
| 5 | 2.20E-08 | 2.16E-33 | 2.16E-33 | 2.16E-33 |
| 6 | 2.22E-09 | 1.64E-31 | 1.64E-31 | 1.64E-31 |
| 7 | 3.22E-07 | 2.72E-27 | 2.72E-27 | 2.72E-27 |
| 8 | 3.22E-07 | 2.72E-27 | 2.72E-27 | 2.72E-27 |
| 9 | 3.22E-07 | 2.72E-27 | 2.72E-27 | 2.72E-27 |
| 10 | 3.22E-07 | 2.72E-27 | 2.72E-27 | 2.72E-27 |
| 11 | 3.22E-07 | 2.72E-27 | 2.72E-27 | 2.72E-27 |
| 12 | 6.85E-07 | 2.72E-27 | 2.72E-27 | 2.72E-27 |
| 13 | 2.90E-07 | 1.05E-25 | 1.05E-25 | 1.05E-25 |
| 14 | 3.22E-07 | 2.72E-27 | 2.72E-27 | 2.72E-27 |
| 15 | 3.22E-07 | 2.72E-27 | 2.72E-27 | 2.72E-27 |
| 16 | 3.22E-07 | 2.72E-27 | 2.72E-27 | 2.72E-27 |
| 17 | 3.22E-07 | 2.72E-27 | 2.72E-27 | 2.72E-27 |
| 18 | 2.90E-07 | 1.05E-25 | 1.05E-25 | 1.05E-25 |
| 19 | 2.90E-07 | 1.05E-25 | 1.05E-25 | 1.05E-25 |
| 20 | 8.65E-04 | 1.18E-21 | 1.18E-21 | 1.18E-21 |
| 21 | 8.65E-04 | 1.18E-21 | 1.18E-21 | 1.18E-21 |

(continued)

| No. | Ampicillin_pv_adj | Ampicillin/Sulbactam_pv_adj | Cefepim_pv_adj | Cefotaxim_pv_adj |
|---|---|---|---|---|
| 22 | 3.21E-03 | 7.21E-21 | 7.21E-21 | 7.21E-21 |
| 23 | 3.21E-03 | 7.21E-21 | 7.21E-21 | 7.21E-21 |
| 24 | 1.55E-03 | 1.27E-19 | 1.27E-19 | 1.27E-19 |
| 25 | 1.83E-03 | 2.01E-19 | 2.01E-19 | 2.01E-19 |
| 26 | 1.26E-02 | 1.32E-20 | 1.32E-20 | 1.32E-20 |
| 27 | 1.18E-02 | 7.81E-20 | 7.81E-20 | 7.81E-20 |
| 28 | 1.18E-02 | 7.81E-20 | 7.81E-20 | 7.81E-20 |
| 29 | 1.26E-02 | 7.25E-19 | 7.25E-19 | 7.25E-19 |
| 30 | 3.28E-02 | 3.76E-18 | 3.76E-18 | 3.76E-18 |
| 31 | 4.05E-03 | 1.35E-15 | 1.35E-15 | 1.35E-15 |
| 32 | 1.24E-02 | 1.13E-19 | 1.13E-19 | 1.13E-19 |
| 33 | 1.11E-03 | 1.73E-15 | 1.73E-15 | 1.73E-15 |
| 34 | 1.27E-02 | 6.35E-19 | 6.35E-19 | 6.35E-19 |
| 35 | 2.32E-02 | 3.05E-13 | 3.05E-13 | 3.05E-13 |
| 36 | 7.10E-03 | 2.03E-18 | 2.03E-18 | 2.03E-18 |
| 37 | 7.10E-03 | 2.03E-18 | 2.03E-18 | 2.03E-18 |
| 38 | 1.24E-02 | 2.91E-15 | 2.91E-15 | 2.91E-15 |
| 39 | 3.37E-02 | 1.60E-16 | 1.60E-16 | 1.60E-16 |
| 40 | 5.70E-02 | 1.88E-12 | 1.88E-12 | 1.88E-12 |
| 41 | 1.15E-01 | 1.25E-15 | 1.25E-15 | 1.25E-15 |
| 42 | 3.84E-02 | 9.78E-11 | 9.78E-11 | 9.78E-11 |
| 43 | 3.56E-02 | 5.77E-17 | 5.77E-17 | 5.77E-17 |
| 44 | 3.37E-02 | 1.60E-16 | 1.60E-16 | 1.60E-16 |
| 45 | 3.37E-02 | 1.60E-16 | 1.60E-16 | 1.60E-16 |
| 46 | 2.90E-07 | 1.61E-15 | 1.61E-15 | 1.61E-15 |
| 47 | 7.69E-02 | 7.81E-20 | 7.81E-20 | 7.81E-20 |
| 48 | 8.07E-02 | 6.08E-12 | 6.08E-12 | 6.08E-12 |
| 49 | 2.90E-07 | 1.61E-15 | 1.61E-15 | 1.61E-15 |
| 50 | 3.04E-06 | 1.82E-17 | 1.82E-17 | 1.82E-17 |

Table 24f: Data for Staphylococcus, particularly S. aureus (continued)

| No. | Cefuroxim_pv_adj | Ciprofloxacin_pv_adj | Clindamycin_pv_adj | Erythromycin_pv_adj |
|---|---|---|---|---|
| 1 | 1.02E-172 | 4.82E-131 | 5.41E-111 | 1.04E-111 |
| 2 | 1.02E-172 | 4.82E-131 | 5.41E-111 | 1.04E-111 |
| 3 | 1.02E-172 | 3.02E-132 | 6.31E-112 | 7.39E-113 |
| 4 | 1.19E-170 | 1.00E-131 | 5.45E-114 | 1.72E-111 |
| 5 | 9.55E-169 | 1.65E-131 | 8.55E-113 | 3.44E-110 |

(continued)

| No. | Cefuroxim_pv_adj | Ciprofloxacin_pv_adj | Clindamycin_pv_adj | Erythromycin_pv_adj |
|---|---|---|---|---|
| 6 | 3.09E-155 | 2.03E-145 | 7.78E-102 | 4.54E-100 |
| 7 | 2.00E-140 | 4.08E-138 | 6.00E-125 | 2.44E-127 |
| 8 | 2.00E-140 | 5.95E-135 | 7.70E-123 | 2.44E-127 |
| 9 | 2.00E-140 | 4.08E-138 | 6.00E-125 | 2.44E-127 |
| 10 | 2.29E-138 | 2.11E-136 | 1.31E-123 | 4.65E-126 |
| 11 | 2.29E-138 | 3.02E-133 | 1.67E-121 | 4.65E-126 |
| 12 | 1.01E-138 | 1.36E-128 | 4.01E-120 | 1.01E-123 |
| 13 | 2.71E-145 | 1.43E-135 | 2.49E-122 | 2.94E-121 |
| 14 | 5.87E-142 | 6.84E-140 | 7.88E-125 | 3.61E-124 |
| 15 | 5.87E-142 | 6.84E-140 | 7.88E-125 | 3.61E-124 |
| 16 | 5.87E-142 | 6.84E-140 | 7.88E-125 | 3.61E-124 |
| 17 | 5.87E-142 | 6.84E-140 | 7.88E-125 | 3.61E-124 |
| 18 | 3.15E-140 | 6.84E-140 | 1.65E-123 | 1.01E-122 |
| 19 | 3.78E-139 | 5.24E-139 | 7.90E-123 | 4.58E-122 |
| 20 | 3.99E-125 | 5.65E-132 | 3.88E-136 | 8.83E-140 |
| 21 | 5.82E-123 | 4.09E-130 | 1.24E-134 | 2.39E-138 |
| 22 | 4.30E-124 | 5.65E-132 | 7.67E-139 | 1.30E-142 |
| 23 | 4.30E-124 | 5.65E-132 | 7.67E-139 | 1.30E-142 |
| 24 | 8.70E-122 | 4.09E-130 | 4.42E-135 | 5.01E-139 |
| 25 | 1.95E-112 | 3.48E-117 | 1.09E-121 | 8.26E-126 |
| 26 | 9.99E-102 | 2.00E-99 | 7.42E-94 | 1.88E-93 |
| 27 | 9.92E-101 | 1.25E-100 | 4.84E-95 | 2.65E-94 |
| 28 | 1.08E-99 | 6.62E-99 | 5.06E-95 | 1.80E-94 |
| 29 | 4.45E-100 | 1.46E-101 | 3.05E-98 | 1.82E-99 |
| 30 | 3.62E-99 | 6.76E-98 | 4.11E-89 | 2.04E-88 |
| 31 | 5.46E-82 | 8.46E-90 | 4.95E-69 | 2.56E-67 |
| 32 | 3.45E-93 | 1.68E-88 | 1.37E-90 | 2.27E-88 |
| 33 | 7.10E-92 | 7.29E-86 | 6.57E-78 | 1.16E-76 |
| 34 | 3.46E-90 | 5.20E-83 | 1.49E-80 | 4.89E-80 |
| 35 | 9.56E-73 | 1.94E-83 | 1.78E-63 | 2.45E-61 |
| 36 | 1.95E-83 | 3.50E-91 | 5.92E-79 | 2.14E-84 |
| 37 | 1.95E-83 | 3.50E-91 | 5.92E-79 | 2.14E-84 |
| 38 | 7.71E-76 | 4.08E-77 | 6.05E-58 | 6.49E-57 |
| 39 | 3.93E-89 | 4.20E-78 | 5.89E-71 | 3.43E-72 |
| 40 | 3.54E-69 | 3.19E-77 | 4.43E-58 | 3.86E-57 |
| 41 | 8.95E-84 | 1.49E-77 | 1.71E-74 | 1.87E-74 |
| 42 | 3.77E-75 | 8.95E-84 | 6.90E-85 | 9.85E-84 |
| 43 | 6.75E-81 | 1.60E-81 | 9.46E-72 | 9.56E-79 |

(continued)

| No. | Cefuroxim_pv_adj | Ciprofloxacin_pv_adj | Clindamycin_pv_adj | Erythromycin_pv_adj |
|-----|------------------|----------------------|--------------------|---------------------|
| 44 | 3.58E-81 | 2.83E-81 | 9.63E-72 | 1.15E-78 |
| 45 | 4.14E-80 | 2.83E-81 | 1.29E-70 | 1.77E-77 |
| 46 | 7.59E-62 | 5.15E-79 | 7.94E-76 | 5.98E-76 |
| 47 | 6.11E-79 | 1.32E-73 | 5.43E-98 | 2.20E-97 |
| 48 | 1.16E-77 | 7.87E-68 | 1.31E-67 | 3.92E-67 |
| 49 | 5.08E-59 | 3.55E-74 | 3.25E-71 | 5.25E-71 |
| 50 | 4.13E-78 | 4.35E-52 | 2.56E-53 | 1.24E-51 |

Table 24g: Data for Staphylococcus, particularly S. aureus (continued)

| No. | Imipenem_pv_adj | Levofloxacin_pv_adj | Moxifloxacin_pv_adj | Oxacillin_pv_adj |
|-----|-----------------|---------------------|---------------------|-------------------|
| 1 | 2.22E-97 | 8.36E-31 | 1.85E-167 | 9.38E-227 |
| 2 | 2.22E-97 | 8.36E-31 | 1.85E-167 | 9.38E-227 |
| 3 | 2.22E-97 | 8.36E-31 | 1.24E-168 | 2.67E-225 |
| 4 | 5.09E-91 | 5.82E-30 | 1.90E-168 | 1.77E-211 |
| 5 | 2.50E-88 | 1.50E-28 | 3.37E-166 | 3.22E-210 |
| 6 | 1.02E-75 | 5.49E-34 | 4.29E-188 | 2.96E-187 |
| 7 | 5.60E-79 | 1.02E-27 | 1.24E-168 | 6.72E-181 |
| 8 | 5.60E-79 | 1.02E-27 | 8.38E-166 | 6.72E-181 |
| 9 | 5.60E-79 | 1.02E-27 | 1.24E-168 | 6.72E-181 |
| 10 | 5.60E-79 | 1.02E-27 | 9.67E-167 | 8.54E-179 |
| 11 | 7.66E-78 | 1.02E-27 | 6.64E-164 | 8.54E-179 |
| 12 | 5.60E-79 | 5.41E-27 | 4.22E-160 | 1.82E-175 |
| 13 | 1.11E-77 | 1.82E-28 | 7.77E-170 | 3.23E-175 |
| 14 | 5.16E-79 | 1.02E-27 | 1.02E-172 | 9.13E-175 |
| 15 | 5.16E-79 | 1.02E-27 | 1.02E-172 | 9.13E-175 |
| 16 | 5.16E-79 | 1.02E-27 | 1.02E-172 | 9.13E-175 |
| 17 | 5.16E-79 | 1.02E-27 | 1.02E-172 | 9.13E-175 |
| 18 | 1.11E-77 | 4.43E-26 | 5.10E-171 | 2.26E-173 |
| 19 | 1.11E-77 | 4.43E-26 | 4.94E-170 | 2.40E-172 |
| 20 | 2.49E-65 | 7.88E-28 | 2.60E-167 | 1.43E-155 |
| 21 | 7.09E-65 | 7.88E-28 | 3.13E-166 | 2.33E-153 |
| 22 | 1.38E-64 | 8.83E-27 | 3.37E-166 | 1.17E-154 |
| 23 | 1.38E-64 | 8.83E-27 | 3.37E-166 | 1.17E-154 |
| 24 | 6.20E-63 | 2.26E-24 | 7.17E-161 | 3.62E-151 |
| 25 | 1.34E-61 | 1.22E-26 | 4.17E-152 | 4.81E-142 |
| 26 | 6.28E-51 | 3.77E-23 | 1.59E-135 | 2.07E-120 |
| 27 | 6.51E-51 | 2.53E-22 | 1.70E-135 | 8.40E-122 |

(continued)

| No. | Imipenem_pv_adj | Levofloxacin_pv_adj | Moxifloxacin_pv_adj | Oxacillin_pv_adj |
|---|---|---|---|---|
| 28 | 6.51E-51 | 2.53E-22 | 1.78E-134 | 8.52E-120 |
| 29 | 5.53E-51 | 1.45E-21 | 4.06E-133 | 1.84E-119 |
| 30 | 8.78E-49 | 9.16E-21 | 1.01E-130 | 3.53E-117 |
| 31 | 8.13E-44 | 1.25E-24 | 4.84E-126 | 3.22E-102 |
| 32 | 2.98E-44 | 2.87E-19 | 6.20E-122 | 5.33E-111 |
| 33 | 6.56E-47 | 5.69E-23 | 5.76E-120 | 7.13E-108 |
| 34 | 4.99E-46 | 2.00E-18 | 1.85E-116 | 2.91E-105 |
| 35 | 1.79E-38 | 1.08E-20 | 7.19E-116 | 1.17E-92 |
| 36 | 4.65E-43 | 6.88E-21 | 4.22E-113 | 2.39E-97 |
| 37 | 4.65E-43 | 6.88E-21 | 4.22E-113 | 2.39E-97 |
| 38 | 1.60E-42 | 7.01E-24 | 4.84E-112 | 7.55E-91 |
| 39 | 1.54E-44 | 3.19E-20 | 4.64E-110 | 2.85E-100 |
| 40 | 4.20E-36 | 3.14E-19 | 5.76E-108 | 5.09E-88 |
| 41 | 1.65E-39 | 8.87E-19 | 2.41E-107 | 6.05E-97 |
| 42 | 8.79E-40 | 1.09E-14 | 3.00E-106 | 3.22E-94 |
| 43 | 1.21E-43 | 1.71E-19 | 1.58E-102 | 1.41E-92 |
| 44 | 2.98E-44 | 8.40E-19 | 1.07E-101 | 4.03E-92 |
| 45 | 8.13E-44 | 8.40E-19 | 1.07E-101 | 4.03E-92 |
| 46 | 2.94E-31 | 2.14E-17 | 5.26E-101 | 5.94E-79 |
| 47 | 1.67E-43 | 1.76E-21 | 8.02E-101 | 1.17E-94 |
| 48 | 2.19E-35 | 1.61E-13 | 1.79E-95 | 4.56E-88 |
| 49 | 4.69E-30 | 2.14E-17 | 1.80E-94 | 5.69E-74 |
| 50 | 3.32E-41 | 8.45E-15 | 1.32E-71 | 2.83E-94 |

Table 24h: Data for Staphylococcus, particularly S. aureus (continued)

| No. | Penicillin G_pv_adj | Piperacillin/Tazobactam_pv_adj | Tetracyclin_pv_adj | Tobramycin_pv_adj |
|---|---|---|---|---|
| 1 | 1.37E-41 | 1.55E-21 | 1.22E-01 | 2.16E-15 |
| 2 | 1.37E-41 | 1.55E-21 | 1.22E-01 | 2.16E-15 |
| 3 | 1.51E-41 | 1.55E-21 | 9.63E-02 | 2.16E-15 |
| 4 | 3.46E-40 | 1.17E-19 | 2.75E-02 | 6.21E-16 |
| 5 | 6.83E-41 | 2.25E-18 | 3.70E-02 | 2.16E-15 |
| 6 | 3.80E-41 | 1.59E-17 | 1.27E-02 | 1.94E-13 |
| 7 | 6.95E-35 | 3.69E-14 | 5.90E-03 | 7.81E-15 |
| 8 | 6.95E-35 | 3.69E-14 | 5.90E-03 | 7.81E-15 |
| 9 | 6.95E-35 | 3.69E-14 | 5.90E-03 | 7.81E-15 |
| 10 | 1.50E-34 | 3.69E-14 | 8.03E-03 | 7.81E-15 |
| 11 | 1.50E-34 | 3.69E-14 | 8.03E-03 | 7.81E-15 |

(continued)

| No. | Penicillin G_pv_adj | Piperacillin/Tazobactam_pv_adj | Tetracyclin_pv_adj | Tobramycin_pv_adj |
|-----|---------------------|-------------------------------|--------------------|--------------------|
| 12 | 5.97E-34 | 3.69E-14 | 8.79E-03 | 7.81E-15 |
| 13 | 5.30E-34 | 5.46E-13 | 9.97E-04 | 9.71E-16 |
| 14 | 1.23E-34 | 3.69E-14 | 1.42E-03 | 7.81E-15 |
| 15 | 1.23E-34 | 3.69E-14 | 1.42E-03 | 7.81E-15 |
| 16 | 1.23E-34 | 3.69E-14 | 1.42E-03 | 7.81E-15 |
| 17 | 1.23E-34 | 3.69E-14 | 1.42E-03 | 7.81E-15 |
| 18 | 1.23E-34 | 5.46E-13 | 1.42E-03 | 3.02E-14 |
| 19 | 1.23E-34 | 5.46E-13 | 1.42E-03 | 3.02E-14 |
| 20 | 5.10E-21 | 3.04E-11 | 5.98E-04 | 7.15E-15 |
| 21 | 9.90E-21 | 3.04E-11 | 2.52E-04 | 7.15E-15 |
| 22 | 2.82E-20 | 9.29E-11 | 5.97E-04 | 2.24E-14 |
| 23 | 2.82E-20 | 9.29E-11 | 5.97E-04 | 2.24E-14 |
| 24 | 1.92E-20 | 4.28E-09 | 8.65E-04 | 4.98E-13 |
| 25 | 1.42E-21 | 6.78E-10 | 2.40E-03 | 6.71E-15 |
| 26 | 2.07E-10 | 4.04E-10 | 5.35E-05 | 4.17E-12 |
| 27 | 1.36E-10 | 1.16E-09 | 2.27E-05 | 1.33E-11 |
| 28 | 2.18E-10 | 1.16E-09 | 3.46E-05 | 1.33E-11 |
| 29 | 4.59E-14 | 1.16E-09 | 8.29E-05 | 7.56E-11 |
| 30 | 6.54E-10 | 2.97E-08 | 3.46E-05 | 2.92E-10 |
| 31 | 4.06E-09 | 7.73E-08 | 2.02E-05 | 2.25E-08 |
| 32 | 2.64E-11 | 3.31E-11 | 2.07E-02 | 6.23E-11 |
| 33 | 2.28E-19 | 2.60E-08 | 2.52E-06 | 9.88E-10 |
| 34 | 1.20E-07 | 3.78E-10 | 1.24E-02 | 9.10E-11 |
| 35 | 4.14E-07 | 1.59E-06 | 1.78E-06 | 6.53E-07 |
| 36 | 4.05E-13 | 7.74E-09 | 1.25E-04 | 1.01E-10 |
| 37 | 4.05E-13 | 7.74E-09 | 1.25E-04 | 1.01E-10 |
| 38 | 4.22E-06 | 1.49E-07 | 1.46E-05 | 5.29E-08 |
| 39 | 1.18E-06 | 8.70E-08 | 2.19E-04 | 7.09E-11 |
| 40 | 1.87E-06 | 1.59E-06 | 3.63E-05 | 1.92E-07 |
| 41 | 4.31E-06 | 6.90E-07 | 1.08E-04 | 2.74E-09 |
| 42 | 6.34E-11 | 4.80E-05 | 2.10E-05 | 6.87E-09 |
| 43 | 2.66E-09 | 4.09E-08 | 4.92E-04 | 1.94E-10 |
| 44 | 7.22E-09 | 8.70E-08 | 3.32E-04 | 6.07E-10 |
| 45 | 2.79E-09 | 8.70E-08 | 3.32E-04 | 6.07E-10 |
| 46 | 1.54E-26 | 8.34E-08 | 2.05E-05 | 1.24E-11 |
| 47 | 4.32E-03 | 1.16E-09 | 4.05E-06 | 1.33E-11 |
| 48 | 1.28E-06 | 1.86E-05 | 1.61E-03 | 1.89E-08 |
| 49 | 3.43E-27 | 8.34E-08 | 1.11E-03 | 1.24E-11 |

(continued)

| No. | Penicillin G_pv_adj | Piperacillin/Tazobactam_pv_adj | Tetracyclin_pv_adj | Tobramycin_pv_adj |
|---|---|---|---|---|
| 50 | 2.12E-15 | 1.05E-10 | 6.95E-03 | 1.62E-07 |

Table 24i: Data for Staphylococcus, particularly S. aureus (continued)

| No. | Locus.Tag | Gene. Symbol | GenBank.Accession | Chromosome |
|---|---|---|---|---|
| 1 | HMPREF0791_ 1752 | mecA | ZP_04797671 | |
| 2 | HMPREF0794_1488 | ugpQ | ZP_06614206 | |
| 3 | HMPREF0794_1485 | mecR | ZP_06614203 | |
| 4 | SIAG_00762 | | ZP_06819251 | |
| 5 | HMPREF0789_ 1682 | | ZP_04825874 | |
| 6 | SAMG_01950 | | ZP_05682863 | |
| 7 | Missing locus_tag:FN433596.cds78.83191..82796 | | CBI47961 | |
| 8 | SATW20_00761 | | CBI67631 | |
| 9 | SERP2515 | | YP_190057 | |
| 10 | SAIG_02579 | | ZP_05694555 | |
| 11 | SERP2519 | mecl | YP_190060 | |
| 12 | SERP2505 | | YP_190047 | |
| 13 | HMPREF0794_ 1451 | | ZP_06614169 | |
| 14 | SERP2490 | kdeP | YP_190033 | |
| 15 | SERP2487 | kdpA | YP_190030 | |
| 16 | SERP2485 | kdpC | YP_190028 | |
| 17 | SERP2489 | kdpD | YP_190032 | |
| 18 | SERP2486 | kdpB | YP_190029 | |
| 19 | SAIG_01884 | | ZP_05693385 | |
| 20 | SERP1347 | tnpA-2 | YP_188919 | |
| 21 | SERP1223 | tnpB-1 | YP_188797 | |
| 22 | SLAG_02844 | | ZP_06930609 | |
| 23 | SA0049 | ant (9) | NP_373289 | |
| 24 | SERP1342 | | YP_188914 | |
| 25 | HMPREF0773_2426 | radC | ZP_04829125 | |
| 26 | SAFG_01021 | | ZP_05703394 | |
| 27 | SAFG_01015 | | ZP_05703388 | |
| 28 | SA2981_2149 | | ADC38360 | |
| 29 | ECTR2_1640 | | CBX35020 | |
| 30 | SA2981_1770 | | ADC37981 | |
| 31 | SAB0169 | | YP_415679 | |
| 32 | SaurJH1_0467 | | YP_001315613 | |
| 33 | SERP1345 | tnpC-2 | YP_188917 | |

(continued)

| No. | Locus.Tag | Gene. Symbol | GenBank.Accession | Chromosome |
|---|---|---|---|---|
| 34 | SGAG_02626 | | ZP_06303506 | |
| 35 | ECTR2_ 2075 | | CBX35404 | |
| 36 | HMPREF0774_2643 | | ZP_04869332 | |
| 37 | SAHG_01730 | | ZP_05696261 | |
| 38 | SAA6159_00204 | coa | ADL22160 | |
| 39 | SARG_01832 | | ZP_06332611 | |
| 40 | SAIG_01373 | | ZP_05693621 | |
| 41 | SANG_00111 | | ZP_05682471 | |
| 42 | SAHG_02693 | | ZP_05696106 | |
| 43 | SAJG_00740 | | ZP_05692517 | |
| 44 | SaurJH1_0188 | | YP_001315337 | |
| 45 | SGAG_00714 | | ZP_06301594 | |
| 46 | ECTR2_2580 | | CBX35910 | plasmid pLUH02 |
| 47 | SA2981_0414 | lpl3 | ADC36625 | |
| 48 | SA2981_1977 | agrB | ADC38188 | |
| 49 | SMAG_02765 | | ZP_06817387 | |
| 50 | HMPREF0798_ 02109 | | ZP_07844597 | |

Table 24j: Data for Staphylococcus, particularly S. aureus (continued)

| No. | Start.Coord | End.Coord | Strand | Scaffold. ID |
|---|---|---|---|---|
| 1 | 1335 | 3341 | + | 645076738 |
| 2 | 7348 | 8091 | - | 647019109 |
| 3 | 3685 | 4599 | - | 647019109 |
| 4 | 3987 | 5336 | - | 647537018 |
| 5 | 1842 | 3491 | - | 645076234 |
| 6 | 89170 | 89277 | - | 645958292 |
| 7 | 82796 | 84130 | - | 646862418 |
| 8 | 81146 | 81214 | - | 646862418 |
| 9 | 2570266 | 2571330 | + | 637000098 |
| 10 | 6265 | 7374 | + | 645958144 |
| 11 | 2574428 | 2574799 | - | 637000098 |
| 12 | 2562107 | 2562424 | + | 637000098 |
| 13 | 8938 | 9252 | + | 647019108 |
| 14 | 2549489 | 2550184 | + | 637000098 |
| 15 | 2544901 | 2546577 | - | 637000098 |
| 16 | 2542288 | 2542845 | - | 637000098 |
| 17 | 2546845 | 2549514 | + | 637000098 |

(continued)

| No. | Start.Coord | End.Coord | Strand | Scaffold. ID |
|---|---|---|---|---|
| 18 | 2542861 | 2544882 | - | 637000098 |
| 19 | 76580 | 76657 | - | 645958116 |
| 20 | 1403752 | 1404837 | - | 637000098 |
| 21 | 1255425 | 1257317 | - | 637000098 |
| 22 | 4557 | 5288 | - | 647016972 |
| 23 | 56859 | 57641 | - | 637000072 |
| 24 | 1399547 | 1399606 | + | 637000098 |
| 25 | 4478 | 5164 | - | 645076413 |
| 26 | 277066 | 278082 | + | 645957989 |
| 27 | 272112 | 272231 | - | 645957989 |
| 28 | 2299803 | 2300126 | - | 646862390 |
| 29 | 1804139 | 1805368 | - | 651053129 |
| 30 | 1899111 | 1899692 | + | 646862390 |
| 31 | 209129 | 211012 | + | 637000460 |
| 32 | 487880 | 489091 | + | 640753011 |
| 33 | 1401479 | 1401856 | - | 637000098 |
| 34 | 245 | 686 | - | 647008393 |
| 35 | 2210164 | 2210319 | - | 651053129 |
| 36 | 16441 | 17124 | + | 645076159 |
| 37 | 85620 | 86303 | + | 645958072 |
| 38 | 242193 | 244169 | + | 648231718 |
| 39 | 177946 | 180372 | + | 647535981 |
| 40 | 38140 | 39216 | + | 645958123 |
| 41 | 77988 | 79490 | - | 645958383 |
| 42 | 5009 | 5929 | - | 645958068 |
| 43 | 78131 | 78853 | + | 645958189 |
| 44 | 221049 | 221729 | + | 640753011 |
| 45 | 44419 | 44616 | + | 647008364 |
| 46 | 12682 | 13458 | - | 651053131 |
| 47 | 456367 | 457155 | + | 646862390 |
| 48 | 2096829 | 2097392 | + | 646862390 |
| 49 | 14918 | 15697 | - | 647017059 |
| 50 | 54037 | 54601 | + | 651285048 |

For examples 1 - 12, as well as further testes microorganisms, a consolidated list of the centroids that were found is given in the following Table 25.

Table 25: List of centroids in Examples

| Organism | # samples | MetaRef centroids | MetaRef centroid notes |
|---|---|---|---|
| Acinetobacter baumannii | 448 | baumannii.1380.centroids | |
| Citrobacter koseri | 108 | Citrobacter.1608.centroids | NA for C. koseri, used centroids of genus |
| Enterobacter aerogenes | 298 | aerogenes.5220.centroids | |
| Enterobacter cloacae | 397 | cloacae.3734.centroids | |
| Escherichia coli | 1144 | coli.318.centroids | |
| Klebsiella oxytoca | 389 | Klebsiella.1640.centroids | NA for K. oxytoca, used centroids of genus |
| Klebsiella pneumoniae | 1179 | pneumoniae. 1641.centroids | |
| Morganella morganii | 297 | NA | NA for M. morgannii or Morganella |
| Proteus | 582 | Proteus.2318.centroids | |
| Pseudomonas aeruginosa | 1042 | aeruginosa.640.centroids | |
| Salmonella | 634 | Salmonella.713.centroids | |
| Shigella | 442 | Shigella.730.centroids | |
| Serratia marcescens | 437 | Serratia.1671.centroids | NA for S. marcescens, used centroids of genus |
| Stenotrophomonas maltophilia | 494 | Stenotrophomonas. 2333.centroids | NA for S. maltophilia, used centroids of genus |
| Staphylococcus aureus | 987 | aureus.754.centroids | |
| Staphylococcus aureus | 985 | aureus.754.centroids | |

| | # significant centroids before/after annotation filtering | |
|---|---|---|
| **Organism** | **before** | **after** |
| Acinetobacter baumannii | 2859 | 810 |
| Citrobacter koseri | 0 | 0 |
| Enterobacter aerogenes | 126 | 56 |
| Enterobacter cloacae | 1417 | 763 |
| Escherichia coli | 6739 | 1625 |
| Klebsiella oxytoca | 951 | 507 |
| Klebsiella pneumoniae | 2571 | 908 |
| Morganella morganii | NA | NA |
| Proteus | 3401 | 1504 |
| Pseudomonas aeruginosa | 1256 | 315 |
| Salmonella | 2333 | 670 |
| Shigella | 2046 | 788 |
| Serratia marcescens | 537 | 299 |
| Stenotrophomonas maltophilia | NA | NA |
| Staphylococcus aureus | 1333 | 367 |
| Staphylococcus aureus | 1435 | 395 |

In Table 25 herein the number of significant centroids (with the respective p-values) found by the present method before and after the above filtering process is given. This shows that, while the best 50 gene sequences are given for the Examples in the odd Tables (Tables 1, 3, 5, etc.), more than those 50 gene sequences were actually found for all the examples after filtering, e.g. 810 for Acinetobacter baumannii.

As noted above, for the even Tables (Tables 2, 4, 6, etc.) the data are further processed.

As can be seen from the Examples, similarities in gene sequences can be found over all the bacterial species, showing that they form clusters, which can also be seen in the fact that for several bacteria centroids from other bacterial species are found as suitable, e.g. from E.coli.

The gene sequences referred to in Tables 1 to 24 are also enclosed in the attached sequence listing with sequence ID numbers (SEQ ID Nos) 1 - 639. The SEQ ID Nos are thereby as given in Table 26 for the above sequences, i.e. structural variants. In Table 26 also the feature ID of the sequences, the Locus.Tag (as in Tables 1 to 24), Gen-Bank.Accession (as in Tables 1 to 24), Start.Coord and End.Coord (both as in Tables 1 to 24) are given as well.

Table 26a: SEQ IDs and feature IDs for the centroids given in Tables 1 and 2 for Acinetobacter, particularly A. baumannii

| featureID | Locus.Tag | GenBank. Accession | Start. Coord | End. Coord | SEQ ID NO |
|---|---|---|---|---|---|
| 650404552 | ABK1_1135 | ADX02769 | 1235814 | 1236425 | 540 |
| 647119723 | A60131_010100011170 | ZP_06783174 | 64 | 768 | 541 |
| 640152869 | A1S_0690 | YP_001083736 | 821275 | 822057 | 542 |
| 643439527 | ABBFA_001710 | YP_002325613 | 1834753 | 1835667 | 543 |
| 643439457 | ABBFA_001640 | YP_002325543 | 1759838 | 1761280 | 544 |
| 643433288 | AB57_1876 | YP_002319238 | 1966831 | 1969149 | 545 |
| 643433557 | AB57_2151 | YP_002319507 | 2232790 | 2233716 | 546 |
| 643439455 | ABBFA_001638 | YP_002325541 | 1757930 | 1758571 | 547 |
| 643433553 | AB57_2147 | YP_002319503 | 2226774 | 2228099 | 548 |
| 645199761 | AbauAB_010100009672 | ZP_04661877 | 63870 | 65015 | 549 |
| 647827358 | HMPREF0014_00181 | ZP_05823167 | 195165 | 195419 | 550 |
| 647829665 | HMPREF0014_02450 | ZP_05825436 | 27528 | 28301 | 551 |
| 647829667 | HMPREF0014_02452 | ZP_05825438 | 31818 | 33023 | 552 |
| 648061129 | AOLE_17735 | YP_003733805 | 3783514 | 3783789 | 553 |
| 646296806 | HMPREF0012_01352 | ZP_06057184 | 1494626 | 1495630 | 554 |
| 647829668 | HMPREF0014_02453 | ZP_05825439 | 33027 | 34796 | 555 |
| 650423983 | BDGL_001498 | ADY82084 | 1603798 | 1604811 | 556 |
| 647829704 | HMPREF0014_02489 | ZP_05825475 | 74534 | 74893 | 557 |
| 647827173 | HMPREF0014_00004 | ZP_05822990 | 1819 | 2325 | 558 |
| 637516960 | ACIAD0370 | YP_045140 | 363575 | 363976 | 559 |
| 647829707 | HMPREF0014_02492 | ZP_05825478 | 76454 | 76681 | 560 |
| 643439454 | ABBFA_001637 | YP_002325540 | 1757594 | 1757890 | 561 |
| 650504248 | ABTW07_1894 | ADX92323 | 2004366 | 2004983 | 562 |
| 643438157 | ABBFA_ 000343 | YP_002324276 | 370977 | 371978 | 563 |
| 643434165 | AB57_2771 | YP_002320110 | 2838051 | 2838620 | 564 |
| 648061120 | AOLE_17690 | YP_003733796 | 3773081 | 3773467 | 565 |
| 646308909 | HMPREF0017_03097 | ZP_06071176 | 716 | 1162 | 566 |
| 647114259 | A6013_010100003618 | ZP_06795427 | 6958 | 7416 | 567 |
| 647830563 | HMPREF0014_03338 | ZP_05826324 | 12541 | 13101 | 568 |

(continued)

| featureID | Locus.Tag | GenBank. Accession | Start. Coord | End. Coord | SEQ ID NO |
|---|---|---|---|---|---|
| 647124672 | A6014_010100015962 | ZP_06788050 | 7588 | 7713 | 569 |
| 642586266 | ACICU_00639 | YP_001845298 | 705957 | 707216 | 570 |
| 647978062 | HMPREF0013_02137 | ZP_06692288 | 856694 | 857281 | 571 |
| 650505219 | ABTW07_2865 | ADX93289 | 3040319 | 3041707 | 572 |
| 646302425 | HMPREF0016_03291 | ZP_06064821 | 4553 | 4768 | 573 |
| 643433537 | AB57_2131 | YP_002319487 | 2213458 | 2214801 | 574 |
| 643435008 | AB57_3624 | YP_002320921 | 3730668 | 3731222 | 575 |
| 642588261 | ACICU_02614 | YP_001847273 | 2775538 | 2776941 | 576 |
| 650505217 | ABTW07_2863 | ADX93287 | 3038397 | 3039893 | 577 |
| 645200562 | AbauAB_010100013685 | ZP_04662667 | 70983 | 71513 | 578 |
| 650405706 | ABK1_2278 | ADX03912 | 2403829 | 2405136 | 579 |
| 647115421 | A6013_010100009420 | ZP_06796568 | 2603 | 3454 | 580 |
| 647834238 | HMPREF0010_03320 | ZP_05829937 | 159696 | 159998 | 581 |
| 642587458 | ACICU_01819 | YP_001846478 | 1941428 | 1942675 | 582 |
| 647467847 | HMP0015_1294 | ZP_06727085 | 1088 | 2629 | 583 |
| 643432855 | AB57_1430 | YP_002318806 | 1505450 | 1506382 | 584 |
| 650423079 | BDGL_000595 | ADY81181 | 622112 | 622732 | 585 |
| 647834237 | HMPREF0010_03319 | ZP_05829936 | 158794 | 159609 | 586 |
| 643439664 | ABBFA_001847 | YP_002325750 | 1980078 | 1981988 | 587 |
| 647828044 | HMPREF0014_00860 | ZP_05823846 | 126664 | 127845 | 588 |
| 650504245 | ABTW07_1891 | ADX92320 | 1999835 | 2001676 | 589 |

Table 26b: SEQ IDs and feature IDs for the centroids given in Tables 3 and 4 for Enterobacter, particularly E. aerogenes

| featureID | Locus.Tag | GenBank. Accession | Start. Coord | End. Coord | SEQ ID NO |
|---|---|---|---|---|---|
| 650930441 | EAE_18905 | YP_004593967 | 4057377 | 4057994 | 439 |
| 650930442 | EAE_18910 | YP_004593968 | 4057994 | 4059313 | 440 |
| 650930808 | EAE_20750 | YP_004594334 | 4444576 | 4447206 | 441 |
| 650930796 | EAE_20680 | YP_004594322 | 4432502 | 4433842 | 442 |
| 650928702 | EAE_10325 | YP_004592260 | 2202535 | 2203893 | 443 |
| 650929846 | EAE_15945 | YP_004593379 | 3439556 | 3442066 | 444 |
| 650931509 | EAE_24270 | YP_004595026 | 5174177 | 5175436 | 445 |
| 650930495 | EAE_19175 | YP_004594021 | 4113374 | 4114306 | 446 |
| 650930133 | EAE_17360 | YP_004593662 | 3748049 | 3748279 | 447 |
| 640901832 | ESA_03685 | YP_001439725 | 3623778 | 3624146 | 448 |
| 650930766 | EAE_20530 | YP_004594292 | 4396852 | 4399119 | 449 |
| 647939146 | HMPREF0485_02019 | ZP_06549619 | 347124 | 347486 | 450 |
| 646545888 | Kvar_3843 | YP_003440755 | 4046094 | 4046396 | 451 |

(continued)

| featureID | Locus.Tag | GenBank. Accession | Start. Coord | End. Coord | SEQ ID NO |
|---|---|---|---|---|---|
| 650930460 | EAE_19000 | YP_004593986 | 4078519 | 4079427 | 452 |
| 650930906 | EAE_21240 | YP_004594432 | 4545796 | 4546983 | 453 |
| 648520091 | HMPREF9552_00257 | ZP_07114469 | 14233 | 15036 | 454 |
| 650928948 | EAE_11550 | YP_004592503 | 2477524 | 2479728 | 455 |
| 650930905 | EAE_21235 | YP_004594431 | 4544187 | 4545296 | 456 |
| 650931576 | EAE_24605 | YP_004595093 | 5241493 | 5242410 | 457 |
| 650930132 | EAE_17355 | YP_004593661 | 3747138 | 3747791 | 458 |
| 647150428 | HMPREF0484_0684 | ZP_06013668 | 5606 | 5998 | 459 |
| 650927979 | EAE_06855 | YP_004591574 | 1421376 | 1421699 | 460 |
| 648520089 | HMPREF9552_00255 | ZP_07114467 | 12357 | 13376 | 461 |
| 648520081 | HMPREF9552_00247 | ZP_07114459 | 7786 | 8685 | 462 |
| 646864676 | B21_02377 | CAQ32894 | 2517630 | 2518469 | 463 |
| 650926710 | EAE_00560 | YP_004590329 | 114571 | 115482 | 464 |
| 650927132 | EAE_02625 | YP_004590739 | 557964 | 558680 | 465 |
| 650928697 | EAE_10300 | YP_004592255 | 2197515 | 2198873 | 466 |
| 650931342 | EAE_23410 | YP_004594860 | 4974131 | 4974946 | 467 |
| 650927135 | EAE_02640 | YP_004590742 | 560166 | 561344 | 468 |
| 650931285 | EAE_23150 | YP_004594808 | 4919559 | 4920611 | 469 |
| 650931326 | EAE_23330 | YP_004594844 | 4955309 | 4956457 | 470 |
| 650927876 | EAE_06345 | YP_004591472 | 1317545 | 1318672 | 471 |
| 650927875 | EAE_06340 | YP_004591471 | 1316472 | 1317548 | 472 |
| 650931347 | EAE_23435 | YP_004594865 | 4979540 | 4980544 | 473 |
| 646600448 | KP1_5319 | YP_002921814 | 5064271 | 5065293 | 474 |
| 650931131 | EAE_22395 | YP_004594657 | 4777378 | 4777638 | 475 |
| 650927962 | EAE_06770 | YP_004591557 | 1404935 | 1406026 | 476 |
| 650927963 | EAE_06775 | YP_004591558 | 1406073 | 1406483 | 477 |
| 650927893 | EAE_06430 | YP_004591489 | 1331758 | 1333431 | 478 |
| 650929673 | EAE_15075 | YP_004593207 | 3235642 | 3239709 | 479 |
| 650927293 | EAE_03420 | YP_004590898 | 718097 | 718942 | 480 |
| 650931349 | EAE_23445 | YP_004594867 | 4982582 | 4983136 | 481 |
| 650927295 | EAE_03430 | YP_004590900 | 720009 | 720686 | 482 |
| 650931343 | EAE_23415 | YP_004594861 | 4975309 | 4976445 | 483 |
| 650927873 | EAE_06330 | YP_004591469 | 1314302 | 1315078 | 484 |
| 650927871 | EAE_06320 | YP_004591467 | 1312221 | 1313129 | 485 |
| 650927872 | EAE_06325 | YP_004591468 | 1313194 | 1314276 | 486 |
| 647940333 | HMPREF0485_03181 | ZP_06550780 | 538177 | 539649 | 487 |
| 650931350 | EAE_23450 | YP_004594868 | 4983148 | 4984038 | 488 |
| 650930450 | EAE_18950 | YP_004593976 | 4069127 | 4070251 | 489 |

Table 26c: SEQ IDs and feature IDs for the centroids given in Tables 5 and 6 for Enterobacter, particularly E. cloacae

| featureID | Locus.Tag | GenBank. Accession | Start. Coord | End. Coord | SEQ ID NO |
|---|---|---|---|---|---|
| 646417690 | ETAE_p041 | YP_003297635 | 32101 | 32916 | 490 |
| 646763809 | ECL_A197 | YP_003602692 | 142295 | 142936 | 491 |
| 640940743 | APECO1_O1R60 | YP_001481413 | 68792 | 69511 | 492 |
| 640940741 | APECO1_O1R58 | YP_001481411 | 67490 | 68131 | 493 |
| 646763827 | ECL_A215 | YP_003602710 | 156077 | 157318 | 494 |
| 646763855 | ECL_A245 | YP_003602738 | 176747 | 177670 | 495 |
| 646763823 | ECL_A211 | YP_003602706 | 153338 | 154378 | 496 |
| 646763825 | ECL_A213 | YP_003602708 | 155073 | 155648 | 497 |
| 646248003 | CSAG_04328 | ZP_04559059 | 377133 | 378173 | 498 |
| 646248015 | CSAG_04340 | ZP_04559071 | 388284 | 389441 | 499 |
| 640940755 | APECO1_O1R72 | YP_001481425 | 80263 | 80841 | 500 |
| 640899944 | ESA_01791 | YP_001437881 | 1731242 | 1732198 | 501 |
| 646763822 | ECL_A210 | YP_003602705 | 152860 | 153315 | 502 |
| 648593831 | HMPREF9551_04611 | ZP_07191964 | 97 | 531 | 503 |
| 646775436 | ECL_03822 | YP_003614305 | 3908948 | 3909223 | 504 |
| 642175308 | Salmoentericaente rica_ 010100000105 | ZP_02702049 | 16271 | 16636 | 505 |
| 637424560 | S2707 | NP_838057 | 2602155 | 2602892 | 506 |
| 646775429 | ECL_03815 | YP_003614298 | 3903410 | 3903712 | 507 |
| 651547539 | HMPREF9538_01300 | ZP_08303641 | 6404 | 6640 | 508 |
| 646772072 | ECL_00479 | YP_003610994 | 482851 | 484329 | 509 |
| 646913350 | SFxv_1737 | ADA73942 | 1636971 | 1637258 | 510 |
| 646935982 | EcDH1_2078 | ACX39734 | 2232606 | 2232845 | 511 |
| 648645422 | HMPREF9347_01086 | ZP_07208640 | 2726 | 5692 | 512 |
| 648566451 | HMPREF9530_03894 | ZP_07153755 | 1918 | 3231 | 513 |
| 646771984 | ECL_00391 | YP_003610906 | 409807 | 410283 | 514 |
| 638666498 | EcolE2_01002790 | ZP_00728858 | 14395 | 15375 | 515 |
| 650541764 | ENC_13410 | CBK85143 | 1397946 | 1398188 | 516 |
| 650540998 | ENC_03420 | CBK84384 | 360699 | 361856 | 517 |
| 650543160 | ENC_31170 | CBK86512 | 3151035 | 3151685 | 518 |
| 651601060 | HMPREF9086_ 2435 | ZP_08498173 | 192614 | 193519 | 519 |
| 650541648 | ENC_11900 | CBK85029 | 1250898 | 1252163 | 520 |
| 650540934 | ENC_02650 | CBK84321 | 274155 | 276332 | 521 |
| 650540938 | ENC_02700 | CBK84325 | 280790 | 281458 | 522 |
| 651603135 | HMPREF9086_ 4455 | ZP_08500191 | 111547 | 112680 | 523 |
| 651603134 | HMPREF9086_ 4454 | ZP_08500190 | 111116 | 111559 | 524 |
| 651600721 | HMPREF9086_2100 | ZP_08497838 | 33364 | 34074 | 525 |

(continued)

| featureID | Locus.Tag | GenBank. Accession | Start. Coord | End. Coord | SEQ ID NO |
|---|---|---|---|---|---|
| 651603140 | HMPREF9086_ 4460 | ZP_08500196 | 117314 | 119053 | 526 |
| 650542971 | ENC_28850 | CBK86326 | 2904606 | 2905577 | 527 |
| 651602175 | HMPREF9086_3524 | ZP_08499260 | 8440 | 9756 | 528 |
| 649676833 | Entcl_2219 | YP_003941757 | 2349542 | 2349967 | 529 |
| 649676821 | Entcl_2207 | YP_003941745 | 2334593 | 2335594 | 530 |
| 643111752 | ENTCAN_03923 | ZP_03284103 | 28272 | 29567 | 531 |
| 646772735 | ECL_01132 | YP_003611642 | 1168855 | 1169388 | 532 |
| 646247884 | CSAG_04209 | ZP_04558940 | 254005 | 255187 | 533 |
| 649675377 | Entcl_0777 | YP_003940336 | 812618 | 813094 | 534 |
| 651599450 | HMPREF9086_0855 | ZP_08496597 | 13947 | 15560 | 535 |
| 650542359 | ENC_21030 | CBK85719 | 2148896 | 2151391 | 536 |
| 650542356 | ENC_20990 | CBK85716 | 2145171 | 2145458 | 537 |
| 650542357 | ENC_21000 | CBK85717 | 2145486 | 2146736 | 538 |
| 650542360 | ENC_21040 | CBK85720 | 2151395 | 2152267 | 539 |

Table 26d: SEQ IDs and feature IDs for the centroids given in Tables 7 and 8 for Escherichia, particularly E. coli

| featureID | Locus.Tag | GenBank. Accession | Start. Coord | End. Coord | SEQ ID NO |
|---|---|---|---|---|---|
| 642741803 | SeHA_C1580 | YP_ 002045448 | 1525960 | 1526820 | 387 |
| 638655900 | EcolB7_01001258 | ZP_ 00717086 | 77515 | 77910 | 388 |
| 648660988 | HMPREF9345_05066 | ZP_ 07100147 | 4715 | 5554 | 389 |
| 640802276 | KPN_pKPN5p08201 | YP_ 001338811 | 34908 | 35255 | 390 |
| 648581897 | HMPREF9547_03104 | ZP_ 07169558 | 3248 | 3841 | 391 |
| 642745126 | SeSA_B0079 | YP_ 002112958 | 68933 | 70240 | 392 |
| 648661153 | HMPREF9345_05232 | ZP_ 07100311 | 5252 | 5572 | 393 |
| 638677782 | EcolB_01004576 | ZP_ 00708527 | 3555 | 4568 | 394 |
| 643705588 | pEFER_0049 | YP_ 002394596 | 48658 | 49503 | 395 |
| 646417689 | ETAE_p040 | YP_ 003297634 | 31237 | 32040 | 396 |
| 647963395 | ECGG_04429 | ZP_ 06648541 | 19278 | 19826 | 397 |

(continued)

| featureID | Locus.Tag | GenBank. Accession | Start. Coord | End. Coord | SEQ ID NO |
|---|---|---|---|---|---|
| 646417690 | ETAE_p041 | YP_003297635 | 32101 | 32916 | 398 |
| 647963399 | ECGG_04434 | ZP_06648545 | 22618 | 23823 | 399 |
| 646477247 | ROD_p1051 | YP_003368450 | 3339 | 3596 | 400 |
| 646477248 | ROD_p1061 | YP_003368451 | 3529 | 3930 | 401 |
| 650120844 | HMPREF9348_ 05540 | ZP_07692671 | 461 | 1045 | 402 |
| 644762363 | ECUMN_4816 | YP_002415408 | 4994882 | 4995670 | 403 |
| 644762372 | ECUMN_4827 | YP_002415417 | 5003053 | 5003958 | 404 |
| 648661145 | HMPREF9345_05223 | ZP_07100302 | 1167 | 2405 | 405 |
| 641614889 | EcSMS35_A0150 | YP_001740030 | 118542 | 119474 | 406 |
| 648540292 | HMPREF9548_03778 | ZP_07141582 | 4493 | 5755 | 407 |
| 650120865 | HMPREF9348_05561 | ZP_07692692 | 209 | 661 | 408 |
| 648540298 | HMPREF9548_ 03784 | ZP_07141588 | 8819 | 9535 | 409 |
| 640802138 | KPN_pKPN4p07064 | YP_001338673 | 23310 | 24614 | 410 |
| 648659448 | HMPREF9345_03525 | ZP_07098656 | 1071 | 1307 | 411 |
| 640802142 | KPN_pKPN4p07068 | YP_001338677 | 25970 | 27664 | 412 |
| 646266520 | ESCG_01010 | ZP_04871321 | 241682 | 242032 | 413 |
| 648531850 | HMPREF9540_00466 | ZP_07133313 | 192 | 443 | 414 |
| 651065806 | STMDT12_C39340 | BAJ38877 | 4084530 | 4085237 | 415 |
| 643362658 | KPK_A0022 | YP_002235674 | 12106 | 13071 | 416 |
| 642292340 | EcoliO157_010100014809 | ZP_02780941 | 123136 | 123969 | 417 |
| 641614819 | EcSMS35_A0072 | YP_001739960 | 53664 | 54635 | 418 |
| 651546329 | HMPREF9538_00090 | ZP_08302456 | 5657 | 6832 | 419 |

(continued)

| featureID | Locus.Tag | GenBank. Accession | Start. Coord | End. Coord | SEQ ID NO |
|---|---|---|---|---|---|
| 651208632 | ECNA114_2444 | AEG37278 | 2476906 | 2477910 | 420 |
| 642246015 | Eschericcoli0157_010100 006568 | ZP_02749755 | 369676 | 370620 | 421 |
| 646243746 | CSAG_00128 | ZP_04560798 | 146099 | 147763 | 422 |
| 643384474 | ECH74115_0422 | YP_002268988 | 430810 | 432474 | 423 |
| 644121896 | CIT292_01335 | ZP_03835561 | 25505 | 26449 | 424 |
| 646870503 | ECSF_1165 | BAI54705 | 1248345 | 1249289 | 425 |
| 646152269 | EscherichiacoliO157EcO_ 010100009454 | ZP_05948572 | 20015 | 20965 | 426 |
| 640428171 | SSON_0329 | YP_309341 | 356486 | 357301 | 427 |
| 644121897 | CIT292_01336 | ZP_03835562 | 26463 | 27344 | 428 |
| 648532368 | HMPREF9540_00984 | ZP_07133822 | 7204 | 8217 | 429 |
| 637000347 | Missing locus_tag:AC_000091.cds34 0.373092..374105 | AP_001004 | 373092 | 374105 | 430 |
| 648593831 | HMPREF9551_04611 | ZP_07191964 | 97 | 531 | 431 |
| 642742817 | SeHA_C2638 | YP_002046450 | 2550781 | 2552010 | 432 |
| 646912448 | SFxv_0758 | ADA73054 | 734541 | 734921 | 433 |
| 648563988 | HMPREF9530_01431 | ZP_07151340 | 8 | 643 | 434 |
| 640802143 | KPN_pKPN4p07069 | YP_001338678 | 27716 | 28177 | 435 |
| 637430821 | CP0077 | NP_858210 | 61397 | 61579 | 436 |
| 640436898 | SDY_P083 | YP_406084 | 69343 | 69615 | 437 |
| 648534780 | HMPREF9540_03398 | ZP_07136184 | 3415 | 4356 | 438 |

Table 26e: SEQ IDs and feature IDs for the centroids given in Tables 9 and 10 for Klebsiella, particularly K. oxytoca

| featureID | Locus.Tag | GenBank. Accession | Start. Coord | End. Coord | SEQ ID NO |
|---|---|---|---|---|---|
| 643365450 | KPK_2590 | YP_002238421 | 2648871 | 2649476 | 281 |
| 647938340 | HMPREF0485_01216 | ZP_06548816 | 1355295 | 1356167 | 282 |
| 643363160 | KPK_0292 | YP_002236174 | 321413 | 321955 | 283 |
| 650929042 | EAE_11985 | YP_004592590 | 2576463 | 2577677 | 284 |
| 646544818 | Kvar_2784 | YP_003439702 | 2889784 | 2890686 | 285 |

(continued)

| featureID | Locus.Tag | GenBank. Accession | Start. Coord | End. Coord | SEQ ID NO |
|-----------|-----------|--------------------|--------------|------------|-----------|
| 647154493 | HMPREF0484_4675 | ZP_06017656 | 12390 | 13076 | 286 |
| 646544421 | Kvar_2381 | YP_003439305 | 2489613 | 2491040 | 287 |
| 651244334 | KPN2242_04295 | AEJ96779 | 925860 | 926771 | 288 |
| 651245227 | KPN2242_08680 | AEJ97652 | 1837699 | 1838718 | 289 |
| 647939042 | HMPREF0485_01916 | ZP_06549516 | 237063 | 237326 | 290 |
| 647938371 | HMPREF0485_01247 | ZP_06548847 | 1387256 | 1388314 | 291 |
| 643365482 | KPK_2622 | YP_002238453 | 2680912 | 2681727 | 292 |
| 646544606 | Kvar_2568 | YP_003439490 | 2679911 | 2680687 | 293 |
| 646542841 | Kvar_0816 | YP_003437758 | 851698 | 852411 | 294 |
| 646546698 | Kvar_4643 | YP_003441547 | 4934923 | 4936932 | 295 |
| 646545816 | Kvar_3771 | YP_003440683 | 3964905 | 3966425 | 296 |
| 646543636 | Kvar_1596 | YP_003438533 | 1721712 | 1722182 | 297 |
| 647941154 | HMPREF0485_03998 | ZP_06551594 | 140912 | 141796 | 298 |
| 646544182 | Kvar_2140 | YP_003439069 | 2255010 | 2255996 | 299 |
| 646545410 | Kvar_3380 | YP_003440292 | 3519306 | 3520322 | 300 |
| 647937557 | HMPREF0485_00446 | ZP_06548046 | 523538 | 524377 | 301 |
| 640797319 | KPN_00275 | YP_001333958 | 311624 | 312142 | 302 |
| 646543629 | Kvar_1589 | YP_003438526 | 1716249 | 1716779 | 303 |
| 646543901 | Kvar_1856 | YP_003438789 | 1971206 | 1973212 | 304 |
| 646598315 | KP1_3016 | YP_002919724 | 2878963 | 2879583 | 305 |
| 646547185 | Kvar_5099 | YP_003442002 | 5455245 | 5456423 | 306 |
| 646542497 | Kvar_0479 | YP_003437421 | 509672 | 510736 | 307 |
| 647937561 | HMPREF0485_00450 | ZP_06548050 | 528513 | 529175 | 308 |
| 646543626 | Kvar_1586 | YP_003438523 | 1711303 | 1712709 | 309 |
| 646543638 | Kvar_1598 | YP_003438535 | 1723564 | 1724937 | 310 |
| 646543637 | Kvar_1597 | YP_003438534 | 1722169 | 1723554 | 311 |
| 647937554 | HMPREF0485_00443 | ZP_06548043 | 520902 | 521159 | 312 |
| 647937564 | HMPREF0485_00453 | ZP_06548053 | 531062 | 532510 | 313 |
| 646598160 | KP1_2851 | YP_002919569 | 2717504 | 2718439 | 314 |
| 651243688 | KPN2242_01120 | AEJ96149 | 230663 | 231895 | 315 |
| 643365791 | KPK_2936 | YP_002238762 | 2983904 | 2984626 | 316 |
| 651550138 | HMPREF9538_03901 | ZP_08306208 | 15395 | 16450 | 317 |
| 647939618 | HMPREF0485_02477 | ZP_06550077 | 872347 | 873972 | 318 |
| 647938957 | HMPREF0485_01831 | ZP_06549431 | 149922 | 150884 | 319 |
| 646545287 | Kvar_3258 | YP_003440171 | 3387706 | 3389424 | 320 |
| 646545426 | Kvar_3395 | YP_003440307 | 3535340 | 3535786 | 321 |
| 646545243 | Kvar_3210 | YP_003440127 | 3340882 | 3341376 | 322 |
| 640797321 | KPN_00277 | YP_001333960 | 312969 | 315452 | 323 |

(continued)

| featureID | Locus.Tag | GenBank. Accession | Start. Coord | End. Coord | SEQ ID NO |
|---|---|---|---|---|---|
| 647939747 | HMPREF0485_ 02605 | ZP_06550204 | 1009891 | 1010898 | 324 |
| 646543644 | Kvar_1604 | YP_003438541 | 1730820 | 1734335 | 325 |
| 646546601 | Kvar_4545 | YP_003441451 | 4822901 | 4823620 | 326 |
| 651551350 | HMPREF9538_05113 | ZP_08307402 | 52 | 390 | 327 |
| 651245230 | KPN2242_08695 | AEJ97655 | 1839612 | 1840562 | 328 |
| 646546801 | Kvar_4742 | YP_003441646 | 5039066 | 5040004 | 329 |
| 646545290 | Kvar_3261 | YP_003440174 | 3391540 | 3392502 | 330 |
| 646544608 | Kvar_2570 | YP_003439492 | 2681431 | 2682066 | 331 |
| 646544607 | Kvar_2569 | YP_003439491 | 2680692 | 2681420 | 332 |
| 640801580 | KPN_04505 | YP_001338127 | 4930190 | 4931542 | 333 |
| 643364565 | KPK_1698 | YP_002237549 | 1752865 | 1754439 | 334 |
| 648184429 | Dda3937_02155 | YP_003884784 | 4321869 | 4322750 | 335 |
| 643364579 | KPK_1712 | YP_002237563 | 1766440 | 1768002 | 336 |

Table 26f: SEQ IDs and feature IDs for the centroids given in Tables 11 and 12 for Klebsiella, particularly K. pneumoniae

| featureID | Locus.Tag | GenBank. Accession | Start. Coord | End. Coord | SEQ ID NO |
|---|---|---|---|---|---|
| 640431575 | SSON_3896 | YP_312670 | 4110458 | 4111246 | 337 |
| 642741803 | SeHA_C1580 | YP_ 002045448 | 1525960 | 1526820 | 338 |
| 648660988 | HMPREF9345_05066 | ZP_07100147 | 4715 | 5554 | 339 |
| 640802276 | KPN_pKPN5p08201 | YP_ 001338811 | 34908 | 35255 | 340 |
| 642175336 | Salmoentericaenterica _010100000245 | ZP_02702077 | 36757 | 37062 | 341 |
| 651244937 | KPN2242_07235 | AEJ97365 | 1535907 | 1536932 | 342 |
| 651243611 | KPN2242_ 00750 | AEJ96075 | 159235 | 160521 | 343 |
| 647151143 | HMPREF0484_ 1390 | ZP_06014374 | 2865 | 3848 | 344 |
| 646545623 | Kvar_3592 | YP_ 003440504 | 3765814 | 3768501 | 345 |
| 643362735 | KPK_A0103 | YP_ 002235751 | 67987 | 70674 | 346 |
| 647151145 | HMPREF0484_ 1392 | ZP_06014376 | 4691 | 5551 | 347 |
| 648556789 | HMPREF9534_04427 | ZP_07188952 | 2120 | 2368 | 348 |
| 640919120 | CK0_pCK03p06155 | YP_ 001456633 | 8632 | 8931 | 349 |
| 647149781 | HMPREF0484_0044 | ZP_06013029 | 12911 | 13663 | 350 |
| 647152479 | HMPREF0484_ 2697 | ZP_06015679 | 20402 | 22975 | 351 |
| 647941925 | HMPREF0485_04762 | ZP_06552358 | 93128 | 94558 | 352 |
| 638677782 | EcolB_01004576 | ZP_00708527 | 3555 | 4568 | 353 |

(continued)

| featureID | Locus.Tag | GenBank. Accession | Start. Coord | End. Coord | SEQ ID NO |
|---|---|---|---|---|---|
| 647149783 | HMPREF0484_0046 | ZP_06013031 | 14573 | 15328 | 354 |
| 651546679 | HMPREF9538_00440 | ZP_08302804 | 933 | 1943 | 355 |
| 640801967 | KPN_pKPN3p05893 | YP_001338502 | 38514 | 39383 | 356 |
| 651244975 | KPN2242_07425 | AEJ97403 | 1563447 | 1563746 | 357 |
| 640801964 | KPN_pKPN3p05890 | YP_001338499 | 35802 | 36665 | 358 |
| 651546682 | HMPREF9538_00443 | ZP_08302807 | 3648 | 4475 | 359 |
| 640801965 | KPN_pKPN3p05891 | YP_001338500 | 36662 | 37501 | 360 |
| 647152468 | HMPREF0484_2686 | ZP_06015668 | 11204 | 11776 | 361 |
| 647152469 | HMPREF0484_ 2687 | ZP_06015669 | 11773 | 12132 | 362 |
| 647152466 | HMPREF0484_ 2684 | ZP_06015666 | 10265 | 10696 | 363 |
| 646776048 | ECL_04426 | YP_003614904 | 4537618 | 4539336 | 364 |
| 651244973 | KPN2242_07415 | AEJ97401 | 1561697 | 1562869 | 365 |
| 647941078 | HMPREF0485_03923 | ZP_06551519 | 51251 | 51370 | 366 |
| 643278463 | Salmonellentericaente rica_ 010100016432 | ZP_03412648 | 154 | 357 | 367 |
| 647152480 | HMPREF0484_2698 | ZP_06015680 | 23022 | 23267 | 368 |
| 647152476 | HMPREF0484_ 2694 | ZP_06015676 | 19408 | 19923 | 369 |
| 651244959 | KPN2242_07345 | AEJ97387 | 1552497 | 1552712 | 370 |
| 651244956 | KPN2242_07330 | AEJ97384 | 1551079 | 1551729 | 371 |
| 651244955 | KPN2242_07325 | AEJ97383 | 1550023 | 1551075 | 372 |
| 647152458 | HMPREF0484_ 2676 | ZP_06015658 | 6165 | 7229 | 373 |
| 646776056 | ECL_04434 | YP_003614912 | 4543715 | 4546123 | 374 |
| 651550973 | HMPREF9538_04737 | ZP_08307036 | 1555 | 2055 | 375 |
| 647152455 | HMPREF0484_2673 | ZP_06015655 | 2380 | 3411 | 376 |
| 642745126 | SeSA_B0079 | YP_002112958 | 68933 | 70240 | 377 |
| 646544565 | Kvar_2527 | YP_003439449 | 2637299 | 2637790 | 378 |
| 644852960 | Dd1591_2579 | YP_003004893 | 2932956 | 2933891 | 379 |
| 646417690 | ETAE_p041 | YP_003297635 | 32101 | 32916 | 380 |
| 651065806 | STMDT12_C39340 | BAJ38877 | 4084530 | 4085237 | 381 |
| 637638656 | SC026 | YP_209331 | 21137 | 21952 | 382 |
| 651065789 | STMDT12_C39170 | BAJ38860 | 4067283 | 4067942 | 383 |

(continued)

| featureID | Locus.Tag | GenBank. Accession | Start. Coord | End. Coord | SEQ ID NO |
|---|---|---|---|---|---|
| 651548058 | HMPREF9538_01820 | ZP_08304144 | 2070 | 2927 | 384 |
| 637405680 | t1924 | NP_805691 | 1967759 | 1968439 | 385 |
| 646564681 | pK2044_00525 | YP_ 001687935 | 78317 | 81286 | 386 |

Table 26g: SEQ IDs and feature IDs for the centroids given in Tables 13 and 14 for Proteus species

| featureID | Locus.Tag | GenBank. Accession | Start. Coord | End. Coord | SEQ ID NO |
|---|---|---|---|---|---|
| 644095953 | PROPEN_00658 | ZP_03802316 | 42080 | 42412 | 231 |
| 644098111 | PROPEN_02821 | ZP_03804437 | 185454 | 186779 | 232 |
| 644098127 | PROPEN_02837 | ZP_03804453 | 201544 | 202038 | 233 |
| 644097036 | PROPEN_01744 | ZP_03803381 | 122639 | 124333 | 234 |
| 644100055 | PROPEN_04772 | ZP_03806369 | 853119 | 853544 | 235 |
| 644095861 | PROPEN_00566 | ZP_03802226 | 57621 | 58505 | 236 |
| 644096232 | PROPEN_00937 | ZP_03802594 | 44265 | 45014 | 237 |
| 642576615 | PMI0230 | YP_002150011 | 273673 | 274467 | 238 |
| 644096675 | PROPEN_01382 | ZP_03803029 | 115768 | 116316 | 239 |
| 644099592 | PROPEN_04308 | ZP_03805908 | 495434 | 496444 | 240 |
| 644099013 | PROPEN_03726 | ZP_03805332 | 53270 | 53875 | 241 |
| 644095410 | PROPEN_ 00113 | ZP_03801788 | 28708 | 29421 | 242 |
| 644099785 | PROPEN_04501 | ZP_03806101 | 639163 | 640215 | 243 |
| 644098241 | PROPEN_02952 | ZP_03804567 | 285467 | 286066 | 244 |
| 644098652 | PROPEN_03365 | ZP_03804978 | 601755 | 602153 | 245 |
| 642580008 | PMI3610 | YP_002153285 | 3948188 | 3948604 | 246 |
| 644096722 | PROPEN_01429 | ZP_03803076 | 160792 | 161340 | 247 |
| 644443857 | HMPREF0693_2570 | ZP_03841679 | 122510 | 123433 | 248 |
| 644099732 | PROPEN_04448 | ZP_03806048 | 593048 | 593632 | 249 |
| 644099456 | PROPEN_04169 | ZP_03805774 | 390159 | 391736 | 250 |
| 644099457 | PROPEN_04170 | ZP_03805775 | 391736 | 392329 | 251 |
| 644096651 | PROPEN_01358 | ZP_03803005 | 99554 | 100063 | 252 |
| 644098121 | PROPEN_02831 | ZP_03804447 | 195059 | 197509 | 253 |
| 644098540 | PROPEN_03253 | ZP_03804866 | 511254 | 511508 | 254 |
| 644098823 | PROPEN_03536 | ZP_03805144 | 711617 | 713044 | 255 |
| 644098655 | PROPEN_03368 | ZP_03804981 | 603518 | 605086 | 256 |
| 644098522 | PROPEN_03235 | ZP_03804848 | 500249 | 500962 | 257 |
| 644098771 | PROPEN_03484 | ZP_03805093 | 676951 | 677898 | 258 |
| 642579917 | PMI3521 | YP_002153198 | 3840767 | 3841663 | 259 |
| 644096203 | PROPEN_00908 | ZP_03802565 | 23566 | 24027 | 260 |

(continued)

| featureID | Locus.Tag | GenBank. Accession | Start. Coord | End. Coord | SEQ ID NO |
|---|---|---|---|---|---|
| 644095949 | PROPEN_00654 | ZP_03802312 | 39429 | 39599 | 261 |
| 644098586 | PROPEN_03299 | ZP_03804912 | 548639 | 549616 | 262 |
| 644098935 | PROPEN_03648 | ZP_03805254 | 797158 | 797820 | 263 |
| 644096944 | PROPEN_01652 | ZP_03803297 | 48570 | 49238 | 264 |
| 644096607 | PROPEN_01314 | ZP_03802961 | 63033 | 63515 | 265 |
| 644097937 | PROPEN_02647 | ZP_03804265 | 56690 | 57196 | 266 |
| 644097758 | PROPEN_02468 | ZP_03804091 | 284519 | 287461 | 267 |
| 644098568 | PROPEN_03281 | ZP_03804894 | 535246 | 536289 | 268 |
| 642576619 | PMI0234 | YP_002150015 | 279605 | 281008 | 269 |
| 644095840 | PROPEN_00544 | ZP_03802209 | 44077 | 44439 | 270 |
| 644095796 | PROPEN_00500 | ZP_03802168 | 12698 | 14710 | 271 |
| 644098220 | PROPEN_02930 | ZP_03804546 | 268387 | 269874 | 272 |
| 644096053 | PROPEN_00758 | ZP_03802416 | 106241 | 106837 | 273 |
| 644097102 | PROPEN_01810 | ZP_03803447 | 175312 | 176142 | 274 |
| 644099420 | PROPEN_04133 | ZP_03805738 | 362809 | 363438 | 275 |
| 644097889 | PROPEN_02599 | ZP_03804222 | 23652 | 24185 | 276 |
| 644096443 | PROPEN_01149 | ZP_03802800 | 200043 | 201293 | 277 |
| 644444520 | HMPREF0693_3224 | ZP_03842333 | 37235 | 38317 | 278 |
| 642579407 | PMI3023 | YP_002152722 | 3320488 | 3321087 | 279 |
| 644096915 | PROPEN_01623 | ZP_03803268 | 30478 | 31134 | 280 |

Table 26h: SEQ IDs and feature IDs for the centroids given in Tables 15 and 16 for Pseudomonas, particularly P. aeruginosa

| featureID | Locus.Tag | GenBank. Accession | Start. Coord | End. Coord | SEQ ID NO |
|---|---|---|---|---|---|
| 640814388 | PSPA7_5342 | YP_001350673 | 5509888 | 5510901 | 181 |
| 640814381 | PSPA7_5335 | YP_001350666 | 5497616 | 5498380 | 182 |
| 650201462 | PA39016_001160000 | ZP_07794022 | 269 | 829 | 183 |
| 641622117 | PputW619_2322 | YP_001749191 | 2600850 | 2601410 | 184 |
| 640809156 | PSPA7_0102 | YP_001345498 | 112316 | 112732 | 185 |
| 651019365 | PSTAB_1238 | YP_004713608 | 1298354 | 1298788 | 186 |
| 651010134 | PPS_5233 | YP_004704640 | 5930458 | 5930823 | 187 |
| 650202418 | PA39016_001640030 | ZP_07794960 | 29403 | 29642 | 188 |
| 638742734 | PaerC_01001712 | ZP_00968897 | 9109 | 10122 | 189 |
| 650201445 | PA39016_001140033 | ZP_07794005 | 35241 | 35606 | 190 |
| 650202848 | PA39016_001790028 | ZP_07795388 | 25607 | 28270 | 191 |
| 638750382 | Paer2_01003738 | ZP_00973073 | 18240 | 19688 | 192 |
| 650199599 | PA39016_000110208 | ZP_07792174 | 212874 | 213821 | 193 |

(continued)

| featureID | Locus.Tag | GenBank. Accession | Start. Coord | End. Coord | SEQ ID NO |
|---|---|---|---|---|---|
| 638752484 | Paer2_01005870 | ZP_00970967 | 22370 | 34666 | 194 |
| 640809171 | PSPA7_0117 | YP_001345513 | 122819 | 123664 | 195 |
| 640812777 | PSPA7_3727 | YP_001349081 | 3864574 | 3865185 | 196 |
| 650201446 | PA39016_001140034 | ZP_07794006 | 35624 | 37309 | 197 |
| 640814300 | PSPA7_5254 | YP_001350586 | 5409448 | 5411001 | 198 |
| 641622116 | PputW619_2321 | YP_001749190 | 2597880 | 2600846 | 199 |
| 640813868 | PSPA7_4822 | YP_001350163 | 4961575 | 4962753 | 200 |
| 638748787 | Paer2_01002125 | ZP_00974743 | 167495 | 167935 | 201 |
| 647023014 | PaerPAb_010100012576 | ZP_06878458 | 28 | 241 | 202 |
| 639323787 | PA14_15470 | YP_789375 | 1310460 | 1310735 | 203 |
| 638748780 | Paer2_01002118 | ZP_00974736 | 161059 | 161934 | 204 |
| 639190289 | PaerPA_01003088 | ZP_01365958 | 3500513 | 3501292 | 205 |
| 643544457 | PLES_26911 | YP_002440283 | 2885013 | 2885837 | 206 |
| 639190293 | PaerPA_01003092 | ZP_01365962 | 3505406 | 3506134 | 207 |
| 638748804 | Paer2_01002142 | ZP_00974760 | 182786 | 184474 | 208 |
| 640809411 | PSPA7_0357 | YP_001345752 | 366452 | 367480 | 209 |
| 650202415 | PA39016_001640027 | ZP_07794957 | 26408 | 27688 | 210 |
| 639326550 | PA14_49520 | YP_792115 | 4403159 | 4405534 | 211 |
| 639190314 | PaerPA_01003114 | ZP_01365983 | 3528459 | 3529049 | 212 |
| 640812768 | PSPA7_3718 | YP_001349072 | 3855983 | 3856195 | 213 |
| 640809167 | PSPA7_0113 | YP_001345509 | 121405 | 121680 | 214 |
| 638748798 | Paer2_01002136 | ZP_00974754 | 179158 | 179895 | 215 |
| 650201130 | PA39016_001010047 | ZP_07793693 | 48194 | 50227 | 216 |
| 639190296 | PaerPA_01003095 | ZP_01365965 | 3507452 | 3509473 | 217 |
| 639190332 | PaerPA_01003132 | ZP_01366001 | 3547764 | 3548102 | 218 |
| 638748783 | Paer2_01002121 | ZP_00974739 | 163874 | 164434 | 219 |
| 643544426 | PLES_26591 | YP_002440252 | 2856655 | 2857038 | 220 |
| 651175149 | PSTAA_1280 | AEA83190 | 1367161 | 1367601 | 221 |
| 639190295 | PaerPA_01003094 | ZP_01365964 | 3506732 | 3507172 | 222 |
| 650202425 | PA39016_001650006 | ZP_07794967 | 6653 | 8650 | 223 |
| 650202420 | PA39016_001650001 | ZP_07794962 | 2001 | 4451 | 224 |
| 639325061 | PA14_30900 | YP_790630 | 2683111 | 2683836 | 225 |
| 638749047 | Paer2_01002387 | ZP_00974497 | 138340 | 138654 | 226 |
| 638748842 | Paer2_01002180 | ZP_00974798 | 214185 | 216386 | 227 |
| 643806436 | Avin_35690 | YP_002800691 | 3640230 | 3642680 | 228 |
| 650202422 | PA39016_001650003 | ZP_07794964 | 4730 | 5116 | 229 |
| 640812748 | PSPA7_3698 | YP_001349052 | 3833901 | 3834893 | 230 |

Table 26i: SEQ IDs and feature IDs for the centroids given in Tables 17 and 18 for Salmonella species

| featureID | Locus.Tag | GenBank. Accession | Start. Coord | End. Coord | SEQ ID NO |
|---|---|---|---|---|---|
| 642741803 | SeHA_C1580 | YP_002045448 | 1525960 | 1526820 | 590 |
| 642745126 | SeSA_B0079 | YP_002112958 | 68933 | 70240 | 591 |
| 648581897 | HMPREF9547_03104 | ZP_07169558 | 3248 | 3841 | 592 |
| 640802276 | KPN_pKPN5p08201 | YP_001338811 | 34908 | 35255 | 593 |
| 646417690 | ETAE_p041 | YP_003297635 | 32101 | 32916 | 594 |
| 642175336 | Salmoentericaenteric a_010100000245 | ZP_02702077 | 36757 | 37062 | 595 |
| 643705588 | pEFER_0049 | YP_002394596 | 48658 | 49503 | 596 |
| 648660988 | HMPREF9345_05066 | ZP_07100147 | 4715 | 5554 | 597 |
| 646417689 | ETAE_p040 | YP_003297634 | 31237 | 32040 | 598 |
| 638677782 | EcolB_01004576 | ZP_00708527 | 3555 | 4568 | 599 |
| 651550973 | HMPREF9538_04737 | ZP_08307036 | 1555 | 2055 | 600 |
| 648540298 | HMPREF9548_03784 | ZP_07141588 | 8819 | 9535 | 601 |
| 646912800 | SFxv_1142 | ADA73400 | 1108351 | 1108854 | 602 |
| 640431575 | SSON_3896 | YP_312670 | 4110458 | 4111246 | 603 |
| 648593831 | HMPREF9551_04611 | ZP_07191964 | 97 | 531 | 604 |
| 642175313 | Salmoentericaenteric a_010100000130 | ZP_02702054 | 19257 | 19691 | 605 |
| 640802138 | KPN_pKPN4p07064 | YP_001338673 | 23310 | 24614 | 606 |
| 642175308 | Salmoentericaenteric a_010100000105 | ZP_02702049 | 16271 | 16636 | 607 |
| 648659448 | HMPREF9345_03525 | ZP_07098656 | 1071 | 1307 | 608 |
| 648650382 | HMPREF9347_06051 | ZP_07213485 | 812 | 1663 | 609 |
| 646912799 | SFxv_1141 | ADA73399 | 1107342 | 1107968 | 610 |
| 642740195 | SeHA_A0031 | YP_002043869 | 16416 | 17195 | 611 |
| 651065811 | STMDT12_C39390 | BAJ38882 | 4088051 | 4088326 | 612 |
| 640802142 | KPN_pKPN4p07068 | YP_001338677 | 25970 | 27664 | 613 |
| 642745129 | SeSA_B0082 | YP_002112961 | 71061 | 71411 | 614 |
| 642404178 | ESCAB7627_0644 | ZP_02900823 | 233492 | 234697 | 615 |
| 650493305 | STM474_p303 | ADX20580 | 1988 | 2827 | 616 |
| 643728087 | SPC_p013 | YP_002635587 | 10060 | 10842 | 617 |
| 640432386 | SSO_P190 | YP_313445 | 166425 | 168626 | 618 |
| 637430982 | CP0245 | NP_858378 | 198117 | 198515 | 619 |
| 637236351 | PSLT105 | NP_490589 | 86566 | 87285 | 620 |
| 646906224 | STM14_5622 | ACY86531 | 84297 | 86459 | 621 |
| 642404184 | ESCAB7627_0650 | ZP_02900555 | 238788 | 239996 | 622 |
| 643728115 | SPC_p042 | YP_002635616 | 28413 | 29153 | 623 |

(continued)

| featureID | Locus.Tag | GenBank. Accession | Start. Coord | End. Coord | SEQ ID NO |
|---|---|---|---|---|---|
| 643730793 | SPC_2707 | YP_002638248 | 2745099 | 2745596 | 624 |
| 650489384 | STM474_1038 | ADX16744 | 1088568 | 1089272 | 625 |
| 642137144 | Senterienterica_0101 00019467 | ZP_02668762 | 62341 | 62874 | 626 |
| 643728114 | SPC_p041 | YP_002635615 | 27795 | 28358 | 627 |
| 643730794 | SPC_2708 | YP_002638249 | 2745695 | 2746027 | 628 |
| 637211791 | STM1046 | NP_460021 | 1131381 | 1132118 | 629 |
| 650493188 | STM474_p1093 | ADX20463 | 73221 | 73526 | 630 |
| 641871419 | Sentent_010100022581 | ZP_02341659 | 23575 | 23910 | 631 |
| 641325694 | SPAB_05276 | YP_001591385 | 4445787 | 4448009 | 632 |
| 651062903 | STMDT12_C10780 | BAJ36021 | 1141208 | 1141399 | 633 |
| 641304680 | SARI_01515 | YP_001570553 | 1476854 | 1478026 | 634 |
| 641304679 | SARI_01514 | YP_001570552 | 1475252 | 1476844 | 635 |
| 651032178 | SBG_1292 | YP_004730162 | 1414087 | 1415115 | 636 |
| 642137089 | Senterienterica_0101 00019192 | ZP_02668707 | 26832 | 28046 | 637 |
| 643728474 | SPC_0340 | YP_002635966 | 367421 | 368839 | 638 |
| 642127425 | Senteenterica_010100 025269 | ZP_02664569 | 35858 | 36535 | 639 |

Table 26j: SEQ IDs and feature IDs for the centroids given in Tables 19 and 20 for Serratia, particularly S. marcescens

| featureID | Locus.Tag | GenBank. Accession | Start. Coord | End. Coord | SEQ ID NO |
|---|---|---|---|---|---|
| 640936168 | Spro_0657 | YP_001476891 | 721013 | 721786 | 127 |
| 647266283 | HMPREF0758_4528 | ZP_06641192 | 113063 | 113683 | 128 |
| 647266034 | HMPREF0758_4282 | ZP_06640946 | 196203 | 196802 | 129 |
| 647263468 | HMPREF0758_1761 | ZP_06638425 | 14537 | 15286 | 130 |
| 651304843 | SSYM_0272 | ZP_08040542 | 1 | 622 | 131 |
| 647261479 | SOD_m00050 | ZP_06193534 | 5289 | 5756 | 132 |
| 647259397 | SOD_d02380 | ZP_06191491 | 276617 | 277270 | 133 |
| 640939470 | Spro_3910 | YP_001480133 | 4328564 | 4329223 | 134 |
| 647261002 | SOD_j00160 | ZP_06193066 | 10640 | 11029 | 135 |
| 647261488 | SOD_m00140 | ZP_06193543 | 12102 | 12680 | 136 |
| 651303018 | SSYM_0779 | ZP_08038782 | 54235 | 55095 | 137 |
| 649847802 | Pat9b_2844 | YP_004116700 | 3117915 | 3119003 | 138 |
| 651304792 | SSYM_0215 | ZP_08040491 | 1 | 203 | 139 |
| 640936818 | Spro_1286 | YP_001477518 | 1415512 | 1416153 | 140 |
| 647264824 | HMPREF0758_3087 | ZP_06639751 | 166028 | 166624 | 141 |
| 647258402 | SOD_b04490 | ZP_06190513 | 563304 | 564224 | 142 |
| 647263472 | HMPREF0758_1765 | ZP_06638429 | 17164 | 19063 | 143 |
| 647264351 | HMPREF0758_2630 | ZP_06639294 | 1577 | 1921 | 144 |

(continued)

| featureID | Locus.Tag | GenBank. Accession | Start. Coord | End. Coord | SEQ ID NO |
|---|---|---|---|---|---|
| 647263469 | HMPREF0758_1762 | ZP_06638426 | 15286 | 15990 | 145 |
| 647266523 | HMPREF0758_4768 | ZP_06641432 | 357014 | 357931 | 146 |
| 647258082 | SOD_b01290 | ZP_06190194 | 207219 | 208373 | 147 |
| 647265190 | HMPREF0758_3449 | ZP_06640113 | 144943 | 145311 | 148 |
| 647262012 | HMPREF0758_0340 | ZP_06637004 | 46700 | 47314 | 149 |
| 647265870 | HMPREF0758_4122 | ZP_06640786 | 40618 | 41358 | 150 |
| 647263342 | HMPREF0758_1641 | ZP_06638305 | 66823 | 67548 | 151 |
| 647257104 | SOD_a01780 | ZP_06189226 | 183381 | 184292 | 152 |
| 647266163 | HMPREF0758_4410 | ZP_06641074 | 12440 | 13084 | 153 |
| 640936517 | Spro_0998 | YP_001477230 | 1101888 | 1102271 | 154 |
| 647266162 | HMPREF0758_4409 | ZP_06641073 | 11210 | 12427 | 155 |
| 647261475 | SOD_m00010 | ZP_06193530 | 189 | 1409 | 156 |
| 647263454 | HMPREF0758_1747 | ZP_06638411 | 1316 | 1822 | 157 |
| 647258113 | SOD_b01600 | ZP_06190225 | 243135 | 244313 | 158 |
| 647258099 | SOD_b01460 | ZP_06190211 | 227360 | 228310 | 159 |
| 647258790 | SOD_c02470 | ZP_06190898 | 276786 | 277940 | 160 |
| 640938397 | Spro_2856 | YP_001479085 | 3134756 | 3135724 | 161 |
| 640936999 | Spro_1467 | YP_001477699 | 1599118 | 1600077 | 162 |
| 640936530 | Spro_1011 | YP_001477243 | 1112389 | 1113570 | 163 |
| 647259323 | S0D_d01640 | ZP_06191418 | 196893 | 197789 | 164 |
| 646248385 | CSAG_04704 | ZP_04558356 | 17332 | 17544 | 165 |
| 640936426 | Spro_0906 | YP_001477140 | 1005118 | 1006293 | 166 |
| 640938394 | Spro_2853 | YP_001479082 | 3131862 | 3132830 | 167 |
| 640940140 | Spro_4574 | YP_001480795 | 5048349 | 5049131 | 168 |
| 647257785 | SQD_a08590 | ZP_06189897 | 927700 | 928224 | 169 |
| 647259508 | SOD_d03490 | ZP_06191602 | 395510 | 396043 | 170 |
| 647258459 | SOD_b05060 | ZP_06190570 | 616091 | 617041 | 171 |
| 647265872 | HMPREF0758_4124 | ZP_06640788 | 42335 | 42577 | 172 |
| 647263586 | HMPREF0758_1879 | ZP_06638543 | 107941 | 108870 | 173 |
| 647261997 | HMPREF0758_0325 | ZP_06636989 | 30480 | 31511 | 174 |
| 640938408 | Spro_2867 | YP_001479096 | 3146974 | 3147780 | 175 |
| 640936424 | Spro_0904 | YP_001477138 | 1003277 | 1004080 | 176 |
| 647258080 | SOD_b01270 | ZP_06190192 | 202590 | 204044 | 177 |
| 640940016 | Spro_4454 | YP_001480676 | 4927194 | 4927727 | 178 |
| 640939822 | Spro_4262 | YP_001480484 | 4725710 | 4726606 | 179 |
| 647263878 | HMPREF0758_2167 | ZP_06638831 | 70350 | 72779 | 180 |

EP 3 332 028 B1

Table 26k: SEQ IDs and feature IDs for the centroids given in Tables 21 and 22 for Shigella species

| featureID | Locus.Tag | GenBank. Accession | Start. Coord | End. Coord | SEQ ID NO |
|---|---|---|---|---|---|
| 648579985 | HMPREF9547_01191 | ZP_07167682 | 6987 | 7817 | 51 |
| 648593441 | HMPREF9551_04221 | ZP_07191576 | 10102 | 10662 | 52 |
| 648645422 | HMPREF9347_01086 | ZP_07208640 | 2726 | 5692 | 53 |
| 651065789 | STMDT12_C39170 | BAJ38860 | 4067283 | 4067942 | 54 |
| 646912825 | SFxv_1168 | ADA73425 | 1128245 | 1129249 | 55 |
| 638862357 | Sdys1_01000012 | ZP_00922750 | 12375 | 12701 | 56 |
| 648581528 | HMPREF9547_02735 | ZP_07169192 | 4692 | 5189 | 57 |
| 638862358 | Sdys1_01000013 | ZP_00922751 | 14252 | 14776 | 58 |
| 646915562 | SFxv_4146 | ADA76106 | 3950954 | 3952570 | 59 |
| 648583224 | HMPREF9547_04432 | ZP_07170853 | 8898 | 9719 | 60 |
| 648579515 | HMPREF9547_00721 | ZP_07167228 | 5423 | 6016 | 61 |
| 646912799 | SFxv_1141 | ADA73399 | 1107342 | 1107968 | 62 |
| 646417690 | ETAE_p041 | YP_003297635 | 32101 | 32916 | 63 |
| 642404178 | ESCAB7627_0644 | ZP_02900823 | 233492 | 234697 | 64 |
| 640431575 | SSON_3896 | YP_312670 | 4110458 | 4111246 | 65 |
| 646915564 | SFxv_4148 | ADA76108 | 3954100 | 3955767 | 66 |
| 650106182 | SD1617_0118 | ZP_07678322 | 94539 | 95570 | 67 |
| 642404184 | ESCAB7627_0650 | ZP_02900555 | 238788 | 239996 | 68 |
| 648566851 | HMPREF9530_04295 | ZP_07154150 | 2693 | 3169 | 69 |
| 650106096 | SD1617_0032 | ZP_07678236 | 9270 | 9803 | 70 |
| 637638736 | SC109 | YP_209411 | 84616 | 85080 | 71 |
| 643705588 | pEFER_0049 | YP_002394596 | 48658 | 49503 | 72 |
| 650110927 | SD1617_4863 | ZP_07682961 | 80678 | 81835 | 73 |
| 650106187 | SD1617_0123 | ZP_07678327 | 98900 | 99016 | 74 |
| 643500581 | ECED1_1050 | YP_002397074 | 1066134 | 1066832 | 75 |
| 650106107 | SD1617_0043 | ZP_07678247 | 17832 | 18008 | 76 |
| 643016922 | ESCCO14588_3315 | ZP_03445386 | 383590 | 384330 | 77 |
| 650106186 | SD1617_0122 | ZP_07678326 | 98413 | 98754 | 78 |
| 651195849 | UMNK88_1471 | AEE56075 | 1420478 | 1421209 | 79 |
| 644755168 | ECS88_2899 | YP_002392541 | 2863316 | 2863705 | 80 |
| 641860565 | Sente_010100023234 | ZP_02563153 | 17544 | 17792 | 81 |
| 646417689 | ETAE_p040 | YP_003297634 | 31237 | 32040 | 82 |
| 642300854 | Ecol0157_010100025333 | ZP_02795604 | 65402 | 65668 | 83 |
| 644777900 | BWG_3989 | YP_002929180 | 4453522 | 4454475 | 84 |
| 648594808 | HMPREF9551_05588 | ZP_07192915 | 1327 | 1764 | 85 |
| 637062840 | Z1242 | NP_286773 | 1164368 | 1165231 | 86 |
| 646920688 | ECD_04153 | ACT45937 | 4424087 | 4424854 | 87 |

313

(continued)

| featureID | Locus.Tag | GenBank. Accession | Start. Coord | End. Coord | SEQ ID NO |
|---|---|---|---|---|---|
| 650543620 | ENC_36610 | CBK86965 | 3698592 | 3699494 | 88 |
| 651549305 | HMPREF9538_03067 | ZP_08305382 | 2039 | 3037 | 89 |
| 648560907 | HMPREF9550_02956 | ZP_07146070 | 271 | 792 | 90 |
| 647149973 | HMPREF0484_0234 | ZP_06013219 | 4245 | 6485 | 91 |
| 640432108 | SSON_4460 | YP_313172 | 4731572 | 4732528 | 92 |
| 650110134 | SD1617_4069 | ZP_07682183 | 500702 | 501520 | 93 |
| 643277885 | Salmonellentericaente rica_ 010100012935 | ZP_03412076 | 43535 | 45085 | 94 |
| 638864373 | Sdys1_01002088 | ZP_00921058 | 19505 | 20209 | 95 |
| 641724509 | SbBS512_E0266 | YP_001879059 | 266116 | 266985 | 96 |
| 646997990 | EC042_2952 | CBG35781 | 3140492 | 3141238 | 97 |
| 648534138 | HMPREF9540_02755 | ZP_07135552 | 1421 | 1912 | 98 |
| 642815612 | EscherichcoliO157_010 100012850 | ZP_03082702 | 103370 | 103972 | 99 |
| 638666226 | EcolE2_01002516 | ZP_00729149 | 35676 | 36281 | 100 |
| 646596923 | KP1_1529 | YP_002918349 | 1469088 | 1470560 | 101 |
| 643703829 | EFER_2751 | YP_002383857 | 2808563 | 2810692 | 102 |
| 643703038 | EFER_1964 | YP_002383093 | 2010341 | 2013862 | 103 |
| 647969921 | ECEG_03397 | ZP_06652297 | 167911 | 168516 | 104 |
| 648557222 | HMPREF9534_04860 | ZP_07189724 | 4513 | 7977 | 105 |
| 638074148 | SFV_0325 | YP_687904 | 342056 | 344089 | 106 |
| 638076808 | SFV_3136 | YP_690497 | 3209986 | 3212835 | 107 |
| 642745176 | SeSA_C0004 | YP_002113008 | 2398 | 3933 | 108 |
| 644120601 | CIT292_00036 | ZP_03834370 | 7614 | 8387 | 109 |
| 643705078 | EFER_4022 | YP_002385049 | 4127070 | 4127795 | 110 |
| 650106953 | SD1617_0888 | ZP_07679073 | 217087 | 217971 | 111 |
| 641728021 | SbBS512_E4201 | YP_001882442 | 3927571 | 3928530 | 112 |
| 643705082 | EFER_4026 | YP_002385053 | 4130610 | 4131650 | 113 |
| 642127425 | Senteenterica_0101000 25269 | ZP_02664569 | 35858 | 36535 | 114 |
| 640463007 | SSON_PA02 | YP_001139953 | 1155 | 2429 | 115 |
| 643705496 | EFER_4455 | YP_002385453 | 4539896 | 4540645 | 116 |
| 642404124 | ESCAB7627_0589 | ZP_02900787 | 182707 | 183141 | 117 |
| 648232098 | ECABU_c04070 | ADN45010 | 425884 | 427554 | 118 |
| 646934470 | EcDH1_0571 | ACX38258 | 602662 | 603063 | 119 |
| 637425159 | S3383 | NP_838641 | 3261857 | 3262366 | 120 |
| 638693225 | Ecol5_01002315 | ZP_00735973 | 39234 | 40157 | 121 |
| 650101120 | EC236275_0400 | ZP_07778877 | 26968 | 27516 | 122 |
| 642404128 | ESCAB7627_0593 | ZP_02900381 | 185313 | 186593 | 123 |

(continued)

| featureID | Locus.Tag | GenBank. Accession | Start. Coord | End. Coord | SEQ ID NO |
|---|---|---|---|---|---|
| 648565722 | HMPREF9530_03165 | ZP_07153036 | 5665 | 6162 | 124 |
| 641725418 | SbBS512_E1297 | YP_001879929 | 1184368 | 1184799 | 125 |
| 646245992 | CSAG_02340 | ZP_04563010 | 2538496 | 2539857 | 126 |

Table 26l: SEQ IDs and feature IDs for the centroids given in Tables 23 and 24 for Staphylococcus, particularly S. aureus

| featureID | Locus.Tag | GenBank. Accession | Start. Coord | End. Coord | SEQ ID NO |
|---|---|---|---|---|---|
| 645472224 | HMPREF0791_1752 | ZP_04797671 | 1335 | 3341 | 1 |
| 647471630 | HMPREF0794_1488 | ZP 06614206 | 7348 | 8091 | 2 |
| 647471627 | HMPREF0794_1485 | ZP_06614203 | 3685 | 4599 | 3 |
| 648002789 | SIAG_00762 | ZP_06819251 | 3987 | 5336 | 4 |
| 645441498 | HMPREF0789_1682 | ZP_04825874 | 1842 | 3491 | 5 |
| 646071191 | SAMG_01950 | ZP_05682863 | 89170 | 89277 | 6 |
| 646992086 | Missing locus_tag:FN433596.cds78.83191..82796 | CBI47961 | 82796 | 84130 | 7 |
| 646992084 | SATW20_00761 | CBI67631 | 81146 | 81214 | 8 |
| 637169188 | SERP2515 | YP_190057 | 2570266 | 2571330 | 9 |
| 646061724 | SAIG_02579 | ZP_05694555 | 6265 | 7374 | 10 |
| 637169191 | SERP2519 | YP_190060 | 2574428 | 2574799 | 11 |
| 637169178 | SERP2505 | YP_190047 | 2562107 | 2562424 | 12 |
| 647471593 | HMPREF0794_1451 | ZP_06614169 | 8938 | 9252 | 13 |
| 637169164 | SERP2490 | YP_190033 | 2549489 | 2550184 | 14 |
| 637169161 | SERP2487 | YP_190030 | 2544901 | 2546577 | 15 |
| 637169159 | SERP2485 | YP_190028 | 2542288 | 2542845 | 16 |
| 637169163 | SERP2489 | YP_190032 | 2546845 | 2549514 | 17 |
| 637169160 | SERP2486 | YP_190029 | 2542861 | 2544882 | 18 |
| 646060510 | SAIG_01884 | ZP_05693385 | 76580 | 76657 | 19 |
| 637167996 | SERP1347 | YP_188919 | 1403752 | 1404837 | 20 |
| 637167874 | SERP1223 | YP_188797 | 1255425 | 1257317 | 21 |
| 647410778 | SLAG_02844 | ZP_06930609 | 4557 | 5288 | 22 |
| 637091648 | SA0049 | NP_373289 | 56859 | 57641 | 23 |
| 637167991 | SERP1342 | YP_188914 | 1399547 | 1399606 | 24 |
| 645444782 | HMPREF0773_2426 | ZP_04829125 | 4478 | 5164 | 25 |
| 646054124 | SAFG_01021 | ZP_05703394 | 277066 | 278082 | 26 |
| 646054118 | SAFG_01015 | ZP_05703388 | 272112 | 272231 | 27 |
| 646947625 | SA2981_2149 | ADC38360 | 2299803 | 2300126 | 28 |
| 651261316 | ECTR2_1640 | CBX35020 | 1804139 | 1805368 | 29 |

(continued)

| featureID | Locus.Tag | GenBank. Accession | Start. Coord | End. Coord | SEQ ID NO |
|---|---|---|---|---|---|
| 646947191 | SA2981_1770 | ADC37981 | 1899111 | 1899692 | 30 |
| 637815129 | SAB0169 | YP_415679 | 209129 | 211012 | 31 |
| 640778534 | SaurJH1_0467 | YP_001315613 | 487880 | 489091 | 32 |
| 637167994 | SERP1345 | YP_188917 | 1401479 | 1401856 | 33 |
| 647113532 | SGAG_02626 | ZP_06303506 | 245 | 686 | 34 |
| 651261751 | ECTR2_ 2075 | CBX35404 | 2210164 | 2210319 | 35 |
| 645439509 | HMPREF0774_2643 | ZP_04869332 | 16441 | 17124 | 36 |
| 646057908 | SAHG_01730 | ZP_05696261 | 85620 | 86303 | 37 |
| 648259170 | SAA6159_00204 | ADL22160 | 242193 | 244169 | 38 |
| 647901715 | SARG_01832 | ZP_06332611 | 177946 | 180372 | 39 |
| 646060752 | SAIG_01373 | ZP_05693621 | 38140 | 39216 | 40 |
| 646076247 | SANG_00111 | ZP_05682471 | 77988 | 79490 | 41 |
| 646057745 | SAHG_02693 | ZP_05696106 | 5009 | 5929 | 42 |
| 646064933 | SAJG_00740 | ZP_05692517 | 78131 | 78853 | 43 |
| 640778258 | SaurJH1_0188 | YP_001315337 | 221049 | 221729 | 44 |
| 647111570 | SGAG_00714 | ZP_06301594 | 44419 | 44616 | 45 |
| 651262257 | ECTR2_2580 | CBX35910 | 12682 | 13458 | 46 |
| 646945812 | SA2981_0414 | ADC36625 | 456367 | 457155 | 47 |
| 646947438 | SA2981_1977 | ADC38188 | 2096829 | 2097392 | 48 |
| 647413760 | SMAG_02765 | ZP_06817387 | 14918 | 15697 | 49 |
| 651300127 | HMPREF0798_02109 | ZP_07844597 | 54037 | 54601 | 50 |

Further, a synergistic effect of individual structural variants was demonstrated by exhaustively comparing significance levels for association of single structural variants with antibiotic susceptibility/resistance and significance levels for association of combinations of structural variants with antibiotic susceptibility/resistance. For a representative example of 2 structural variants the significance level for synergistic association of two SNPs was improved with the values given in the following compared to the association of either structural variants alone, given for exemplary different antibiotics.

For Acinetobacter baumannii, a combination of featureID numbers 650404552 and 640152869 (as in Table 26a) for CP resulted in a p-value of 1.74373e-72, whereas the minimal p-value of a single feature was 3.053e-66 (non-adjusted) for feature 650404552 for CP, representing an improvement of 175084399.0 percent.

For Enterobacter aerogenes, a combination of featureID numbers 650930441 and 650931509 (as in Table 26b) for CP resulted in a p-value of 3.34152e-15, whereas the minimal p-value of a single feature was 3.88493e-14 (non-adjusted) for feature 650930441 for CP, representing an improvement of 1162.6 percent.

For Enterobacter cloacae, a combination of featureID numbers 646417690 and 646763809 (as in Table 26c) for T/S resulted in a p-value of 5.51273e-44, whereas the minimal p-value of a single feature was 1.51283e-31 (non-adjusted) for feature 646417690 for T/S, representing an improvement of 274425302650065.6 percent.

For Escherichia coli, a combination of featureID numbers 642741803 and 648660988 (as in Table 26d) for AM resulted in a p-value of 3.77569e-236, whereas the minimal p-value of a single feature was 3.87701e-212 (non-adjusted) for feature 642741803 for AM, representing an improvement of 10268341110907467658061414400 percent.

For Klebsiella oxytoca, a combination of featureID numbers 643365450 and 643363160 (as in Table 26e) for A/S resulted in a p-value of 5.39843e-26, whereas the minimal p-value of a single feature was 8.80799e-23 (non-adjusted) for feature 643365450 for A/S, representing an improvement of 163158.3 percent.

For Klebsiella pneumoniae, a combination of featureID numbers 640431575 and 642741803 (as in Table 26f) for A/S resulted in a p-value of 1.0461e-124, whereas the minimal p-value of a single feature was 5.86879e-124 (non-adjusted) for feature 640431575 for TO, representing an improvement of 561.0 percent.

For Pseudomonas aeruginosa, a combination of featureID numbers 640814388 and 640814381 (as in Table 26h) for TO resulted in a p-value of 1.02879e-101, whereas the minimal p-value of a single feature was 4.21928e-82 (non-adjusted) for feature 640814388 for TO, representing an improvement of 4101192210581809004544.0 percent.

For Proteus, a combination of featureID numbers 644095953 and 644444520 (as in Table 26g) for CRM resulted in a p-value of 2.80046e-65, whereas the minimal p-value of a single feature was 5.04929e-65 (non-adjusted) for feature 644099457 for CRM, representing an improvement of 180.3 percent.

For Salmonella, a combination of featureID numbers 642741803 and 640802276 (as in Table 26i) for AM resulted in a p-value of 2.12481e-78, whereas the minimal p-value of a single feature was 1.02922e-63 (non-adjusted) for feature 642741803 for AM, representing an improvement of 48438123192303928.0 percent.

For Shigella, a combination of featureID numbers 648579985 and 648593441 (as in Table 26k) for AUG resulted in a p-value of 1.71179e-63, whereas the minimal p-value of a single feature was 5.83313e-60 (non-adjusted) for feature 648579985 for AUG, representing an improvement of 340761.3 percent.

For Serratia marcescens, a combination of featureID numbers 640936168 and 647266283 (as in Table 26j) for CP resulted in a p-value of 1.81386e-12, whereas the minimal p-value of a single feature was 2.69007e-10 (non-adjusted) for feature 640936168 for LVX, representing an improvement of 14830.6 percent.

Similar results were obtained for other structural variations and combinations thereof with respect to other bacterial species like Staphylococcus, particularly S. aureus, as well as with respect to other antibiotics, but are left out for brevity sake.

A genetic test for the combined pathogen identification and antimicrobial susceptibility testing direct from the patient sample can reduce the time-to actionable result significantly from several days to hours, thereby enabling targeted treatment. Furthermore, this approach will not be restricted to central labs, but point of care devices can be developed that allow for respective tests. Such technology along with the present methods and computer program products could revolutionize the care, e.g. in intense care units or for admissions to hospitals in general. Furthermore, even applications like real time outbreak monitoring can be achieved using the present methods.

Compared to approaches using MALDI-TOF MS, the present approach has the advantage that it covers almost the complete genome and thus enables us to identify the potential genomic structural changes that might be related to resistance. While MALDI-TOF MS can also be used to identify point mutations in bacterial proteins, this technology only detects a subset of proteins and of these not all are equally well covered. In addition, the identification and differentiation of certain related strains is not always feasible.

The present method allows computing a best breakpoint for the separation of isolates into resistant and susceptible groups. The inventors designed a flexible software tool that allows to consider - besides the best breakpoints - also values defined by different guidelines (e.g. European and US guidelines), preparing for an application of the GAST in different countries.

The current approach enables

  a. Identification and validation of markers for genetic identification and susceptibility/resistance testing within one diagnostic test
  b. Validation of known drug targets and modes of action
  c. Detection of potentially novel resistance mechanisms leading to putative novel target / secondary target genes for new therapies

The current method is faster than classical culture-based predictions and has the potential to be faster than tests based on single bases, e.g. SNPs. Most remarkably the current method can help improve the accuracy of novel genetic tests.

With the present methods, structural variations can be detected, which particularly also include copy-number variations that can lead to gene dosage effects.

**Claims**

1.  A computer implemented method of determining structural variations in a genome of a microorganism, particularly a bacterial microorganism, comprising:

     obtaining or providing a first data set of gene sequences of a plurality of clinical isolates of the microorganism, wherein optionally at least a part of the gene sequences of the first data set are assembled;
     analyzing the gene sequences of the first data set for structural variations of the genome to obtain a third data set of structural variants, wherein a structural variation of the genome is where at least two adjacent bases in a gene sequence are changed, and wherein the structural variations are detected alignment-free or annotated to a pan-genome;

providing a second data set of antimicrobial drug, e.g. antibiotic, resistance and/or susceptibility of the plurality of clinical isolates of the microorganism;

correlating the third data set with the second data set and statistically analyzing the correlation; and determining the structural variations in the genome of the microorganism associated with antimicrobial drug, e.g. antibiotic, resistance.

2. The method of claim 1, wherein the structural variation is a deletion of multiple (2 or more) adjacent nucleotides, an insertion of multiple (2 or more) adjacent nucleotides, a substitution of multiple (2 or more) adjacent nucleotides, a duplication of a sequence of multiple (2 or more) adjacent nucleotides, or a translocation of a sequence of multiple (2 or more) adjacent nucleotides.

3. The method of claim 2, wherein the structural variations are annotated to a pan-genome of the microorganism and/or annotated to one or more reference sequences.

4. A diagnostic method of determining an infection of a patient with a microorganism, particularly a bacterial microorganism potentially resistant to antimicrobial drug treatment, comprising the steps of:

   a) determining, in sample obtained or provided from the patient, the presence of at least one structural variation in at least one genetic sequence of the microorganism, particularly bacterial microorganism contained in the sample,
   wherein the structural variation is where at least two adjacent bases in a gene sequence are changed, and wherein the structural variation is determined alignment-free or annotated to a pan-genome, and
   b) determining whether the at least one structural variation is associated with antimicrobial drug, e.g., antibiotic, resistance by correlating the at least one structural variation with a data set of antimicrobial drug, e.g., antibiotic, resistance and/or susceptibility of a plurality of clinical isolates of the microorganism and statistically analyzing the correlation, wherein the presence of said at least one structural variation associated with antimicrobial drug, e.g., antibiotic, resistance is indicative of an infection with an antimicrobial drug resistant microorganism in said patient.

5. A method of selecting a treatment of a patient suffering from an infection with a potentially resistant microorganism, particularly bacterial microorganism, comprising the steps of:

   a) determining, in a sample obtained or provided from the patient, the presence of at least one structural variation in at least one genetic sequence of the microorganism, particularly bacterial microorganism contained in the sample,
   wherein the structural variation is where at least two adjacent bases in a gene sequence are changed, and wherein the structural variation is determined alignment-free or annotated to a pan-genome,
   b) determining whether the at least one structural variation is associated with antimicrobial drug, e.g., antibiotic, resistance by correlating the at least one structural variation with a data set of antimicrobial drug, e.g., antibiotic, resistance and/or susceptibility of a plurality of clinical isolates of the microorganism and statistically analyzing the correlation, wherein the presence of said at least one structural variation associated with antimicrobial drug, e.g., antibiotic, resistance is indicative of a resistance to one or more antimicrobial drugs;
   c) identifying said at least one or more antimicrobial drugs; and
   d) selecting one or more antimicrobial drugs different from the ones identified in step c) and being suitable for the treatment of the infection with the microorganism, particularly the bacterial microorganism.

6. A method of determining structural variations of a genome of a microorganism for a clinical isolate of the microorganism, particularly a bacterial microorganism, comprising obtaining or providing at least one gene sequence of the clinical isolate of the microorganism, particularly the bacterial microorganism; and
determining the presence of structural variations in the at least one gene sequence of the clinical isolate of the microorganism, particularly bacterial microorganism, wherein a structural variation of the genome is where at least two adjacent bases in a gene sequence are changed, and wherein the structural variation is determined alignment-free or annotated to a pan-genome, providing a data set of antimicrobial drug, e.g. antibiotic, resistance and/or susceptibility of the plurality of clinical isolates of the microorganism;
correlating the data set with the structural variations and statistically analyzing the correlation; and determining the structural variations in the genome of the microorganism associated with antimicrobial drug, e.g.

antibiotic, resistance.

7. Computer-readable storage medium storing program code comprising computer executable instructions which, when executed, perform a method according to any one of claims 1 to 3.

8. The method of any one of claims 4 to 6, wherein the structural variation is a deletion of multiple (2 or more) adjacent nucleotides, an insertion of multiple (2 or more) adjacent nucleotides, a substitution of multiple (2 or more) adjacent nucleotides, a duplication of a sequence of multiple (2 or more) adjacent nucleotides, or a translocation of a sequence of multiple (2 or more) adjacent nucleotides.

9. The method of claim 8, wherein the structural variations are annotated to a pan-genome of the microorganism and/or annotated to one or more reference sequences.

**Patentansprüche**

1. Computerimplementiertes Verfahren zum Bestimmen struktureller Variationen in einem Genom eines Mikroorganismus, insbesondere eines bakteriellen Mikroorganismus, das umfasst:

Erhalten oder Bereitstellen eines ersten Datensatzes von Gensequenzen einer Vielzahl von klinischen Isolaten des Mikroorganismus,
wobei gegebenenfalls mindestens ein Teil der Gensequenzen des ersten Datensatzes zusammengesetzt sind,
Analysieren der Gensequenzen des ersten Datensatzes auf strukturelle Variationen des Genoms, um einen dritten Datensatz struktureller Variationen zu erhalten, wobei eine strukturelle Variation des Genoms vorliegt, wenn mindestens zwei benachbarte Basen in einer Gensequenz verändert sind, und wobei die strukturellen Variationen Alignment-frei nachgewiesen werden oder zu einem Pan-Genom annotiert werden,
Bereitstellen eines zweiten Datensatzes antimikrobieller Arzneimittel-, z.B. Antibiotikum-, Resistenz und/oder Empfindlichkeit der Vielzahl von klinischen Isolaten des Mikroorganismus,
Korrelieren des dritten Datensatzes mit dem zweiten Datensatz und statistisches Analysieren der Korrelation, und
Bestimmen der strukturellen Variationen in dem Genom des Mikroorganismus, die mit antimikrobieller Arzneimittel-, z.B. Antibiotikum-, Resistenz assoziiert sind.

2. Verfahren nach Anspruch 1, wobei die strukturelle Variation eine Deletion von mehreren (2 oder mehr) benachbarten Nukleotiden, eine Insertion von mehreren (2 oder mehr) benachbarten Nukleotiden, eine Substitution von mehreren (2 oder mehr) benachbarten Nukleotiden, eine Verdopplung einer Sequenz von mehreren (2 oder mehr) benachbarten Nukleotiden oder eine Translokation einer Sequenz von mehreren (2 oder mehr) benachbarten Nukleotiden ist.

3. Verfahren nach Anspruch 2, wobei die strukturellen Variationen zu einem Pan-Genom des Mikroorganismus annotiert werden und/oder zu einer oder mehreren Referenzsequenzen annotiert werden.

4. Diagnostisches Verfahren zum Bestimmen einer Infektion eines Patienten mit einem Mikroorganismus, insbesondere einem bakteriellen Mikroorganismus, der potentiell resistent gegenüber Behandlung mit antimikrobiellem Arzneimittel ist, das die Schritte umfasst:

a) Bestimmen in einer Probe, die von dem Patienten erhalten oder bereitgestellt wurde, des Vorliegens von mindestens einer strukturellen Variation in mindestens einer genetischen Sequenz des Mikroorganismus, insbesondere bakteriellen Mikroorganismus, der in der Probe enthalten ist,
wobei die strukturelle Variation vorliegt, wenn mindestens zwei benachbarte Basen in einer Gensequenz verändert sind, und wobei die strukturelle Variation Alignment-frei bestimmt wird oder zu einem Pan-Genom annotiert wird, und
b) Bestimmen, ob die mindestens eine strukturelle Variation mit antimikrobieller Arzneimittel-, z.B. Antibiotikum-, Resistenz assoziiert ist durch Korrelieren der mindestens einen strukturellen Variation mit einem Datensatz antimikrobieller Arzneimittel-, z.B. Antibiotikum-, Resistenz und/oder Empfindlichkeit einer Vielzahl von klinischen Isolaten des Mikroorganismus und statistisches Analysieren der Korrelation, wobei das Vorliegen der mindestens einen strukturellen Variation, die mit antimikrobieller Arzneimittel-, z.B. Antibiotikum-, Resistenz assoziiert ist, auf eine Infektion mit einem Mikroorganismus, der gegen ein antimikrobielles Arzneimittel resistent ist, bei dem Patienten hinweist.

**5.** Verfahren zum Auswählen einer Behandlung eines Patienten, der an einer Infektion mit einem potentiell resistenten Mikroorganismus, insbesondere bakteriellen Mikroorganismus, leidet, das die Schritte umfasst:

a) Bestimmen in einer Probe, die von dem Patienten erhalten oder bereitgestellt wurde, des Vorliegens von mindestens einer strukturellen Variation in mindestens einer genetischen Sequenz des Mikroorganismus, insbesondere bakteriellen Mikroorganismus, der in der Probe enthalten ist,

wobei die strukturelle Variation vorliegt, wenn mindestens zwei benachbarte Basen in einer Gensequenz verändert sind, und wobei die strukturelle Variation Alignment-frei bestimmt wird oder zu einem Pan-Genom annotiert wird,

b) Bestimmen, ob die mindestens eine strukturelle Variation mit antimikrobieller Arzneimittel-, z.B. Antibiotikum-, Resistenz assoziiert ist durch Korrelieren der mindestens einen strukturellen Variation mit einem Datensatz antimikrobieller Arzneimittel-, z.B. Antibiotikum-, Resistenz und/oder Empfindlichkeit einer Vielzahl von klinischen Isolaten des Mikroorganismus und statisches Analysieren der Korrelation, wobei das Vorliegen der mindestens einen strukturellen Variation, die mit antimikrobieller Arzneimittel-, z.B. Antibiotikum-, Resistenz assoziiert ist, auf eine Resistenz gegenüber einem oder mehreren antimikrobiellen Arzneimitteln hinweist,

c) Identifizieren des/der mindestens einen oder mehreren antimikrobiellen Arzneimittel und

d) Auswählen eines oder mehrerer antimikrobieller Arzneimittel, das/die sich von denen, die in Schritt c) identifiziert wurden, unterscheidet/unterscheiden und für die Behandlung der Infektion mit dem Mikroorganismus, insbesondere dem bakteriellen Mikroorganismus, geeignet ist/sind.

**6.** Verfahren zum Bestimmen struktureller Variationen eines Genoms eines Mikroorganismus für ein klinisches Isolat des Mikroorganismus, insbesondere eines bakteriellen Mikroorganismus, das

Erhalten oder Bereitstellen von mindestens einer Gensequenz des klinischen Isolats des Mikroorganismus, insbesondere des bakteriellen Mikroorganismus, und

Bestimmen des Vorliegens struktureller Variationen in der mindestens einen Gensequenz des klinischen Isolats des Mikroorganismus, insbesondere bakteriellen Mikroorganismus, wobei eine strukturelle Variation des Genoms vorliegt, wenn mindestens zwei benachbarte Basen in einer Gensequenz verändert sind, und wobei die strukturelle Variation Alignment-frei bestimmt wird oder zu einem Pan-Genom annotiert wird,

Bereitstellen eines Datensatzes antimikrobieller Arzneimittel-, z.B. Antibiotikum-, Resistenz und/oder Empfindlichkeit der Vielzahl von klinischen Isolaten des Mikroorganismus, Korrelieren des Datensatzes mit den strukturellen Variationen und statistisches Analysieren der Korrelation

und

Bestimmen der strukturellen Variationen in dem Genom des Mikroorganismus, die mit antimikrobieller Arzneimittel-, z.B. Antibiotikum-, Resistenz assoziiert sind, umfasst.

**7.** Computerlesbares Speichermedium, das Programmcode speichert, der computerausführbare Befehle umfasst, die, wenn sie ausgeführt werden, ein Verfahren gemäß einem der Ansprüche 1 bis 3 ausführen.

**8.** Verfahren nach einem der Ansprüche 4 bis 6, wobei die strukturelle Variation eine Deletion von mehreren (2 oder mehr) benachbarten Nukleotiden, eine Insertion von mehreren (2 oder mehr) benachbarten Nukleotiden, eine Substitution von mehreren (2 oder mehr) benachbarten Nukleotiden, eine Verdopplung einer Sequenz von mehreren (2 oder mehr) benachbarten Nukleotiden oder eine Translokation einer Sequenz von mehreren (2 oder mehr) benachbarten Nukleotiden ist.

**9.** Verfahren nach Anspruch 8, wobei die strukturellen Variationen zu einem Pan-Genom des Mikroorganismus annotiert werden und/oder zu einer oder mehreren Referenzsequenzen annotiert werden.

**Revendications**

**1.** Procédé, mis en œuvre par ordinateur, de détermination de variations structurelles dans un génome d'un microorganisme, en particulier d'un microorganisme bactérien, comprenant les étapes ci-dessous consistant à :

obtenir ou fournir un premier ensemble de données de séquences de gènes d'une pluralité d'isolats cliniques du microorganisme ;

dans lequel, facultativement, au moins une partie des séquences de gènes du premier ensemble de données est assemblée ;

analyser les séquences de gènes du premier ensemble de données en ce qui concerne les variations structurelles du génome, afin d'obtenir un troisième ensemble de données de variations structurelles, dans lequel une variation structurelle du génome correspond à l'endroit où au moins deux bases adjacentes dans une séquence de gènes sont modifiées, et dans lequel les variations structurelles sont détectées sans alignement ou sont annotées au niveau d'un pangénome ;

fournir un deuxième ensemble de données de résistance et/ou de sensibilité aux médicaments antimicrobiens, par exemple aux antibiotiques, de la pluralité d'isolats cliniques du microorganisme ;

mettre en corrélation le troisième ensemble de données et le deuxième ensemble de données et analyser statistiquement la corrélation ; et

déterminer les variations structurelles dans le génome du microorganisme associées à une résistance aux médicaments antimicrobiens, par exemple aux antibiotiques.

2. Procédé selon la revendication 1, dans lequel la variation structurelle est une délétion de multiples nucléotides adjacents (2 ou plus), une insertion de multiples nucléotides adjacents (2 ou plus), une substitution de multiples nucléotides adjacents (2 ou plus), une duplication d'une séquence de multiples nucléotides adjacents (2 ou plus), ou une translocation d'une séquence de multiples nucléotides adjacents (2 ou plus).

3. Procédé selon la revendication 2, dans lequel les variations structurelles sont annotées au niveau d'un pangénome du microorganisme et/ou sont annotées au niveau d'une ou plusieurs séquences de référence.

4. Procédé de diagnostic pour déterminer une infection d'un patient par un microorganisme, en particulier un microorganisme bactérien potentiellement résistant à un traitement médicamenteux antimicrobien, comprenant les étapes ci-dessous consistant à :

a) déterminer, dans un échantillon obtenu ou fourni par le patient, la présence d'au moins une variation structurelle dans au moins une séquence génétique du microorganisme, en particulier d'un microorganisme bactérien contenu dans l'échantillon ; dans lequel la variation structurelle correspond à l'endroit où au moins deux bases adjacentes dans une séquence de gènes sont modifiées, et dans lequel la variation structurelle est déterminée sans alignement ou est annotée au niveau d'un pangénome ; et

b) déterminer si ladite au moins une variation structurelle est associée à une résistance aux médicaments antimicrobiens, par exemple aux antibiotiques, en mettant en corrélation ladite au moins une variation structurelle avec un ensemble de données de résistance et/ou de sensibilité aux médicaments antimicrobiens, par exemple, aux antibiotiques, d'une pluralité d'isolats cliniques du microorganisme, et en analysant statistiquement la corrélation, dans lequel la présence de ladite au moins une variation structurelle associée à une résistance aux médicaments antimicrobiens, par exemple aux antibiotiques, est indicative d'une infection par un microorganisme résistant aux médicaments antimicrobiens chez ledit patient.

5. Procédé de sélection d'un traitement thérapeutique d'un patient souffrant d'une infection par un microorganisme potentiellement résistant, en particulier un microorganisme bactérien, comprenant les étapes ci-dessous consistant à :

a) déterminer, dans un échantillon obtenu ou fourni par le patient, la présence d'au moins une variation structurelle dans au moins une séquence génétique du microorganisme, en particulier du microorganisme bactérien contenu dans l'échantillon ; dans lequel la variation structurelle correspond à l'endroit où au moins deux bases adjacentes dans une séquence de gènes sont modifiées, et dans lequel la variation structurelle est déterminée sans alignement ou est annotée au niveau d'un pangénome ;

b) déterminer si ladite au moins une variation structurelle est associée à une résistance aux médicaments antimicrobiens, par exemple aux antibiotiques, en mettant en corrélation ladite au moins une variation structurelle avec un ensemble de données de résistance et/ou de sensibilité aux médicaments antimicrobiens, par exemple, aux antibiotiques, d'une pluralité d'isolats cliniques du microorganisme, et en analysant statistiquement la corrélation, dans lequel la présence de ladite au moins une variation structurelle associée à une résistance aux médicaments antimicrobiens, par exemple aux antibiotiques, est indicative d'une résistance à un ou plusieurs médicaments antimicrobiens ;

c) identifier ledit au moins un ou lesdits plusieurs médicaments antimicrobiens ; et

d) sélectionner un ou plusieurs médicaments antimicrobiens différents de ceux identifiés à l'étape c), et qui sont pertinents pour le traitement thérapeutique de l'infection par le microorganisme, en particulier le microorganisme bactérien.

6. Procédé de détermination de variations structurelles d'un génome d'un microorganisme en ce qui concerne un isolat clinique du microorganisme, en particulier un microorganisme bactérien, comprenant les étapes consistant à :

obtenir ou fournir au moins une séquence de gènes de l'isolat clinique du microorganisme, en particulier du microorganisme bactérien ; et

déterminer la présence de variations structurelles dans ladite au moins une séquence de gènes de l'isolat clinique du microorganisme, en particulier du microorganisme bactérien, dans lequel une variation structurelle du génome correspond à l'endroit où au moins deux bases adjacentes dans une séquence de gènes sont modifiées, et dans lequel la variation structurelle est déterminée sans alignement ou est annotée au niveau d'un pangénome ;

fournir un ensemble de données de résistance et/ou de sensibilité aux médicaments antimicrobiens, par exemple aux antibiotiques, de la pluralité d'isolats cliniques du microorganisme ;

mettre en corrélation l'ensemble de données avec les variations structurelles et analyser statistiquement la corrélation ; et

déterminer les variations structurelles dans le génome du microorganisme associées à une résistance aux médicaments antimicrobiens, par exemple aux antibiotiques.

7. Support de stockage lisible par ordinateur stockant un code de programme comprenant des instructions exécutables par ordinateur qui, lorsqu'elles sont exécutées, mettent en œuvre un procédé selon l'une quelconque des revendications 1 à 3.

8. Procédé selon l'une quelconque des revendications 4 à 6, dans lequel la variation structurelle est une délétion de multiples nucléotides adjacents (2 ou plus), une insertion de multiples nucléotides adjacents (2 ou plus), une substitution de multiples nucléotides adjacents (2 ou plus), une duplication d'une séquence de multiples nucléotides adjacents (2 ou plus), ou une translocation d'une séquence de multiples nucléotides adjacents (2 ou plus).

9. Procédé selon la revendication 8, dans lequel les variations structurelles sont annotées au niveau d'un pangénome du microorganisme et/ou sont annotées au niveau d'une ou plusieurs séquences de référence.

**EP 3 332 028 B1**

**323**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012027302 A2
- WO 2013142378 A1
- WO 2012106432 A2
- WO 2006097232 A2

### Non-patent literature cited in the description

- **WOZNIAK et al.** *BMC Genomics,* 2012, vol. 13 (7), S23
- *J Antimicrob Chemother,* 2013, vol. 68, 2234-2244
- **CHEWAPREECHA et al.** Comprehensive Identification of single nucleotid polymorphisms associated with beta-lactam resistance within pneumococcal mosaic genes. *PLoS Genet,* 2014, vol. 10 (8), e1004547
- **RASKO et al.** *J. Bacteriology,* 2008, vol. 190, 6881-6893
- **HOWDEN et al.** *PLoS,* 2011, vol. 7, e1002359
- **YU.** *BMC Bioinformatics,* 2010, vol. 11, 508
- **HOWDEN et al.** *Antimicrobial Agents and Chemotherapy,* 2008, vol. 52, 3755-3762
- **KENNEDY et al.** *Proc. Natl. Acad. Sci. USA,* 2008, vol. 105, 1327-1332
- **BANKEVICH et al.** *J Comp. Biol.,* 2012, vol. 19, 455-477
- **PAGE et al.** *Bioinformatics,* 2015, vol. 31, 3691-3693
- **FORTH ; HENSCHLER ; RUMMEL.** Allgemeine und spezielle Pharmakologie und Toxikologie. 2005, 781-919
- Remington, The Science and Practice of Pharmacy. 2013, 777-1070